# EUROPEAN PATENT APPLICATION

(11) **EP 4 035 674 A1**
(43) Date of publication of application: **03.08.2022**
(21) Application number: 22150767.6
(22) Date of filing: 17.09.2018
(51) Int. Cl.: A61K 39/00, C07K 16/30, A61P 35/00

(54) **METHODS AND SYSTEMS FOR PERFORMING A PATIENT-SPECIFIC IMMUNOTHERAPY PROCEDURE WITH CHAIN-OF-CUSTODY AND CHAIN-OF-IDENTITY BIOLOGICAL SAMPLE TRACKING**

(30) Priority: 15.09.2017 US 201762559330 P; 02.10.2017 US 201762566912 P
(62) Divisional of application: 18788926.6
(71) Applicant: Kite Pharma, Inc., Santa Monica, CA 90404 (US)
(72) Inventor: SUSARCHICK, Debra, Summit, 07901 (US); UHRIN, John, Birmingham, 35213 (US); KORFIN, Michele, Glen Gardner, 08826 (US)
(74) Representative: Schnappauf, Georg

(57) **Abstract**

Methods and apparatuses are described for performing a patient-specific immunotherapy procedure. A computing device receives a request to create transfected T cells for a patient. The computing device generates a patient-specific identifier associated with the cell order request. The computing device initiates a process to create transfected T cells for infusion into the patient's bloodstream, comprising: performing a leukapheresis procedure on a sample of the patient's blood to collect T cells from the sample, transferring the collected T cells to a container, labeling the container with the patient-specific identifier, transmitting the collected T cells to a manufacturing facility, creating transfected T cells from the collected T cells using a cell modification technique, receiving the transfected T cells from the manufacturing facility, and infusing the transfected T cells into the patient's bloodstream. The computing device records a tracking event for each step, including the patient-specific identifier, to generate a chain of custody of the patient's T cells.

SEQUENCE LISTING

<110> KITE PHARMA, INC.

<120> METHODS AND SYSTEMS FOR PERFORMING A PATIENT-SPECIFIC
      IMMUNOTHERAPY PROCEDURE WITH CHAIN-OF-CUSTODY AND
      CHAIN-OF-IDENTITY BIOLOGICAL SAMPLE TRACKING

<130> 411-209 PCTEPT1

<140>
<141> 2018-09-17

<150> 62/566,912
<151> 2017-10-02

<150> 62/559,330
<151> 2017-09-15

<160> 326

<170> PatentIn version 3.5

<210> 1
<211> 294
<212> DNA
<213> Artificial Sequence

<220>
<221> source
<223> /note="Description of Artificial Sequence: Synthetic
      polynucleotide"

<400> 1
cttgataatg aaaagtcaaa cggaacaatc attcacgtga agggcaagca cctctgtccg      60

tcacccttgt tccctggtcc atccaagcca ttctgggtgt tggtcgtagt gggtggagtc     120

ctcgcttgtt actctctgct cgtcaccgtg gcttttataa tcttctgggt tagatccaaa     180

agaagccgcc tgctccatag cgattacatg aatatgactc cacgccgccc tggccccaca     240

aggaaacact accagcctta cgcaccacct agagatttcg ctgcctatcg gagc           294


<210> 2
<211> 98
<212> PRT
<213> Artificial Sequence

<220>
<221> source
<223> /note="Description of Artificial Sequence: Synthetic
      polypeptide"

<400> 2
Leu Asp Asn Glu Lys Ser Asn Gly Thr Ile Ile His Val Lys Gly Lys
1               5                   10                  15


His Leu Cys Pro Ser Pro Leu Phe Pro Gly Pro Ser Lys Pro Phe Trp
            20                  25                  30

Val Leu Val Val Val Gly Gly Val Leu Ala Cys Tyr Ser Leu Leu Val
        35                  40                  45

Thr Val Ala Phe Ile Ile Phe Trp Val Arg Ser Lys Arg Ser Arg Leu
    50                  55                  60

Leu His Ser Asp Tyr Met Asn Met Thr Pro Arg Arg Pro Gly Pro Thr
65                  70                  75                  80

Arg Lys His Tyr Gln Pro Tyr Ala Pro Pro Arg Asp Phe Ala Ala Tyr
                85                  90                  95

Arg Ser

<210> 3
<211> 90
<212> DNA
<213> Artificial Sequence

<220>
<221> source
<223> /note="Description of Artificial Sequence: Synthetic
      oligonucleotide"

<400> 3
cttgataatg aaaagtcaaa cggaacaatc attcacgtga agggcaagca cctctgtccg      60

tcacccttgt tccctggtcc atccaagcca                                        90

<210> 4
<211> 30
<212> PRT
<213> Artificial Sequence

<220>
<221> source
<223> /note="Description of Artificial Sequence: Synthetic
      polypeptide"

<400> 4
Leu Asp Asn Glu Lys Ser Asn Gly Thr Ile Ile His Val Lys Gly Lys
1               5                   10                  15

His Leu Cys Pro Ser Pro Leu Phe Pro Gly Pro Ser Lys Pro
            20                  25                  30

<210> 5
<211> 81
<212> DNA
<213> Artificial Sequence

<220>
<221> source

<223> /note="Description of Artificial Sequence: Synthetic
      oligonucleotide"

<400> 5
ttctgggtgt tggtcgtagt gggtggagtc ctcgcttgtt actctctgct cgtcaccgtg      60

gcttttataa tcttctgggt t                                                81


<210> 6
<211> 27
<212> PRT
<213> Artificial Sequence

<220>
<221> source
<223> /note="Description of Artificial Sequence: Synthetic
      peptide"

<400> 6
Phe Trp Val Leu Val Val Val Gly Gly Val Leu Ala Cys Tyr Ser Leu
1               5                   10                  15


Leu Val Thr Val Ala Phe Ile Ile Phe Trp Val
            20                  25


<210> 7
<211> 123
<212> DNA
<213> Artificial Sequence

<220>
<221> source
<223> /note="Description of Artificial Sequence: Synthetic
      polynucleotide"

<400> 7
agatccaaaa gaagccgcct gctccatagc gattacatga atatgactcc acgccgccct      60

ggccccacaa ggaaacacta ccagccttac gcaccaccta gagatttcgc tgcctatcgg     120

agc                                                                   123


<210> 8
<211> 41
<212> PRT
<213> Artificial Sequence

<220>
<221> source
<223> /note="Description of Artificial Sequence: Synthetic
      polypeptide"

<400> 8
Arg Ser Lys Arg Ser Arg Leu Leu His Ser Asp Tyr Met Asn Met Thr
1               5                   10                  15


Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 9
<211> 336
<212> DNA
<213> Artificial Sequence

<220>
<221> source
<223> /note="Description of Artificial Sequence: Synthetic
      polynucleotide"

<400> 9
agggtgaagt tttccagatc tgcagatgca ccagcgtatc agcagggcca gaaccaactg        60

tataacgagc tcaacctggg acgcagggaa gagtatgacg ttttggacaa gcgcagagga       120

cgggaccctg agatgggtgg caaaccaaga cgaaaaaacc cccaggaggg tctctataat       180

gagctgcaga aggataagat ggctgaagcc tattctgaaa taggcatgaa aggagagcgg       240

agaaggggaa aagggcacga cggtttgtac cagggactca gcactgctac gaaggatact       300

tatgacgctc tccacatgca agccctgcca cctagg                                 336

<210> 10
<211> 112
<212> PRT
<213> Artificial Sequence

<220>
<221> source
<223> /note="Description of Artificial Sequence: Synthetic
      polypeptide"

<400> 10
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210> 11
<211> 117
<212> DNA
<213> Artificial Sequence

<220>
<221> source
<223> /note="Description of Artificial Sequence: Synthetic
      polynucleotide"

<400> 11
attgaggtga tgtatccacc gccttacctg gataacgaaa agagtaacgg taccatcatt      60

cacgtgaaag gtaaacacct gtgtccttct cccctcttcc ccgggccatc aaagccc        117

<210> 12
<211> 39
<212> PRT
<213> Artificial Sequence

<220>
<221> source
<223> /note="Description of Artificial Sequence: Synthetic
      polypeptide"

<400> 12
Ile Glu Val Met Tyr Pro Pro Pro Tyr Leu Asp Asn Glu Lys Ser Asn
1               5                   10                  15

Gly Thr Ile Ile His Val Lys Gly Lys His Leu Cys Pro Ser Pro Leu
            20                  25                  30

Phe Pro Gly Pro Ser Lys Pro
        35

<210> 13
<211> 288
<212> DNA
<213> Artificial Sequence

<220>
<221> source
<223> /note="Description of Artificial Sequence: Synthetic
      polynucleotide"

<400> 13
   gctgcagcat tgagcaactc aataatgtat tttagtcact ttgtaccagt gttcttgccg      60
   gctaagccta ctaccacacc cgctccacgg ccacctaccc cagctcctac catcgcttca     120
   cagcctctgt ccctgcgccc agaggcttgc cgaccggccg cagggggcgc tgttcatacc      180
   agaggactgg atttcgcctg cgatatctat atctgggcac ccctggccgg aacctgcggc     240
   gtactcctgc tgtccctggt catcacgctc tattgtaatc acaggaac                288

<210> 14
<211> 96
<212> PRT
<213> Artificial Sequence

<220>
<221> source
<223> /note="Description of Artificial Sequence: Synthetic
      polypeptide"

<400> 14
Ala Ala Ala Leu Ser Asn Ser Ile Met Tyr Phe Ser His Phe Val Pro
1               5                   10                  15

Val Phe Leu Pro Ala Lys Pro Thr Thr Thr Pro Ala Pro Arg Pro Pro
            20                  25                  30

Thr Pro Ala Pro Thr Ile Ala Ser Gln Pro Leu Ser Leu Arg Pro Glu
        35                  40                  45

Ala Cys Arg Pro Ala Ala Gly Gly Ala Val His Thr Arg Gly Leu Asp
    50                  55                  60

Phe Ala Cys Asp Ile Tyr Ile Trp Ala Pro Leu Ala Gly Thr Cys Gly
65                  70                  75                  80

Val Leu Leu Leu Ser Leu Val Ile Thr Leu Tyr Cys Asn His Arg Asn
                85                  90                  95

<210> 15
<211> 366
<212> DNA
<213> Artificial Sequence

<220>
<221> source
<223> /note="Description of Artificial Sequence: Synthetic
      polynucleotide"

<400> 15
caggtgcagc tgcaggaatc cggaccgggg ctggtgaagc ccagcgagac tctgagtctc      60

acgtgtacag tttctggagg tagcattagc tcctactatt ggtcatggat aaggcagccc     120

cccgggaagg gattggaatg gatcggctat atttactaca gtgggagcac caattacaac     180

ccctcactga agtctagagt tacaatcagc gttgacacct caaagaatca gttcagtttg     240

aaattgtcta gcgtcacagc agctgataca gccgtctatt attgtgtttc tctggtctat     300

tgcggtgggg attgttacag tggctttgac tattgggggc agggtactct ggttacagtt     360

tcttcc                                                                366

<210> 16
<211> 122
<212> PRT
<213> Artificial Sequence

<220>
<221> source
<223> /note="Description of Artificial Sequence: Synthetic
      polypeptide"

<400> 16
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Val
                85                  90                  95

Ser Leu Val Tyr Cys Gly Gly Asp Cys Tyr Ser Gly Phe Asp Tyr Trp
            100                 105                 110

Gly Gln Gly Thr Leu Val Thr Val Ser Ser
        115                 120


<210> 17
<211> 7
<212> PRT
<213> Artificial Sequence

<220>
<221> source
<223> /note="Description of Artificial Sequence: Synthetic
      peptide"

<400> 17
Gly Gly Ser Ile Ser Ser Tyr
1               5


<210> 18
<211> 5
<212> PRT

<213> Artificial Sequence

<220>
<221> source
<223> /note="Description of Artificial Sequence: Synthetic
      peptide"

<400> 18
Tyr Tyr Ser Gly Ser
1               5


<210> 19
<211> 14
<212> PRT
<213> Artificial Sequence

<220>
<221> source
<223> /note="Description of Artificial Sequence: Synthetic
      peptide"

<400> 19
Leu Val Tyr Cys Gly Gly Asp Cys Tyr Ser Gly Phe Asp Tyr
1               5                   10


<210> 20
<211> 324
<212> DNA
<213> Artificial Sequence

<220>
<221> source
<223> /note="Description of Artificial Sequence: Synthetic
      polynucleotide"

<400> 20
gacatccagt tgacacagag cccgagttcc ttgtccgcct ccgtcgggga tagagtgtca        60

tttacctgtc aggcctctca ggatattaat aactttctga attggtatca gcaaaagccc       120

ggaaaggcac ccaagctgtt gatttacgac gccagtaacc tggagacagg cgtgccctcc       180

cggtttagtg gtagcggaag cggtacggat tttaccttta ctatcagctc tctccaaccc       240

gaagacattg caacctacta ttgtcaacaa tatggaaacc tgccttttac atttggcggc       300

ggcaccaagg tggagattaa gcgg                                              324


<210> 21
<211> 108
<212> PRT
<213> Artificial Sequence

<220>
<221> source
<223> /note="Description of Artificial Sequence: Synthetic
      polypeptide"

<400> 21
Asp Ile Gln Leu Thr Gln Ser Pro Ser Ser Leu Ser Ala Ser Val Gly

1               5                   10                  15

Asp Arg Val Ser Phe Thr Cys Gln Ala Ser Gln Asp Ile Asn Asn Phe
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Gly Asn Leu Pro Phe
                85                  90                  95

Thr Phe Gly Gly Gly Thr Lys Val Glu Ile Lys Arg
            100                 105

<210> 22
<211> 11
<212> PRT
<213> Artificial Sequence

<220>
<221> source
<223> /note="Description of Artificial Sequence: Synthetic
      peptide"

<400> 22
Gln Ala Ser Gln Asp Ile Asn Asn Phe Leu Asn
1               5                   10

<210> 23
<211> 7
<212> PRT
<213> Artificial Sequence

<220>
<221> source
<223> /note="Description of Artificial Sequence: Synthetic
      peptide"

<400> 23
Asp Ala Ser Asn Leu Glu Thr
1               5

<210> 24
<211> 9
<212> PRT
<213> Artificial Sequence

<220>

<221> source
<223> /note="Description of Artificial Sequence: Synthetic
      peptide"

<400> 24
Gln Gln Tyr Gly Asn Leu Pro Phe Thr
1               5


<210> 25
<211> 1440
<212> DNA
<213> Artificial Sequence

<220>
<221> source
<223> /note="Description of Artificial Sequence: Synthetic
      polynucleotide"

<400> 25
atggcactcc ccgtaactgc tctgctgctg ccgttggcat tgctcctgca cgccgcacgc      60

ccgcaggtcc aactgcaaga aagcggaccc ggactggtga agccttctga gacacttagt     120

ctgacgtgca cggtcagtgg cggctccatc tcctcctatt attggtcatg gatacgacaa     180

cccccaggta agggcctgga atggattggc tatatctact attcaggaag cacgaactac     240

aatcccagcc tgaagtcccg agtgacaatt tcagtagata ccagtaaaaa ccagttcagt     300

cttaaactgt caagcgtgac agctgccgac accgctgtgt attactgcgt ctcactggtg     360

tattgtggag gggattgtta tagcgggttc gattattggg gacagggaac cctggtgact     420

gtatcttccg gcggcggcgg ctcagggggt ggcggtagtg gcggtggggg ttccgatatt     480

caactgacac aatcccccag ctcactcagc gccagcgtgg gggacagggt tagctttacc     540

tgtcaagcct ctcaggatat aaataacttt ctgaactggt atcaacagaa gcctgggaag     600

gcgcccaaac tcctgatcta tgatgcgtcc aacctggaaa ctggcgtgcc ttcacgcttt     660

agcggctctg gcagtggtac agacttcact tttaccatct cttcacttca gccggaggac     720

atcgccacat attactgtca acagtacgga aacttgccct ttacttttgg aggcggcacc     780

aaagttgaaa tcaaaagggc cgctgccctg gataacgaaa agagcaatgg gactataata     840

catgttaaag gaaaacacct gtgtccatct cccctgttcc ctggaccgtc aaagccattt     900

tgggtgctcg tggttgtcgg tggcgttctc gcctgttata gcttgctggt gacagtagcc     960

ttcattatct tttgggtgag atccaaaaga agccgcctgc tccatagcga ttacatgaat    1020

atgactccac gccgccctgg ccccacaagg aaacactacc agccttacgc accacctaga    1080

gatttcgctg cctatcggag cagggtgaag ttttccagat ctgcagatgc accagcgtat    1140

cagcagggcc agaaccaact gtataacgag ctcaacctgg gacgcaggga agagtatgac    1200

gttttggaca agcgcagagg acgggaccct gagatgggtg gcaaaccaag acgaaaaaac    1260

ccccaggagg gtctctataa tgagctgcag aaggataaga tggctgaagc ctattctgaa    1320

ataggcatga aaggagagcg gagaagggga aaagggcacg acggtttgta ccagggactc 1380

agcactgcta cgaaggatac ttatgacgct ctccacatgc aagccctgcc acctaggtaa 1440

<210> 26
<211> 479
<212> PRT
<213> Artificial Sequence

<220>
<221> source
<223> /note="Description of Artificial Sequence: Synthetic
      polypeptide"

<400> 26
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Pro Gly Leu
            20                  25                  30

Val Lys Pro Ser Glu Thr Leu Ser Leu Thr Cys Thr Val Ser Gly Gly
        35                  40                  45

Ser Ile Ser Ser Tyr Tyr Trp Ser Trp Ile Arg Gln Pro Pro Gly Lys
    50                  55                  60

Gly Leu Glu Trp Ile Gly Tyr Ile Tyr Tyr Ser Gly Ser Thr Asn Tyr
65                  70                  75                  80

Asn Pro Ser Leu Lys Ser Arg Val Thr Ile Ser Val Asp Thr Ser Lys
                85                  90                  95

Asn Gln Phe Ser Leu Lys Leu Ser Ser Val Thr Ala Ala Asp Thr Ala
            100                 105                 110

Val Tyr Tyr Cys Val Ser Leu Val Tyr Cys Gly Gly Asp Cys Tyr Ser
        115                 120                 125

Gly Phe Asp Tyr Trp Gly Gln Gly Thr Leu Val Thr Val Ser Ser Gly
    130                 135                 140

Gly Gly Gly Ser Gly Gly Gly Gly Ser Gly Gly Gly Gly Ser Asp Ile
145                 150                 155                 160

Gln Leu Thr Gln Ser Pro Ser Ser Leu Ser Ala Ser Val Gly Asp Arg
                165                 170                 175

Val Ser Phe Thr Cys Gln Ala Ser Gln Asp Ile Asn Asn Phe Leu Asn
            180                 185                 190

Trp Tyr Gln Gln Lys Pro Gly Lys Ala Pro Lys Leu Leu Ile Tyr Asp
        195                 200                 205

Ala Ser Asn Leu Glu Thr Gly Val Pro Ser Arg Phe Ser Gly Ser Gly
    210                 215                 220

Ser Gly Thr Asp Phe Thr Phe Thr Ile Ser Ser Leu Gln Pro Glu Asp
225                 230                 235                 240

Ile Ala Thr Tyr Tyr Cys Gln Gln Tyr Gly Asn Leu Pro Phe Thr Phe
                245                 250                 255

Gly Gly Gly Thr Lys Val Glu Ile Lys Arg Ala Ala Ala Leu Asp Asn
            260                 265                 270

Glu Lys Ser Asn Gly Thr Ile Ile His Val Lys Gly Lys His Leu Cys
        275                 280                 285

Pro Ser Pro Leu Phe Pro Gly Pro Ser Lys Pro Phe Trp Val Leu Val
    290                 295                 300

Val Val Gly Gly Val Leu Ala Cys Tyr Ser Leu Leu Val Thr Val Ala
305                 310                 315                 320

Phe Ile Ile Phe Trp Val Arg Ser Lys Arg Ser Arg Leu Leu His Ser
                325                 330                 335

Asp Tyr Met Asn Met Thr Pro Arg Arg Pro Gly Pro Thr Arg Lys His
            340                 345                 350

Tyr Gln Pro Tyr Ala Pro Pro Arg Asp Phe Ala Ala Tyr Arg Ser Arg
        355                 360                 365

Val Lys Phe Ser Arg Ser Ala Asp Ala Pro Ala Tyr Gln Gln Gly Gln
    370                 375                 380

Asn Gln Leu Tyr Asn Glu Leu Asn Leu Gly Arg Arg Glu Glu Tyr Asp
385                 390                 395                 400

Val Leu Asp Lys Arg Arg Gly Arg Asp Pro Glu Met Gly Gly Lys Pro
                405                 410                 415

Arg Arg Lys Asn Pro Gln Glu Gly Leu Tyr Asn Glu Leu Gln Lys Asp
            420                 425                 430

Lys Met Ala Glu Ala Tyr Ser Glu Ile Gly Met Lys Gly Glu Arg Arg

435                 440                 445

Arg Gly Lys Gly His Asp Gly Leu Tyr Gln Gly Leu Ser Thr Ala Thr
    450                 455                 460

Lys Asp Thr Tyr Asp Ala Leu His Met Gln Ala Leu Pro Pro Arg
465                 470                 475

<210> 27
<211> 1374
<212> DNA
<213> Artificial Sequence

<220>
<221> source
<223> /note="Description of Artificial Sequence: Synthetic
      polynucleotide"

<400> 27
caggtccaac tgcaagaaag cggacccgga ctggtgaagc cttctgagac acttagtctg      60

acgtgcacgg tcagtggcgg ctccatctcc tcctattatt ggtcatggat acgacaaccc     120

ccaggtaagg gcctggaatg gattggctat atctactatt caggaagcac gaactacaat     180

cccagcctga agtcccgagt gacaatttca gtagatacca gtaaaaacca gttcagtctt     240

aaactgtcaa gcgtgacagc tgccgacacc gctgtgtatt actgcgtctc actggtgtat     300

tgtggagggg attgttatag cgggttcgat tattggggac agggaaccct ggtgactgta     360

tcttccggcg gcggcggctc agggggtggc ggtagtggcg gtgggggttc cgatattcaa     420

ctgacacaat cccccagctc actcagcgcc agcgtggggg acagggttag ctttacctgt     480

caagcctctc aggatataaa taactttctg aactggtatc aacagaagcc tgggaaggcg     540

cccaaactcc tgatctatga tgcgtccaac ctggaaactg gcgtgccttc acgctttagc     600

ggctctggca gtggtacaga cttcactttt accatctctt cacttcagcc ggaggacatc     660

gccacatatt actgtcaaca gtacggaaac ttgcccttta cttttggagg cggcaccaaa     720

gttgaaatca aaagggccgc tgccctggat aacgaaaaga gcaatgggac tataatacat     780

gttaaaggaa aacacctgtg tccatctccc ctgttccctg gaccgtcaaa gccattttgg     840

gtgctcgtgg ttgtcggtgg cgttctcgcc tgttatagct tgctggtgac agtagccttc     900

attatctttt gggtgagatc caaaagaagc cgcctgctcc atagcgatta catgaatatg     960

actccacgcc gccctggccc cacaaggaaa cactaccagc cttacgcacc acctagagat    1020

ttcgctgcct atcggagcag ggtgaagttt tccagatctg cagatgcacc agcgtatcag    1080

cagggccaga accaactgta taacgagctc aacctgggac gcagggaaga gtatgacgtt    1140

ttggacaagc gcagaggacg ggaccctgag atgggtggca aaccaagacg aaaaaacccc    1200

caggagggtc tctataatga gctgcagaag gataagatgg ctgaagccta ttctgaaata    1260

ggcatgaaag gagagcggag aaggggaaaa gggcacgacg gtttgtacca gggactcagc 1320

actgctacga aggatactta tgacgctctc cacatgcaag ccctgccacc tagg 1374

<210> 28
<211> 458
<212> PRT
<213> Artificial Sequence

<220>
<221> source
<223> /note="Description of Artificial Sequence: Synthetic
      polypeptide"

<400> 28
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Val
                85                  90                  95

Ser Leu Val Tyr Cys Gly Gly Asp Cys Tyr Ser Gly Phe Asp Tyr Trp
            100                 105                 110

Gly Gln Gly Thr Leu Val Thr Val Ser Ser Gly Gly Gly Gly Ser Gly
        115                 120                 125

Gly Gly Gly Ser Gly Gly Gly Gly Ser Asp Ile Gln Leu Thr Gln Ser
    130                 135                 140

Pro Ser Ser Leu Ser Ala Ser Val Gly Asp Arg Val Ser Phe Thr Cys
145                 150                 155                 160

Gln Ala Ser Gln Asp Ile Asn Asn Phe Leu Asn Trp Tyr Gln Gln Lys
                165                 170                 175

Pro Gly Lys Ala Pro Lys Leu Leu Ile Tyr Asp Ala Ser Asn Leu Glu
            180                 185                 190

Thr Gly Val Pro Ser Arg Phe Ser Gly Ser Gly Ser Gly Thr Asp Phe
        195                 200                 205

Thr Phe Thr Ile Ser Ser Leu Gln Pro Glu Asp Ile Ala Thr Tyr Tyr
    210                 215                 220

Cys Gln Gln Tyr Gly Asn Leu Pro Phe Thr Phe Gly Gly Gly Thr Lys
225                 230                 235                 240

Val Glu Ile Lys Arg Ala Ala Ala Leu Asp Asn Glu Lys Ser Asn Gly
                245                 250                 255

Thr Ile Ile His Val Lys Gly Lys His Leu Cys Pro Ser Pro Leu Phe
            260                 265                 270

Pro Gly Pro Ser Lys Pro Phe Trp Val Leu Val Val Val Gly Gly Val
        275                 280                 285

Leu Ala Cys Tyr Ser Leu Leu Val Thr Val Ala Phe Ile Ile Phe Trp
    290                 295                 300

Val Arg Ser Lys Arg Ser Arg Leu Leu His Ser Asp Tyr Met Asn Met
305                 310                 315                 320

Thr Pro Arg Arg Pro Gly Pro Thr Arg Lys His Tyr Gln Pro Tyr Ala
                325                 330                 335

Pro Pro Arg Asp Phe Ala Ala Tyr Arg Ser Arg Val Lys Phe Ser Arg
            340                 345                 350

Ser Ala Asp Ala Pro Ala Tyr Gln Gln Gly Gln Asn Gln Leu Tyr Asn
        355                 360                 365

Glu Leu Asn Leu Gly Arg Arg Glu Glu Tyr Asp Val Leu Asp Lys Arg
    370                 375                 380

Arg Gly Arg Asp Pro Glu Met Gly Gly Lys Pro Arg Arg Lys Asn Pro
385                 390                 395                 400

Gln Glu Gly Leu Tyr Asn Glu Leu Gln Lys Asp Lys Met Ala Glu Ala
                405                 410                 415

Tyr Ser Glu Ile Gly Met Lys Gly Glu Arg Arg Arg Gly Lys Gly His
            420                 425                 430

Asp Gly Leu Tyr Gln Gly Leu Ser Thr Ala Thr Lys Asp Thr Tyr Asp

435                 440                 445

Ala Leu His Met Gln Ala Leu Pro Pro Arg
    450                 455

<210> 29
<211> 1467
<212> DNA
<213> Artificial Sequence

<220>
<221> source
<223> /note="Description of Artificial Sequence: Synthetic
      polynucleotide"

<400> 29
atggcactcc ccgtaactgc tctgctgctg ccgttggcat tgctcctgca cgccgcacgc      60

ccgcaggtgc agctgcagga atccggaccg gggctggtga agcccagcga gactctgagt     120

ctcacgtgta cagtttctgg aggtagcatt agctcctact attggtcatg gataaggcag     180

ccccccggga agggattgga atggatcggc tatatttact acagtgggag caccaattac     240

aacccctcac tgaagtctag agttacaatc agcgttgaca cctcaaagaa tcagttcagt     300

ttgaaattgt ctagcgtcac agcagctgat acagccgtct attattgtgt ttctctggtc     360

tattgcggtg gggattgtta cagtggcttt gactattggg ggcagggtac tctggttaca     420

gtttcttccg gggggggagg ctctgggggc ggaggctcag gtggtggagg cagcgacatc     480

cagttgacac agagcccgag ttccttgtcc gcctccgtcg gggatagagt gtcatttacc     540

tgtcaggcct ctcaggatat taataacttt ctgaattggt atcagcaaaa gcccggaaag     600

gcacccaagc tgttgattta cgacgccagt aacctggaga caggcgtgcc ctcccggttt     660

agtggtagcg gaagcggtac ggattttacc tttactatca gctctctcca acccgaagac     720

attgcaacct actattgtca acaatatgga aacctgcctt ttacatttgg cggcggcacc     780

aaggtggaga ttaagcgggc ggcagctatt gaggtgatgt atccaccgcc ttacctggat     840

aacgaaaaga gtaacggtac catcattcac gtgaaaggta aacacctgtg tccttctccc     900

ctcttccccg ggccatcaaa gcccttctgg gttcttgtgg tcgtgggagg cgtgcttgct     960

tgttattctc tgctcgttac cgtggcgttt atcatttttt gggttagatc caaaagaagc    1020

cgcctgctcc atagcgatta catgaatatg actccacgcc gccctggccc cacaaggaaa    1080

cactaccagc cttacgcacc acctagagat ttcgctgcct atcggagcag ggtgaagttt    1140

tccagatctg cagatgcacc agcgtatcag cagggccaga accaactgta taacgagctc    1200

aacctgggac gcagggaaga gtatgacgtt ttggacaagc gcagaggacg ggaccctgag    1260

atgggtggca aaccaagacg aaaaaacccc caggagggtc tctataatga gctgcagaag    1320

gataagatgg ctgaagccta ttctgaaata ggcatgaaag gagagcggag aaggggaaaa    1380

gggcacgacg gtttgtacca gggactcagc actgctacga aggatactta tgacgctctc 1440

cacatgcaag ccctgccacc taggtaa 1467

<210> 30
<211> 488
<212> PRT
<213> Artificial Sequence

<220>
<221> source
<223> /note="Description of Artificial Sequence: Synthetic
      polypeptide"

<400> 30
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Pro Gly Leu
            20                  25                  30

Val Lys Pro Ser Glu Thr Leu Ser Leu Thr Cys Thr Val Ser Gly Gly
        35                  40                  45

Ser Ile Ser Ser Tyr Tyr Trp Ser Trp Ile Arg Gln Pro Pro Gly Lys
    50                  55                  60

Gly Leu Glu Trp Ile Gly Tyr Ile Tyr Tyr Ser Gly Ser Thr Asn Tyr
65                  70                  75                  80

Asn Pro Ser Leu Lys Ser Arg Val Thr Ile Ser Val Asp Thr Ser Lys
                85                  90                  95

Asn Gln Phe Ser Leu Lys Leu Ser Ser Val Thr Ala Ala Asp Thr Ala
            100                 105                 110

Val Tyr Tyr Cys Val Ser Leu Val Tyr Cys Gly Gly Asp Cys Tyr Ser
        115                 120                 125

Gly Phe Asp Tyr Trp Gly Gln Gly Thr Leu Val Thr Val Ser Ser Gly
    130                 135                 140

Gly Gly Gly Ser Gly Gly Gly Gly Ser Gly Gly Gly Gly Ser Asp Ile
145                 150                 155                 160

Gln Leu Thr Gln Ser Pro Ser Ser Leu Ser Ala Ser Val Gly Asp Arg
                165                 170                 175

Val Ser Phe Thr Cys Gln Ala Ser Gln Asp Ile Asn Asn Phe Leu Asn
            180                 185                 190

Trp Tyr Gln Gln Lys Pro Gly Lys Ala Pro Lys Leu Leu Ile Tyr Asp
        195                 200                 205

Ala Ser Asn Leu Glu Thr Gly Val Pro Ser Arg Phe Ser Gly Ser Gly
    210                 215                 220

Ser Gly Thr Asp Phe Thr Phe Thr Ile Ser Ser Leu Gln Pro Glu Asp
225                 230                 235                 240

Ile Ala Thr Tyr Tyr Cys Gln Gln Tyr Gly Asn Leu Pro Phe Thr Phe
                245                 250                 255

Gly Gly Gly Thr Lys Val Glu Ile Lys Arg Ala Ala Ala Ile Glu Val
            260                 265                 270

Met Tyr Pro Pro Pro Tyr Leu Asp Asn Glu Lys Ser Asn Gly Thr Ile
        275                 280                 285

Ile His Val Lys Gly Lys His Leu Cys Pro Ser Pro Leu Phe Pro Gly
    290                 295                 300

Pro Ser Lys Pro Phe Trp Val Leu Val Val Val Gly Gly Val Leu Ala
305                 310                 315                 320

Cys Tyr Ser Leu Leu Val Thr Val Ala Phe Ile Ile Phe Trp Val Arg
                325                 330                 335

Ser Lys Arg Ser Arg Leu Leu His Ser Asp Tyr Met Asn Met Thr Pro
            340                 345                 350

Arg Arg Pro Gly Pro Thr Arg Lys His Tyr Gln Pro Tyr Ala Pro Pro
        355                 360                 365

Arg Asp Phe Ala Ala Tyr Arg Ser Arg Val Lys Phe Ser Arg Ser Ala
    370                 375                 380

Asp Ala Pro Ala Tyr Gln Gln Gly Gln Asn Gln Leu Tyr Asn Glu Leu
385                 390                 395                 400

Asn Leu Gly Arg Arg Glu Glu Tyr Asp Val Leu Asp Lys Arg Arg Gly
                405                 410                 415

Arg Asp Pro Glu Met Gly Gly Lys Pro Arg Arg Lys Asn Pro Gln Glu
            420                 425                 430

Gly Leu Tyr Asn Glu Leu Gln Lys Asp Lys Met Ala Glu Ala Tyr Ser

435                 440                 445

Glu Ile Gly Met Lys Gly Glu Arg Arg Arg Gly Lys Gly His Asp Gly
    450                 455                 460

Leu Tyr Gln Gly Leu Ser Thr Ala Thr Lys Asp Thr Tyr Asp Ala Leu
465                 470                 475                 480

His Met Gln Ala Leu Pro Pro Arg
                485

<210> 31
<211> 1401
<212> DNA
<213> Artificial Sequence

<220>
<221> source
<223> /note="Description of Artificial Sequence: Synthetic
      polynucleotide"

<400> 31
caggtgcagc tgcaggaatc cggaccgggg ctggtgaagc ccagcgagac tctgagtctc      60

acgtgtacag tttctggagg tagcattagc tcctactatt ggtcatggat aaggcagccc     120

cccgggaagg gattggaatg gatcggctat atttactaca gtgggagcac caattacaac     180

ccctcactga agtctagagt tacaatcagc gttgacacct caaagaatca gttcagtttg     240

aaattgtcta gcgtcacagc agctgataca gccgtctatt attgtgtttc tctggtctat     300

tgcggtgggg attgttacag tggctttgac tattgggggc agggtactct ggttacagtt     360

tcttccgggg ggggaggctc tgggggcgga ggctcaggtg gtggaggcag cgacatccag     420

ttgacacaga gcccgagttc cttgtccgcc tccgtcgggg atagagtgtc atttacctgt     480

caggcctctc aggatattaa taactttctg aattggtatc agcaaaagcc cggaaaggca     540

cccaagctgt tgatttacga cgccagtaac ctggagacag gcgtgccctc ccggtttagt     600

ggtagcggaa gcggtacgga ttttaccttt actatcagct ctctccaacc cgaagacatt     660

gcaacctact attgtcaaca atatggaaac ctgcctttta catttggcgg cggcaccaag     720

gtggagatta agcgggcggc agctattgag gtgatgtatc caccgcctta cctggataac     780

gaaaagagta acggtaccat cattcacgtg aaaggtaaac acctgtgtcc ttctcccctc     840

ttccccgggc catcaaagcc cttctgggtt cttgtggtcg tgggaggcgt gcttgcttgt     900

tattctctgc tcgttaccgt ggcgtttatc atttttgggg ttagatccaa aagaagccgc     960

ctgctccata gcgattacat gaatatgact ccacgccgcc ctggccccac aaggaaacac    1020

taccagcctt acgcaccacc tagagatttc gctgcctatc ggagcagggt gaagttttcc    1080

agatctgcag atgcaccagc gtatcagcag ggccagaacc aactgtataa cgagctcaac    1140

ctgggacgca gggaagagta tgacgttttg gacaagcgca gaggacggga ccctgagatg 1200

ggtggcaaac caagacgaaa aaacccccag gagggtctct ataatgagct gcagaaggat 1260

aagatggctg aagcctattc tgaaataggc atgaaaggag agcggagaag gggaaaaggg 1320

cacgacggtt tgtaccaggg actcagcact gctacgaagg atacttatga cgctctccac 1380

atgcaagccc tgccacctag g 1401

<210> 32
<211> 467
<212> PRT
<213> Artificial Sequence

<220>
<221> source
<223> /note="Description of Artificial Sequence: Synthetic
      polypeptide"

<400> 32
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Val
                85                  90                  95

Ser Leu Val Tyr Cys Gly Gly Asp Cys Tyr Ser Gly Phe Asp Tyr Trp
            100                 105                 110

Gly Gln Gly Thr Leu Val Thr Val Ser Ser Gly Gly Gly Gly Ser Gly
        115                 120                 125

Gly Gly Gly Ser Gly Gly Gly Gly Ser Asp Ile Gln Leu Thr Gln Ser
    130                 135                 140

Pro Ser Ser Leu Ser Ala Ser Val Gly Asp Arg Val Ser Phe Thr Cys
145                 150                 155                 160

Gln Ala Ser Gln Asp Ile Asn Asn Phe Leu Asn Trp Tyr Gln Gln Lys
                165                 170                 175

Pro Gly Lys Ala Pro Lys Leu Leu Ile Tyr Asp Ala Ser Asn Leu Glu
            180                 185                 190

Thr Gly Val Pro Ser Arg Phe Ser Gly Ser Gly Ser Gly Thr Asp Phe
        195                 200                 205

Thr Phe Thr Ile Ser Ser Leu Gln Pro Glu Asp Ile Ala Thr Tyr Tyr
    210                 215                 220

Cys Gln Gln Tyr Gly Asn Leu Pro Phe Thr Phe Gly Gly Gly Thr Lys
225                 230                 235                 240

Val Glu Ile Lys Arg Ala Ala Ala Ile Glu Val Met Tyr Pro Pro Pro
                245                 250                 255

Tyr Leu Asp Asn Glu Lys Ser Asn Gly Thr Ile Ile His Val Lys Gly
            260                 265                 270

Lys His Leu Cys Pro Ser Pro Leu Phe Pro Gly Pro Ser Lys Pro Phe
        275                 280                 285

Trp Val Leu Val Val Val Gly Gly Val Leu Ala Cys Tyr Ser Leu Leu
    290                 295                 300

Val Thr Val Ala Phe Ile Ile Phe Trp Val Arg Ser Lys Arg Ser Arg
305                 310                 315                 320

Leu Leu His Ser Asp Tyr Met Asn Met Thr Pro Arg Arg Pro Gly Pro
                325                 330                 335

Thr Arg Lys His Tyr Gln Pro Tyr Ala Pro Pro Arg Asp Phe Ala Ala
            340                 345                 350

Tyr Arg Ser Arg Val Lys Phe Ser Arg Ser Ala Asp Ala Pro Ala Tyr
        355                 360                 365

Gln Gln Gly Gln Asn Gln Leu Tyr Asn Glu Leu Asn Leu Gly Arg Arg
    370                 375                 380

Glu Glu Tyr Asp Val Leu Asp Lys Arg Arg Gly Arg Asp Pro Glu Met
385                 390                 395                 400

Gly Gly Lys Pro Arg Arg Lys Asn Pro Gln Glu Gly Leu Tyr Asn Glu
                405                 410                 415

Leu Gln Lys Asp Lys Met Ala Glu Ala Tyr Ser Glu Ile Gly Met Lys
            420                 425                 430

Gly Glu Arg Arg Arg Gly Lys Gly His Asp Gly Leu Tyr Gln Gly Leu
        435                 440                 445

Ser Thr Ala Thr Lys Asp Thr Tyr Asp Ala Leu His Met Gln Ala Leu
    450                 455                 460

Pro Pro Arg
465

<210> 33
<211> 1548
<212> DNA
<213> Artificial Sequence

<220>
<221> source
<223> /note="Description of Artificial Sequence: Synthetic
      polynucleotide"

<400> 33
atggcactcc ccgtaactgc tctgctgctg ccgttggcat tgctcctgca cgccgcacgc      60

ccgcaggtgc aattgcaaga gtccggcccc ggactcgtta aacccagtga gacgcttagc     120

ctgacctgta ccgtctcagg gggcagcatc tcctcttatt actggagctg gatcaggcag     180

cctccaggaa aaggccttga atggattggg tacatctact actctggctc aacaaattat     240

aatccatccc tgaagtcccg cgtgactatc tctgtggaca ccagcaagaa tcagttttca     300

ctgaagttgt ctagtgttac cgcggccgac accgccgtat actactgtgt gtctcttgtg     360

tactgtggcg gcgactgcta ttccgggttc gactactggg gccaagggac tctggtaacc     420

gtgtcctcag gcggcggcgg gtcaggagga ggcggcagtg gaggtggcgg ctccgacatc     480

cagctgacac aatcaccatc ttccctttca gcttcagtcg gggacagagt gtccttcaca     540

tgccaggcca gccaggatat caataacttc ctgaactggt accaacagaa acccggaaag     600

gctccaaagc tcctgatcta tgatgcttcc aacctggaga ccggcgtgcc ctccaggttc     660

agtggttcag gatcaggcac tgactttacg ttcaccatat ccagtcttca gcccgaagac     720

attgcaacct attactgcca acaatacggg aaccttccct ttacattcgg aggcggcacc     780

aaggtggaaa tcaaaagggc tgcagcattg agcaactcaa taatgtattt tagtcacttt     840

gtaccagtgt tcttgccggc taagcctact accacacccg ctccacggcc acctacccca     900

gctcctacca tcgcttcaca gcctctgtcc ctgcgcccag aggcttgccg accggccgca     960

gggggcgctg ttcataccag aggactggat ttcgcctgcg atatctatat ctgggcaccc    1020

ctggccggaa cctgcggcgt actcctgctg tccctggtca tcacgctcta ttgtaatcac    1080

aggaacagat ccaaaagaag ccgcctgctc catagcgatt acatgaatat gactccacgc 1140

cgccctggcc ccacaaggaa acactaccag ccttacgcac cacctagaga tttcgctgcc 1200

tatcggagca gggtgaagtt ttccagatct gcagatgcac cagcgtatca gcagggccag 1260

aaccaactgt ataacgagct caacctggga cgcagggaag agtatgacgt tttggacaag 1320

cgcagaggac gggaccctga gatgggtggc aaaccaagac gaaaaaaccc ccaggagggt 1380

ctctataatg agctgcagaa ggataagatg gctgaagcct attctgaaat aggcatgaaa 1440

ggagagcgga gaaggggaaa agggcacgac ggtttgtacc agggactcag cactgctacg 1500

aaggatactt atgacgctct ccacatgcaa gccctgccac ctaggtaa 1548

<210> 34
<211> 515
<212> PRT
<213> Artificial Sequence

<220>
<221> source
<223> /note="Description of Artificial Sequence: Synthetic
      polypeptide"

<400> 34
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Pro Gly Leu
            20                  25                  30

Val Lys Pro Ser Glu Thr Leu Ser Leu Thr Cys Thr Val Ser Gly Gly
        35                  40                  45

Ser Ile Ser Ser Tyr Tyr Trp Ser Trp Ile Arg Gln Pro Pro Gly Lys
    50                  55                  60

Gly Leu Glu Trp Ile Gly Tyr Ile Tyr Tyr Ser Gly Ser Thr Asn Tyr
65                  70                  75                  80

Asn Pro Ser Leu Lys Ser Arg Val Thr Ile Ser Val Asp Thr Ser Lys
                85                  90                  95

Asn Gln Phe Ser Leu Lys Leu Ser Ser Val Thr Ala Ala Asp Thr Ala
            100                 105                 110

Val Tyr Tyr Cys Val Ser Leu Val Tyr Cys Gly Gly Asp Cys Tyr Ser
        115                 120                 125

Gly Phe Asp Tyr Trp Gly Gln Gly Thr Leu Val Thr Val Ser Ser Gly
    130                 135                 140

Gly Gly Gly Ser Gly Gly Gly Gly Ser Gly Gly Gly Gly Ser Asp Ile
145                 150                 155                 160

Gln Leu Thr Gln Ser Pro Ser Ser Leu Ser Ala Ser Val Gly Asp Arg
                165                 170                 175

Val Ser Phe Thr Cys Gln Ala Ser Gln Asp Ile Asn Asn Phe Leu Asn
            180                 185                 190

Trp Tyr Gln Gln Lys Pro Gly Lys Ala Pro Lys Leu Leu Ile Tyr Asp
        195                 200                 205

Ala Ser Asn Leu Glu Thr Gly Val Pro Ser Arg Phe Ser Gly Ser Gly
    210                 215                 220

Ser Gly Thr Asp Phe Thr Phe Thr Ile Ser Ser Leu Gln Pro Glu Asp
225                 230                 235                 240

Ile Ala Thr Tyr Tyr Cys Gln Gln Tyr Gly Asn Leu Pro Phe Thr Phe
                245                 250                 255

Gly Gly Gly Thr Lys Val Glu Ile Lys Arg Ala Ala Ala Leu Ser Asn
            260                 265                 270

Ser Ile Met Tyr Phe Ser His Phe Val Pro Val Phe Leu Pro Ala Lys
        275                 280                 285

Pro Thr Thr Thr Pro Ala Pro Arg Pro Pro Thr Pro Ala Pro Thr Ile
    290                 295                 300

Ala Ser Gln Pro Leu Ser Leu Arg Pro Glu Ala Cys Arg Pro Ala Ala
305                 310                 315                 320

Gly Gly Ala Val His Thr Arg Gly Leu Asp Phe Ala Cys Asp Ile Tyr
                325                 330                 335

Ile Trp Ala Pro Leu Ala Gly Thr Cys Gly Val Leu Leu Leu Ser Leu
            340                 345                 350

Val Ile Thr Leu Tyr Cys Asn His Arg Asn Arg Ser Lys Arg Ser Arg
        355                 360                 365

Leu Leu His Ser Asp Tyr Met Asn Met Thr Pro Arg Arg Pro Gly Pro
    370                 375                 380

Thr Arg Lys His Tyr Gln Pro Tyr Ala Pro Pro Arg Asp Phe Ala Ala

385                 390                 395                 400

Tyr Arg Ser Arg Val Lys Phe Ser Arg Ser Ala Asp Ala Pro Ala Tyr
                405                 410                 415

Gln Gln Gly Gln Asn Gln Leu Tyr Asn Glu Leu Asn Leu Gly Arg Arg
            420                 425                 430

Glu Glu Tyr Asp Val Leu Asp Lys Arg Arg Gly Arg Asp Pro Glu Met
        435                 440                 445

Gly Gly Lys Pro Arg Arg Lys Asn Pro Gln Glu Gly Leu Tyr Asn Glu
    450                 455                 460

Leu Gln Lys Asp Lys Met Ala Glu Ala Tyr Ser Glu Ile Gly Met Lys
465                 470                 475                 480

Gly Glu Arg Arg Arg Gly Lys Gly His Asp Gly Leu Tyr Gln Gly Leu
                485                 490                 495

Ser Thr Ala Thr Lys Asp Thr Tyr Asp Ala Leu His Met Gln Ala Leu
            500                 505                 510

Pro Pro Arg
        515

<210> 35
<211> 1482
<212> DNA
<213> Artificial Sequence

<220>
<221> source
<223> /note="Description of Artificial Sequence: Synthetic
      polynucleotide"

<400> 35
caggtgcaat tgcaagagtc cggccccgga ctcgttaaac ccagtgagac gcttagcctg      60

acctgtaccg tctcaggggg cagcatctcc tcttattact ggagctggat caggcagcct     120

ccaggaaaag gccttgaatg gattgggtac atctactact ctggctcaac aaattataat     180

ccatccctga agtcccgcgt gactatctct gtggacacca gcaagaatca gttttcactg     240

aagttgtcta gtgttaccgc ggccgacacc gccgtatact actgtgtgtc tcttgtgtac     300

tgtggcggcg actgctattc cgggttcgac tactggggcc aagggactct ggtaaccgtg     360

tcctcaggcg gcggcgggtc aggaggaggc ggcagtggag gtggcggctc cgacatccag     420

ctgacacaat caccatcttc cctttcagct tcagtcgggg acagagtgtc cttcacatgc     480

caggccagcc aggatatcaa taacttcctg aactggtacc aacagaaacc cggaaaggct     540

ccaaagctcc tgatctatga tgcttccaac ctggagaccg gcgtgccctc caggttcagt 600

ggttcaggat caggcactga ctttacgttc accatatcca gtcttcagcc cgaagacatt 660

gcaacctatt actgccaaca atacggaaac cttcccttta cattcggagg cggcaccaag 720

gtggaaatca aaagggctgc agcattgagc aactcaataa tgtattttag tcactttgta 780

ccagtgttct tgccggctaa gcctactacc acacccgctc cacggccacc taccccagct 840

cctaccatcg cttcacagcc tctgtccctg cgcccagagg cttgccgacc ggccgcaggg 900

ggcgctgttc ataccagagg actggatttc gcctgcgata tctatatctg ggcacccctg 960

gccggaacct gcggcgtact cctgctgtcc ctggtcatca cgctctattg taatcacagg 1020

aacagatcca aaagaagccg cctgctccat agcgattaca tgaatatgac tccacgccgc 1080

cctggcccca caaggaaaca ctaccagcct tacgcaccac ctagagattt cgctgcctat 1140

cggagcaggg tgaagttttc cagatctgca gatgcaccag cgtatcagca gggccagaac 1200

caactgtata acgagctcaa cctgggacgc agggaagagt atgacgtttt ggacaagcgc 1260

agaggacggg accctgagat gggtggcaaa ccaagacgaa aaaaccccca ggagggtctc 1320

tataatgagc tgcagaagga taagatggct gaagcctatt ctgaaatagg catgaaagga 1380

gagcggagaa ggggaaaagg gcacgacggt ttgtaccagg gactcagcac tgctacgaag 1440

gatacttatg acgctctcca catgcaagcc ctgccaccta gg 1482

<210> 36
<211> 494
<212> PRT
<213> Artificial Sequence

<220>
<221> source
<223> /note="Description of Artificial Sequence: Synthetic
      polypeptide"

<400> 36
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Val
                85                  90                  95

Ser Leu Val Tyr Cys Gly Gly Asp Cys Tyr Ser Gly Phe Asp Tyr Trp
            100                 105                 110

Gly Gln Gly Thr Leu Val Thr Val Ser Ser Gly Gly Gly Gly Ser Gly
        115                 120                 125

Gly Gly Gly Ser Gly Gly Gly Gly Ser Asp Ile Gln Leu Thr Gln Ser
    130                 135                 140

Pro Ser Ser Leu Ser Ala Ser Val Gly Asp Arg Val Ser Phe Thr Cys
145                 150                 155                 160

Gln Ala Ser Gln Asp Ile Asn Asn Phe Leu Asn Trp Tyr Gln Gln Lys
                165                 170                 175

Pro Gly Lys Ala Pro Lys Leu Leu Ile Tyr Asp Ala Ser Asn Leu Glu
            180                 185                 190

Thr Gly Val Pro Ser Arg Phe Ser Gly Ser Gly Ser Gly Thr Asp Phe
        195                 200                 205

Thr Phe Thr Ile Ser Ser Leu Gln Pro Glu Asp Ile Ala Thr Tyr Tyr
    210                 215                 220

Cys Gln Gln Tyr Gly Asn Leu Pro Phe Thr Phe Gly Gly Gly Thr Lys
225                 230                 235                 240

Val Glu Ile Lys Arg Ala Ala Ala Leu Ser Asn Ser Ile Met Tyr Phe
                245                 250                 255

Ser His Phe Val Pro Val Phe Leu Pro Ala Lys Pro Thr Thr Thr Pro
            260                 265                 270

Ala Pro Arg Pro Pro Thr Pro Ala Pro Thr Ile Ala Ser Gln Pro Leu
        275                 280                 285

Ser Leu Arg Pro Glu Ala Cys Arg Pro Ala Ala Gly Gly Ala Val His
    290                 295                 300

Thr Arg Gly Leu Asp Phe Ala Cys Asp Ile Tyr Ile Trp Ala Pro Leu
305                 310                 315                 320

Ala Gly Thr Cys Gly Val Leu Leu Leu Ser Leu Val Ile Thr Leu Tyr

325                 330                 335

Cys Asn His Arg Asn Arg Ser Lys Arg Ser Arg Leu Leu His Ser Asp
            340                 345                 350

Tyr Met Asn Met Thr Pro Arg Arg Pro Gly Pro Thr Arg Lys His Tyr
        355                 360                 365

Gln Pro Tyr Ala Pro Pro Arg Asp Phe Ala Ala Tyr Arg Ser Arg Val
    370                 375                 380

Lys Phe Ser Arg Ser Ala Asp Ala Pro Ala Tyr Gln Gln Gly Gln Asn
385                 390                 395                 400

Gln Leu Tyr Asn Glu Leu Asn Leu Gly Arg Arg Glu Glu Tyr Asp Val
                405                 410                 415

Leu Asp Lys Arg Arg Gly Arg Asp Pro Glu Met Gly Gly Lys Pro Arg
            420                 425                 430

Arg Lys Asn Pro Gln Glu Gly Leu Tyr Asn Glu Leu Gln Lys Asp Lys
        435                 440                 445

Met Ala Glu Ala Tyr Ser Glu Ile Gly Met Lys Gly Glu Arg Arg Arg
    450                 455                 460

Gly Lys Gly His Asp Gly Leu Tyr Gln Gly Leu Ser Thr Ala Thr Lys
465                 470                 475                 480

Asp Thr Tyr Asp Ala Leu His Met Gln Ala Leu Pro Pro Arg
                485                 490

<210> 37
<211> 1440
<212> DNA
<213> Artificial Sequence

<220>
<221> source
<223> /note="Description of Artificial Sequence: Synthetic
      polynucleotide"

<400> 37
atggcactcc ccgtaactgc tctgctgctg ccgttggcat tgctcctgca cgccgcacgc      60

ccggatatcc agctcacgca atccccctca agcttgagtg cctccgtggg cgaccgggtg      120

tccttcacat gtcaggcaag ccaagacata aataatttcc tgaattggta ccaacaaaaa      180

cccggcaagg ctcccaaact cctgatttat gatgcctcca atctggagac cggggtccct      240

tctagattca gcggaagtgg cagcggcaca gactttacat ttactatctc ttctctgcaa      300

ccagaggaca tcgccacata ctattgccag caatacggca atctgccctt caccttcgga 360

ggcggaacca aggtagaaat taaaaggggc ggtggaggct ccggaggggg gggctctggc 420

ggagggggct cccaagtaca attgcaggag tcagggcctg gactcgtgaa gccttcagaa 480

actttgtcac tgacatgtac agtgtccggc ggaagcattt ccagttacta ttggtcctgg 540

attagacagc cacccggcaa aggactggaa tggattggat atatctacta ctctggatct 600

acaaactata atcccagcct caaatccagg gtcactatta gtgtggatac atcaaagaat 660

cagttctcct tgaagctgag ctcagtcact gctgccgaca ccgcagtgta ctattgtgtg 720

agcctggtct actgcggcgg agattgctac agcggtttcg attactgggg ccagggcacc 780

ctggttaccg ttagttccgc ggctgctctt gataacgaga agtccaacgg tacgattatc 840

cacgttaagg gtaagcacct ttgccctagc ccgctgttcc caggccccag taagcccttt 900

tgggtcctcg ttgtggtagg tggggtactc gcctgctact ccctgctcgt cactgtcgca 960

ttcatcatct tctgggtcag atccaaaaga agccgcctgc tccatagcga ttacatgaat 1020

atgactccac gccgccctgg ccccacaagg aaacactacc agccttacgc accacctaga 1080

gatttcgctg cctatcggag cagggtgaag ttttccagat ctgcagatgc accagcgtat 1140

cagcagggcc agaaccaact gtataacgag ctcaacctgg gacgcaggga agagtatgac 1200

gttttggaca agcgcagagg acgggaccct gagatgggtg gcaaaccaag acgaaaaaac 1260

ccccaggagg gtctctataa tgagctgcag aaggataaga tggctgaagc ctattctgaa 1320

ataggcatga aaggagagcg gagaagggga aaagggcacg acggtttgta ccagggactc 1380

agcactgcta cgaaggatac ttatgacgct ctccacatgc aagccctgcc acctaggtaa 1440

<210> 38
<211> 470
<212> PRT
<213> Artificial Sequence

<220>
<221> source
<223> /note="Description of Artificial Sequence: Synthetic
      polypeptide"

<400> 38
Met Ala Leu Pro Val Thr Ala Leu Leu Leu Pro Leu Ala Leu Leu Leu
1               5                   10                  15

His Ala Ala Arg Pro Asp Ile Gln Leu Thr Gln Ser Pro Ser Ser Leu
            20                  25                  30

Ser Ala Ser Val Gly Asp Arg Val Ser Phe Thr Cys Gln Ala Ser Gln
        35                  40                  45

Asp Ile Asn Asn Phe Leu Asn Trp Tyr Gln Gln Lys Pro Gly Lys Ala
    50                  55                  60

Pro Lys Leu Leu Ile Tyr Asp Ala Ser Asn Leu Glu Thr Gly Val Pro
65                  70                  75                  80

Ser Arg Phe Ser Gly Ser Gly Ser Gly Thr Asp Phe Thr Phe Thr Ile
                85                  90                  95

Ser Ser Leu Gln Pro Glu Asp Ile Ala Thr Tyr Tyr Cys Gln Gln Tyr
            100                 105                 110

Gly Asn Leu Pro Phe Thr Phe Gly Gly Gly Thr Lys Val Glu Ile Lys
        115                 120                 125

Arg Gly Gly Gly Gly Ser Gly Gly Gly Gly Ser Gly Gly Gly Gly Ser
    130                 135                 140

Gln Val Gln Leu Gln Glu Ser Gly Pro Gly Leu Val Lys Pro Ser Glu
145                 150                 155                 160

Thr Leu Ser Leu Thr Cys Thr Val Ser Gly Gly Ser Ile Ser Ser Tyr
                165                 170                 175

Tyr Trp Ser Trp Ile Arg Gln Pro Pro Gly Lys Gly Leu Glu Trp Ile
            180                 185                 190

Gly Tyr Ile Tyr Tyr Ser Gly Ser Thr Asn Tyr Asn Pro Ser Leu Lys
        195                 200                 205

Ser Arg Val Thr Ile Ser Val Asp Thr Ser Lys Asn Gln Phe Ser Leu
    210                 215                 220

Lys Leu Ser Ser Val Thr Ala Ala Asp Thr Ala Val Tyr Tyr Cys Val
225                 230                 235                 240

Ser Leu Val Tyr Cys Gly Gly Asp Cys Tyr Ser Gly Phe Asp Tyr Trp
                245                 250                 255

Gly Gln Gly Thr Leu Val Thr Val Ser Ser Ala Ala Ala Leu Asp Asn
            260                 265                 270

Glu Lys Ser Asn Gly Thr Ile Ile His Val Lys Gly Lys His Leu Cys
        275                 280                 285

Pro Ser Pro Leu Phe Pro Gly Pro Ser Lys Pro Phe Trp Val Leu Val
    290                 295                 300

Val Val Gly Gly Val Leu Ala Cys Tyr Ser Leu Leu Val Thr Val Ala
305                 310                 315                 320

Phe Ile Ile Phe Trp Val Arg Ser Lys Arg Ser Arg Leu Leu His Ser
                325                 330                 335

Asp Tyr Met Asn Met Thr Pro Arg Arg Pro Gly Pro Thr Arg Lys His
            340                 345                 350

Tyr Gln Pro Tyr Ala Pro Pro Arg Asp Phe Ala Ala Tyr Arg Ser Arg
        355                 360                 365

Val Lys Phe Ser Arg Ser Ala Asp Ala Pro Ala Tyr Gln Gln Gly Gln
    370                 375                 380

Asn Gln Leu Tyr Asn Glu Leu Asn Leu Gly Arg Arg Glu Glu Tyr Asp
385                 390                 395                 400

Val Leu Asp Lys Arg Arg Gly Arg Asp Pro Glu Met Gly Gly Lys Pro
                405                 410                 415

Arg Arg Lys Asn Pro Gln Glu Gly Leu Tyr Asn Glu Leu Gln Lys Asp
            420                 425                 430

Lys Met Ala Glu Ala Tyr Ser Glu Ile Gly Met Lys Gly Glu Arg Arg
        435                 440                 445

Arg Gly Lys Gly His Asp Gly Leu Tyr Gln Gly Leu Ser Thr Ala Thr
    450                 455                 460

Lys Asp Thr Tyr Asp Ala
465                 470

<210> 39
<211> 1374
<212> DNA
<213> Artificial Sequence

<220>
<221> source
<223> /note="Description of Artificial Sequence: Synthetic
      polynucleotide"

<400> 39
gatatccagc tcacgcaatc cccctcaagc ttgagtgcct ccgtgggcga ccgggtgtcc      60

ttcacatgtc aggcaagcca agacataaat aatttcctga attggtacca acaaaaaccc     120

ggcaaggctc ccaaactcct gatttatgat gcctccaatc tggagaccgg ggtcccttct     180

agattcagcg gaagtggcag cggcacagac tttacattta ctatctcttc tctgcaacca     240

gaggacatcg ccacatacta ttgccagcaa tacggcaatc tgcccttcac cttcggaggc 300

ggaaccaagg tagaaattaa aaggggcggt ggaggctccg gagggggggg ctctggcgga 360

gggggctccc aagtacaatt gcaggagtca gggcctggac tcgtgaagcc ttcagaaact 420

ttgtcactga catgtacagt gtccggcgga agcatttcca gttactattg gtcctggatt 480

agacagccac ccggcaaagg actggaatgg attggatata tctactactc tggatctaca 540

aactataatc ccagcctcaa atccagggtc actattagtg tggatacatc aaagaatcag 600

ttctccttga agctgagctc agtcactgct gccgacaccg cagtgtacta ttgtgtgagc 660

ctggtctact gcggcggaga ttgctacagc ggtttcgatt actggggcca gggcaccctg 720

gttaccgtta gttccgcggc tgctcttgat aacgagaagt ccaacggtac gattatccac 780

gttaagggta agcacctttg ccctagcccg ctgttcccag gccccagtaa gcccttttgg 840

gtcctcgttg tggtaggtgg ggtactcgcc tgctactccc tgctcgtcac tgtcgcattc 900

atcatcttct gggtcagatc caaaagaagc cgcctgctcc atagcgatta catgaatatg 960

actccacgcc gccctggccc cacaaggaaa cactaccagc cttacgcacc acctagagat 1020

ttcgctgcct atcggagcag ggtgaagttt tccagatctg cagatgcacc agcgtatcag 1080

cagggccaga accaactgta taacgagctc aacctgggac gcagggaaga gtatgacgtt 1140

ttggacaagc gcagaggacg ggaccctgag atgggtggca aaccaagacg aaaaaacccc 1200

caggagggtc tctataatga gctgcagaag gataagatgg ctgaagccta ttctgaaata 1260

ggcatgaaag gagagcggag aaggggaaaa gggcacgacg gtttgtacca gggactcagc 1320

actgctacga aggatactta tgacgctctc cacatgcaag ccctgccacc tagg 1374

<210> 40
<211> 458
<212> PRT
<213> Artificial Sequence

<220>
<221> source
<223> /note="Description of Artificial Sequence: Synthetic
      polypeptide"

<400> 40
Asp Ile Gln Leu Thr Gln Ser Pro Ser Ser Leu Ser Ala Ser Val Gly
1               5                   10                  15

Asp Arg Val Ser Phe Thr Cys Gln Ala Ser Gln Asp Ile Asn Asn Phe
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Gly Asn Leu Pro Phe
                85                  90                  95

Thr Phe Gly Gly Gly Thr Lys Val Glu Ile Lys Arg Gly Gly Gly Gly
            100                 105                 110

Ser Gly Gly Gly Gly Ser Gly Gly Gly Gly Ser Gln Val Gln Leu Gln
        115                 120                 125

Glu Ser Gly Pro Gly Leu Val Lys Pro Ser Glu Thr Leu Ser Leu Thr
    130                 135                 140

Cys Thr Val Ser Gly Gly Ser Ile Ser Ser Tyr Tyr Trp Ser Trp Ile
145                 150                 155                 160

Arg Gln Pro Pro Gly Lys Gly Leu Glu Trp Ile Gly Tyr Ile Tyr Tyr
                165                 170                 175

Ser Gly Ser Thr Asn Tyr Asn Pro Ser Leu Lys Ser Arg Val Thr Ile
            180                 185                 190

Ser Val Asp Thr Ser Lys Asn Gln Phe Ser Leu Lys Leu Ser Ser Val
        195                 200                 205

Thr Ala Ala Asp Thr Ala Val Tyr Tyr Cys Val Ser Leu Val Tyr Cys
    210                 215                 220

Gly Gly Asp Cys Tyr Ser Gly Phe Asp Tyr Trp Gly Gln Gly Thr Leu
225                 230                 235                 240

Val Thr Val Ser Ser Ala Ala Ala Leu Asp Asn Glu Lys Ser Asn Gly
                245                 250                 255

Thr Ile Ile His Val Lys Gly Lys His Leu Cys Pro Ser Pro Leu Phe
            260                 265                 270

Pro Gly Pro Ser Lys Pro Phe Trp Val Leu Val Val Val Gly Gly Val
        275                 280                 285

Leu Ala Cys Tyr Ser Leu Leu Val Thr Val Ala Phe Ile Ile Phe Trp
    290                 295                 300

Val Arg Ser Lys Arg Ser Arg Leu Leu His Ser Asp Tyr Met Asn Met
305                 310                 315                 320

Thr Pro Arg Arg Pro Gly Pro Thr Arg Lys His Tyr Gln Pro Tyr Ala
                325                 330                 335

Pro Pro Arg Asp Phe Ala Ala Tyr Arg Ser Arg Val Lys Phe Ser Arg
            340                 345                 350

Ser Ala Asp Ala Pro Ala Tyr Gln Gln Gly Gln Asn Gln Leu Tyr Asn
        355                 360                 365

Glu Leu Asn Leu Gly Arg Arg Glu Glu Tyr Asp Val Leu Asp Lys Arg
    370                 375                 380

Arg Gly Arg Asp Pro Glu Met Gly Gly Lys Pro Arg Arg Lys Asn Pro
385                 390                 395                 400

Gln Glu Gly Leu Tyr Asn Glu Leu Gln Lys Asp Lys Met Ala Glu Ala
                405                 410                 415

Tyr Ser Glu Ile Gly Met Lys Gly Glu Arg Arg Arg Gly Lys Gly His
            420                 425                 430

Asp Gly Leu Tyr Gln Gly Leu Ser Thr Ala Thr Lys Asp Thr Tyr Asp
        435                 440                 445

Ala Leu His Met Gln Ala Leu Pro Pro Arg
    450                 455

<210> 41
<211> 1467
<212> DNA
<213> Artificial Sequence

<220>
<221> source
<223> /note="Description of Artificial Sequence: Synthetic
      polynucleotide"

<400> 41
atggcactcc ccgtaactgc tctgctgctg ccgttggcat tgctcctgca cgccgcacgc      60

ccggatatcc agctgaccca gtctccatcc tctttgagtg cctccgtggg tgaccgcgtc     120

tctttcactt gccaagccag ccaagacatc aacaactttc tgaattggta ccagcagaaa     180

ccaggcaaag caccaaagct cctcatctac gacgcctcca acctggaaac cggggtgccc     240

agcaggttta gcgggagcgg ttctggcacg gattttacgt tcaccatctc ctctctgcag     300

cccgaggata tagctactta ttactgtcag cagtacggga atctgccatt tacttttggg     360

ggtggaacta aggtggaaat caaaaggggc ggcgggggaa gcgggggcgg gggctcaggt 420

ggcggaggga gccaggtgca actccaggaa agtggcccag gattggtgaa gcccagcgag 480

accctttccc ttacttgtac tgttagcgga ggcagcataa gcagctacta ttggtcctgg 540

atcagacagc caccagggaa agggcttgaa tggattggct acatttacta ttccgggtcc 600

accaactaca acccatccct caagtcccgc gtgacaattt ccgtcgacac aagcaagaac 660

cagttctccc tgaaacttag tagcgtcact gctgcagata cagcagtgta ctattgtgtc 720

agccttgtct actgtggcgg cgactgctac agtggctttg attactgggg acagggcacg 780

ctcgtgacag tgtccagcgc tgcggctatc gaggtaatgt atccgccacc gtatctggac 840

aacgagaagt ctaatgggac aatcattcac gtgaagggga agcacctgtg tccatccccc 900

ctgtttccgg gtcccagtaa acccttctgg gtgcttgttg tcgttggcgg ggtgctggcc 960

tgctattccc tgctggtgac cgtcgcgttt attattttct gggttagatc caaaagaagc 1020

cgcctgctcc atagcgatta catgaatatg actccacgcc gccctggccc cacaaggaaa 1080

cactaccagc cttacgcacc acctagagat ttcgctgcct atcggagcag ggtgaagttt 1140

tccagatctg cagatgcacc agcgtatcag cagggccaga accaactgta taacgagctc 1200

aacctgggac gcagggaaga gtatgacgtt ttggacaagc gcagaggacg ggaccctgag 1260

atgggtggca aaccaagacg aaaaaacccc caggagggtc tctataatga gctgcagaag 1320

gataagatgg ctgaagccta ttctgaaata ggcatgaaag gagagcggag aaggggaaaa 1380

gggcacgacg gtttgtacca gggactcagc actgctacga aggatactta tgacgctctc 1440

cacatgcaag ccctgccacc taggtaa 1467

<210> 42
<211> 488
<212> PRT
<213> Artificial Sequence

<220>
<221> source
<223> /note="Description of Artificial Sequence: Synthetic
      polypeptide"

<400> 42
Met Ala Leu Pro Val Thr Ala Leu Leu Leu Pro Leu Ala Leu Leu Leu
1               5                   10                  15

His Ala Ala Arg Pro Asp Ile Gln Leu Thr Gln Ser Pro Ser Ser Leu
            20                  25                  30

Ser Ala Ser Val Gly Asp Arg Val Ser Phe Thr Cys Gln Ala Ser Gln
        35                  40                  45

Asp Ile Asn Asn Phe Leu Asn Trp Tyr Gln Gln Lys Pro Gly Lys Ala
    50                  55                  60

Pro Lys Leu Leu Ile Tyr Asp Ala Ser Asn Leu Glu Thr Gly Val Pro
65                  70                  75                  80

Ser Arg Phe Ser Gly Ser Gly Ser Gly Thr Asp Phe Thr Phe Thr Ile
                85                  90                  95

Ser Ser Leu Gln Pro Glu Asp Ile Ala Thr Tyr Tyr Cys Gln Gln Tyr
            100                 105                 110

Gly Asn Leu Pro Phe Thr Phe Gly Gly Gly Thr Lys Val Glu Ile Lys
        115                 120                 125

Arg Gly Gly Gly Gly Ser Gly Gly Gly Gly Ser Gly Gly Gly Gly Ser
    130                 135                 140

Gln Val Gln Leu Gln Glu Ser Gly Pro Gly Leu Val Lys Pro Ser Glu
145                 150                 155                 160

Thr Leu Ser Leu Thr Cys Thr Val Ser Gly Gly Ser Ile Ser Ser Tyr
                165                 170                 175

Tyr Trp Ser Trp Ile Arg Gln Pro Pro Gly Lys Gly Leu Glu Trp Ile
            180                 185                 190

Gly Tyr Ile Tyr Tyr Ser Gly Ser Thr Asn Tyr Asn Pro Ser Leu Lys
        195                 200                 205

Ser Arg Val Thr Ile Ser Val Asp Thr Ser Lys Asn Gln Phe Ser Leu
    210                 215                 220

Lys Leu Ser Ser Val Thr Ala Ala Asp Thr Ala Val Tyr Tyr Cys Val
225                 230                 235                 240

Ser Leu Val Tyr Cys Gly Gly Asp Cys Tyr Ser Gly Phe Asp Tyr Trp
                245                 250                 255

Gly Gln Gly Thr Leu Val Thr Val Ser Ser Ala Ala Ala Ile Glu Val
            260                 265                 270

Met Tyr Pro Pro Pro Tyr Leu Asp Asn Glu Lys Ser Asn Gly Thr Ile
        275                 280                 285

Ile His Val Lys Gly Lys His Leu Cys Pro Ser Pro Leu Phe Pro Gly
    290                 295                 300

Pro Ser Lys Pro Phe Trp Val Leu Val Val Val Gly Gly Val Leu Ala
305                 310                 315                 320

Cys Tyr Ser Leu Leu Val Thr Val Ala Phe Ile Ile Phe Trp Val Arg
                325                 330                 335

Ser Lys Arg Ser Arg Leu Leu His Ser Asp Tyr Met Asn Met Thr Pro
            340                 345                 350

Arg Arg Pro Gly Pro Thr Arg Lys His Tyr Gln Pro Tyr Ala Pro Pro
        355                 360                 365

Arg Asp Phe Ala Ala Tyr Arg Ser Arg Val Lys Phe Ser Arg Ser Ala
    370                 375                 380

Asp Ala Pro Ala Tyr Gln Gln Gly Gln Asn Gln Leu Tyr Asn Glu Leu
385                 390                 395                 400

Asn Leu Gly Arg Arg Glu Glu Tyr Asp Val Leu Asp Lys Arg Arg Gly
                405                 410                 415

Arg Asp Pro Glu Met Gly Gly Lys Pro Arg Arg Lys Asn Pro Gln Glu
            420                 425                 430

Gly Leu Tyr Asn Glu Leu Gln Lys Asp Lys Met Ala Glu Ala Tyr Ser
        435                 440                 445

Glu Ile Gly Met Lys Gly Glu Arg Arg Arg Gly Lys Gly His Asp Gly
    450                 455                 460

Leu Tyr Gln Gly Leu Ser Thr Ala Thr Lys Asp Thr Tyr Asp Ala Leu
465                 470                 475                 480

His Met Gln Ala Leu Pro Pro Arg
                485

<210> 43
<211> 1401
<212> DNA
<213> Artificial Sequence

<220>
<221> source
<223> /note="Description of Artificial Sequence: Synthetic
      polynucleotide"

<400> 43
gatatccagc tgacccagtc tccatcctct ttgagtgcct ccgtgggtga ccgcgtctct      60

ttcacttgcc aagccagcca agacatcaac aactttctga attggtacca gcagaaacca     120

ggcaaagcac caaagctcct catctacgac gcctccaacc tggaaaccgg ggtgcccagc 180

aggtttagcg ggagcggttc tggcacggat tttacgttca ccatctcctc tctgcagccc 240

gaggatatag ctacttatta ctgtcagcag tacgggaatc tgccatttac ttttgggggt 300

ggaactaagg tggaaatcaa aaggggcggc gggggaagcg gggcgggggg ctcaggtggc 360

ggagggagcc aggtgcaact ccaggaaagt ggcccaggat tggtgaagcc cagcgagacc 420

ctttccctta cttgtactgt tagcggaggc agcataagca gctactattg gtcctggatc 480

agacagccac cagggaaagg gcttgaatgg attggctaca tttactattc cgggtccacc 540

aactacaacc catccctcaa gtcccgcgtg acaatttccg tcgacacaag caagaaccag 600

ttctccctga aacttagtag cgtcactgct gcagatacag cagtgtacta ttgtgtcagc 660

cttgtctact gtggcggcga ctgctacagt ggctttgatt actggggaca gggcacgctc 720

gtgacagtgt ccagcgctgc ggctatcgag gtaatgtatc cgccaccgta tctggacaac 780

gagaagtcta atgggacaat cattcacgtg aaggggaagc acctgtgtcc atcccccctg 840

tttccgggtc ccagtaaacc cttctgggtg cttgttgtcg ttggcggggt gctggcctgc 900

tattccctgc tggtgaccgt cgcgtttatt attttctggg ttagatccaa aagaagccgc 960

ctgctccata gcgattacat gaatatgact ccacgccgcc ctggccccac aaggaaacac 1020

taccagcctt acgcaccacc tagagatttc gctgcctatc ggagcagggt gaagttttcc 1080

agatctgcag atgcaccagc gtatcagcag ggccagaacc aactgtataa cgagctcaac 1140

ctgggacgca gggaagagta tgacgttttg gacaagcgca gaggacggga ccctgagatg 1200

ggtggcaaac caagacgaaa aaacccccag gagggtctct ataatgagct gcagaaggat 1260

aagatggctg aagcctattc tgaaataggc atgaaaggag agcggagaag gggaaaaggg 1320

cacgacggtt tgtaccaggg actcagcact gctacgaagg atacttatga cgctctccac 1380

atgcaagccc tgccacctag g 1401

<210> 44
<211> 467
<212> PRT
<213> Artificial Sequence

<220>
<221> source
<223> /note="Description of Artificial Sequence: Synthetic
      polypeptide"

<400> 44
Asp Ile Gln Leu Thr Gln Ser Pro Ser Ser Leu Ser Ala Ser Val Gly
1               5                   10                  15

Asp Arg Val Ser Phe Thr Cys Gln Ala Ser Gln Asp Ile Asn Asn Phe
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Gly Asn Leu Pro Phe
                85                  90                  95

Thr Phe Gly Gly Gly Thr Lys Val Glu Ile Lys Arg Gly Gly Gly Gly
            100                 105                 110

Ser Gly Gly Gly Gly Ser Gly Gly Gly Gly Ser Gln Val Gln Leu Gln
        115                 120                 125

Glu Ser Gly Pro Gly Leu Val Lys Pro Ser Glu Thr Leu Ser Leu Thr
    130                 135                 140

Cys Thr Val Ser Gly Gly Ser Ile Ser Ser Tyr Tyr Trp Ser Trp Ile
145                 150                 155                 160

Arg Gln Pro Pro Gly Lys Gly Leu Glu Trp Ile Gly Tyr Ile Tyr Tyr
                165                 170                 175

Ser Gly Ser Thr Asn Tyr Asn Pro Ser Leu Lys Ser Arg Val Thr Ile
            180                 185                 190

Ser Val Asp Thr Ser Lys Asn Gln Phe Ser Leu Lys Leu Ser Ser Val
        195                 200                 205

Thr Ala Ala Asp Thr Ala Val Tyr Tyr Cys Val Ser Leu Val Tyr Cys
    210                 215                 220

Gly Gly Asp Cys Tyr Ser Gly Phe Asp Tyr Trp Gly Gln Gly Thr Leu
225                 230                 235                 240

Val Thr Val Ser Ser Ala Ala Ala Ile Glu Val Met Tyr Pro Pro Pro
                245                 250                 255

Tyr Leu Asp Asn Glu Lys Ser Asn Gly Thr Ile Ile His Val Lys Gly
            260                 265                 270

Lys His Leu Cys Pro Ser Pro Leu Phe Pro Gly Pro Ser Lys Pro Phe

275                 280                 285

Trp Val Leu Val Val Val Gly Gly Val Leu Ala Cys Tyr Ser Leu Leu
    290                 295                 300

Val Thr Val Ala Phe Ile Ile Phe Trp Val Arg Ser Lys Arg Ser Arg
305                 310                 315                 320

Leu Leu His Ser Asp Tyr Met Asn Met Thr Pro Arg Arg Pro Gly Pro
                325                 330                 335

Thr Arg Lys His Tyr Gln Pro Tyr Ala Pro Pro Arg Asp Phe Ala Ala
            340                 345                 350

Tyr Arg Ser Arg Val Lys Phe Ser Arg Ser Ala Asp Ala Pro Ala Tyr
        355                 360                 365

Gln Gln Gly Gln Asn Gln Leu Tyr Asn Glu Leu Asn Leu Gly Arg Arg
    370                 375                 380

Glu Glu Tyr Asp Val Leu Asp Lys Arg Arg Gly Arg Asp Pro Glu Met
385                 390                 395                 400

Gly Gly Lys Pro Arg Arg Lys Asn Pro Gln Glu Gly Leu Tyr Asn Glu
                405                 410                 415

Leu Gln Lys Asp Lys Met Ala Glu Ala Tyr Ser Glu Ile Gly Met Lys
            420                 425                 430

Gly Glu Arg Arg Arg Gly Lys Gly His Asp Gly Leu Tyr Gln Gly Leu
        435                 440                 445

Ser Thr Ala Thr Lys Asp Thr Tyr Asp Ala Leu His Met Gln Ala Leu
    450                 455                 460

Pro Pro Arg
465

<210> 45
<211> 1548
<212> DNA
<213> Artificial Sequence

<220>
<221> source
<223> /note="Description of Artificial Sequence: Synthetic
      polynucleotide"

<400> 45
atggcactcc ccgtaactgc tctgctgctg ccgttggcat tgctcctgca cgccgcacgc        60

ccggacattc aattgaccca gtcccctagc agtctctcag caagtgtggg agatagggtg 120

tcattcacct gtcaggcttc acaggacatc aacaacttcc tcaattggta tcagcagaag 180

ccagggaagg caccaaagct gctcatatat gacgcttcaa accttgaaac cggagtacct 240

agccgcttca gcggaagcgg atcagggact gacttcactt ttaccatctc ttcactgcag 300

cccgaagaca tcgccacata ctactgccag cagtacggaa acttgccttt tacatttggg 360

ggcggcacca aagtggagat taagcgaggg ggaggcggct caggaggcgg tggctccgga 420

ggcgggggtt cccaggtcca gctccaggaa tccggcccag gtctggttaa gcccagtgaa 480

actttgtccc tcacgtgtac tgtgagcggt ggttcaatct cctcatacta ttggtcttgg 540

atacggcaac ctcctggaaa gggcctcgag tggatcggct atatctacta tagtggctcc 600

actaattaca acccttccct caagtccaga gtcaccattt ccgtggacac atctaagaac 660

cagttcagtc tgaagttgtc cagcgttaca gccgcagaca cagccgttta ttactgtgtg 720

tctcttgttt actgcggggg agactgttat agcggcttcg attactgggg ccagggcacc 780

ttggtcacag tctcttccgc ggccgccctc tctaacagta ttatgtactt ttctcatttt 840

gtacccgtgt tccttcccgc taagccaact actaccccgg ccccacggcc gcctacccct 900

gcacccacaa tagccagtca gcctttgagc ctgagacctg aggcttgtcg gccggctgct 960

gggggtgcag tgcacacacg aggtcttgat tttgcttgcg acatatacat ctgggcccct 1020

ctggccggga cctgtggggt gctgcttctg agcttggtca tcacgctcta ttgcaaccat 1080

cgcaacagat ccaaaagaag ccgcctgctc catagcgatt acatgaatat gactccacgc 1140

cgccctggcc ccacaaggaa acactaccag ccttacgcac cacctagaga tttcgctgcc 1200

tatcggagca gggtgaagtt ttccagatct gcagatgcac cagcgtatca gcagggccag 1260

aaccaactgt ataacgagct caacctggga cgcagggaag agtatgacgt tttggacaag 1320

cgcagaggac gggaccctga gatgggtggc aaaccaagac gaaaaaaccc ccaggagggt 1380

ctctataatg agctgcagaa ggataagatg gctgaagcct attctgaaat aggcatgaaa 1440

ggagagcgga gaaggggaaa agggcacgac ggtttgtacc agggactcag cactgctacg 1500

aaggatactt atgacgctct ccacatgcaa gccctgccac ctaggtaa 1548

<210> 46
<211> 515
<212> PRT
<213> Artificial Sequence

<220>
<221> source
<223> /note="Description of Artificial Sequence: Synthetic
      polypeptide"

<400> 46

Met Ala Leu Pro Val Thr Ala Leu Leu Leu Pro Leu Ala Leu Leu Leu
1               5                   10                  15

His Ala Ala Arg Pro Asp Ile Gln Leu Thr Gln Ser Pro Ser Ser Leu
            20                  25                  30

Ser Ala Ser Val Gly Asp Arg Val Ser Phe Thr Cys Gln Ala Ser Gln
        35                  40                  45

Asp Ile Asn Asn Phe Leu Asn Trp Tyr Gln Gln Lys Pro Gly Lys Ala
    50                  55                  60

Pro Lys Leu Leu Ile Tyr Asp Ala Ser Asn Leu Glu Thr Gly Val Pro
65                  70                  75                  80

Ser Arg Phe Ser Gly Ser Gly Ser Gly Thr Asp Phe Thr Phe Thr Ile
                85                  90                  95

Ser Ser Leu Gln Pro Glu Asp Ile Ala Thr Tyr Tyr Cys Gln Gln Tyr
            100                 105                 110

Gly Asn Leu Pro Phe Thr Phe Gly Gly Gly Thr Lys Val Glu Ile Lys
        115                 120                 125

Arg Gly Gly Gly Gly Ser Gly Gly Gly Gly Ser Gly Gly Gly Gly Ser
    130                 135                 140

Gln Val Gln Leu Gln Glu Ser Gly Pro Gly Leu Val Lys Pro Ser Glu
145                 150                 155                 160

Thr Leu Ser Leu Thr Cys Thr Val Ser Gly Gly Ser Ile Ser Ser Tyr
                165                 170                 175

Tyr Trp Ser Trp Ile Arg Gln Pro Pro Gly Lys Gly Leu Glu Trp Ile
            180                 185                 190

Gly Tyr Ile Tyr Tyr Ser Gly Ser Thr Asn Tyr Asn Pro Ser Leu Lys
        195                 200                 205

Ser Arg Val Thr Ile Ser Val Asp Thr Ser Lys Asn Gln Phe Ser Leu
    210                 215                 220

Lys Leu Ser Ser Val Thr Ala Ala Asp Thr Ala Val Tyr Tyr Cys Val
225                 230                 235                 240

Ser Leu Val Tyr Cys Gly Gly Asp Cys Tyr Ser Gly Phe Asp Tyr Trp
                245                 250                 255

Gly Gln Gly Thr Leu Val Thr Val Ser Ser Ala Ala Ala Leu Ser Asn
        260                 265                 270

Ser Ile Met Tyr Phe Ser His Phe Val Pro Val Phe Leu Pro Ala Lys
    275                 280                 285

Pro Thr Thr Thr Pro Ala Pro Arg Pro Pro Thr Pro Ala Pro Thr Ile
    290                 295                 300

Ala Ser Gln Pro Leu Ser Leu Arg Pro Glu Ala Cys Arg Pro Ala Ala
305                 310                 315                 320

Gly Gly Ala Val His Thr Arg Gly Leu Asp Phe Ala Cys Asp Ile Tyr
                325                 330                 335

Ile Trp Ala Pro Leu Ala Gly Thr Cys Gly Val Leu Leu Leu Ser Leu
            340                 345                 350

Val Ile Thr Leu Tyr Cys Asn His Arg Asn Arg Ser Lys Arg Ser Arg
        355                 360                 365

Leu Leu His Ser Asp Tyr Met Asn Met Thr Pro Arg Arg Pro Gly Pro
    370                 375                 380

Thr Arg Lys His Tyr Gln Pro Tyr Ala Pro Pro Arg Asp Phe Ala Ala
385                 390                 395                 400

Tyr Arg Ser Arg Val Lys Phe Ser Arg Ser Ala Asp Ala Pro Ala Tyr
                405                 410                 415

Gln Gln Gly Gln Asn Gln Leu Tyr Asn Glu Leu Asn Leu Gly Arg Arg
            420                 425                 430

Glu Glu Tyr Asp Val Leu Asp Lys Arg Arg Gly Arg Asp Pro Glu Met
        435                 440                 445

Gly Gly Lys Pro Arg Arg Lys Asn Pro Gln Glu Gly Leu Tyr Asn Glu
    450                 455                 460

Leu Gln Lys Asp Lys Met Ala Glu Ala Tyr Ser Glu Ile Gly Met Lys
465                 470                 475                 480

Gly Glu Arg Arg Arg Gly Lys Gly His Asp Gly Leu Tyr Gln Gly Leu
                485                 490                 495

Ser Thr Ala Thr Lys Asp Thr Tyr Asp Ala Leu His Met Gln Ala Leu
            500                 505                 510

Pro Pro Arg
        515

<210> 47
<211> 1482
<212> DNA
<213> Artificial Sequence

<220>
<221> source
<223> /note="Description of Artificial Sequence: Synthetic
      polynucleotide"

<400> 47
gacattcaat tgacccagtc ccctagcagt ctctcagcaa gtgtgggaga tagggtgtca      60

ttcacctgtc aggcttcaca ggacatcaac aacttcctca attggtatca gcagaagcca     120

gggaaggcac caaagctgct catatatgac gcttcaaacc ttgaaaccgg agtacctagc     180

cgcttcagcg gaagcggatc agggactgac ttcactttta ccatctcttc actgcagccc     240

gaagacatcg ccacatacta ctgccagcag tacggaaact tgccttttac atttgggggc     300

ggcaccaaag tggagattaa gcgaggggga ggcggctcag gaggcggtgg ctccggaggc     360

gggggttccc aggtccagct ccaggaatcc ggcccaggtc tggttaagcc cagtgaaact     420

ttgtccctca cgtgtactgt gagcggtggt tcaatctcct catactattg gtcttggata     480

cggcaacctc ctggaaaggg cctcgagtgg atcggctata tctactatag tggctccact     540

aattacaacc cttccctcaa gtccagagtc accatttccg tggacacatc taagaaccag     600

ttcagtctga agttgtccag cgttacagcc gcagacacag ccgtttatta ctgtgtgtct     660

cttgtttact gcgggggaga ctgttatagc ggcttcgatt actggggcca gggcaccttg     720

gtcacagtct cttccgcggc cgccctctct aacagtatta tgtacttttc tcattttgta     780

cccgtgttcc ttcccgctaa gccaactact accccggccc cacggccgcc tacccctgca     840

cccacaatag ccagtcagcc tttgagcctg agacctgagg cttgtcggcc ggctgctggg     900

ggtgcagtgc acacacgagg tcttgatttt gcttgcgaca tatacatctg ggcccctctg     960

gccgggacct gtggggtgct gcttctgagc ttggtcatca cgctctattg caaccatcgc    1020

aacagatcca aaagaagccg cctgctccat agcgattaca tgaatatgac tccacgccgc    1080

cctggcccca caaggaaaca ctaccagcct tacgcaccac ctagagattt cgctgcctat    1140

cggagcaggg tgaagttttc cagatctgca gatgcaccag cgtatcagca gggccagaac    1200

caactgtata acgagctcaa cctgggacgc agggaagagt atgacgtttt ggacaagcgc    1260

agaggacggg accctgagat gggtggcaaa ccaagacgaa aaaaccccca ggagggtctc    1320

tataatgagc tgcagaagga taagatggct gaagcctatt ctgaaatagg catgaaagga    1380

gagcggagaa ggggaaaagg gcacgacggt ttgtaccagg gactcagcac tgctacgaag 1440

gatacttatg acgctctcca catgcaagcc ctgccaccta gg 1482

<210> 48
<211> 492
<212> PRT
<213> Artificial Sequence

<220>
<221> source
<223> /note="Description of Artificial Sequence: Synthetic
      polypeptide"

<400> 48
Asp Ile Gln Leu Thr Gln Ser Pro Ser Ser Leu Ser Ala Ser Val Gly
1               5                   10                  15

Asp Arg Val Ser Phe Thr Cys Gln Ala Ser Gln Asp Ile Asn Asn Phe
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Gly Asn Leu Pro Phe
                85                  90                  95

Thr Phe Gly Gly Gly Thr Lys Val Glu Ile Lys Arg Gly Gly Gly Gly
            100                 105                 110

Ser Gly Gly Gly Gly Ser Gly Gly Gly Gly Ser Gln Val Gln Leu Gln
        115                 120                 125

Glu Ser Gly Pro Gly Leu Val Lys Pro Ser Glu Thr Leu Ser Leu Thr
    130                 135                 140

Cys Thr Val Ser Gly Gly Ser Ile Ser Ser Tyr Tyr Trp Ser Trp Ile
145                 150                 155                 160

Arg Gln Pro Pro Gly Lys Gly Leu Glu Trp Ile Gly Tyr Ile Tyr Tyr
                165                 170                 175

Ser Gly Ser Thr Asn Tyr Asn Pro Ser Leu Lys Ser Arg Val Thr Ile
            180                 185                 190

Ser Val Asp Thr Ser Lys Asn Gln Phe Ser Leu Lys Leu Ser Ser Val
        195                 200                 205

Thr Ala Asp Thr Ala Val Tyr Tyr Cys Val Ser Leu Val Tyr Cys Gly
    210                 215                 220

Gly Asp Cys Tyr Ser Gly Phe Asp Tyr Trp Gly Gln Gly Thr Leu Val
225                 230                 235                 240

Thr Val Ser Ser Ala Ala Ala Leu Ser Asn Ser Ile Met Tyr Phe Ser
                245                 250                 255

His Phe Val Pro Val Phe Leu Pro Ala Lys Pro Thr Thr Thr Pro Ala
            260                 265                 270

Pro Arg Pro Pro Thr Pro Ala Pro Thr Ile Ala Ser Gln Pro Leu Ser
        275                 280                 285

Leu Arg Pro Glu Ala Cys Arg Pro Ala Ala Gly Gly Ala Val His Thr
    290                 295                 300

Arg Gly Leu Asp Phe Ala Cys Asp Ile Tyr Ile Trp Ala Pro Leu Ala
305                 310                 315                 320

Gly Thr Cys Gly Val Leu Leu Leu Ser Leu Val Ile Thr Leu Tyr Cys
                325                 330                 335

Asn His Arg Asn Arg Ser Lys Arg Ser Arg Leu Leu His Ser Asp Tyr
            340                 345                 350

Met Asn Met Thr Pro Arg Arg Pro Gly Pro Thr Arg Lys His Tyr Gln
        355                 360                 365

Pro Tyr Ala Pro Pro Arg Asp Phe Ala Ala Tyr Arg Ser Arg Val Lys
    370                 375                 380

Phe Ser Arg Ser Ala Asp Ala Pro Ala Tyr Gln Gln Gly Gln Asn Gln
385                 390                 395                 400

Leu Tyr Asn Glu Leu Asn Leu Gly Arg Glu Glu Tyr Asp Val Leu Asp
                405                 410                 415

Lys Arg Arg Gly Arg Asp Pro Glu Met Gly Gly Lys Pro Arg Arg Lys
            420                 425                 430

Asn Pro Gln Glu Gly Leu Tyr Asn Glu Leu Gln Lys Asp Lys Met Ala
        435                 440                 445

Glu Ala Tyr Ser Glu Ile Gly Met Lys Gly Glu Arg Arg Arg Gly Lys
    450                 455                 460

Gly His Asp Gly Leu Tyr Gln Gly Leu Ser Thr Ala Thr Lys Asp Thr
465                 470                 475                 480

Tyr Asp Ala Leu His Met Gln Ala Leu Pro Pro Arg
                485                 490

<210> 49
<211> 372
<212> DNA
<213> Artificial Sequence

<220>
<221> source
<223> /note="Description of Artificial Sequence: Synthetic
      polynucleotide"

<400> 49
caggtacagc tgcaggaatc tgggcccgga cttgtcaagc caagtcagac actttctctt      60

acatgtaccg tgagcggcgg aagtataagc agtggaggct tttactggtc ttggatacgg     120

cagcacccag gcaaaggctt ggagtggatt ggatacattc atcattcagg atctacacac     180

tataatccat cccttaagtc ccgggtcacc attagcattg atacgtctaa gaatctgttc     240

agtctcaggc tgtcctccgt cactgctgcc gacacagccg tgtactactg cgcctccttg     300

gtttactgcg gaggcgactg ttatagcggc tttgattatt gggggcaggg gaccctcgta     360

accgtgagct ct                                                         372

<210> 50
<211> 124
<212> PRT
<213> Artificial Sequence

<220>
<221> source
<223> /note="Description of Artificial Sequence: Synthetic
      polypeptide"

<400> 50
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Phe Tyr Trp Ser Trp Ile Arg Gln His Pro Gly Lys Gly Leu Glu
        35                  40                  45

Trp Ile Gly Tyr Ile His His Ser Gly Ser Thr His Tyr Asn Pro Ser
    50                  55                  60

Leu Lys Ser Arg Val Thr Ile Ser Ile Asp Thr Ser Lys Asn Leu Phe
65                  70                  75                  80

Ser Leu Arg Leu Ser Ser Val Thr Ala Ala Asp Thr Ala Val Tyr Tyr
                85                  90                  95

Cys Ala Ser Leu Val Tyr Cys Gly Gly Asp Cys Tyr Ser Gly Phe Asp
            100                 105                 110

Tyr Trp Gly Gln Gly Thr Leu Val Thr Val Ser Ser
        115                 120

<210> 51
<211> 9
<212> PRT
<213> Artificial Sequence

<220>
<221> source
<223> /note="Description of Artificial Sequence: Synthetic
      peptide"

<400> 51
Gly Gly Ser Ile Ser Ser Gly Gly Phe
1               5

<210> 52
<211> 5
<212> PRT
<213> Artificial Sequence

<220>
<221> source
<223> /note="Description of Artificial Sequence: Synthetic
      peptide"

<400> 52
His His Ser Gly Ser
1               5

<210> 53
<211> 14
<212> PRT
<213> Artificial Sequence

<220>
<221> source
<223> /note="Description of Artificial Sequence: Synthetic
      peptide"

<400> 53
Leu Val Tyr Cys Gly Gly Asp Cys Tyr Ser Gly Phe Asp Tyr
1               5                   10

<210> 54
<211> 324
<212> DNA
<213> Artificial Sequence

<220>
<221> source
<223> /note="Description of Artificial Sequence: Synthetic
      polynucleotide"

<400> 54
gatatccagc tcactcaaag cccctctagt ctctctgcct cagtggggga tcgggtcagt      60

tttacttgtc aagcttcaca ggatatcaac aacttcctta attggtatca gcagaagcca     120

ggaaaagcac ccaagctgct catctatgat gcctcaaatt tggagacggg tgttcccagt     180

cgattctctg ggtcagggtc cgggaccgac tttacgttta cgatctcctc tctgcagccc     240

gaagacatcg ccacatacta ttgtcaacag tacggcaact tgcctttcac atttgggggc     300

gggactaagg ttgaaatcaa gagg                                            324


<210> 55
<211> 108
<212> PRT
<213> Artificial Sequence

<220>
<221> source
<223> /note="Description of Artificial Sequence: Synthetic
      polypeptide"

<400> 55
Asp Ile Gln Leu Thr Gln Ser Pro Ser Ser Leu Ser Ala Ser Val Gly
1               5                   10                  15

Asp Arg Val Ser Phe Thr Cys Gln Ala Ser Gln Asp Ile Asn Asn Phe
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Gly Asn Leu Pro Phe
                85                  90                  95

Thr Phe Gly Gly Gly Thr Lys Val Glu Ile Lys Arg
            100                 105

<210> 56
<211> 11
<212> PRT
<213> Artificial Sequence

<220>
<221> source
<223> /note="Description of Artificial Sequence: Synthetic
      peptide"

<400> 56
Gln Ala Ser Gln Asp Ile Asn Asn Phe Leu Asn
1               5                   10


<210> 57
<211> 7
<212> PRT
<213> Artificial Sequence

<220>
<221> source
<223> /note="Description of Artificial Sequence: Synthetic
      peptide"

<400> 57
Asp Ala Ser Asn Leu Glu Thr
1               5


<210> 58
<211> 9
<212> PRT
<213> Artificial Sequence

<220>
<221> source
<223> /note="Description of Artificial Sequence: Synthetic
      peptide"

<400> 58
Gln Gln Tyr Gly Asn Leu Pro Phe Thr
1               5


<210> 59
<211> 1446
<212> DNA
<213> Artificial Sequence

<220>
<221> source
<223> /note="Description of Artificial Sequence: Synthetic
      polynucleotide"

<400> 59
atggcactcc ccgtaactgc tctgctgctg ccgttggcat tgctcctgca cgccgcacgc      60

ccgcaggtac agctgcagga atctgggccc ggacttgtca agccaagtca gacactttct     120

cttacatgta ccgtgagcgg cggaagtata agcagtggag gcttttactg gtcttggata     180

cggcagcacc caggcaaagg cttggagtgg attggataca ttcatcattc aggatctaca 240

cactataatc catcccttaa gtcccgggtc accattagca ttgatacgtc taagaatctg 300

ttcagtctca ggctgtcctc cgtcactgct gccgacacag ccgtgtacta ctgcgcctcc 360

ttggtttact gcggaggcga ctgttatagc ggctttgatt attgggggca ggggaccctc 420

gtaaccgtga gctctggagg gggtgggagc gggggaggag gttcaggggg gggcggctcc 480

gatatccagc tcactcaaag cccctctagt ctctctgcct cagtggggga tcgggtcagt 540

tttacttgtc aagcttcaca ggatatcaac aacttcctta attggtatca gcagaagcca 600

ggaaaagcac ccaagctgct catctatgat gcctcaaatt tggagacggg tgttcccagt 660

cgattctctg ggtcagggtc cgggaccgac tttacgttta cgatctcctc tctgcagccc 720

gaagacatcg ccacatacta ttgtcaacag tacggcaact tgcctttcac atttgggggc 780

gggactaagg ttgaaatcaa gagggccgct gcactggaca atgagaagtc caacggcacc 840

atcatccacg tgaagggcaa gcacctgtgc cctagtcctc tgttcccagg cccatccaaa 900

cctttttggg ttcttgttgt ggtcgggggg gtgctggcct gctattctct gctggtcacg 960

gtggccttca taattttctg ggttagatcc aaaagaagcc gcctgctcca tagcgattac 1020

atgaatatga ctccacgccg ccctggcccc acaaggaaac actaccagcc ttacgcacca 1080

cctagagatt tcgctgccta tcggagcagg gtgaagtttt ccagatctgc agatgcacca 1140

gcgtatcagc agggccagaa ccaactgtat aacgagctca acctgggacg cagggaagag 1200

tatgacgttt tggacaagcg cagaggacgg gaccctgaga tgggtggcaa accaagacga 1260

aaaaaccccc aggagggtct ctataatgag ctgcagaagg ataagatggc tgaagcctat 1320

tctgaaatag gcatgaaagg agagcggaga aggggaaaag ggcacgacgg tttgtaccag 1380

ggactcagca ctgctacgaa ggatacttat gacgctctcc acatgcaagc cctgccacct 1440

aggtaa 1446

<210> 60
<211> 481
<212> PRT
<213> Artificial Sequence

<220>
<221> source
<223> /note="Description of Artificial Sequence: Synthetic
      polypeptide"

<400> 60
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Pro Gly Leu
            20                  25                  30

Val Lys Pro Ser Gln Thr Leu Ser Leu Thr Cys Thr Val Ser Gly Gly
        35                  40                  45

Ser Ile Ser Ser Gly Gly Phe Tyr Trp Ser Trp Ile Arg Gln His Pro
    50                  55                  60

Gly Lys Gly Leu Glu Trp Ile Gly Tyr Ile His His Ser Gly Ser Thr
65                  70                  75                  80

His Tyr Asn Pro Ser Leu Lys Ser Arg Val Thr Ile Ser Ile Asp Thr
                85                  90                  95

Ser Lys Asn Leu Phe Ser Leu Arg Leu Ser Ser Val Thr Ala Ala Asp
            100                 105                 110

Thr Ala Val Tyr Tyr Cys Ala Ser Leu Val Tyr Cys Gly Gly Asp Cys
        115                 120                 125

Tyr Ser Gly Phe Asp Tyr Trp Gly Gln Gly Thr Leu Val Thr Val Ser
    130                 135                 140

Ser Gly Gly Gly Gly Ser Gly Gly Gly Gly Ser Gly Gly Gly Gly Ser
145                 150                 155                 160

Asp Ile Gln Leu Thr Gln Ser Pro Ser Ser Leu Ser Ala Ser Val Gly
                165                 170                 175

Asp Arg Val Ser Phe Thr Cys Gln Ala Ser Gln Asp Ile Asn Asn Phe
            180                 185                 190

Leu Asn Trp Tyr Gln Gln Lys Pro Gly Lys Ala Pro Lys Leu Leu Ile
        195                 200                 205

Tyr Asp Ala Ser Asn Leu Glu Thr Gly Val Pro Ser Arg Phe Ser Gly
    210                 215                 220

Ser Gly Ser Gly Thr Asp Phe Thr Phe Thr Ile Ser Ser Leu Gln Pro
225                 230                 235                 240

Glu Asp Ile Ala Thr Tyr Tyr Cys Gln Gln Tyr Gly Asn Leu Pro Phe
                245                 250                 255

Thr Phe Gly Gly Gly Thr Lys Val Glu Ile Lys Arg Ala Ala Ala Leu
            260                 265                 270

Asp Asn Glu Lys Ser Asn Gly Thr Ile Ile His Val Lys Gly Lys His

275                 280                 285

Leu Cys Pro Ser Pro Leu Phe Pro Gly Pro Ser Lys Pro Phe Trp Val
    290                 295                 300

Leu Val Val Val Gly Gly Val Leu Ala Cys Tyr Ser Leu Leu Val Thr
305                 310                 315                 320

Val Ala Phe Ile Ile Phe Trp Val Arg Ser Lys Arg Ser Arg Leu Leu
                325                 330                 335

His Ser Asp Tyr Met Asn Met Thr Pro Arg Arg Pro Gly Pro Thr Arg
            340                 345                 350

Lys His Tyr Gln Pro Tyr Ala Pro Pro Arg Asp Phe Ala Ala Tyr Arg
        355                 360                 365

Ser Arg Val Lys Phe Ser Arg Ser Ala Asp Ala Pro Ala Tyr Gln Gln
    370                 375                 380

Gly Gln Asn Gln Leu Tyr Asn Glu Leu Asn Leu Gly Arg Arg Glu Glu
385                 390                 395                 400

Tyr Asp Val Leu Asp Lys Arg Arg Gly Arg Asp Pro Glu Met Gly Gly
                405                 410                 415

Lys Pro Arg Arg Lys Asn Pro Gln Glu Gly Leu Tyr Asn Glu Leu Gln
            420                 425                 430

Lys Asp Lys Met Ala Glu Ala Tyr Ser Glu Ile Gly Met Lys Gly Glu
        435                 440                 445

Arg Arg Arg Gly Lys Gly His Asp Gly Leu Tyr Gln Gly Leu Ser Thr
    450                 455                 460

Ala Thr Lys Asp Thr Tyr Asp Ala Leu His Met Gln Ala Leu Pro Pro
465                 470                 475                 480

Arg

<210> 61
<211> 1380
<212> DNA
<213> Artificial Sequence

<220>
<221> source
<223> /note="Description of Artificial Sequence: Synthetic

polynucleotide"

<400> 61
caggtacagc tgcaggaatc tgggcccgga cttgtcaagc caagtcagac actttctctt 60

acatgtaccg tgagcggcgg aagtataagc agtggaggct tttactggtc ttggatacgg 120

cagcacccag gcaaaggctt ggagtggatt ggatacattc atcattcagg atctacacac 180

tataatccat cccttaagtc ccgggtcacc attagcattg atacgtctaa gaatctgttc 240

agtctcaggc tgtcctccgt cactgctgcc gacacagccg tgtactactg cgcctccttg 300

gtttactgcg gaggcgactg ttatagcggc tttgattatt gggggcaggg gaccctcgta 360

accgtgagct ctggaggggg tgggagcggg ggaggaggtt cagggggggg cggctccgat 420

atccagctca ctcaaagccc ctctagtctc tctgcctcag tgggggatcg ggtcagtttt 480

acttgtcaag cttcacagga tatcaacaac ttccttaatt ggtatcagca gaagccagga 540

aaagcaccca agctgctcat ctatgatgcc tcaaatttgg agacgggtgt tcccagtcga 600

ttctctgggt cagggtccgg gaccgacttt acgtttacga tctcctctct gcagcccgaa 660

gacatcgcca catactattg tcaacagtac ggcaacttgc ctttcacatt tgggggcggg 720

actaaggttg aaatcaagag ggccgctgca ctggacaatg agaagtccaa cggcaccatc 780

atccacgtga agggcaagca cctgtgccct agtcctctgt tcccaggccc atccaaacct 840

ttttgggttc ttgttgtggt cgggggggtg ctggcctgct attctctgct ggtcacggtg 900

gccttcataa ttttctgggt tagatccaaa agaagccgcc tgctccatag cgattacatg 960

aatatgactc cacgccgccc tggccccaca aggaaacact accagcctta cgcaccacct 1020

agagatttcg ctgcctatcg gagcagggtg aagttttcca gatctgcaga tgcaccagcg 1080

tatcagcagg gccagaacca actgtataac gagctcaacc tgggacgcag ggaagagtat 1140

gacgttttgg acaagcgcag aggacgggac cctgagatgg gtggcaaacc aagacgaaaa 1200

aacccccagg agggtctcta taatgagctg cagaaggata agatggctga agcctattct 1260

gaaataggca tgaaaggaga gcggagaagg ggaaaagggc acgacggttt gtaccaggga 1320

ctcagcactg ctacgaagga tacttatgac gctctccaca tgcaagccct gccacctagg 1380

<210> 62
<211> 460
<212> PRT
<213> Artificial Sequence

<220>
<221> source
<223> /note="Description of Artificial Sequence: Synthetic
      polypeptide"

<400> 62
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
                20                  25                  30

Gly Phe Tyr Trp Ser Trp Ile Arg Gln His Pro Gly Lys Gly Leu Glu
            35                  40                  45

Trp Ile Gly Tyr Ile His His Ser Gly Ser Thr His Tyr Asn Pro Ser
    50                  55                  60

Leu Lys Ser Arg Val Thr Ile Ser Ile Asp Thr Ser Lys Asn Leu Phe
65                  70                  75                  80

Ser Leu Arg Leu Ser Ser Val Thr Ala Ala Asp Thr Ala Val Tyr Tyr
                85                  90                  95

Cys Ala Ser Leu Val Tyr Cys Gly Gly Asp Cys Tyr Ser Gly Phe Asp
                100                 105                 110

Tyr Trp Gly Gln Gly Thr Leu Val Thr Val Ser Ser Gly Gly Gly Gly
            115                 120                 125

Ser Gly Gly Gly Gly Ser Gly Gly Gly Gly Ser Asp Ile Gln Leu Thr
    130                 135                 140

Gln Ser Pro Ser Ser Leu Ser Ala Ser Val Gly Asp Arg Val Ser Phe
145                 150                 155                 160

Thr Cys Gln Ala Ser Gln Asp Ile Asn Asn Phe Leu Asn Trp Tyr Gln
                165                 170                 175

Gln Lys Pro Gly Lys Ala Pro Lys Leu Leu Ile Tyr Asp Ala Ser Asn
                180                 185                 190

Leu Glu Thr Gly Val Pro Ser Arg Phe Ser Gly Ser Gly Ser Gly Thr
            195                 200                 205

Asp Phe Thr Phe Thr Ile Ser Ser Leu Gln Pro Glu Asp Ile Ala Thr
    210                 215                 220

Tyr Tyr Cys Gln Gln Tyr Gly Asn Leu Pro Phe Thr Phe Gly Gly Gly
225                 230                 235                 240

Thr Lys Val Glu Ile Lys Arg Ala Ala Ala Leu Asp Asn Glu Lys Ser
                245                 250                 255

Asn Gly Thr Ile Ile His Val Lys Gly Lys His Leu Cys Pro Ser Pro

260                 265                 270

Leu Phe Pro Gly Pro Ser Lys Pro Phe Trp Val Leu Val Val Val Gly
        275                 280                 285

Gly Val Leu Ala Cys Tyr Ser Leu Leu Val Thr Val Ala Phe Ile Ile
    290                 295                 300

Phe Trp Val Arg Ser Lys Arg Ser Arg Leu Leu His Ser Asp Tyr Met
305                 310                 315                 320

Asn Met Thr Pro Arg Arg Pro Gly Pro Thr Arg Lys His Tyr Gln Pro
                325                 330                 335

Tyr Ala Pro Pro Arg Asp Phe Ala Ala Tyr Arg Ser Arg Val Lys Phe
            340                 345                 350

Ser Arg Ser Ala Asp Ala Pro Ala Tyr Gln Gln Gly Gln Asn Gln Leu
        355                 360                 365

Tyr Asn Glu Leu Asn Leu Gly Arg Arg Glu Glu Tyr Asp Val Leu Asp
    370                 375                 380

Lys Arg Arg Gly Arg Asp Pro Glu Met Gly Gly Lys Pro Arg Arg Lys
385                 390                 395                 400

Asn Pro Gln Glu Gly Leu Tyr Asn Glu Leu Gln Lys Asp Lys Met Ala
                405                 410                 415

Glu Ala Tyr Ser Glu Ile Gly Met Lys Gly Glu Arg Arg Arg Gly Lys
            420                 425                 430

Gly His Asp Gly Leu Tyr Gln Gly Leu Ser Thr Ala Thr Lys Asp Thr
        435                 440                 445

Tyr Asp Ala Leu His Met Gln Ala Leu Pro Pro Arg
    450                 455                 460

<210> 63
<211> 1473
<212> DNA
<213> Artificial Sequence

<220>
<221> source
<223> /note="Description of Artificial Sequence: Synthetic
      polynucleotide"

<400> 63
atggcactcc ccgtaactgc tctgctgctg ccgttggcat tgctcctgca cgccgcacgc        60

ccgcaggtgc agctgcagga aagcggtccg ggacttgtca agccgtccca aacgctgagt 120

ctgacgtgta ctgtctctgg tggctctatt tcttccgggg gcttttattg gtcttggatc 180

agacaacacc ctggcaaagg gctggagtgg atagggtata ttcaccactc tgggtccact 240

cactacaacc catcattgaa atccagagtg actatctcaa tcgacacatc caagaacctt 300

ttcagcctga ggttgtcatc agttaccgcc gctgacaccg cggtgtatta ttgcgcctct 360

ctcgtgtact gcggtggcga ttgttatagt ggctttgact actgggggca ggggacattg 420

gttaccgttt caagtggagg cggtgggtct ggcgggggcg gtagcggagg tggggggagc 480

gacatacagc ttacgcagag cccctccagc ctttcagcct ccgtggggga tagggtgtcc 540

tttacctgcc aggcttccca ggacataaac aacttcctca attggtatca gcaaaagccc 600

gggaaagcac caaagctgct catctacgat gccagcaacc tggaaaccgg agtgccgtct 660

cgcttctctg gaagtggcag tgggaccgat ttcactttta caatctcaag tttgcagcca 720

gaagacattg caacatacta ctgtcaacag tacggcaatc tcccctttac atttgggggg 780

ggaactaaag tggagattaa gcgcgctgca gccattgaag ttatgtatcc gcccccgtat 840

ctggataacg agaaatctaa tggtaccata atacatgtga aggggaagca cctctgtcca 900

tcaccgctgt tccccggccc ttcaaaacct ttctgggtac tcgttgtcgt gggtggagtt 960

ctggcctgct atagtctgct ggtgaccgtg gcgtttatca tcttctgggt aagatccaaa 1020

agaagccgcc tgctccatag cgattacatg aatatgactc cacgccgccc tggccccaca 1080

aggaaacact accagcctta cgcaccacct agagatttcg ctgcctatcg gagcagggtg 1140

aagttttcca gatctgcaga tgcaccagcg tatcagcagg gccagaacca actgtataac 1200

gagctcaacc tgggacgcag ggaagagtat gacgttttgg acaagcgcag aggacgggac 1260

cctgagatgg gtggcaaacc aagacgaaaa aacccccagg agggtctcta taatgagctg 1320

cagaaggata agatggctga agcctattct gaaataggca tgaaaggaga gcggagaagg 1380

ggaaaagggc acgacggttt gtaccaggga ctcagcactg ctacgaagga tacttatgac 1440

gctctccaca tgcaagccct gccacctagg taa 1473

<210> 64
<211> 490
<212> PRT
<213> Artificial Sequence

<220>
<221> source
<223> /note="Description of Artificial Sequence: Synthetic
      polypeptide"

<400> 64
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Pro Gly Leu
            20                  25                  30

Val Lys Pro Ser Gln Thr Leu Ser Leu Thr Cys Thr Val Ser Gly Gly
        35                  40                  45

Ser Ile Ser Ser Gly Gly Phe Tyr Trp Ser Trp Ile Arg Gln His Pro
    50                  55                  60

Gly Lys Gly Leu Glu Trp Ile Gly Tyr Ile His His Ser Gly Ser Thr
65                  70                  75                  80

His Tyr Asn Pro Ser Leu Lys Ser Arg Val Thr Ile Ser Ile Asp Thr
                85                  90                  95

Ser Lys Asn Leu Phe Ser Leu Arg Leu Ser Ser Val Thr Ala Ala Asp
            100                 105                 110

Thr Ala Val Tyr Tyr Cys Ala Ser Leu Val Tyr Cys Gly Gly Asp Cys
        115                 120                 125

Tyr Ser Gly Phe Asp Tyr Trp Gly Gln Gly Thr Leu Val Thr Val Ser
    130                 135                 140

Ser Gly Gly Gly Gly Ser Gly Gly Gly Gly Ser Gly Gly Gly Gly Ser
145                 150                 155                 160

Asp Ile Gln Leu Thr Gln Ser Pro Ser Ser Leu Ser Ala Ser Val Gly
                165                 170                 175

Asp Arg Val Ser Phe Thr Cys Gln Ala Ser Gln Asp Ile Asn Asn Phe
            180                 185                 190

Leu Asn Trp Tyr Gln Gln Lys Pro Gly Lys Ala Pro Lys Leu Leu Ile
        195                 200                 205

Tyr Asp Ala Ser Asn Leu Glu Thr Gly Val Pro Ser Arg Phe Ser Gly
    210                 215                 220

Ser Gly Ser Gly Thr Asp Phe Thr Phe Thr Ile Ser Ser Leu Gln Pro
225                 230                 235                 240

Glu Asp Ile Ala Thr Tyr Tyr Cys Gln Gln Tyr Gly Asn Leu Pro Phe
                245                 250                 255

Thr Phe Gly Gly Gly Thr Lys Val Glu Ile Lys Arg Ala Ala Ala Ile

260                 265                 270

Glu Val Met Tyr Pro Pro Pro Tyr Leu Asp Asn Glu Lys Ser Asn Gly
        275                 280                 285

Thr Ile Ile His Val Lys Gly Lys His Leu Cys Pro Ser Pro Leu Phe
    290                 295                 300

Pro Gly Pro Ser Lys Pro Phe Trp Val Leu Val Val Val Gly Gly Val
305                 310                 315                 320

Leu Ala Cys Tyr Ser Leu Leu Val Thr Val Ala Phe Ile Ile Phe Trp
                325                 330                 335

Val Arg Ser Lys Arg Ser Arg Leu Leu His Ser Asp Tyr Met Asn Met
            340                 345                 350

Thr Pro Arg Arg Pro Gly Pro Thr Arg Lys His Tyr Gln Pro Tyr Ala
        355                 360                 365

Pro Pro Arg Asp Phe Ala Ala Tyr Arg Ser Arg Val Lys Phe Ser Arg
    370                 375                 380

Ser Ala Asp Ala Pro Ala Tyr Gln Gln Gly Gln Asn Gln Leu Tyr Asn
385                 390                 395                 400

Glu Leu Asn Leu Gly Arg Arg Glu Glu Tyr Asp Val Leu Asp Lys Arg
                405                 410                 415

Arg Gly Arg Asp Pro Glu Met Gly Gly Lys Pro Arg Arg Lys Asn Pro
            420                 425                 430

Gln Glu Gly Leu Tyr Asn Glu Leu Gln Lys Asp Lys Met Ala Glu Ala
        435                 440                 445

Tyr Ser Glu Ile Gly Met Lys Gly Glu Arg Arg Arg Gly Lys Gly His
    450                 455                 460

Asp Gly Leu Tyr Gln Gly Leu Ser Thr Ala Thr Lys Asp Thr Tyr Asp
465                 470                 475                 480

Ala Leu His Met Gln Ala Leu Pro Pro Arg
                485                 490

<210> 65
<211> 1407
<212> DNA
<213> Artificial Sequence

<220>
<221> source
<223> /note="Description of Artificial Sequence: Synthetic
      polynucleotide"

<400> 65
caggtgcagc tgcaggaaag cggtccggga cttgtcaagc cgtcccaaac gctgagtctg      60

acgtgtactg tctctggtgg ctctatttct tccgggggct tttattggtc ttggatcaga     120

caacaccctg gcaaagggct ggagtggata gggtatattc accactctgg gtccactcac     180

tacaacccat cattgaaatc cagagtgact atctcaatcg acacatccaa gaaccttttc     240

agcctgaggt tgtcatcagt taccgccgct gacaccgcgg tgtattattg cgcctctctc     300

gtgtactgcg gtggcgattg ttatagtggc tttgactact gggggcaggg gacattggtt     360

accgtttcaa gtggaggcgg tgggtctggc gggggcggta gcggaggtgg ggggagcgac     420

atacagctta cgcagagccc ctccagcctt tcagcctccg tgggggatag ggtgtccttt     480

acctgccagg cttcccagga cataaacaac ttcctcaatt ggtatcagca aaagcccggg     540

aaagcaccaa agctgctcat ctacgatgcc agcaacctgg aaaccggagt gccgtctcgc     600

ttctctggaa gtggcagtgg gaccgatttc acttttacaa tctcaagttt gcagccagaa     660

gacattgcaa catactactg tcaacagtac ggcaatctcc cctttacatt tgggggggga      720

actaaagtgg agattaagcg cgctgcagcc attgaagtta tgtatccgcc cccgtatctg     780

gataacgaga aatctaatgg taccataata catgtgaagg ggaagcacct ctgtccatca     840

ccgctgttcc ccggcccttc aaaacctttc tgggtactcg ttgtcgtggg tggagttctg     900

gcctgctata gtctgctggt gaccgtggcg tttatcatct tctgggtaag atccaaaaga     960

agccgcctgc tccatagcga ttacatgaat atgactccac gccgccctgg ccccacaagg    1020

aaacactacc agccttacgc accacctaga gatttcgctg cctatcggag cagggtgaag    1080

ttttccagat ctgcagatgc accagcgtat cagcagggcc agaaccaact gtataacgag    1140

ctcaacctgg gacgcaggga agagtatgac gttttggaca agcgcagagg acgggaccct    1200

gagatgggtg gcaaaccaag acgaaaaaac ccccaggagg gtctctataa tgagctgcag    1260

aaggataaga tggctgaagc ctattctgaa ataggcatga aaggagagcg gagaagggga    1320

aaagggcacg acggtttgta ccagggactc agcactgcta cgaaggatac ttatgacgct    1380

ctccacatgc aagccctgcc acctagg                                        1407


<210> 66
<211> 469
<212> PRT
<213> Artificial Sequence

<220>
<221> source

<223> /note="Description of Artificial Sequence: Synthetic
      polypeptide"

<400> 66
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Phe Tyr Trp Ser Trp Ile Arg Gln His Pro Gly Lys Gly Leu Glu
        35                  40                  45

Trp Ile Gly Tyr Ile His His Ser Gly Ser Thr His Tyr Asn Pro Ser
    50                  55                  60

Leu Lys Ser Arg Val Thr Ile Ser Ile Asp Thr Ser Lys Asn Leu Phe
65                  70                  75                  80

Ser Leu Arg Leu Ser Ser Val Thr Ala Ala Asp Thr Ala Val Tyr Tyr
                85                  90                  95

Cys Ala Ser Leu Val Tyr Cys Gly Gly Asp Cys Tyr Ser Gly Phe Asp
            100                 105                 110

Tyr Trp Gly Gln Gly Thr Leu Val Thr Val Ser Ser Gly Gly Gly Gly
        115                 120                 125

Ser Gly Gly Gly Gly Ser Gly Gly Gly Gly Ser Asp Ile Gln Leu Thr
    130                 135                 140

Gln Ser Pro Ser Ser Leu Ser Ala Ser Val Gly Asp Arg Val Ser Phe
145                 150                 155                 160

Thr Cys Gln Ala Ser Gln Asp Ile Asn Asn Phe Leu Asn Trp Tyr Gln
                165                 170                 175

Gln Lys Pro Gly Lys Ala Pro Lys Leu Leu Ile Tyr Asp Ala Ser Asn
            180                 185                 190

Leu Glu Thr Gly Val Pro Ser Arg Phe Ser Gly Ser Gly Ser Gly Thr
        195                 200                 205

Asp Phe Thr Phe Thr Ile Ser Ser Leu Gln Pro Glu Asp Ile Ala Thr
    210                 215                 220

Tyr Tyr Cys Gln Gln Tyr Gly Asn Leu Pro Phe Thr Phe Gly Gly Gly
225                 230                 235                 240

Thr Lys Val Glu Ile Lys Arg Ala Ala Ala Ile Glu Val Met Tyr Pro
                245                 250                 255

Pro Pro Tyr Leu Asp Asn Glu Lys Ser Asn Gly Thr Ile Ile His Val
            260                 265                 270

Lys Gly Lys His Leu Cys Pro Ser Pro Leu Phe Pro Gly Pro Ser Lys
        275                 280                 285

Pro Phe Trp Val Leu Val Val Val Gly Gly Val Leu Ala Cys Tyr Ser
    290                 295                 300

Leu Leu Val Thr Val Ala Phe Ile Ile Phe Trp Val Arg Ser Lys Arg
305                 310                 315                 320

Ser Arg Leu Leu His Ser Asp Tyr Met Asn Met Thr Pro Arg Arg Pro
                325                 330                 335

Gly Pro Thr Arg Lys His Tyr Gln Pro Tyr Ala Pro Pro Arg Asp Phe
            340                 345                 350

Ala Ala Tyr Arg Ser Arg Val Lys Phe Ser Arg Ser Ala Asp Ala Pro
        355                 360                 365

Ala Tyr Gln Gln Gly Gln Asn Gln Leu Tyr Asn Glu Leu Asn Leu Gly
    370                 375                 380

Arg Arg Glu Glu Tyr Asp Val Leu Asp Lys Arg Arg Gly Arg Asp Pro
385                 390                 395                 400

Glu Met Gly Gly Lys Pro Arg Arg Lys Asn Pro Gln Glu Gly Leu Tyr
                405                 410                 415

Asn Glu Leu Gln Lys Asp Lys Met Ala Glu Ala Tyr Ser Glu Ile Gly
            420                 425                 430

Met Lys Gly Glu Arg Arg Arg Gly Lys Gly His Asp Gly Leu Tyr Gln
        435                 440                 445

Gly Leu Ser Thr Ala Thr Lys Asp Thr Tyr Asp Ala Leu His Met Gln
    450                 455                 460

Ala Leu Pro Pro Arg
465

<210> 67
<211> 1554

<212> DNA
<213> Artificial Sequence

<220>
<221> source
<223> /note="Description of Artificial Sequence: Synthetic
      polynucleotide"

<400> 67
atggcactcc ccgtaactgc tctgctgctg ccgttggcat tgctcctgca cgccgcacgc      60

ccgcaggtgc agttgcagga aagcgggcct ggccttgtga aaccaagcca gacactgagc     120

ctgacatgca ctgtgtccgg cgggtccata tcttccgggg gtttttattg gtcctggata     180

cgccagcatc ccgggaaagg acttgaatgg attggatata tccaccattc cggaagcacc     240

cactacaatc caagccttaa atcccgggtg acaatctcca tcgacacctc aaagaatctt     300

ttttccctgc ggttgtcttc agtaactgcc gccgataccg ctgtgtacta ctgtgccagc     360

ctcgtctatt gcggcggaga ttgttattct gggttcgatt attggggtca aggcacactg     420

gtaactgtca gcagcggagg cggcggttcc gggggcgggg gcagtggagg gggcggatct     480

gacattcagc ttacgcagtc cccatcttca cttagcgcca gcgttggcga tcgggtcagc     540

ttcacgtgtc aagcaagtca ggatatcaac aactttctta actggtacca gcagaagcca     600

ggcaaggcac ccaagttgct gatttacgat gcttctaacc tcgagacggg agtgcctagc     660

cgcttctccg ggagcggcag cggcacagac tttaccttta cgatttccag tctgcagcca     720

gaggatatag caacttatta ctgtcagcag tatggcaacc tcccttttac cttcggtggt     780

ggcacaaagg tcgagattaa aagagccgca gcgttgtcca actccataat gtatttttct     840

cattttgtgc ccgtctttct gcctgccaaa cctaccacca cccccgcccc acgaccacct     900

actccagccc ccaccatcgc ctcccagccc ctcagcctga ggccagaggc ttgtcgccct     960

gctgcggggg gcgctgtcca taccagagga ctcgacttcg cctgcgatat ttatatatgg    1020

gcccccctcg ccggcacctg cggagtcttg ctcctgagcc ttgtgatcac gctttattgt    1080

aaccatcgga atagatccaa aagaagccgc ctgctccata gcgattacat gaatatgact    1140

ccacgccgcc ctggccccac aaggaaacac taccagcctt acgcaccacc tagagatttc    1200

gctgcctatc ggagcagggt gaagttttcc agatctgcag atgcaccagc gtatcagcag    1260

ggccagaacc aactgtataa cgagctcaac ctgggacgca gggaagagta tgacgttttg    1320

gacaagcgca gaggacggga ccctgagatg ggtggcaaac caagacgaaa aaacccccag    1380

gagggtctct ataatgagct gcagaaggat aagatggctg aagcctattc tgaaataggc    1440

atgaaaggag agcggagaag gggaaaaggg cacgacggtt tgtaccaggg actcagcact    1500

gctacgaagg atacttatga cgctctccac atgcaagccc tgccacctag gtaa          1554

<210> 68

<211> 515
<212> PRT
<213> Artificial Sequence

<220>
<221> source
<223> /note="Description of Artificial Sequence: Synthetic
      polypeptide"

<400> 68
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Pro Gly Leu
            20                  25                  30

Val Lys Pro Ser Gln Thr Leu Ser Leu Thr Cys Thr Val Ser Gly Gly
        35                  40                  45

Ser Ile Ser Ser Gly Gly Phe Tyr Trp Ser Trp Ile Arg Gln His Pro
    50                  55                  60

Gly Lys Gly Leu Glu Trp Ile Gly Tyr Ile His His Ser Gly Ser Thr
65                  70                  75                  80

His Tyr Asn Pro Ser Leu Lys Ser Arg Val Thr Ile Ser Ile Asp Thr
                85                  90                  95

Ser Lys Asn Leu Phe Ser Leu Arg Leu Ser Ser Val Thr Ala Ala Asp
            100                 105                 110

Thr Ala Val Tyr Tyr Cys Ala Ser Leu Val Tyr Cys Gly Gly Asp Cys
        115                 120                 125

Tyr Ser Gly Phe Asp Tyr Trp Gly Gln Gly Thr Leu Val Thr Val Ser
    130                 135                 140

Ser Gly Gly Gly Gly Ser Gly Gly Gly Gly Ser Gly Gly Gly Gly Ser
145                 150                 155                 160

Asp Ile Gln Leu Thr Gln Ser Pro Ser Ser Leu Ser Ala Ser Val Gly
                165                 170                 175

Asp Arg Val Ser Phe Thr Cys Gln Ala Ser Gln Asp Ile Asn Asn Phe
            180                 185                 190

Leu Asn Trp Tyr Gln Gln Lys Pro Gly Lys Ala Pro Lys Leu Leu Ile
        195                 200                 205

Tyr Asp Ala Ser Asn Leu Glu Thr Gly Val Pro Arg Phe Ser Gly Ser

210                 215                 220

Gly Ser Gly Thr Asp Phe Thr Phe Thr Ile Ser Ser Leu Gln Pro Glu
225                 230                 235                 240

Asp Ile Ala Thr Tyr Tyr Cys Gln Tyr Gly Asn Leu Pro Phe Thr Phe
                245                 250                 255

Gly Gly Gly Thr Lys Val Glu Ile Lys Arg Ala Ala Ala Leu Ser Asn
            260                 265                 270

Ser Ile Met Tyr Phe Ser His Phe Val Pro Val Phe Leu Pro Ala Lys
        275                 280                 285

Pro Thr Thr Thr Pro Ala Pro Arg Pro Pro Thr Pro Ala Pro Thr Ile
    290                 295                 300

Ala Ser Gln Pro Leu Ser Leu Arg Pro Glu Ala Cys Arg Pro Ala Ala
305                 310                 315                 320

Gly Gly Ala Val His Thr Arg Gly Leu Asp Phe Ala Cys Asp Ile Tyr
                325                 330                 335

Ile Trp Ala Pro Leu Ala Gly Thr Cys Gly Val Leu Leu Leu Ser Leu
            340                 345                 350

Val Ile Thr Leu Tyr Cys Asn His Arg Asn Arg Ser Lys Arg Ser Arg
        355                 360                 365

Leu Leu His Ser Asp Tyr Met Asn Met Thr Pro Arg Arg Pro Gly Pro
    370                 375                 380

Thr Arg Lys His Tyr Gln Pro Tyr Ala Pro Pro Arg Asp Phe Ala Ala
385                 390                 395                 400

Tyr Arg Ser Arg Val Lys Phe Ser Arg Ser Ala Asp Ala Pro Ala Tyr
                405                 410                 415

Gln Gln Gly Gln Asn Gln Leu Tyr Asn Glu Leu Asn Leu Gly Arg Arg
            420                 425                 430

Glu Glu Tyr Asp Val Leu Asp Lys Arg Arg Gly Arg Asp Pro Glu Met
        435                 440                 445

Gly Gly Lys Pro Arg Arg Lys Asn Pro Gln Glu Gly Leu Tyr Asn Glu
    450                 455                 460

Leu Gln Lys Asp Lys Met Ala Glu Ala Tyr Ser Glu Ile Gly Met Lys
465                 470                 475                 480

Gly Glu Arg Arg Arg Gly Lys Gly His Asp Gly Leu Tyr Gln Gly Leu
                485                 490                 495

Ser Thr Ala Thr Lys Asp Thr Tyr Asp Ala Leu His Met Gln Ala Leu
            500                 505                 510

Pro Pro Arg
        515

<210> 69
<211> 1488
<212> DNA
<213> Artificial Sequence

<220>
<221> source
<223> /note="Description of Artificial Sequence: Synthetic
      polynucleotide"

<400> 69
caggtgcagt tgcaggaaag cgggcctggc cttgtgaaac caagccagac actgagcctg      60

acatgcactg tgtccggcgg gtccatatct tccgggggtt tttattggtc ctggatacgc     120

cagcatcccg ggaaaggact tgaatggatt ggatatatcc accattccgg aagcacccac     180

tacaatccaa gccttaaatc ccgggtgaca atctccatcg acacctcaaa gaatcttttt     240

tccctgcggt tgtcttcagt aactgccgcc gataccgctg tgtactactg tgccagcctc     300

gtctattgcg gcggagattg ttattctggg ttcgattatt ggggtcaagg cacactggta     360

actgtcagca gcggaggcgg cggttccggg ggcgggggca gtggaggggg cggatctgac     420

attcagctta cgcagtcccc atcttcactt agcgccagcg ttggcgatcg ggtcagcttc     480

acgtgtcaag caagtcagga tatcaacaac tttcttaact ggtaccagca gaagccaggc     540

aaggcaccca agttgctgat ttacgatgct tctaacctcg agacgggagt gcctagccgc     600

ttctccggga gcggcagcgg cacagacttt acctttacga tttccagtct gcagccagag     660

gatatagcaa cttattactg tcagcagtat ggcaacctcc cttttacctt cggtggtggc     720

acaaaggtcg agattaaaag agccgcagcg ttgtccaact ccataatgta tttttctcat     780

tttgtgcccg tctttctgcc tgccaaacct accaccaccc ccgccccacg accacctact     840

ccagccccca ccatcgcctc ccagcccctc agcctgaggc cagaggcttg tcgccctgct     900

gcggggggcg ctgtccatac cagaggactc gacttcgcct gcgatattta tatatgggcc     960

cccctcgccg gcacctgcgg agtcttgctc ctgagccttg tgatcacgct ttattgtaac    1020

catcggaata gatccaaaag aagccgcctg ctccatagcg attacatgaa tatgactcca    1080

cgccgccctg gccccacaag gaaacactac cagccttacg caccacctag agatttcgct 1140

gcctatcgga gcagggtgaa gttttccaga tctgcagatg caccagcgta tcagcagggc 1200

cagaaccaac tgtataacga gctcaacctg ggacgcaggg aagagtatga cgttttggac 1260

aagcgcagag gacgggaccc tgagatgggt ggcaaaccaa gacgaaaaaa cccccaggag 1320

ggtctctata atgagctgca gaaggataag atggctgaag cctattctga aataggcatg 1380

aaaggagagc ggagaagggg aaaagggcac gacggtttgt accagggact cagcactgct 1440

acgaaggata cttatgacgc tctccacatg caagccctgc cacctagg 1488

<210> 70
<211> 496
<212> PRT
<213> Artificial Sequence

<220>
<221> source
<223> /note="Description of Artificial Sequence: Synthetic
      polypeptide"

<400> 70
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Phe Tyr Trp Ser Trp Ile Arg Gln His Pro Gly Lys Gly Leu Glu
        35                  40                  45

Trp Ile Gly Tyr Ile His His Ser Gly Ser Thr His Tyr Asn Pro Ser
    50                  55                  60

Leu Lys Ser Arg Val Thr Ile Ser Ile Asp Thr Ser Lys Asn Leu Phe
65                  70                  75                  80

Ser Leu Arg Leu Ser Ser Val Thr Ala Ala Asp Thr Ala Val Tyr Tyr
                85                  90                  95

Cys Ala Ser Leu Val Tyr Cys Gly Gly Asp Cys Tyr Ser Gly Phe Asp
            100                 105                 110

Tyr Trp Gly Gln Gly Thr Leu Val Thr Val Ser Ser Gly Gly Gly Gly
        115                 120                 125

Ser Gly Gly Gly Gly Ser Gly Gly Gly Gly Ser Asp Ile Gln Leu Thr
    130                 135                 140

Gln Ser Pro Ser Ser Leu Ser Ala Ser Val Gly Asp Arg Val Ser Phe

145                 150                 155                 160

Thr Cys Gln Ala Ser Gln Asp Ile Asn Asn Phe Leu Asn Trp Tyr Gln
                165                 170                 175

Gln Lys Pro Gly Lys Ala Pro Lys Leu Leu Ile Tyr Asp Ala Ser Asn
            180                 185                 190

Leu Glu Thr Gly Val Pro Ser Arg Phe Ser Gly Ser Gly Ser Gly Thr
        195                 200                 205

Asp Phe Thr Phe Thr Ile Ser Ser Leu Gln Pro Glu Asp Ile Ala Thr
    210                 215                 220

Tyr Tyr Cys Gln Gln Tyr Gly Asn Leu Pro Phe Thr Phe Gly Gly Gly
225                 230                 235                 240

Thr Lys Val Glu Ile Lys Arg Ala Ala Ala Leu Ser Asn Ser Ile Met
                245                 250                 255

Tyr Phe Ser His Phe Val Pro Val Phe Leu Pro Ala Lys Pro Thr Thr
            260                 265                 270

Thr Pro Ala Pro Arg Pro Pro Thr Pro Ala Pro Thr Ile Ala Ser Gln
        275                 280                 285

Pro Leu Ser Leu Arg Pro Glu Ala Cys Arg Pro Ala Ala Gly Gly Ala
    290                 295                 300

Val His Thr Arg Gly Leu Asp Phe Ala Cys Asp Ile Tyr Ile Trp Ala
305                 310                 315                 320

Pro Leu Ala Gly Thr Cys Gly Val Leu Leu Leu Ser Leu Val Ile Thr
                325                 330                 335

Leu Tyr Cys Asn His Arg Asn Arg Ser Lys Arg Ser Arg Leu Leu His
            340                 345                 350

Ser Asp Tyr Met Asn Met Thr Pro Arg Arg Pro Gly Pro Thr Arg Lys
        355                 360                 365

His Tyr Gln Pro Tyr Ala Pro Pro Arg Asp Phe Ala Ala Tyr Arg Ser
    370                 375                 380

Arg Val Lys Phe Ser Arg Ser Ala Asp Ala Pro Ala Tyr Gln Gln Gly
385                 390                 395                 400

Gln Asn Gln Leu Tyr Asn Glu Leu Asn Leu Gly Arg Arg Glu Glu Tyr
                405                 410                 415

Asp Val Leu Asp Lys Arg Arg Gly Arg Asp Pro Glu Met Gly Gly Lys
            420                 425                 430

Pro Arg Arg Lys Asn Pro Gln Glu Gly Leu Tyr Asn Glu Leu Gln Lys
        435                 440                 445

Asp Lys Met Ala Glu Ala Tyr Ser Glu Ile Gly Met Lys Gly Glu Arg
    450                 455                 460

Arg Arg Gly Lys Gly His Asp Gly Leu Tyr Gln Gly Leu Ser Thr Ala
465                 470                 475                 480

Thr Lys Asp Thr Tyr Asp Ala Leu His Met Gln Ala Leu Pro Pro Arg
                485                 490                 495

<210> 71
<211> 363
<212> DNA
<213> Artificial Sequence

<220>
<221> source
<223> /note="Description of Artificial Sequence: Synthetic
      polynucleotide"

<400> 71
caggtccaac tggtgcagtc cggagccgaa gtcaagaaac caggtgcctc cgttaaagtg      60

agttgcaaag tctctggata cactctgacc gagctctcta tgcactgggt ccggcaggcc     120

cccggcaagg gattggaatg gatgggcggg ttcgatcctg aggacggaga gactatctac     180

gctcaaaaat tccagggacg agtgactgtg accgaagaca ctagtaccga cactgcctac     240

atggaacttt cctctctgcg atcagaagat accgcagtgt actactgtgc tactgaatct     300

aggggcattg gatggcccta cttcgattac tggggtcagg gaactctggt gactgtctcc     360

agc                                                                   363

<210> 72
<211> 121
<212> PRT
<213> Artificial Sequence

<220>
<221> source
<223> /note="Description of Artificial Sequence: Synthetic
      polypeptide"

<400> 72
Gln Val Gln Leu Val Gln Ser Gly Ala Glu Val Lys Lys Pro Gly Ala
1               5                   10                  15

Ser Val Lys Val Ser Cys Lys Val Ser Gly Tyr Thr Leu Thr Glu Leu
            20                  25                  30

Ser Met His Trp Val Arg Gln Ala Pro Gly Lys Gly Leu Glu Trp Met
        35                  40                  45

Gly Gly Phe Asp Pro Glu Asp Gly Glu Thr Ile Tyr Ala Gln Lys Phe
    50                  55                  60

Gln Gly Arg Val Thr Val Thr Glu Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Thr Glu Ser Arg Gly Ile Gly Trp Pro Tyr Phe Asp Tyr Trp Gly
            100                 105                 110

Gln Gly Thr Leu Val Thr Val Ser Ser
        115                 120


<210> 73
<211> 7
<212> PRT
<213> Artificial Sequence

<220>
<221> source
<223> /note="Description of Artificial Sequence: Synthetic
      peptide"

<400> 73
Gly Tyr Thr Leu Thr Glu Leu
1               5


<210> 74
<211> 6
<212> PRT
<213> Artificial Sequence

<220>
<221> source
<223> /note="Description of Artificial Sequence: Synthetic
      peptide"

<400> 74
Asp Pro Glu Asp Gly Glu
1               5


<210> 75
<211> 12
<212> PRT

<213> Artificial Sequence

<220>
<221> source
<223> /note="Description of Artificial Sequence: Synthetic
      peptide"

<400> 75
Glu Ser Arg Gly Ile Gly Trp Pro Tyr Phe Asp Tyr
1               5                   10


<210> 76
<211> 324
<212> DNA
<213> Artificial Sequence

<220>
<221> source
<223> /note="Description of Artificial Sequence: Synthetic
      polynucleotide"

<400> 76
gatattcaga tgactcaatc tccttcttct ctgtccgctt ccgtgggcga tagagtgacc      60

attacttgta gggcgtccca gtcaatctcc agttatttga attggtatca gcagaagccc     120

gggaaagcac ctaagctgtt gatcagcggg gcttctagcc tgaagagtgg ggtaccttca     180

cggttcagcg gaagcggaag cggaaccgat ttcaccctga ctatcagcag cctgccacct     240

gaggactttg caacttacta ctgccaacag tcatacagca ctccgatcac tttcggccag     300

ggcacccggc tcgaaatcaa gcgc                                            324


<210> 77
<211> 108
<212> PRT
<213> Artificial Sequence

<220>
<221> source
<223> /note="Description of Artificial Sequence: Synthetic
      polypeptide"

<400> 77
Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Ser Ser Tyr
            20                  25                  30


Leu Asn Trp Tyr Gln Gln Lys Pro Gly Lys Ala Pro Lys Leu Leu Ile
        35                  40                  45


Ser Gly Ala Ser Ser Leu Lys Ser Gly Val Pro Ser Arg Phe Ser Gly
    50                  55                  60

Ser Gly Ser Gly Thr Asp Phe Thr Leu Thr Ile Ser Ser Leu Pro Pro
65                  70                  75                  80

Glu Asp Phe Ala Thr Tyr Tyr Cys Gln Gln Ser Tyr Ser Thr Pro Ile
                85                  90                  95

Thr Phe Gly Gln Gly Thr Arg Leu Glu Ile Lys Arg
            100                 105

<210> 78
<211> 11
<212> PRT
<213> Artificial Sequence

<220>
<221> source
<223> /note="Description of Artificial Sequence: Synthetic
      peptide"

<400> 78
Arg Ala Ser Gln Ser Ile Ser Ser Tyr Leu Asn
1               5                   10

<210> 79
<211> 7
<212> PRT
<213> Artificial Sequence

<220>
<221> source
<223> /note="Description of Artificial Sequence: Synthetic
      peptide"

<400> 79
Gly Ala Ser Ser Leu Lys Ser
1               5

<210> 80
<211> 9
<212> PRT
<213> Artificial Sequence

<220>
<221> source
<223> /note="Description of Artificial Sequence: Synthetic
      peptide"

<400> 80
Gln Gln Ser Tyr Ser Thr Pro Ile Thr
1               5

<210> 81
<211> 1437
<212> DNA
<213> Artificial Sequence

<220>

<221> source
<223> /note="Description of Artificial Sequence: Synthetic
      polynucleotide"

<400> 81
atggcactcc ccgtaactgc tctgctgctg ccgttggcat tgctcctgca cgccgcacgc      60

ccgcaggtcc aactggtgca gtccggagcc gaagtcaaga aaccaggtgc ctccgttaaa     120

gtgagttgca aagtctctgg atacactctg accgagctct ctatgcactg ggtccggcag     180

gcccccggca agggattgga atggatgggc gggttcgatc ctgaggacgg agagactatc     240

tacgctcaaa aattccaggg acgagtgact gtgaccgaag acactagtac cgacactgcc     300

tacatggaac tttcctctct gcgatcagaa gataccgcag tgtactactg tgctactgaa     360

tctaggggca ttggatggcc ctacttcgat tactggggtc agggaactct ggtgactgtc     420

tccagcggtg gaggtggcag cggtggtggc ggaagcgggg ggggcggctc tgatattcag     480

atgactcaat ctccttcttc tctgtccgct tccgtgggcg atagagtgac cattacttgt     540

agggcgtccc agtcaatctc cagttatttg aattggtatc agcagaagcc cgggaaagca     600

cctaagctgt tgatcagcgg ggcttctagc ctgaagagtg gggtaccttc acggttcagc     660

ggaagcggaa gcggaaccga tttcaccctg actatcagca gcctgccacc tgaggacttt     720

gcaacttact actgccaaca gtcatacagc actccgatca ctttcggcca gggcacccgg     780

ctcgaaatca agcgcgctgc tgctttggac aatgagaagt caaacggcac catcatacat     840

gttaaaggta aacatctgtg tccctccccg ctgttccccg gcccttccaa accgttctgg     900

gttctggtgg tggtcggagg cgtactcgct tgctatagtc tgctggtaac tgtcgccttc     960

atcatctttt gggtgagatc caaaagaagc cgcctgctcc atagcgatta catgaatatg    1020

actccacgcc gccctggccc cacaaggaaa cactaccagc cttacgcacc acctagagat    1080

ttcgctgcct atcggagcag ggtgaagttt tccagatctg cagatgcacc agcgtatcag    1140

cagggccaga accaactgta taacgagctc aacctgggac gcagggaaga gtatgacgtt    1200

ttggacaagc gcagaggacg ggaccctgag atgggtggca aaccaagacg aaaaaacccc    1260

caggagggtc tctataatga gctgcagaag gataagatgg ctgaagccta ttctgaaata    1320

ggcatgaaag gagagcggag aaggggaaaa gggcacgacg gtttgtacca gggactcagc    1380

actgctacga aggatactta tgacgctctc cacatgcaag ccctgccacc taggtaa       1437


<210> 82
<211> 478
<212> PRT
<213> Artificial Sequence

<220>
<221> source
<223> /note="Description of Artificial Sequence: Synthetic
      polypeptide"

<400> 82
Met Ala Leu Pro Val Thr Ala Leu Leu Leu Pro Leu Ala Leu Leu Leu
1               5                   10                  15

His Ala Ala Arg Pro Gln Val Gln Leu Val Gln Ser Gly Ala Glu Val
            20                  25                  30

Lys Lys Pro Gly Ala Ser Val Lys Val Ser Cys Lys Val Ser Gly Tyr
        35                  40                  45

Thr Leu Thr Glu Leu Ser Met His Trp Val Arg Gln Ala Pro Gly Lys
    50                  55                  60

Gly Leu Glu Trp Met Gly Gly Phe Asp Pro Glu Asp Gly Glu Thr Ile
65                  70                  75                  80

Tyr Ala Gln Lys Phe Gln Gly Arg Val Thr Val Thr Glu Asp Thr Ser
                85                  90                  95

Thr Asp Thr Ala Tyr Met Glu Leu Ser Ser Leu Arg Ser Glu Asp Thr
            100                 105                 110

Ala Val Tyr Tyr Cys Ala Thr Glu Ser Arg Gly Ile Gly Trp Pro Tyr
        115                 120                 125

Phe Asp Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asp Ile Gln
145                 150                 155                 160

Met Thr Gln Ser Pro Ser Ser Leu Ser Ala Ser Val Gly Asp Arg Val
                165                 170                 175

Thr Ile Thr Cys Arg Ala Ser Gln Ser Ile Ser Ser Tyr Leu Asn Trp
            180                 185                 190

Tyr Gln Gln Lys Pro Gly Lys Ala Pro Lys Leu Leu Ile Ser Gly Ala
        195                 200                 205

Ser Ser Leu Lys Ser Gly Val Pro Ser Arg Phe Ser Gly Ser Gly Ser
    210                 215                 220

Gly Thr Asp Phe Thr Leu Thr Ile Ser Ser Leu Pro Pro Glu Asp Phe
225                 230                 235                 240

Ala Thr Tyr Tyr Cys Gln Gln Ser Tyr Ser Thr Pro Ile Thr Phe Gly

245                 250                 255

Gln Gly Thr Arg Leu Glu Ile Lys Arg Ala Ala Ala Leu Asp Asn Glu
        260                 265                 270

Lys Ser Asn Gly Thr Ile Ile His Val Lys Gly Lys His Leu Cys Pro
    275                 280                 285

Ser Pro Leu Phe Pro Gly Pro Ser Lys Pro Phe Trp Val Leu Val Val
    290                 295                 300

Val Gly Gly Val Leu Ala Cys Tyr Ser Leu Leu Val Thr Val Ala Phe
305                 310                 315                 320

Ile Ile Phe Trp Val Arg Ser Lys Arg Ser Arg Leu Leu His Ser Asp
                325                 330                 335

Tyr Met Asn Met Thr Pro Arg Arg Pro Gly Pro Thr Arg Lys His Tyr
        340                 345                 350

Gln Pro Tyr Ala Pro Pro Arg Asp Phe Ala Ala Tyr Arg Ser Arg Val
    355                 360                 365

Lys Phe Ser Arg Ser Ala Asp Ala Pro Ala Tyr Gln Gln Gly Gln Asn
    370                 375                 380

Gln Leu Tyr Asn Glu Leu Asn Leu Gly Arg Arg Glu Glu Tyr Asp Val
385                 390                 395                 400

Leu Asp Lys Arg Arg Gly Arg Asp Pro Glu Met Gly Gly Lys Pro Arg
                405                 410                 415

Arg Lys Asn Pro Gln Glu Gly Leu Tyr Asn Glu Leu Gln Lys Asp Lys
        420                 425                 430

Met Ala Glu Ala Tyr Ser Glu Ile Gly Met Lys Gly Glu Arg Arg Arg
    435                 440                 445

Gly Lys Gly His Asp Gly Leu Tyr Gln Gly Leu Ser Thr Ala Thr Lys
    450                 455                 460

Asp Thr Tyr Asp Ala Leu His Met Gln Ala Leu Pro Pro Arg
465                 470                 475

<210> 83
<211> 1371
<212> DNA
<213> Artificial Sequence

<220>
<221> source
<223> /note="Description of Artificial Sequence: Synthetic
      polynucleotide"

<400> 83
caggtccaac tggtgcagtc cggagccgaa gtcaagaaac caggtgcctc cgttaaagtg      60

agttgcaaag tctctggata cactctgacc gagctctcta tgcactgggt ccggcaggcc     120

cccggcaagg gattggaatg gatgggcggg ttcgatcctg aggacggaga gactatctac     180

gctcaaaaat tccagggacg agtgactgtg accgaagaca ctagtaccga cactgcctac     240

atggaacttt cctctctgcg atcagaagat accgcagtgt actactgtgc tactgaatct     300

aggggcattg gatggcccta cttcgattac tggggtcagg gaactctggt gactgtctcc     360

agcggtggag gtggcagcgg tggtggcgga agcggggggg gcggctctga tattcagatg     420

actcaatctc cttcttctct gtccgcttcc gtgggcgata gagtgaccat tacttgtagg     480

gcgtcccagt caatctccag ttatttgaat tggtatcagc agaagcccgg gaaagcacct     540

aagctgttga tcagcggggc ttctagcctg aagagtgggg taccttcacg gttcagcgga     600

agcggaagcg gaaccgattt caccctgact atcagcagcc tgccacctga ggactttgca     660

acttactact gccaacagtc atacagcact ccgatcactt tcggccaggg cacccggctc     720

gaaatcaagc gcgctgctgc tttggacaat gagaagtcaa acggcaccat catacatgtt     780

aaaggtaaac atctgtgtcc ctccccgctg ttccccggcc cttccaaacc gttctgggtt     840

ctggtggtgg tcggaggcgt actcgcttgc tatagtctgc tggtaactgt cgccttcatc     900

atcttttggg tgagatccaa aagaagccgc ctgctccata gcgattacat gaatatgact     960

ccacgccgcc ctggccccac aaggaaacac taccagcctt acgcaccacc tagagatttc    1020

gctgcctatc ggagcagggt gaagttttcc agatctgcag atgcaccagc gtatcagcag    1080

ggccagaacc aactgtataa cgagctcaac ctgggacgca gggaagagta tgacgttttg    1140

gacaagcgca gaggacggga ccctgagatg ggtggcaaac caagacgaaa aaacccccag    1200

gagggtctct ataatgagct gcagaaggat aagatggctg aagcctattc tgaaataggc    1260

atgaaaggag agcggagaag gggaaaaggg cacgacggtt tgtaccaggg actcagcact    1320

gctacgaagg atacttatga cgctctccac atgcaagccc tgccacctag g             1371

<210> 84
<211> 457
<212> PRT
<213> Artificial Sequence

<220>
<221> source
<223> /note="Description of Artificial Sequence: Synthetic
      polypeptide"

<400> 84
Gln Val Gln Leu Val Gln Ser Gly Ala Glu Val Lys Lys Pro Gly Ala
1               5                   10                  15

Ser Val Lys Val Ser Cys Lys Val Ser Gly Tyr Thr Leu Thr Glu Leu
            20                  25                  30

Ser Met His Trp Val Arg Gln Ala Pro Gly Lys Gly Leu Glu Trp Met
        35                  40                  45

Gly Gly Phe Asp Pro Glu Asp Gly Glu Thr Ile Tyr Ala Gln Lys Phe
    50                  55                  60

Gln Gly Arg Val Thr Val Thr Glu Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Thr Glu Ser Arg Gly Ile Gly Trp Pro Tyr Phe Asp Tyr Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Asp Ile Gln Met Thr Gln Ser Pro
    130                 135                 140

Ser Ser Leu Ser Ala Ser Val Gly Asp Arg Val Thr Ile Thr Cys Arg
145                 150                 155                 160

Ala Ser Gln Ser Ile Ser Ser Tyr Leu Asn Trp Tyr Gln Gln Lys Pro
                165                 170                 175

Gly Lys Ala Pro Lys Leu Leu Ile Ser Gly Ala Ser Ser Leu Lys Ser
            180                 185                 190

Gly Val Pro Ser Arg Phe Ser Gly Ser Gly Ser Gly Thr Asp Phe Thr
        195                 200                 205

Leu Thr Ile Ser Ser Leu Pro Pro Glu Asp Phe Ala Thr Tyr Tyr Cys
    210                 215                 220

Gln Gln Ser Tyr Ser Thr Pro Ile Thr Phe Gly Gln Gly Thr Arg Leu
225                 230                 235                 240

Glu Ile Lys Arg Ala Ala Ala Leu Asp Asn Glu Lys Ser Asn Gly Thr

245                 250                 255

Ile Ile His Val Lys Gly Lys His Leu Cys Pro Ser Pro Leu Phe Pro
            260                 265                 270

Gly Pro Ser Lys Pro Phe Trp Val Leu Val Val Val Gly Gly Val Leu
        275                 280                 285

Ala Cys Tyr Ser Leu Leu Val Thr Val Ala Phe Ile Ile Phe Trp Val
    290                 295                 300

Arg Ser Lys Arg Ser Arg Leu Leu His Ser Asp Tyr Met Asn Met Thr
305                 310                 315                 320

Pro Arg Arg Pro Gly Pro Thr Arg Lys His Tyr Gln Pro Tyr Ala Pro
                325                 330                 335

Pro Arg Asp Phe Ala Ala Tyr Arg Ser Arg Val Lys Phe Ser Arg Ser
            340                 345                 350

Ala Asp Ala Pro Ala Tyr Gln Gln Gly Gln Asn Gln Leu Tyr Asn Glu
        355                 360                 365

Leu Asn Leu Gly Arg Arg Glu Glu Tyr Asp Val Leu Asp Lys Arg Arg
    370                 375                 380

Gly Arg Asp Pro Glu Met Gly Gly Lys Pro Arg Arg Lys Asn Pro Gln
385                 390                 395                 400

Glu Gly Leu Tyr Asn Glu Leu Gln Lys Asp Lys Met Ala Glu Ala Tyr
                405                 410                 415

Ser Glu Ile Gly Met Lys Gly Glu Arg Arg Arg Gly Lys Gly His Asp
            420                 425                 430

Gly Leu Tyr Gln Gly Leu Ser Thr Ala Thr Lys Asp Thr Tyr Asp Ala
        435                 440                 445

Leu His Met Gln Ala Leu Pro Pro Arg
    450                 455

<210> 85
<211> 1464
<212> DNA
<213> Artificial Sequence

<220>
<221> source
<223> /note="Description of Artificial Sequence: Synthetic

polynucleotide"

<400> 85
atggcactcc ccgtaactgc tctgctgctg ccgttggcat tgctcctgca cgccgcacgc      60

ccgcaggtgc agcttgtgca gagcggggcc gaggtgaaga agcccggggc cagcgtcaaa     120

gtgtcctgta aggtcagcgg ttacaccctc accgagctga gcatgcactg ggtacggcag     180

gctcccggca aaggtcttga gtggatgggt ggatttgatc cagaagatgg agagactatc     240

tacgcccaga agttccaggg ccgggtcacc gtaacagaag acacctcaac tgacaccgct     300

tacatggagc tgagttcact gcggtccgag gacacggccg tgtattattg tgccaccgag     360

agccgcggaa tcggatggcc ttacttcgac tactggggac agggtacact tgttacagta     420

tcatccgggg gtggcggctc tggtgggggc ggctccggag ggggtggatc agatatccaa     480

atgactcaaa gtccaagttc cctgtctgcc tcagtcggag atagagtcac cataacctgc     540

agggcaagtc agtccatctc ctcctatctg aactggtacc aacagaaacc tggaaaggcg     600

cctaagctcc tgatctccgg agcctcatct ttgaaatccg gtgtcccatc tcgcttcagt     660

ggctctggaa gcggtacaga ttttactttg accattagca gcctcccacc ggaagacttt     720

gctacatatt actgccagca gtcttactca accccaatca ccttcgggca aggcaccaga     780

ctcgaaataa aaagagcagc tgctatcgag gttatgtacc caccgccgta cttggataac     840

gaaaaaagca atgggaccat cattcatgtg aagggtaagc acctttgccc tagcccactg     900

tttcctggcc cgagtaaacc cttttgggta cttgtggtcg tcggcggcgt gctggcctgc     960

tactcactcc tggttaccgt cgcattcatc atcttttggg tgagatccaa aagaagccgc    1020

ctgctccata gcgattacat gaatatgact ccacgccgcc ctggccccac aaggaaacac    1080

taccagcctt acgcaccacc tagagatttc gctgcctatc ggagcagggt gaagttttcc    1140

agatctgcag atgcaccagc gtatcagcag ggccagaacc aactgtataa cgagctcaac    1200

ctgggacgca gggaagagta tgacgttttg gacaagcgca gaggacggga ccctgagatg    1260

ggtggcaaac caagacgaaa aaacccccag gagggtctct ataatgagct gcagaaggat    1320

aagatggctg aagcctattc tgaaataggc atgaaaggag agcggagaag gggaaaaggg    1380

cacgacggtt tgtaccaggg actcagcact gctacgaagg atacttatga cgctctccac    1440

atgcaagccc tgccacctag gtaa                                           1464

<210> 86
<211> 487
<212> PRT
<213> Artificial Sequence

<220>
<221> source
<223> /note="Description of Artificial Sequence: Synthetic
      polypeptide"

<400> 86
Met Ala Leu Pro Val Thr Ala Leu Leu Leu Pro Leu Ala Leu Leu Leu
1               5                   10                  15

His Ala Ala Arg Pro Gln Val Gln Leu Val Gln Ser Gly Ala Glu Val
            20                  25                  30

Lys Lys Pro Gly Ala Ser Val Lys Val Ser Cys Lys Val Ser Gly Tyr
        35                  40                  45

Thr Leu Thr Glu Leu Ser Met His Trp Val Arg Gln Ala Pro Gly Lys
    50                  55                  60

Gly Leu Glu Trp Met Gly Gly Phe Asp Pro Glu Asp Gly Glu Thr Ile
65                  70                  75                  80

Tyr Ala Gln Lys Phe Gln Gly Arg Val Thr Val Thr Glu Asp Thr Ser
                85                  90                  95

Thr Asp Thr Ala Tyr Met Glu Leu Ser Ser Leu Arg Ser Glu Asp Thr
            100                 105                 110

Ala Val Tyr Tyr Cys Ala Thr Glu Ser Arg Gly Ile Gly Trp Pro Tyr
        115                 120                 125

Phe Asp Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asp Ile Gln
145                 150                 155                 160

Met Thr Gln Ser Pro Ser Ser Leu Ser Ala Ser Val Gly Asp Arg Val
                165                 170                 175

Thr Ile Thr Cys Arg Ala Ser Gln Ser Ile Ser Ser Tyr Leu Asn Trp
            180                 185                 190

Tyr Gln Gln Lys Pro Gly Lys Ala Pro Lys Leu Leu Ile Ser Gly Ala
        195                 200                 205

Ser Ser Leu Lys Ser Gly Val Pro Ser Arg Phe Ser Gly Ser Gly Ser
    210                 215                 220

Gly Thr Asp Phe Thr Leu Thr Ile Ser Ser Leu Pro Pro Glu Asp Phe
225                 230                 235                 240

Ala Thr Tyr Tyr Cys Gln Gln Ser Tyr Ser Thr Pro Ile Thr Phe Gly

245                 250                 255

Gln Gly Thr Arg Leu Glu Ile Lys Arg Ala Ala Ala Ile Glu Val Met
            260                 265                 270

Tyr Pro Pro Pro Tyr Leu Asp Asn Glu Lys Ser Asn Gly Thr Ile Ile
        275                 280                 285

His Val Lys Gly Lys His Leu Cys Pro Ser Pro Leu Phe Pro Gly Pro
    290                 295                 300

Ser Lys Pro Phe Trp Val Leu Val Val Val Gly Gly Val Leu Ala Cys
305                 310                 315                 320

Tyr Ser Leu Leu Val Thr Val Ala Phe Ile Ile Phe Trp Val Arg Ser
                325                 330                 335

Lys Arg Ser Arg Leu Leu His Ser Asp Tyr Met Asn Met Thr Pro Arg
            340                 345                 350

Arg Pro Gly Pro Thr Arg Lys His Tyr Gln Pro Tyr Ala Pro Pro Arg
        355                 360                 365

Asp Phe Ala Ala Tyr Arg Ser Arg Val Lys Phe Ser Arg Ser Ala Asp
    370                 375                 380

Ala Pro Ala Tyr Gln Gln Gly Gln Asn Gln Leu Tyr Asn Glu Leu Asn
385                 390                 395                 400

Leu Gly Arg Arg Glu Glu Tyr Asp Val Leu Asp Lys Arg Arg Gly Arg
                405                 410                 415

Asp Pro Glu Met Gly Gly Lys Pro Arg Arg Lys Asn Pro Gln Glu Gly
            420                 425                 430

Leu Tyr Asn Glu Leu Gln Lys Asp Lys Met Ala Glu Ala Tyr Ser Glu
        435                 440                 445

Ile Gly Met Lys Gly Glu Arg Arg Arg Gly Lys Gly His Asp Gly Leu
    450                 455                 460

Tyr Gln Gly Leu Ser Thr Ala Thr Lys Asp Thr Tyr Asp Ala Leu His
465                 470                 475                 480

Met Gln Ala Leu Pro Pro Arg
                485

<210> 87
<211> 1398
<212> DNA
<213> Artificial Sequence

<220>
<221> source
<223> /note="Description of Artificial Sequence: Synthetic
      polynucleotide"

<400> 87
caggtgcagc ttgtgcagag cggggccgag gtgaagaagc ccggggccag cgtcaaagtg      60

tcctgtaagg tcagcggtta caccctcacc gagctgagca tgcactgggt acggcaggct     120

cccggcaaag gtcttgagtg gatgggtgga tttgatccag aagatggaga gactatctac     180

gcccagaagt tccagggccg ggtcaccgta acagaagaca cctcaactga caccgcttac     240

atggagctga gttcactgcg gtccgaggac acggccgtgt attattgtgc caccgagagc     300

cgcggaatcg gatggcctta cttcgactac tggggacagg gtacacttgt tacagtatca     360

tccgggggtg gcggctctgg tgggggcggc tccggagggg gtggatcaga tatccaaatg     420

actcaaagtc caagttccct gtctgcctca gtcggagata gagtcaccat aacctgcagg     480

gcaagtcagt ccatctcctc ctatctgaac tggtaccaac agaaacctgg aaaggcgcct     540

aagctcctga tctccggagc ctcatctttg aaatccggtg tcccatctcg cttcagtggc     600

tctggaagcg gtacagattt tactttgacc attagcagcc tcccaccgga agactttgct     660

acatattact gccagcagtc ttactcaacc ccaatcacct tcgggcaagg caccagactc     720

gaaataaaaa gagcagctgc tatcgaggtt atgtacccac cgccgtactt ggataacgaa     780

aaaagcaatg ggaccatcat tcatgtgaag ggtaagcacc tttgccctag cccactgttt     840

cctggcccga gtaaaccctt ttgggtactt gtggtcgtcg gcggcgtgct ggcctgctac     900

tcactcctgg ttaccgtcgc attcatcatc ttttgggtga gatccaaaag aagccgcctg     960

ctccatagcg attacatgaa tatgactcca cgccgccctg gccccacaag gaaacactac    1020

cagccttacg caccacctag agatttcgct gcctatcgga gcagggtgaa gttttccaga    1080

tctgcagatg caccagcgta tcagcagggc cagaaccaac tgtataacga gctcaacctg    1140

ggacgcaggg aagagtatga cgttttggac aagcgcagag gacgggaccc tgagatgggt    1200

ggcaaaccaa gacgaaaaaa cccccaggag ggtctctata atgagctgca gaaggataag    1260

atggctgaag cctattctga aataggcatg aaaggagagc ggagaagggg aaaagggcac    1320

gacggtttgt accagggact cagcactgct acgaaggata cttatgacgc tctccacatg    1380

caagccctgc cacctagg                                                  1398


<210> 88
<211> 466
<212> PRT

<213> Artificial Sequence

<220>
<221> source
<223> /note="Description of Artificial Sequence: Synthetic
      polypeptide"

<400> 88
Gln Val Gln Leu Val Gln Ser Gly Ala Glu Val Lys Lys Pro Gly Ala
1               5                   10                  15

Ser Val Lys Val Ser Cys Lys Val Ser Gly Tyr Thr Leu Thr Glu Leu
            20                  25                  30

Ser Met His Trp Val Arg Gln Ala Pro Gly Lys Gly Leu Glu Trp Met
        35                  40                  45

Gly Gly Phe Asp Pro Glu Asp Gly Glu Thr Ile Tyr Ala Gln Lys Phe
    50                  55                  60

Gln Gly Arg Val Thr Val Thr Glu Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Thr Glu Ser Arg Gly Ile Gly Trp Pro Tyr Phe Asp Tyr Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Asp Ile Gln Met Thr Gln Ser Pro
    130                 135                 140

Ser Ser Leu Ser Ala Ser Val Gly Asp Arg Val Thr Ile Thr Cys Arg
145                 150                 155                 160

Ala Ser Gln Ser Ile Ser Ser Tyr Leu Asn Trp Tyr Gln Gln Lys Pro
                165                 170                 175

Gly Lys Ala Pro Lys Leu Leu Ile Ser Gly Ala Ser Ser Leu Lys Ser
            180                 185                 190

Gly Val Pro Ser Arg Phe Ser Gly Ser Gly Ser Gly Thr Asp Phe Thr
        195                 200                 205

Leu Thr Ile Ser Ser Leu Pro Pro Glu Asp Phe Ala Thr Tyr Tyr Cys
    210                 215                 220

Gln Gln Ser Tyr Ser Thr Pro Ile Thr Phe Gly Gln Gly Thr Arg Leu
225                 230                 235                 240

Glu Ile Lys Arg Ala Ala Ala Ile Glu Val Met Tyr Pro Pro Pro Tyr
                245                 250                 255

Leu Asp Asn Glu Lys Ser Asn Gly Thr Ile Ile His Val Lys Gly Lys
            260                 265                 270

His Leu Cys Pro Ser Pro Leu Phe Pro Gly Pro Ser Lys Pro Phe Trp
        275                 280                 285

Val Leu Val Val Val Gly Gly Val Leu Ala Cys Tyr Ser Leu Leu Val
    290                 295                 300

Thr Val Ala Phe Ile Ile Phe Trp Val Arg Ser Lys Arg Ser Arg Leu
305                 310                 315                 320

Leu His Ser Asp Tyr Met Asn Met Thr Pro Arg Arg Pro Gly Pro Thr
                325                 330                 335

Arg Lys His Tyr Gln Pro Tyr Ala Pro Pro Arg Asp Phe Ala Ala Tyr
            340                 345                 350

Arg Ser Arg Val Lys Phe Ser Arg Ser Ala Asp Ala Pro Ala Tyr Gln
        355                 360                 365

Gln Gly Gln Asn Gln Leu Tyr Asn Glu Leu Asn Leu Gly Arg Arg Glu
    370                 375                 380

Glu Tyr Asp Val Leu Asp Lys Arg Arg Gly Arg Asp Pro Glu Met Gly
385                 390                 395                 400

Gly Lys Pro Arg Arg Lys Asn Pro Gln Glu Gly Leu Tyr Asn Glu Leu
                405                 410                 415

Gln Lys Asp Lys Met Ala Glu Ala Tyr Ser Glu Ile Gly Met Lys Gly
            420                 425                 430

Glu Arg Arg Arg Gly Lys Gly His Asp Gly Leu Tyr Gln Gly Leu Ser
        435                 440                 445

Thr Ala Thr Lys Asp Thr Tyr Asp Ala Leu His Met Gln Ala Leu Pro
    450                 455                 460

Pro Arg
465

<210> 89
<211> 1545
<212> DNA
<213> Artificial Sequence

<220>
<221> source
<223> /note="Description of Artificial Sequence: Synthetic
      polynucleotide"

<400> 89
atggcactcc ccgtaactgc tctgctgctg ccgttggcat tgctcctgca cgccgcacgc      60

ccgcaggtgc agttggtgca aagcggcgca gaagttaaga aacctggggc gtcagttaag     120

gtgtcttgca aagtatctgg ctataccctc actgagctgt ccatgcattg ggtaaggcag     180

gctcctggaa aggggctcga atggatggga ggatttgacc ctgaagacgg agagaccatc     240

tacgcccaga aattccaggg tagagtaaca gtgactgagg acactagcac tgacacagcg     300

tacatggagc tgagttctct gagaagtgag gacacagccg tttactactg cgctaccgag     360

tccagaggta ttggctggcc atacttcgac tattggggtc agggcaccct ggttacagtg     420

agttcaggag gcgggggctc tggggggggc ggttccggag gggggggctc agatatacag     480

atgacgcaga gtccatcaag tctctcagcc agcgtgggag atcgcgtgac tattacttgc     540

cgcgccagcc agagtattag ctcctatctg aattggtacc agcaaaagcc cgggaaggcc     600

cctaagcttc tgatttctgg cgcctcctct ttgaagtcag gtgtgccaag cagatttagc     660

gggtctggaa gtggcactga ctttacactt actatctcca gcctgccccc agaggatttt     720

gccacatatt actgtcagca aagctactct actccaatca ctttcggcca gggcacaaga     780

ttggagatta agagggctgc cgcactttca aattccatca tgtatttcag ccattttgtg     840

cctgtttttc ttccggccaa acctacaacc actcccgccc cacgcccacc tactcccgcc     900

cctaccattg cctcccagcc tctgtctctt agacctgagg cttgtagacc tgctgccggc     960

ggagccgtgc acactcgcgg tctggacttc gcctgcgaca tctatatctg ggcccctctg    1020

gccggcacct gcggcgttct ccttctctca ctcgtaatca cactctattg caatcacagg    1080

aacagatcca aaagaagccg cctgctccat agcgattaca tgaatatgac tccacgccgc    1140

cctggcccca caaggaaaca ctaccagcct tacgcaccac ctagagattt cgctgcctat    1200

cggagcaggg tgaagttttc cagatctgca gatgcaccag cgtatcagca gggccagaac    1260

caactgtata acgagctcaa cctgggacgc agggaagagt atgacgtttt ggacaagcgc    1320

agaggacggg accctgagat gggtggcaaa ccaagacgaa aaaaccccca ggagggtctc    1380

tataatgagc tgcagaagga taagatggct gaagcctatt ctgaaatagg catgaaagga    1440

gagcggagaa ggggaaaagg gcacgacggt ttgtaccagg gactcagcac tgctacgaag    1500

gatacttatg acgctctcca catgcaagcc ctgccaccta ggtaa                    1545

<210> 90
<211> 514
<212> PRT
<213> Artificial Sequence

<220>
<221> source
<223> /note="Description of Artificial Sequence: Synthetic
      polypeptide"

<400> 90
Met Ala Leu Pro Val Thr Ala Leu Leu Leu Pro Leu Ala Leu Leu Leu
1               5                   10                  15

His Ala Ala Arg Pro Gln Val Gln Leu Val Gln Ser Gly Ala Glu Val
            20                  25                  30

Lys Lys Pro Gly Ala Ser Val Lys Val Ser Cys Lys Val Ser Gly Tyr
        35                  40                  45

Thr Leu Thr Glu Leu Ser Met His Trp Val Arg Gln Ala Pro Gly Lys
    50                  55                  60

Gly Leu Glu Trp Met Gly Gly Phe Asp Pro Glu Asp Gly Glu Thr Ile
65                  70                  75                  80

Tyr Ala Gln Lys Phe Gln Gly Arg Val Thr Val Thr Glu Asp Thr Ser
                85                  90                  95

Thr Asp Thr Ala Tyr Met Glu Leu Ser Ser Leu Arg Ser Glu Asp Thr
            100                 105                 110

Ala Val Tyr Tyr Cys Ala Thr Glu Ser Arg Gly Ile Gly Trp Pro Tyr
        115                 120                 125

Phe Asp Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asp Ile Gln
145                 150                 155                 160

Met Thr Gln Ser Pro Ser Ser Leu Ser Ala Ser Val Gly Asp Arg Val
                165                 170                 175

Thr Ile Thr Cys Arg Ala Ser Gln Ser Ile Ser Ser Tyr Leu Asn Trp
            180                 185                 190

Tyr Gln Gln Lys Pro Gly Lys Ala Pro Lys Leu Leu Ile Ser Gly Ala

195                 200                 205

Ser Ser Leu Lys Ser Gly Val Pro Ser Arg Phe Ser Gly Ser Gly Ser
    210                 215                 220

Gly Thr Asp Phe Thr Leu Thr Ile Ser Ser Leu Pro Pro Glu Asp Phe
225                 230                 235                 240

Ala Thr Tyr Tyr Cys Gln Gln Ser Tyr Ser Thr Pro Ile Thr Phe Gly
                245                 250                 255

Gln Gly Thr Arg Leu Glu Ile Lys Arg Ala Ala Ala Leu Ser Asn Ser
            260                 265                 270

Ile Met Tyr Phe Ser His Phe Val Pro Val Phe Leu Pro Ala Lys Pro
        275                 280                 285

Thr Thr Thr Pro Ala Pro Arg Pro Pro Thr Pro Ala Pro Thr Ile Ala
    290                 295                 300

Ser Gln Pro Leu Ser Leu Arg Pro Glu Ala Cys Arg Pro Ala Ala Gly
305                 310                 315                 320

Gly Ala Val His Thr Arg Gly Leu Asp Phe Ala Cys Asp Ile Tyr Ile
                325                 330                 335

Trp Ala Pro Leu Ala Gly Thr Cys Gly Val Leu Leu Leu Ser Leu Val
            340                 345                 350

Ile Thr Leu Tyr Cys Asn His Arg Asn Arg Ser Lys Arg Ser Arg Leu
        355                 360                 365

Leu His Ser Asp Tyr Met Asn Met Thr Pro Arg Arg Pro Gly Pro Thr
    370                 375                 380

Arg Lys His Tyr Gln Pro Tyr Ala Pro Pro Arg Asp Phe Ala Ala Tyr
385                 390                 395                 400

Arg Ser Arg Val Lys Phe Ser Arg Ser Ala Asp Ala Pro Ala Tyr Gln
                405                 410                 415

Gln Gly Gln Asn Gln Leu Tyr Asn Glu Leu Asn Leu Gly Arg Arg Glu
            420                 425                 430

Glu Tyr Asp Val Leu Asp Lys Arg Arg Gly Arg Asp Pro Glu Met Gly
        435                 440                 445

Gly Lys Pro Arg Arg Lys Asn Pro Gln Glu Gly Leu Tyr Asn Glu Leu
    450                 455                 460

Gln Lys Asp Lys Met Ala Glu Ala Tyr Ser Glu Ile Gly Met Lys Gly
465                 470                 475                 480

Glu Arg Arg Arg Gly Lys Gly His Asp Gly Leu Tyr Gln Gly Leu Ser
                485                 490                 495

Thr Ala Thr Lys Asp Thr Tyr Asp Ala Leu His Met Gln Ala Leu Pro
            500                 505                 510

Pro Arg

<210> 91
<211> 1479
<212> DNA
<213> Artificial Sequence

<220>
<221> source
<223> /note="Description of Artificial Sequence: Synthetic
      polynucleotide"

<400> 91
caggtgcagt tggtgcaaag cggcgcagaa gttaagaaac ctggggcgtc agttaaggtg      60

tcttgcaaag tatctggcta taccctcact gagctgtcca tgcattgggt aaggcaggct     120

cctggaaagg ggctcgaatg gatgggagga tttgaccctg aagacggaga gaccatctac     180

gcccagaaat tccagggtag agtaacagtg actgaggaca ctagcactga cacagcgtac     240

atggagctga gttctctgag aagtgaggac acagccgttt actactgcgc taccgagtcc     300

agaggtattg gctggccata cttcgactat tggggtcagg gcaccctggt tacagtgagt     360

tcaggaggcg ggggctctgg ggggggcggt tccggagggg ggggctcaga tatacagatg     420

acgcagagtc catcaagtct ctcagccagc gtgggagatc gcgtgactat tacttgccgc     480

gccagccaga gtattagctc ctatctgaat tggtaccagc aaaagcccgg gaaggcccct     540

aagcttctga tttctggcgc ctcctctttg aagtcaggtg tgccaagcag atttagcggg     600

tctggaagtg gcactgactt tacacttact atctccagcc tgcccccaga ggattttgcc     660

acatattact gtcagcaaag ctactctact ccaatcactt tcggccaggg cacaagattg     720

gagattaaga gggctgccgc actttcaaat tccatcatgt atttcagcca ttttgtgcct     780

gtttttcttc cggccaaacc tacaaccact cccgccccac gcccacctac tcccgcccct     840

accattgcct cccagcctct gtctcttaga cctgaggctt gtagacctgc tgccggcgga     900

gccgtgcaca ctcgcggtct ggacttcgcc tgcgacatct atatctgggc ccctctggcc     960

ggcacctgcg gcgttctcct tctctcactc gtaatcacac tctattgcaa tcacaggaac 1020

agatccaaaa gaagccgcct gctccatagc gattacatga atatgactcc acgccgccct 1080

ggccccacaa ggaaacacta ccagccttac gcaccaccta gagatttcgc tgcctatcgg 1140

agcagggtga agttttccag atctgcagat gcaccagcgt atcagcaggg ccagaaccaa 1200

ctgtataacg agctcaacct gggacgcagg gaagagtatg acgttttgga caagcgcaga 1260

ggacgggacc ctgagatggg tggcaaacca agacgaaaaa acccccagga gggtctctat 1320

aatgagctgc agaaggataa gatggctgaa gcctattctg aaataggcat gaaaggagag 1380

cggagaaggg gaaaaggcca cgacggtttg taccagggac tcagcactgc tacgaaggat 1440

acttatgacg ctctccacat gcaagccctg ccacctagg 1479

<210> 92
<211> 493
<212> PRT
<213> Artificial Sequence

<220>
<221> source
<223> /note="Description of Artificial Sequence: Synthetic
      polypeptide"

<400> 92
Gln Val Gln Leu Val Gln Ser Gly Ala Glu Val Lys Lys Pro Gly Ala
1               5                   10                  15

Ser Val Lys Val Ser Cys Lys Val Ser Gly Tyr Thr Leu Thr Glu Leu
            20                  25                  30

Ser Met His Trp Val Arg Gln Ala Pro Gly Lys Gly Leu Glu Trp Met
        35                  40                  45

Gly Gly Phe Asp Pro Glu Asp Gly Glu Thr Ile Tyr Ala Gln Lys Phe
    50                  55                  60

Gln Gly Arg Val Thr Val Thr Glu Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Thr Glu Ser Arg Gly Ile Gly Trp Pro Tyr Phe Asp Tyr Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Asp Ile Gln Met Thr Gln Ser Pro

130                 135                 140

Ser Ser Leu Ser Ala Ser Val Gly Asp Arg Val Thr Ile Thr Cys Arg
145                 150                 155                 160

Ala Ser Gln Ser Ile Ser Ser Tyr Leu Asn Trp Tyr Gln Gln Lys Pro
                165                 170                 175

Gly Lys Ala Pro Lys Leu Leu Ile Ser Gly Ala Ser Ser Leu Lys Ser
            180                 185                 190

Gly Val Pro Ser Arg Phe Ser Gly Ser Gly Ser Gly Thr Asp Phe Thr
        195                 200                 205

Leu Thr Ile Ser Ser Leu Pro Pro Glu Asp Phe Ala Thr Tyr Tyr Cys
    210                 215                 220

Gln Gln Ser Tyr Ser Thr Pro Ile Thr Phe Gly Gln Gly Thr Arg Leu
225                 230                 235                 240

Glu Ile Lys Arg Ala Ala Ala Leu Ser Asn Ser Ile Met Tyr Phe Ser
                245                 250                 255

His Phe Val Pro Val Phe Leu Pro Ala Lys Pro Thr Thr Thr Pro Ala
            260                 265                 270

Pro Arg Pro Pro Thr Pro Ala Pro Thr Ile Ala Ser Gln Pro Leu Ser
        275                 280                 285

Leu Arg Pro Glu Ala Cys Arg Pro Ala Ala Gly Gly Ala Val His Thr
    290                 295                 300

Arg Gly Leu Asp Phe Ala Cys Asp Ile Tyr Ile Trp Ala Pro Leu Ala
305                 310                 315                 320

Gly Thr Cys Gly Val Leu Leu Leu Ser Leu Val Ile Thr Leu Tyr Cys
                325                 330                 335

Asn His Arg Asn Arg Ser Lys Arg Ser Arg Leu Leu His Ser Asp Tyr
            340                 345                 350

Met Asn Met Thr Pro Arg Arg Pro Gly Pro Thr Arg Lys His Tyr Gln
        355                 360                 365

Pro Tyr Ala Pro Pro Arg Asp Phe Ala Ala Tyr Arg Ser Arg Val Lys
    370                 375                 380

Phe Ser Arg Ser Ala Asp Ala Pro Ala Tyr Gln Gln Gly Gln Asn Gln
385                 390                 395                 400

Leu Tyr Asn Glu Leu Asn Leu Gly Arg Arg Glu Glu Tyr Asp Val Leu
                405                 410                 415

Asp Lys Arg Arg Gly Arg Asp Pro Glu Met Gly Gly Lys Pro Arg Arg
            420                 425                 430

Lys Asn Pro Gln Glu Gly Leu Tyr Asn Glu Leu Gln Lys Asp Lys Met
        435                 440                 445

Ala Glu Ala Tyr Ser Glu Ile Gly Met Lys Gly Glu Arg Arg Arg Gly
    450                 455                 460

Lys Gly His Asp Gly Leu Tyr Gln Gly Leu Ser Thr Ala Thr Lys Asp
465                 470                 475                 480

Thr Tyr Asp Ala Leu His Met Gln Ala Leu Pro Pro Arg
                485                 490

<210> 93
<211> 351
<212> DNA
<213> Artificial Sequence

<220>
<221> source
<223> /note="Description of Artificial Sequence: Synthetic
      polynucleotide"

<400> 93
caggtccagt tggtcgaaag tggcggtggt gtagtgcagc cgggccgcag tttgaggctt      60

tcctgtgcgg cttcaggctt tactttttcc agctatggaa tgcactgggt gcggcaggcc     120

cccggcaaag gacttgagtg ggtggccgtc atttcttatg acggatcaga taagtactac     180

gtggacagcg tcaagggcag attcaccatc tctagggaca acagtaaaaa tagactctac     240

ctccagatga atagcctcag agctgaagac acggccgtct actattgtgc tcgggagcgg     300

tatagtggca gagactactg ggggcagggc acactcgtta cagtgagtag c               351

<210> 94
<211> 117
<212> PRT
<213> Artificial Sequence

<220>
<221> source
<223> /note="Description of Artificial Sequence: Synthetic
      polypeptide"

<400> 94

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asp Lys Tyr Tyr Val Asp Ser Val
    50                  55                  60

Lys Gly Arg Phe Thr Ile Ser Arg Asp Asn Ser Lys Asn Arg Leu Tyr
65                  70                  75                  80

Leu Gln Met Asn Ser Leu Arg Ala Glu Asp Thr Ala Val Tyr Tyr Cys
                85                  90                  95

Ala Arg Glu Arg Tyr Ser Gly Arg Asp Tyr Trp Gly Gln Gly Thr Leu
            100                 105                 110

Val Thr Val Ser Ser
        115


<210> 95
<211> 7
<212> PRT
<213> Artificial Sequence

<220>
<221> source
<223> /note="Description of Artificial Sequence: Synthetic
      peptide"

<400> 95
Gly Phe Thr Phe Ser Ser Tyr
1               5


<210> 96
<211> 6
<212> PRT
<213> Artificial Sequence

<220>
<221> source
<223> /note="Description of Artificial Sequence: Synthetic
      peptide"

<400> 96
Ser Tyr Asp Gly Ser Asp
1               5


<210> 97

<211> 8
<212> PRT
<213> Artificial Sequence

<220>
<221> source
<223> /note="Description of Artificial Sequence: Synthetic
      peptide"

<400> 97
Glu Arg Tyr Ser Gly Arg Asp Tyr
1               5


<210> 98
<211> 324
<212> DNA
<213> Artificial Sequence

<220>
<221> source
<223> /note="Description of Artificial Sequence: Synthetic
      polynucleotide"

<400> 98
gagattgtta tgacccagag tcctgcgacc ctctcagtca gccccgggga gcgcgcaact        60

ttgtcttgca gagctagtca gtccgtgtcc tctcttctga catggtacca gcaaaagccc       120

gggcaggctc cgcgcctttt gatctttggg gcttcaacaa gagccactgg gattcccgca       180

cgattctctg gctccgggag cggtactggt ttcaccctga cgattagcag tctccagagc       240

gaggacttcg ccgtatacta ctgccagcag tacgatacgt ggccattcac ttttggacca       300

gggactaaag tggattttaa gcgc                                              324


<210> 99
<211> 108
<212> PRT
<213> Artificial Sequence

<220>
<221> source
<223> /note="Description of Artificial Sequence: Synthetic
      polypeptide"

<400> 99
Glu Ile Val Met Thr Gln Ser Pro Ala Thr Leu Ser Val Ser Pro Gly
1               5                   10                  15


Glu Arg Ala Thr Leu Ser Cys Arg Ala Ser Gln Ser Val Ser Ser Leu
            20                  25                  30


Leu Thr Trp Tyr Gln Gln Lys Pro Gly Gln Ala Pro Arg Leu Leu Ile
        35                  40                  45


Phe Gly Ala Ser Thr Arg Ala Thr Gly Ile Pro Ala Arg Phe Ser Gly
    50                  55                  60

Ser Gly Ser Gly Thr Gly Phe Thr Leu Thr Ile Ser Ser Leu Gln Ser
65                  70                  75                  80

Glu Asp Phe Ala Val Tyr Tyr Cys Gln Gln Tyr Asp Thr Trp Pro Phe
                85                  90                  95

Thr Phe Gly Pro Gly Thr Lys Val Asp Phe Lys Arg
            100                 105

<210> 100
<211> 11
<212> PRT
<213> Artificial Sequence

<220>
<221> source
<223> /note="Description of Artificial Sequence: Synthetic
      peptide"

<400> 100
Arg Ala Ser Gln Ser Val Ser Ser Leu Leu Thr
1               5                   10

<210> 101
<211> 7
<212> PRT
<213> Artificial Sequence

<220>
<221> source
<223> /note="Description of Artificial Sequence: Synthetic
      peptide"

<400> 101
Gly Ala Ser Thr Arg Ala Thr
1               5

<210> 102
<211> 9
<212> PRT
<213> Artificial Sequence

<220>
<221> source
<223> /note="Description of Artificial Sequence: Synthetic
      peptide"

<400> 102
Gln Gln Tyr Asp Thr Trp Pro Phe Thr
1               5

<210> 103
<211> 1425
<212> DNA
<213> Artificial Sequence

<220>
<221> source
<223> /note="Description of Artificial Sequence: Synthetic
      polynucleotide"

<400> 103
atggcactcc ccgtaactgc tctgctgctg ccgttggcat tgctcctgca cgccgcacgc      60

ccgcaggtcc agttggtcga aagtggcggt ggtgtagtgc agccgggccg cagtttgagg     120

ctttcctgtg cggcttcagg ctttactttt tccagctatg gaatgcactg ggtgcggcag     180

gcccccggca aaggacttga gtgggtggcc gtcatttctt atgacggatc agataagtac     240

tacgtggaca gcgtcaaggg cagattcacc atctctaggg acaacagtaa aaatagactc     300

tacctccaga tgaatagcct cagagctgaa gacacggccg tctactattg tgctcgggag     360

cggtatagtg gcagagacta ctgggggcag ggcacactcg ttacagtgag tagcggcgga     420

ggagggagtg ggggcggtgg ctccggtgga ggaggttctg agattgttat gacccagagt      480

cctgcgaccc tctcagtcag ccccggggag cgcgcaactt tgtcttgcag agctagtcag     540

tccgtgtcct ctcttctgac atggtaccag caaaagcccg ggcaggctcc gcgccttttg     600

atctttgggg cttcaacaag agccactggg attcccgcac gattctctgg ctccgggagc     660

ggtactggtt tcaccctgac gattagcagt ctccagagcg aggacttcgc cgtatactac     720

tgccagcagt acgatacgtg gccattcact tttggaccag ggactaaagt ggattttaag     780

cgcgccgccg ctctcgataa cgaaaagtca aatggcacca taatccacgt caaaggcaag     840

cacctgtgcc cttccccgct cttccccgga cccagtaaac cattttgggt gctggttgtt      900

gtggggggcg tgctggcctg ctatagcctt ttggtcactg tagccttcat tattttttgg       960

gtcagatcca aaagaagccg cctgctccat agcgattaca tgaatatgac tccacgccgc    1020

cctggcccca caaggaaaca ctaccagcct tacgcaccac ctagagattt cgctgcctat    1080

cggagcaggg tgaagttttc cagatctgca gatgcaccag cgtatcagca gggccagaac    1140

caactgtata acgagctcaa cctgggacgc agggaagagt atgacgtttt ggacaagcgc    1200

agaggacggg accctgagat gggtggcaaa ccaagacgaa aaaaccccca ggagggtctc    1260

tataatgagc tgcagaagga taagatggct gaagcctatt ctgaaatagg catgaaagga    1320

gagcggagaa ggggaaaagg gcacgacggt ttgtaccagg gactcagcac tgctacgaag    1380

gatacttatg acgctctcca catgcaagcc ctgccaccta ggtaa                    1425

<210> 104
<211> 474
<212> PRT
<213> Artificial Sequence

<220>
<221> source

<223> /note="Description of Artificial Sequence: Synthetic
      polypeptide"

<400> 104
Met Ala Leu Pro Val Thr Ala Leu Leu Leu Pro Leu Ala Leu Leu Leu
1               5                   10                  15

His Ala Ala Arg Pro Gln Val Gln Leu Val Glu Ser Gly Gly Gly Val
            20                  25                  30

Val Gln Pro Gly Arg Ser Leu Arg Leu Ser Cys Ala Ala Ser Gly Phe
        35                  40                  45

Thr Phe Ser Ser Tyr Gly Met His Trp Val Arg Gln Ala Pro Gly Lys
    50                  55                  60

Gly Leu Glu Trp Val Ala Val Ile Ser Tyr Asp Gly Ser Asp Lys Tyr
65                  70                  75                  80

Tyr Val Asp Ser Val Lys Gly Arg Phe Thr Ile Ser Arg Asp Asn Ser
                85                  90                  95

Lys Asn Arg Leu Tyr Leu Gln Met Asn Ser Leu Arg Ala Glu Asp Thr
            100                 105                 110

Ala Val Tyr Tyr Cys Ala Arg Glu Arg Tyr Ser Gly Arg Asp Tyr Trp
        115                 120                 125

Gly Gln Gly Thr Leu Val Thr Val Ser Ser Gly Gly Gly Gly Ser Gly
    130                 135                 140

Gly Gly Gly Ser Gly Gly Gly Gly Ser Glu Ile Val Met Thr Gln Ser
145                 150                 155                 160

Pro Ala Thr Leu Ser Val Ser Pro Gly Glu Arg Ala Thr Leu Ser Cys
                165                 170                 175

Arg Ala Ser Gln Ser Val Ser Ser Leu Leu Thr Trp Tyr Gln Gln Lys
            180                 185                 190

Pro Gly Gln Ala Pro Arg Leu Leu Ile Phe Gly Ala Ser Thr Arg Ala
        195                 200                 205

Thr Gly Ile Pro Ala Arg Phe Ser Gly Ser Gly Ser Gly Thr Gly Phe
    210                 215                 220

Thr Leu Thr Ile Ser Ser Leu Gln Ser Glu Asp Phe Ala Val Tyr Tyr
225                 230                 235                 240

Cys Gln Gln Tyr Asp Thr Trp Pro Phe Thr Phe Gly Pro Gly Thr Lys
                245                 250                 255

Val Asp Phe Lys Arg Ala Ala Ala Leu Asp Asn Glu Lys Ser Asn Gly
            260                 265                 270

Thr Ile Ile His Val Lys Gly Lys His Leu Cys Pro Ser Pro Leu Phe
        275                 280                 285

Pro Gly Pro Ser Lys Pro Phe Trp Val Leu Val Val Val Gly Gly Val
    290                 295                 300

Leu Ala Cys Tyr Ser Leu Leu Val Thr Val Ala Phe Ile Ile Phe Trp
305                 310                 315                 320

Val Arg Ser Lys Arg Ser Arg Leu Leu His Ser Asp Tyr Met Asn Met
                325                 330                 335

Thr Pro Arg Arg Pro Gly Pro Thr Arg Lys His Tyr Gln Pro Tyr Ala
            340                 345                 350

Pro Pro Arg Asp Phe Ala Ala Tyr Arg Ser Arg Val Lys Phe Ser Arg
        355                 360                 365

Ser Ala Asp Ala Pro Ala Tyr Gln Gln Gly Gln Asn Gln Leu Tyr Asn
    370                 375                 380

Glu Leu Asn Leu Gly Arg Arg Glu Glu Tyr Asp Val Leu Asp Lys Arg
385                 390                 395                 400

Arg Gly Arg Asp Pro Glu Met Gly Gly Lys Pro Arg Arg Lys Asn Pro
                405                 410                 415

Gln Glu Gly Leu Tyr Asn Glu Leu Gln Lys Asp Lys Met Ala Glu Ala
            420                 425                 430

Tyr Ser Glu Ile Gly Met Lys Gly Glu Arg Arg Arg Gly Lys Gly His
        435                 440                 445

Asp Gly Leu Tyr Gln Gly Leu Ser Thr Ala Thr Lys Asp Thr Tyr Asp
    450                 455                 460

Ala Leu His Met Gln Ala Leu Pro Pro Arg
465                 470

<210> 105
<211> 1359

<212> DNA
<213> Artificial Sequence

<220>
<221> source
<223> /note="Description of Artificial Sequence: Synthetic
      polynucleotide"

<400> 105
   caggtccagt tggtcgaaag tggcggtggt gtagtgcagc cgggccgcag tttgaggctt      60
   tcctgtgcgg cttcaggctt tactttttcc agctatggaa tgcactgggt gcggcaggcc      120
   cccggcaaag gacttgagtg ggtggccgtc atttcttatg acggatcaga taagtactac      180
   gtggacagcg tcaagggcag attcaccatc tctagggaca acagtaaaaa tagactctac      240
   ctccagatga atagcctcag agctgaagac acggccgtct actattgtgc tcgggagcgg      300
   tatagtggca gagactactg ggggcagggc acactcgtta cagtgagtag cggcggagga      360
   gggagtgggg gcggtggctc cggtggagga ggttctgaga ttgttatgac ccagagtcct      420
   gcgaccctct cagtcagccc cggggagcgc gcaactttgt cttgcagagc tagtcagtcc      480
   gtgtcctctc ttctgacatg gtaccagcaa aagcccgggc aggctccgcg ccttttgatc      540
   tttggggctt caacaagagc cactgggatt cccgcacgat tctctggctc cgggagcggt      600
   actggtttca ccctgacgat tagcagtctc cagagcgagg acttcgccgt atactactgc      660
   cagcagtacg atacgtggcc attcactttt ggaccaggga ctaaagtgga ttttaagcgc      720
   gccgccgctc tcgataacga aaagtcaaat ggcaccataa tccacgtcaa aggcaagcac      780
   ctgtgccctt ccccgctctt ccccggaccc agtaaaccat tttgggtgct ggttgttgtg      840
   gggggcgtgc tggcctgcta tagccttttg gtcactgtag ccttcattat tttttgggtc      900
   agatccaaaa gaagccgcct gctccatagc gattacatga atatgactcc acgccgccct      960
   ggccccacaa ggaaacacta ccagccttac gcaccaccta gagatttcgc tgcctatcgg      1020
   agcagggtga agttttccag atctgcagat gcaccagcgt atcagcaggg ccagaaccaa      1080
   ctgtataacg agctcaacct gggacgcagg gaagagtatg acgttttgga caagcgcaga      1140
   ggacgggacc ctgagatggg tggcaaacca agacgaaaaa acccccagga gggtctctat      1200
   aatgagctgc agaaggataa gatggctgaa gcctattctg aaataggcat gaaaggagag      1260
   cggagaaggg gaaaagggca cgacggtttg taccagggac tcagcactgc tacgaaggat      1320
   acttatgacg ctctccacat gcaagccctg ccacctagg                            1359

<210> 106
<211> 453
<212> PRT
<213> Artificial Sequence

<220>
<221> source

<223> /note="Description of Artificial Sequence: Synthetic
      polypeptide"

<400> 106
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asp Lys Tyr Tyr Val Asp Ser Val
    50                  55                  60

Lys Gly Arg Phe Thr Ile Ser Arg Asp Asn Ser Lys Asn Arg Leu Tyr
65                  70                  75                  80

Leu Gln Met Asn Ser Leu Arg Ala Glu Asp Thr Ala Val Tyr Tyr Cys
                85                  90                  95

Ala Arg Glu Arg Tyr Ser Gly Arg Asp Tyr Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Glu Ile Val Met Thr Gln Ser Pro Ala Thr Leu Ser
    130                 135                 140

Val Ser Pro Gly Glu Arg Ala Thr Leu Ser Cys Arg Ala Ser Gln Ser
145                 150                 155                 160

Val Ser Ser Leu Leu Thr Trp Tyr Gln Gln Lys Pro Gly Gln Ala Pro
                165                 170                 175

Arg Leu Leu Ile Phe Gly Ala Ser Thr Arg Ala Thr Gly Ile Pro Ala
            180                 185                 190

Arg Phe Ser Gly Ser Gly Ser Gly Thr Gly Phe Thr Leu Thr Ile Ser
        195                 200                 205

Ser Leu Gln Ser Glu Asp Phe Ala Val Tyr Tyr Cys Gln Gln Tyr Asp
    210                 215                 220

Thr Trp Pro Phe Thr Phe Gly Pro Gly Thr Lys Val Asp Phe Lys Arg
225                 230                 235                 240

Ala Ala Ala Leu Asp Asn Glu Lys Ser Asn Gly Thr Ile Ile His Val
                245                 250                 255

Lys Gly Lys His Leu Cys Pro Ser Pro Leu Phe Pro Gly Pro Ser Lys
            260                 265                 270

Pro Phe Trp Val Leu Val Val Val Gly Gly Val Leu Ala Cys Tyr Ser
        275                 280                 285

Leu Leu Val Thr Val Ala Phe Ile Ile Phe Trp Val Arg Ser Lys Arg
    290                 295                 300

Ser Arg Leu Leu His Ser Asp Tyr Met Asn Met Thr Pro Arg Arg Pro
305                 310                 315                 320

Gly Pro Thr Arg Lys His Tyr Gln Pro Tyr Ala Pro Pro Arg Asp Phe
                325                 330                 335

Ala Ala Tyr Arg Ser Arg Val Lys Phe Ser Arg Ser Ala Asp Ala Pro
            340                 345                 350

Ala Tyr Gln Gln Gly Gln Asn Gln Leu Tyr Asn Glu Leu Asn Leu Gly
        355                 360                 365

Arg Arg Glu Glu Tyr Asp Val Leu Asp Lys Arg Arg Gly Arg Asp Pro
    370                 375                 380

Glu Met Gly Gly Lys Pro Arg Arg Lys Asn Pro Gln Glu Gly Leu Tyr
385                 390                 395                 400

Asn Glu Leu Gln Lys Asp Lys Met Ala Glu Ala Tyr Ser Glu Ile Gly
                405                 410                 415

Met Lys Gly Glu Arg Arg Arg Gly Lys Gly His Asp Gly Leu Tyr Gln
            420                 425                 430

Gly Leu Ser Thr Ala Thr Lys Asp Thr Tyr Asp Ala Leu His Met Gln
        435                 440                 445

Ala Leu Pro Pro Arg
    450

<210> 107
<211> 1452
<212> DNA
<213> Artificial Sequence

<220>

<221> source
<223> /note="Description of Artificial Sequence: Synthetic
      polynucleotide"

<400> 107
atggcactcc ccgtaactgc tctgctgctg ccgttggcat tgctcctgca cgccgcacgc      60

ccgcaggtgc agctcgtgga gtctggcggc ggcgtggtcc agcccggccg gtccctgcgc     120

ctgtcctgcg ccgccagcgg gtttactttt tcctcctacg gcatgcactg ggtgcgccag     180

gctcccggca agggcctcga gtgggtcgcc gtgatctcat acgatgggtc agacaaatac     240

tatgtcgatt ctgttaaagg gcggtttacc atttcaagag ataactctaa gaataggctg     300

tatttgcaga tgaacagcct gagggctgaa gataccgcag tgtactattg cgctagggag     360

cggtatagtg gccgcgatta ctggggacag ggtacactgg tgaccgtgag ctctgggggt     420

ggcggaagcg ggggtggcgg aagcggcgga gggggtagtg aaattgtgat gacccagtct     480

ccggctacac tttcagtctc ccctggggag agagctacac tgtcatgcag agcgtcccag     540

tccgtctctt ctctccttac ctggtatcag cagaagcccg gccaggctcc tcgactgctg     600

atcttcggtg cctccacaag ggcgaccggg attccagccc gcttctcagg ttctgggagc     660

ggaactggtt tcactttgac aatcagttca ctgcagtcag aggatttcgc cgtgtactac     720

tgccagcaat acgacacatg gccattcact ttcggacccg gtaccaaagt cgatttcaag     780

agagccgcgg ccatcgaggt tatgtaccca ccaccatatc tggacaatga aaaaagcaat     840

ggaaccatta tccatgtgaa gggtaaacac ctctgcccta gcccactttt ccctggccca     900

tcaaagccct tctgggtctt ggtggtcgtg gggggtgtgc tggcctgtta cagccttctg     960

gtgacggttg ctttcattat cttctgggtt agatccaaaa gaagccgcct gctccatagc    1020

gattacatga atatgactcc acgccgccct ggccccacaa ggaaacacta ccagccttac    1080

gcaccaccta gagatttcgc tgcctatcgg agcagggtga agttttccag atctgcagat    1140

gcaccagcgt atcagcaggg ccagaaccaa ctgtataacg agctcaacct gggacgcagg    1200

gaagagtatg acgttttgga caagcgcaga ggacgggacc ctgagatggg tggcaaacca    1260

agacgaaaaa acccccagga gggtctctat aatgagctgc agaaggataa gatggctgaa    1320

gcctattctg aaataggcat gaaaggagag cggagaaggg gaaaagggca cgacggtttg    1380

taccagggac tcagcactgc tacgaaggat acttatgacg ctctccacat gcaagccctg    1440

ccacctaggt aa                                                        1452

<210> 108
<211> 483
<212> PRT
<213> Artificial Sequence

<220>
<221> source

<223> /note="Description of Artificial Sequence: Synthetic
      polypeptide"

<400> 108
Met Ala Leu Pro Val Thr Ala Leu Leu Leu Pro Leu Ala Leu Leu Leu
1               5                   10                  15

His Ala Ala Arg Pro Gln Val Gln Leu Val Glu Ser Gly Gly Gly Val
            20                  25                  30

Val Gln Pro Gly Arg Ser Leu Arg Leu Ser Cys Ala Ala Ser Gly Phe
        35                  40                  45

Thr Phe Ser Ser Tyr Gly Met His Trp Val Arg Gln Ala Pro Gly Lys
    50                  55                  60

Gly Leu Glu Trp Val Ala Val Ile Ser Tyr Asp Gly Ser Asp Lys Tyr
65                  70                  75                  80

Tyr Val Asp Ser Val Lys Gly Arg Phe Thr Ile Ser Arg Asp Asn Ser
                85                  90                  95

Lys Asn Arg Leu Tyr Leu Gln Met Asn Ser Leu Arg Ala Glu Asp Thr
            100                 105                 110

Ala Val Tyr Tyr Cys Ala Arg Glu Arg Tyr Ser Gly Arg Asp Tyr Trp
        115                 120                 125

Gly Gln Gly Thr Leu Val Thr Val Ser Ser Gly Gly Gly Gly Ser Gly
    130                 135                 140

Gly Gly Gly Ser Gly Gly Gly Gly Ser Glu Ile Val Met Thr Gln Ser
145                 150                 155                 160

Pro Ala Thr Leu Ser Val Ser Pro Gly Glu Arg Ala Thr Leu Ser Cys
                165                 170                 175

Arg Ala Ser Gln Ser Val Ser Ser Leu Leu Thr Trp Tyr Gln Gln Lys
            180                 185                 190

Pro Gly Gln Ala Pro Arg Leu Leu Ile Phe Gly Ala Ser Thr Arg Ala
        195                 200                 205

Thr Gly Ile Pro Ala Arg Phe Ser Gly Ser Gly Ser Gly Thr Gly Phe
    210                 215                 220

Thr Leu Thr Ile Ser Ser Leu Gln Ser Glu Asp Phe Ala Val Tyr Tyr
225                 230                 235                 240

Cys Gln Gln Tyr Asp Thr Trp Pro Phe Thr Phe Gly Pro Gly Thr Lys
                245                 250                 255

Val Asp Phe Lys Arg Ala Ala Ala Ile Glu Val Met Tyr Pro Pro Pro
            260                 265                 270

Tyr Leu Asp Asn Glu Lys Ser Asn Gly Thr Ile Ile His Val Lys Gly
        275                 280                 285

Lys His Leu Cys Pro Ser Pro Leu Phe Pro Gly Pro Ser Lys Pro Phe
    290                 295                 300

Trp Val Leu Val Val Val Gly Gly Val Leu Ala Cys Tyr Ser Leu Leu
305                 310                 315                 320

Val Thr Val Ala Phe Ile Ile Phe Trp Val Arg Ser Lys Arg Ser Arg
                325                 330                 335

Leu Leu His Ser Asp Tyr Met Asn Met Thr Pro Arg Arg Pro Gly Pro
            340                 345                 350

Thr Arg Lys His Tyr Gln Pro Tyr Ala Pro Pro Arg Asp Phe Ala Ala
        355                 360                 365

Tyr Arg Ser Arg Val Lys Phe Ser Arg Ser Ala Asp Ala Pro Ala Tyr
    370                 375                 380

Gln Gln Gly Gln Asn Gln Leu Tyr Asn Glu Leu Asn Leu Gly Arg Arg
385                 390                 395                 400

Glu Glu Tyr Asp Val Leu Asp Lys Arg Arg Gly Arg Asp Pro Glu Met
                405                 410                 415

Gly Gly Lys Pro Arg Arg Lys Asn Pro Gln Glu Gly Leu Tyr Asn Glu
            420                 425                 430

Leu Gln Lys Asp Lys Met Ala Glu Ala Tyr Ser Glu Ile Gly Met Lys
        435                 440                 445

Gly Glu Arg Arg Arg Gly Lys Gly His Asp Gly Leu Tyr Gln Gly Leu
    450                 455                 460

Ser Thr Ala Thr Lys Asp Thr Tyr Asp Ala Leu His Met Gln Ala Leu
465                 470                 475                 480

Pro Pro Arg

<210> 109
<211> 1386
<212> DNA
<213> Artificial Sequence

<220>
<221> source
<223> /note="Description of Artificial Sequence: Synthetic
      polynucleotide"

<400> 109
caggtgcagc tcgtggagtc tggcggcggc gtggtccagc ccggccggtc cctgcgcctg      60

tcctgcgccg ccagcgggtt tactttttcc tcctacggca tgcactgggt gcgccaggct     120

cccggcaagg gcctcgagtg ggtcgccgtg atctcatacg atgggtcaga caaatactat     180

gtcgattctg ttaaagggcg gtttaccatt tcaagagata actctaagaa taggctgtat     240

ttgcagatga acagcctgag ggctgaagat accgcagtgt actattgcgc tagggagcgg     300

tatagtggcc gcgattactg gggacagggt acactggtga ccgtgagctc tgggggtggc     360

ggaagcgggg gtggcggaag cggcggaggg ggtagtgaaa ttgtgatgac ccagtctccg     420

gctacacttt cagtctcccc tggggagaga gctacactgt catgcagagc gtcccagtcc     480

gtctcttctc tccttacctg gtatcagcag aagcccggcc aggctcctcg actgctgatc     540

ttcggtgcct ccacaagggc gaccgggatt ccagcccgct tctcaggttc tgggagcgga     600

actggtttca ctttgacaat cagttcactg cagtcagagg atttcgccgt gtactactgc     660

cagcaatacg acacatggcc attcactttc ggacccggta ccaaagtcga tttcaagaga     720

gccgcggcca tcgaggttat gtacccacca ccatatctgg acaatgaaaa aagcaatgga     780

accattatcc atgtgaaggg taaacacctc tgccctagcc cacttttccc tggcccatca     840

aagcccttct gggtcttggt ggtcgtgggg ggtgtgctgg cctgttacag ccttctggtg     900

acggttgctt tcattatctt ctgggttaga tccaaaagaa gccgcctgct ccatagcgat     960

tacatgaata tgactccacg ccgccctggc cccacaagga aacactacca gccttacgca    1020

ccacctagag atttcgctgc ctatcggagc agggtgaagt tttccagatc tgcagatgca    1080

ccagcgtatc agcagggcca gaaccaactg tataacgagc tcaacctggg acgcagggaa    1140

gagtatgacg ttttggacaa gcgcagagga cgggaccctg agatgggtgg caaaccaaga    1200

cgaaaaaacc cccaggaggg tctctataat gagctgcaga aggataagat ggctgaagcc    1260

tattctgaaa taggcatgaa aggagagcgg agaaggggaa aagggcacga cggtttgtac    1320

cagggactca gcactgctac gaaggatact tatgacgctc tccacatgca agccctgcca    1380

cctagg                                                                1386

<210> 110

<211> 462
<212> PRT
<213> Artificial Sequence

<220>
<221> source
<223> /note="Description of Artificial Sequence: Synthetic
      polypeptide"

<400> 110
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asp Lys Tyr Tyr Val Asp Ser Val
    50                  55                  60

Lys Gly Arg Phe Thr Ile Ser Arg Asp Asn Ser Lys Asn Arg Leu Tyr
65                  70                  75                  80

Leu Gln Met Asn Ser Leu Arg Ala Glu Asp Thr Ala Val Tyr Tyr Cys
                85                  90                  95

Ala Arg Glu Arg Tyr Ser Gly Arg Asp Tyr Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Glu Ile Val Met Thr Gln Ser Pro Ala Thr Leu Ser
    130                 135                 140

Val Ser Pro Gly Glu Arg Ala Thr Leu Ser Cys Arg Ala Ser Gln Ser
145                 150                 155                 160

Val Ser Ser Leu Leu Thr Trp Tyr Gln Gln Lys Pro Gly Gln Ala Pro
                165                 170                 175

Arg Leu Leu Ile Phe Gly Ala Ser Thr Arg Ala Thr Gly Ile Pro Ala
            180                 185                 190

Arg Phe Ser Gly Ser Gly Ser Gly Thr Gly Phe Thr Leu Thr Ile Ser
        195                 200                 205

Ser Leu Gln Ser Glu Asp Phe Ala Val Tyr Tyr Cys Gln Gln Tyr Asp

210                 215                 220

Thr Trp Pro Phe Thr Phe Gly Pro Gly Thr Lys Val Asp Phe Lys Arg
225                 230                 235                 240

Ala Ala Ala Ile Glu Val Met Tyr Pro Pro Pro Tyr Leu Asp Asn Glu
                245                 250                 255

Lys Ser Asn Gly Thr Ile Ile His Val Lys Gly Lys His Leu Cys Pro
            260                 265                 270

Ser Pro Leu Phe Pro Gly Pro Ser Lys Pro Phe Trp Val Leu Val Val
        275                 280                 285

Val Gly Gly Val Leu Ala Cys Tyr Ser Leu Leu Val Thr Val Ala Phe
    290                 295                 300

Ile Ile Phe Trp Val Arg Ser Lys Arg Ser Arg Leu Leu His Ser Asp
305                 310                 315                 320

Tyr Met Asn Met Thr Pro Arg Arg Pro Gly Pro Thr Arg Lys His Tyr
                325                 330                 335

Gln Pro Tyr Ala Pro Pro Arg Asp Phe Ala Ala Tyr Arg Ser Arg Val
            340                 345                 350

Lys Phe Ser Arg Ser Ala Asp Ala Pro Ala Tyr Gln Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460

<210> 111
<211> 1533
<212> DNA
<213> Artificial Sequence

<220>
<221> source
<223> /note="Description of Artificial Sequence: Synthetic
      polynucleotide"

<400> 111
atggcactcc ccgtaactgc tctgctgctg ccgttggcat tgctcctgca cgccgcacgc      60

ccgcaggtgc agttggttga atcaggaggg ggtgtggtgc aacccggtcg gtcactgcgc     120

ctcagttgtg ctgcttccgg gtttactttc agctcatatg ggatgcactg ggtacggcag     180

gctccaggta aaggcttgga atgggtggcg gtgatcagct atgacggctc tgacaaatat     240

tatgtggact ccgtgaaagg cagattcacc atcagtcgag acaactcaaa gaatagactc     300

tacttgcaga tgaatagcct ccgggccgaa gatactgcag tctattattg cgcccgggag     360

cgctacagtg gaagagacta ttgggggcaa ggaactcttg tcacagtctc atctggcggc     420

ggcggcagcg gtgggggcgg atctggcggg ggcggcagcg aaatcgttat gactcagagt     480

cctgccacac tgagcgttag ccctggtgag agagcaacac ttagctgcag agctagtcag     540

agtgtttcca gtcttttgac atggtaccaa cagaagcccg gtcaagctcc acgactgctc     600

atcttcggtg catccacccg cgcaaccggg atacccgccc ggttttccgg ttctggaagt     660

ggcacaggat tcacgctcac catttcttct ctgcagtctg aagactttgc cgtgtattac     720

tgccagcagt acgatacctg gccctttacc tttggcccag gtactaaagt ggattttaaa     780

cgagctgctg cactttccaa tagtattatg tacttttcac atttgtgccc cgtgttcctg     840

cctgcgaagc ctacgacaac cccagcccct aggccgccca caccggcccc aactattgcc     900

tcccagccat tgtctctgag acccgaagct tgcagacctg ctgctggagg cgccgttcac     960

acccgaggat tggatttcgc atgtgacatt tacatctggg cccctttggc cggaacctgc    1020

ggtgtgctgc tgctgtcact cgtgattaca ctttactgca accaccgaaa cagatccaaa    1080

agaagccgcc tgctccatag cgattacatg aatatgactc cacgccgccc tggccccaca    1140

aggaaacact accagcctta cgcaccacct agagatttcg ctgcctatcg gagcagggtg    1200

aagttttcca gatctgcaga tgcaccagcg tatcagcagg gccagaacca actgtataac    1260

gagctcaacc tgggacgcag ggaagagtat gacgttttgg acaagcgcag aggacgggac    1320

cctgagatgg gtggcaaacc aagacgaaaa aacccccagg agggtctcta taatgagctg    1380

cagaaggata agatggctga agcctattct gaaataggca tgaaaggaga gcggagaagg    1440

ggaaaagggc acgacggttt gtaccaggga ctcagcactg ctacgaagga tacttatgac    1500

gctctccaca tgcaagccct gccacctagg taa                                 1533

<210> 112
<211> 509
<212> PRT
<213> Artificial Sequence

<220>
<221> source
<223> /note="Description of Artificial Sequence: Synthetic
      polypeptide"

<400> 112
Met Ala Leu Pro Val Thr Ala Leu Leu Leu Pro Leu Ala Leu Leu Leu
1               5                   10                  15

His Ala Ala Arg Pro Gln Val Gln Leu Val Glu Ser Gly Gly Gly Val
            20                  25                  30

Val Gln Pro Gly Arg Ser Leu Arg Leu Ser Cys Ala Ala Ser Gly Phe
        35                  40                  45

Thr Phe Ser Ser Tyr Gly Met His Trp Val Arg Gln Ala Pro Gly Lys
    50                  55                  60

Gly Leu Glu Trp Val Ala Val Ile Ser Tyr Asp Gly Ser Asp Lys Tyr
65                  70                  75                  80

Tyr Val Asp Ser Val Lys Gly Arg Phe Thr Ile Ser Arg Asp Asn Ser
                85                  90                  95

Lys Asn Arg Leu Tyr Leu Gln Met Asn Ser Leu Arg Ala Glu Asp Thr
            100                 105                 110

Ala Val Tyr Tyr Cys Ala Arg Glu Arg Tyr Ser Gly Arg Asp Tyr Trp
        115                 120                 125

Gly Gly Thr Leu Val Thr Val Ser Ser Gly Gly Gly Gly Ser Gly Gly
    130                 135                 140

Gly Gly Ser Gly Gly Gly Gly Ser Glu Ile Val Met Thr Gln Ser Pro
145                 150                 155                 160

Ala Thr Leu Ser Val Ser Pro Gly Glu Arg Ala Thr Leu Ser Cys Arg
                165                 170                 175

Ala Ser Gln Ser Val Ser Ser Leu Leu Thr Trp Tyr Gln Gln Lys Pro
            180                 185                 190

Gly Gln Ala Pro Arg Leu Leu Ile Phe Gly Ala Ser Thr Arg Ala Thr
        195                 200                 205

Gly Ile Pro Ala Arg Phe Ser Gly Ser Gly Ser Gly Thr Gly Phe Thr
    210                 215                 220

Leu Thr Ile Ser Ser Leu Gln Ser Glu Asp Phe Ala Val Tyr Tyr Cys
225                 230                 235                 240

Gln Gln Tyr Asp Thr Trp Pro Phe Thr Phe Gly Pro Gly Thr Lys Val
                245                 250                 255

Asp Phe Lys Arg Ala Ala Ala Leu Ser Asn Ser Ile Met Tyr Phe Ser
            260                 265                 270

His Phe Val Pro Val Phe Leu Pro Ala Lys Pro Thr Thr Thr Pro Ala
        275                 280                 285

Pro Arg Pro Pro Thr Pro Ala Pro Thr Ile Ala Ser Gln Pro Leu Ser
    290                 295                 300

Leu Arg Pro Glu Ala Cys Arg Pro Ala Ala Gly Gly Ala Val His Thr
305                 310                 315                 320

Arg Gly Leu Asp Phe Ala Cys Asp Ile Tyr Ile Trp Ala Pro Leu Ala
                325                 330                 335

Gly Thr Cys Gly Val Leu Leu Leu Ser Leu Val Ile Thr Leu Tyr Cys
            340                 345                 350

Asn His Arg Asn Arg Ser Lys Arg Ser Arg Leu Leu His Ser Asp Tyr
        355                 360                 365

Met Asn Met Thr Pro Arg Arg Pro Gly Pro Thr Arg Lys His Tyr Gln
    370                 375                 380

Pro Tyr Ala Pro Pro Arg Asp Phe Ala Ala Tyr Arg Ser Arg Val Lys
385                 390                 395                 400

Phe Ser Arg Ser Ala Asp Ala Pro Ala Tyr Gln Gln Gly Gln Asn Gln
                405                 410                 415

Leu Tyr Asn Glu Leu Asn Leu Gly Arg Arg Glu Glu Tyr Asp Val Leu
            420                 425                 430

Asp Lys Arg Arg Gly Arg Asp Pro Glu Met Gly Gly Lys Pro Arg Arg
        435                 440                 445

Lys Asn Pro Gln Glu Gly Leu Tyr Asn Glu Leu Gln Lys Asp Lys Met
    450                 455                 460

Ala Glu Ala Tyr Ser Glu Ile Gly Met Lys Gly Glu Arg Arg Arg Gly
465                 470                 475                 480

Lys Gly His Asp Gly Leu Tyr Gln Gly Leu Ser Thr Ala Thr Lys Asp
                485                 490                 495

Thr Tyr Asp Ala Leu His Met Gln Ala Leu Pro Pro Arg
            500                 505

<210> 113
<211> 1467
<212> DNA
<213> Artificial Sequence

<220>
<221> source
<223> /note="Description of Artificial Sequence: Synthetic
      polynucleotide"

<400> 113
caggtgcagt tggttgaatc aggagggggt gtggtgcaac ccggtcggtc actgcgcctc      60

agttgtgctg cttccgggtt tactttcagc tcatatggga tgcactgggt acggcaggct     120

ccaggtaaag gcttggaatg ggtggcggtg atcagctatg acggctctga caaatattat     180

gtggactccg tgaaaggcag attcaccatc agtcgagaca actcaaagaa tagactctac     240

ttgcagatga atagcctccg ggccgaagat actgcagtct attattgcgc ccgggagcgc     300

tacagtggaa gagactattg ggggcaagga actcttgtca cagtctcatc tggcggcggc     360

ggcagcggtg ggggcggatc tggcgggggc ggcagcgaaa tcgttatgac tcagagtcct     420

gccacactga gcgttagccc tggtgagaga gcaacactta gctgcagagc tagtcagagt     480

gtttccagtc ttttgacatg gtaccaacag aagcccggtc aagctccacg actgctcatc     540

ttcggtgcat ccacccgcgc aaccgggata cccgcccggt tttccggttc tggaagtggc     600

acaggattca cgctcaccat ttcttctctg cagtctgaag actttgccgt gtattactgc     660

cagcagtacg atacctggcc ctttaccttt ggcccaggta ctaaagtgga ttttaaacga     720

gctgctgcac tttccaatag tattatgtac ttttcacatt ttgtgcccgt gttcctgcct     780

gcgaagccta cgacaacccc agcccctagg ccgcccacac cggccccaac tattgcctcc     840

cagccattgt ctctgagacc cgaagcttgc agacctgctg ctggaggcgc cgttcacacc     900

cgaggattgg atttcgcatg tgacatttac atctgggccc ctttggccgg aacctgcggt     960

gtgctgctgc tgtcactcgt gattacactt tactgcaacc accgaaacag atccaaaaga    1020

agccgcctgc tccatagcga ttacatgaat atgactccac gccgccctgg ccccacaagg    1080

aaacactacc agccttacgc accacctaga gatttcgctg cctatcggag cagggtgaag    1140

ttttccagat ctgcagatgc accagcgtat cagcagggcc agaaccaact gtataacgag 1200

ctcaacctgg gacgcaggga agagtatgac gttttggaca agcgcagagg acgggaccct 1260

gagatgggtg gcaaaccaag acgaaaaaac ccccaggagg gtctctataa tgagctgcag 1320

aaggataaga tggctgaagc ctattctgaa ataggcatga aaggagagcg gagaagggga 1380

aaagggcacg acggtttgta ccagggactc agcactgcta cgaaggatac ttatgacgct 1440

ctccacatgc aagccctgcc acctagg 1467

<210> 114
<211> 489
<212> PRT
<213> Artificial Sequence

<220>
<221> source
<223> /note="Description of Artificial Sequence: Synthetic
      polypeptide"

<400> 114
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asp Lys Tyr Tyr Val Asp Ser Val
    50                  55                  60

Lys Gly Arg Phe Thr Ile Ser Arg Asp Asn Ser Lys Asn Arg Leu Tyr
65                  70                  75                  80

Leu Gln Met Asn Ser Leu Arg Ala Glu Asp Thr Ala Val Tyr Tyr Cys
                85                  90                  95

Ala Arg Glu Arg Tyr Ser Gly Arg Asp Tyr Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Glu Ile Val Met Thr Gln Ser Pro Ala Thr Leu Ser
    130                 135                 140

Val Ser Pro Gly Glu Arg Ala Thr Leu Ser Cys Arg Ala Ser Gln Ser
145                 150                 155                 160

Val Ser Ser Leu Leu Thr Trp Tyr Gln Gln Lys Pro Gly Gln Ala Pro
                165                 170                 175

Arg Leu Leu Ile Phe Gly Ala Ser Thr Arg Ala Thr Gly Ile Pro Ala
            180                 185                 190

Arg Phe Ser Gly Ser Gly Ser Gly Thr Gly Phe Thr Leu Thr Ile Ser
        195                 200                 205

Ser Leu Gln Ser Glu Asp Phe Ala Val Tyr Tyr Cys Gln Gln Tyr Asp
    210                 215                 220

Thr Trp Pro Phe Thr Phe Gly Pro Gly Thr Lys Val Asp Phe Lys Arg
225                 230                 235                 240

Ala Ala Ala Leu Ser Asn Ser Ile Met Tyr Phe Ser His Phe Val Pro
                245                 250                 255

Val Phe Leu Pro Ala Lys Pro Thr Thr Thr Pro Ala Pro Arg Pro Pro
            260                 265                 270

Thr Pro Ala Pro Thr Ile Ala Ser Gln Pro Leu Ser Leu Arg Pro Glu
        275                 280                 285

Ala Cys Arg Pro Ala Ala Gly Gly Ala Val His Thr Arg Gly Leu Asp
    290                 295                 300

Phe Ala Cys Asp Ile Tyr Ile Trp Ala Pro Leu Ala Gly Thr Cys Gly
305                 310                 315                 320

Val Leu Leu Leu Ser Leu Val Ile Thr Leu Tyr Cys Asn His Arg Asn
                325                 330                 335

Arg Ser Lys Arg Ser Arg Leu Leu His Ser Asp Tyr Met Asn Met Thr
            340                 345                 350

Pro Arg Arg Pro Gly Pro Thr Arg Lys His Tyr Gln Pro Tyr Ala Pro
        355                 360                 365

Pro Arg Asp Phe Ala Ala Tyr Arg Ser Arg Val Lys Phe Ser Arg Ser
    370                 375                 380

Ala Asp Ala Pro Ala Tyr Gln Gln Gly Gln Asn Gln Leu Tyr Asn Glu
385                 390                 395                 400

Leu Asn Leu Gly Arg Arg Glu Glu Tyr Asp Val Leu Asp Lys Arg Arg
                405                 410                 415

Gly Arg Asp Pro Glu Met Gly Gly Lys Pro Arg Arg Lys Asn Pro Gln
            420                 425                 430

Glu Gly Leu Tyr Asn Glu Leu Gln Lys Asp Lys Met Ala Glu Ala Tyr
        435                 440                 445

Ser Glu Ile Gly Met Lys Gly Glu Arg Arg Arg Gly Lys Gly His Asp
    450                 455                 460

Gly Leu Tyr Gln Gly Leu Ser Thr Ala Thr Lys Asp Thr Tyr Asp Ala
465                 470                 475                 480

Leu His Met Gln Ala Leu Pro Pro Arg
                485

<210> 115
<211> 1425
<212> DNA
<213> Artificial Sequence

<220>
<221> source
<223> /note="Description of Artificial Sequence: Synthetic
      polynucleotide"

<400> 115
atggcactcc ccgtaactgc tctgctgctg ccgttggcat tgctcctgca cgccgcacgc      60

ccggagattg tgatgaccca gtcccctgct accctgtccg tcagtccggg cgagagagcc     120

accttgtcat gccgggccag ccagtccgtc agcagtctcc tgacttggta tcagcaaaaa     180

ccagggcagg caccgcggct tttgattttt ggtgcaagca cacgcgccac tggcattcca     240

gctaggtttt ctggaagtgg atctgggaca ggcttcactc tgacaatcag tagcctgcag     300

agtgaggact ttgctgttta ctactgtcaa cagtacgaca cctggccatt cacattcggg     360

cccggcacca aggtcgactt caagaggggc ggtggaggtt caggtggtgg cgggtcaggc     420

ggcggtgggt ctcaggttca actggtggaa tcaggtggcg gcgttgtcca accggggcga     480

tcacttcgac tttcctgtgc tgcctcaggc tttacttttt catcctatgg gatgcactgg     540

gttcggcagg ctcccggaaa aggactcgag tgggttgcag tgatctctta cgatggctca     600

gacaagtatt atgtggactc agtcaagggg agattcacaa taagccgaga caactccaaa     660

aaccggcttt atctccagat gaacagcctt agagcggaag ataccgcggt atactactgt     720

gcccgcgaga ggtattccgg cagagactac tggggacagg gcacactggt caccgtgagt     780

tctgccgcag cgctcgataa cgaaaagagc aacggaacca ttatccacgt taagggcaag     840

cacctgtgcc ccagtcccct cttcccagga ccatctaaac ccttctgggt tctggtagta     900

gttggagggg tccttgcatg ttactccctt ttggtcaccg tcgccttcat tattttctgg 960

gtgagatcca aaagaagccg cctgctccat agcgattaca tgaatatgac tccacgccgc 1020

cctggcccca caaggaaaca ctaccagcct tacgcaccac ctagagattt cgctgcctat 1080

cggagcaggg tgaagttttc cagatctgca gatgcaccag cgtatcagca gggccagaac 1140

caactgtata acgagctcaa cctgggacgc agggaagagt atgacgtttt ggacaagcgc 1200

agaggacggg accctgagat gggtggcaaa ccaagacgaa aaaaccccca ggagggtctc 1260

tataatgagc tgcagaagga taagatggct gaagcctatt ctgaaatagg catgaaagga 1320

gagcggagaa ggggaaaagg gcacgacggt ttgtaccagg gactcagcac tgctacgaag 1380

gatacttatg acgctctcca catgcaagcc ctgccaccta ggtaa 1425

<210> 116
<211> 474
<212> PRT
<213> Artificial Sequence

<220>
<221> source
<223> /note="Description of Artificial Sequence: Synthetic
      polypeptide"

<400> 116
Met Ala Leu Pro Val Thr Ala Leu Leu Leu Pro Leu Ala Leu Leu Leu
1               5                   10                  15

His Ala Ala Arg Pro Glu Ile Val Met Thr Gln Ser Pro Ala Thr Leu
            20                  25                  30

Ser Val Ser Pro Gly Glu Arg Ala Thr Leu Ser Cys Arg Ala Ser Gln
        35                  40                  45

Ser Val Ser Ser Leu Leu Thr Trp Tyr Gln Gln Lys Pro Gly Gln Ala
    50                  55                  60

Pro Arg Leu Leu Ile Phe Gly Ala Ser Thr Arg Ala Thr Gly Ile Pro
65                  70                  75                  80

Ala Arg Phe Ser Gly Ser Gly Ser Gly Thr Gly Phe Thr Leu Thr Ile
                85                  90                  95

Ser Ser Leu Gln Ser Glu Asp Phe Ala Val Tyr Tyr Cys Gln Gln Tyr
            100                 105                 110

Asp Thr Trp Pro Phe Thr Phe Gly Pro Gly Thr Lys Val Asp Phe Lys
        115                 120                 125

Arg Gly Gly Gly Gly Ser Gly Gly Gly Gly Ser Gly Gly Gly Gly Ser

130                 135                 140

Gln Val Gln Leu Val Glu Ser Gly Gly Gly Val Val Gln Pro Gly Arg
145                 150                 155                 160

Ser Leu Arg Leu Ser Cys Ala Ala Ser Gly Phe Thr Phe Ser Ser Tyr
                165                 170                 175

Gly Met His Trp Val Arg Gln Ala Pro Gly Lys Gly Leu Glu Trp Val
            180                 185                 190

Ala Val Ile Ser Tyr Asp Gly Ser Asp Lys Tyr Tyr Val Asp Ser Val
        195                 200                 205

Lys Gly Arg Phe Thr Ile Ser Arg Asp Asn Ser Lys Asn Arg Leu Tyr
    210                 215                 220

Leu Gln Met Asn Ser Leu Arg Ala Glu Asp Thr Ala Val Tyr Tyr Cys
225                 230                 235                 240

Ala Arg Glu Arg Tyr Ser Gly Arg Asp Tyr Trp Gly Gln Gly Thr Leu
                245                 250                 255

Val Thr Val Ser Ser Ala Ala Ala Leu Asp Asn Glu Lys Ser Asn Gly
            260                 265                 270

Thr Ile Ile His Val Lys Gly Lys His Leu Cys Pro Ser Pro Leu Phe
        275                 280                 285

Pro Gly Pro Ser Lys Pro Phe Trp Val Leu Val Val Val Gly Gly Val
    290                 295                 300

Leu Ala Cys Tyr Ser Leu Leu Val Thr Val Ala Phe Ile Ile Phe Trp
305                 310                 315                 320

Val Arg Ser Lys Arg Ser Arg Leu Leu His Ser Asp Tyr Met Asn Met
                325                 330                 335

Thr Pro Arg Arg Pro Gly Pro Thr Arg Lys His Tyr Gln Pro Tyr Ala
            340                 345                 350

Pro Pro Arg Asp Phe Ala Ala Tyr Arg Ser Arg Val Lys Phe Ser Arg
        355                 360                 365

Ser Ala Asp Ala Pro Ala Tyr Gln Gln Gly Gln Asn Gln Leu Tyr Asn
    370                 375                 380

Glu Leu Asn Leu Gly Arg Arg Glu Glu Tyr Asp Val Leu Asp Lys Arg
385                 390                 395                 400

Arg Gly Arg Asp Pro Glu Met Gly Gly Lys Pro Arg Arg Lys Asn Pro
                405                 410                 415

Gln Glu Gly Leu Tyr Asn Glu Leu Gln Lys Asp Lys Met Ala Glu Ala
            420                 425                 430

Tyr Ser Glu Ile Gly Met Lys Gly Glu Arg Arg Arg Gly Lys Gly His
        435                 440                 445

Asp Gly Leu Tyr Gln Gly Leu Ser Thr Ala Thr Lys Asp Thr Tyr Asp
    450                 455                 460

Ala Leu His Met Gln Ala Leu Pro Pro Arg
465                 470

<210> 117
<211> 1359
<212> DNA
<213> Artificial Sequence

<220>
<221> source
<223> /note="Description of Artificial Sequence: Synthetic
      polynucleotide"

<400> 117
gagattgtga tgacccagtc ccctgctacc ctgtccgtca gtccgggcga gagagccacc      60

ttgtcatgcc gggccagcca gtccgtcagc agtctcctga cttggtatca gcaaaaacca     120

gggcaggcac cgcggctttt gatttttggt gcaagcacac gcgccactgg cattccagct     180

aggttttctg gaagtggatc tgggacaggc ttcactctga caatcagtag cctgcagagt     240

gaggactttg ctgtttacta ctgtcaacag tacgacacct ggccattcac attcgggccc     300

ggcaccaagg tcgacttcaa gaggggcggt ggaggttcag gtggtggcgg gtcaggcggc     360

ggtgggtctc aggttcaact ggtggaatca ggtggcggcg ttgtccaacc ggggcgatca     420

cttcgacttt cctgtgctgc ctcaggcttt actttttcat cctatgggat gcactgggtt     480

cggcaggctc ccggaaaagg actcgagtgg gttgcagtga tctcttacga tggctcagac     540

aagtattatg tggactcagt caaggggaga ttcacaataa gccgagacaa ctccaaaaac     600

cggctttatc tccagatgaa cagccttaga gcggaagata ccgcggtata ctactgtgcc     660

cgcgagaggt attccggcag agactactgg ggacagggca cactggtcac cgtgagttct     720

gccgcagcgc tcgataacga aaagagcaac ggaaccatta tccacgttaa gggcaagcac     780

ctgtgcccca gtcccctctt cccaggacca tctaaaccct tctgggttct ggtagtagtt     840

ggaggggtcc ttgcatgtta ctcccttttg gtcaccgtcg ccttcattat tttctgggtg 900

agatccaaaa gaagccgcct gctccatagc gattacatga atatgactcc acgccgccct 960

ggccccacaa ggaaacacta ccagccttac gcaccaccta gagatttcgc tgcctatcgg 1020

agcagggtga agttttccag atctgcagat gcaccagcgt atcagcaggg ccagaaccaa 1080

ctgtataacg agctcaacct gggacgcagg gaagagtatg acgttttgga caagcgcaga 1140

ggacgggacc ctgagatggg tggcaaacca agacgaaaaa acccccagga gggtctctat 1200

aatgagctgc agaaggataa gatggctgaa gcctattctg aaataggcat gaaaggagag 1260

cggagaaggg gaaaagggca cgacggtttg taccagggac tcagcactgc tacgaaggat 1320

acttatgacg ctctccacat gcaagccctg ccacctagg 1359

<210> 118
<211> 453
<212> PRT
<213> Artificial Sequence

<220>
<221> source
<223> /note="Description of Artificial Sequence: Synthetic
      polypeptide"

<400> 118
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Leu
            20                  25                  30

Leu Thr Trp Tyr Gln Gln Lys Pro Gly Gln Ala Pro Arg Leu Leu Ile
        35                  40                  45

Phe Gly Ala Ser Thr Arg Ala Thr Gly Ile Pro Ala Arg Phe Ser Gly
    50                  55                  60

Ser Gly Ser Gly Thr Gly Phe Thr Leu Thr Ile Ser Ser Leu Gln Ser
65                  70                  75                  80

Glu Asp Phe Ala Val Tyr Tyr Cys Gln Gln Tyr Asp Thr Trp Pro Phe
                85                  90                  95

Thr Phe Gly Pro Gly Thr Lys Val Asp Phe Lys Arg Gly Gly Gly Gly
            100                 105                 110

Ser Gly Gly Gly Gly Ser Gly Gly Gly Gly Ser Gln Val Gln Leu Val
        115                 120                 125

Glu Ser Gly Gly Gly Val Val Gln Pro Gly Arg Ser Leu Arg Leu Ser

130                 135                 140

Cys Ala Ala Ser Gly Phe Thr Phe Ser Ser Tyr Gly Met His Trp Val
145                 150                 155                 160

Arg Gln Ala Pro Gly Lys Gly Leu Glu Trp Val Ala Val Ile Ser Tyr
                165                 170                 175

Asp Gly Ser Asp Lys Tyr Tyr Val Asp Ser Val Lys Gly Arg Phe Thr
            180                 185                 190

Ile Ser Arg Asp Asn Ser Lys Asn Arg Leu Tyr Leu Gln Met Asn Ser
        195                 200                 205

Leu Arg Ala Glu Asp Thr Ala Val Tyr Tyr Cys Ala Arg Glu Arg Tyr
    210                 215                 220

Ser Gly Arg Asp Tyr Trp Gly Gln Gly Thr Leu Val Thr Val Ser Ser
225                 230                 235                 240

Ala Ala Ala Leu Asp Asn Glu Lys Ser Asn Gly Thr Ile Ile His Val
                245                 250                 255

Lys Gly Lys His Leu Cys Pro Ser Pro Leu Phe Pro Gly Pro Ser Lys
            260                 265                 270

Pro Phe Trp Val Leu Val Val Val Gly Gly Val Leu Ala Cys Tyr Ser
        275                 280                 285

Leu Leu Val Thr Val Ala Phe Ile Ile Phe Trp Val Arg Ser Lys Arg
    290                 295                 300

Ser Arg Leu Leu His Ser Asp Tyr Met Asn Met Thr Pro Arg Arg Pro
305                 310                 315                 320

Gly Pro Thr Arg Lys His Tyr Gln Pro Tyr Ala Pro Pro Arg Asp Phe
                325                 330                 335

Ala Ala Tyr Arg Ser Arg Val Lys Phe Ser Arg Ser Ala Asp Ala Pro
            340                 345                 350

Ala Tyr Gln Gln Gly Gln Asn Gln Leu Tyr Asn Glu Leu Asn Leu Gly
        355                 360                 365

Arg Arg Glu Glu Tyr Asp Val Leu Asp Lys Arg Arg Gly Arg Asp Pro
    370                 375                 380

Glu Met Gly Gly Lys Pro Arg Arg Lys Asn Pro Gln Glu Gly Leu Tyr
385                 390                 395                 400

Asn Glu Leu Gln Lys Asp Lys Met Ala Glu Ala Tyr Ser Glu Ile Gly
                405                 410                 415

Met Lys Gly Glu Arg Arg Arg Gly Lys Gly His Asp Gly Leu Tyr Gln
            420                 425                 430

Gly Leu Ser Thr Ala Thr Lys Asp Thr Tyr Asp Ala Leu His Met Gln
        435                 440                 445

Ala Leu Pro Pro Arg
    450

<210> 119
<211> 1452
<212> DNA
<213> Artificial Sequence

<220>
<221> source
<223> /note="Description of Artificial Sequence: Synthetic
      polynucleotide"

<400> 119
atggcactcc ccgtaactgc tctgctgctg ccgttggcat tgctcctgca cgccgcacgc      60

ccggagatcg tcatgacaca gagtccagct accctgagcg tgtcccctgg agagagagcc     120

accctgtcct gtagggctag tcagagtgtg tccagcctcc tcacctggta tcaacagaag     180

cctggtcaag ctccccggct gcttatcttc ggggccagca cgcgagccac aggcatcccg     240

gccagattct ctggctctgg cagtggcacc gggttcactc tcacgatctc atccctgcag     300

tcagaggatt tcgctgtgta ttactgtcag cagtacgata catggccctt caccttcggc     360

ccgggcacaa aagtagattt caagcgcggc ggcgggggta gtgggggcgg gggatcagga     420

ggagggggct cccaagtaca gctggttgag agcggcggcg gggtggttca gcccgggcgc     480

agcctcaggc tgagttgcgc agcatcagga ttcacattca gttcttatgg aatgcattgg     540

gtcagacagg ctcccgggaa gggccttgaa tgggtggcag tcattagcta cgacggaagc     600

gataagtact atgtggactc agttaaaggg agatttacta tcagccgcga caattccaaa     660

aacagattgt atttgcagat gaactccctc agggcggagg acactgctgt atattactgc     720

gcacgagaga gatactccgg ccgagactat tggggccaag gaacattggt aactgtgagc     780

tccgccgcag ctattgaggt catgtacccc ccaccttatc tcgataatga gaagagtaat     840

gggactataa ttcacgtaaa gggcaaacac ctgtgccctt ccccgctgtt tccaggtcca     900

agtaagccgt tctgggtcct ggttgtggtg ggaggggtgc tggcctgcta ttctctgttg     960

gttaccgtgg cctttatcat tttctgggtg agatccaaaa gaagccgcct gctccatagc 1020

gattacatga atatgactcc acgccgccct ggccccacaa ggaaacacta ccagccttac 1080

gcaccaccta gagatttcgc tgcctatcgg agcagggtga agttttccag atctgcagat 1140

gcaccagcgt atcagcaggg ccagaaccaa ctgtataacg agctcaacct gggacgcagg 1200

gaagagtatg acgttttgga caagcgcaga ggacgggacc ctgagatggg tggcaaacca 1260

agacgaaaaa acccccagga gggtctctat aatgagctgc agaaggataa gatggctgaa 1320

gcctattctg aaataggcat gaaaggagag cggagaaggg gaaaagggca cgacggtttg 1380

taccagggac tcagcactgc tacgaaggat acttatgacg ctctccacat gcaagccctg 1440

ccacctaggt aa 1452

<210> 120
<211> 483
<212> PRT
<213> Artificial Sequence

<220>
<221> source
<223> /note="Description of Artificial Sequence: Synthetic
      polypeptide"

<400> 120
Met Ala Leu Pro Val Thr Ala Leu Leu Leu Pro Leu Ala Leu Leu Leu
1               5                   10                  15

His Ala Ala Arg Pro Glu Ile Val Met Thr Gln Ser Pro Ala Thr Leu
            20                  25                  30

Ser Val Ser Pro Gly Glu Arg Ala Thr Leu Ser Cys Arg Ala Ser Gln
        35                  40                  45

Ser Val Ser Ser Leu Leu Thr Trp Tyr Gln Gln Lys Pro Gly Gln Ala
    50                  55                  60

Pro Arg Leu Leu Ile Phe Gly Ala Ser Thr Arg Ala Thr Gly Ile Pro
65                  70                  75                  80

Ala Arg Phe Ser Gly Ser Gly Ser Gly Thr Gly Phe Thr Leu Thr Ile
                85                  90                  95

Ser Ser Leu Gln Ser Glu Asp Phe Ala Val Tyr Tyr Cys Gln Gln Tyr
            100                 105                 110

Asp Thr Trp Pro Phe Thr Phe Gly Pro Gly Thr Lys Val Asp Phe Lys
        115                 120                 125

Arg Gly Gly Gly Gly Ser Gly Gly Gly Gly Ser Gly Gly Gly Gly Ser

130                 135                 140

Gln Val Gln Leu Val Glu Ser Gly Gly Gly Val Val Gln Pro Gly Arg
145                 150                 155                 160

Ser Leu Arg Leu Ser Cys Ala Ala Ser Gly Phe Thr Phe Ser Ser Tyr
                165                 170                 175

Gly Met His Trp Val Arg Gln Ala Pro Gly Lys Gly Leu Glu Trp Val
            180                 185                 190

Ala Val Ile Ser Tyr Asp Gly Ser Asp Lys Tyr Tyr Val Asp Ser Val
        195                 200                 205

Lys Gly Arg Phe Thr Ile Ser Arg Asp Asn Ser Lys Asn Arg Leu Tyr
    210                 215                 220

Leu Gln Met Asn Ser Leu Arg Ala Glu Asp Thr Ala Val Tyr Tyr Cys
225                 230                 235                 240

Ala Arg Glu Arg Tyr Ser Gly Arg Asp Tyr Trp Gly Gln Gly Thr Leu
                245                 250                 255

Val Thr Val Ser Ser Ala Ala Ala Ile Glu Val Met Tyr Pro Pro Pro
            260                 265                 270

Tyr Leu Asp Asn Glu Lys Ser Asn Gly Thr Ile Ile His Val Lys Gly
        275                 280                 285

Lys His Leu Cys Pro Ser Pro Leu Phe Pro Gly Pro Ser Lys Pro Phe
    290                 295                 300

Trp Val Leu Val Val Val Gly Gly Val Leu Ala Cys Tyr Ser Leu Leu
305                 310                 315                 320

Val Thr Val Ala Phe Ile Ile Phe Trp Val Arg Ser Lys Arg Ser Arg
                325                 330                 335

Leu Leu His Ser Asp Tyr Met Asn Met Thr Pro Arg Arg Pro Gly Pro
            340                 345                 350

Thr Arg Lys His Tyr Gln Pro Tyr Ala Pro Pro Arg Asp Phe Ala Ala
        355                 360                 365

Tyr Arg Ser Arg Val Lys Phe Ser Arg Ser Ala Asp Ala Pro Ala Tyr
    370                 375                 380

Gln Gln Gly Gln Asn Gln Leu Tyr Asn Glu Leu Asn Leu Gly Arg Arg
385                 390                 395                 400

Glu Glu Tyr Asp Val Leu Asp Lys Arg Arg Gly Arg Asp Pro Glu Met
                405                 410                 415

Gly Gly Lys Pro Arg Arg Lys Asn Pro Gln Glu Gly Leu Tyr Asn Glu
            420                 425                 430

Leu Gln Lys Asp Lys Met Ala Glu Ala Tyr Ser Glu Ile Gly Met Lys
        435                 440                 445

Gly Glu Arg Arg Arg Gly Lys Gly His Asp Gly Leu Tyr Gln Gly Leu
    450                 455                 460

Ser Thr Ala Thr Lys Asp Thr Tyr Asp Ala Leu His Met Gln Ala Leu
465                 470                 475                 480

Pro Pro Arg

<210> 121
<211> 1386
<212> DNA
<213> Artificial Sequence

<220>
<221> source
<223> /note="Description of Artificial Sequence: Synthetic
      polynucleotide"

<400> 121
gagatcgtca tgacacagag tccagctacc ctgagcgtgt cccctggaga gagagccacc      60

ctgtcctgta gggctagtca gagtgtgtcc agcctcctca cctggtatca acagaagcct     120

ggtcaagctc cccggctgct tatcttcggg gccagcacgc gagccacagg catcccggcc     180

agattctctg gctctggcag tggcaccggg ttcactctca cgatctcatc cctgcagtca     240

gaggatttcg ctgtgtatta ctgtcagcag tacgatacat ggcccttcac cttcggcccg     300

ggcacaaaag tagatttcaa gcgcggcggc gggggtagtg ggggcggggg atcaggagga     360

gggggctccc aagtacagct ggttgagagc ggcggcgggg tggttcagcc cgggcgcagc     420

ctcaggctga gttgcgcagc atcaggattc acattcagtt cttatggaat gcattgggtc     480

agacaggctc ccgggaaggg ccttgaatgg gtggcagtca ttagctacga cggaagcgat     540

aagtactatg tggactcagt taaaggagaa tttactatca gccgcgacaa ttccaaaaac     600

agattgtatt tgcagatgaa ctccctcagg gcggaggaca ctgctgtata ttactgcgca     660

cgagagagat actccggccg agactattgg ggccaaggaa cattggtaac tgtgagctcc     720

gccgcagcta ttgaggtcat gtacccccca ccttatctcg ataatgagaa gagtaatggg     780

actataattc acgtaaaggg caaacacctg tgcccttccc cgctgtttcc aggtccaagt     840

aagccgttct gggtcctggt tgtggtggga ggggtgctgg cctgctattc tctgttggtt     900

accgtggcct ttatcatttt ctgggtgaga tccaaaagaa gccgcctgct ccatagcgat     960

tacatgaata tgactccacg ccgccctggc cccacaagga aacactacca gccttacgca    1020

ccacctagag atttcgctgc ctatcggagc agggtgaagt tttccagatc tgcagatgca    1080

ccagcgtatc agcagggcca gaaccaactg tataacgagc tcaacctggg acgcagggaa    1140

gagtatgacg ttttggacaa gcgcagagga cgggaccctg agatgggtgg caaaccaaga    1200

cgaaaaaacc cccaggaggg tctctataat gagctgcaga aggataagat ggctgaagcc    1260

tattctgaaa taggcatgaa aggagagcgg agaaggggaa aaggcacgga cggtttgtac    1320

cagggactca gcactgctac gaaggatact tatgacgctc tccacatgca agccctgcca    1380

cctagg                                                                1386

<210> 122
<211> 462
<212> PRT
<213> Artificial Sequence

<220>
<221> source
<223> /note="Description of Artificial Sequence: Synthetic
      polypeptide"

<400> 122
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Leu
            20                  25                  30

Leu Thr Trp Tyr Gln Gln Lys Pro Gly Gln Ala Pro Arg Leu Leu Ile
        35                  40                  45

Phe Gly Ala Ser Thr Arg Ala Thr Gly Ile Pro Ala Arg Phe Ser Gly
    50                  55                  60

Ser Gly Ser Gly Thr Gly Phe Thr Leu Thr Ile Ser Ser Leu Gln Ser
65                  70                  75                  80

Glu Asp Phe Ala Val Tyr Tyr Cys Gln Gln Tyr Asp Thr Trp Pro Phe
                85                  90                  95

Thr Phe Gly Pro Gly Thr Lys Val Asp Phe Lys Arg Gly Gly Gly Gly
            100                 105                 110

Ser Gly Gly Gly Gly Ser Gly Gly Gly Gly Ser Gln Val Gln Leu Val
        115                 120                 125

Glu Ser Gly Gly Gly Val Val Gln Pro Gly Arg Ser Leu Arg Leu Ser
    130                 135                 140

Cys Ala Ala Ser Gly Phe Thr Phe Ser Ser Tyr Gly Met His Trp Val
145                 150                 155                 160

Arg Gln Ala Pro Gly Lys Gly Leu Glu Trp Val Ala Val Ile Ser Tyr
                165                 170                 175

Asp Gly Ser Asp Lys Tyr Tyr Val Asp Ser Val Lys Gly Arg Phe Thr
            180                 185                 190

Ile Ser Arg Asp Asn Ser Lys Asn Arg Leu Tyr Leu Gln Met Asn Ser
        195                 200                 205

Leu Arg Ala Glu Asp Thr Ala Val Tyr Tyr Cys Ala Arg Glu Arg Tyr
    210                 215                 220

Ser Gly Arg Asp Tyr Trp Gly Gln Gly Thr Leu Val Thr Val Ser Ser
225                 230                 235                 240

Ala Ala Ala Ile Glu Val Met Tyr Pro Pro Pro Tyr Leu Asp Asn Glu
                245                 250                 255

Lys Ser Asn Gly Thr Ile Ile His Val Lys Gly Lys His Leu Cys Pro
            260                 265                 270

Ser Pro Leu Phe Pro Gly Pro Ser Lys Pro Phe Trp Val Leu Val Val
        275                 280                 285

Val Gly Gly Val Leu Ala Cys Tyr Ser Leu Leu Val Thr Val Ala Phe
    290                 295                 300

Ile Ile Phe Trp Val Arg Ser Lys Arg Ser Arg Leu Leu His Ser Asp
305                 310                 315                 320

Tyr Met Asn Met Thr Pro Arg Arg Pro Gly Pro Thr Arg Lys His Tyr
                325                 330                 335

Gln Pro Tyr Ala Pro Pro Arg Asp Phe Ala Ala Tyr Arg Ser Arg Val
            340                 345                 350

Lys Phe Ser Arg Ser Ala Asp Ala Pro Ala Tyr Gln Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460

<210> 123
<211> 1533
<212> DNA
<213> Artificial Sequence

<220>
<221> source
<223> /note="Description of Artificial Sequence: Synthetic
      polynucleotide"

<400> 123
atggcactcc ccgtaactgc tctgctgctg ccgttggcat tgctcctgca cgccgcacgc      60

ccggaaatag tgatgactca gtccccggcc accctcagcg tgtcccccgg ggagcgagcg     120

accctgtcat gcagggcttc ccagagtgtc agctccctgc tcacttggta tcagcaaaag     180

ccggggcagg ctccccgcct cctcatcttc ggggcatcaa ctagggccac cggcattcct     240

gcaagatttt ccgggtctgg cagcggcacc ggcttcaccc ttaccattag ctctctgcag     300

tctgaggact tcgccgttta ctattgtcag cagtatgata cttggccctt taccttcggt     360

cccggaacta aggtggactt caagcgcggg gggggtggat ctggaggtgg tggctccggg     420

ggcggtggaa gccaggtcca gttggttgag agcggcggcg gagtggtgca gcccgggagg     480

tccttgcggc tgagctgtgc agcctccggt tttacttttt ctagctatgg aatgcattgg     540

gtaagacagg ctcccggaaa aggcctcgag tgggtggcgg tcattagcta tgatggatct     600

gataaatact atgtggactc agttaagggg cgcttcacaa tctcaagaga caatagcaaa     660

aatagactgt acctgcagat gaatagtctg cgcgccgagg acactgccgt gtactactgc     720

gcccgcgaga gatacagcgg acgggattac tggggccagg gtaccctcgt aacggtgtcc     780

tccgctgccg cccttagcaa cagcattatg tacttttctc atttcgtgcc agtctttctc 840

ccagcaaagc ccaccactac cccggccccc aggccgccta ctcctgcccc cactatcgcg 900

tctcagcctc tctccttgcg gcccgaggcc tgccggccag ccgcaggggg cgccgtacat 960

actcggggtt tggatttcgc ttgcgacata tatatttggg cccccctcgc cggcacatgt 1020

ggagtgctgc tcctgagtct cgttataacc ctctattgca accatagaaa cagatccaaa 1080

agaagccgcc tgctccatag cgattacatg aatatgactc cacgccgccc tggccccaca 1140

aggaaacact accagcctta cgcaccacct agagatttcg ctgcctatcg gagcagggtg 1200

aagttttcca gatctgcaga tgcaccagcg tatcagcagg gccagaacca actgtataac 1260

gagctcaacc tgggacgcag ggaagagtat gacgttttgg acaagcgcag aggacgggac 1320

cctgagatgg gtggcaaacc aagacgaaaa aacccccagg agggtctcta taatgagctg 1380

cagaaggata agatggctga agcctattct gaaataggca tgaaaggaga gcggagaagg 1440

ggaaaagggc acgacggttt gtaccaggga ctcagcactg ctacgaagga tacttatgac 1500

gctctccaca tgcaagccct gccacctagg taa 1533

<210> 124
<211> 510
<212> PRT
<213> Artificial Sequence

<220>
<221> source
<223> /note="Description of Artificial Sequence: Synthetic
      polypeptide"

<400> 124
Met Ala Leu Pro Val Thr Ala Leu Leu Leu Pro Leu Ala Leu Leu Leu
1               5                   10                  15

His Ala Ala Arg Pro Glu Ile Val Met Thr Gln Ser Pro Ala Thr Leu
            20                  25                  30

Ser Val Ser Pro Gly Glu Arg Ala Thr Leu Ser Cys Arg Ala Ser Gln
        35                  40                  45

Ser Val Ser Ser Leu Leu Thr Trp Tyr Gln Gln Lys Pro Gly Gln Ala
    50                  55                  60

Pro Arg Leu Leu Ile Phe Gly Ala Ser Thr Arg Ala Thr Gly Ile Pro
65                  70                  75                  80

Ala Arg Phe Ser Gly Ser Gly Ser Gly Thr Gly Phe Thr Leu Thr Ile
                85                  90                  95

Ser Ser Leu Gln Ser Glu Asp Phe Ala Val Tyr Tyr Cys Gln Gln Tyr

            100                 105                 110

Asp Thr Trp Pro Phe Thr Phe Gly Pro Gly Thr Lys Val Asp Phe Lys
        115                 120                 125

Arg Gly Gly Gly Gly Ser Gly Gly Gly Gly Ser Gly Gly Gly Gly Ser
    130                 135                 140

Gln Val Gln Leu Val Glu Ser Gly Gly Gly Val Val Gln Pro Gly Arg
145                 150                 155                 160

Ser Leu Arg Leu Ser Cys Ala Ala Ser Gly Phe Thr Phe Ser Ser Tyr
                165                 170                 175

Gly Met His Trp Val Arg Gln Ala Pro Gly Lys Gly Leu Glu Trp Val
            180                 185                 190

Ala Val Ile Ser Tyr Asp Gly Ser Asp Lys Tyr Tyr Val Asp Ser Val
        195                 200                 205

Lys Gly Arg Phe Thr Ile Ser Arg Asp Asn Ser Lys Asn Arg Leu Tyr
    210                 215                 220

Leu Gln Met Asn Ser Leu Arg Ala Glu Asp Thr Ala Val Tyr Tyr Cys
225                 230                 235                 240

Ala Arg Glu Arg Tyr Ser Gly Arg Asp Tyr Trp Gly Gln Gly Thr Leu
                245                 250                 255

Val Thr Val Ser Ser Ala Ala Ala Leu Ser Asn Ser Ile Met Tyr Phe
            260                 265                 270

Ser His Phe Val Pro Val Phe Leu Pro Ala Lys Pro Thr Thr Thr Pro
        275                 280                 285

Ala Pro Arg Pro Pro Thr Pro Ala Pro Thr Ile Ala Ser Gln Pro Leu
    290                 295                 300

Ser Leu Arg Pro Glu Ala Cys Arg Pro Ala Ala Gly Gly Ala Val His
305                 310                 315                 320

Thr Arg Gly Leu Asp Phe Ala Cys Asp Ile Tyr Ile Trp Ala Pro Leu
                325                 330                 335

Ala Gly Thr Cys Gly Val Leu Leu Leu Ser Leu Val Ile Thr Leu Tyr
            340                 345                 350

Cys Asn His Arg Asn Arg Ser Lys Arg Ser Arg Leu Leu His Ser Asp
        355                 360                 365

Tyr Met Asn Met Thr Pro Arg Arg Pro Gly Pro Thr Arg Lys His Tyr
    370                 375                 380

Gln Pro Tyr Ala Pro Pro Arg Asp Phe Ala Ala Tyr Arg Ser Arg Val
385                 390                 395                 400

Lys Phe Ser Arg Ser Ala Asp Ala Pro Ala Tyr Gln Gln Gly Gln Asn
                405                 410                 415

Gln Leu Tyr Asn Glu Leu Asn Leu Gly Arg Arg Glu Glu Tyr Asp Val
            420                 425                 430

Leu Asp Lys Arg Arg Gly Arg Asp Pro Glu Met Gly Gly Lys Pro Arg
        435                 440                 445

Arg Lys Asn Pro Gln Glu Gly Leu Tyr Asn Glu Leu Gln Lys Asp Lys
    450                 455                 460

Met Ala Glu Ala Tyr Ser Glu Ile Gly Met Lys Gly Glu Arg Arg Arg
465                 470                 475                 480

Gly Lys Gly His Asp Gly Leu Tyr Gln Gly Leu Ser Thr Ala Thr Lys
                485                 490                 495

Asp Thr Tyr Asp Ala Leu His Met Gln Ala Leu Pro Pro Arg
            500                 505                 510

<210> 125
<211> 1467
<212> DNA
<213> Artificial Sequence

<220>
<221> source
<223> /note="Description of Artificial Sequence: Synthetic
      polynucleotide"

<400> 125
gaaatagtga tgactcagtc cccggccacc ctcagcgtgt cccccgggga gcgagcgacc      60

ctgtcatgca gggcttccca gagtgtcagc tccctgctca cttggtatca gcaaaagccg     120

gggcaggctc cccgcctcct catcttcggg gcatcaacta gggccaccgg cattcctgca     180

agattttccg ggtctggcag cggcaccggc ttcaccctta ccattagctc tctgcagtct     240

gaggacttcg ccgtttacta ttgtcagcag tatgatactt ggccctttac cttcggtccc     300

ggaactaagg tggacttcaa gcgcgggggg ggtggatctg gaggtggtgg ctccgggggc     360

ggtggaagcc aggtccagtt ggttgagagc ggcggcggag tggtgcagcc cgggaggtcc 420

ttgcggctga gctgtgcagc ctccggtttt actttttcta gctatggaat gcattgggta 480

agacaggctc ccggaaaagg cctcgagtgg gtggcggtca ttagctatga tggatctgat 540

aaatactatg tggactcagt taaggggcgc ttcacaatct caagagacaa tagcaaaaat 600

agactgtacc tgcagatgaa tagtctgcgc gccgaggaca ctgccgtgta ctactgcgcc 660

cgcgagagat acagcggacg ggattactgg ggccagggta ccctcgtaac ggtgtcctcc 720

gctgccgccc ttagcaacag cattatgtac ttttctcatt tcgtgccagt ctttctccca 780

gcaaagccca ccactacccc ggcccccagg ccgcctactc ctgcccccac tatcgcgtct 840

cagcctctct ccttgcggcc cgaggcctgc cggccagccg cagggggcgc cgtacatact 900

cggggtttgg atttcgcttg cgacatatat atttgggccc ccctcgccgg cacatgtgga 960

gtgctgctcc tgagtctcgt tataaccctc tattgcaacc atagaaacag atccaaaaga 1020

agccgcctgc tccatagcga ttacatgaat atgactccac gccgccctgg ccccacaagg 1080

aaacactacc agccttacgc accacctaga gatttcgctg cctatcggag cagggtgaag 1140

ttttccagat ctgcagatgc accagcgtat cagcagggcc agaaccaact gtataacgag 1200

ctcaacctgg gacgcaggga agagtatgac gttttggaca agcgcagagg acgggaccct 1260

gagatgggtg gcaaaccaag acgaaaaaac ccccaggagg gtctctataa tgagctgcag 1320

aaggataaga tggctgaagc ctattctgaa ataggcatga aaggagagcg gagaagggga 1380

aaagggcacg acggtttgta ccagggactc agcactgcta cgaaggatac ttatgacgct 1440

ctccacatgc aagccctgcc acctagg 1467

<210> 126
<211> 489
<212> PRT
<213> Artificial Sequence

<220>
<221> source
<223> /note="Description of Artificial Sequence: Synthetic
      polypeptide"

<400> 126
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Leu
            20                  25                  30

Leu Thr Trp Tyr Gln Gln Lys Pro Gly Gln Ala Pro Arg Leu Leu Ile
        35                  40                  45

Phe Gly Ala Ser Thr Arg Ala Thr Gly Ile Pro Ala Arg Phe Ser Gly

50                  55                  60

Ser Gly Ser Gly Thr Gly Phe Thr Leu Thr Ile Ser Ser Leu Gln Ser
65                  70                  75                  80

Glu Asp Phe Ala Val Tyr Tyr Cys Gln Gln Tyr Asp Thr Trp Pro Phe
                85                  90                  95

Thr Phe Gly Pro Gly Thr Lys Val Asp Phe Lys Arg Gly Gly Gly Gly
            100                 105                 110

Ser Gly Gly Gly Gly Ser Gly Gly Gly Gly Ser Gln Val Gln Leu Val
        115                 120                 125

Glu Ser Gly Gly Gly Val Val Gln Pro Gly Arg Ser Leu Arg Leu Ser
    130                 135                 140

Cys Ala Ala Ser Gly Phe Thr Phe Ser Ser Tyr Gly Met His Trp Val
145                 150                 155                 160

Arg Gln Ala Pro Gly Lys Gly Leu Glu Trp Val Ala Val Ile Ser Tyr
                165                 170                 175

Asp Gly Ser Asp Lys Tyr Tyr Val Asp Ser Val Lys Gly Arg Phe Thr
            180                 185                 190

Ile Ser Arg Asp Asn Ser Lys Asn Arg Leu Tyr Leu Gln Met Asn Ser
        195                 200                 205

Leu Arg Ala Glu Asp Thr Ala Val Tyr Tyr Cys Ala Arg Glu Arg Tyr
    210                 215                 220

Ser Gly Arg Asp Tyr Trp Gly Gln Gly Thr Leu Val Thr Val Ser Ser
225                 230                 235                 240

Ala Ala Ala Leu Ser Asn Ser Ile Met Tyr Phe Ser His Phe Val Pro
                245                 250                 255

Val Phe Leu Pro Ala Lys Pro Thr Thr Thr Pro Ala Pro Arg Pro Pro
            260                 265                 270

Thr Pro Ala Pro Thr Ile Ala Ser Gln Pro Leu Ser Leu Arg Pro Glu
        275                 280                 285

Ala Cys Arg Pro Ala Ala Gly Gly Ala Val His Thr Arg Gly Leu Asp
    290                 295                 300

Phe Ala Cys Asp Ile Tyr Ile Trp Ala Pro Leu Ala Gly Thr Cys Gly
305                 310                 315                 320

Val Leu Leu Leu Ser Leu Val Ile Thr Leu Tyr Cys Asn His Arg Asn
                325                 330                 335

Arg Ser Lys Arg Ser Arg Leu Leu His Ser Asp Tyr Met Asn Met Thr
            340                 345                 350

Pro Arg Arg Pro Gly Pro Thr Arg Lys His Tyr Gln Pro Tyr Ala Pro
        355                 360                 365

Pro Arg Asp Phe Ala Ala Tyr Arg Ser Arg Val Lys Phe Ser Arg Ser
    370                 375                 380

Ala Asp Ala Pro Ala Tyr Gln Gln Gly Gln Asn Gln Leu Tyr Asn Glu
385                 390                 395                 400

Leu Asn Leu Gly Arg Arg Glu Glu Tyr Asp Val Leu Asp Lys Arg Arg
                405                 410                 415

Gly Arg Asp Pro Glu Met Gly Gly Lys Pro Arg Arg Lys Asn Pro Gln
            420                 425                 430

Glu Gly Leu Tyr Asn Glu Leu Gln Lys Asp Lys Met Ala Glu Ala Tyr
        435                 440                 445

Ser Glu Ile Gly Met Lys Gly Glu Arg Arg Arg Gly Lys Gly His Asp
    450                 455                 460

Gly Leu Tyr Gln Gly Leu Ser Thr Ala Thr Lys Asp Thr Tyr Asp Ala
465                 470                 475                 480

Leu His Met Gln Ala Leu Pro Pro Arg
                485

<210> 127
<211> 63
<212> DNA
<213> Artificial Sequence

<220>
<221> source
<223> /note="Description of Artificial Sequence: Synthetic
      oligonucleotide"

<400> 127
atggcactcc ccgtaactgc tctgctgctg ccgttggcat tgctcctgca cgccgcacgc       60

ccg                                                                     63

<210> 128
<211> 21
<212> PRT
<213> Artificial Sequence

<220>
<221> source
<223> /note="Description of Artificial Sequence: Synthetic
      peptide"

<400> 128
Met Ala Leu Pro Val Thr Ala Leu Leu Leu Pro Leu Ala Leu Leu Leu
1               5                   10                  15

His Ala Ala Arg Pro
            20

<210> 129
<211> 45
<212> DNA
<213> Artificial Sequence

<220>
<221> source
<223> /note="Description of Artificial Sequence: Synthetic
      oligonucleotide"

<400> 129
ggcggtggag gctccggagg ggggggctct ggcggagggg gctcc                        45

<210> 130
<211> 15
<212> PRT
<213> Artificial Sequence

<220>
<221> source
<223> /note="Description of Artificial Sequence: Synthetic
      peptide"

<400> 130
Gly Gly Gly Gly Ser Gly Gly Gly Gly Ser Gly Gly Gly Gly Ser
1               5                   10                  15

<210> 131
<211> 54
<212> DNA
<213> Artificial Sequence

<220>
<221> source
<223> /note="Description of Artificial Sequence: Synthetic
      oligonucleotide"

<400> 131
gggtctacat ccggctccgg gaagcccgga agtggcgaag gtagtacaaa gggg             54

<210> 132
<211> 18
<212> PRT
<213> Artificial Sequence

<220>
<221> source
<223> /note="Description of Artificial Sequence: Synthetic
      peptide"

<400> 132
Gly Ser Thr Ser Gly Ser Gly Lys Pro Gly Ser Gly Glu Gly Ser Thr
1               5                   10                  15

Lys Gly

<210> 133
<211> 152
<212> PRT
<213> Artificial Sequence

<220>
<221> source
<223> /note="Description of Artificial Sequence: Synthetic
      polypeptide"

<400> 133
Met Leu Arg Leu Leu Leu Ala Leu Asn Leu Phe Pro Ser Ile Gln Val
1               5                   10                  15

Thr Gly Asn Lys Ile Leu Val Lys Gln Ser Pro Met Leu Val Ala Tyr
            20                  25                  30

Asp Asn Ala Val Asn Leu Ser Cys Lys Tyr Ser Tyr Asn Leu Phe Ser
        35                  40                  45

Arg Glu Phe Arg Ala Ser Leu His Lys Gly Leu Asp Ser Ala Val Glu
    50                  55                  60

Val Cys Val Val Tyr Gly Asn Tyr Ser Gln Gln Leu Gln Val Tyr Ser
65                  70                  75                  80

Lys Thr Gly Phe Asn Cys Asp Gly Lys Leu Gly Asn Glu Ser Val Thr
                85                  90                  95

Phe Tyr Leu Gln Asn Leu Tyr Val Asn Gln Thr Asp Ile Tyr Phe Cys
            100                 105                 110

Lys Ile Glu Val Met Tyr Pro Pro Pro Tyr Leu Asp Asn Glu Lys Ser
        115                 120                 125

Asn Gly Thr Ile Ile His Val Lys Gly Lys His Leu Cys Pro Ser Pro

130                    135                    140

Leu Phe Pro Gly Pro Ser Lys Pro
145                 150

<210> 134
<211> 9
<212> PRT
<213> Artificial Sequence

<220>
<221> source
<223> /note="Description of Artificial Sequence: Synthetic
      peptide"

<220>
<221> MOD_RES
<222> (2)..(9)
<223> Any amino acid

<400> 134
Gly Xaa Xaa Xaa Xaa Xaa Xaa Xaa Xaa
1               5

<210> 135
<211> 6
<212> PRT
<213> Artificial Sequence

<220>
<221> source
<223> /note="Description of Artificial Sequence: Synthetic
      peptide"

<220>
<221> MOD_RES
<222> (1)..(6)
<223> Any amino acid

<400> 135
Xaa Xaa Xaa Xaa Xaa Xaa
1               5

<210> 136
<211> 14
<212> PRT
<213> Artificial Sequence

<220>
<221> source
<223> /note="Description of Artificial Sequence: Synthetic
      peptide"

<220>
<221> MOD_RES
<222> (1)..(12)

<223> Any amino acid

<400> 136
Xaa Xaa Xaa Xaa Xaa Xaa Xaa Xaa Xaa Xaa Xaa Xaa Asp Tyr
1               5                   10


<210> 137
<211> 11
<212> PRT
<213> Artificial Sequence

<220>
<221> source
<223> /note="Description of Artificial Sequence: Synthetic
      peptide"


<220>
<221> MOD_RES
<222> (1)..(1)
<223> Any amino acid

<220>
<221> MOD_RES
<222> (5)..(9)
<223> Any amino acid

<220>
<221> MOD_RES
<222> (11)..(11)
<223> Any amino acid

<400> 137
Xaa Ala Ser Gln Xaa Xaa Xaa Xaa Xaa Leu Xaa
1               5                   10


<210> 138
<211> 7
<212> PRT
<213> Artificial Sequence

<220>
<221> source
<223> /note="Description of Artificial Sequence: Synthetic
      peptide"


<220>
<221> MOD_RES
<222> (1)..(1)
<223> Any amino acid

<220>
<221> MOD_RES
<222> (4)..(7)
<223> Any amino acid

<400> 138
Xaa Ala Ser Xaa Xaa Xaa Xaa
1               5

<210> 139
<211> 9
<212> PRT
<213> Artificial Sequence

<220>
<221> source
<223> /note="Description of Artificial Sequence: Synthetic
      peptide"

<220>
<221> MOD_RES
<222> (3)..(6)
<223> Any amino acid

<220>
<221> MOD_RES
<222> (8)..(8)
<223> Any amino acid

<400> 139
Gln Gln Xaa Xaa Xaa Xaa Pro Xaa Thr
1               5

<210> 140
<211> 126
<212> DNA
<213> Artificial Sequence

<220>
<221> source
<223> /note="Description of Artificial Sequence: Synthetic
      polynucleotide"

<400> 140
aagcgcggca ggaagaagct cctctacatt tttaagcagc cttttatgag gcccgtacag       60

acaacacagg aggaagatgg ctgtagctgc agatttcccg aggaggagga aggtgggtgc      120

gagctg                                                                 126

<210> 141
<211> 42
<212> PRT
<213> Artificial Sequence

<220>
<221> source
<223> /note="Description of Artificial Sequence: Synthetic
      polypeptide"

<400> 141
Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210> 142
<211> 37
<212> PRT
<213> Artificial Sequence

<220>
<221> source
<223> /note="Description of Artificial Sequence: Synthetic
      polypeptide"

<400> 142
Arg Arg Asp Gln Arg Leu Pro Pro Asp Ala His Lys Pro Pro Gly Gly
1               5                   10                  15

Gly Ser Phe Arg Thr Pro Ile Gln Glu Glu Gln Ala Asp Ala His Ser
            20                  25                  30

Thr Leu Ala Lys Ile
        35

<210> 143
<211> 21
<212> PRT
<213> Artificial Sequence

<220>
<221> source
<223> /note="Description of Artificial Sequence: Synthetic
      peptide"

<400> 143
Met Ala Leu Pro Val Thr Ala Leu Leu Leu Pro Leu Ala Leu Leu Leu
1               5                   10                  15

His Ala Ala Arg Pro
            20

<210> 144
<211> 20
<212> PRT
<213> Artificial Sequence

<220>
<221> source
<223> /note="Description of Artificial Sequence: Synthetic
      peptide"

<400> 144
Gly Gly Gly Gly Ser Gly Gly Gly Gly Ser Gly Gly Gly Gly Ser Gly
1               5                   10                  15

Gly Gly Gly Ser

            20

<210> 145
<211> 112
<212> PRT
<213> Artificial Sequence

<220>
<221> source
<223> /note="Description of Artificial Sequence: Synthetic
      polypeptide"

<400> 145
Arg Val Lys Phe Ser Arg Ser Ala Asp Ala Pro Ala Tyr Lys Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210> 146
<211> 1467
<212> DNA
<213> Artificial Sequence

<220>
<221> source
<223> /note="Description of Artificial Sequence: Synthetic
      polynucleotide"

<400> 146
atgcttctcc tggtgacaag ccttctgctc tgtgagttac cacacccagc attcctcctg      60

atcccagaca tccagatgac acagactaca tcctccctgt ctgcctctct gggagacaga     120

gtcaccatca gttgcagggc aagtcaggac attagtaaat atttaaattg gtatcagcag     180

aaaccagatg gaactgttaa actcctgatc taccatacat caagattaca ctcaggagtc     240

ccatcaaggt tcagtggcag tgggtctgga acagattatt ctctcaccat tagcaacctg     300

gagcaagaag atattgccac ttacttttgc caacaggtaa atacgcttcc gtacacgttc 360

ggagggggga ctaagttgga aataacaggc tccacctctg gatccggcaa gcccggatct 420

ggcgagggat ccaccaaggg cgaggtgaaa ctgcaggagt caggacctgg cctggtggcg 480

ccctcacaga gcctgtccgt cacatgcact gtctcagggg tctcattacc cgactatggt 540

gtaagctgga ttcgccagcc tccacgaaag ggtctggagt ggctgggagt aatatggggt 600

agtgaaacca catactataa ttcagctctc aaatccagac tgaccatcat caaggacaac 660

tccaagagcc aagttttctt aaaaatgaac agtctgcaaa ctgatgacac agccatttac 720

tactgtgcca aacattatta ctacggtggt agctatgcta tggactactg gggtcaagga 780

acctcagtca ccgtctcctc agcggccgca attgaagtta tgtatcctcc tccttaccta 840

gacaatgaga agagcaatgg aaccattatc catgtgaaag ggaaacacct ttgtccaagt 900

cccctatttc ccggaccttc taagcccttt tgggtgctgg tggtggttgg gggagtcctg 960

gcttgctata gcttgctagt aacagtggcc tttattattt tctgggtgag gagtaagagg 1020

agcaggctcc tgcacagtga ctacatgaac atgactcccc gccgccccgg gcccacccgc 1080

aagcattacc agccctatgc cccaccacgc gacttcgcag cctatcgctc cagagtgaag 1140

ttcagcagga gcgcagacgc ccccgcgtac cagcagggcc agaaccagct ctataacgag 1200

ctcaatctag gacgaagaga ggagtacgat gttttggaca agagacgtgg ccgggaccct 1260

gagatggggg gaaagccgag aaggaagaac cctcaggaag gcctgtacaa tgaactgcag 1320

aaagataaga tggcggaggc ctacagtgag attgggatga aaggcgagcg ccggaggggc 1380

aaggggcacg atggccttta ccagggtctc agtacagcca ccaaggacac ctacgacgcc 1440

cttcacatgc aggccctgcc ccctcgc 1467

<210> 147
<211> 489
<212> PRT
<213> Artificial Sequence

<220>
<221> source
<223> /note="Description of Artificial Sequence: Synthetic
      polypeptide"

<400> 147
Met Leu Leu Leu Val Thr Ser Leu Leu Leu Cys Glu Leu Pro His Pro
1               5                   10                  15

Ala Phe Leu Leu Ile Pro Asp Ile Gln Met Thr Gln Thr Thr Ser Ser
            20                  25                  30

Leu Ser Ala Ser Leu Gly Asp Arg Val Thr Ile Ser Cys Arg Ala Ser
        35                  40                  45

Gln Asp Ile Ser Lys Tyr Leu Asn Trp Tyr Gln Gln Lys Pro Asp Gly
    50                  55                  60

Thr Val Lys Leu Leu Ile Tyr His Thr Ser Arg Leu His Ser Gly Val
65                  70                  75                  80

Pro Ser Arg Phe Ser Gly Ser Gly Ser Gly Thr Asp Tyr Ser Leu Thr
                85                  90                  95

Ile Ser Asn Leu Glu Gln Glu Asp Ile Ala Thr Tyr Phe Cys Gln Gln
            100                 105                 110

Gly Asn Thr Leu Pro Tyr Thr Phe Gly Gly Gly Thr Lys Leu Glu Ile
        115                 120                 125

Thr Gly Ser Thr Ser Gly Ser Gly Lys Pro Gly Ser Gly Glu Gly Ser
    130                 135                 140

Thr Lys Gly Glu Val Lys Leu Gln Glu Ser Gly Pro Gly Leu Val Ala
145                 150                 155                 160

Pro Ser Gln Ser Leu Ser Val Thr Cys Thr Val Ser Gly Val Ser Leu
                165                 170                 175

Pro Asp Tyr Gly Val Ser Trp Ile Arg Gln Pro Pro Arg Lys Gly Leu
            180                 185                 190

Glu Trp Leu Gly Val Ile Trp Gly Ser Glu Thr Thr Tyr Tyr Asn Ser
        195                 200                 205

Ala Leu Lys Ser Arg Leu Thr Ile Ile Lys Asp Asn Ser Lys Ser Gln
    210                 215                 220

Val Phe Leu Lys Met Asn Ser Leu Gln Thr Asp Asp Thr Ala Ile Tyr
225                 230                 235                 240

Tyr Cys Ala Lys His Tyr Tyr Tyr Gly Gly Ser Tyr Ala Met Asp Tyr
                245                 250                 255

Trp Gly Gln Gly Thr Ser Val Thr Val Ser Ser Ala Ala Ala Ile Glu
            260                 265                 270

Val Met Tyr Pro Pro Pro Tyr Leu Asp Asn Glu Lys Ser Asn Gly Thr
        275                 280                 285

Ile Ile His Val Lys Gly Lys His Leu Cys Pro Ser Pro Leu Phe Pro
    290                 295                 300

Gly Pro Ser Lys Pro Phe Trp Val Leu Val Val Val Gly Gly Val Leu
305                 310                 315                 320

Ala Cys Tyr Ser Leu Leu Val Thr Val Ala Phe Ile Ile Phe Trp Val
                325                 330                 335

Arg Ser Lys Arg Ser Arg Leu Leu His Ser Asp Tyr Met Asn Met Thr
            340                 345                 350

Pro Arg Arg Pro Gly Pro Thr Arg Lys His Tyr Gln Pro Tyr Ala Pro
        355                 360                 365

Pro Arg Asp Phe Ala Ala Tyr Arg Ser Arg Val Lys Phe Ser Arg Ser
    370                 375                 380

Ala Asp Ala Pro Ala Tyr Gln Gln Gly Gln Asn Gln Leu Tyr Asn Glu
385                 390                 395                 400

Leu Asn Leu Gly Arg Arg Glu Glu Tyr Asp Val Leu Asp Lys Arg Arg
                405                 410                 415

Gly Arg Asp Pro Glu Met Gly Gly Lys Pro Arg Arg Lys Asn Pro Gln
            420                 425                 430

Glu Gly Leu Tyr Asn Glu Leu Gln Lys Asp Lys Met Ala Glu Ala Tyr
        435                 440                 445

Ser Glu Ile Gly Met Lys Gly Glu Arg Arg Arg Gly Lys Gly His Asp
    450                 455                 460

Gly Leu Tyr Gln Gly Leu Ser Thr Ala Thr Lys Asp Thr Tyr Asp Ala
465                 470                 475                 480

Leu His Met Gln Ala Leu Pro Pro Arg
                485

<210> 148
<211> 1437
<212> DNA
<213> Artificial Sequence

<220>
<221> source
<223> /note="Description of Artificial Sequence: Synthetic
      polynucleotide"

<400> 148
atggcactcc ccgtaactgc tctgctgctg ccgttggcat tgctcctgca cgccgcacgc        60

ccggatattc aaatgaccca gtccccgtcc tccctgagtg cctccgtcgg tgaccgtgtt 120

acgattacct gccgtgcgag ccaagacatc tctaaatacc tgaactggta tcagcaaaaa 180

ccggatcagg caccgaaact gctgatcaaa catacctcac gtctgcactc gggtgtgccg 240

agccgcttta gtggttccgg ctcaggtacc gattacaccc tgacgatcag ctctctgcag 300

ccggaagact ttgccacgta ttactgccag caaggtaata ccctgccgta tacgttcggc 360

caaggtacca aactggaaat caaaggctcg acgagcggct ctggtaaacc gggctctggt 420

gaaggcagta ccaaaggtga agtgcagctg gttgaaagcg gtggtggtct ggttcaaccg 480

ggtcgttccc tgcgtctgtc atgtacggcg agtggtgtct ccctgccgga ctatggcgtg 540

tcctggattc gtcagccgcc gggtaaaggc ctggaatgga ttggtgtcat ctggggcagt 600

gaaaccacgt attacaactc ggccctgaaa agccgtttca ccatctctcg cgataacagt 660

aaaaatacgc tgtacctgca gatgaatagc ctgcgcgcgg aagacaccgc cgtttactac 720

tgcgcaaaac attactacta cggtggcagc tatgctatgg attactgggg tcaaggcacg 780

ctggtcaccg tttcgtcagc cgctgccctt gataatgaaa agtcaaacgg aacaatcatt 840

cacgtgaagg gcaagcacct ctgtccgtca cccttgttcc ctggtccatc caagccattc 900

tgggtgttgg tcgtagtggg tggagtcctc gcttgttact ctctgctcgt caccgtggct 960

tttataatct tctgggttag atccaaaaga agccgcctgc tccatagcga ttacatgaat 1020

atgactccac gccgccctgg ccccacaagg aaacactacc agccttacgc accacctaga 1080

gatttcgctg cctatcggag cagggtgaag ttttccagat ctgcagatgc accagcgtat 1140

cagcagggcc agaaccaact gtataacgag ctcaacctgg gacgcaggga agagtatgac 1200

gttttggaca agcgcagagg acgggaccct gagatgggtg gcaaaccaag acgaaaaaac 1260

ccccaggagg gtctctataa tgagctgcag aaggataaga tggctgaagc ctattctgaa 1320

ataggcatga aaggagagcg gagaagggga aaagggcacg acggtttgta ccagggactc 1380

agcactgcta cgaaggatac ttatgacgct ctccacatgc aagccctgcc acctagg 1437

<210> 149
<211> 479
<212> PRT
<213> Artificial Sequence

<220>
<221> source
<223> /note="Description of Artificial Sequence: Synthetic
      polypeptide"

<400> 149
Met Ala Leu Pro Val Thr Ala Leu Leu Leu Pro Leu Ala Leu Leu Leu
1               5                   10                  15

His Ala Ala Arg Pro Asp Ile Gln Met Thr Gln Ser Pro Ser Ser Leu

20                  25                  30

Ser Ala Ser Val Gly Asp Arg Val Thr Ile Thr Cys Arg Ala Ser Gln
        35                  40                  45

Asp Ile Ser Lys Tyr Leu Asn Trp Tyr Gln Gln Lys Pro Asp Gln Ala
    50                  55                  60

Pro Lys Leu Leu Ile Lys His Thr Ser Arg Leu His Ser Gly Val Pro
65                  70                  75                  80

Ser Arg Phe Ser Gly Ser Gly Ser Gly Thr Asp Tyr Thr Leu Thr Ile
                85                  90                  95

Ser Ser Leu Gln Pro Glu Asp Phe Ala Thr Tyr Tyr Cys Gln Gln Gly
            100                 105                 110

Asn Thr Leu Pro Tyr Thr Phe Gly Gln Gly Thr Lys Leu Glu Ile Lys
        115                 120                 125

Gly Ser Thr Ser Gly Ser Gly Lys Pro Gly Ser Gly Glu Gly Ser Thr
    130                 135                 140

Lys Gly Glu Val Gln Leu Val Glu Ser Gly Gly Gly Leu Val Gln Pro
145                 150                 155                 160

Gly Arg Ser Leu Arg Leu Ser Cys Thr Ala Ser Gly Val Ser Leu Pro
                165                 170                 175

Asp Tyr Gly Val Ser Trp Ile Arg Gln Pro Pro Gly Lys Gly Leu Glu
            180                 185                 190

Trp Ile Gly Val Ile Trp Gly Ser Glu Thr Thr Tyr Tyr Asn Ser Ala
        195                 200                 205

Leu Lys Ser Arg Phe Thr Ile Ser Arg Asp Asn Ser Lys Asn Thr Leu
    210                 215                 220

Tyr Leu Gln Met Asn Ser Leu Arg Ala Glu Asp Thr Ala Val Tyr Tyr
225                 230                 235                 240

Cys Ala Lys His Tyr Tyr Tyr Gly Gly Ser Tyr Ala Met Asp Tyr Trp
                245                 250                 255

Gly Gln Gly Thr Leu Val Thr Val Ser Ser Ala Ala Ala Leu Asp Asn
            260                 265                 270

Glu Lys Ser Asn Gly Thr Ile Ile His Val Lys Gly Lys His Leu Cys
        275                 280                 285

Pro Ser Pro Leu Phe Pro Gly Pro Ser Lys Pro Phe Trp Val Leu Val
    290                 295                 300

Val Val Gly Gly Val Leu Ala Cys Tyr Ser Leu Leu Val Thr Val Ala
305                 310                 315                 320

Phe Ile Ile Phe Trp Val Arg Ser Lys Arg Ser Arg Leu Leu His Ser
                325                 330                 335

Asp Tyr Met Asn Met Thr Pro Arg Arg Pro Gly Pro Thr Arg Lys His
            340                 345                 350

Tyr Gln Pro Tyr Ala Pro Pro Arg Asp Phe Ala Ala Tyr Arg Ser Arg
        355                 360                 365

Val Lys Phe Ser Arg Ser Ala Asp Ala Pro Ala Tyr Gln Gln Gly Gln
    370                 375                 380

Asn Gln Leu Tyr Asn Glu Leu Asn Leu Gly Arg Arg Glu Glu Tyr Asp
385                 390                 395                 400

Val Leu Asp Lys Arg Arg Gly Arg Asp Pro Glu Met Gly Gly Lys Pro
                405                 410                 415

Arg Arg Lys Asn Pro Gln Glu Gly Leu Tyr Asn Glu Leu Gln Lys Asp
            420                 425                 430

Lys Met Ala Glu Ala Tyr Ser Glu Ile Gly Met Lys Gly Glu Arg Arg
        435                 440                 445

Arg Gly Lys Gly His Asp Gly Leu Tyr Gln Gly Leu Ser Thr Ala Thr
    450                 455                 460

Lys Asp Thr Tyr Asp Ala Leu His Met Gln Ala Leu Pro Pro Arg
465                 470                 475

<210> 150
<211> 1506
<212> DNA
<213> Artificial Sequence

<220>
<221> source
<223> /note="Description of Artificial Sequence: Synthetic
      polynucleotide"

<400> 150

atggccctgc ctgtgacagc tctgctgctg cccctggccc tgctgctgca tgccgccaga 60

cctgagatcg tgctgaccca gtctcccggc accctgtctc tcagcccagg agagagagcc 120

accctgagct gcagagccag ccagagcgtg tccagcagct acctggcctg gtatcagcag 180

aagcccggac aggcccccag actgctgatc tacggcgcca gctctagagc caccggcatc 240

cccgacagat tcagcggcag cggcagtggc accgacttca ccctgaccat cagcagactg 300

gaacccgagg acttcgccgt gtactactgc cagcagtacg gcagcagccg gttcaccttc 360

ggccctggca ccaaggtgga catcaagggc agcacctccg gcagcggcaa gcctggctct 420

ggcgagggct ctaccaaggg ccaggtgcag ctggtgcagt ctggcgccga agtgaagaaa 480

cccggctcta gcgtgaaggt gtcctgcaag gacagcggcg gcaccttcag cagctacgcc 540

atcagctggg tgcgccaggc cccaggacag gggctggaat ggatgggcgg catcatcccc 600

atcttcggca ccaccaacta cgcccagcag ttccagggca gagtgaccat caccgccgac 660

gagagcacca gcaccgccta catggaactg agcagcctgc ggagcgagga cacagccgtg 720

tattactgtg cccgcgaggc cgtggccgcc gactggctgg atccttgggg acagggcacc 780

ctggtgacag tgtccagctt cgtgcccgtg ttcctgcccg ccaagcctac caccacccct 840

gcccctagac ctcccacccc agccccaaca atcgccagcc agcctctgtc cctgcggccc 900

gaagcctgta gacctgctgc cggcggagcc gtgcacacca gaggcctgga cttcgcctgc 960

gatatctaca tctgggcccc tctggccggc acctgtggcg tgctgctgct gagcctggtg 1020

atcaccctgt actgcaacca ccggaacaga agcaagcgga gccggctgct gcacagcgac 1080

tacatgaaca tgaccccaag acggcctggc cccacccgga agcactacca gccttacgcc 1140

cctcccagag acttcgccgc ctaccggtcc agagtgaagt tcagcagatc cgccgacgcc 1200

cctgcctacc agcagggaca gaaccagctg tacaacgagc tgaacctggg cagacgggaa 1260

gagtacgacg tgctggacaa gcggagaggc cgggaccccg agatggcggg aaagcccaga 1320

cggaagaacc cccaggaagg cctgtataac gaactgcaga aagacaagat ggccgaggcc 1380

tacagcgaga tcggcatgaa gggcgagcgg aggcgcggca agggccacga tggcctgtac 1440

cagggcctga gcaccgccac caaggacacc tacgacgccc tgcacatgca ggccctgccc 1500

cccaga 1506

<210> 151
<211> 502
<212> PRT
<213> Artificial Sequence

<220>
<221> source
<223> /note="Description of Artificial Sequence: Synthetic
      polypeptide"

<400> 151
Met Ala Leu Pro Val Thr Ala Leu Leu Leu Pro Leu Ala Leu Leu Leu
1               5                   10                  15

His Ala Ala Arg Pro Glu Ile Val Leu Thr Gln Ser Pro Gly Thr Leu
            20                  25                  30

Ser Leu Ser Pro Gly Glu Arg Ala Thr Leu Ser Cys Arg Ala Ser Gln
        35                  40                  45

Ser Val Ser Ser Ser Tyr Leu Ala Trp Tyr Gln Gln Lys Pro Gly Gln
    50                  55                  60

Ala Pro Arg Leu Leu Ile Tyr Gly Ala Ser Ser Arg Ala Thr Gly Ile
65                  70                  75                  80

Pro Asp Arg Phe Ser Gly Ser Gly Ser Gly Thr Asp Phe Thr Leu Thr
                85                  90                  95

Ile Ser Arg Leu Glu Pro Glu Asp Phe Ala Val Tyr Tyr Cys Gln Gln
            100                 105                 110

Tyr Gly Ser Ser Arg Phe Thr Phe Gly Pro Gly Thr Lys Val Asp Ile
        115                 120                 125

Lys Gly Ser Thr Ser Gly Ser Gly Lys Pro Gly Ser Gly Glu Gly Ser
    130                 135                 140

Thr Lys Gly Gln Val Gln Leu Val Gln Ser Gly Ala Glu Val Lys Lys
145                 150                 155                 160

Pro Gly Ser Ser Val Lys Val Ser Cys Lys Asp Ser Gly Gly Thr Phe
                165                 170                 175

Ser Ser Tyr Ala Ile Ser Trp Val Arg Gln Ala Pro Gly Gln Gly Leu
            180                 185                 190

Glu Trp Met Gly Gly Ile Ile Pro Ile Phe Gly Thr Thr Asn Tyr Ala
        195                 200                 205

Gln Gln Phe Gln Gly Arg Val Thr Ile Thr Ala Asp Glu Ser Thr Ser
    210                 215                 220

Thr Ala Tyr Met Glu Leu Ser Ser Leu Arg Ser Glu Asp Thr Ala Val
225                 230                 235                 240

Tyr Tyr Cys Ala Arg Glu Ala Val Ala Ala Asp Trp Leu Asp Pro Trp
                245                 250                 255

Gly Gln Gly Thr Leu Val Thr Val Ser Ser Phe Val Pro Val Phe Leu
        260                 265                 270

Pro Ala Lys Pro Thr Thr Thr Pro Ala Pro Arg Pro Pro Thr Pro Ala
    275                 280                 285

Pro Thr Ile Ala Ser Gln Pro Leu Ser Leu Arg Pro Glu Ala Cys Arg
    290                 295                 300

Pro Ala Ala Gly Gly Ala Val His Thr Arg Gly Leu Asp Phe Ala Cys
305                 310                 315                 320

Asp Ile Tyr Ile Trp Ala Pro Leu Ala Gly Thr Cys Gly Val Leu Leu
                325                 330                 335

Leu Ser Leu Val Ile Thr Leu Tyr Cys Asn His Arg Asn Arg Ser Lys
            340                 345                 350

Arg Ser Arg Leu Leu His Ser Asp Tyr Met Asn Met Thr Pro Arg Arg
        355                 360                 365

Pro Gly Pro Thr Arg Lys His Tyr Gln Pro Tyr Ala Pro Pro Arg Asp
    370                 375                 380

Phe Ala Ala Tyr Arg Ser Arg Val Lys Phe Ser Arg Ser Ala Asp Ala
385                 390                 395                 400

Pro Ala Tyr Gln Gln Gly Gln Asn Gln Leu Tyr Asn Glu Leu Asn Leu
                405                 410                 415

Gly Arg Arg Glu Glu Tyr Asp Val Leu Asp Lys Arg Arg Gly Arg Asp
            420                 425                 430

Pro Glu Met Gly Gly Lys Pro Arg Arg Lys Asn Pro Gln Glu Gly Leu
        435                 440                 445

Tyr Asn Glu Leu Gln Lys Asp Lys Met Ala Glu Ala Tyr Ser Glu Ile
    450                 455                 460

Gly Met Lys Gly Glu Arg Arg Arg Gly Lys Gly His Asp Gly Leu Tyr
465                 470                 475                 480

Gln Gly Leu Ser Thr Ala Thr Lys Asp Thr Tyr Asp Ala Leu His Met
                485                 490                 495

Gln Ala Leu Pro Pro Arg

            500

<210> 152
<211> 1824
<212> DNA
<213> Artificial Sequence

<220>
<221> source
<223> /note="Description of Artificial Sequence: Synthetic
      polynucleotide"

<400> 152
atggcatgcc ctggcttcct gtgggcactt gtgatctcca cctgtcttga atttagcatg      60

gctcagacag tcactcagtc tcaaccagag atgtctgtgc aggaggcaga gaccgtgacc     120

ctgagctgca catatgacac cagtgagagt gattattatt tattctggta caagcagcct     180

cccagcaggc agatgattct cgttattcgc caagaagctt ataagcaaca gaatgcaaca     240

gagaatcgtt tctctgtgaa cttccagaaa gcagccaaat ccttcagtct caagatctca     300

gactcacagc tgggggatgc cgcgatgtat ttctgtgctc tccggagctc aggaacctac     360

aaatacatct ttggaacagg caccaggctg aaggttttag caaatatcca gaaccctgaa     420

cctgctgtgt accagttaaa agatcctcgg tctcaggaca gcaccctctg cctgttcacc     480

gactttgact cccaaatcaa tgtgccgaaa accatggaat ctggaacgtt catcactgac     540

aaaactgtgc tggacatgaa agctatggat tccaagagca atggggccat tgcctggagc     600

aaccagacaa gcttcacctg ccaagatatc ttcaaagaga ccaacgccac ctaccccagt     660

tcagacgttc cctgtgatgc cacgttgact gagaaaagct ttgaaacaga tatgaaccta     720

aactttcaaa acctgtcagt tatgggactc cgaatcctcc tgctgaaagt agccggattt     780

aacctgctca tgacgctgag gctgtggtcc agtcgggcca agcggtccgg atccggagcc     840

accaacttca gcctgctgaa gcaggccggc gacgtggagg agaaccccgg ccccatgggc     900

accaggctcc tcttctgggt ggccttctgt ctcctggggg cagatcacac aggagctgga     960

gtctcccagt cccccagtaa caaggtcaca gagaagggaa aggatgtaga gctcaggtgt    1020

gatccaattt caggtcatac tgccctttac tggtaccgac agagcctggg gcagggcctg    1080

gagtttttaa tttacttcca aggcaacagt gcaccagaca aatcagggct gcccagtgat    1140

cgcttctctg cagagaggac tgggggatcc gtctccactc tgacgatcca gcgcacacag    1200

caggaggact cggccgtgta tctctgtgcc agcatccgga cagggccttt tttctctgga    1260

aacaccatat attttggaga gggaagttgg ctcactgttg tagaggacct gagaaacgtg    1320

accccaccca aggtctcctt gtttgagcca tcaaaagcag agattgcaaa caaacaaaag    1380

gctaccctcg tgtgcttggc caggggcttc ttccctgacc acgtggagct gagctggtgg    1440

gtgaatggca aggaggtcca cagtggggtc agcacggacc ctcaggccta caaggagagc    1500

aattatagct actgcctgag cagccgcctg agggtctctg ctaccttctg gcacaatcct      1560

cgaaaccact tccgctgcca agtgcagttc catgggcttt cagaggagga caagtggcca      1620

gagggctcac ccaaacctgt cacacagaac atcagtgcag aggcctgggg ccgagcagac      1680

tgtggaatca cttcagcatc ctatcatcag ggggttctgt ctgcaaccat cctctatgag      1740

atcctactgg ggaaggccac cctatatgct gtgctggtca gtggcctggt gctgatggct      1800

atggtcaaaa gaaagaactc atga                                              1824

<210> 153
<211> 607
<212> PRT
<213> Artificial Sequence

<220>
<221> source
<223> /note="Description of Artificial Sequence: Synthetic
      polypeptide"

<400> 153
Met Ala Cys Pro Gly Phe Leu Trp Ala Leu Val Ile Ser Thr Cys Leu
1               5                   10                  15

Glu Phe Ser Met Ala Gln Thr Val Thr Gln Ser Gln Pro Glu Met Ser
            20                  25                  30

Val Gln Glu Ala Glu Thr Val Thr Leu Ser Cys Thr Tyr Asp Thr Ser
        35                  40                  45

Glu Ser Asp Tyr Tyr Leu Phe Trp Tyr Lys Gln Pro Pro Ser Arg Gln
    50                  55                  60

Met Ile Leu Val Ile Arg Gln Glu Ala Tyr Lys Gln Gln Asn Ala Thr
65                  70                  75                  80

Glu Asn Arg Phe Ser Val Asn Phe Gln Lys Ala Ala Lys Ser Phe Ser
                85                  90                  95

Leu Lys Ile Ser Asp Ser Gln Leu Gly Asp Ala Ala Met Tyr Phe Cys
            100                 105                 110

Ala Leu Arg Ser Ser Gly Thr Tyr Lys Tyr Ile Phe Gly Thr Gly Thr
        115                 120                 125

Arg Leu Lys Val Leu Ala Asn Ile Gln Asn Pro Glu Pro Ala Val Tyr
    130                 135                 140

Gln Leu Lys Asp Pro Arg Ser Gln Asp Ser Thr Leu Cys Leu Phe Thr
145                 150                 155                 160

Asp Phe Asp Ser Gln Ile Asn Val Pro Lys Thr Met Glu Ser Gly Thr
                165                 170                 175

Phe Ile Thr Asp Lys Thr Val Leu Asp Met Lys Ala Met Asp Ser Lys
            180                 185                 190

Ser Asn Gly Ala Ile Ala Trp Ser Asn Gln Thr Ser Phe Thr Cys Gln
        195                 200                 205

Asp Ile Phe Lys Glu Thr Asn Ala Thr Tyr Pro Ser Ser Asp Val Pro
    210                 215                 220

Cys Asp Ala Thr Leu Thr Glu Lys Ser Phe Glu Thr Asp Met Asn Leu
225                 230                 235                 240

Asn Phe Gln Asn Leu Ser Val Met Gly Leu Arg Ile Leu Leu Leu Lys
                245                 250                 255

Val Ala Gly Phe Asn Leu Leu Met Thr Leu Arg Leu Trp Ser Ser Arg
            260                 265                 270

Ala Lys Arg Ser Gly Ser Gly Ala Thr Asn Phe Ser Leu Leu Lys Gln
        275                 280                 285

Ala Gly Asp Val Glu Glu Asn Pro Gly Pro Met Gly Thr Arg Leu Leu
    290                 295                 300

Phe Trp Val Ala Phe Cys Leu Leu Gly Ala Asp His Thr Gly Ala Gly
305                 310                 315                 320

Val Ser Gln Ser Pro Ser Asn Lys Val Thr Glu Lys Gly Lys Asp Val
                325                 330                 335

Glu Leu Arg Cys Asp Pro Ile Ser Gly His Thr Ala Leu Tyr Trp Tyr
            340                 345                 350

Arg Gln Ser Leu Gly Gln Gly Leu Glu Phe Leu Ile Tyr Phe Gln Gly
        355                 360                 365

Asn Ser Ala Pro Asp Lys Ser Gly Leu Pro Ser Asp Arg Phe Ser Ala
    370                 375                 380

Glu Arg Thr Gly Gly Ser Val Ser Thr Leu Thr Ile Gln Arg Thr Gln
385                 390                 395                 400

Gln Glu Asp Ser Ala Val Tyr Leu Cys Ala Ser Ile Arg Thr Gly Pro

405                 410                 415

Phe Phe Ser Gly Asn Thr Ile Tyr Phe Gly Glu Gly Ser Trp Leu Thr
            420                 425                 430

Val Val Glu Asp Leu Arg Asn Val Thr Pro Pro Lys Val Ser Leu Phe
        435                 440                 445

Glu Pro Ser Lys Ala Glu Ile Ala Asn Lys Gln Lys Ala Thr Leu Val
    450                 455                 460

Cys Leu Ala Arg Gly Phe Phe Pro Asp His Val Glu Leu Ser Trp Trp
465                 470                 475                 480

Val Asn Gly Lys Glu Val His Ser Gly Val Ser Thr Asp Pro Gln Ala
                485                 490                 495

Tyr Lys Glu Ser Asn Tyr Ser Tyr Cys Leu Ser Ser Arg Leu Arg Val
            500                 505                 510

Ser Ala Thr Phe Trp His Asn Pro Arg Asn His Phe Arg Cys Gln Val
        515                 520                 525

Gln Phe His Gly Leu Ser Glu Glu Asp Lys Trp Pro Glu Gly Ser Pro
    530                 535                 540

Lys Pro Val Thr Gln Asn Ile Ser Ala Glu Ala Trp Gly Arg Ala Asp
545                 550                 555                 560

Cys Gly Ile Thr Ser Ala Ser Tyr His Gln Gly Val Leu Ser Ala Thr
                565                 570                 575

Ile Leu Tyr Glu Ile Leu Leu Gly Lys Ala Thr Leu Tyr Ala Val Leu
            580                 585                 590

Val Ser Gly Leu Val Leu Met Ala Met Val Lys Arg Lys Asn Ser
        595                 600                 605

<210> 154
<211> 1437
<212> DNA
<213> Artificial Sequence

<220>
<221> source
<223> /note="Description of Artificial Sequence: Synthetic
      polynucleotide"

<400> 154
atggcactcc ccgtaactgc tctgctgctg ccgttggcat tgctcctgca cgccgcacgc      60

ccgcaggtcc aactgcaaga aagcggaccc ggactggtga agccttctga gacacttagt 120

ctgacgtgca cggtcagtgg cggctccatc tcctcctatt attggtcatg gatacgacaa 180

cccccaggta agggcctgga atggattggc tatatctact attcaggaag cacgaactac 240

aatcccagcc tgaagtcccg agtgacaatt tcagtagata ccagtaaaaa ccagttcagt 300

cttaaactgt caagcgtgac agctgccgac accgctgtgt attactgcgt ctcactggtg 360

tattgtggag gggattgtta tagcgggttc gattattggg gacagggaac cctggtgact 420

gtatcttccg gcggcggcgg ctcagggggt ggcggtagtg gcggtggggg ttccgatatt 480

caactgacac aatcccccag ctcactcagc gccagcgtgg gggacagggt tagctttacc 540

tgtcaagcct ctcaggatat aaataacttt ctgaactggt atcaacagaa gcctgggaag 600

gcgcccaaac tcctgatcta tgatgcgtcc aacctggaaa ctggcgtgcc ttcacgcttt 660

agcggctctg gcagtggtac agacttcact tttaccatct cttcacttca gccggaggac 720

atcgccacat attactgtca acagtacgga aacttgccct ttacttttgg aggcggcacc 780

aaagttgaaa tcaaaagggc cgctgccctg gataacgaaa agagcaatgg gactataata 840

catgttaaag gaaaacacct gtgtccatct cccctgttcc ctggaccgtc aaagccattt 900

tgggtgctcg tggttgtcgg tggcgttctc gcctgttata gcttgctggt gacagtagcc 960

ttcattatct tttgggtgag atccaaaaga agccgcctgc tccatagcga ttacatgaat 1020

atgactccac gccgccctgg ccccacaagg aaacactacc agccttacgc accacctaga 1080

gatttcgctg cctatcggag ccgagtgaaa ttttctagat cagctgatgc tcccgcctat 1140

cagcagggac agaatcaact ttacaatgag ctgaacctgg gtcgcagaga agagtacgac 1200

gttttggaca aacgccgggg ccgagatcct gagatggggg ggaagccgag aaggaagaat 1260

cctcaagaag gcctgtacaa cgagcttcaa aaagacaaaa tggctgaggc gtactctgag 1320

atcggcatga agggcgagcg gagacgaggc aagggtcacg atggcttgta tcagggcctg 1380

agtacagcca caaaggacac ctatgacgcc ctccacatgc aggcactgcc cccacgc 1437

<210> 155
<211> 479
<212> PRT
<213> Artificial Sequence

<220>
<221> source
<223> /note="Description of Artificial Sequence: Synthetic
      polypeptide"

<400> 155
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Pro Gly Leu
            20                  25                  30

Val Lys Pro Ser Glu Thr Leu Ser Leu Thr Cys Thr Val Ser Gly Gly
        35                  40                  45

Ser Ile Ser Ser Tyr Tyr Trp Ser Trp Ile Arg Gln Pro Pro Gly Lys
    50                  55                  60

Gly Leu Glu Trp Ile Gly Tyr Ile Tyr Tyr Ser Gly Ser Thr Asn Tyr
65                  70                  75                  80

Asn Pro Ser Leu Lys Ser Arg Val Thr Ile Ser Val Asp Thr Ser Lys
                85                  90                  95

Asn Gln Phe Ser Leu Lys Leu Ser Ser Val Thr Ala Ala Asp Thr Ala
            100                 105                 110

Val Tyr Tyr Cys Val Ser Leu Val Tyr Cys Gly Gly Asp Cys Tyr Ser
        115                 120                 125

Gly Phe Asp Tyr Trp Gly Gln Gly Thr Leu Val Thr Val Ser Ser Gly
    130                 135                 140

Gly Gly Gly Ser Gly Gly Gly Gly Ser Gly Gly Gly Gly Ser Asp Ile
145                 150                 155                 160

Gln Leu Thr Gln Ser Pro Ser Ser Leu Ser Ala Ser Val Gly Asp Arg
                165                 170                 175

Val Ser Phe Thr Cys Gln Ala Ser Gln Asp Ile Asn Asn Phe Leu Asn
            180                 185                 190

Trp Tyr Gln Gln Lys Pro Gly Lys Ala Pro Lys Leu Leu Ile Tyr Asp
        195                 200                 205

Ala Ser Asn Leu Glu Thr Gly Val Pro Ser Arg Phe Ser Gly Ser Gly
    210                 215                 220

Ser Gly Thr Asp Phe Thr Phe Thr Ile Ser Ser Leu Gln Pro Glu Asp
225                 230                 235                 240

Ile Ala Thr Tyr Tyr Cys Gln Gln Tyr Gly Asn Leu Pro Phe Thr Phe
                245                 250                 255

Gly Gly Gly Thr Lys Val Glu Ile Lys Arg Ala Ala Ala Leu Asp Asn
            260                 265                 270

Glu Lys Ser Asn Gly Thr Ile Ile His Val Lys Gly Lys His Leu Cys
        275                 280                 285

Pro Ser Pro Leu Phe Pro Gly Pro Ser Lys Pro Phe Trp Val Leu Val
    290                 295                 300

Val Val Gly Gly Val Leu Ala Cys Tyr Ser Leu Leu Val Thr Val Ala
305                 310                 315                 320

Phe Ile Ile Phe Trp Val Arg Ser Lys Arg Ser Arg Leu Leu His Ser
                325                 330                 335

Asp Tyr Met Asn Met Thr Pro Arg Arg Pro Gly Pro Thr Arg Lys His
            340                 345                 350

Tyr Gln Pro Tyr Ala Pro Pro Arg Asp Phe Ala Ala Tyr Arg Ser Arg
        355                 360                 365

Val Lys Phe Ser Arg Ser Ala Asp Ala Pro Ala Tyr Gln Gln Gly Gln
    370                 375                 380

Asn Gln Leu Tyr Asn Glu Leu Asn Leu Gly Arg Arg Glu Glu Tyr Asp
385                 390                 395                 400

Val Leu Asp Lys Arg Arg Gly Arg Asp Pro Glu Met Gly Gly Lys Pro
                405                 410                 415

Arg Arg Lys Asn Pro Gln Glu Gly Leu Tyr Asn Glu Leu Gln Lys Asp
            420                 425                 430

Lys Met Ala Glu Ala Tyr Ser Glu Ile Gly Met Lys Gly Glu Arg Arg
        435                 440                 445

Arg Gly Lys Gly His Asp Gly Leu Tyr Gln Gly Leu Ser Thr Ala Thr
    450                 455                 460

Lys Asp Thr Tyr Asp Ala Leu His Met Gln Ala Leu Pro Pro Arg
465                 470                 475

<210> 156
<211> 1449
<212> DNA
<213> Artificial Sequence

<220>
<221> source
<223> /note="Description of Artificial Sequence: Synthetic
      polynucleotide"

<400> 156
atggcactcc ccgtaactgc tctgctgctg ccgttggcat tgctcctgca cgccgcacgc      60

ccgcaggtgc agctggtgga gtctggggga ggcgtggtcc agcctgggag gtccctgaga     120

ctctcctgtg cagcgtctgg attcaccttc agtagctatg gcatgcactg ggtccgccag     180

gctccaggca aggggctgga gtgggtggca gttatatcgt atgatggaag taataaatac     240

tatgcagact ccgtgaaggg ccgattcacc atctccagag acaattccaa gaacacgctg     300

tatctgcaaa tgaacagcct gagagccgag gacacggcgg tgtactactg cgtcaagggg     360

ccgttgcagg agccgccata cgattatgga atggacgtat ggggccaggg aacaactgtc     420

accgtctcct cagggtctac atccggctcc gggaagcccg gaagtggcga aggtagtaca     480

aagggggaaa tagtgatgac gcagtctcca gccaccctgt ctgtgtctcc aggggaaaga     540

gccaccctct cctgcagggc cagtcagagt gttagcagca acttagcctg gtaccagcag     600

aaacctggcc aggctcccag gctcctcatc tatagcgcat ccaccagggc cactggtatc     660

ccagccaggt tcagtggcag tgggtctggg acagagttca ctctcaccat cagcagcctg     720

cagtctgaag attttgcagt ttattactgt cagcagcacc acgtctggcc tctcactttt     780

ggcggaggga ccaaggttga gatcaaacgg gccgctgccc ttgataatga aaagtcaaac     840

ggaacaatca ttcacgtgaa gggcaagcac ctctgtccgt cacccttgtt ccctggtcca     900

tccaagccat tctgggtgtt ggtcgtagtg ggtggagtcc tcgcttgtta ctctctgctc     960

gtcaccgtgg cttttataat cttctgggtt agatccaaaa gaagccgcct gctccatagc    1020

gattacatga atatgactcc acgccgccct ggccccacaa ggaaacacta ccagccttac    1080

gcaccaccta gagatttcgc tgcctatcgg agcagggtga agttttccag atctgcagat    1140

gcaccagcgt atcagcaggg ccagaaccaa ctgtataacg agctcaacct gggacgcagg    1200

gaagagtatg acgttttgga caagcgcaga ggacgggacc ctgagatggg tggcaaacca    1260

agacgaaaaa acccccagga gggtctctat aatgagctgc agaaggataa gatggctgaa    1320

gcctattctg aaataggcat gaaaggagag cggagaaggg gaaaagggca cgacggtttg    1380

taccagggac tcagcactgc tacgaaggat acttatgacg ctctccacat gcaagccctg    1440

ccacctagg                                                            1449

<210> 157
<211> 483
<212> PRT
<213> Artificial Sequence

<220>
<221> source
<223> /note="Description of Artificial Sequence: Synthetic
      polypeptide"

<400> 157

Met Ala Leu Pro Val Thr Ala Leu Leu Leu Pro Leu Ala Leu Leu Leu
1               5                   10                  15

His Ala Ala Arg Pro Gln Val Gln Leu Val Glu Ser Gly Gly Gly Val
            20                  25                  30

Val Gln Pro Gly Arg Ser Leu Arg Leu Ser Cys Ala Ala Ser Gly Phe
        35                  40                  45

Thr Phe Ser Ser Tyr Gly Met His Trp Val Arg Gln Ala Pro Gly Lys
    50                  55                  60

Gly Leu Glu Trp Val Ala Val Ile Ser Tyr Asp Gly Ser Asn Lys Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Val Lys Gly Pro Leu Gln Glu Pro Pro Tyr Asp
        115                 120                 125

Tyr Gly Met Asp Val Trp Gly Gln Gly Thr Thr Val Thr Val Ser Ser
    130                 135                 140

Gly Ser Thr Ser Gly Ser Gly Lys Pro Gly Ser Gly Glu Gly Ser Thr
145                 150                 155                 160

Lys Gly Glu Ile Val Met Thr Gln Ser Pro Ala Thr Leu Ser Val Ser
                165                 170                 175

Pro Gly Glu Arg Ala Thr Leu Ser Cys Arg Ala Ser Gln Ser Val Ser
            180                 185                 190

Ser Asn Leu Ala Trp Tyr Gln Gln Lys Pro Gly Gln Ala Pro Arg Leu
        195                 200                 205

Leu Ile Tyr Ser Ala Ser Thr Arg Ala Thr Gly Ile Pro Ala Arg Phe
    210                 215                 220

Ser Gly Ser Gly Ser Gly Thr Glu Phe Thr Leu Thr Ile Ser Ser Leu
225                 230                 235                 240

Gln Ser Glu Asp Phe Ala Val Tyr Tyr Cys Gln Gln His His Val Trp
                245                 250                 255

Pro Leu Thr Phe Gly Gly Gly Thr Lys Val Glu Ile Lys Arg Ala Ala
            260                 265                 270

Ala Leu Asp Asn Glu Lys Ser Asn Gly Thr Ile Ile His Val Lys Gly
        275                 280                 285

Lys His Leu Cys Pro Ser Pro Leu Phe Pro Gly Pro Ser Lys Pro Phe
    290                 295                 300

Trp Val Leu Val Val Val Gly Gly Val Leu Ala Cys Tyr Ser Leu Leu
305                 310                 315                 320

Val Thr Val Ala Phe Ile Ile Phe Trp Val Arg Ser Lys Arg Ser Arg
                325                 330                 335

Leu Leu His Ser Asp Tyr Met Asn Met Thr Pro Arg Arg Pro Gly Pro
            340                 345                 350

Thr Arg Lys His Tyr Gln Pro Tyr Ala Pro Pro Arg Asp Phe Ala Ala
        355                 360                 365

Tyr Arg Ser Arg Val Lys Phe Ser Arg Ser Ala Asp Ala Pro Ala Tyr
    370                 375                 380

Gln Gln Gly Gln Asn Gln Leu Tyr Asn Glu Leu Asn Leu Gly Arg Arg
385                 390                 395                 400

Glu Glu Tyr Asp Val Leu Asp Lys Arg Arg Gly Arg Asp Pro Glu Met
                405                 410                 415

Gly Gly Lys Pro Arg Arg Lys Asn Pro Gln Glu Gly Leu Tyr Asn Glu
            420                 425                 430

Leu Gln Lys Asp Lys Met Ala Glu Ala Tyr Ser Glu Ile Gly Met Lys
        435                 440                 445

Gly Glu Arg Arg Arg Gly Lys Gly His Asp Gly Leu Tyr Gln Gly Leu
    450                 455                 460

Ser Thr Ala Thr Lys Asp Thr Tyr Asp Ala Leu His Met Gln Ala Leu
465                 470                 475                 480

Pro Pro Arg

<210> 158
<211> 1900

<212> DNA
<213> Artificial Sequence

<220>
<221> source
<223> /note="Description of Artificial Sequence: Synthetic
      polynucleotide"

<400> 158
atgggcacca gactgttctt ttatgtggcc ctgtgtctgc tgtggaccgg ccacatggat      60

gccggaatta cacagagccc cagacacaaa gtgaccgaga caggcacccc tgtgacactg     120

agatgccacc agaccgagaa ccaccgctac atgtactggt acagacagga tccaggccac     180

ggcctgagac tgatccacta cagctacggc gtgaaggaca ccgacaaggg cgaagtgtct     240

gacggctaca gcgtgtccag aagcaagacc gaggatttcc tgctgaccct ggaaagcgcc     300

acaagcagcc agaccagcgt gtacttttgt gccatcagcg gctacaagaa caccgaggcc     360

tttttcggcc aaggcaccag gctgacagtg gtggaagatc tgaagaacgt gttcccacct     420

gaggtggccg tgtttgagcc ttctgaggcc gagatcagcc acacacagaa agccacactc     480

gtgtgcctgg ccaccggctt ttatcccgat cacgtggaac tgtcttggtg ggtcaacggc     540

aaagaggtgc acagcggcgt tagcacagac cctcagcctc tgaaagagca gcccgctctg     600

aacgacagca gatactgtct gagcagcaga ctgagagtgt ccgccacctt ctggcagaac     660

cccagaaacc acttcagatg ccaggtgcag ttctacggcc tgtccgagaa tgacgagtgg     720

acccaggata gagccaagcc agtgacacag attgtgtctg ccgaagcctg gggcagagcc     780

gattgtggct ttacaagcga gagctaccag cagggcgtgc tgtctgccac aatcctgtat     840

gagatcctgc tgggcaaagc cactctgtac gctgtgctgg tgtctgccct ggtgctgatg     900

gccatggtca agagaaagga cagcagaggc agagccaaga gatctggcag cggcgccaca     960

aactttagcc tgctgaaaca ggccggcgac gtggaagaga accctggacc tatgaagtcc    1020

ctgcgggtgc tgctggttat tctgtggctg cagctgagct gggtttggag ccagggacag    1080

caagtgatgc agatccctca gtaccagcac gtgcaagaag gcgaggactt caccacctac    1140

tgcaacagca gcacaaccct gagcaacatc cagtggtaca agcagaggcc tggcgggcac    1200

cctgtgtttc tgatccagct ggttaagagc ggcgaagtga agaagcagaa gcggctgacc    1260

tttcagttcg gcgaggccaa gaagaacagc agcctgcaca ttaccgccac acagaccacc    1320

gacgtgggca catatttttg cgctggcaga gaaggcggca gcgagaagct ggtttttggc    1380

aagggcacca aactgaccgt gaatcccgac attcagaacc ccgatccagc cgtgtaccag    1440

ctgagagaca gcaagagcag cgacaagagc gtgtgtctgt tcaccgactt cgactcccag    1500

accaatgtgt cccagagcaa ggactccgac gtgtacatca ccgataagac cgtgctggac    1560

atgcggagca tggacttcaa gagcaatagc gccgtggctt ggagcaacaa gagcgacttt    1620

gcctgcgcca acgccttcaa caacagcatc atccccgagg acacattctt cccaagtcct     1680

gagagcagct gcgacgtgaa gctggtggaa aagagcttcg agacagacac caacctgaac     1740

ttccagaacc tgagcgtgat cggcttcaga atcctgctgc tgaaggtggc cggcttcaac     1800

ctgctgatga ctctgagact gtggtccagc tgaattcgga tccaagctta ggcctgctcg     1860

ctttcttgct gtcccatttc tattaaaggt tcctttgttc                           1900

<210> 159
<211> 610
<212> PRT
<213> Artificial Sequence

<220>
<221> source
<223> /note="Description of Artificial Sequence: Synthetic
      polypeptide"

<400> 159
Met Gly Thr Arg Leu Phe Phe Tyr Val Ala Leu Cys Leu Leu Trp Thr
1               5                   10                  15

Gly His Met Asp Ala Gly Ile Thr Gln Ser Pro Arg His Lys Val Thr
            20                  25                  30

Glu Thr Gly Thr Pro Val Thr Leu Arg Cys His Gln Thr Glu Asn His
        35                  40                  45

Arg Tyr Met Tyr Trp Tyr Arg Gln Asp Pro Gly His Gly Leu Arg Leu
    50                  55                  60

Ile His Tyr Ser Tyr Gly Val Lys Asp Thr Asp Lys Gly Glu Val Ser
65                  70                  75                  80

Asp Gly Tyr Ser Val Ser Arg Ser Lys Thr Glu Asp Phe Leu Leu Thr
                85                  90                  95

Leu Glu Ser Ala Thr Ser Ser Gln Thr Ser Val Tyr Phe Cys Ala Ile
            100                 105                 110

Ser Gly Tyr Lys Asn Thr Glu Ala Phe Phe Gly Gln Gly Thr Arg Leu
        115                 120                 125

Thr Val Val Glu Asp Leu Lys Asn Val Phe Pro Pro Glu Val Ala Val
    130                 135                 140

Phe Glu Pro Ser Glu Ala Glu Ile Ser His Thr Gln Lys Ala Thr Leu
145                 150                 155                 160

Val Cys Leu Ala Thr Gly Phe Tyr Pro Asp His Val Glu Leu Ser Trp

                165                 170                 175

Trp Val Asn Gly Lys Glu Val His Ser Gly Val Ser Thr Asp Pro Gln
            180                 185                 190

Pro Leu Lys Glu Gln Pro Ala Leu Asn Asp Ser Arg Tyr Cys Leu Ser
        195                 200                 205

Ser Arg Leu Arg Val Ser Ala Thr Phe Trp Gln Asn Pro Arg Asn His
    210                 215                 220

Phe Arg Cys Gln Val Gln Phe Tyr Gly Leu Ser Glu Asn Asp Glu Trp
225                 230                 235                 240

Thr Gln Asp Arg Ala Lys Pro Val Thr Gln Ile Val Ser Ala Glu Ala
                245                 250                 255

Trp Gly Arg Ala Asp Cys Gly Phe Thr Ser Glu Ser Tyr Gln Gln Gly
            260                 265                 270

Val Leu Ser Ala Thr Ile Leu Tyr Glu Ile Leu Leu Gly Lys Ala Thr
        275                 280                 285

Leu Tyr Ala Val Leu Val Ser Ala Leu Val Leu Met Ala Met Val Lys
    290                 295                 300

Arg Lys Asp Ser Arg Gly Arg Ala Lys Arg Ser Gly Ser Gly Ala Thr
305                 310                 315                 320

Asn Phe Ser Leu Leu Lys Gln Ala Gly Asp Val Glu Glu Asn Pro Gly
                325                 330                 335

Pro Met Lys Ser Leu Arg Val Leu Leu Val Ile Leu Trp Leu Gln Leu
            340                 345                 350

Ser Trp Val Trp Ser Gln Gly Gln Gln Val Met Gln Ile Pro Gln Tyr
        355                 360                 365

Gln His Val Gln Glu Gly Glu Asp Phe Thr Thr Tyr Cys Asn Ser Ser
    370                 375                 380

Thr Thr Leu Ser Asn Ile Gln Trp Tyr Lys Gln Arg Pro Gly Gly His
385                 390                 395                 400

Pro Val Phe Leu Ile Gln Leu Val Lys Ser Gly Glu Val Lys Lys Gln
                405                 410                 415

Lys Arg Leu Thr Phe Gln Phe Gly Glu Ala Lys Lys Asn Ser Ser Leu
            420                 425                 430

His Ile Thr Ala Thr Gln Thr Thr Asp Val Gly Thr Tyr Phe Cys Ala
        435                 440                 445

Gly Arg Glu Gly Gly Ser Glu Lys Leu Val Phe Gly Lys Gly Thr Lys
    450                 455                 460

Leu Thr Val Asn Pro Asp Ile Gln Asn Pro Asp Pro Ala Val Tyr Gln
465                 470                 475                 480

Leu Arg Asp Ser Lys Ser Ser Asp Lys Ser Val Cys Leu Phe Thr Asp
                485                 490                 495

Phe Asp Ser Gln Thr Asn Val Ser Gln Ser Lys Asp Ser Asp Val Tyr
            500                 505                 510

Ile Thr Asp Lys Thr Val Leu Asp Met Arg Ser Met Asp Phe Lys Ser
        515                 520                 525

Asn Ser Ala Val Ala Trp Ser Asn Lys Ser Asp Phe Ala Cys Ala Asn
    530                 535                 540

Ala Phe Asn Asn Ser Ile Ile Pro Glu Asp Thr Phe Phe Pro Ser Pro
545                 550                 555                 560

Glu Ser Ser Cys Asp Val Lys Leu Val Glu Lys Ser Phe Glu Thr Asp
                565                 570                 575

Thr Asn Leu Asn Phe Gln Asn Leu Ser Val Ile Gly Phe Arg Ile Leu
            580                 585                 590

Leu Leu Lys Val Ala Gly Phe Asn Leu Leu Met Thr Leu Arg Leu Trp
        595                 600                 605

Ser Ser
    610

<210> 160
<211> 1906
<212> DNA
<213> Artificial Sequence

<220>
<221> source
<223> /note="Description of Artificial Sequence: Synthetic
      polynucleotide"

<400> 160

atgggacctg gattgctttg ttgggccctg ctgtgtctgc ttggagctgg acttgtggat 60

gccggcgtga cacagtctcc cacacacctg atcaagacca gaggccagca agtgaccctg 120

agatgtagcc ctaagagcgg ccacgacacc gtgtcttggt atcagcaggc tcttggccag 180

ggacctcagt tcatcttcca gtactacgag gaagaggaac ggcagcgggg caacttccct 240

gatagattct ctggccatca gttccccaac tacagcagcg agctgaacgt gaacgctctg 300

ctgctgggcg atagcgccct gtatctgtgt gccagttctc ttggttggag aggcggcaga 360

tacaacgagc agttctttgg ccctggcacc agactgaccg tgctggaaga tctgaagaac 420

gtgttcccac ctgaggtggc cgtgtttgag ccttctgagg ccgagatcag ccacacacag 480

aaagccacac tcgtgtgcct ggccaccggc ttttatcccg atcacgtgga actgtcttgg 540

tgggtcaacg gcaaagaggt gcacagcggc gttagcacag accctcagcc tctgaaagag 600

cagcccgctc tgaacgacag cagatactgt ctgagcagca gactgagagt gtccgccacc 660

ttctggcaga accccagaaa ccacttcaga tgccaggtgc agttctacgg cctgtccgag 720

aatgacgagt ggacccagga tagagccaag ccagtgacac agattgtgtc tgccgaagcc 780

tggggcagag ccgattgtgg ctttacaagc gagagctacc agcagggcgt gctgtctgcc 840

acaatcctgt atgagatcct gctgggcaaa gccactctgt acgctgtgct ggtgtctgcc 900

ctggtgctga tggccatggt caagagaaag gacagcagag gcagagccaa gagatctggc 960

agcggcgcca caaactttag cctgctgaaa caggccggcg acgtggaaga gaaccctgga 1020

cctatgaagt ccctgcgggt gctgctggtt attctgtggc tgcagctgag ctgggtttgg 1080

agccagggac agaacatcga ccagcctacc gagatgacag ccaccgaagg cgccatcgtg 1140

cagatcaatt gcacctacca gaccagcggc ttcaacggcc tgttctggta tcaacagcat 1200

gccggcgagg cccctacctt cctgagctat aatgtgctgg acggcctgga agaaaagggc 1260

agattcagca gcttcctgtc cagaagcaag ggctacagct acctgctgct gaaagaactc 1320

cagatgaagg acagcgcctc ctacctgtgt gcctccgtgg atggaaacaa cagactggcc 1380

ttcggcaagg gcaaccaggt ggtggtcatc cccgacattc agaaccccga tccagccgtg 1440

taccagctga gagacagcaa gagcagcgac aagagcgtgt gtctgttcac cgacttcgac 1500

tcccagacca atgtgtccca gagcaaggac tccgacgtgt acatcaccga taagaccgtg 1560

ctggacatgc ggagcatgga cttcaagagc aatagcgccg tggcttggag caacaagagc 1620

gactttgcct gcgccaacgc cttcaacaac agcatcatcc ccgaggacac attcttccca 1680

agtcctgaga gcagctgcga cgtgaagctg gtggaaaaga gcttcgagac agacaccaac 1740

ctgaacttcc agaacctgag cgtgatcggc ttcagaatcc tgctgctgaa ggtggccggc 1800

ttcaacctgc tgatgactct gagactgtgg tccagctgaa ttcggatcca agcttaggcc 1860

tgctcgcttt cttgctgtcc catttctatt aaaggttcct ttgttc 1906

<210> 161
<211> 612
<212> PRT
<213> Artificial Sequence

<220>
<221> source
<223> /note="Description of Artificial Sequence: Synthetic
      polypeptide"

<400> 161
   Met Gly Pro Gly Leu Leu Cys Trp Ala Leu Leu Cys Leu Leu Gly Ala
   1               5                   10                  15
   Gly Leu Val Asp Ala Gly Val Thr Gln Ser Pro Thr His Leu Ile Lys
               20                  25                  30
   Thr Arg Gly Gln Gln Val Thr Leu Arg Cys Ser Pro Lys Ser Gly His
           35                  40                  45
   Asp Thr Val Ser Trp Tyr Gln Gln Ala Leu Gly Gln Gly Pro Gln Phe
       50                  55                  60
   Ile Phe Gln Tyr Tyr Glu Glu Glu Glu Arg Gln Arg Gly Asn Phe Pro
   65                  70                  75                  80
   Asp Arg Phe Ser Gly His Gln Phe Pro Asn Tyr Ser Ser Glu Leu Asn
                   85                  90                  95
   Val Asn Ala Leu Leu Leu Gly Asp Ser Ala Leu Tyr Leu Cys Ala Ser
               100                 105                 110
   Ser Leu Gly Trp Arg Gly Gly Arg Tyr Asn Glu Gln Phe Phe Gly Pro
           115                 120                 125
   Gly Thr Arg Leu Thr Val Leu Glu Asp Leu Lys Asn Val Phe Pro Pro
       130                 135                 140
   Glu Val Ala Val Phe Glu Pro Ser Glu Ala Glu Ile Ser His Thr Gln
   145                 150                 155                 160
   Lys Ala Thr Leu Val Cys Leu Ala Thr Gly Phe Tyr Pro Asp His Val
                   165                 170                 175
   Glu Leu Ser Trp Trp Val Asn Gly Lys Glu Val His Ser Gly Val Ser
               180                 185                 190
   Thr Asp Pro Gln Pro Leu Lys Glu Gln Pro Ala Leu Asn Asp Ser Arg
           195                 200                 205
   Tyr Cys Leu Ser Ser Arg Leu Arg Val Ser Ala Thr Phe Trp Gln Asn
       210                 215                 220
   Pro Arg Asn His Phe Arg Cys Gln Val Gln Phe Tyr Gly Leu Ser Glu
   225                 230                 235                 240
   Asn Asp Glu Trp Thr Gln Asp Arg Ala Lys Pro Val Thr Gln Ile Val
                   245                 250                 255
   Ser Ala Glu Ala Trp Gly Arg Ala Asp Cys Gly Phe Thr Ser Glu Ser
               260                 265                 270
   Tyr Gln Gln Gly Val Leu Ser Ala Thr Ile Leu Tyr Glu Ile Leu Leu
           275                 280                 285
   Gly Lys Ala Thr Leu Tyr Ala Val Leu Val Ser Ala Leu Val Leu Met
       290                 295                 300
   Ala Met Val Lys Arg Lys Asp Ser Arg Gly Arg Ala Lys Arg Ser Gly
   305                 310                 315                 320
   Ser Gly Ala Thr Asn Phe Ser Leu Leu Lys Gln Ala Gly Asp Val Glu
                   325                 330                 335
   Glu Asn Pro Gly Pro Met Lys Ser Leu Arg Val Leu Leu Val Ile Leu
               340                 345                 350
   Trp Leu Gln Leu Ser Trp Val Trp Ser Gln Gly Gln Asn Ile Asp Gln
           355                 360                 365
   Pro Thr Glu Met Thr Ala Thr Glu Gly Ala Ile Val Gln Ile Asn Cys
       370                 375                 380
   Thr Tyr Gln Thr Ser Gly Phe Asn Gly Leu Phe Trp Tyr Gln Gln His
   385                 390                 395                 400
   Ala Gly Glu Ala Pro Thr Phe Leu Ser Tyr Asn Val Leu Asp Gly Leu
                   405                 410                 415
   Glu Glu Lys Gly Arg Phe Ser Ser Phe Leu Ser Arg Ser Lys Gly Tyr
               420                 425                 430
   Ser Tyr Leu Leu Leu Lys Glu Leu Gln Met Lys Asp Ser Ala Ser Tyr
           435                 440                 445
   Leu Cys Ala Ser Val Asp Gly Asn Asn Arg Leu Ala Phe Gly Lys Gly
   450                 455                 460
   Asn Gln Val Val Val Ile Pro Asp Ile Gln Asn Pro Asp Pro Ala Val
   465                 470                 475                 480
   Tyr Gln Leu Arg Asp Ser Lys Ser Ser Asp Lys Ser Val Cys Leu Phe
                   485                 490                 495
   Thr Asp Phe Asp Ser Gln Thr Asn Val Ser Gln Ser Lys Asp Ser Asp
               500                 505                 510
   Val Tyr Ile Thr Asp Lys Thr Val Leu Asp Met Arg Ser Met Asp Phe
           515                 520                 525
   Lys Ser Asn Ser Ala Val Ala Trp Ser Asn Lys Ser Asp Phe Ala Cys
       530                 535                 540
   Ala Asn Ala Phe Asn Asn Ser Ile Ile Pro Glu Asp Thr Phe Phe Pro
   545                 550                 555                 560
   Ser Pro Glu Ser Ser Cys Asp Val Lys Leu Val Glu Lys Ser Phe Glu
                   565                 570                 575
   Thr Asp Thr Asn Leu Asn Phe Gln Asn Leu Ser Val Ile Gly Phe Arg
               580                 585                 590
   Ile Leu Leu Leu Lys Val Ala Gly Phe Asn Leu Leu Met Thr Leu Arg
           595                 600                 605
   Leu Trp Ser Ser
       610

<210> 162
<211> 66
<212> DNA
<213> Artificial Sequence

<220>
<221> source
<223> /note="Description of Artificial Sequence: Synthetic
      oligonucleotide"

<400> 162
atgcttctcc tggtgacaag ccttctgctc tgtgagttac cacacccagc attcctcctg      60

atccca                                                                  66

<210> 163
<211> 22
<212> PRT
<213> Artificial Sequence

<220>
<221> source
<223> /note="Description of Artificial Sequence: Synthetic
      peptide"

<400> 163
Met Leu Leu Leu Val Thr Ser Leu Leu Leu Cys Glu Leu Pro His Pro
1               5                   10                  15

Ala Phe Leu Leu Ile Pro
            20

<210> 164
<211> 321
<212> DNA
<213> Artificial Sequence

<220>
<221> source
<223> /note="Description of Artificial Sequence: Synthetic
      polynucleotide"

<400> 164
gacatccaga tgacacagac tacatcctcc ctgtctgcct ctctgggaga cagagtcacc      60

atcagttgca gggcaagtca ggacattagt aaatatttaa attggtatca gcagaaacca     120

gatggaactg ttaaactcct gatctaccat acatcaagat tacactcagg agtcccatca     180

aggttcagtg gcagtgggtc tggaacagat tattctctca ccattagcaa cctggagcaa     240

gaagatattg ccacttactt ttgccaacag ggtaatacgc ttccgtacac gttcggaggg     300

gggactaagt tggaaataac a                                               321

<210> 165
<211> 107
<212> PRT
<213> Artificial Sequence

<220>
<221> source
<223> /note="Description of Artificial Sequence: Synthetic
      polypeptide"

<400> 165
Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly

50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr
            100                 105

<210> 166
<211> 54
<212> DNA
<213> Artificial Sequence

<220>
<221> source
<223> /note="Description of Artificial Sequence: Synthetic
      oligonucleotide"

<400> 166
ggctccacct ctggatccgg caagcccgga tctggcgagg gatccaccaa gggc          54

<210> 167
<211> 18
<212> PRT
<213> Artificial Sequence

<220>
<221> source
<223> /note="Description of Artificial Sequence: Synthetic
      peptide"

<400> 167
Gly Ser Thr Ser Gly Ser Gly Lys Pro Gly Ser Gly Glu Gly Ser Thr
1               5                   10                  15

Lys Gly

<210> 168
<211> 360
<212> DNA
<213> Artificial Sequence

<220>
<221> source
<223> /note="Description of Artificial Sequence: Synthetic
      polynucleotide"

<400> 168
gaggtgaaac tgcaggagtc aggacctggc ctggtggcgc cctcacagag cctgtccgtc      60

acatgcactg tctcaggggt ctcattaccc gactatggtg taagctggat tcgccagcct     120

ccacgaaagg gtctggagtg gctgggagta atatggggta gtgaaaccac atactataat 180

tcagctctca aatccagact gaccatcatc aaggacaact ccaagagcca agttttctta 240

aaaatgaaca gtctgcaaac tgatgacaca gccatttact actgtgccaa acattattac 300

tacggtggta gctatgctat ggactactgg ggtcaaggaa cctcagtcac cgtctcctca 360

<210> 169
<211> 120
<212> PRT
<213> Artificial Sequence

<220>
<221> source
<223> /note="Description of Artificial Sequence: Synthetic
      polypeptide"

<400> 169
Glu Val Lys Leu Gln Glu Ser Gly Pro Gly Leu Val Ala Pro Ser Gln
1               5                   10                  15

Ser Leu Ser Val Thr Cys Thr Val Ser Gly Val Ser Leu Pro Asp Tyr
            20                  25                  30

Gly Val Ser Trp Ile Arg Gln Pro Pro Arg Lys Gly Leu Glu Trp Leu
        35                  40                  45

Gly Val Ile Trp Gly Ser Glu Thr Thr Tyr Tyr Asn Ser Ala Leu Lys
    50                  55                  60

Ser Arg Leu Thr Ile Ile Lys Asp Asn Ser Lys Ser Gln Val Phe Leu
65                  70                  75                  80

Lys Met Asn Ser Leu Gln Thr Asp Asp Thr Ala Ile Tyr Tyr Cys Ala
                85                  90                  95

Lys His Tyr Tyr Tyr Gly Gly Ser Tyr Ala Met Asp Tyr Trp Gly Gln
            100                 105                 110

Gly Thr Ser Val Thr Val Ser Ser
        115                 120

<210> 170
<211> 9
<212> DNA
<213> Artificial Sequence

<220>
<221> source
<223> /note="Description of Artificial Sequence: Synthetic
      oligonucleotide"

<400> 170

gcggccgca                                                              9


<210> 171
<211> 3
<212> PRT
<213> Artificial Sequence

<220>
<221> source
<223> /note="Description of Artificial Sequence: Synthetic
      peptide"

<400> 171
Ala Ala Ala
1


<210> 172
<211> 198
<212> DNA
<213> Artificial Sequence

<220>
<221> source
<223> /note="Description of Artificial Sequence: Synthetic
      polynucleotide"

<400> 172
attgaagtta tgtatcctcc tccttaccta gacaatgaga agagcaatgg aaccattatc      60

catgtgaaag ggaaacacct ttgtccaagt cccctatttc ccggaccttc taagcccttt     120

tgggtgctgg tggtggttgg gggagtcctg gcttgctata gcttgctagt aacagtggcc     180

tttattattt tctgggtg                                                   198


<210> 173
<211> 66
<212> PRT
<213> Artificial Sequence

<220>
<221> source
<223> /note="Description of Artificial Sequence: Synthetic
      polypeptide"

<400> 173
Ile Glu Val Met Tyr Pro Pro Pro Tyr Leu Asp Asn Glu Lys Ser Asn
1               5                   10                  15


Gly Thr Ile Ile His Val Lys Gly Lys His Leu Cys Pro Ser Pro Leu
            20                  25                  30


Phe Pro Gly Pro Ser Lys Pro Phe Trp Val Leu Val Val Val Gly Gly
        35                  40                  45


Val Leu Ala Cys Tyr Ser Leu Leu Val Thr Val Ala Phe Ile Ile Phe
    50                  55                  60

Trp Val
65

<210> 174
<211> 123
<212> DNA
<213> Artificial Sequence

<220>
<221> source
<223> /note="Description of Artificial Sequence: Synthetic
      polynucleotide"

<400> 174
aggagtaaga ggagcaggct cctgcacagt gactacatga acatgactcc ccgccgcccc      60

gggcccaccc gcaagcatta ccagccctat gccccaccac gcgacttcgc agcctatcgc     120

tcc                                                                   123

<210> 175
<211> 41
<212> PRT
<213> Artificial Sequence

<220>
<221> source
<223> /note="Description of Artificial Sequence: Synthetic
      polypeptide"

<400> 175
Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 176
<211> 336
<212> DNA
<213> Artificial Sequence

<220>
<221> source
<223> /note="Description of Artificial Sequence: Synthetic
      polynucleotide"

<400> 176
agagtgaagt tcagcaggag cgcagacgcc cccgcgtacc agcagggcca gaaccagctc      60

tataacgagc tcaatctagg acgaagagag gagtacgatg ttttggacaa gagacgtggc     120

cgggaccctg agatgggggg aaagccgaga aggaagaacc ctcaggaagg cctgtacaat     180

gaactgcaga aagataagat ggcggaggcc tacagtgaga ttgggatgaa aggcgagcgc 240

cggaggggca aggggcacga tggcctttac cagggtctca gtacagccac caaggacacc 300

tacgacgccc ttcacatgca ggccctgccc cctcgc 336

<210> 177
<211> 112
<212> PRT
<213> Artificial Sequence

<220>
<221> source
<223> /note="Description of Artificial Sequence: Synthetic
      polypeptide"

<400> 177
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210> 178
<211> 11
<212> PRT
<213> Artificial Sequence

<220>
<221> source
<223> /note="Description of Artificial Sequence: Synthetic
      peptide"

<400> 178
Arg Ala Ser Gln Asp Ile Ser Lys Tyr Leu Asn
1               5                   10

<210> 179

<211> 7
<212> PRT
<213> Artificial Sequence

<220>
<221> source
<223> /note="Description of Artificial Sequence: Synthetic
      peptide"

<400> 179
His Thr Ser Arg Leu His Ser
1               5


<210> 180
<211> 9
<212> PRT
<213> Artificial Sequence

<220>
<221> source
<223> /note="Description of Artificial Sequence: Synthetic
      peptide"

<400> 180
Gln Gln Gly Asn Thr Leu Pro Tyr Thr
1               5


<210> 181
<211> 7
<212> PRT
<213> Artificial Sequence

<220>
<221> source
<223> /note="Description of Artificial Sequence: Synthetic
      peptide"

<400> 181
Gly Val Ser Leu Pro Asp Tyr
1               5


<210> 182
<211> 5
<212> PRT
<213> Artificial Sequence

<220>
<221> source
<223> /note="Description of Artificial Sequence: Synthetic
      peptide"

<400> 182
Trp Gly Ser Glu Thr
1               5


<210> 183
<211> 12
<212> PRT
<213> Artificial Sequence

<220>
<221> source
<223> /note="Description of Artificial Sequence: Synthetic
      peptide"

<400> 183
His Tyr Tyr Tyr Gly Gly Ser Tyr Ala Met Asp Tyr
1               5                   10


<210> 184
<211> 11
<212> PRT
<213> Artificial Sequence

<220>
<221> source
<223> /note="Description of Artificial Sequence: Synthetic
      peptide"

<400> 184
Arg Ala Ser Gln Asp Ile Ser Lys Tyr Leu Asn
1               5                   10


<210> 185
<211> 7
<212> PRT
<213> Artificial Sequence

<220>
<221> source
<223> /note="Description of Artificial Sequence: Synthetic
      peptide"

<400> 185
His Thr Ser Arg Leu His Ser
1               5


<210> 186
<211> 9
<212> PRT
<213> Artificial Sequence

<220>
<221> source
<223> /note="Description of Artificial Sequence: Synthetic
      peptide"

<400> 186
Gln Gln Gly Asn Thr Leu Pro Tyr Thr
1               5


<210> 187
<211> 5
<212> PRT
<213> Artificial Sequence

<220>
<221> source

<223> /note="Description of Artificial Sequence: Synthetic
      peptide"

<400> 187
Asp Tyr Gly Val Ser
1               5


<210> 188
<211> 16
<212> PRT
<213> Artificial Sequence

<220>
<221> source
<223> /note="Description of Artificial Sequence: Synthetic
      peptide"

<400> 188
Val Ile Trp Gly Ser Glu Thr Thr Tyr Tyr Asn Ser Ala Leu Lys Ser
1               5                   10                  15


<210> 189
<211> 12
<212> PRT
<213> Artificial Sequence

<220>
<221> source
<223> /note="Description of Artificial Sequence: Synthetic
      peptide"

<400> 189
His Tyr Tyr Tyr Gly Gly Ser Tyr Ala Met Asp Tyr
1               5                   10


<210> 190
<211> 63
<212> DNA
<213> Artificial Sequence

<220>
<221> source
<223> /note="Description of Artificial Sequence: Synthetic
      oligonucleotide"

<400> 190
atggcactcc ccgtaactgc tctgctgctg ccgttggcat tgctcctgca cgccgcacgc      60

ccg                                                                    63


<210> 191
<211> 21
<212> PRT
<213> Artificial Sequence

<220>
<221> source
<223> /note="Description of Artificial Sequence: Synthetic
      peptide"

<400> 191
Met Ala Leu Pro Val Thr Ala Leu Leu Leu Pro Leu Ala Leu Leu Leu
1               5                   10                  15

His Ala Ala Arg Pro
            20

<210> 192
<211> 321
<212> DNA
<213> Artificial Sequence

<220>
<221> source
<223> /note="Description of Artificial Sequence: Synthetic
      polynucleotide"

<400> 192
gatattcaaa tgacccagtc cccgtcctcc ctgagtgcct ccgtcggtga ccgtgttacg       60

attacctgcc gtgcgagcca agacatctct aaatacctga actggtatca gcaaaaaccg      120

gatcaggcac cgaaactgct gatcaaacat acctcacgtc tgcactcggg tgtgccgagc      180

cgctttagtg gttccggctc aggtaccgat tacaccctga cgatcagctc tctgcagccg      240

gaagactttg ccacgtatta ctgccagcaa ggtaataccc tgccgtatac gttcggccaa      300

ggtaccaaac tggaaatcaa a                                                321

<210> 193
<211> 107
<212> PRT
<213> Artificial Sequence

<220>
<221> source
<223> /note="Description of Artificial Sequence: Synthetic
      polypeptide"

<400> 193
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Lys Tyr
            20                  25                  30

Leu Asn Trp Tyr Gln Gln Lys Pro Asp Gln Ala Pro Lys Leu Leu Ile
        35                  40                  45

Lys His Thr Ser Arg Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Tyr
                85                  90                  95

Thr Phe Gly Gln Gly Thr Lys Leu Glu Ile Lys
            100                 105

<210> 194
<211> 54
<212> DNA
<213> Artificial Sequence

<220>
<221> source
<223> /note="Description of Artificial Sequence: Synthetic
      oligonucleotide"

<400> 194
ggctcgacga gcggctctgg taaaccgggc tctggtgaag gcagtaccaa aggt          54

<210> 195
<211> 18
<212> PRT
<213> Artificial Sequence

<220>
<221> source
<223> /note="Description of Artificial Sequence: Synthetic
      peptide"

<400> 195
Gly Ser Thr Ser Gly Ser Gly Lys Pro Gly Ser Gly Glu Gly Ser Thr
1               5                   10                  15

Lys Gly

<210> 196
<211> 360
<212> DNA
<213> Artificial Sequence

<220>
<221> source
<223> /note="Description of Artificial Sequence: Synthetic
      polynucleotide"

<400> 196
gaagtgcagc tggttgaaag cggtggtggt ctggttcaac cgggtcgttc cctgcgtctg      60

tcatgtacgg cgagtggtgt ctccctgccg gactatggcg tgtcctggat tcgtcagccg     120

ccgggtaaag gcctggaatg gattggtgtc atctggggca gtgaaaccac gtattacaac     180

tcggccctga aaagccgttt caccatctct cgcgataaca gtaaaaatac gctgtacctg     240

cagatgaata gcctgcgcgc ggaagacacc gccgtttact actgcgcaaa acattactac     300

tacggtggca gctatgctat ggattactgg ggtcaaggca cgctggtcac cgtttcgtca     360

<210> 197
<211> 120
<212> PRT
<213> Artificial Sequence

<220>
<221> source
<223> /note="Description of Artificial Sequence: Synthetic
      polypeptide"

<400> 197
Glu Val Gln Leu Val Glu Ser Gly Gly Gly Leu Val Gln Pro Gly Arg
1               5                   10                  15

Ser Leu Arg Leu Ser Cys Thr Ala Ser Gly Val Ser Leu Pro Asp Tyr
            20                  25                  30

Gly Val Ser Trp Ile Arg Gln Pro Pro Gly Lys Gly Leu Glu Trp Ile
        35                  40                  45

Gly Val Ile Trp Gly Ser Glu Thr Thr Tyr Tyr Asn Ser Ala Leu Lys
    50                  55                  60

Ser Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys His Tyr Tyr Tyr Gly Gly Ser Tyr Ala Met Asp Tyr Trp Gly Gln
            100                 105                 110

Gly Thr Leu Val Thr Val Ser Ser
        115                 120


<210> 198
<211> 9
<212> DNA
<213> Artificial Sequence

<220>
<221> source
<223> /note="Description of Artificial Sequence: Synthetic
      oligonucleotide"

<400> 198
gccgctgcc                                                              9


<210> 199
<211> 3

<212> PRT
<213> Artificial Sequence

<220>
<221> source
<223> /note="Description of Artificial Sequence: Synthetic
      peptide"

<400> 199
Ala Ala Ala
1


<210> 200
<211> 171
<212> DNA
<213> Artificial Sequence

<220>
<221> source
<223> /note="Description of Artificial Sequence: Synthetic
      polynucleotide"

<400> 200
cttgataatg aaaagtcaaa cggaacaatc attcacgtga agggcaagca cctctgtccg       60

tcacccttgt tccctggtcc atccaagcca ttctgggtgt tggtcgtagt gggtggagtc      120

ctcgcttgtt actctctgct cgtcaccgtg gcttttataa tcttctgggt t               171


<210> 201
<211> 57
<212> PRT
<213> Artificial Sequence

<220>
<221> source
<223> /note="Description of Artificial Sequence: Synthetic
      polypeptide"

<400> 201
Leu Asp Asn Glu Lys Ser Asn Gly Thr Ile Ile His Val Lys Gly Lys
1               5                   10                  15


His Leu Cys Pro Ser Pro Leu Phe Pro Gly Pro Ser Lys Pro Phe Trp
            20                  25                  30


Val Leu Val Val Val Gly Gly Val Leu Ala Cys Tyr Ser Leu Leu Val
        35                  40                  45


Thr Val Ala Phe Ile Ile Phe Trp Val
    50                  55


<210> 202
<211> 123
<212> DNA
<213> Artificial Sequence

<220>
<221> source
<223> /note="Description of Artificial Sequence: Synthetic
      polynucleotide"

<400> 202
agatccaaaa gaagccgcct gctccatagc gattacatga atatgactcc acgccgccct      60

ggccccacaa ggaaacacta ccagccttac gcaccaccta gagatttcgc tgcctatcgg     120

agc                                                                   123


<210> 203
<211> 41
<212> PRT
<213> Artificial Sequence

<220>
<221> source
<223> /note="Description of Artificial Sequence: Synthetic
      polypeptide"

<400> 203
Arg Ser Lys Arg Ser Arg Leu Leu His Ser Asp Tyr Met Asn Met Thr
1               5                   10                  15


Pro Arg Arg Pro Gly Pro Thr Arg Lys His Tyr Gln Pro Tyr Ala Pro
            20                  25                  30


Pro Arg Asp Phe Ala Ala Tyr Arg Ser
        35                  40


<210> 204
<211> 336
<212> DNA
<213> Artificial Sequence

<220>
<221> source
<223> /note="Description of Artificial Sequence: Synthetic
      polynucleotide"

<400> 204
agggtgaagt tttccagatc tgcagatgca ccagcgtatc agcagggcca gaaccaactg      60

tataacgagc tcaacctggg acgcagggaa gagtatgacg ttttggacaa gcgcagagga     120

cgggaccctg agatgggtgg caaaccaaga cgaaaaaacc cccaggaggg tctctataat     180

gagctgcaga aggataagat ggctgaagcc tattctgaaa taggcatgaa aggagagcgg     240

agaaggggaa aagggcacga cggtttgtac cagggactca gcactgctac gaaggatact     300

tatgacgctc tccacatgca agccctgcca cctagg                               336


<210> 205
<211> 112
<212> PRT

<213> Artificial Sequence

<220>
<221> source
<223> /note="Description of Artificial Sequence: Synthetic
      polypeptide"

<400> 205
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110


<210> 206
<211> 12
<212> PRT
<213> Artificial Sequence

<220>
<221> source
<223> /note="Description of Artificial Sequence: Synthetic
      peptide"

<400> 206
Arg Ala Ser Gln Ser Val Ser Ser Ser Tyr Leu Ala
1               5                   10


<210> 207
<211> 7
<212> PRT
<213> Artificial Sequence

<220>
<221> source
<223> /note="Description of Artificial Sequence: Synthetic
      peptide"

<400> 207
Gly Ala Ser Ser Arg Ala Thr

1               5


<210> 208
<211> 9
<212> PRT
<213> Artificial Sequence

<220>
<221> source
<223> /note="Description of Artificial Sequence: Synthetic
      peptide"

<400> 208
Gln Gln Tyr Gly Ser Ser Arg Phe Thr
1               5


<210> 209
<211> 7
<212> PRT
<213> Artificial Sequence

<220>
<221> source
<223> /note="Description of Artificial Sequence: Synthetic
      peptide"

<400> 209
Gly Gly Thr Phe Ser Ser Tyr
1               5


<210> 210
<211> 6
<212> PRT
<213> Artificial Sequence

<220>
<221> source
<223> /note="Description of Artificial Sequence: Synthetic
      peptide"

<400> 210
Ile Pro Ile Phe Gly Thr
1               5


<210> 211
<211> 10
<212> PRT
<213> Artificial Sequence

<220>
<221> source
<223> /note="Description of Artificial Sequence: Synthetic
      peptide"

<400> 211
Glu Ala Val Ala Ala Asp Trp Leu Asp Pro
1               5                   10

<210> 212
<211> 12
<212> PRT
<213> Artificial Sequence

<220>
<221> source
<223> /note="Description of Artificial Sequence: Synthetic
      peptide"

<400> 212
Arg Ala Ser Gln Ser Val Ser Ser Ser Tyr Leu Ala
1               5                   10


<210> 213
<211> 7
<212> PRT
<213> Artificial Sequence

<220>
<221> source
<223> /note="Description of Artificial Sequence: Synthetic
      peptide"

<400> 213
Gly Ala Ser Ser Arg Ala Thr
1               5


<210> 214
<211> 9
<212> PRT
<213> Artificial Sequence

<220>
<221> source
<223> /note="Description of Artificial Sequence: Synthetic
      peptide"

<400> 214
Gln Gln Tyr Gly Ser Ser Arg Phe Thr
1               5


<210> 215
<211> 5
<212> PRT
<213> Artificial Sequence

<220>
<221> source
<223> /note="Description of Artificial Sequence: Synthetic
      peptide"

<400> 215
Ser Tyr Ala Ile Ser
1               5


<210> 216
<211> 17
<212> PRT

<213> Artificial Sequence

<220>
<221> source
<223> /note="Description of Artificial Sequence: Synthetic
      peptide"

<400> 216
Gly Ile Ile Pro Ile Phe Gly Thr Thr Asn Tyr Ala Gln Gln Phe Gln
1               5                   10                  15

Gly

<210> 217
<211> 10
<212> PRT
<213> Artificial Sequence

<220>
<221> source
<223> /note="Description of Artificial Sequence: Synthetic
      peptide"

<400> 217
Glu Ala Val Ala Ala Asp Trp Leu Asp Pro
1               5                   10

<210> 218
<211> 63
<212> DNA
<213> Artificial Sequence

<220>
<221> source
<223> /note="Description of Artificial Sequence: Synthetic
      oligonucleotide"

<400> 218
atggccctgc ctgtgacagc tctgctgctg cccctggccc tgctgctgca tgccgccaga      60

cct                                                                    63

<210> 219
<211> 21
<212> PRT
<213> Artificial Sequence

<220>
<221> source
<223> /note="Description of Artificial Sequence: Synthetic
      peptide"

<400> 219
Met Ala Leu Pro Val Thr Ala Leu Leu Leu Pro Leu Ala Leu Leu Leu
1               5                   10                  15

His Ala Ala Arg Pro

            20

<210> 220
<211> 324
<212> DNA
<213> Artificial Sequence

<220>
<221> source
<223> /note="Description of Artificial Sequence: Synthetic
      polynucleotide"

<400> 220
gagatcgtgc tgacccagtc tcccggcacc ctgtctctca gcccaggaga gagagccacc      60

ctgagctgca gagccagcca gagcgtgtcc agcagctacc tggcctggta tcagcagaag     120

cccggacagg cccccagact gctgatctac ggcgccagct ctagagccac cggcatcccc     180

gacagattca gcggcagcgg cagtggcacc gacttcaccc tgaccatcag cagactggaa     240

cccgaggact tcgccgtgta ctactgccag cagtacggca gcagccggtt caccttcggc     300

cctggcacca aggtggacat caag                                            324


<210> 221
<211> 108
<212> PRT
<213> Artificial Sequence

<220>
<221> source
<223> /note="Description of Artificial Sequence: Synthetic
      polypeptide"

<400> 221
Glu Ile Val Leu Thr Gln Ser Pro Gly Thr Leu Ser Leu Ser Pro Gly
1               5                   10                  15


Glu Arg Ala Thr Leu Ser Cys Arg Ala Ser Gln Ser Val Ser Ser Ser
            20                  25                  30


Tyr Leu Ala Trp Tyr Gln Gln Lys Pro Gly Gln Ala Pro Arg Leu Leu
        35                  40                  45


Ile Tyr Gly Ala Ser Ser Arg Ala Thr Gly Ile Pro Asp Arg Phe Ser
    50                  55                  60


Gly Ser Gly Ser Gly Thr Asp Phe Thr Leu Thr Ile Ser Arg Leu Glu
65                  70                  75                  80


Pro Glu Asp Phe Ala Val Tyr Tyr Cys Gln Gln Tyr Gly Ser Ser Arg
                85                  90                  95


Phe Thr Phe Gly Pro Gly Thr Lys Val Asp Ile Lys

            100                 105

<210> 222
<211> 54
<212> DNA
<213> Artificial Sequence

<220>
<221> source
<223> /note="Description of Artificial Sequence: Synthetic
      oligonucleotide"

<400> 222
ggcagcacct ccggcagcgg caagcctggc tctggcgagg gctctaccaa gggc 54

<210> 223
<211> 18
<212> PRT
<213> Artificial Sequence

<220>
<221> source
<223> /note="Description of Artificial Sequence: Synthetic
      peptide"

<400> 223
Gly Ser Thr Ser Gly Ser Gly Lys Pro Gly Ser Gly Glu Gly Ser Thr
1               5                   10                  15

Lys Gly

<210> 224
<211> 357
<212> DNA
<213> Artificial Sequence

<220>
<221> source
<223> /note="Description of Artificial Sequence: Synthetic
      polynucleotide"

<400> 224
caggtgcagc tggtgcagtc tggcgccgaa gtgaagaaac ccggctctag cgtgaaggtg 60

tcctgcaagg acagcggcgg caccttcagc agctacgcca tcagctgggt gcgccaggcc 120

ccaggacagg ggctggaatg gatgggcggc atcatcccca tcttcggcac caccaactac 180

gcccagcagt tccagggcag agtgaccatc accgccgacg agagcaccag caccgcctac 240

atggaactga gcagcctgcg gagcgaggac acagccgtgt attactgtgc ccgcgaggcc 300

gtggccgccg actggctgga tccttgggga cagggcaccc tggtgacagt gtccagc 357

<210> 225
<211> 119
<212> PRT

<213> Artificial Sequence

<220>
<221> source
<223> /note="Description of Artificial Sequence: Synthetic
      polypeptide"

<400> 225
Gln Val Gln Leu Val Gln Ser Gly Ala Glu Val Lys Lys Pro Gly Ser
1               5                   10                  15

Ser Val Lys Val Ser Cys Lys Asp Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Thr Asn Tyr Ala Gln Gln Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Ala Val Ala Ala Asp Trp Leu Asp Pro Trp Gly Gln Gly
            100                 105                 110

Thr Leu Val Thr Val Ser Ser
        115


<210> 226
<211> 249
<212> DNA
<213> Artificial Sequence

<220>
<221> source
<223> /note="Description of Artificial Sequence: Synthetic
      polynucleotide"

<400> 226
ttcgtgcccg tgttcctgcc cgccaagcct accaccaccc ctgcccctag acctcccacc      60

ccagccccaa caatcgccag ccagcctctg tccctgcggc ccgaagcctg tagacctgct     120

gccggcggag ccgtgcacac cagaggcctg gacttcgcct gcgatatcta catctgggcc     180

cctctggccg gcacctgtgg cgtgctgctg ctgagcctgg tgatcaccct gtactgcaac     240

caccggaac                                                             249


<210> 227

<211> 83
<212> PRT
<213> Artificial Sequence

<220>
<221> source
<223> /note="Description of Artificial Sequence: Synthetic
      polypeptide"

<400> 227
Phe Val Pro Val Phe Leu Pro Ala Lys Pro Thr Thr Thr Pro Ala Pro
1               5                   10                  15

Arg Pro Pro Thr Pro Ala Pro Thr Ile Ala Ser Gln Pro Leu Ser Leu
            20                  25                  30

Arg Pro Glu Ala Cys Arg Pro Ala Ala Gly Gly Ala Val His Thr Arg
        35                  40                  45

Gly Leu Asp Phe Ala Cys Asp Ile Tyr Ile Trp Ala Pro Leu Ala Gly
    50                  55                  60

Thr Cys Gly Val Leu Leu Leu Ser Leu Val Ile Thr Leu Tyr Cys Asn
65                  70                  75                  80

His Arg Asn

<210> 228
<211> 123
<212> DNA
<213> Artificial Sequence

<220>
<221> source
<223> /note="Description of Artificial Sequence: Synthetic
      polynucleotide"

<400> 228
agaagcaagc ggagccggct gctgcacagc gactacatga acatgacccc aagacggcct      60

ggccccaccc ggaagcacta ccagccttac gcccctccca gagacttcgc cgcctaccgg     120

tcc                                                                   123

<210> 229
<211> 41
<212> PRT
<213> Artificial Sequence

<220>
<221> source
<223> /note="Description of Artificial Sequence: Synthetic
      polypeptide"

<400> 229

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 230
<211> 336
<212> DNA
<213> Artificial Sequence

<220>
<221> source
<223> /note="Description of Artificial Sequence: Synthetic
      polynucleotide"

<400> 230
agagtgaagt tcagcagatc cgccgacgcc cctgcctacc agcagggaca gaaccagctg      60

tacaacgagc tgaacctggg cagacgggaa gagtacgacg tgctggacaa gcggagaggc     120

cgggaccccg agatgggcgg aaagcccaga cggaagaacc cccaggaagg cctgtataac     180

gaactgcaga aagacaagat ggccgaggcc tacagcgaga tcggcatgaa gggcgagcgg     240

aggcgcggca agggccacga tggcctgtac cagggcctga gcaccgccac caaggacacc     300

tacgacgccc tgcacatgca ggccctgccc cccaga                               336

<210> 231
<211> 112
<212> PRT
<213> Artificial Sequence

<220>
<221> source
<223> /note="Description of Artificial Sequence: Synthetic
      polypeptide"

<400> 231
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210> 232
<211> 339
<212> DNA
<213> Artificial Sequence

<220>
<221> source
<223> /note="Description of Artificial Sequence: Synthetic
      polynucleotide"

<400> 232
atggcatgcc ctggcttcct gtgggcactt gtgatctcca cctgtcttga atttagcatg      60

gctcagacag tcactcagtc tcaaccagag atgtctgtgc aggaggcaga gaccgtgacc     120

ctgagctgca catatgacac cagtgagagt gattattatt tattctggta caagcagcct     180

cccagcaggc agatgattct cgttattcgc caagaagctt ataagcaaca gaatgcaaca     240

gagaatcgtt tctctgtgaa cttccagaaa gcagccaaat ccttcagtct caagatctca     300

gactcacagc tgggggatgc cgcgatgtat ttctgtgct                            339

<210> 233
<211> 135
<212> PRT
<213> Artificial Sequence

<220>
<221> source
<223> /note="Description of Artificial Sequence: Synthetic
      polypeptide"

<400> 233
Met Ala Cys Pro Gly Phe Leu Trp Ala Leu Val Ile Ser Thr Cys Leu
1               5                   10                  15

Glu Phe Ser Met Ala Gln Thr Val Thr Gln Ser Gln Pro Glu Met Ser
            20                  25                  30

Val Gln Glu Ala Glu Thr Val Thr Leu Ser Cys Thr Tyr Asp Thr Ser
        35                  40                  45

Glu Ser Asp Tyr Tyr Leu Phe Trp Tyr Lys Gln Pro Pro Ser Arg Gln
    50                  55                  60

Met Ile Leu Val Ile Arg Gln Glu Ala Tyr Lys Gln Gln Asn Ala Thr
65                  70                  75                  80

Glu Asn Arg Phe Ser Val Asn Phe Gln Lys Ala Ala Lys Ser Phe Ser
                85                  90                  95

Leu Lys Ile Ser Asp Ser Gln Leu Gly Asp Ala Ala Met Tyr Phe Cys
            100                 105                 110

Ala Leu Arg Ser Ser Gly Thr Tyr Lys Tyr Ile Phe Gly Thr Gly Thr
        115                 120                 125

Arg Leu Lys Val Leu Ala Asn
    130                 135

<210> 234
<211> 66
<212> DNA
<213> Artificial Sequence

<220>
<221> source
<223> /note="Description of Artificial Sequence: Synthetic
      oligonucleotide"

<400> 234
ctccggagct caggaaccta caaatacatc tttggaacag gcaccaggct gaaggtttta      60

gcaaat                                                                  66

<210> 235
<211> 408
<212> DNA
<213> Artificial Sequence

<220>
<221> source
<223> /note="Description of Artificial Sequence: Synthetic
      polynucleotide"

<400> 235
atccagaacc ctgaacctgc tgtgtaccag ttaaaagatc ctcggtctca ggacagcacc      60

ctctgcctgt tcaccgactt tgactcccaa atcaatgtgc cgaaaaccat ggaatctgga     120

acgttcatca ctgacaaaac tgtgctggac atgaaagcta tggattccaa gagcaatggg     180

gccattgcct ggagcaacca gacaagcttc acctgccaag atatcttcaa agagaccaac     240

gccacctacc ccagttcaga cgttccctgt gatgccacgt tgactgagaa aagctttgaa     300

acagatatga acctaaactt tcaaaacctg tcagttatgg gactccgaat cctcctgctg     360

aaagtagccg gatttaacct gctcatgacg ctgaggctgt ggtccagt                  408

<210> 236

<211> 136
<212> PRT
<213> Artificial Sequence

<220>
<221> source
<223> /note="Description of Artificial Sequence: Synthetic
      polypeptide"

<400> 236
Ile Gln Asn Pro Glu Pro Ala Val Tyr Gln Leu Lys Asp Pro Arg Ser
1               5                   10                  15

Gln Asp Ser Thr Leu Cys Leu Phe Thr Asp Phe Asp Ser Gln Ile Asn
            20                  25                  30

Val Pro Lys Thr Met Glu Ser Gly Thr Phe Ile Thr Asp Lys Thr Val
        35                  40                  45

Leu Asp Met Lys Ala Met Asp Ser Lys Ser Asn Gly Ala Ile Ala Trp
    50                  55                  60

Ser Asn Gln Thr Ser Phe Thr Cys Gln Asp Ile Phe Lys Glu Thr Asn
65                  70                  75                  80

Ala Thr Tyr Pro Ser Ser Asp Val Pro Cys Asp Ala Thr Leu Thr Glu
                85                  90                  95

Lys Ser Phe Glu Thr Asp Met Asn Leu Asn Phe Gln Asn Leu Ser Val
            100                 105                 110

Met Gly Leu Arg Ile Leu Leu Leu Lys Val Ala Gly Phe Asn Leu Leu
        115                 120                 125

Met Thr Leu Arg Leu Trp Ser Ser
    130                 135


<210> 237
<211> 81
<212> DNA
<213> Artificial Sequence

<220>
<221> source
<223> /note="Description of Artificial Sequence: Synthetic
      oligonucleotide"

<400> 237
cgggccaagc ggtccggatc cggagccacc aacttcagcc tgctgaagca ggccggcgac      60

gtggaggaga accccggccc c                                                81


<210> 238

<211> 27
<212> PRT
<213> Artificial Sequence

<220>
<221> source
<223> /note="Description of Artificial Sequence: Synthetic
      peptide"

<400> 238
Arg Ala Lys Arg Ser Gly Ser Gly Ala Thr Asn Phe Ser Leu Leu Lys
1               5                   10                  15

Gln Ala Gly Asp Val Glu Glu Asn Pro Gly Pro
            20                  25

<210> 239
<211> 340
<212> DNA
<213> Artificial Sequence

<220>
<221> source
<223> /note="Description of Artificial Sequence: Synthetic
      polynucleotide"

<400> 239
atgggcacca ggctcctctt ctgggtggcc ttctgtctcc tgggggcaga tcacacagga      60

gctggagtct cccagtcccc cagtaacaag gtcacagaga agggaaagga tgtagagctc     120

aggtgtgatc caatttcagg tcatactgcc ctttactggt accgacagag cctggggcag     180

ggcctggagt ttttaattta cttccaaggc aacagtgcac cagacaaatc agggctgccc     240

agtgatcgct tctctgcaga gaggactggg ggatccgtct ccactctgac gatccagcgc     300

acacagcagg aggactcggc cgtgtatctc tgtgccagca                           340

<210> 240
<211> 137
<212> PRT
<213> Artificial Sequence

<220>
<221> source
<223> /note="Description of Artificial Sequence: Synthetic
      polypeptide"

<400> 240
Met Gly Thr Arg Leu Leu Phe Trp Val Ala Phe Cys Leu Leu Gly Ala
1               5                   10                  15

Asp His Thr Gly Ala Gly Val Ser Gln Ser Pro Ser Asn Lys Val Thr
            20                  25                  30

Glu Lys Gly Lys Asp Val Glu Leu Arg Cys Asp Pro Ile Ser Gly His
        35                  40                  45

Thr Ala Leu Tyr Trp Tyr Arg Gln Ser Leu Gly Gln Gly Leu Glu Phe
    50                  55                  60

Leu Ile Tyr Phe Gln Gly Asn Ser Ala Pro Asp Lys Ser Gly Leu Pro
65                  70                  75                  80

Ser Asp Arg Phe Ser Ala Glu Arg Thr Gly Gly Ser Val Ser Thr Leu
                85                  90                  95

Thr Ile Gln Arg Thr Gln Gln Glu Asp Ser Ala Val Tyr Leu Cys Ala
            100                 105                 110

Ser Ile Arg Thr Gly Pro Phe Phe Ser Gly Asn Thr Ile Tyr Phe Gly
        115                 120                 125

Glu Gly Ser Trp Leu Thr Val Val Glu
    130                 135

<210> 241
<211> 71
<212> DNA
<213> Artificial Sequence

<220>
<221> source
<223> /note="Description of Artificial Sequence: Synthetic
      oligonucleotide"

<400> 241
tccggacagg gccttttttc tctggaaaca ccatatattt tggagaggga agttggctca      60

ctgttgtaga g                                                          71

<210> 242
<211> 519
<212> DNA
<213> Artificial Sequence

<220>
<221> source
<223> /note="Description of Artificial Sequence: Synthetic
      polynucleotide"

<400> 242
gacctgagaa acgtgacccc acccaaggtc tccttgtttg agccatcaaa agcagagatt      60

gcaaacaaac aaaaggctac cctcgtgtgc ttggccaggg gcttcttccc tgaccacgtg     120

gagctgagct ggtgggtgaa tggcaaggag gtccacagtg gggtcagcac ggaccctcag     180

gcctacaagg agagcaatta tagctactgc ctgagcagcc gcctgagggt ctctgctacc     240

ttctggcaca atcctcgaaa ccacttccgc tgccaagtgc agttccatgg gctttcagag     300

gaggacaagt ggccagaggg ctcacccaaa cctgtcacac agaacatcag tgcagaggcc 360

tggggccgag cagactgtgg aatcacttca gcatcctatc atcagggggt tctgtctgca 420

accatcctct atgagatcct actggggaag gccaccctat atgctgtgct ggtcagtggc 480

ctggtgctga tggctatggt caaaagaaag aactcatga 519

<210> 243
<211> 172
<212> PRT
<213> Artificial Sequence

<220>
<221> source
<223> /note="Description of Artificial Sequence: Synthetic
      polypeptide"

<400> 243
Asp Leu Arg Asn Val Thr Pro Pro Lys Val Ser Leu Phe Glu Pro Ser
1               5                   10                  15

Lys Ala Glu Ile Ala Asn Lys Gln Lys Ala Thr Leu Val Cys Leu Ala
            20                  25                  30

Arg Gly Phe Phe Pro Asp His Val Glu Leu Ser Trp Trp Val Asn Gly
        35                  40                  45

Lys Glu Val His Ser Gly Val Ser Thr Asp Pro Gln Ala Tyr Lys Glu
    50                  55                  60

Ser Asn Tyr Ser Tyr Cys Leu Ser Ser Arg Leu Arg Val Ser Ala Thr
65                  70                  75                  80

Phe Trp His Asn Pro Arg Asn His Phe Arg Cys Gln Val Gln Phe His
                85                  90                  95

Gly Leu Ser Glu Glu Asp Lys Trp Pro Glu Gly Ser Pro Lys Pro Val
            100                 105                 110

Thr Gln Asn Ile Ser Ala Glu Ala Trp Gly Arg Ala Asp Cys Gly Ile
        115                 120                 125

Thr Ser Ala Ser Tyr His Gln Gly Val Leu Ser Ala Thr Ile Leu Tyr
    130                 135                 140

Glu Ile Leu Leu Gly Lys Ala Thr Leu Tyr Ala Val Leu Val Ser Gly
145                 150                 155                 160

Leu Val Leu Met Ala Met Val Lys Arg Lys Asn Ser
                165                 170

<210> 244
<211> 12
<212> PRT
<213> Artificial Sequence

<220>
<221> source
<223> /note="Description of Artificial Sequence: Synthetic
      peptide"

<400> 244
Thr Tyr Asp Thr Ser Glu Ser Asp Tyr Tyr Leu Phe
1               5                   10


<210> 245
<211> 7
<212> PRT
<213> Artificial Sequence

<220>
<221> source
<223> /note="Description of Artificial Sequence: Synthetic
      peptide"

<400> 245
Gln Glu Ala Tyr Lys Gln Gln
1               5


<210> 246
<211> 11
<212> PRT
<213> Artificial Sequence

<220>
<221> source
<223> /note="Description of Artificial Sequence: Synthetic
      peptide"

<400> 246
Ala Leu Arg Ser Ser Gly Thr Tyr Lys Tyr Ile
1               5                   10


<210> 247
<211> 10
<212> PRT
<213> Artificial Sequence

<220>
<221> source
<223> /note="Description of Artificial Sequence: Synthetic
      peptide"

<400> 247
Asp Pro Ile Ser Gly His Thr Ala Leu Tyr
1               5                   10


<210> 248
<211> 11

<212> PRT
<213> Artificial Sequence

<220>
<221> source
<223> /note="Description of Artificial Sequence: Synthetic
      peptide"

<400> 248
Phe Gln Gly Asn Ser Ala Pro Asp Lys Ser Gly
1               5                   10


<210> 249
<211> 15
<212> PRT
<213> Artificial Sequence

<220>
<221> source
<223> /note="Description of Artificial Sequence: Synthetic
      peptide"

<400> 249
Ala Ser Ile Arg Thr Gly Pro Phe Phe Ser Gly Asn Thr Ile Tyr
1               5                   10                  15


<210> 250
<211> 63
<212> DNA
<213> Artificial Sequence

<220>
<221> source
<223> /note="Description of Artificial Sequence: Synthetic
      oligonucleotide"

<400> 250
atggcactcc ccgtaactgc tctgctgctg ccgttggcat tgctcctgca cgccgcacgc      60

ccg                                                                    63


<210> 251
<211> 21
<212> PRT
<213> Artificial Sequence

<220>
<221> source
<223> /note="Description of Artificial Sequence: Synthetic
      peptide"

<400> 251
Met Ala Leu Pro Val Thr Ala Leu Leu Leu Pro Leu Ala Leu Leu Leu
1               5                   10                  15


His Ala Ala Arg Pro
            20

<210> 252
<211> 366
<212> DNA
<213> Artificial Sequence

<220>
<221> source
<223> /note="Description of Artificial Sequence: Synthetic
      polynucleotide"

<400> 252
caggtccaac tgcaagaaag cggacccgga ctggtgaagc cttctgagac acttagtctg      60

acgtgcacgg tcagtggcgg ctccatctcc tcctattatt ggtcatggat acgacaaccc     120

ccaggtaagg gcctggaatg gattggctat atctactatt caggaagcac gaactacaat     180

cccagcctga agtcccgagt gacaatttca gtagatacca gtaaaaacca gttcagtctt     240

aaactgtcaa gcgtgacagc tgccgacacc gctgtgtatt actgcgtctc actggtgtat     300

tgtggagggg attgttatag cgggttcgat tattggggac agggaaccct ggtgactgta     360

tcttcc                                                                366


<210> 253
<211> 122
<212> PRT
<213> Artificial Sequence

<220>
<221> source
<223> /note="Description of Artificial Sequence: Synthetic
      polypeptide"

<400> 253
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Val
                85                  90                  95

Ser Leu Val Tyr Cys Gly Gly Asp Cys Tyr Ser Gly Phe Asp Tyr Trp
            100                 105                 110

Gly Gln Gly Thr Leu Val Thr Val Ser Ser
        115                 120

<210> 254
<211> 45
<212> DNA
<213> Artificial Sequence

<220>
<221> source
<223> /note="Description of Artificial Sequence: Synthetic
      oligonucleotide"

<400> 254
ggcggcggcg gctcaggggg tggcggtagt ggcggtgggg gttcc                45

<210> 255
<211> 15
<212> PRT
<213> Artificial Sequence

<220>
<221> source
<223> /note="Description of Artificial Sequence: Synthetic
      peptide"

<400> 255
Gly Gly Gly Gly Ser Gly Gly Gly Gly Ser Gly Gly Gly Gly Ser
1               5                   10                  15

<210> 256
<211> 324
<212> DNA
<213> Artificial Sequence

<220>
<221> source
<223> /note="Description of Artificial Sequence: Synthetic
      polynucleotide"

<400> 256
gatattcaac tgacacaatc ccccagctca ctcagcgcca gcgtggggga cagggttagc      60

tttacctgtc aagcctctca ggatataaat aactttctga actggtatca acagaagcct     120

gggaaggcgc ccaaactcct gatctatgat gcgtccaacc tggaaactgg cgtgccttca     180

cgctttagcg gctctggcag tggtacagac ttcactttta ccatctcttc acttcagccg     240

gaggacatcg ccacatatta ctgtcaacag tacggaaact tgccctttac ttttggaggc     300

ggcaccaaag ttgaaatcaa aagg                                            324

<210> 257
<211> 108
<212> PRT
<213> Artificial Sequence

<220>
<221> source
<223> /note="Description of Artificial Sequence: Synthetic
      polypeptide"

<400> 257
Asp Ile Gln Leu Thr Gln Ser Pro Ser Ser Leu Ser Ala Ser Val Gly
1               5                   10                  15

Asp Arg Val Ser Phe Thr Cys Gln Ala Ser Gln Asp Ile Asn Asn Phe
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Gly Asn Leu Pro Phe
                85                  90                  95

Thr Phe Gly Gly Gly Thr Lys Val Glu Ile Lys Arg
            100                 105

<210> 258
<211> 9
<212> DNA
<213> Artificial Sequence

<220>
<221> source
<223> /note="Description of Artificial Sequence: Synthetic
      oligonucleotide"

<400> 258
gccgctgcc                                                              9

<210> 259
<211> 3
<212> PRT
<213> Artificial Sequence

<220>
<221> source
<223> /note="Description of Artificial Sequence: Synthetic
      peptide"

<400> 259
Ala Ala Ala
1

<210> 260
<211> 171
<212> DNA
<213> Artificial Sequence

<220>
<221> source
<223> /note="Description of Artificial Sequence: Synthetic
      polynucleotide"

<400> 260
ctggataacg aaaagagcaa tgggactata atacatgtta aaggaaaaca cctgtgtcca      60

tctcccctgt tccctggacc gtcaaagcca ttttgggtgc tcgtggttgt cggtggcgtt     120

ctcgcctgtt atagcttgct ggtgacagta gccttcatta tcttttgggt g              171


<210> 261
<211> 57
<212> PRT
<213> Artificial Sequence

<220>
<221> source
<223> /note="Description of Artificial Sequence: Synthetic
      polypeptide"

<400> 261
Leu Asp Asn Glu Lys Ser Asn Gly Thr Ile Ile His Val Lys Gly Lys
1               5                   10                  15


His Leu Cys Pro Ser Pro Leu Phe Pro Gly Pro Ser Lys Pro Phe Trp
            20                  25                  30


Val Leu Val Val Val Gly Gly Val Leu Ala Cys Tyr Ser Leu Leu Val
        35                  40                  45


Thr Val Ala Phe Ile Ile Phe Trp Val
    50                  55


<210> 262
<211> 123
<212> DNA
<213> Artificial Sequence

<220>
<221> source
<223> /note="Description of Artificial Sequence: Synthetic
      polynucleotide"

<400> 262
agatccaaaa gaagccgcct gctccatagc gattacatga atatgactcc acgccgccct      60

ggccccacaa ggaaacacta ccagccttac gcaccaccta gagatttcgc tgcctatcgg     120

agc                                                                   123

<210> 263
<211> 41
<212> PRT
<213> Artificial Sequence

<220>
<221> source
<223> /note="Description of Artificial Sequence: Synthetic
      polypeptide"

<400> 263
Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 264
<211> 336
<212> DNA
<213> Artificial Sequence

<220>
<221> source
<223> /note="Description of Artificial Sequence: Synthetic
      polynucleotide"

<400> 264
cgagtgaaat tttctagatc agctgatgct cccgcctatc agcagggaca gaatcaactt      60

tacaatgagc tgaacctggg tcgcagagaa gagtacgacg ttttggacaa acgccggggc     120

cgagatcctg agatgggggg gaagccgaga aggaagaatc ctcaagaagg cctgtacaac     180

gagcttcaaa aagacaaaat ggctgaggcg tactctgaga tcggcatgaa gggcgagcgg     240

agacgaggca agggtcacga tggcttgtat cagggcctga gtacagccac aaaggacacc     300

tatgacgccc tccacatgca ggcactgccc ccacgc                               336

<210> 265
<211> 112
<212> PRT
<213> Artificial Sequence

<220>
<221> source
<223> /note="Description of Artificial Sequence: Synthetic
      polypeptide"

<400> 265
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210> 266
<211> 11
<212> PRT
<213> Artificial Sequence

<220>
<221> source
<223> /note="Description of Artificial Sequence: Synthetic
      peptide"

<400> 266
Gln Ala Ser Gln Asp Ile Asn Asn Phe Leu Asn
1               5                   10

<210> 267
<211> 7
<212> PRT
<213> Artificial Sequence

<220>
<221> source
<223> /note="Description of Artificial Sequence: Synthetic
      peptide"

<400> 267
Asp Ala Ser Asn Leu Glu Thr
1               5

<210> 268
<211> 9
<212> PRT
<213> Artificial Sequence

<220>
<221> source
<223> /note="Description of Artificial Sequence: Synthetic
      peptide"

<400> 268
Gln Gln Tyr Gly Asn Leu Pro Phe Thr
1               5


<210> 269
<211> 7
<212> PRT
<213> Artificial Sequence

<220>
<221> source
<223> /note="Description of Artificial Sequence: Synthetic
      peptide"

<400> 269
Gly Gly Ser Ile Ser Ser Tyr
1               5


<210> 270
<211> 5
<212> PRT
<213> Artificial Sequence

<220>
<221> source
<223> /note="Description of Artificial Sequence: Synthetic
      peptide"

<400> 270
Tyr Tyr Ser Gly Ser
1               5


<210> 271
<211> 14
<212> PRT
<213> Artificial Sequence

<220>
<221> source
<223> /note="Description of Artificial Sequence: Synthetic
      peptide"

<400> 271
Leu Val Tyr Cys Gly Gly Asp Cys Tyr Ser Gly Phe Asp Tyr
1               5                   10


<210> 272
<211> 63
<212> DNA
<213> Artificial Sequence

<220>
<221> source
<223> /note="Description of Artificial Sequence: Synthetic
      oligonucleotide"

<400> 272
atggcactcc ccgtaactgc tctgctgctg ccgttggcat tgctcctgca cgccgcacgc        60

ccg                                                                    63

<210> 273
<211> 21
<212> PRT
<213> Artificial Sequence

<220>
<221> source
<223> /note="Description of Artificial Sequence: Synthetic
      peptide"

<400> 273
Met Ala Leu Pro Val Thr Ala Leu Leu Leu Pro Leu Ala Leu Leu Leu
1               5                   10                  15

His Ala Ala Arg Pro
            20

<210> 274
<211> 369
<212> DNA
<213> Artificial Sequence

<220>
<221> source
<223> /note="Description of Artificial Sequence: Synthetic
      polynucleotide"

<400> 274
caggtgcagc tggtggagtc tgggggaggc gtggtccagc ctgggaggtc cctgagactc      60

tcctgtgcag cgtctggatt caccttcagt agctatggca tgcactgggt ccgccaggct     120

ccaggcaagg ggctggagtg ggtggcagtt atatcgtatg atggaagtaa taaatactat     180

gcagactccg tgaagggccg attcaccatc tccagagaca attccaagaa cacgctgtat     240

ctgcaaatga acagcctgag agccgaggac acggcggtgt actactgcgt caaggggccg     300

ttgcaggagc cgccatacga ttatggaatg gacgtatggg gccagggaac aactgtcacc     360

gtctcctca                                                             369

<210> 275
<211> 123
<212> PRT
<213> Artificial Sequence

<220>
<221> source
<223> /note="Description of Artificial Sequence: Synthetic
      polypeptide"

<400> 275
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Lys Gly Pro Leu Gln Glu Pro Pro Tyr Asp Tyr Gly Met Asp Val
            100                 105                 110

Trp Gly Gln Gly Thr Thr Val Thr Val Ser Ser
        115                 120

<210> 276
<211> 54
<212> DNA
<213> Artificial Sequence

<220>
<221> source
<223> /note="Description of Artificial Sequence: Synthetic
      oligonucleotide"

<400> 276
gggtctacat ccggctccgg gaagcccgga agtggcgaag gtagtacaaa gggg          54

<210> 277
<211> 18
<212> PRT
<213> Artificial Sequence

<220>
<221> source
<223> /note="Description of Artificial Sequence: Synthetic
      peptide"

<400> 277
Gly Ser Thr Ser Gly Ser Gly Lys Pro Gly Ser Gly Glu Gly Ser Thr
1               5                   10                  15

Lys Gly

<210> 278

<211> 324
<212> DNA
<213> Artificial Sequence

<220>
<221> source
<223> /note="Description of Artificial Sequence: Synthetic
      polynucleotide"

<400> 278
gaaatagtga tgacgcagtc tccagccacc ctgtctgtgt ctccagggga aagagccacc      60

ctctcctgca gggccagtca gagtgttagc agcaacttag cctggtacca gcagaaacct     120

ggccaggctc ccaggctcct catctatagc gcatccacca gggccactgg tatcccagcc     180

aggttcagtg gcagtgggtc tgggacagag ttcactctca ccatcagcag cctgcagtct     240

gaagattttg cagtttatta ctgtcagcag caccacgtct ggcctctcac ttttggcgga     300

gggaccaagg ttgagatcaa acgg                                            324


<210> 279
<211> 108
<212> PRT
<213> Artificial Sequence

<220>
<221> source
<223> /note="Description of Artificial Sequence: Synthetic
      polypeptide"

<400> 279
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Tyr Ser Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln His His Val Trp Pro Leu
                85                  90                  95

Thr Phe Gly Gly Gly Thr Lys Val Glu Ile Lys Arg
            100                 105


<210> 280

<211> 9
<212> DNA
<213> Artificial Sequence

<220>
<221> source
<223> /note="Description of Artificial Sequence: Synthetic
      oligonucleotide"

<400> 280
gccgctgcc                                                              9


<210> 281
<211> 3
<212> PRT
<213> Artificial Sequence

<220>
<221> source
<223> /note="Description of Artificial Sequence: Synthetic
      peptide"

<400> 281
Ala Ala Ala
1


<210> 282
<211> 171
<212> DNA
<213> Artificial Sequence

<220>
<221> source
<223> /note="Description of Artificial Sequence: Synthetic
      polynucleotide"

<400> 282
cttgataatg aaaagtcaaa cggaacaatc attcacgtga agggcaagca cctctgtccg      60

tcacccttgt tccctggtcc atccaagcca ttctgggtgt tggtcgtagt gggtggagtc     120

ctcgcttgtt actctctgct cgtcaccgtg gcttttataa tcttctgggt t              171


<210> 283
<211> 57
<212> PRT
<213> Artificial Sequence

<220>
<221> source
<223> /note="Description of Artificial Sequence: Synthetic
      polypeptide"

<400> 283
Leu Asp Asn Glu Lys Ser Asn Gly Thr Ile Ile His Val Lys Gly Lys
1               5                   10                  15


His Leu Cys Pro Ser Pro Leu Phe Pro Gly Pro Ser Lys Pro Phe Trp
            20                  25                  30

Val Leu Val Val Val Gly Gly Val Leu Ala Cys Tyr Ser Leu Leu Val
        35                  40                  45

Thr Val Ala Phe Ile Ile Phe Trp Val
    50                  55

<210> 284
<211> 123
<212> DNA
<213> Artificial Sequence

<220>
<221> source
<223> /note="Description of Artificial Sequence: Synthetic
      polynucleotide"

<400> 284
agatccaaaa gaagccgcct gctccatagc gattacatga atatgactcc acgccgccct      60

ggccccacaa ggaaacacta ccagccttac gcaccaccta gagatttcgc tgcctatcgg     120

agc                                                                   123

<210> 285
<211> 41
<212> PRT
<213> Artificial Sequence

<220>
<221> source
<223> /note="Description of Artificial Sequence: Synthetic
      polypeptide"

<400> 285
Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 286
<211> 336
<212> DNA
<213> Artificial Sequence

<220>
<221> source
<223> /note="Description of Artificial Sequence: Synthetic
      polynucleotide"

<400> 286
agggtgaagt tttccagatc tgcagatgca ccagcgtatc agcagggcca gaaccaactg      60

tataacgagc tcaacctggg acgcagggaa gagtatgacg ttttggacaa gcgcagagga     120

cgggaccctg agatgggtgg caaaccaaga cgaaaaaacc cccaggaggg tctctataat     180

gagctgcaga aggataagat ggctgaagcc tattctgaaa taggcatgaa aggagagcgg     240

agaaggggaa aagggcacga cggtttgtac cagggactca gcactgctac gaaggatact     300

tatgacgctc tccacatgca agccctgcca cctagg                               336

<210> 287
<211> 112
<212> PRT
<213> Artificial Sequence

<220>
<221> source
<223> /note="Description of Artificial Sequence: Synthetic
      polypeptide"

<400> 287
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210> 288
<211> 11
<212> PRT
<213> Artificial Sequence

<220>
<221> source
<223> /note="Description of Artificial Sequence: Synthetic
      peptide"

<400> 288
Arg Ala Ser Gln Ser Val Ser Ser Asn Leu Ala

1               5                   10


<210> 289
<211> 7
<212> PRT
<213> Artificial Sequence

<220>
<221> source
<223> /note="Description of Artificial Sequence: Synthetic
      peptide"

<400> 289
Ser Ala Ser Thr Arg Ala Thr
1               5


<210> 290
<211> 10
<212> PRT
<213> Artificial Sequence

<220>
<221> source
<223> /note="Description of Artificial Sequence: Synthetic
      peptide"

<400> 290
Gln Gln His His Val Trp Pro Leu Thr Phe
1               5                   10


<210> 291
<211> 7
<212> PRT
<213> Artificial Sequence

<220>
<221> source
<223> /note="Description of Artificial Sequence: Synthetic
      peptide"

<400> 291
Gly Phe Thr Phe Ser Ser Tyr
1               5


<210> 292
<211> 17
<212> PRT
<213> Artificial Sequence

<220>
<221> source
<223> /note="Description of Artificial Sequence: Synthetic
      peptide"

<400> 292
Val Ile Ser Tyr Asp Gly Ser Asn Lys Tyr Tyr Ala Asp Ser Val Lys
1               5                   10                  15

Gly

<210> 293
<211> 16
<212> PRT
<213> Artificial Sequence

<220>
<221> source
<223> /note="Description of Artificial Sequence: Synthetic
      peptide"

<400> 293
Val Lys Gly Pro Leu Gln Glu Pro Pro Tyr Asp Tyr Gly Met Asp Val
1               5                   10                  15

<210> 294
<211> 393
<212> DNA
<213> Artificial Sequence

<220>
<221> source
<223> /note="Description of Artificial Sequence: Synthetic
      polynucleotide"

<400> 294
atgggcacca gactgttctt ttatgtggcc ctgtgtctgc tgtggaccgg ccacatggat      60

gccggaatta cacagagccc cagacacaaa gtgaccgaga caggcacccc tgtgacactg     120

agatgccacc agaccgagaa ccaccgctac atgtactggt acagacagga tccaggccac     180

ggcctgagac tgatccacta cagctacggc gtgaaggaca ccgacaaggg cgaagtgtct     240

gacggctaca gcgtgtccag aagcaagacc gaggatttcc tgctgaccct ggaaagcgcc     300

acaagcagcc agaccagcgt gtacttttgt gccatcagcg gctacaagaa caccgaggcc     360

tttttcggcc aaggcaccag gctgacagtg gtg                                  393

<210> 295
<211> 131
<212> PRT
<213> Artificial Sequence

<220>
<221> source
<223> /note="Description of Artificial Sequence: Synthetic
      polypeptide"

<400> 295
Met Gly Thr Arg Leu Phe Phe Tyr Val Ala Leu Cys Leu Leu Trp Thr
1               5                   10                  15

Gly His Met Asp Ala Gly Ile Thr Gln Ser Pro Arg His Lys Val Thr
            20                  25                  30

Glu Thr Gly Thr Pro Val Thr Leu Arg Cys His Gln Thr Glu Asn His
        35                  40                  45

Arg Tyr Met Tyr Trp Tyr Arg Gln Asp Pro Gly His Gly Leu Arg Leu
    50                  55                  60

Ile His Tyr Ser Tyr Gly Val Lys Asp Thr Asp Lys Gly Glu Val Ser
65                  70                  75                  80

Asp Gly Tyr Ser Val Ser Arg Ser Lys Thr Glu Asp Phe Leu Leu Thr
                85                  90                  95

Leu Glu Ser Ala Thr Ser Ser Gln Thr Ser Val Tyr Phe Cys Ala Ile
            100                 105                 110

Ser Gly Tyr Lys Asn Thr Glu Ala Phe Phe Gly Gln Gly Thr Arg Leu
        115                 120                 125

Thr Val Val
    130

<210> 296
<211> 537
<212> DNA
<213> Artificial Sequence

<220>
<221> source
<223> /note="Description of Artificial Sequence: Synthetic
      polynucleotide"

<400> 296
gaagatctga agaacgtgtt cccacctgag gtggccgtgt ttgagccttc tgaggccgag      60

atcagccaca cacagaaagc cacactcgtg tgcctggcca ccggctttta tcccgatcac     120

gtggaactgt cttggtgggt caacggcaaa gaggtgcaca gcggcgttag cacagaccct    180

cagcctctga aagagcagcc cgctctgaac gacagcagat actgtctgag cagcagactg     240

agagtgtccg ccaccttctg gcagaacccc agaaaccact tcagatgcca ggtgcagttc     300

tacggcctgt ccgagaatga cgagtggacc caggatagag ccaagccagt gacacagatt     360

gtgtctgccg aagcctgggg cagagccgat tgtggcttta caagcgagag ctaccagcag     420

ggcgtgctgt ctgccacaat cctgtatgag atcctgctgg gcaaagccac tctgtacgct     480

gtgctggtgt ctgccctggt gctgatggcc atggtcaaga gaaaggacag cagaggc        537

<210> 297
<211> 179
<212> PRT
<213> Artificial Sequence

<220>
<221> source
<223> /note="Description of Artificial Sequence: Synthetic
      polypeptide"

<400> 297
Glu Asp Leu Lys Asn Val Phe Pro Pro Glu Val Ala Val Phe Glu Pro
1               5                   10                  15

Ser Glu Ala Glu Ile Ser His Thr Gln Lys Ala Thr Leu Val Cys Leu
            20                  25                  30

Ala Thr Gly Phe Tyr Pro Asp His Val Glu Leu Ser Trp Trp Val Asn
        35                  40                  45

Gly Lys Glu Val His Ser Gly Val Ser Thr Asp Pro Gln Pro Leu Lys
    50                  55                  60

Glu Gln Pro Ala Leu Asn Asp Ser Arg Tyr Cys Leu Ser Ser Arg Leu
65                  70                  75                  80

Arg Val Ser Ala Thr Phe Trp Gln Asn Pro Arg Asn His Phe Arg Cys
                85                  90                  95

Gln Val Gln Phe Tyr Gly Leu Ser Glu Asn Asp Glu Trp Thr Gln Asp
            100                 105                 110

Arg Ala Lys Pro Val Thr Gln Ile Val Ser Ala Glu Ala Trp Gly Arg
        115                 120                 125

Ala Asp Cys Gly Phe Thr Ser Glu Ser Tyr Gln Gln Gly Val Leu Ser
    130                 135                 140

Ala Thr Ile Leu Tyr Glu Ile Leu Leu Gly Lys Ala Thr Leu Tyr Ala
145                 150                 155                 160

Val Leu Val Ser Ala Leu Val Leu Met Ala Met Val Lys Arg Lys Asp
                165                 170                 175

Ser Arg Gly

<210> 298
<211> 81
<212> DNA
<213> Artificial Sequence

<220>
<221> source
<223> /note="Description of Artificial Sequence: Synthetic

oligonucleotide"

<400> 298
agagccaaga gatctggcag cggcgccaca aactttagcc tgctgaaaca ggccggcgac 60

gtggaagaga accctggacc t 81


<210> 299
<211> 27
<212> PRT
<213> Artificial Sequence

<220>
<221> source
<223> /note="Description of Artificial Sequence: Synthetic
      peptide"

<400> 299
Arg Ala Lys Arg Ser Gly Ser Gly Ala Thr Asn Phe Ser Leu Leu Lys
1               5                   10                  15


Gln Ala Gly Asp Val Glu Glu Asn Pro Gly Pro
            20                  25


<210> 300
<211> 396
<212> DNA
<213> Artificial Sequence

<220>
<221> source
<223> /note="Description of Artificial Sequence: Synthetic
      polynucleotide"

<400> 300
atgaagtccc tgcgggtgct gctggttatt ctgtggctgc agctgagctg ggtttggagc 60

cagggacagc aagtgatgca gatccctcag taccagcacg tgcaagaagg cgaggacttc 120

accacctact gcaacagcag cacaaccctg agcaacatcc agtggtacaa gcagaggcct 180

ggcgggcacc ctgtgtttct gatccagctg gttaagagcg gcgaagtgaa gaagcagaag 240

cggctgacct ttcagttcgg cgaggccaag aagaacagca gcctgcacat taccgccaca 300

cagaccaccg acgtgggcac atatttttgc gctggcagag aaggcggcag cgagaagctg 360

gtttttggca agggcaccaa actgaccgtg aatccc 396


<210> 301
<211> 132
<212> PRT
<213> Artificial Sequence

<220>
<221> source
<223> /note="Description of Artificial Sequence: Synthetic
      polypeptide"

<400> 301
Met Lys Ser Leu Arg Val Leu Leu Val Ile Leu Trp Leu Gln Leu Ser
1               5                   10                  15

Trp Val Trp Ser Gln Gly Gln Gln Val Met Gln Ile Pro Gln Tyr Gln
            20                  25                  30

His Val Gln Glu Gly Glu Asp Phe Thr Thr Tyr Cys Asn Ser Ser Thr
        35                  40                  45

Thr Leu Ser Asn Ile Gln Trp Tyr Lys Gln Arg Pro Gly Gly His Pro
    50                  55                  60

Val Phe Leu Ile Gln Leu Val Lys Ser Gly Glu Val Lys Lys Gln Lys
65                  70                  75                  80

Arg Leu Thr Phe Gln Phe Gly Glu Ala Lys Lys Asn Ser Ser Leu His
                85                  90                  95

Ile Thr Ala Thr Gln Thr Thr Asp Val Gly Thr Tyr Phe Cys Ala Gly
            100                 105                 110

Arg Glu Gly Gly Ser Glu Lys Leu Val Phe Gly Lys Gly Thr Lys Leu
        115                 120                 125

Thr Val Asn Pro
    130

<210> 302
<211> 493
<212> DNA
<213> Artificial Sequence

<220>
<221> source
<223> /note="Description of Artificial Sequence: Synthetic
      polynucleotide"

<400> 302
gacattcaga accccgatcc agccgtgtac cagctgagag acagcaagag cagcgacaag      60

agcgtgtgtc tgttcaccga cttcgactcc cagaccaatg tgtcccagag caaggactcc     120

gacgtgtaca tcaccgataa gaccgtgctg gacatgcgga gcatggactt caagagcaat     180

agcgccgtgg cttggagcaa caagagcgac tttgcctgcg ccaacgcctt caacaacagc     240

atcatccccg aggacacatt cttcccaagt cctgagagca gctgcgacgt gaagctggtg     300

gaaaagagct tcgagacaga caccaacctg aacttccaga acctgagcgt gatcggcttc     360

agaatcctgc tgctgaaggt ggccggcttc aacctgctga tgactctgag actgtggtcc     420

agctgaattc ggatccaagc ttaggcctgc tcgctttctt gctgtcccat ttctattaaa     480

ggttcctttg ttc                                                        493

<210> 303
<211> 141
<212> PRT
<213> Artificial Sequence

<220>
<221> source
<223> /note="Description of Artificial Sequence: Synthetic
      polypeptide"

<400> 303
Asp Ile Gln Asn Pro Asp Pro Ala Val Tyr Gln Leu Arg Asp Ser Lys
1               5                   10                  15

Ser Ser Asp Lys Ser Val Cys Leu Phe Thr Asp Phe Asp Ser Gln Thr
            20                  25                  30

Asn Val Ser Gln Ser Lys Asp Ser Asp Val Tyr Ile Thr Asp Lys Thr
        35                  40                  45

Val Leu Asp Met Arg Ser Met Asp Phe Lys Ser Asn Ser Ala Val Ala
    50                  55                  60

Trp Ser Asn Lys Ser Asp Phe Ala Cys Ala Asn Ala Phe Asn Asn Ser
65                  70                  75                  80

Ile Ile Pro Glu Asp Thr Phe Phe Pro Ser Pro Glu Ser Ser Cys Asp
                85                  90                  95

Val Lys Leu Val Glu Lys Ser Phe Glu Thr Asp Thr Asn Leu Asn Phe
            100                 105                 110

Gln Asn Leu Ser Val Ile Gly Phe Arg Ile Leu Leu Leu Lys Val Ala
        115                 120                 125

Gly Phe Asn Leu Leu Met Thr Leu Arg Leu Trp Ser Ser
    130                 135                 140

<210> 304
<211> 5
<212> PRT
<213> Artificial Sequence

<220>
<221> source
<223> /note="Description of Artificial Sequence: Synthetic
      peptide"

<400> 304
Thr Thr Leu Ser Asn

1               5


<210> 305
<211> 7
<212> PRT
<213> Artificial Sequence

<220>
<221> source
<223> /note="Description of Artificial Sequence: Synthetic
      peptide"

<400> 305
Leu Val Lys Ser Gly Glu Val
1               5


<210> 306
<211> 11
<212> PRT
<213> Artificial Sequence

<220>
<221> source
<223> /note="Description of Artificial Sequence: Synthetic
      peptide"

<400> 306
Ala Gly Arg Glu Gly Gly Ser Glu Lys Leu Val
1               5                   10


<210> 307
<211> 5
<212> PRT
<213> Artificial Sequence

<220>
<221> source
<223> /note="Description of Artificial Sequence: Synthetic
      peptide"

<400> 307
Glu Asn His Arg Tyr
1               5


<210> 308
<211> 6
<212> PRT
<213> Artificial Sequence

<220>
<221> source
<223> /note="Description of Artificial Sequence: Synthetic
      peptide"

<400> 308
Ser Tyr Gly Val Lys Asp
1               5

<210> 309
<211> 11
<212> PRT
<213> Artificial Sequence

<220>
<221> source
<223> /note="Description of Artificial Sequence: Synthetic
      peptide"

<400> 309
Ala Ile Ser Gly Tyr Lys Asn Thr Glu Ala Phe
1               5                   10


<210> 310
<211> 405
<212> DNA
<213> Artificial Sequence

<220>
<221> source
<223> /note="Description of Artificial Sequence: Synthetic
      polynucleotide"

<400> 310
atgggacctg gattgctttg ttgggccctg ctgtgtctgc ttggagctgg acttgtggat      60

gccggcgtga cacagtctcc cacacacctg atcaagacca gaggccagca agtgaccctg     120

agatgtagcc ctaagagcgg ccacgacacc gtgtcttggt atcagcaggc tcttggccag     180

ggacctcagt tcatcttcca gtactacgag gaagaggaac ggcagcgggg caacttccct     240

gatagattct ctggccatca gttccccaac tacagcagcg agctgaacgt gaacgctctg     300

ctgctgggcg atagcgccct gtatctgtgt gccagttctc ttggttggag aggcggcaga     360

tacaacgagc agttctttgg ccctggcacc agactgaccg tgctg                     405


<210> 311
<211> 135
<212> PRT
<213> Artificial Sequence

<220>
<221> source
<223> /note="Description of Artificial Sequence: Synthetic
      polypeptide"

<400> 311
Met Gly Pro Gly Leu Leu Cys Trp Ala Leu Leu Cys Leu Leu Gly Ala
1               5                   10                  15


Gly Leu Val Asp Ala Gly Val Thr Gln Ser Pro Thr His Leu Ile Lys
            20                  25                  30


Thr Arg Gly Gln Gln Val Thr Leu Arg Cys Ser Pro Lys Ser Gly His
        35                  40                  45

Asp Thr Val Ser Trp Tyr Gln Gln Ala Leu Gly Gln Gly Pro Gln Phe
    50                  55                  60

Ile Phe Gln Tyr Tyr Glu Glu Glu Glu Arg Gln Arg Gly Asn Phe Pro
65                  70                  75                  80

Asp Arg Phe Ser Gly His Gln Phe Pro Asn Tyr Ser Ser Glu Leu Asn
                85                  90                  95

Val Asn Ala Leu Leu Leu Gly Asp Ser Ala Leu Tyr Leu Cys Ala Ser
            100                 105                 110

Ser Leu Gly Trp Arg Gly Gly Arg Tyr Asn Glu Gln Phe Phe Gly Pro
        115                 120                 125

Gly Thr Arg Leu Thr Val Leu
    130                 135

<210> 312
<211> 537
<212> DNA
<213> Artificial Sequence

<220>
<221> source
<223> /note="Description of Artificial Sequence: Synthetic
      polynucleotide"

<400> 312
gaagatctga agaacgtgtt cccacctgag gtggccgtgt ttgagccttc tgaggccgag      60

atcagccaca cacagaaagc cacactcgtg tgcctggcca ccggctttta tcccgatcac     120

gtggaactgt cttggtgggt caacggcaaa gaggtgcaca gcggcgttag cacagaccct     180

cagcctctga aagagcagcc cgctctgaac gacagcagat actgtctgag cagcagactg     240

agagtgtccg ccaccttctg gcagaacccc agaaaccact tcagatgcca ggtgcagttc     300

tacggcctgt ccgagaatga cgagtggacc caggatagag ccaagccagt gacacagatt     360

gtgtctgccg aagcctgggg cagagccgat tgtggcttta caagcgagag ctaccagcag     420

ggcgtgctgt ctgccacaat cctgtatgag atcctgctgg gcaaagccac tctgtacgct     480

gtgctggtgt ctgccctggt gctgatggcc atggtcaaga gaaaggacag cagaggc        537

<210> 313
<211> 179
<212> PRT
<213> Artificial Sequence

<220>
<221> source
<223> /note="Description of Artificial Sequence: Synthetic

polypeptide"

<400> 313
Glu Asp Leu Lys Asn Val Phe Pro Pro Glu Val Ala Val Phe Glu Pro
1               5                   10                  15

Ser Glu Ala Glu Ile Ser His Thr Gln Lys Ala Thr Leu Val Cys Leu
            20                  25                  30

Ala Thr Gly Phe Tyr Pro Asp His Val Glu Leu Ser Trp Trp Val Asn
        35                  40                  45

Gly Lys Glu Val His Ser Gly Val Ser Thr Asp Pro Gln Pro Leu Lys
    50                  55                  60

Glu Gln Pro Ala Leu Asn Asp Ser Arg Tyr Cys Leu Ser Ser Arg Leu
65                  70                  75                  80

Arg Val Ser Ala Thr Phe Trp Gln Asn Pro Arg Asn His Phe Arg Cys
                85                  90                  95

Gln Val Gln Phe Tyr Gly Leu Ser Glu Asn Asp Glu Trp Thr Gln Asp
            100                 105                 110

Arg Ala Lys Pro Val Thr Gln Ile Val Ser Ala Glu Ala Trp Gly Arg
        115                 120                 125

Ala Asp Cys Gly Phe Thr Ser Glu Ser Tyr Gln Gln Gly Val Leu Ser
    130                 135                 140

Ala Thr Ile Leu Tyr Glu Ile Leu Leu Gly Lys Ala Thr Leu Tyr Ala
145                 150                 155                 160

Val Leu Val Ser Ala Leu Val Leu Met Ala Met Val Lys Arg Lys Asp
                165                 170                 175

Ser Arg Gly

<210> 314
<211> 81
<212> DNA
<213> Artificial Sequence

<220>
<221> source
<223> /note="Description of Artificial Sequence: Synthetic
      oligonucleotide"

<400> 314
agagccaaga gatctggcag cggcgccaca aactttagcc tgctgaaaca ggccggcgac      60

gtggaagaga accctggacc t                                                81

<210> 315
<211> 27
<212> PRT
<213> Artificial Sequence

<220>
<221> source
<223> /note="Description of Artificial Sequence: Synthetic
      peptide"

<400> 315
Arg Ala Lys Arg Ser Gly Ser Gly Ala Thr Asn Phe Ser Leu Leu Lys
1               5                   10                  15

Gln Ala Gly Asp Val Glu Glu Asn Pro Gly Pro
            20                  25

<210> 316
<211> 390
<212> DNA
<213> Artificial Sequence

<220>
<221> source
<223> /note="Description of Artificial Sequence: Synthetic
      polynucleotide"

<400> 316
atgaagtccc tgcgggtgct gctggttatt ctgtggctgc agctgagctg ggtttggagc      60

cagggacaga acatcgacca gcctaccgag atgacagcca ccgaaggcgc catcgtgcag     120

atcaattgca cctaccagac cagcggcttc aacggcctgt tctggtatca acagcatgcc     180

ggcgaggccc ctaccttcct gagctataat gtgctggacg gcctggaaga aaagggcaga     240

ttcagcagct tcctgtccag aagcaagggc tacagctacc tgctgctgaa agaactccag     300

atgaaggaca gcgcctccta cctgtgtgcc tccgtggatg gaaacaacag actggccttc     360

ggcaagggca accaggtggt ggtcatcccc                                      390

<210> 317
<211> 130
<212> PRT
<213> Artificial Sequence

<220>
<221> source
<223> /note="Description of Artificial Sequence: Synthetic
      polypeptide"

<400> 317
Met Lys Ser Leu Arg Val Leu Leu Val Ile Leu Trp Leu Gln Leu Ser
1               5                   10                  15

Trp Val Trp Ser Gln Gly Gln Asn Ile Asp Gln Pro Thr Glu Met Thr
            20                  25                  30

Ala Thr Glu Gly Ala Ile Val Gln Ile Asn Cys Thr Tyr Gln Thr Ser
        35                  40                  45

Gly Phe Asn Gly Leu Phe Trp Tyr Gln Gln His Ala Gly Glu Ala Pro
    50                  55                  60

Thr Phe Leu Ser Tyr Asn Val Leu Asp Gly Leu Glu Glu Lys Gly Arg
65                  70                  75                  80

Phe Ser Ser Phe Leu Ser Arg Ser Lys Gly Tyr Ser Tyr Leu Leu Leu
                85                  90                  95

Lys Glu Leu Gln Met Lys Asp Ser Ala Ser Tyr Leu Cys Ala Ser Val
            100                 105                 110

Asp Gly Asn Asn Arg Leu Ala Phe Gly Lys Gly Asn Gln Val Val Val
        115                 120                 125

Ile Pro
    130

<210> 318
<211> 493
<212> DNA
<213> Artificial Sequence

<220>
<221> source
<223> /note="Description of Artificial Sequence: Synthetic
      polynucleotide"

<400> 318
gacattcaga accccgatcc agccgtgtac cagctgagag acagcaagag cagcgacaag      60

agcgtgtgtc tgttcaccga cttcgactcc cagaccaatg tgtcccagag caaggactcc     120

gacgtgtaca tcaccgataa gaccgtgctg gacatgcgga gcatggactt caagagcaat     180

agcgccgtgg cttggagcaa caagagcgac tttgcctgcg ccaacgcctt caacaacagc     240

atcatccccg aggacacatt cttcccaagt cctgagagca gctgcgacgt gaagctggtg     300

gaaaagagct tcgagacaga caccaacctg aacttccaga acctgagcgt gatcggcttc     360

agaatcctgc tgctgaaggt ggccggcttc aacctgctga tgactctgag actgtggtcc     420

agctgaattc ggatccaagc ttaggcctgc tcgctttctt gctgtcccat ttctattaaa     480

ggttcctttg ttc                                                        493

<210> 319
<211> 141
<212> PRT
<213> Artificial Sequence

<220>
<221> source
<223> /note="Description of Artificial Sequence: Synthetic
      polypeptide"

<400> 319
Asp Ile Gln Asn Pro Asp Pro Ala Val Tyr Gln Leu Arg Asp Ser Lys
1               5                   10                  15

Ser Ser Asp Lys Ser Val Cys Leu Phe Thr Asp Phe Asp Ser Gln Thr
            20                  25                  30

Asn Val Ser Gln Ser Lys Asp Ser Asp Val Tyr Ile Thr Asp Lys Thr
        35                  40                  45

Val Leu Asp Met Arg Ser Met Asp Phe Lys Ser Asn Ser Ala Val Ala
    50                  55                  60

Trp Ser Asn Lys Ser Asp Phe Ala Cys Ala Asn Ala Phe Asn Asn Ser
65                  70                  75                  80

Ile Ile Pro Glu Asp Thr Phe Phe Pro Ser Pro Glu Ser Ser Cys Asp
                85                  90                  95

Val Lys Leu Val Glu Lys Ser Phe Glu Thr Asp Thr Asn Leu Asn Phe
            100                 105                 110

Gln Asn Leu Ser Val Ile Gly Phe Arg Ile Leu Leu Leu Lys Val Ala
        115                 120                 125

Gly Phe Asn Leu Leu Met Thr Leu Arg Leu Trp Ser Ser
    130                 135                 140

<210> 320
<211> 6
<212> PRT
<213> Artificial Sequence

<220>
<221> source
<223> /note="Description of Artificial Sequence: Synthetic
      peptide"

<400> 320
Thr Ser Gly Phe Asn Gly
1               5

<210> 321

<211> 6
<212> PRT
<213> Artificial Sequence

<220>
<221> source
<223> /note="Description of Artificial Sequence: Synthetic
      peptide"

<400> 321
Asn Val Leu Asp Gly Leu
1               5


<210> 322
<211> 10
<212> PRT
<213> Artificial Sequence

<220>
<221> source
<223> /note="Description of Artificial Sequence: Synthetic
      peptide"

<400> 322
Ala Ser Val Asp Gly Asn Asn Arg Leu Ala
1               5                   10


<210> 323
<211> 5
<212> PRT
<213> Artificial Sequence

<220>
<221> source
<223> /note="Description of Artificial Sequence: Synthetic
      peptide"

<400> 323
Ser Gly His Asp Thr
1               5


<210> 324
<211> 6
<212> PRT
<213> Artificial Sequence

<220>
<221> source
<223> /note="Description of Artificial Sequence: Synthetic
      peptide"

<400> 324
Tyr Tyr Glu Glu Glu Glu
1               5


<210> 325
<211> 15
<212> PRT
<213> Artificial Sequence

<220>
<221> source
<223> /note="Description of Artificial Sequence: Synthetic
      peptide"

<400> 325
Ala Ser Ser Leu Gly Trp Arg Gly Gly Arg Tyr Asn Glu Gln Phe
1               5                   10                  15


<210> 326
<211> 6762
<212> DNA
<213> Artificial Sequence

<220>
<221> source
<223> /note="Description of Artificial Sequence: Synthetic
      polynucleotide"

<400> 326
   ctgacgcgcc ctgtagcggc gcattaagcg cggcgggtgt ggtggttacg cgcagcgtga      60
   ccgctacact tgccagcgcc ctagcgcccg ctcctttcgc tttcttccct tcctttctcg     120
   ccacgttcgc cggctttccc cgtcaagctc taaatcgggg gctcccttta gggttccgat     180
   ttagtgcttt acggcacctc gaccccaaaa aacttgatta gggtgatggt tcacgtagtg     240
   ggccatcgcc ctgatagacg gtttttcgcc ctttgacgtt ggagtccacg ttctttaata     300
   gtggactctt gttccaaact ggaacaacac tcaaccctat ctcggtctat tcttttgatt     360
   tataagggat tttgccgatt tcggcctatt ggttaaaaaa tgagctgatt taacaaaaat     420
   ttaacgcgaa ttttaacaaa atattaacgc ttacaatttg ccattcgcca ttcaggctgc     480
   gcaactgttg ggaagggcga tcggtgcggg cctcttcgct attacgccag ctggcgaaag     540
   ggggatgtgc tgcaaggcga ttaagttggg taacgccagg gttttcccag tcacgacgtt     600
   gtaaaacgac ggccagtgaa ttgtaatacg actcactata gggcgacccg gggatggcgc     660
   gccagtaatc aattacgggg tcattagttc atagcccata tatggagttc cgcgttacat     720
   aacttacggt aaatggcccg cctggctgac cgcccaacga cccccgccca ttgacgtcaa     780
   taatgacgta tgttcccata gtaacgccaa tagggacttt ccattgacgt caatgggtgg     840
   agtatttacg gtaaactgcc cacttggcag tacatcaagt gtatcatatg ccaagtacgc     900
   cccctattga cgtcaatgac ggtaaatggc ccgcctggca ttatgcccag tacatgacct     960
   tatgggactt tcctacttgg cagtacatct acgtattagt catcgctatt accatgctga    1020
   tgcggttttg gcagtacatc aatgggcgtg gatagcggtt tgactcacgg ggatttccaa    1080
   gtctccaccc cattgacgtc aatgggagtt tgttttggca ccaaaatcaa cgggactttc    1140
   caaaatgtcg taacaactcc gccccattga cgcaaatggg cggtaggcgt gtacggtggg    1200
   aggtctatat aagcagagct ggtttagtga accggggtct ctctggttag accagatctg    1260
   agcctgggag ctctctggct aactagggaa cccactgctt aagcctcaat aaagcttgcc 1320
   ttgagtgctt caagtagtgt gtgcccgtct gttgtgtgac tctggtaact agagatccct 1380
   cagacccttt tagtcagtgt ggaaaatctc tagcagtggc gcccgaacag ggacttgaaa 1440
   gcgaaaggga aaccagagga gctctctcga cgcaggactc ggcttgctga agcgcgcacg 1500
   gcaagaggcg aggggcggcg actggtgagt acgccaaaaa ttttgactag cggaggctag 1560
   aaggagagag atgggtgcga gagcgtcagt attaagcggg ggagaattag atcgcgatgg 1620
   gaaaaaattc ggttaaggcc agggggaaag aaaaaatata aattaaaaca tatagtatgg 1680
   gcaagcaggg agctagaacg attcgcagtt aatcctggcc tgttagaaac atcagaaggc 1740
   tgtagacaaa tactgggaca gctacaacca tcccttcaga caggatcaga agaacttaga 1800
   tcattatata atacagtagc aaccctctat tgtgtgcatc aaaggataga gataaaagac 1860
   accaaggaag ctttagacaa gatagaggaa gagcaaaaca aaagtaagac caccgcacag 1920
   caagccgccg ctgatcttca gacctggagg aggagatatg agggacaatt ggagaagtga 1980
   attatataaa tataaagtag taaaaattga accattagga gtagcaccca ccaaggcaaa 2040
   gagaagagtg gtgcagagag aaaaaagagc agtgggaata ggagctttgt tccttgggtt 2100
   cttgggagca gcaggaagca ctatgggcgc agcgtcaatg acgctgacgg tacaggccag 2160
   acaattattg tctggtatag tgcagcagca gaacaatttg ctgagggcta ttgaggcgca 2220
   acagcatctg ttgcaactca cagtctgggg catcaagcag ctccaggcaa gaatcctggc 2280
   tgtggaaaga tacctaaagg atcaacagct cctggggatt tggggttgct ctggaaaact 2340
   catttgcacc actgctgtgc cttggaatgc tagttggagt aataaatctc tggaacagat 2400
   ttggaatcac acgacctgga tggagtggga cagagaaatt aacaattaca caagcttaat 2460
   acactcctta attgaagaat cgcaaaacca gcaagaaaag aatgaacaag aattattgga 2520
   attagataaa tgggcaagtt tgtggaattg gtttaacata acaaattggc tgtggtatat 2580
   aaaattattc ataatgatag taggaggctt ggtaggttta agaatagttt ttgctgtact 2640
   ttctatagtg aatagagtta ggcagggata ttcaccatta tcgtttcaga cccacctccc 2700
   aaccccgagg ggacccgaca ggcccgaagg aatagaagaa gaaggtggag agagagacag 2760
   agacagatcc attcgattag tgaacggatc tcgacggtat cggttaactt ttaaaagaaa 2820
   aggggggatt ggggggtaca gtgcagggga aagaatagta gacataatag caacagacat 2880
   acaaactaaa gaattacaaa aacaaattac aaaattcaaa attttatcgc gatcgcggaa 2940
   tgaaagaccc cacctgtagg tttggcaagc tagcttaagt aacgccattt tgcaaggcat 3000
   ggaaaataca taactgagaa tagagaagtt cagatcaagg ttaggaacag agagacagca 3060
   gaatatgggc caaacaggat atctgtggta agcagttcct gccccggctc agggccaaga 3120
   acagatggtc cccagatgcg gtcccgccct cagcagtttc tagagaacca tcagatgttt 3180
   ccagggtgcc ccaaggacct gaaaatgacc ctgtgcctta tttgaactaa ccaatcagtt 3240
   cgcttctcgc ttctgttcgc gcgcttctgc tccccgagct caataaaaga gcccacaacc 3300
   cctcactcgg cgcgccagtc cttcgaagta gatctttgtc gatcctacca tccactcgac 3360
   acacccgcca gcggccgctg ccaagcttcc gagctctcga attaattcac ggtacccacc 3420
   atggcctagg gagactagtc gaatcgatat caacctctgg attacaaaat ttgtgaaaga 3480
   ttgactggta ttcttaacta tgttgctcct tttacgctat gtggatacgc tgctttaatg 3540
   cctttgtatc atgctattgc ttcccgtatg gctttcattt tctcctcctt gtataaatcc 3600
   tggttgctgt ctctttatga ggagttgtgg cccgttgtca ggcaacgtgg cgtggtgtgc 3660
   actgtgtttg ctgacgcaac ccccactggt tggggcattg ccaccacctg tcagctcctt 3720
   tccgggactt tcgctttccc cctccctatt gccacggcgg aactcatcgc cgcctgcctt 3780
   gcccgctgct ggacaggggc tcggctgttg ggcactgaca attccgtggt gttgtcgggg 3840
   aagctgacgt cctttcatgg gctgctcgcc tgtgttgcca cctggattct gcgcgggacg 3900
   tccttctgct acgtcccttc ggccctcaat ccagcggacc ttccttcccg cggcctgctg 3960
   ccggctctgc ggcctcttcc gcgtcttcgc cttcgccctc agacgagtcg gatctccctt 4020
   tgggccgcct ccccgcctgg ttaattaaag tacctttaag accaatgact tacaaggcag 4080
   ctgtagatct tagccacttt ttaaaagaaa aggggggact ggaagggcga attcactccc 4140
   aacgaagaca agatctgctt tttgcttgta ctgggtctct ctggttagac cagatctgag 4200
   cctgggagct ctctggctaa ctagggaacc cactgcttaa gcctcaataa agcttgcctt 4260
   gagtgcttca agtagtgtgt gcccgtctgt tgtgtgactc tggtaactag agatccctca 4320
   gaccctttta gtcagtgtgg aaaatctcta gcaggcatgc cagacatgat aagatacatt 4380
   gatgagtttg gacaaaccac aactagaatg cagtgaaaaa aatgctttat ttgtgaaatt 4440
   tgtgatgcta ttgctttatt tgtaaccatt ataagctgca ataaacaagt taacaacaac 4500
   aattgcattc attttatgtt tcaggttcag ggggaggtgt gggaggtttt ttggcgcgcc 4560
   atcgtcgagg ttccctttag tgagggttaa ttgcgagctt ggcgtaatca tggtcatagc 4620
   tgtttcctgt gtgaaattgt tatccgctca caattccaca caacatacga gccggaagca 4680
   taaagtgtaa agcctggggt gcctaatgag tgagctaact cacattaatt gcgttgcgct 4740
   cactgcccgc tttccagtcg ggaaacctgt cgtgccagct gcattaatga atcggccaac 4800
   gcgcggggag aggcggtttg cgtattgggc gctcttccgc ttcctcgctc actgactcgc 4860
   tgcgctcggt cgttcggctg cggcgagcgg tatcagctca ctcaaaggcg gtaatacggt 4920
   tatccacaga atcaggggat aacgcaggaa agaacatgtg agcaaaaggc cagcaaaagg 4980
   ccaggaaccg taaaaaggcc gcgttgctgg cgtttttcca taggctccgc ccccctgacg 5040
   agcatcacaa aaatcgacgc tcaagtcaga ggtggcgaaa cccgacagga ctataaagat 5100
   accaggcgtt tccccctgga agctccctcg tgcgctctcc tgttccgacc ctgccgctta 5160
   ccggatacct gtccgccttt ctcccttcgg gaagcgtggc gctttctcat agctcacgct 5220
   gtaggtatct cagttcggtg taggtcgttc gctccaagct gggctgtgtg cacgaacccc 5280
   ccgttcagcc cgaccgctgc gccttatccg gtaactatcg tcttgagtcc aacccggtaa 5340
   gacacgactt atcgccactg gcagcagcca ctggtaacag gattagcaga gcgaggtatg 5400
   taggcggtgc tacagagttc ttgaagtggt ggcctaacta cggctacact agaagaacag 5460
   tatttggtat ctgcgctctg ctgaagccag ttaccttcgg aaaaagagtt ggtagctctt 5520
   gatccggcaa acaaaccacc gctggtagcg gtggtttttt tgtttgcaag cagcagatta 5580
   cgcgcagaaa aaaaggatct caagaagatc ctttgatctt ttctacgggg tctgacgctc 5640
   agtggaacga aaactcacgt taagggattt tggtcatgag attatcaaaa aggatcttca 5700
   cctagatcct tttaaattaa aaatgaagtt ttaaatcaat ctaaagtata tatgagtaaa 5760
   cttggtctga cagttaccaa tgcttaatca gtgaggcacc tatctcagcg atctgtctat 5820
   ttcgttcatc catagttgcc tgactccccg tcgtgtagat aactacgata cgggagggct 5880
   taccatctgg ccccagtgct gcaatgatac cgcgagaccc acgctcaccg gctccagatt 5940
   tatcagcaat aaaccagcca gccggaaggg ccgagcgcag aagtggtcct gcaactttat 6000
   ccgcctccat ccagtctatt aattgttgcc gggaagctag agtaagtagt tcgccagtta 6060
   atagtttgcg caacgttgtt gccattgcta caggcatcgt ggtgtcacgc tcgtcgtttg 6120
   gtatggcttc attcagctcc ggttcccaac gatcaaggcg agttacatga tcccccatgt 6180
   tgtgcaaaaa agcggttagc tccttcggtc ctccgatcgt tgtcagaagt aagttggccg 6240
   cagtgttatc actcatggtt atggcagcac tgcataattc tcttactgtc atgccatccg 6300
   taagatgctt ttctgtgact ggtgagtact caaccaagtc attctgagaa tagtgtatgc 6360
   ggcgaccgag ttgctcttgc ccggcgtcaa tacgggataa taccgcgcca catagcagaa 6420
   ctttaaaagt gctcatcatt ggaaaacgtt cttcggggcg aaaactctca aggatcttac 6480
   cgctgttgag atccagttcg atgtaaccca ctcgtgcacc caactgatct tcagcatctt 6540
   ttactttcac cagcgtttct gggtgagcaa aaacaggaag gcaaaatgcc gcaaaaaagg 6600
   gaataagggc gacacggaaa tgttgaatac tcatactctt cctttttcaa tattattgaa 6660
   gcatttatca gggttattgt ctcatgagcg gatacatatt tgaatgtatt tagaaaaata 6720
   aacaaatagg ggttccgcgc acatttcccc gaaaagtgcc ac 6762

## Description

### RELATED APPLICATIONS

This application claims priority to U.S. Provisional Patent Application No. 62/559,330, filed September 15, 2017, and to U.S. Provisional Patent Application No. 62/566,912, filed October 2, 2017, each of which is incorporated by reference in its entirety.

### TECHNICAL FIELD

This application relates generally to methods and apparatuses, including computer program products, for performing a patient-specific immunotherapy procedure with chain-of-custody and chain-of-identity biological sample tracking

### BACKGROUND

In recent years, advances in medical technology have led to the emerging use of immunotherapies to treat different types of illnesses and diseases, including various forms of cancer. Generally, immunotherapy is the treatment of disease by stimulating or suppressing an immune response. Often, modified versions of a patient's own biological material, such as immune cells, are reintroduced into the patient in order to initiate and/or supplement the immune response.

For example, engineered immune cells have been shown to possess desired qualities in therapeutic treatments, particularly in oncology. Two main types of engineered immune cells are those that contain chimeric antigen receptors (termed "CARs" or "CAR-Ts") and T-cell receptors ("TCRs"). These engineered cells are engineered to endow them with antigen specificity while retaining or enhancing their ability to recognize and kill a target cell. Chimeric antigen receptors may comprise, for example, (i) an antigen-specific component ("antigen binding molecule"), (ii) an extracellular domain, (iii) one or more costimulatory domains, and (iv) one or more activating domains. Each domain may be heterogeneous, that is, comprised of sequences derived from (or corresponding to) different protein chains.

Because many patients that undergo immunotherapy are critically ill, a crucial factor in the efficacy of such immunotherapy procedures is the ability to provide the modified biological material to the patient as quickly as possible, so that the therapeutic benefits may be maximized. Also, because many types of immunotherapy are tailored for the specific patient (i.e., using the patient's own cells), it is important to ensure that the patient's biological material is accurately tracked throughout the immunotherapy process-from extraction, to modification, and then to infusion back into the patient-to avoid delays in manufacturing, mislabeling of material, and misidentification of the patient. However, existing immunotherapy procedures generally lack a technical mechanism to track the patient's biological material automatically and to ensure that the biological material is tied to the specific patient's identity throughout the manufacturing process.

### SUMMARY

Therefore, what is needed are methods and systems for performing a patient-specific immunotherapy procedure with chain-of-custody and chain-of-identity biological sample tracking. The techniques described herein provide the specific technical advantage over existing systems of providing a continuous and automatic chain of custody and chain of identity for a patient-specific biological sample during an immunotherapy procedure, to create a computerized information portal that interested parties-such as the patient, physician, manufacturer, and other medical personnel- may use to quickly understand and track the current phase of the immunotherapy procedure and the status of the patient's biological sample during the procedure. Such advanced tracking is an improvement over existing systems that do not have technological solutions for maintaining a chain of custody and chain of identity-resulting in delays during the manufacturing process which, for a patient dealing with a life-threatening illness, may be immeasurably severe.

The invention, in one aspect, features a method of performing a patient-specific immunotherapy procedure. A computing device receives a cell order request to create transfected T cells for a patient. The computing device generates a patient-specific identifier associated with the cell order request, the patient-specific identifier comprising a patient identity element, a sales order identifier, and a cell order lot number. The computing device initiates a process to create transfected T cells for infusion into the patient's bloodstream, the process comprising: performing a leukapheresis procedure on a sample of the patient's blood to collect T cells from the sample, transferring the collected T cells to a container, labeling the container with the patient-specific identifier, transmitting the collected T cells to a manufacturing facility, creating transfected T cells from the collected T cells using a cell modification technique, receiving the transfected T cells from the manufacturing facility, and infusing the transfected T cells into the patient's bloodstream. The computing device records a tracking event for each step in the process, each tracking event including the patient-specific identifier. The tracking events comprise a chain of custody of the patient's T cells during the process.

The invention, in another aspect, features a method of tracking a cell order during an immunotherapy procedure. A computing device receives a cell order request for creating transfected T cells for a patient. The computing device generates a patient-specific identifier associated with the cell order request, the patient-specific identifier comprising a patient identity element, a sales order identifier, and a cell order lot number. The computing device monitors a process to create transfected T cells for infusion into the patient's bloodstream, the process comprising: receiving indicia that a leukapheresis procedure has been performed on a sample of the patient's blood to collect T cells from the sample, receiving indicia that the collected T cells have been transferred to a container, receiving indicia that the container has been labeled with the patient-specific identifier, receiving indicia that the collected T cells have been transmitted to a manufacturing facility, receiving indicia that transfected T cells have been created from the collected T cells using a cell modification technique, receiving indicia that the transfected T cells have been received from the manufacturing facility, and receiving indicia that the transfected T cells have been infused into the patient's bloodstream. The computing device records a tracking event when an indicia is received, each tracking event including the patient-specific identifier. The computing device maintains a chain of custody of the patient's T cells by storing the tracking events during the process.

The invention, in another aspect, features a method of performing a patient-specific immunotherapy procedure. A cell order request to create transfected T cells for a patient is received. An event tracking module executed on a processor generates a patient-specific identifier associated with the cell order request. A process to create transfected T cells for infusion into the patient's bloodstream is initiated, comprising: performing a leukapheresis procedure on a sample of the patient's blood to collect T cells from the sample, transferring the collected T cells to a container, labeling the container with the patient-specific identifier, transmitting the collected T cells to a manufacturing facility, creating transfected T cells from the collected T cells using a cell modification technique, receiving the transfected T cells from the manufacturing facility, and infusing the transfected T cells into the patient's bloodstream. The event tracking module receives, from a first client device located at the point of the leukapheresis procedure, a first tracking event that confirms the leukapheresis procedure and contains the patient-specific identifier. The event tracking module integrates the first tracking event in a data structure pertaining to the patient-specific identifier, where the data structure is stored in a database and the integrating step records a first timestamp with the first tracking event. The event tracking module receives, from a second client device located at the manufacturing facility, a second tracking event that confirms the receipt of the collected T cells at the manufacturing facility and contains the patient-specific identifier. The event tracking module integrates the second tracking event in the data structure pertaining to the patient-specific identifier, where the integrating step records a second timestamp with the second tracking event.

The invention, in another aspect, features a method of performing a patient-specific immunotherapy procedure. A tracking module executed on a processor receives a cell order request to create transfected T cells for a patient. The tracking module generates a patient-specific identifier associated with the cell order request, the patient-specific identifier identifying a patient, and a cell order lot. A database generates a data record for tracking the cell order, the data record identified in the database according to the patient-specific identifier. The tracking module receives a first tracking event indicating that the collected T cells are ready for shipment to a manufacturing facility. The data record corresponding to the patient-specific identifier is updated in accordance with the first tracking event. The tracking module receives, based on the container having been received by the manufacturing facility, a second tracking event indicating that the collected T cells have been received by a manufacturing facility. The data record corresponding to the patient-specific identifier is updated in accordance with the second tracking event. The tracking module receives, based on the manufacturing facility having created transfected T cells from the collected T cells using a cell modification technique, a third tracking event indicating that the transfected T cells have been created. The data record corresponding to the patient-specific identifier is updated in accordance with the third tracking event. ;The tracking module receives, based on the transfected T cells having been received from the manufacturing facility, a fourth tracking event indicating that the transfected T cells have been received. The data record corresponding to the patient-specific identifier is updated in accordance with the fourth tracking event. The tracking module receives, based on the transfected T cells having been infused into the patient's bloodstream, a fifth tracking event indicating that the transfected T cells have been infused into the patient's bloodstream. The data record corresponding to the patient-specific identifier is updated in accordance with the fifth tracking event, where each of the first, second, third, fourth, and fifth tracking events contains the patient-specific identifier, a timestamp, and an event identifier, and where the data record corresponding to the patient-specific identifier stores, in an ordered sequence, the first, second, third, fourth, and fifth tracking events when the data record is updated in accordance with the respective events.

Any of the above aspects may include one or more of the following features:

In some embodiments, the transfected T cells are created by transfecting the collected T cells with a polynucleotide encoding a chimeric antigen receptor (CAR), the CAR comprising an antigen binding molecule that specifically binds to a target molecule. Preferably, the antigen binding molecule is a single chain variable fragment (scFv).

It is envisioned that in some embodiments, the target molecule is a blood borne cancer-associated antigen. The blood borne cancer-associated antigens of the present invention are associated with one or more cancers selected from the group consisting of: acute myeloid leukemia (AML), chronic myelogenous leukemia (CML), chronic myelomonocytic leukemia (CMML), juvenile myelomonocytic leukemia, atypical chronic myeloid leukemia, acute promyelocytic leukemia (APL), acute monoblastic leukemia, acute erythroid leukemia, acute megakaryoblastic leukemia, lymphoblastic leukemia, B-lineage acute lymphoblastic leukemia, B-cell chronic lymphocytic leukemia, B-cell non-Hodgkin's lymphoma, myelodysplastic syndrome (MDS), myeloproliferative disorder, myeloid neoplasm, myeloid sarcoma), and Blastic Plasmacytoid Dendritic Cell Neoplasm (BPDCN).

In some embodiments, the target molecule is a viral infection-associated antigen. The viral infections of the present invention may be caused by any virus, including, for example, HIV.

The chimeric antigen receptors (CARs) of the present invention may further comprise at least one costimulatory domain. The costimulatory domains of the present invention include, but are not limited to, a signaling region, or other suitable portion, of CD28, OX-40, 4-1BB/CD137, CD2, CD7, CD27, CD30, CD40, programmed death-1 (PD-1), inducible T cell costimulator (ICOS), lymphocyte function-associated antigen-1 (LFA-1 (CD1 la/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class I molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof.

Preferably, the costimulatory domain comprises CD28. CD28 costimulatory domains of the present invention comprise, for example, a sequence selected from the group consisting of SEQ ID NO. 2, SEQ ID NO. 4, SEQ ID NO. 6, and SEQ ID NO. 8.

In other embodiments, the costimulatory domain comprises CD8. CD8 costimulatory domains of the present invention comprise, for example, SEQ ID NO. 14.

The chimeric antigen receptors (CARs) of the present invention may further comprise at least one activating domain. The activating domains of the present invention comprise, for example, CD3. Preferably, the CD3 activating domains comprise CD3 zeta. CD3 zeta activating domains of the present invention comprise, for example, SEQ ID NO. 10.

In a preferred embodiment, the chimeric antigen receptor (CAR) comprises all or part of: an anti-CD 19 scFv, CD28, and CD3 zeta.

In additional preferred embodiments, the chimeric antigen receptor (CAR) comprises:
- all or part of: an anti-CD19 scFv, CD8, and 4-1BB;
- all or part of: an anti-BCMA scFv and CD8;
- all or part of: an anti-CD19 scFv, CD28, and 4-1BB;
- all or part of: an anti-CD22 scFv and CD8;
- all or part of: an anti-CD19 scFv, CD28, and EGFRt/19-28z/4-1BBL;
- all or part of: an anti-MUC16 scFv, and CD28;
- all or part of: an anti-CD171;
- all or part of: an anti-CD123, and CD28;
- all or part of: an anti-BCMA, CD8, and 4-1BB;
- all or part of: an anti-CD19 and CD28;
- all or part of: an anti-CD19 and CD8; or,
- all or part of: CD28.

In a further preferred embodiment, the CAR comprises a leader sequence (CSF2RA), an anti-CD19 scFv, a Whitlow linker, a CD28 spacer, a CD28 costimulatory domain, and CD3 zeta. One example of such a CAR is encoded by the nucleotide sequence of SEQ ID NO. 146 and the amino acid sequence of SEQ ID NO. 147.

In an additional preferred embodiment, the CAR comprises a leader sequence (CD8), an anti-CD19 scFv, a Whitlow linker, a CD28T spacer, a CD28 costimulatory domain, and CD3 zeta. One example of such a CAR is encoded by the nucleotide sequence of SEQ ID NO. 148 and the amino acid sequence of SEQ ID NO. 149.

In another preferred embodiment, the CAR comprises a leader sequence (CD8a), an anti-CD19 scFv, a Whitlow linker, a CD8a spacer and transmembrane domain, a CD28 costimulatory domain, and CD3 zeta. One example of such a CAR is encoded by the nucleotide sequence of SEQ ID NO. 150 and the amino acid sequence of SEQ ID NO. 151.

In a further preferred embodiment, the CAR comprises a leader sequence (CD8), an anti-CLL-1 scFv, a G4S linker, a Minispacer, a CD28T (extracellular / transmembrane region of CD28), CD28 (an intracellular costimulatory region of CD28), and CD3 zeta. One example of such a CAR is encoded by the nucleotide sequence of SEQ ID NO. 154 and the amino acid sequence of SEQ ID NO. 155.

In an additional preferred embodiment, the CAR comprises a leader sequence (CD8a), an anti-BCMA scFv, a Whitlow linker, a CD28T spacer, a CD28 costimulatory domain, and CD3 zeta. One example of such a CAR is encoded by the nucleotide sequence of SEQ ID NO. 156 and the amino acid sequence of SEQ ID NO. 157.

In some embodiments, the transfected T cells are created by transfecting the collected T cells with a polynucleotide encoding a T cell receptor (TCR). The TCRs of the present invention may bind to a tumor-associated antigen. Tumor-associated antigens are associated with one or more cancers selected from the group consisting of: adrenocortical carcinoma, anal cancer, bladder cancer, bone cancer, brain cancer, breast cancer, carcinoid cancer, carcinoma, cervical cancer, colon cancer, endometrial cancer, esophageal cancer, extrahepatic bile duct cancer, extracranial germ cell cancer, eye cancer, gallbladder cancer, gastric cancer, germ cell tumor, gestational trophoblastic tumor, head and neck cancer, hypopharyngeal cancer, islet cell carcinoma, kidney cancer, large intestine cancer, laryngeal cancer, leukemia, lip and oral cavity cancer, liver cancer, lung cancer, lymphoma, malignant mesothelioma, Merkel cell carcinoma, mycosis fungoides, myelodysplastic syndrome, myeloproliferative disorders, nasopharyngeal cancer, neuroblastoma, oral cancer, oropharyngeal cancer, osteosarcoma, ovarian epithelial cancer, ovarian germ cell cancer, pancreatic cancer, paranasal sinus and nasal cavity cancer, parathyroid cancer, penile cancer, pituitary cancer, plasma cell neoplasm, prostate cancer, rhabdomyosarcoma, rectal cancer, renal cell cancer, transitional cell cancer of the renal pelvis and ureter, salivary gland cancer, Sezary syndrome, skin cancers, small intestine cancer, soft tissue sarcoma, stomach cancer, testicular cancer, thymoma, thyroid cancer, urethral cancer, uterine cancer, vaginal cancer, vulvar cancer, and Wilms' tumor.

In certain aspects, the present application may be suitable for target molecule to hematologic cancer. In some embodiments, the cancer is of the white blood cells. In other embodiments, the cancer is of the plasma cells. In some embodiments, the cancer is leukemia, lymphoma, or myeloma. In certain embodiments, the cancer is acute lymphoblastic leukemia (ALL) (including non T cell ALL), acute lymphoid leukemia (ALL), and hemophagocytic lymphohistocytosis (HLH)), B cell prolymphocytic leukemia, B-cell acute lymphoid leukemia ("BALL"), blastic plasmacytoid dendritic cell neoplasm, Burkitt's lymphoma, chronic lymphocytic leukemia (CLL), chronic myelogenous leukemia (CML), chronic myeloid leukemia (CML), chronic or acute granulomatous disease, chronic or acute leukemia, diffuse large B cell lymphoma, diffuse large B cell lymphoma (DLBCL), follicular lymphoma, follicular lymphoma (FL), hairy cell leukemia, hemophagocytic syndrome (Macrophage Activating Syndrome (MAS), Hodgkin's Disease, large cell granuloma, leukocyte adhesion deficiency, malignant lymphoproliferative conditions, MALT lymphoma, mantle cell lymphoma, Marginal zone lymphoma, monoclonal gammapathy of undetermined significance (MGUS), multiple myeloma, myelodysplasia and myelodysplastic syndrome (MDS), myeloid diseases including but not limited to acute myeloid leukemia (AML), non-Hodgkin's lymphoma (NHL), plasma cell proliferative disorders (e.g., asymptomatic myeloma (smoldering multiple myeloma or indolent myeloma), plasmablastic lymphoma, plasmacytoid dendritic cell neoplasm, plasmacytomas (e.g., plasma cell dyscrasia; solitary myeloma; solitary plasmacytoma; extramedullary plasmacytoma; and multiple plasmacytoma), POEMS syndrome (Crow-Fukase syndrome; Takatsuki disease; PEP syndrome), primary mediastinal large B cell lymphoma (PMBCL), small cell- or a large cell-follicular lymphoma, splenic marginal zone lymphoma (SMZL), systemic amyloid light chain amyloidosis, T-cell acute lymphoid leukemia (TALL), T-cell lymphoma, transformed follicular lymphoma, Waldenstrom macroglobulinemia, or a combination thereof.

In additional preferred embodiments, the TCR comprises an AV38-2 variable alpha chain, a BV7-2 variable beta chain, a murine constant alpha domain, a murine constant beta domain, TRAJ40, Furin-SG SG-P2A, and TRBJ1-3. One example of such a TCR is encoded by the nucleotide sequence of SEQ ID NO. 152 and the amino acid sequence of SEQ ID NO. 153.

In further preferred embodiments, the TCR comprises a TCR beta chain variable region, a TCR beta chain constant region, P2A peptide (with Furin cleavage site and linker), a TCR alpha chain variable region, and a TCR alpha chain constant region. One example of such a TCR is encoded by the nucleotide sequence of SEQ ID NO. 158 and the amino acid sequence of SEQ ID NO. 159. Another example of such a TCR is encoded by the nucleotide sequence of SEQ ID NO. 160 and the amino acid sequence of SEQ ID NO. 161.

In other embodiments, the TCRs of the present invention bind to a viral infection-associated antigen. Viral infections of the present invention may be caused by any virus, including, for example, HIV.

Polynucleotides of the present invention may be a component of a vector. Suitable vectors include, but are not limited to, retroviral vectors, DNA vectors, plasmids, RNA vectors, adenoviral vectors, adenovirus associated vectors, lentiviral vectors, or any combination thereof. Preferably, the vector is a lentiviral vector. The lentiviral vectors of the present invention include, but are not limited to, pGAR and derivatives thereof.

In some embodiments, initiating a process to create transfected T cells further comprises requesting a timeslot for performing the leukapheresis procedure from a remote computing device, receiving indicia responsive to the request for a timeslot that a timeslot is available to perform the leukapheresis procedure, and confirming acceptance of the available timeslot with the remote computing device.

In some embodiments, initiating a process to create transfected T cells further comprises receiving indicia that the transfected T cells have been shipped from a first site after being created and receiving indicia that the transfected T cells have been received at a second site before being infused, where the computing device records a tracking event upon receiving the indicia that the transfected T cells have been shipped, and the computing device records a tracking event upon receiving the indicia that the transfected T cells have been received at a second site.

In some embodiments, the patient identity element comprises a first patient ID associated with the immunotherapy procedure and a second patient ID associated with a facility that administers one or more of: the leukapheresis procedure or the infusion of the transfected T cells. In some embodiments, the computing device stores the tracking events in an ordered sequence. In some embodiments, the ordered sequence is arranged in chronological order.

Other aspects and advantages of the invention will become apparent from the following detailed description, taken in conjunction with the accompanying drawings, illustrating the principles of the invention by way of example only.

### BRIEF DESCRIPTION OF THE DRAWINGS

The advantages of the invention described above, together with further advantages, may be better understood by referring to the following description taken in conjunction with the accompanying drawings. The drawings are not necessarily to scale, emphasis instead generally being placed upon illustrating the principles of the invention.
FIG. 1A is a block diagram of a system for performing a patient-specific immunotherapy procedure with chain-of-custody and chain-of-identity biological sample tracking.
FIG. 1B is a detailed block diagram of a system for performing a patient-specific immunotherapy procedure with chain-of-custody and chain-of-identity biological sample tracking.
FIG. 2 is a flow diagram of a computerized method of performing a patient-specific immunotherapy procedure with chain-of-custody and chain-of-identity biological sample tracking.
FIGS. 3A and 3B are exemplary screenshots generated by a user interface module to receive patient-specific information during a patient-specific immunotherapy procedure.
FIGS. 4A to 4D are exemplary screenshots generated by a user interface module to receive confirmation of extraction and infusion sites, and to schedule an appointment, during a patient-specific immunotherapy procedure.
FIGS. 5A and 5B are exemplary screenshots generated by a user interface module to display a chain of custody for biological material during a patient-specific immunotherapy procedure.

### DETAILED DESCRIPTION

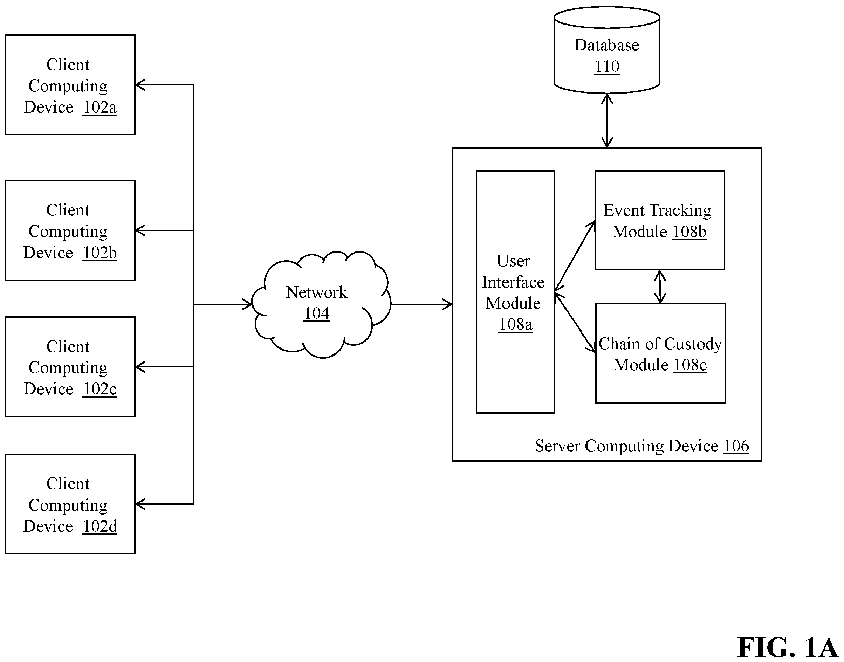

FIG. 1A is a block diagram of a system 100 for performing a patient-specific immunotherapy procedure with chain-of-custody and chain-of-identity biological sample tracking. The system of FIG. 1A includes a plurality of client computing devices 102a-102d, a communications network 104, a server computing device 106 with a user interface module 108a, an event tracking module 108b, and a chain of custody module 108c, and a database 110.

The client computing devices 102a-102d are connected to the communications network 104 in order to communicate with the server computing device 106 to provide input and receive output relating to the process of performing a patient-specific immunotherapy procedure with chain-of-custody and chain-of-identity biological sample tracking as described herein. In some embodiments, each client computing device 102a-102d may be coupled to a respective display device for, e.g., providing a detailed graphical user interface (GUI) that receives input for and presents output resulting from the methods and systems described herein. For example, the client computing device 102a-102d may connect to the user interface module 108a of server computing device 106, which provides, e.g., a web-based portal for users of the client computing devices 102a-102c to access functionality associated with the methods described herein.

Exemplary client devices 102a-102d include but are not limited to desktop computers, laptop computers, tablets, mobile devices, smartphones, and internet appliances. It should be appreciated that other types of computing devices that are capable of connecting to the components of the system of FIG. 1A may be used without departing from the scope of invention. It also should be appreciated that each of the client computing devices 102a-102d may be associated with a different user type-for example, client computing device 102a may be associated with a patient accessing the system of FIG. 1A to generate a user profile and receive updates on a patient-specific immunotherapy procedure; client computing device 102b may be associated with a physician who is treating the patient and who accesses the system of FIG. 1A to initiate an immunotherapy procedure for the patient; client computing device 102c may be associated with a hospital or other facility that is administering the immunotherapy procedure to the patient; and client computing device 102d may be associated with a manufacturing facility that is creating patient-specific immunotherapy product (as will be described herein) for use in the immunotherapy procedure.

The communications network 104 enables the other components of the system 100 to communicate with each other in order to conduct the process of performing a patient-specific immunotherapy procedure with chain-of-custody and chain-of-identity biological sample tracking as described herein. The network 104 may be a local network, such as a LAN, or a wide area network, such as the Internet and/or a cellular network. In some embodiments, the network 104 is comprised of several discrete networks and/or sub-networks (*e.g*., cellular to Internet) that enable the components of the system of FIG. 1A to communicate with each other.

The server computing device 106 is a combination of hardware and software modules that includes specialized hardware and/or software modules that execute on a processor and interact with memory modules of the server computing device 106 to perform functions for performing a patient-specific immunotherapy procedure with chain-of-custody and chain-of-identity biological sample tracking as described herein. The server computing device 106 includes a user interface module 108a, an event tracking module 108b, and a chain of custody module 108c (as mentioned above) that execute on and/or interact with the processor of the server computing device 106.

In some embodiments, the user interface module 108a, the event tracking module 108b, and the chain of custody module 108c are specialized sets of computer software instructions programmed onto one or more dedicated processors in the server computing device 106 and may include specifically-designated memory locations and/or registers for executing the specialized computer software instructions. Although the modules 108a-108c are shown in FIG. 1A as executing within the same server computing device 106, in some embodiments the functionality of the modules 108a-108c may be distributed among a plurality of server computing devices. As shown in FIG. 1A, the server computing device 106 enables the modules 108a-108c to communicate with each other in order to exchange data for the purposes of performing the described functions. It should be appreciated that any number of computing devices, arranged in a variety of architectures, resources, and configurations (e.g., cluster computing, virtual computing, cloud computing) may be used without departing from the scope of the invention. The exemplary functionality of the modules 108a-108c is described in detail below.

The database 110 is a computing device (or in some embodiments, a set of computing devices) coupled to the server computing device 106 and is configured to receive, generate, and store specific segments of data relating to the process of performing a patient-specific immunotherapy procedure with chain-of-custody and chain-of-identity biological sample tracking as described herein. In some embodiments, all or a portion of the database 110 may be integrated with the server computing device 106 or be located on a separate computing device or devices. The database 110 may comprise one or more databases configured to store portions of data used by the other components of the system of FIG. 1A, as will be described in greater detail below. In some embodiments, the database 110 comprises an enterprise business suite, such as Oracle E-Business Suite (EBS), that includes various modules that enable a spectrum of functionality to support the methods and systems described herein-including logistics, supply chain, transportation, CRM, and other types of modules.

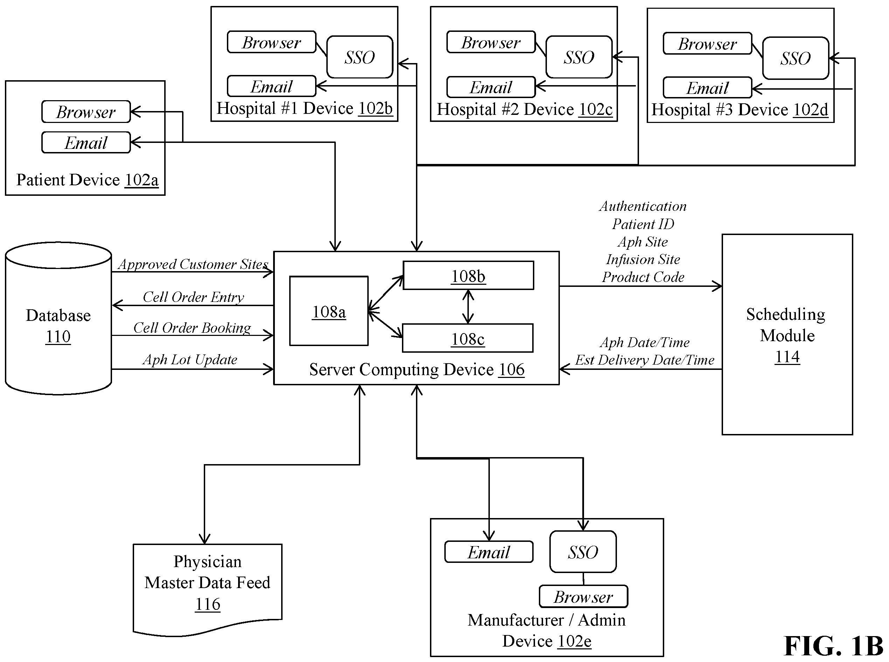

FIG. 1B is a detailed block diagram of the system of FIG. 1A for performing a patient-specific immunotherapy procedure with chain-of-custody and chain-of-identity biological sample tracking. As shown in FIG. 1B, the server computing device 106 is the central component in the overall hardware architecture, interfacing with client computing devices 102a-102e and database 110, and also interfacing with a scheduling module 114 and a physician master data feed 116. In some embodiments, the server computing device 106 and the corresponding modules 108a-108c leverage the Salesforce platform, available from Salesforce.com, Inc. of San Francisco, California, to integrate certain of the functions described herein. The client computing devices 102a-102e communicate with the server computing device 106 to perform patient enrollment in the immunotherapy procedure and to monitor the chain-of-custody and chain-of-identity tracking (e.g., via browser-based user interfaces) as described herein.

For example, client computing device 102a may be associated with the patient undergoing the immunotherapy procedure and may include browser software and email software to enable the patient to both monitor the tracking and to electronically sign documents required to participate in the immunotherapy procedure (e.g., via DocuSign or other similar technology). Similarly, the client computing devices 102b-102d may be located at different hospitals where a treating physician may enroll a patient in the immunotherapy procedure, place a cell order with the system, and monitor the chain-of-custody and chain-of identity tracking using the browser software. The client computing devices 102b-102d also include a single-sign-on (SSO) module that enables the devices to authenticate to the server computing device 106 (e.g., using SAML 2.0 supported SSO or a specific username/password for the server). The client computing device 102e may be located at an administration or manufacturing site to enable an administrator of the server computing device 106 to communicate with the server, receive communications such as emails from other participants in the system, and monitor the chain-of-custody and chain-of identity tracking using the browser software.

As described above, the database 110 may comprise an enterprise business suite that manages the data for the server computing device 106 and includes modules to enable chain-of-custody and chain-of-identity tracking and logistics for the biological sample. For example, the database 110 may transmit approved customer sites to the server 106 upon request, receive cell order entry data from the server 106, and provide cell order booking and apheresis lot update information to the server 106.

The scheduling module 114 may be integrated into the server computing device 106 or reside on a separate computing device. The scheduling module 114 may authenticate to and communicate with the server computing device 106 to receive certain information about the cell order and immunotherapy procedure (e.g., patient ID, apheresis site, infusion site, and product code) and provide calendaring and scheduling functionality to the server 106 (e.g., enabling a treating physician to select an apheresis date/time and provide an estimated delivery date/time for the biological sample once it has gone through the manufacturing process). Also, the server computing device 106 may communicate with a physician master data feed 116 (e.g., provided using the Veeva^{™} CRM platform integrated with the Heroku^{™} application) to receive certain information about treating physicians.

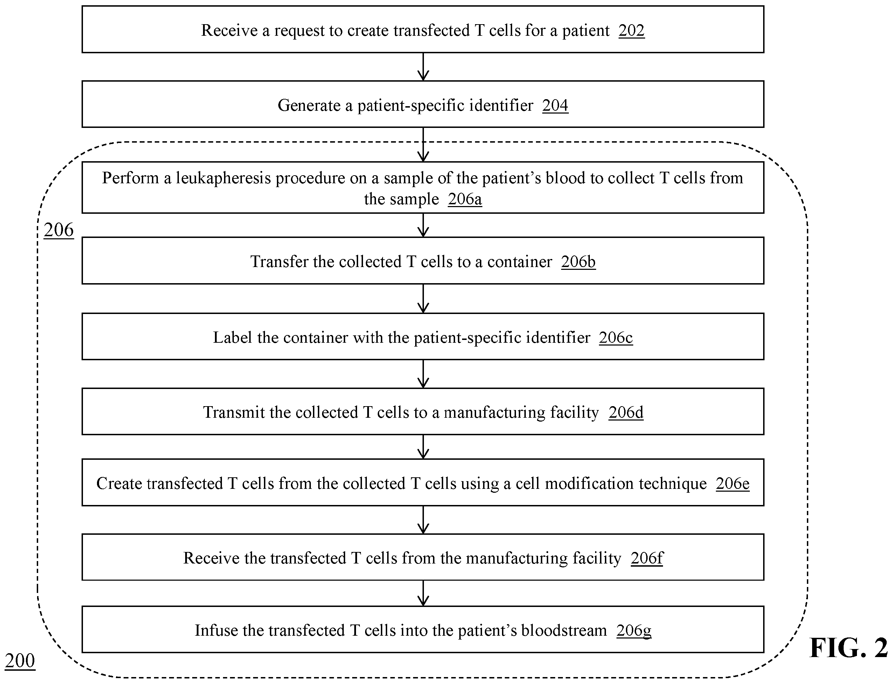

FIG. 2 is a flow diagram of a computerized method 200 of performing a patient-specific immunotherapy procedure with chain-of-custody and chain-of-identity biological sample tracking, using the system of FIG. 1A and/or the system of FIG. 1B. As will be appreciated, the methods and systems described herein are presented in the context of performing a patient-specific immunotherapy procedure. As used herein, "patient-specific immunotherapy procedure" means any procedure that uses molecular or cellular components of the immune system to target and/or destroy cancer, pathogenic, or other disease-causing cells. An immunotherapy procedure is "patient-specific" if it utilizes components of a patient's immune system to treat that patient's own cancer, pathology, or other disease.

As used herein, the terms "patient" and "subject" are used interchangeably and include human and non-human animals, as well as those with formally diagnosed disorders, those without formally recognized disorders, those receiving medical attention, those at risk of developing disorders, etc. In addition to humans, categories of animals within the scope of the present invention include, for example, agricultural animals, domestic animals, laboratory animals, etc. Some examples of agricultural animals include cows, pigs, horses, goats, etc. Some examples of domestic animals include dogs, cats, etc. Some examples of laboratory animals include rats, mice, rabbits, guinea pigs, etc.

The term "leukapheresis" refers to a specific form of apheresis which involves the selective separation and removal of leukocytes from a blood sample. During leukapheresis, the removed blood is passed through a cell separation device which separates nucleated white blood cells, including T cells, from red blood cells and plasma. The separated T cells may then be collected to be used in the cell modification techniques of the present invention. In certain embodiments, the red blood cells and plasma are returned to the individual as part of the separation process. In additional embodiments, the red blood cells and plasma are discarded or stored for further analysis.

As used herein, the terms "T cell" and "T lymphocyte" are interchangeable. T cells are a subset of lymphocytes defined by their development in the thymus and by heterodimeric receptors associated with the proteins of the CD3 complex. T cells of the present invention include, but are not limited to, naive T cells, cytotoxic T cells, helper T cells, suppressor T cells, regulatory T cells, memory T cells, NKT cells, γδ cells, CD8αα cells, lymphokine activated cells, TCR-expressing cells, subtypes thereof, and any other cell type which may express chimeric receptor chain.

T cells may be engineered to possess specificity to one or more desired targets. For example, T cells may be transduced with DNA or other genetic material encoding an antigen binding molecule, such as one or more single chain variable fragment ("scFv") of an antibody, in conjunction with one or more signaling molecules, and/or one or more activating domains, such as CD3 zeta. In addition to the CAR-T cells' ability to recognize and destroy the targeted cells, successful T cell therapy benefits from the CAR-T cells' ability to persist and maintain the ability to proliferate in response to antigen.

As used herein, the term "cell modification technique" includes, but is not limited to, transfection and transduction. The term "transfection" and grammatical variations thereof, refer to the introduction of foreign or exogenous DNA into a cell. A number of transfection techniques are well known in the art and are disclosed herein. See, *e.g.*, Graham et al., 1973, Virology 52:456; Sambrook et al., 2001, Molecular Cloning: A Laboratory Manual, supra; Davis et al., 1986, Basic Methods in Molecular Biology, Elsevier; Chu et al., 1981, Gene 13:197. Transfection techniques include, but are not limited to, calcium phosphate-DNA co-precipitation, DEAE-dextran-mediated transfection, cationic lipid-mediated delivery, polybrene-mediated transfection, electroporation, sonoporation, microinjection, liposome fusion, lipofection (lipid transfection), polymer transfection, nanoparticles, polyplexes, receptor-mediated gene delivery, delivery mediated by polylysine, histone, chitosan, and peptides, protoplast fusion, retroviral infection, and biolistics (*e.g*. Gene Gun). The term "transduction" and grammatical variations thereof refer to the process whereby foreign DNA is introduced into a cell via viral vector. See Jones et al., (1998). Genetics: principles and analysis. Boston: Jones & Bartlett Publ.

As used herein, the term "infuse" and grammatical variations thereof mean to introduce a solution into a body through a blood vessel. An infusion of the present invention includes, but is not limited to, therapeutic introduction of a fluid other than whole blood into a blood vessel. For example, transfected T cells of the present invention may be infused into a patient's bloodstream, for example, intramuscularly, intravenously, intraarterially, intraperitoneally, or subcutaneously.

It will be appreciated that chimeric antigen receptors (CARs or CAR-Ts) are, and T cell receptors (TCRs) may, be genetically engineered receptors. These engineered receptors may be readily inserted into and expressed by immune cells, including T cells in accordance with techniques known in the art. With a CAR, a single receptor may be programmed to both recognize a specific antigen and, when bound to that antigen, activate the immune cell to attack and destroy the cell bearing that antigen. When these antigens exist on tumor cells, an immune cell that expresses the CAR may target and kill the tumor cell.

CARs may be engineered to bind to an antigen (such as a cell-surface antigen) by incorporating an antigen binding molecule that interacts with that targeted antigen. An "antigen binding molecule" as used herein means any protein that binds a specified target molecule. Antigen binding molecules include, but are not limited to antibodies and binding parts thereof, such as immunologically functional fragments. Peptibodies (*i.e*., Fc fusion molecules comprising peptide binding domains) are another example of suitable antigen binding molecules.

The term "immunologically functional fragment" (or "fragment") of an antigen binding molecule is a species of antigen binding molecule comprising a portion (regardless of how that portion is obtained or synthesized) of an antibody that lacks at least some of the amino acids present in a full-length chain but which is still capable of specifically binding to an antigen. Such fragments are biologically active in that they bind to the target antigen and may compete with other antigen binding molecules, including intact antibodies, for binding to a given epitope. In some embodiments, the fragments are neutralizing fragments. In one aspect, such a fragment will retain at least one CDR present in the full-length light or heavy chain, and in some embodiments will comprise a single heavy chain and/or light chain or portion thereof. These fragments may be produced by recombinant DNA techniques, or may be produced by enzymatic or chemical cleavage of antigen binding molecules, including intact antibodies.

Immunologically functional immunoglobulin fragments include, but are not limited to, scFv fragments, Fab fragments (Fab', F(ab')₂, and the like), one or more CDR, a diabody (heavy chain variable domain on the same polypeptide as a light chain variable domain, connected via a short peptide linker that is too short to permit pairing between the two domains on the same chain), domain antibodies, and single-chain antibodies. These fragments may be derived from any mammalian source, including but not limited to human, mouse, rat, camelid or rabbit. As will be appreciated by one of skill in the art, an antigen binding molecule may include non-protein components.

In some embodiments, the antigen binding molecule binds to an antigen on a tumor cell. In some embodiments, the antigen binding molecule binds to an antigen on a cell involved in a hyperproliferative disease or to a viral or bacterial antigen. In further embodiments, the antigen binding molecule is an antibody or fragment thereof, including one or more of the complementarity determining regions (CDRs) thereof. In further embodiments, the antigen binding molecule is a single chain variable fragment (scFv).

Preferably, the antigen binding molecule is an antibody fragment thereof. More preferably, the antigen binding molecule comprises one or more single chain variable fragment ("scFv"). An scFv is a single chain antibody fragment having the variable regions of the heavy and light chains of an antibody linked together. See U.S. Patent Nos. 7,741,465, 6,319,494, PCT application WO88/01649, and U.S. Patent Nos. 4,946,778 and 5,260,203, the disclosures of which are incorporated by reference in their entirety, as well as Eshhar et al., Cancer Immunol Immunotherapy (1997) 45: 131-136. An scFv retains the parent antibody's ability to specifically interact with a target antigen. scFvs are preferred for use in chimeric antigen receptors because they may be engineered to be expressed as part of a single chain along with the other CAR components. *Id.* See also Krause et al., J. Exp. Med., Volume 188, No. 4, 1998 (619-626); Finney et al., Journal of Immunology, 1998, 161: 2791-2797.

It will be appreciated that the antigen binding molecule is typically contained within the extracellular portion of the CAR such that it is capable of recognizing and binding to the antigen of interest. Bispecific and multispecific CARs are contemplated within the scope of the invention, with specificity to more than one target of interest.

In certain embodiments, the polypeptide structure of the antigen binding molecules is based on antibodies, including, but not limited to, monoclonal antibodies, bispecific antibodies, minibodies, domain antibodies, synthetic antibodies (sometimes referred to herein as "antibody mimetics"), chimeric antibodies, humanized antibodies, human antibodies, antibody fusions (sometimes referred to herein as "antibody conjugates"), and fragments thereof, respectively. In some embodiments, the antigen binding molecule comprises or consists of avimers.

An antigen binding molecule is said to "specifically bind" its target antigen when the dissociation constant (K_{d}) is ∼1×10⁻⁷ M. The antigen binding molecule specifically binds antigen with "high affinity" when the K_{d} is 1-5×10⁻⁹ M, and with "very high affinity" when the K_{d} is 1-5×10⁻¹⁰ M. In one embodiment, the antigen binding molecule has a K_{d} of 10⁻⁹ M. In one embodiment, the off-rate is <1×10⁻⁵.

Those of ordinary skill in the art will understand that the target molecules of the present invention may be any molecule, the specificity for which is desired to be transferred to transfected T cells. The terms "target molecule" or "antigen" refers to a molecule or a portion of a molecule capable of being bound by an antigen binding molecule. In certain embodiments, a target may have one or more epitopes.

Preferably, target molecules of the present invention include, but are not limited to, blood borne cancer-associated antigens. Non-limiting examples of blood borne cancer-associated antigens include antigens associated with one or more cancers selected from the group consisting of acute myeloid leukemia (AML), chronic myelogenous leukemia (CML), chronic myelomonocytic leukemia (CMML), juvenile myelomonocytic leukemia, atypical chronic myeloid leukemia, acute promyelocytic leukemia (APL), acute monoblastic leukemia, acute erythroid leukemia, acute megakaryoblastic leukemia, lymphoblastic leukemia, B-lineage acute lymphoblastic leukemia, B-cell chronic lymphocytic leukemia, B-cell non-Hodgkin's lymphoma, myelodysplastic syndrome (MDS), myeloproliferative disorder, myeloid neoplasm, myeloid sarcoma), and Blastic Plasmacytoid Dendritic Cell Neoplasm (BPDCN).

In some embodiments, the antigen is selected from a tumor-associated surface antigen, such as 5T4, alphafetoprotein (AFP), B7-1 (CD80), B7-2 (CD86), BCMA, B-human chorionic gonadotropin, CA-125, carcinoembryonic antigen (CEA), carcinoembryonic antigen (CEA), CD123, CD133, CD138, CD19, CD20, CD22, CD23, CD24, CD25, CD30, CD33, CD34, CD4, CD40, CD44, CD56, CD8, CLL-1, c-Met, CMV-specific antigen, CSPG4, CTLA-4, disialoganglioside GD2, ductal-epithelial mucine, EBV-specific antigen, EGFR variant III (EGFRvIII), ELF2M, endoglin, ephrin B2, epidermal growth factor receptor (EGFR), epithelial cell adhesion molecule (EpCAM), epithelial tumor antigen, ErbB2 (HER2/neu), fibroblast associated protein (fap), FLT3, folate binding protein, GD2, GD3, glioma-associated antigen, glycosphingolipids, gp36, HBV- specific antigen, HCV-specific antigen, HER1-HER2, HER2-HER3 in combination, HERV-K, high molecular weight-melanoma associated antigen (HMW-MAA), HIV-1 envelope glycoprotein gp41, HPV-specific antigen, human telomerase reverse transcriptase, IGFI receptor, IGF-II, IL-11Ralpha, IL-13R-a2, Influenza Virus-specific antigen; CD38, insulin growth factor (IGFl)-l, intestinal carboxyl esterase, kappa chain, LAGA-la, lambda chain, Lassa Virus-specific antigen, lectin-reactive AFP, lineage-specific or tissue specific antigen such as CD3, MAGE, MAGE-A1, major histocompatibility complex (MHC) molecule, major histocompatibility complex (MHC) molecule presenting a tumor-specific peptide epitope, M-CSF, melanoma-associated antigen, mesothelin, mesothelin, MN-CA IX, MUC-1, mut hsp72, mutated p53, mutated p53, mutated ras, neutrophil elastase, NKG2D, Nkp30, NY-ESO-1, p53, PAP, prostase, prostase specific antigen (PSA), prostate carcinoma tumor antigen-1 (PCTA-1), prostate-specific antigen, prostein, PSMA, RAGE-1, ROR1, RU1, RU2 (AS), surface adhesion molecule, surviving and telomerase, TAG-72, the extra domain A (EDA) and extra domain B (EDB) of fibronectin and the Al domain of tenascin-C (TnC Al) , thyroglobulin, tumor stromal antigens, vascular endothelial growth factor receptor-2 (VEGFR2), virus-specific surface antigen such as an HIV-specific antigen (such as HIV gpl20), as well as any derivate or variant of these surface markers.

In some embodiments, target molecules of the present invention include viral infection-associated antigens. Viral infections of the present invention may be caused by any virus, including, for example, HIV.

This list of possible target molecules is not intended to be exclusive and those of ordinary skill in the art will be aware of many additional molecules that would be useful to target with the chimeric antigen receptors of the present invention.

Chimeric antigen receptors may incorporate costimulatory (signaling) domains to increase their potency. See U.S. Patent Nos. 7,741,465, and 6,319,494, as well as Krause *et al.* and Finney *et al.* (supra), Song et al., Blood 119:696-706 (2012); Kalos et al., Sci Transl. Med. 3:95 (2011); Porter et al., N. Engl. J. Med. 365:725-33 (2011), and Gross et al., Annu. Rev. Pharmacol. Toxicol. 56:59-83 (2016). For example, CD28 is a costimulatory protein found naturally on T-cells. A variety of costimulatory molecules are set forth herein, but it will be appreciated that additional costimulatory molecules are also included within the scope of this invention.

The complete native amino acid sequence of CD28 is described in NCBI Reference Sequence: NP_006130.1. The complete native CD28 nucleic acid sequence is described in NCBI Reference Sequence: NM_006139.1.

Certain CD28 domains have been used in chimeric antigen receptors. In accordance with the present invention, it has now been found that a novel CD28 extracellular (hinge) construct, termed "CD28T", unexpectedly provides certain benefits when utilized in a CAR construct. This construct demonstrates the ability to retain (and at times exceed) the properties of CD28-containing CARs, despite truncation (removal) of multiple amino acids from the extracellular CD28 sequence. These benefits include equivalent or superior cytokine production, equivalent or superior cytolytic activity, and/or equivalent or superior CAR expression levels.

The nucleotide sequence of the CD28T molecule, including the extracellular domain, and the CD28 transmembrane and intracellular domains is set forth in SEQ ID NO. 1:

CTTGATAATGAAAAGTCAAACGGAACAATCATTCACGTGAAGGGCAAGCACCTC

TGTCCGTCACCCTTGTTCCCTGGTCCATCCAAGCCATTCTGGGTGTTGGTCGTAGT

GGGTGGAGTCCTCGCTTGTTACTCTCTGCTCGTCACCGTGGCTTTTATAATCTTCT
GGGTTAGATCCAAAAGAAGCCGCCTGCTCCATAGCGATTACATGAATATGACTC
CACGCCGCCCTGGCCCCACAAGGAAACACTACCAGCCTTACGCACCACCTAGAG
ATTTCGCTGCCTATCGGAGC

The corresponding amino acid sequence is set forth in SEQ ID NO. 2:

LDNEKSNGTIIHVKGKHLCPSPLFPGPSKPFWVLVVVGGVLACYSLLVTVAFIIFWVR

SK RSRLLHSDYM NMTPRRPGPT RKHYQPYAPP RDFAAYRS

The nucleotide sequence of the extracellular portion of CD28T is set forth in SEQ ID NO. 3:

CTTGATAATGAAAAGTCAAACGGAACAATCATTCACGTGAAGGGCAAGCACCTC

TGTCCGTCACCCTTGTTCCCTGGTCCATCCAAGCCA

The corresponding amino acid sequence of the CD28T extracellular domain is set forth in SEQ ID NO. 4:
LDNEKSNGTI IHVKGKHLCP SPLFPGPSKP

The nucleotide sequence of the CD28 transmembrane domain is set forth in SEQ ID NO. 5:

TTCTGGGTGTTGGTCGTAGTGGGTGGAGTCCTCGCTTGTTACTCTCTGCTCGTCAC

CGTGGCTTTTATAATCTTCTGGGTT

The amino acid sequence of the CD28 transmembrane domain is set forth in SEQ ID NO. 6:
FWVLVVVGGV LACYSLLVTV AFIIFWV

The nucleotide sequence of the CD28 intracellular signaling domain is set forth in SEQ ID NO. 7:

AGATCCAAAAGAAGCCGCCTGCTCCATAGCGATTACATGAATATGACTCCACGC

CGCCCTGGCCCCACAAGGAAACACTACCAGCCTTACGCACCACCTAGAGATTTC

GCTGCCTATCGGAGC

The amino acid sequence of the CD28 intracellular signaling domain is set forth in SEQ ID NO. 8:
RSKRSRLLHSDYMNMTPRRPGPTRKHYQPYAPPRDFAAYRS

Additional CD28 sequences suitable for use in the invention include the CD28 nucleotide sequence set forth in SEQ ID NO. 11:

ATTGAGGTGATGTATCCACCGCCTTACCTGGATAACGAAAAGAGTAACGGTACC

ATCATTCACGTGAAAGGTAAACACCTGTGTCCTTCTCCCCTCTTCCCCGGGCCAT

CAAAGCCC

The corresponding amino acid sequence is set forth in SEQ ID NO. 12:
IEVMYPPPYLDNEKSNGTIIHVKGKHLCPSPLFPGPSKP

It will be appreciated that the invention relates to antigen binding molecules, CARs, TCRs, and the like comprising at least one isolated nucleic acid sequence of SEQ ID NO. 1 or SEQ ID NO. 3. It will further be appreciated that the invention relates to antigen binding molecules, CARs, TCRs, and the like wherein the extracellular portion consists of at least one isolated nucleic acid sequence of SEQ ID NO. 1 or SEQ ID NO. 3. Additionally, it will be appreciated that the invention relates to antigen binding molecules, CARs, TCRs, and the like wherein the extracellular portion consists essentially of at least one isolated nucleic acid sequence of SEQ ID NO. 1 or SEQ ID NO. 3.

It will be appreciated that the invention relates to antigen binding molecules, CARs, TCRs, and the like comprising at least one amino acid sequence of SEQ ID NO. 2 or SEQ ID NO. 4. It will further be appreciated that the invention relates to antigen binding molecules, CARs, TCRs, and the like wherein the extracellular portion consists of at least one amino acid sequence of SEQ ID NO. 2 or SEQ ID NO. 4. It will also be appreciated that the invention relates to antigen binding molecules, CARs, TCRs, and the like wherein the extracellular portion consists essentially of at least one amino acid sequence of SEQ ID NO. 2 or SEQ ID NO. 4.

Another suitable source of extracellular and/or transmembrane domains may be derived from (or correspond to) some or all of CD8.

The nucleotide sequence of a suitable CD8 extracellular and transmembrane domain is set forth in SEQ ID NO. 13:

GCTGCAGCATTGAGCAACTCAATAATGTATTTTAGTCACTTTGTACCAGTGTTCTT
GCCGGCTAAGCCTACTACCACACCCGCTCCACGGCCACCTACCCCAGCTCCTACC
ATCGCTTCACAGCCTCTGTCCCTGCGCCCAGAGGCTTGCCGACCGGCCGCAGGGG
GCGCTGTTCATACCAGAGGACTGGATTTCGCCTGCGATATCTATATCTGGGCACC
CCTGGCCGGAACCTGCGGCGTACTCCTGCTGTCCCTGGTCATCACGCTCTATTGT
AATCACAGGAAC

The corresponding amino acid sequence is set forth in SEQ ID NO. 14:

AAALSNSIMYFSHFVPVFLPAKPTTTPAPRPPTPAPTIASQPLSLRPEACRPAAGGAVH

TRGLDFACDIYIWAPLAGTCGVLLLSLVITLYCNHRN

Structurally, it will be appreciated that the domains described herein correspond to locations relative to an immune or other cell. These domains thus may be part of the (i) "hinge" or extracellular (EC) domain, (ii) the transmembrane (TM) domain, and/or (iii) the intracellular/cytoplasmic domain (IC). The intracellular component frequently comprises, in part, an activating domain such as a portion of a member of the CD3 family, preferably CD3 zeta. This domain is capable of activating the T cell upon binding of the antigen binding molecule to its target. It will be appreciated that the intracellular domain typically further comprises one or more costimulatory molecules as described herein.

A "costimulatory molecule" as used herein refers to a molecule that provides a signal which mediates a T cell response, including, but not limited to, proliferation, activation, differentiation, and the like. Costimulatory molecules may provide a signal in addition to the primary signal provided by an activating molecule as described herein.

It will be appreciated that suitable costimulatory domains within the scope of the invention may be derived from (or correspond to) costimulatory molecules, such as, for example, CD28, CD28T, OX40, 4-1BB/CD137, CD2, CD3 (alpha, beta, delta, epsilon, gamma, zeta), CD4, CD5, CD7, CD9, CD16, CD22, CD27, CD30, CD 33, CD37, CD40, CD 45, CD64, CD80, CD86, CD134, CD137, CD154, PD-1, ICOS, lymphocyte function-associated antigen-1 (LFA-1 (CDl la/CD18), CD247, CD276 (B7-H3), LIGHT (tumor necrosis factor superfamily member 14; TNFSF14), NKG2C, Ig alpha (CD79a), DAP-10, Fc gamma receptor, MHC class I molecule, TNF, TNFr, integrin, signaling lymphocytic activation molecule, BTLA, Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl-ld, ITGAE, CD103, ITGAL, CDl-la, LFA-1, ITGAM, CDl-lb, ITGAX, CDl-lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, CD83 ligand, or fragments or combinations thereof. It will be appreciated that additional costimulatory molecules, or fragments thereof, not listed above are within the scope of the invention.

In some embodiments, the costimulatory domain may comprise all or a portion of the 4-1BB nucleic acid sequence set forth in SEQ ID NO. 140, and the corresponding amino acid sequence as set forth in SEQ ID NO. 141. In other embodiments, the costimulatory domain may comprise all or a portion of the amino acid sequence of OX40 as set forth in SEQ ID NO. 142. See also Hombach et al., Oncoimmunology. 2012 Jul. 1; 1(4): 458-466. In still other embodiments, the costimulatory domain may comprise all or a portion of the ICOS molecule as described in Guedan et al., August 14, 2014; Blood: 124 (7) and Shen et al., Journal of Hematology & Oncology (2013) 6:33. In still other embodiments, the costimulatory domain may comprise all or a portion of CD27 as described in Song et al., Oncoimmunology. 2012 Jul. 1;1(4): 547-549.

The engineered T cells of the invention comprise an antigen binding molecule (such as an scFv), an extracellular domain (which may comprise a "hinge" domain), a transmembrane domain, and an intracellular domain. The intracellular domain may comprise at least in part an activating domain, preferably comprised of a CD3 family member such as CD3 zeta, CD3 epsilon, CD3 gamma, or portions thereof.

It will further be appreciated that the antigen binding molecule (*e.g*., one or more scFvs) is engineered such that it is located in the extracellular portion of the molecule/construct, such that it is capable of recognizing and binding to its target or targets.

It will be appreciated that the hinge region may contain some or all of a member of the immunoglobulin family such as IgG1, IgG2, IgG3, IgG4, IgA, IgD, IgE, IgM, or fragment thereof.

In some embodiments, the extracellular domain is positioned between the antigen binding molecule and the transmembrane domain.

Extracellular domains of particular use in this invention may be derived from (*i.e*., comprise) all or some of CD28, OX-40, 4-1BB/CD137, CD2, CD7, CD27, CD30, CD40, programmed death-1 (PD-1), inducible T cell costimulator (ICOS), lymphocyte function-associated antigen-1 (LFA-1, CDl-la/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof. The extracellular domain may be derived either from a natural or from a synthetic source.

Extracellular domains often comprise the hinge portion, sometimes referred to as the "spacer" region. A variety of hinges may be employed in accordance with the invention, including portions or derivatives of the molecules described herein.

The CAR may be designed with a transmembrane domain that is fused to the extracellular domain of the CAR. It may similarly be fused to the intracellular domain of the CAR. In some instances, the transmembrane domain may be selected or modified by amino acid substitution to avoid binding of such domains to the transmembrane domains of the same or different surface membrane proteins to minimize interactions with other members of the receptor complex. The transmembrane domain may be derived either from a natural or from a synthetic source. Where the source is natural, the domain may be derived from any membrane-bound or transmembrane protein. Transmembrane regions of particular use in this invention may be derived from (comprise, or correspond to) CD28, CD28T, OX-40, 4-1BB/CD137, CD2, CD7, CD27, CD30, CD40, programmed death-1 (PD-1), inducible T cell costimulator (ICOS), lymphocyte function-associated antigen-1 (LFA-1, CDl-la/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof.

Optionally, short linkers may form linkages between any or some of the extracellular, transmembrane, and intracellular domains of the CAR.

In other embodiments, the transmembrane domain in the CAR of the invention is a CD8 transmembrane domain. In one embodiment, the CD8 transmembrane domain comprises the transmembrane portion of the nucleic acid sequence of SEQ ID NO: 13. In another embodiment, the CD8 transmembrane domain comprises the nucleic acid sequence that encodes the transmembrane amino acid sequence contained within SEQ ID NO: 14.

In certain embodiments, the transmembrane domain in the CAR of the invention is the CD28 transmembrane domain. In one embodiment, the CD28 transmembrane domain comprises the nucleic acid sequence of SEQ ID NO: 5. In one embodiment, the CD28 transmembrane domain comprises the nucleic acid sequence that encodes the amino acid sequence of SEQ ID NO: 6. In another embodiment, the CD28 transmembrane domain comprises the amino acid sequence of SEQ ID NO: 6.

The intracellular (cytoplasmic) domain of the engineered T cells of the invention may provide activation of at least one of the normal effector functions of the immune cell. Effector function of a T cell, for example, may refer to cytolytic activity or helper activity, including the secretion of cytokines.

It will be appreciated that suitable intracellular molecules include (*i.e*., comprise), but are not limited to signaling domains derived from (or corresponding to) CD28, CD28T, OX-40, 4-1BB/CD137, CD2, CD7, CD27, CD30, CD40, programmed death-1 (PD-1), inducible T cell costimulator (ICOS), lymphocyte function-associated antigen-1 (LFA-1, CDl-la/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof.

In a preferred embodiment, the intracellular/cytoplasmic domain of the CAR may be designed to comprise the CD3 zeta domain by itself or combined with any other desired intracellular domain(s) useful in the context of the CAR of the invention. For example, the intracellular domain of the CAR may comprise a CD3 zeta chain portion and a portion of a costimulatory signaling molecule. The intracellular signaling sequences within the intracellular signaling portion of the CAR of the invention may be linked to each other in a random or specified order.

In another preferred embodiment, the intracellular domain is designed to comprise the activating domain of CD3 zeta and a signaling domain of CD28. In another embodiment, the intracellular domain is designed to comprise the activating domain of CD3 zeta and a signaling domain of 4-1BB. In another embodiment, the intracellular domain in the CAR is designed to comprise a portion of CD28 and CD3 zeta, wherein the intracellular CD28 comprises the nucleic acid sequence set forth in SEQ ID NO: 7 and the amino acid sequence set forth in SEQ ID NO. 8. The CD3 zeta nucleic acid sequence is set forth in SEQ ID NO: 9, and the amino acid sequence is set forth in SEQ ID NO. 8.

"Activation" or "stimulation" as used herein, refers to a primary response induced by binding of an activating molecule with its cognate ligand, wherein the binding mediates a signal transduction event.

An "activating molecule" or "stimulating molecule" refers to a molecule on a T cell, *e.g*., the TCR/CD3 complex that specifically binds with a cognate stimulatory ligand present on an antigen present cell. Suitable activating molecules are described herein.

It will be appreciated that suitable activation domains within the scope of the invention may be derived from (or correspond to) activating / stimulating molecules, such as, for example, CD3 or CD3 zeta. CD3 is an element of the T cell receptor on native T cells, and has been shown to be an important intracellular activating element in CARs.

In a preferred embodiment, the CD3 is CD3 zeta, the nucleotide sequence of which is set forth in SEQ ID NO. 9:

AGGGTGAAGTTTTCCAGATCTGCAGATGCACCAGCGTATCAGCAGGGCCAGAAC
CAACTGTATAACGAGCTCAACCTGGGACGCAGGGAAGAGTATGACGTTTTGGAC
AAGCGCAGAGGACGGGACCCTGAGATGGGTGGCAAACCAAGACGAAAAAACCC
CCAGGAGGGTCTCTATAATGAGCTGCAGAAGGATAAGATGGCTGAAGCCTATTC
TGAAATAGGCATGAAAGGAGAGCGGAGAAGGGGAAAAGGGCACGACGGTTTGT
ACCAGGGACTCAGCACTGCTACGAAGGATACTTATGACGCTCTCCACATGCAAG
CCCTGCCACCTAGG

The corresponding amino acid of intracellular CD3 zeta is set forth in SEQ ID NO. 10:

RVKFSRSADAPAYQQGQNQLYNELNLGRREEYDVLDKRRGRDPEMGGKPR

RKNPQEGLYNELQKDKMAEAYSEIGMKGERRRGKGHDGLYQGLSTATKDTYDALH

MQALPPR

It will be appreciated that one preferred orientation of the CARs in accordance with the invention comprises an antigen binding molecule (such as scFv) in tandem with an extracellular and/or hinge domain, a costimulatory domain, and an activating domain. It will be further appreciated that multiple domains may be utilized in tandem.

Exemplary CAR constructs in accordance with the invention are set forth in Table 1:

**Table 1**

| **Construct Name** | **scFv** | **Hinge Domain** | **Activating Domain** |
|---|---|---|---|
| **24C1 CD28T** | 24C1 | CD28T | CD3 zeta |
| **24C1 CD28** | 24C1 | CD28 | CD3 zeta |
| **24C1 CD8** | 24C1 | CD8 | CD3 zeta |
| **24C8 CD28T** | 24C8 | CD28T | CD3 zeta |
| **24C8 CD28** | 24C8 | CD28 | CD3 zeta |
| **24C8 CD8** | 24C8 | CD8 | CD3 zeta |
| **20C5.1 CD28T** | 20C5.1 | CD28T | CD3 zeta |
| **20C5.1 CD28** | 20C5.1 | CD28 | CD3 zeta |
| **20C5.1 CD8** | 20C5.1 | CD8 | CD3 zeta |
| **20C5.2 CD28T** | 20C5.2 | CD28T | CD3 zeta |
| **20C5.2 CD28** | 20C5.2 | CD28 | CD3 zeta |
| **20C5.2 CD8** | 20C5.2 | CD8 | CD3 zeta |

The term "vector" means any molecule or entity (e.g., nucleic acid, plasmid, bacteriophage or virus) used to transfer protein coding information into a host cell. The term "expression vector" or "expression construct" refers to a vector that is suitable for transformation of a host cell and contains nucleic acid sequences that direct and/or control (in conjunction with the host cell) expression of one or more heterologous coding regions operatively linked thereto. An expression construct may include, but is not limited to, sequences that affect or control transcription, translation, and, if introns are present, affect RNA splicing of a coding region operably linked thereto.

The term "host cell" refers to a cell that has been transformed, or is capable of being transformed, with a nucleic acid sequence and thereby expresses a gene of interest. The term includes the progeny of the parent cell, whether or not the progeny is identical in morphology or in genetic make-up to the original parent cell, so long as the gene of interest is present.

The term "transformation" refers to a change in a cell's genetic characteristics, and a cell has been transformed when it has been modified to contain new DNA or RNA. For example, a cell is transformed where it is genetically modified from its native state by introducing new genetic material via transfection, transduction, or other techniques. Following transfection or transduction, the transforming DNA may recombine with that of the cell by physically integrating into a chromosome of the cell, or may be maintained transiently as an episomal element without being replicated, or may replicate independently as a plasmid. A cell is considered to have been "stably transformed" when the transforming DNA is replicated with the division of the cell.

The invention further relates to isolated polynucleotides encoding the chimeric antigen receptors (CARs) and T cell receptors (TCRs) of the present invention, as well as vectors comprising the polynucleotides. Any vector known in the art may be suitable for the present invention. In some embodiments, the vector is a viral vector. In some embodiments, the vector is a retroviral vector (such as pMSVG1), a DNA vector, a murine leukemia virus vector, an SFG vector, a plasmid, a RNA vector, an adenoviral vector, a baculoviral vector, an Epstein Barr viral vector, a papovaviral vector, a vaccinia viral vector, a herpes simplex viral vector, an adenovirus associated vector (AAV), a lentiviral vector (such as pGAR), or any derivative or combination thereof.

The pGAR sequence is as follows:

CTGACGCGCCCTGTAGCGGCGCATTAAGCGCGGCGGGTGTGGTGGTTACGCGCA
GCGTGACCGCTACACTTGCCAGCGCCCTAGCGCCCGCTCCTTTCGCTTTCTTCCCT
TCCTTTCTCGCCACGTTCGCCGGCTTTCCCCGTCAAGCTCTAAATCGGGGGCTCCC
TTTAGGGTTCCGATTTAGTGCTTTACGGCACCTCGACCCCAAAAAACTTGATTAG
GGTGATGGTTCACGTAGTGGGCCATCGCCCTGATAGACGGTTTTTCGCCCTTTGA
CGTTGGAGTCCACGTTCTTTAATAGTGGACTCTTGTTCCAAACTGGAACAACACT

CAACCCTATCTCGGTCTATTCTTTTGATTTATAAGGGATTTTGCCGATTTCGGCCT
ATTGGTTAAAAAATGAGCTGATTTAACAAAAATTTAACGCGAATTTTAACAAAAT
ATTAACGCTTACAATTTGCCATTCGCCATTCAGGCTGCGCAACTGTTGGGAAGGG
CGATCGGTGCGGGCCTCTTCGCTATTACGCCAGCTGGCGAAAGGGGGATGTGCT
GCAAGGCGATTAAGTTGGGTAACGCCAGGGTTTTCCCAGTCACGACGTTGTAAA
ACGACGGCCAGTGAATTGTAATACGACTCACTATAGGGCGACCCGGGGATGGCG
CGCCAGTAATCAATTACGGGGTCATTAGTTCATAGCCCATATATGGAGTTCCGCG
TTACATAACTTACGGTAAATGGCCCGCCTGGCTGACCGCCCAACGACCCCCGCCC
ATTGACGTCAATAATGACGTATGTTCCCATAGTAACGCCAATAGGGACTTTCCAT
TGACGTCAATGGGTGGAGTATTTACGGTAAACTGCCCACTTGGCAGTACATCAAG
TGTATCATATGCCAAGTACGCCCCCTATTGACGTCAATGACGGTAAATGGCCCGC
CTGGCATTATGCCCAGTACATGACCTTATGGGACTTTCCTACTTGGCAGTACATC
TACGTATTAGTCATCGCTATTACCATGCTGATGCGGTTTTGGCAGTACATCAATG
GGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAAC
AACTCCGCCCCATTGACGCAAATGGGCGGTAGGCGTGTACGGTGGGAGGTCTAT
ATAAGCAGAGCTGGTTTAGTGAACCGGGGTCTCTCTGGTTAGACCAGATCTGAGC
CTGGGAGCTCTCTGGCTAACTAGGGAACCCACTGCTTAAGCCTCAATAAAGCTTG
CCTTGAGTGCTTCAAGTAGTGTGTGCCCGTCTGTTGTGTGACTCTGGTAACTAGA
GATCCCTCAGACCCTTTTAGTCAGTGTGGAAAATCTCTAGCAGTGGCGCCCGAAC
AGGGACTTGAAAGCGAAAGGGAAACCAGAGGAGCTCTCTCGACGCAGGACTCG
GCTTGCTGAAGCGCGCACGGCAAGAGGCGAGGGGCGGCGACTGGTGAGTACGCC
AAAAATTTTGACTAGCGGAGGCTAGAAGGAGAGAGATGGGTGCGAGAGCGTCA
GTATTAAGCGGGGGAGAATTAGATCGCGATGGGAAAAAATTCGGTTAAGGCCAG
GGGGAAAGAAAAAATATAAATTAAAACATATAGTATGGGCAAGCAGGGAGCTA

GAACGATTCGCAGTTAATCCTGGCCTGTTAGAAACATCAGAAGGCTGTAGACAA
ATACTGGGACAGCTACAACCATCCCTTCAGACAGGATCAGAAGAACTTAGATCA
TTATATAATACAGTAGCAACCCTCTATTGTGTGCATCAAAGGATAGAGATAAAAG
ACACCAAGGAAGCTTTAGACAAGATAGAGGAAGAGCAAAACAAAAGTAAGACC
ACCGCACAGCAAGCCGCCGCTGATCTTCAGACCTGGAGGAGGAGATATGAGGGA
CAATTGGAGAAGTGAATTATATAAATATAAAGTAGTAAAAATTGAACCATTAGG
AGTAGCACCCACCAAGGCAAAGAGAAGAGTGGTGCAGAGAGAAAAAAGAGCAG
TGGGAATAGGAGCTTTGTTCCTTGGGTTCTTGGGAGCAGCAGGAAGCACTATGGG
CGCAGCGTCAATGACGCTGACGGTACAGGCCAGACAATTATTGTCTGGTATAGT
GCAGCAGCAGAACAATTTGCTGAGGGCTATTGAGGCGCAACAGCATCTGTTGCA
ACTCACAGTCTGGGGCATCAAGCAGCTCCAGGCAAGAATCCTGGCTGTGGAAAG
ATACCTAAAGGATCAACAGCTCCTGGGGATTTGGGGTTGCTCTGGAAAACTCATT
TGCACCACTGCTGTGCCTTGGAATGCTAGTTGGAGTAATAAATCTCTGGAACAGA
TTTGGAATCACACGACCTGGATGGAGTGGGACAGAGAAATTAACAATTACACAA
GCTTAATACACTCCTTAATTGAAGAATCGCAAAACCAGCAAGAAAAGAATGAAC
AAGAATTATTGGAATTAGATAAATGGGCAAGTTTGTGGAATTGGTTTAACATAAC
AAATTGGCTGTGGTATATAAAATTATTCATAATGATAGTAGGAGGCTTGGTAGGT
TTAAGAATAGTTTTTGCTGTACTTTCTATAGTGAATAGAGTTAGGCAGGGATATT
CACCATTATCGTTTCAGACCCACCTCCCAACCCCGAGGGGACCCGACAGGCCCG
AAGGAATAGAAGAAGAAGGTGGAGAGAGAGACAGAGACAGATCCATTCGATTA
GTGAACGGATCTCGACGGTATCGGTTAACTTTTAAAAGAAAAGGGGGGATTGGG
GGGTACAGTGCAGGGGAAAGAATAGTAGACATAATAGCAACAGACATACAAAC
TAAAGAATTACAAAAACAAATTACAAAATTCAAAATTTTATCGCGATCGCGGAA
TGAAAGACCCCACCTGTAGGTTTGGCAAGCTAGCTTAAGTAACGCCATTTTGCAA
GGCATGGAAAATACATAACTGAGAATAGAGAAGTTCAGATCAAGGTTAGGAACA

GAGAGACAGCAGAATATGGGCCAAACAGGATATCTGTGGTAAGCAGTTCCTGCC
CCGGCTCAGGGCCAAGAACAGATGGTCCCCAGATGCGGTCCCGCCCTCAGCAGT
TTCTAGAGAACCATCAGATGTTTCCAGGGTGCCCCAAGGACCTGAAAATGACCCT
GTGCCTTATTTGAACTAACCAATCAGTTCGCTTCTCGCTTCTGTTCGCGCGCTTCT
GCTCCCCGAGCTCAATAAAAGAGCCCACAACCCCTCACTCGGCGCGCCAGTCCTT
CGAAGTAGATCTTTGTCGATCCTACCATCCACTCGACACACCCGCCAGCGGCCGC
TGCCAAGCTTCCGAGCTCTCGAATTAATTCACGGTACCCACCATGGCCTAGGGAG
ACTAGTCGAATCGATATCAACCTCTGGATTACAAAATTTGTGAAAGATTGACTGG
TATTCTTAACTATGTTGCTCCTTTTACGCTATGTGGATACGCTGCTTTAATGCCTTT
GTATCATGCTATTGCTTCCCGTATGGCTTTCATTTTCTCCTCCTTGTATAAATCCTG
GTTGCTGTCTCTTTATGAGGAGTTGTGGCCCGTTGTCAGGCAACGTGGCGTGGTG
TGCACTGTGTTTGCTGACGCAACCCCCACTGGTTGGGGCATTGCCACCACCTGTC
AGCTCCTTTCCGGGACTTTCGCTTTCCCCCTCCCTATTGCCACGGCGGAACTCATC
GCCGCCTGCCTTGCCCGCTGCTGGACAGGGGCTCGGCTGTTGGGCACTGACAATT
CCGTGGTGTTGTCGGGGAAGCTGACGTCCTTTCATGGCTGCTCGCCTGTGTTGC
CACCTGGATTCTGCGCGGGACGTCCTTCTGCTACGTCCCTTCGGCCCTCAATCCA
GCGGACCTTCCTTCCCGCGGCCTGCTGCCGGCTCTGCGGCCTCTTCCGCGTCTTCG
CCTTCGCCCTCAGACGAGTCGGATCTCCCTTTGGGCCGCCTCCCCGCCTGGTTAA
TTAAAGTACCTTTAAGACCAATGACTTACAAGGCAGCTGTAGATCTTAGCCACTT
TTTAAAAGAAAAGGGGGGACTGGAAGGGCGAATTCACTCCCAACGAAGACAAG
ATCTGCTTTTTGCTTGTACTGGGTCTCTCTGGTTAGACCAGATCTGAGCCTGGGAG
CTCTCTGGCTAACTAGGGAACCCACTGCTTAAGCCTCAATAAAGCTTGCCTTGAG
TGCTTCAAGTAGTGTGTGCCCGTCTGTTGTGTGACTCTGGTAACTAGAGATCCCT
CAGACCCTTTTAGTCAGTGTGGAAAATCTCTAGCAGGCATGCCAGACATGATAA
GATACATTGATGAGTTTGGACAAACCACAACTAGAATGCAGTGAAAAAAATGCT

TTATTTGTGAAATTTGTGATGCTATTGCTTTATTTGTAACCATTATAAGCTGCAAT
AAACAAGTTAACAACAACAATTGCATTCATTTTATGTTTCAGGTTCAGGGGGAGG
TGTGGGAGGTTTTTTGGCGCGCCATCGTCGAGGTTCCCTTTAGTGAGGGTTAATT
GCGAGCTTGGCGTAATCATGGTCATAGCTGTTTCCTGTGTGAAATTGTTATCCGCT
CACAATTCCACACAACATACGAGCCGGAAGCATAAAGTGTAAAGCCTGGGGTGC
CTAATGAGTGAGCTAACTCACATTAATTGCGTTGCGCTCACTGCCCGCTTTCCAG
TCGGGAAACCTGTCGTGCCAGCTGCATTAATGAATCGGCCAACGCGCGGGGAGA
GGCGGTTTGCGTATTGGGCGCTCTTCCGCTTCCTCGCTCACTGACTCGCTGCGCTC
GGTCGTTCGGCTGCGGCGAGCGGTATCAGCTCACTCAAAGGCGGTAATACGGTT
ATCCACAGAATCAGGGGATAACGCAGGAAAGAACATGTGAGCAAAAGGCCAGC
AAAAGGCCAGGAACCGTAAAAAGGCCGCGTTGCTGGCGTTTTTCCATAGGCTCC
GCCCCCCTGACGAGCATCACAAAAATCGACGCTCAAGTCAGAGGTGGCGAAACC
CGACAGGACTATAAAGATACCAGGCGTTTCCCCCTGGAAGCTCCCTCGTGCGCTC
TCCTGTTCCGACCCTGCCGCTTACCGGATACCTGTCCGCCTTTCTCCCTTCGGGAA
GCGTGGCGCTTTCTCATAGCTCACGCTGTAGGTATCTCAGTTCGGTGTAGGTCGT
TCGCTCCAAGCTGGGCTGTGTGCACGAACCCCCCGTTCAGCCCGACCGCTGCGCC
TTATCCGGTAACTATCGTCTTGAGTCCAACCCGGTAAGACACGACTTATCGCCAC
TGGCAGCAGCCACTGGTAACAGGATTAGCAGAGCGAGGTATGTAGGCGGTGCTA
CAGAGTTCTTGAAGTGGTGGCCTAACTACGGCTACACTAGAAGAACAGTATTTG
GTATCTGCGCTCTGCTGAAGCCAGTTACCTTCGGAAAAAGAGTTGGTAGCTCTTG
ATCCGGCAAACAAACCACCGCTGGTAGCGGTGGTTTTTTTGTTTGCAAGCAGCAG
ATTACGCGCAGAAAAAAAGGATCTCAAGAAGATCCTTTGATCTTTTCTACGGGGT
CTGACGCTCAGTGGAACGAAAACTCACGTTAAGGGATTTTGGTCATGAGATTATC
AAAAAGGATCTTCACCTAGATCCTTTTAAATTAAAAATGAAGTTTTAAATCAATC
TAAAGTATATATGAGTAAACTTGGTCTGACAGTTACCAATGCTTAATCAGTGAGG

CACCTATCTCAGCGATCTGTCTATTTCGTTCATCCATAGTTGCCTGACTCCCCGTC
GTGTAGATAACTACGATACGGGAGGGCTTACCATCTGGCCCCAGTGCTGCAATG
ATACCGCGAGACCCACGCTCACCGGCTCCAGATTTATCAGCAATAAACCAGCCA
GCCGGAAGGGCCGAGCGCAGAAGTGGTCCTGCAACTTTATCCGCCTCCATCCAG
TCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTAT
GGCTTCATTCAGCTCCGGTTCCCAACGATCAAGGCGAGTTACATGATCCCCCATG
TTGTGCAAAAAAGCGGTTAGCTCCTTCGGTCCTCCGATCGTTGTCAGAAGTAAGT
TGGCCGCAGTGTTATCACTCATGGTTATGGCAGCACTGCATAATTCTCTTACTGTC
ATGCCATCCGTAAGATGCTTTTCTGTGACTGGTGAGTACTCAACCAAGTCATTCT
GAGAATAGTGTATGCGGCGACCGAGTTGCTCTTGCCCGGCGTCAATACGGGATA
ATACCGCGCCACATAGCAGAACTTTAAAAGTGCTCATCATTGGAAAACGTTCTTC
GGGGCGAAAACTCTCAAGGATCTTACCGCTGTTGAGATCCAGTTCGATGTAACCC
ACTCGTGCACCCAACTGATCTTCAGCATCTTTTACTTTCACCAGCGTTTCTGGGTG
AGCAAAAACAGGAAGGCAAAATGCCGCAAAAAAGGGAATAAGGGCGACACGGA
AATGTTGAATACTCATACTCTTCCTTTTTCAATATTATTGAAGCATTTATCAGGGT
TATTGTCTCATGAGCGGATACATATTTGAATGTATTTAGAAAAATAAACAAATAG
GGGTTCCGCGCACATTTCCCCGAAAAGTGCCAC

Suitable additional exemplary vectors include e.g., pBABE-puro, pBABE-neo largeTcDNA, pBABE-hygro-hTERT, pMKO.1 GFP, MSCV-IRES-GFP, pMSCV PIG (Puro IRES GFP empty plasmid), pMSCV-loxp-dsRed-loxp-eGFP-Puro-WPRE, MSCV IRES Luciferase, pMIG, MDH1-PGK-GFP_2.0, TtRMPVIR, pMSCV-IRES-mCherry FP, pRetroX GFP T2A Cre, pRXTN, pLncEXP, and pLXIN-Luc.

In a further embodiment, a mixture of different expression vectors may be used in genetically modifying a donor population of immune effector cells wherein each vector encodes a different CAR as disclosed herein. The resulting transduced immune effector cells form a mixed population of engineered cells, with a proportion of the engineered cells expressing more than one different CARs.

In a preferred embodiment of the present invention, the CAR comprises all or part of an anti-CD 19 scFv, CD28, and CD3 zeta.

Transfected T cells of the present invention may also be created by transfecting the collected T cells with a polynucleotide encoding a T cell receptor (TCR). T cell receptors (TCRs) are molecules found on the surface of T cells that are responsible for recognizing antigen fragments as peptides bound to major histocompatibility complex (MHC) molecules. The TCR is comprised of two different protein chains - in approximately 95% of human TCRs, the TCR consists of an alpha (α) and beta (β) chain. In approximately 5% of human T cells the TCR consists of gamma and delta (γ/δ) chains. Each chain is composed of two extracellular domains: a variable (V) region and a constant (C) region, both of the immunoglobulin superfamily. As in other immunoglobulins, the variable domains of the TCR α-chain and β-chain (or gamma and delta (γ/δ) chains) each have three hypervariable or complementarity determining regions (CDRs). When the TCR engages with antigenic peptide and MHC (peptide/MHC), the T cell becomes activated, enabling it to attack and destroy the target cell.

The TCRs of the present invention may bind to, for example, a tumor-associated antigen. As used herein, "tumor-associated antigen" refers to any antigen that is associated with one or more cancers selected from the group consisting of: adrenocortical carcinoma, anal cancer, bladder cancer, bone cancer, brain cancer, breast cancer, carcinoid cancer, carcinoma, cervical cancer, colon cancer, endometrial cancer, esophageal cancer, extrahepatic bile duct cancer, extracranial germ cell cancer, eye cancer, gallbladder cancer, gastric cancer, germ cell tumor, gestational trophoblastic tumor, head and neck cancer, hypopharyngeal cancer, islet cell carcinoma, kidney cancer, large intestine cancer, laryngeal cancer, leukemia, lip and oral cavity cancer, liver cancer, lung cancer, lymphoma, malignant mesothelioma, Merkel cell carcinoma, mycosis fungoides, myelodysplastic syndrome, myeloproliferative disorders, nasopharyngeal cancer, neuroblastoma, oral cancer, oropharyngeal cancer, osteosarcoma, ovarian epithelial cancer, ovarian germ cell cancer, pancreatic cancer, paranasal sinus and nasal cavity cancer, parathyroid cancer, penile cancer, pituitary cancer, plasma cell neoplasm, prostate cancer, rhabdomyosarcoma, rectal cancer, renal cell cancer, transitional cell cancer of the renal pelvis and ureter, salivary gland cancer, Sezary syndrome, skin cancers, small intestine cancer, soft tissue sarcoma, stomach cancer, testicular cancer, thymoma, thyroid cancer, urethral cancer, uterine cancer, vaginal cancer, vulvar cancer, and Wilms' tumor.

In certain aspects, the present application may be suitable for target molecule to hematologic cancer. In some embodiments, the cancer is of the white blood cells. In other embodiments, the cancer is of the plasma cells. In some embodiments, the cancer is leukemia, lymphoma, or myeloma. In certain embodiments, the cancer is acute lymphoblastic leukemia (ALL) (including non T cell ALL), acute lymphoid leukemia (ALL), and hemophagocytic lymphohistocytosis (HLH)), B cell prolymphocytic leukemia, B-cell acute lymphoid leukemia ("BALL"), blastic plasmacytoid dendritic cell neoplasm, Burkitt's lymphoma, chronic lymphocytic leukemia (CLL), chronic myelogenous leukemia (CML), chronic myeloid leukemia (CML), chronic or acute granulomatous disease, chronic or acute leukemia, diffuse large B cell lymphoma, diffuse large B cell lymphoma (DLBCL), follicular lymphoma, follicular lymphoma (FL), hairy cell leukemia, hemophagocytic syndrome (Macrophage Activating Syndrome (MAS), Hodgkin's Disease, large cell granuloma, leukocyte adhesion deficiency, malignant lymphoproliferative conditions, MALT lymphoma, mantle cell lymphoma, Marginal zone lymphoma, monoclonal gammapathy of undetermined significance (MGUS), multiple myeloma, myelodysplasia and myelodysplastic syndrome (MDS), myeloid diseases including but not limited to acute myeloid leukemia (AML), non-Hodgkin's lymphoma (NHL), plasma cell proliferative disorders (e.g., asymptomatic myeloma (smoldering multiple myeloma or indolent myeloma), plasmablastic lymphoma, plasmacytoid dendritic cell neoplasm, plasmacytomas (e.g., plasma cell dyscrasia; solitary myeloma; solitary plasmacytoma; extramedullary plasmacytoma; and multiple plasmacytoma), POEMS syndrome (Crow-Fukase syndrome; Takatsuki disease; PEP syndrome), primary mediastinal large B cell lymphoma (PMBCL), small cell- or a large cell-follicular lymphoma, splenic marginal zone lymphoma (SMZL), systemic amyloid light chain amyloidosis, T-cell acute lymphoid leukemia (TALL), T-cell lymphoma, transformed follicular lymphoma, Waldenstrom macroglobulinemia, or a combination thereof.

In some embodiments, the antigen is selected from a tumor-associated surface antigen, such as 5T4, alphafetoprotein (AFP), B7-1 (CD80), B7-2 (CD86), BCMA, B-human chorionic gonadotropin, CA-125, carcinoembryonic antigen (CEA), carcinoembryonic antigen (CEA), CD123, CD133, CD138, CD19, CD20, CD22, CD23, CD24, CD25, CD30, CD33, CD34, CD4, CD40, CD44, CD56, CD8, CLL-1, c-Met, CMV-specific antigen, CSPG4, CTLA-4, disialoganglioside GD2, ductal-epithelial mucine, EBV-specific antigen, EGFR variant III (EGFRvIII), ELF2M, endoglin, ephrin B2, epidermal growth factor receptor (EGFR), epithelial cell adhesion molecule (EpCAM), epithelial tumor antigen, ErbB2 (HER2/neu), fibroblast associated protein (fap), FLT3, folate binding protein, GD2, GD3, glioma-associated antigen, glycosphingolipids, gp36, HBV- specific antigen, HCV-specific antigen, HER1-HER2, HER2-HER3 in combination, HERV-K, high molecular weight-melanoma associated antigen (HMW-MAA), HIV-1 envelope glycoprotein gp41, HPV-specific antigen, human telomerase reverse transcriptase, IGFI receptor, IGF-II, IL-11Ralpha, IL-13R-a2, Influenza Virus-specific antigen; CD38, insulin growth factor (IGFl)-l, intestinal carboxyl esterase, kappa chain, LAGA-la, lambda chain, Lassa Virus-specific antigen, lectin-reactive AFP, lineage-specific or tissue specific antigen such as CD3, MAGE, MAGE-A1, major histocompatibility complex (MHC) molecule, major histocompatibility complex (MHC) molecule presenting a tumor-specific peptide epitope, M-CSF, melanoma-associated antigen, mesothelin, mesothelin, MN-CA IX, MUC-1, mut hsp72, mutated p53, mutated p53, mutated ras, neutrophil elastase, NKG2D, Nkp30, NY-ESO-1, p53, PAP, prostase, prostase specific antigen (PSA), prostate carcinoma tumor antigen-1 (PCTA-1), prostate-specific antigen, prostein, PSMA, RAGE-1, ROR1, RU1, RU2 (AS), surface adhesion molecule, surviving and telomerase, TAG-72, the extra domain A (EDA) and extra domain B (EDB) of fibronectin and the Al domain of tenascin-C (TnC Al) , thyroglobulin, tumor stromal antigens, vascular endothelial growth factor receptor-2 (VEGFR2), virus-specific surface antigen such as an HIV-specific antigen (such as HIV gpl20), as well as any derivate or variant of these surface markers.

The TCRs of the present invention may also bind to a viral infection-associated antigen. Viral infection-associated antigens include antigens associated with any viral infection, including, for example, viral infection caused by HIV.

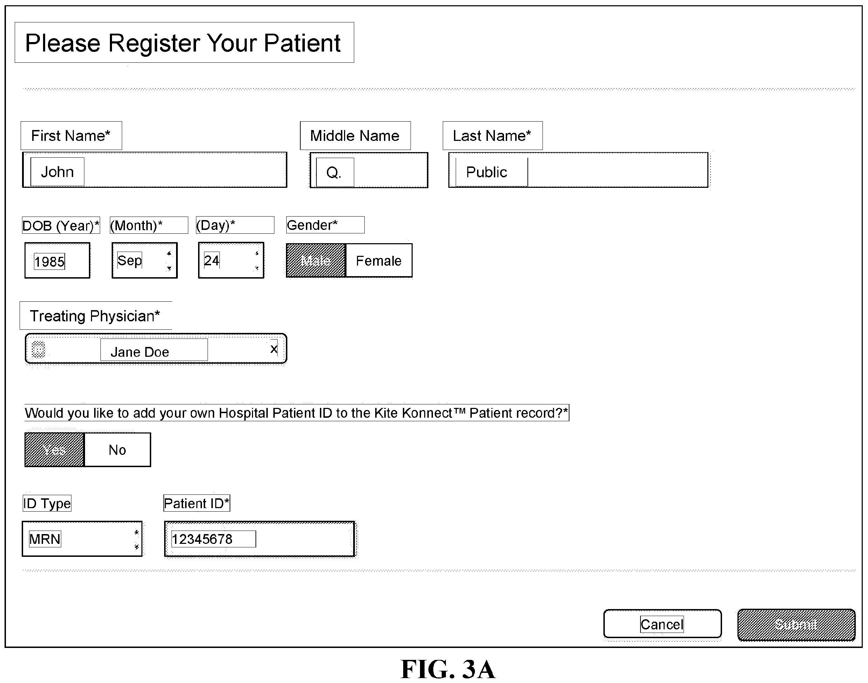

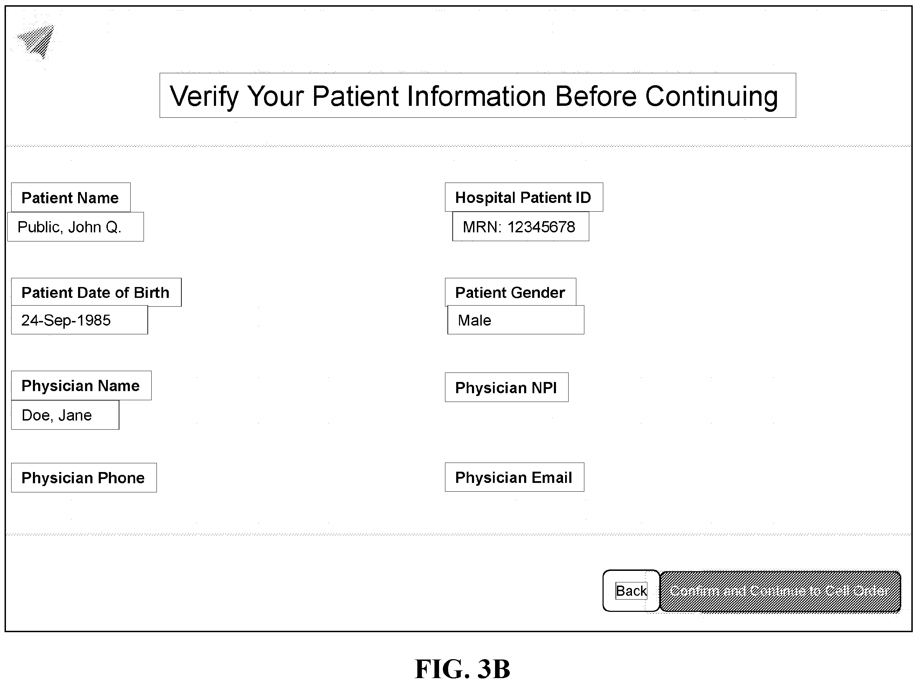

To initiate the patient-specific immunotherapy procedure described herein, a physician or other medical personnel at client computing device 102a accesses the user interface module 108a of server computing device 106 (e.g., via a web portal, web site, or other similar platform). The user interface module 108a generates user interface screens and/or elements for presentation to the physician on the client computing device 102a, in order for the physician to enroll the patient and initiate the patient-specific immunotherapy procedure. The user interface module 108a may generate UI screens to enable the physician to enter the patient's identifying information (e.g., full name, date of birth), demographics (e.g., gender), and healthcare provider information (e.g., physician name, hospital name). The user interface module 108a may also provide a UI element for entry of a healthcare-provider-specific or hospital-specific user identifier (e.g., medical record number, hospital patient ID). FIGS. 3A and 3B are exemplary screenshots generated by the user interface module 108a that enable enrollment of new patients into the system; FIG. 3A depicts the patient enrollment data entry screen, and FIG. 3B depicts a patent information review and confirmation screen.

Turning back to FIG. 2, the client computing device 102a generates a request to create transfected T cells for a patient, and the server computing device 106 receives (202) the request. As described above, the physician at client computing device 102a interacts with the user interface module 108a to enroll the patient by providing the necessary patent information. Once the user interface module 108a receives confirmation from the client computing device 102a that the patient information has been fully entered and is accurate, the user interface module 108a stores the data in database 110. The user interface module 108a also generates (204) a patient-specific identifier that will be used as part of the sample tracking and chain-of-custody / chain-of-identity process described below. In one embodiment, the patient-specific identifier comprises a patient identity element (e.g., a patient ID number), a sales order identifier, and a cell order lot number. For example, the user interface module 108a may generate the patient-specific identifier by mapping the patient identity element, sales order number, and cell order lot number into a database table that is indexed with an identifier (e.g., a nine-digit numeric code) that uniquely identifies the patient, sales order, and cell lot combination.

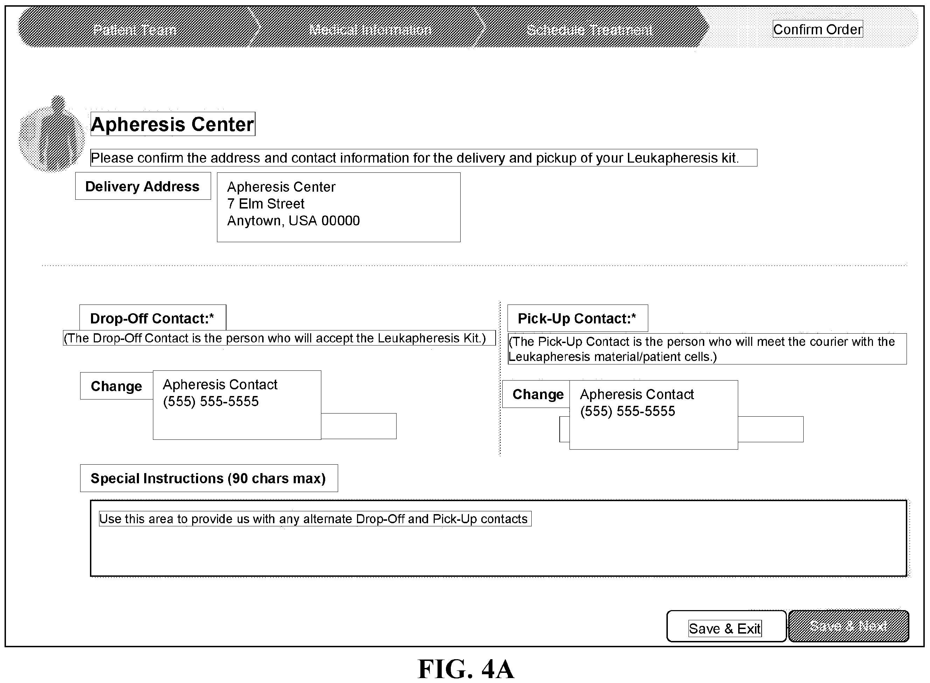

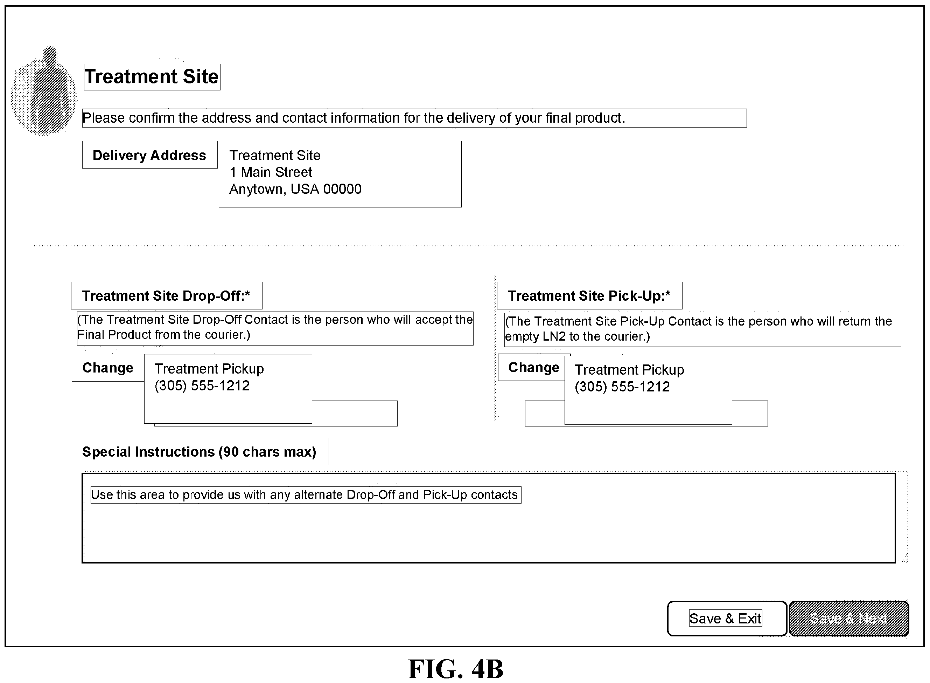

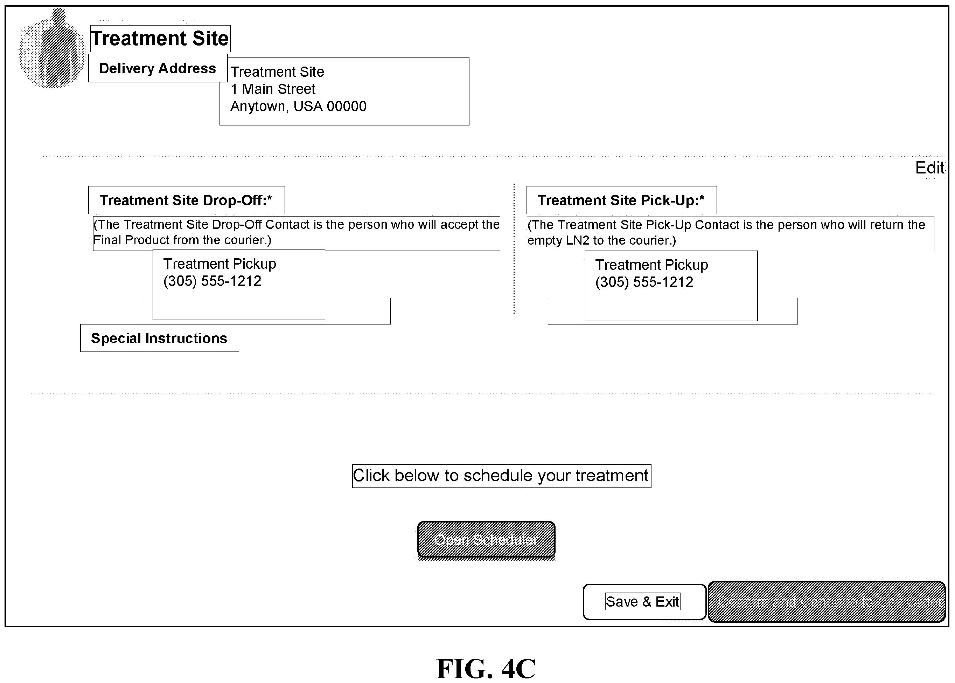

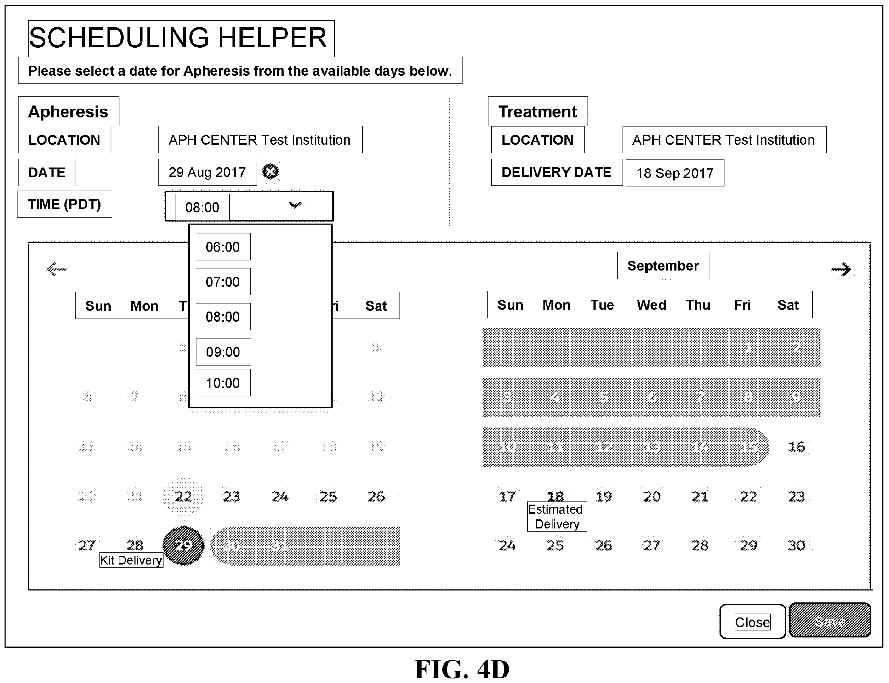

Next, the physician at client computing device 102a interacts with the user interface module 108a to schedule an appointment to obtain the biological material from the patient and, due to the time sensitivity of providing the altered biological material back to the patient quickly, confirming that the manufacturing facility has availability to process the biological material shortly after the material is obtained. The user interface module 108a requests confirmation of the material extraction site (e.g., site name, address, contact information) for drop-off of an extraction kit (e.g., leukapheresis kit) and confirmation of the altered material delivery and treatment site (e.g., site name, address, contact information) for delivery of the material (e.g., transfected T cells) from the manufacturing facility. FIGS. 4A-4D are exemplary screenshots generated by the user interface module 108a that enable confirmation of these sites and scheduling of the appointment; FIG. 4A depicts the drop-off site confirmation screen, FIG. 4B depicts the material delivery site confirmation screen; FIG. 4C depicts the screen to open the appointment scheduler; and FIG. 4D depicts the appointment scheduler. In some embodiments, the user interface module 108a communicates with a remote computing device of the manufacturing facility, in conjunction with the database 110, to coordinate scheduling of the biological material modification to ensure the most efficient processing schedule so that the modified material is returned quickly back to the patient.

Turning back to FIG. 2, once the cell order process is complete as described above, a process 206 is initiated to perform the biological material extraction procedure at the extraction site, ship the extracted material to the manufacturing facility for modification, and send the modified material back to a delivery site for infusion back into the patient's bloodstream. First, the patient arrives at the material extraction site and a procedure (e.g., a leukapheresis procedure) is performed (206a) on a sample of the patient's blood to collect T cells from the sample. When the procedure is performed, a client computing device (e.g., device 102b) at the extraction site communicates with the event tracking module 108b of server computing device 106 to transmit a tracking event to the module 108b that corresponds to performance of the procedure. For example, a clinician at client computing device 102b may submit the tracking event by entering information into a user interface. In another example, the client computing device 102b may automatically transmit the tracking event to the module 108b (e.g., via API) when information about the procedure is captured by the client computing device 102b (e.g., scanning a barcode).

The tracking event may comprise the patient-specific identifier, a timestamp, an event ID (e.g., that indicates a material extraction procedure was performed), and other information relevant to the process (e.g., cell order lot number, sales order number, site location, etc.). The event tracking module 108b stores the tracking event in database 110 based upon the information received from the client computing device 102b. Because this is the first step in the biological material extraction and modification process, the event tracking module 108b notifies the chain of custody module 108c of receipt of the tracking event. The chain of custody module 108c generates a chain of custody data structure (e.g., in database 110) that incorporates the tracking event (and each subsequent tracking event described herein) in an ordered sequence that enables the patient, the physician, the manufacturer, and other parties to understand the precise status of the biological material and to ensure that the biological material is accounted for at all times in avoidance of loss or mishandling. In an example, the chain of custody data structure may be a linked list that connects each of the tracking events together in a sequential manner according to, e.g., timestamp of the tracking event.

Next, the collected T cells are transferred (206b) to a container (e.g., a tube, vial, or other type of biological material carrier) and another tracking event is captured and transmitted to the event tracking module 108b for integration into the chain of custody data structure described above. Then, the container is labeled (206c) with the patient-specific identifier, and another tracking event is captured and transmitted to the event tracking module 108b for communication with the chain of custody module 108c to integrate into the chain of custody data structure. For example, the container that houses the collected T cells is labeled with a barcode comprising the patient-specific identifier, which is then scanned at the extraction site-indicating that the collected T cells are ready for shipment to the manufacturing facility. Upon scanning the barcode, the client computing device 102b generates the tracking event and transmits the event to the event tracking module 108b.

Then, the extraction site transmits (206d) the collected T cells to the manufacturing facility, which performs the procedure to generate the transfected T cells. Both when the collected T cells are shipped to the manufacturing facility and when the collected T cells are received at the manufacturing facility, one or more of the devices used to record the shipment and receipt of the T cells communicate with the event tracking module 108b to transmit a tracking event associated with the particular activity for communication with the chain of custody module 108c to integrate into the chain of custody. In this way, the chain of custody module 108c automatically and continuously updates the chain of custody data structure with the latest information, and that information is reflected in one or more screens generated by the user interface module 108a.

The manufacturing facility then creates (206e) transfected T cells from the collected T cells using a cell modification technique, and a client computing device (e.g., device 102c) generates one or more tracking events based upon the particular cell modification technique being used. For example, a cell modification technique may comprise several phases-such as (i) quality assurance of the collected T cells prior to modification, (ii) modification of the T cells; (ii) release testing of the transfected T cells, and (iv) finalization of the transfected T cells for shipment back to the infusion site. For each of these phases, the client computing device 102c captures a tracking event and transmits the tracking event to the event tracking module 108b for integration by the chain of custody module 108c into the chain of custody data structure.

Once the transfected T cells are shipped, the infusion site receives (206f) the transfected T cells and a client computing device (e.g., device 102d) generates a tracking event for transmission to the event tracking module 108b for integration by the chain of custody module 108c into the chain of custody data structure. For example, the client computing device 102d may scan a barcode associated with the shipment and/or the transfected T cells to automatically generate the tracking event and transmit the event to the server computing device 106.

After receipt, the transfected T cells are infused (206g) into the patient's bloodstream, thereby completing the process. At the same time, the client computing device 102d generates a tracking event and transmits the event to the event tracking module 108b for integration by the chain of custody module 108c into the chain of custody data structure.

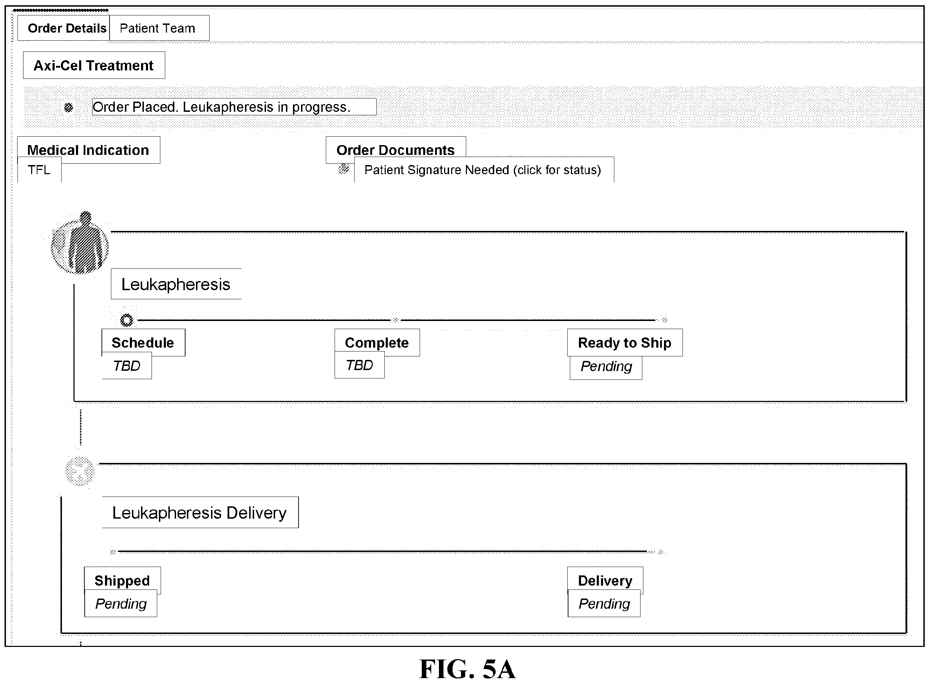

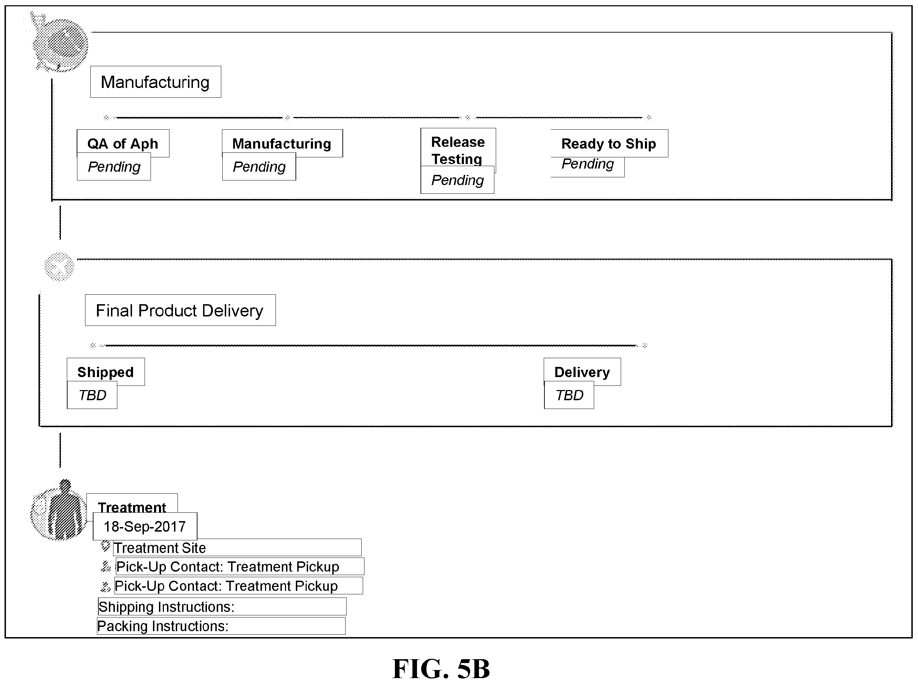

FIGS. 5A and 5B are exemplary screenshots generated by the user interface module 108a to enable the client computing devices 102a-102d to view the chain of custody associated with a particular patient, biological material, and cell modification process. As shown in FIG. 5A, the chain of custody of the biological material during the leukapheresis process (including the steps of scheduling the procedure, completing the procedure, and having the extracted T cells ready for shipment) is captured in a timeline at the top of the screen, where each step of the leukapheresis process is associated with a point on the timeline, and the chain of custody of the biological material during the delivery process (e.g., T cells shipped from extraction site, T cells delivered to manufacturing facility) is captured in a timeline at the bottom of the screen. When the event tracking module 108b and chain of custody module 108c record a tracking event as described above, the user interface module 108a traverses the chain of custody data structure to graphically represent the current status of the chain of custody on screen.

As shown in FIG. 5B, the chain of custody of the biological material during the manufacturing process (including QA, manufacturing, release testing, and finalizing for shipment) is shown in a timeline at the top of the screen, and the chain of custody of the biological material during the final product delivery process (including shipment and delivery to the infusion site) is shown in the middle of the screen. In addition, the treatment details, including the treatment date, are displayed at the bottom of the screen. Also, the chain of custody is constantly associated with the specific patient-thereby ensuring a complete chain of identity between the patient and the biological material during all phases of manufacturing.

The above-described techniques may be implemented in digital and/or analog electronic circuitry, or in computer hardware, firmware, software, or in combinations of them. The implementation may be as a computer program product, i.e., a computer program tangibly embodied in a machine-readable storage device, for execution by, or to control the operation of, a data processing apparatus, e.g., a programmable processor, a computer, and/or multiple computers. A computer program may be written in any form of computer or programming language, including source code, compiled code, interpreted code and/or machine code, and the computer program may be deployed in any form, including as a stand-alone program or as a subroutine, element, or other unit suitable for use in a computing environment. A computer program may be deployed to be executed on one computer or on multiple computers at one or more sites. The computer program may be deployed in a cloud computing environment (e.g., Amazon^{®} AWS, Microsoft^{®} Azure, IBM^{®}).

Method steps may be performed by one or more processors executing a computer program to perform functions of the invention by operating on input data and/or generating output data. Method steps may also be performed by, and an apparatus may be implemented as, special purpose logic circuitry, e.g., a FPGA (field programmable gate array), a FPAA (field-programmable analog array), a CPLD (complex programmable logic device), a PSoC (Programmable System-on-Chip), ASIP (application-specific instruction-set processor), or an ASIC (application-specific integrated circuit), or the like. Subroutines may refer to portions of the stored computer program and/or the processor, and/or the special circuitry that implement one or more functions.

Processors suitable for the execution of a computer program include, by way of example, special purpose microprocessors specifically programmed with instructions executable to perform the methods described herein. Generally, a processor receives instructions and data from a read-only memory or a random access memory or both. The essential elements of a computer are a processor for executing instructions and one or more memory devices for storing instructions and/or data. Memory devices, such as a cache, may be used to temporarily store data. Memory devices may also be used for long-term data storage. Generally, a computer also includes, or is operatively coupled to receive data from or transfer data to, or both, one or more mass storage devices for storing data, e.g., magnetic, magneto-optical disks, or optical disks. A computer may also be operatively coupled to a communications network in order to receive instructions and/or data from the network and/or to transfer instructions and/or data to the network. Computer-readable storage mediums suitable for embodying computer program instructions and data include all forms of volatile and non-volatile memory, including by way of example semiconductor memory devices, e.g., DRAM, SRAM, EPROM, EEPROM, and flash memory devices; magnetic disks, e.g., internal hard disks or removable disks; magneto-optical disks; and optical disks, e.g., CD, DVD, HD-DVD, and Blu-ray disks. The processor and the memory may be supplemented by and/or incorporated in special purpose logic circuitry.

To provide for interaction with a user, the above described techniques may be implemented on a computing device in communication with a display device, e.g., a CRT (cathode ray tube), plasma, or LCD (liquid crystal display) monitor, a mobile device display or screen, a holographic device and/or projector, for displaying information to the user and a keyboard and a pointing device, e.g., a mouse, a trackball, a touchpad, or a motion sensor, by which the user may provide input to the computer (e.g., interact with a user interface element). Other kinds of devices may be used to provide for interaction with a user as well; for example, feedback provided to the user may be any form of sensory feedback, e.g., visual feedback, auditory feedback, or tactile feedback; and input from the user may be received in any form, including acoustic, speech, and/or tactile input.

The above-described techniques may be implemented in a distributed computing system that includes a back-end component. The back-end component may, for example, be a data server, a middleware component, and/or an application server. The above described techniques may be implemented in a distributed computing system that includes a front-end component. The front-end component may, for example, be a client computer having a graphical user interface, a Web browser through which a user may interact with an example implementation, and/or other graphical user interfaces for a transmitting device. The above described techniques may be implemented in a distributed computing system that includes any combination of such back-end, middleware, or front-end components.

The components of the computing system may be interconnected by transmission medium, which may include any form or medium of digital or analog data communication (e.g., a communication network). Transmission medium may include one or more packet-based networks and/or one or more circuit-based networks in any configuration. Packet-based networks may include, for example, the Internet, a carrier internet protocol (IP) network (e.g., local area network (LAN), wide area network (WAN), campus area network (CAN), metropolitan area network (MAN), home area network (HAN)), a private IP network, an IP private branch exchange (IPBX), a wireless network (e.g., radio access network (RAN), Bluetooth, near field communications (NFC) network, Wi-Fi, WiMAX, general packet radio service (GPRS) network, HiperLAN), and/or other packet-based networks. Circuit-based networks may include, for example, the public switched telephone network (PSTN), a legacy private branch exchange (PBX), a wireless network (e.g., RAN, code-division multiple access (CDMA) network, time division multiple access (TDMA) network, global system for mobile communications (GSM) network), and/or other circuit-based networks.

Information transfer over transmission medium may be based on one or more communication protocols. Communication protocols may include, for example, Ethernet protocol, Internet Protocol (IP), Voice over IP (VOIP), a Peer-to-Peer (P2P) protocol, Hypertext Transfer Protocol (HTTP), Session Initiation Protocol (SIP), H.323, Media Gateway Control Protocol (MGCP), Signaling System #7 (SS7), a Global System for Mobile Communications (GSM) protocol, a Push-to-Talk (PTT) protocol, a PTT over Cellular (POC) protocol, Universal Mobile Telecommunications System (UMTS), 3GPP Long Term Evolution (LTE) and/or other communication protocols.

Devices of the computing system may include, for example, a computer, a computer with a browser device, a telephone, an IP phone, a mobile device (e.g., cellular phone, personal digital assistant (PDA) device, smart phone, tablet, laptop computer, electronic mail device), and/or other communication devices. The browser device includes, for example, a computer (e.g., desktop computer and/or laptop computer) with a World Wide Web browser (e.g., Chrome^{™} from Google, Inc., Microsoft^{®} Internet Explorer^{®} available from Microsoft Corporation, and/or Mozilla^{®} Firefox available from Mozilla Corporation). Mobile computing device include, for example, a Blackberry^{®} from Research in Motion, an iPhone^{®} from Apple Corporation, and/or an Android^{™}-based device. IP phones include, for example, a Cisco^{®} Unified IP Phone 7985G and/or a Cisco^{®} Unified Wireless Phone 7920 available from Cisco Systems, Inc.

### Additional Definitions

The terms "polypeptide" or "protein" refer to a macromolecule having the amino acid sequence of a protein, including deletions from, additions to, and/or substitutions of one or more amino acids of the native sequence, and preferably no more than 8 amino acid substitutions therein. Preferably, the polypeptides or proteins are isolated as defined herein. The term "polypeptide fragment" refers to an isolated polypeptide that has an amino-terminal deletion, a carboxyl-terminal deletion, and/or an internal deletion as compared with the full-length native protein. Such fragments may also contain modified amino acids as compared with the native protein. Useful polypeptide fragments include immunologically functional fragments of antigen binding molecules. Useful fragments include but are not limited to one or more CDR regions, variable domains of a heavy and/or light chain, a portion of other portions of an antibody chain, and the like.

The term "antibody" refers to an intact immunoglobulin of any isotype, or a fragment thereof that may compete with the intact antibody for specific binding to the target antigen / molecule, and includes, for instance, chimeric, humanized, fully human, and bispecific antibodies. An "antibody" is a species of an antigen binding molecule as defined herein. An intact antibody will generally comprise at least two full-length heavy chains and two full-length light chains, but in some instances may include fewer chains such as antibodies naturally occurring in camelids which may comprise only heavy chains. Antibodies may be derived solely from a single source, or may be chimeric, that is, different portions of the antibody may be derived from two different antibodies as described further below. The antigen binding molecules, antibodies, or binding fragments may be produced in hybridomas, by recombinant DNA techniques, or by enzymatic or chemical cleavage of intact antibodies. Unless otherwise indicated, the term "antibody" includes, in addition to antibodies comprising two full-length heavy chains and two full-length light chains, derivatives, variants, fragments, and muteins thereof, examples of which are described below. Furthermore, unless explicitly excluded, antibodies include monoclonal antibodies, bispecific antibodies, minibodies, domain antibodies, synthetic antibodies (sometimes referred to herein as "antibody mimetics"), chimeric antibodies, humanized antibodies, human antibodies, antibody fusions (sometimes referred to herein as "antibody conjugates") and fragments thereof, respectively.

The variable regions typically exhibit the same general structure of relatively conserved framework regions (FR) joined by the 3 hypervariable regions (*i.e*., "CDRs"). The CDRs from the two chains of each pair typically are aligned by the framework regions, which may enable binding to a specific epitope. From N-terminal to C-terminal, both light and heavy chain variable regions typically comprise the domains FR1, CDR1, FR2, CDR2, FR3, CDR3 and FR4. By convention, CDR regions in the heavy chain are typically referred to as HC CDR1, CDR2, and CDR3. The CDR regions in the light chain are typically referred to as LC CDR1, CDR2, and CDR3. The assignment of amino acids to each domain is typically in accordance with the definitions of Kabat, Chothia, or the AbM definition.

The term "Kabat numbering" and like terms are recognized in the art and refer to a system of numbering amino acid residues in the heavy and light chain variable regions of an antibody, or an antigen-binding portion thereof. In certain aspects, the CDRs of an antibody may be determined according to the Kabat numbering system (*see*, *e.g.*, Kabat EA & Wu TT (1971) Ann NY Acad Sci 190: 382-391 and Kabat EA et al., (1991) Sequences of Proteins of Immunological Interest, Fifth Edition, U.S. Department of Health and Human Services, NIH Publication No. 91-3242). Using the Kabat numbering system, CDRs within an antibody heavy chain molecule are typically present at amino acid positions 31 to 35, which optionally may include one or two additional amino acids, following 35 (referred to in the Kabat numbering scheme as 35A and 35B) (CDR1), amino acid positions 50 to 65 (CDR2), and amino acid positions 95 to 102 (CDR3). Using the Kabat numbering system, CDRs within an antibody light chain molecule are typically present at amino acid positions 24 to 34 (CDR1), amino acid positions 50 to 56 (CDR2), and amino acid positions 89 to 97 (CDR3). In a specific embodiment, the CDRs of the antibodies described herein have been determined according to the Kabat numbering scheme.

In certain aspects, the CDRs of an antibody may be determined according to the Chothia numbering scheme, which refers to the location of immunoglobulin structural loops (*see*, *e.g*., Chothia C & Lesk AM, (1987), J Mol Biol 196: 901-917; Al-Lazikani B et al., (1997) J Mol Biol 273: 927-948; Chothia C et al., (1992) J Mol Biol 227: 799-817; Tramontano A et al., (1990) J Mol Biol 215(1): 175-82; and U.S. Patent No. 7,709,226). Typically, when using the Kabat numbering convention, the Chothia CDR-H1 loop is present at heavy chain amino acids 26 to 32, 33, or 34, the Chothia CDR-H2 loop is present at heavy chain amino acids 52 to 56, and the Chothia CDR-H3 loop is present at heavy chain amino acids 95 to 102, while the Chothia CDR-L1 loop is present at light chain amino acids 24 to 34, the Chothia CDR-L2 loop is present at light chain amino acids 50 to 56, and the Chothia CDR-L3 loop is present at light chain amino acids 89 to 97. The end of the Chothia CDR-HI loop when numbered using the Kabat numbering convention varies between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34).

In a specific embodiment, the CDRs of the antibodies described herein have been determined according to the Chothia numbering scheme.

A number of definitions of the CDRs are commonly in use: Kabat numbering, Chothia numbering, AbM numbering, or contact numbering. The AbM definition is a compromise between the two used by Oxford Molecular's AbM antibody modelling software. The contact definition is based on an analysis of the available complex crystal structures.

**Table 2: CDR Numbering**

| **Loop** | **Kabat** | **AbM** | **Chothia** | **Contact** |
|---|---|---|---|---|
| L1 | L24--L34 | L24--L34 | L24--L34 | L30--L36 |
| L2 | L50--L56 | L50--L56 | L50--L56 | L46--L55 |
| L3 | L89--L97 | L89--L97 | L89--L97 | L89--L96 |
| H1 | H31--H35B (Kabat Numbering) | H26--H35B | H26--H32..34 | H30--H35B |
| H1 | H31--H35 (Chothia Numbering) | H26--H35 | H26--H32 | H30--H35 |
| H2 | H50--H65 | H50--H58 | H52--H56 | H47--H58 |
| H3 | H95--H102 | H95--H102 | H95--H102 | H93--H101 |

As used herein, the term "heavy chain" when used in reference to an antibody may refer to any distinct type, *e.g.,* alpha (α), delta (δ), epsilon (ε), gamma (γ) and mu (µ), based on the amino acid sequence of the constant domain, which give rise to IgA, IgD, IgE, IgG and IgM classes of antibodies, respectively, including subclasses of IgG, *e.g*., IgG₁, IgG₂, IgG₃ and IgG₄.

As used herein, the term "light chain" when used in reference to an antibody may refer to any distinct type, *e.g.,* kappa (κ) or lambda (λ) based on the amino acid sequence of the constant domains. Light chain amino acid sequences are well known in the art. In specific embodiments, the light chain is a human light chain.

The term "variable region" or "variable domain" refers to a portion of the light and/or heavy chains of an antibody, typically including approximately the amino-terminal 120 to 130 amino acids in the heavy chain and about 100 to 110 amino terminal amino acids in the light chain. The variable region of an antibody typically determines specificity of a particular antibody for its target.

Variability is not evenly distributed throughout the variable domains of antibodies or antigen binding molecules; it is concentrated in sub-domains of each of the heavy and light chain variable regions. These subdomains are called "hypervariable regions" or "complementarity determining regions" (CDRs) as further described herein. The more conserved (*i.e*., non-hypervariable) portions of the variable domains are called the "framework" regions (FRM or FR) and provide a scaffold for the six CDRs in three dimensional space to form an antigen-binding surface. The variable domains of naturally occurring heavy and light chains each comprise four FRM regions (FR1, FR2, FR3, and FR4), largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding site (see Kabat *et al.,* described further herein.

Typically, CDRs form a loop structure that may be classified as a canonical structure. The term "canonical structure" refers to the main chain conformation that is adopted by the antigen binding (CDR) loops. From comparative structural studies, it has been found that five of the six antigen binding loops have only a limited repertoire of available conformations. Each canonical structure may be characterized by the torsion angles of the polypeptide backbone. Correspondent loops between antibodies may, therefore, have very similar three dimensional structures, despite high amino acid sequence variability in most parts of the loops (Chothia and Lesk, J. Mol. Biol., 1987, 196: 901; Chothia et al., Nature, 1989, 342: 877; Martin and Thornton, J. Mol. Biol, 1996, 263: 800). Furthermore, there is a relationship between the adopted loop structure and the amino acid sequences surrounding it. The conformation of a particular canonical class is determined by the length of the loop and the amino acid residues residing at key positions within the loop, as well as within the conserved framework (i.e., outside of the loop). Assignment to a particular canonical class may therefore be made based on the presence of these key amino acid residues.

The term "canonical structure" may also include considerations as to the linear sequence of the antibody, for example, as catalogued by Kabat (Kabat *et al.,* herein). The Kabat numbering scheme (system) is a widely adopted standard for numbering the amino acid residues of an antibody variable domain in a consistent manner and is the preferred scheme applied in the present invention as also mentioned elsewhere herein. Additional structural considerations may also be used to determine the canonical structure of an antibody. For example, those differences not fully reflected by Kabat numbering may be described by the numbering system of Chothia *et al.* and/or revealed by other techniques, for example, crystallography and two- or three-dimensional computational modeling. Accordingly, a given antibody sequence may be placed into a canonical class which allows for, among other things, identifying appropriate chassis sequences (e.g., based on a desire to include a variety of canonical structures in a library). Kabat numbering of antibody amino acid sequences and structural considerations as described by Chothia *et al.* (herein) and their implications for construing canonical aspects of antibody structure, are described in the literature. The subunit structures and three-dimensional configurations of different classes of immunoglobulins are well known in the art. For a review of the antibody structure, see Antibodies: A Laboratory Manual, Cold Spring Harbor Laboratory, eds. Harlow et al., 1988.

The CDR3 of the light chain and, particularly, the CDR3 of the heavy chain may constitute the most important determinants in antigen binding within the light and heavy chain variable regions. In some antibody constructs, the heavy chain CDR3 appears to constitute the major area of contact between the antigen and the antibody. In vitro selection schemes in which CDR3 alone is varied may be used to vary the binding properties of an antibody or determine which residues contribute to the binding of an antigen. Hence, CDR3 is typically the greatest source of molecular diversity within the antibody-binding site. H3, for example, may be as short as two amino acid residues or greater than 26 amino acids.

As used herein, the terms "constant region" and "constant domain" are interchangeable and have a meaning common in the art. The constant region is an antibody portion, *e.g.,* a carboxyl terminal portion of a light and/or heavy chain which is not directly involved in binding of an antibody to antigen but which may exhibit various effector functions, such as interaction with the Fc receptor. The constant region of an immunoglobulin molecule generally has a more conserved amino acid sequence relative to an immunoglobulin variable domain.

An "Fc" region comprises two heavy chain fragments comprising the CH1 and CH2 domains of an antibody. The two heavy chain fragments are held together by two or more disulfide bonds and by hydrophobic interactions of the CH3 domains.

A "Fab fragment" comprises one light chain and the CH1 and variable regions of one heavy chain. The heavy chain of a Fab molecule cannot form a disulfide bond with another heavy chain molecule. A "Fab'" fragment" comprises one light chain and a portion of one heavy chain that contains the VH domain and the CH1 domain and also the region between the CH1 and CH2 domains, such that an interchain disulfide bond may be formed between the two heavy chains of two Fab' fragments to form an F(ab')₂ molecule. An "F(ab')₂ fragment" contains two light chains and two heavy chains containing a portion of the constant region between the CH1 and CH2 domains, such that an interchain disulfide bond is formed between the two heavy chains. An F(ab')₂ fragment thus is composed of two Fab' fragments that are held together by a disulfide bond between the two heavy chains.

The "Fv region" comprises the variable regions from both the heavy and light chains, but lacks the constant regions.

A "bivalent antigen binding molecule" comprises two antigen binding sites. In some instances, the two binding sites have the same antigen specificities. Bivalent antigen binding molecules may be bispecific. A "multispecific antigen binding molecule" is one that targets more than one antigen or epitope. A "bispecific," "dual-specific" or "bifunctional" antigen binding molecule is a hybrid antigen binding molecule or antibody, respectively, having two different antigen binding sites. The two binding sites of a bispecific antigen binding molecule will bind to two different epitopes, which may reside on the same or different protein targets.

"Binding affinity" generally refers to the strength of the sum total of noncovalent interactions between a single binding site of a molecule (*e.g*., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y may generally be represented by the dissociation constant (K_{D}). Affinity may be measured and/or expressed in a number of ways known in the art, including, but not limited to, equilibrium dissociation constant (K_{D}), and equilibrium association constant (K_{A}). The K_{D} is calculated from the quotient of k_{off}/kₒₙ, whereas K_{A} is calculated from the quotient of kₒₙ/k_{off}. kₒₙ refers to the association rate constant of, *e.g.,* an antibody to an antigen, and k_{off} refers to the dissociation of, *e.g.,* an antibody to an antigen. The kₒₙ and k_{off} may be determined by techniques known to one of ordinary skill in the art, such as BIAcore^{®} or KinExA.

The term "neutralizing" refers to an antigen binding molecule, scFv, or antibody, respectively, that binds to a ligand and prevents or reduces the biological effect of that ligand. This may be done, for example, by directly blocking a binding site on the ligand or by binding to the ligand and altering the ligand's ability to bind through indirect means (such as structural or energetic alterations in the ligand). In some embodiments, the term may also denote an antigen binding molecule that prevents the protein to which it is bound from performing a biological function.

The term "compete" when used in the context of antigen binding molecules that compete for the same epitope means competition between antigen binding molecules as determined by an assay in which the antigen binding molecule (e.g., antibody or immunologically functional fragment thereof) being tested prevents or inhibits (e.g., reduces) specific binding of a reference antigen binding molecule to an antigen. Numerous types of competitive binding assays may be used to determine if one antigen binding molecule competes with another, for example: solid phase direct or indirect radioimmunoassay (RIA), solid phase direct or indirect enzyme immunoassay (EIA), sandwich competition assay (Stahli et al., 1983, Methods in Enzymology 9:242-253); solid phase direct biotin-avidin EIA (Kirkland et al., 1986, J. Immunol. 137:3614-3619), solid phase direct labeled assay, solid phase direct labeled sandwich assay (Harlow and Lane, 1988, Antibodies, A Laboratory Manual, Cold Spring Harbor Press); solid phase direct label RIA using 1-125 label (Morel et al., 1988, Molec. Immunol. 25:7-15); solid phase direct biotin-avidin EIA (Cheung, et al., 1990, Virology 176:546-552); and direct labeled RIA (Moldenhauer et al., 1990, Scand. J. Immunol. 32:77-82).

As used herein, the term "epitope" refers to a localized region of an antigen to which an antibody may specifically bind. An epitope may be, for example, contiguous amino acids of a polypeptide (linear or contiguous epitope) or an epitope may, for example, come together from two or more non-contiguous regions of a polypeptide or polypeptides (conformational, non-linear, discontinuous, or non-contiguous epitope). In certain embodiments, the epitope to which an antibody binds may be determined by, *e.g.,* NMR spectroscopy, X-ray diffraction crystallography studies, ELISA assays, hydrogen/deuterium exchange coupled with mass spectrometry (*e*.*g*., liquid chromatography electrospray mass spectrometry), array-based oligo-peptide scanning assays, and/or mutagenesis mapping (*e*.*g*., site-directed mutagenesis mapping). For X-ray crystallography, crystallization may be accomplished using any of the known methods in the art (*e.g*., Giegé R. et al., (1994) Acta Crystallogr D Biol Crystallogr 50(Pt 4): 339-350; McPherson A (1990) Eur J Biochem 189: 1-23; Chayen NE (1997) Structure 5: 1269-1274; McPherson A (1976) J Biol Chem 251: 6300-6303). Antibody:antigen crystals may be studied using well known X-ray diffraction techniques and may be refined using computer software such as X-PLOR (Yale University, 1992, distributed by Molecular Simulations, Inc.; see *e.g.* Meth Enzymol (1985) volumes 114 & 115, eds Wyckoff HW *et al*.,; U.S. 2004/0014194), and BUSTER (Bricogne G (1993) Acta Crystallogr D Biol Crystallogr 49(Pt 1): 37-60; Bricogne G (1997) Meth Enzymol 276A: 361-423, ed Carter CW; Roversi P et al., (2000) Acta Crystallogr D Biol Crystallogr 56(Pt 10): 1316-1323). Mutagenesis mapping studies may be accomplished using any method known to one of skill in the art. *See*, *e.g*., Champe M et al., (1995) J Biol Chem 270: 1388-1394 and Cunningham BC & Wells JA (1989) Science 244: 1081-1085 for a description of mutagenesis techniques, including alanine scanning mutagenesis techniques.

The term "genetically engineered" or "engineered" refers to a method of modifying the genome of a cell, including, but not limited to, deleting a coding or non-coding region or a portion thereof or inserting a coding region or a portion thereof. In some embodiments, the cell that is modified is a lymphocyte, *e*.*g*., a T cell, which may either be obtained from a patient or a donor. The cell may be modified to express an exogenous construct, such as, *e.g.,* a chimeric antigen receptor (CAR) or a T cell receptor (TCR), which is incorporated into the cell's genome.

An "immune response" refers to the action of a cell of the immune system (for example, T lymphocytes, B lymphocytes, natural killer (NK) cells, macrophages, eosinophils, mast cells, dendritic cells and neutrophils) and soluble macromolecules produced by any of these cells or the liver (including Abs, cytokines, and complement) that results in selective targeting, binding to, damage to, destruction of, and/or elimination from a vertebrate's body of invading pathogens, cells or tissues infected with pathogens, cancerous or other abnormal cells, or, in cases of autoimmunity or pathological inflammation, normal human cells or tissues.

The term "immunotherapy" refers to the treatment of a subject afflicted with, or at risk of contracting or suffering a recurrence of, a disease by a method comprising inducing, enhancing, suppressing or otherwise modifying an immune response. Examples of immunotherapy include, but are not limited to, T cell therapies. T cell therapy may include adoptive T cell therapy, tumor-infiltrating lymphocyte (TIL) immunotherapy, autologous cell therapy, engineered autologous cell therapy (eACT), and allogeneic T cell transplantation. However, one of skill in the art would recognize that the conditioning methods disclosed herein would enhance the effectiveness of any transplanted T cell therapy. Examples of T cell therapies are described in U.S. Patent Publication Nos. 2014/0154228 and 2002/0006409, U.S. Patent No. 5,728,388, and International Publication No. WO 2008/081035.

The T cells of the immunotherapy may come from any source known in the art. For example, T cells may be differentiated *in vitro* from a hematopoietic stem cell population, or T cells may be obtained from a subject. T cells may be obtained from, *e.g.,* peripheral blood mononuclear cells (PBMCs), bone marrow, lymph node tissue, cord blood, thymus tissue, tissue from a site of infection, ascites, pleural effusion, spleen tissue, and tumors. In addition, the T cells may be derived from one or more T cell lines available in the art. T cells may also be obtained from a unit of blood collected from a subject using any number of techniques known to the skilled artisan, such as FICOLL^{™} separation and/or apheresis. Additional methods of isolating T cells for a T cell therapy are disclosed in U.S. Patent Publication No. 2013/0287748, which is herein incorporated by references in its entirety.

The term "engineered Autologous Cell Therapy," which may be abbreviated as "eACT^{™}," also known as adoptive cell transfer, is a process by which a patient's own T cells are collected and subsequently genetically altered to recognize and target one or more antigens expressed on the cell surface of one or more specific tumor cells or malignancies. T cells may be engineered to express, for example, chimeric antigen receptors (CAR) or T cell receptor (TCR). CAR positive (+) T cells are engineered to express an extracellular single chain variable fragment (scFv) with specificity for a particular tumor antigen linked to an intracellular signaling part comprising at least one costimulatory domain and at least one activating domain. The costimulatory domain may be derived from (or correspond to), *e.g*., CD28, and the activating domain may be derived from (or correspond to) *e.g*., CD3-zeta. In certain embodiments, the CAR is designed to have two, three, four, or more costimulatory domains.

The term "autologous" refers to any material derived from the same individual to which it is later to be re-introduced. For example, the engineered autologous cell therapy (eACT^{™}) method described herein involves collection of lymphocytes from a patient, which are then engineered to express, *e.g.,* a CAR construct, and then administered back to the same patient.

The term "allogeneic" refers to any material derived from one individual which is then introduced to another individual of the same species, *e*.*g*., allogeneic T cell transplantation.

Standard techniques may be used for recombinant DNA, oligonucleotide synthesis, and tissue culture and transformation (*e*.*g*., electroporation, lipofection). Enzymatic reactions and purification techniques may be performed according to manufacturer's specifications or as commonly accomplished in the art or as described herein. The foregoing techniques and procedures may be generally performed according to conventional methods well known in the art and as described in various general and more specific references that are cited and discussed throughout the present specification. See, *e.g.,* Sambrook et al., Molecular Cloning: A Laboratory Manual (2d ed., Cold Spring Harbor Laboratory Press, Cold Spring Harbor, N.Y. (1989)), which is incorporated herein by reference for any purpose.

Comprise, include, and/or plural forms of each are open ended and include the listed parts and may include additional parts that are not listed. And/or is open ended and includes one or more of the listed parts and combinations of the listed parts.

It will be understood that descriptions herein are exemplary and explanatory only and are not restrictive of the invention as claimed. In this application, the use of the singular includes the plural unless specifically stated otherwise.

All documents, or portions of documents, cited in this application, including but not limited to patents, patent applications, articles, books, and treatises, are hereby expressly incorporated by reference in their entirety for any purpose. As utilized in accordance with the present disclosure, the following terms, unless otherwise indicated, shall be understood to have the following meanings:

In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise.

The citation of a reference herein should not be construed as an acknowledgement that such reference is prior art to the present invention. To the extent that any of the definitions or terms provided in the references incorporated by reference differ from the terms and discussion provided herein, the present terms and definitions control.

One skilled in the art will realize the subject matter may be embodied in other specific forms without departing from the spirit or essential characteristics thereof. The foregoing embodiments are therefore to be considered in all respects illustrative rather than limiting of the subject matter described herein.

The following sequences will further exemplify the invention:
CD28T DNA Extracellular, transmembrane, intracellular 
CTTGATAATGAAAAGTCAAACGGAACAATCATTCACGTGAAGGGC
AAGCACCTCTGTCCGTCACCCTTGTTCCCTGGTCCATCCAAGCCATT
CTGGGTGTTGGTCGTAGTGGGTGGAGTCCTCGCTTGTTACTCTCTGC
TCGTCACCGTGGCTTTTATAATCTTCTGGGTTAGATCCAAAAGAAG
CCGCCTGCTCCATAGCGATTACATGAATATGACTCCACGCCGCCCT
GGCCCCACAAGGAAACACTACCAGCCTTACGCACCACCTAGAGAT
TTCGCTGCCTATCGGAGC (SEQ ID NO. 1)
CD28T Extracellular, transmembrane, intracellular AA 
LDNEKSNGTIIHVKGKHLCPSPLFPGPSKPFWVLVVVGGVLACYSLLV
TVAFIIFWVRSKRSRLLHSDYMNMTPRRPGPTRKHYQPYAPPRDFAAY
RS (SEQ ID NO. 2)
CD28T DNA - Extracellular 
CTTGATAATGAAAAGTCAAACGGAACAATCATTCACGTGAAGGGC
AAGCACCTCTGTCCGTCACCCTTGTTCCCTGGTCCATCCAAGCCA
 (SEQ ID NO. 3)
CD28T AA - Extracellular
   LDNEKSNGTI IHVKGKHLCP SPLFPGPSKP (SEQ ID NO. 4)
CD28 DNA Transmembrane Domain 
TTCTGGGTGTTGGTCGTAGTGGGTGGAGTCCTCGCTTGTTACTCTCT
GCTCGTCACCGTGGCTTTTATAATCTTCTGGGTT (SEQ ID NO. 5)
CD28 AA Transmembrane Domain
   FWVLVVVGGV LACYSLLVTV AFIIFWV (SEQ ID NO. 6)
CD28 DNA Intracellular Domain 
AGATCCAAAAGAAGCCGCCTGCTCCATAGCGATTACATGAATATG
ACTCCACGCCGCCCTGGCCCCACAAGGAAACACTACCAGCCTTACG
CACCACCTAGAGATTTCGCTGCCTATCGGAGC (SEQ ID NO. 7)
CD28 AA Intracellular Domain
   RSKRSRLLHSDYMNMTPRRPGPTRKHYQPYAPPRDFAAYRS (SEQ ID NO. 8)
CD3 zeta DNA 
AGGGTGAAGTTTTCCAGATCTGCAGATGCACCAGCGTATCAGCAGG
GCCAGAACCAACTGTATAACGAGCTCAACCTGGGACGCAGGGAAG
AGTATGACGTTTTGGACAAGCGCAGAGGACGGGACCCTGAGATGG
GTGGCAAACCAAGACGAAAAAACCCCCAGGAGGGTCTCTATAATG
AGCTGCAGAAGGATAAGATGGCTGAAGCCTATTCTGAAATAGGCA
TGAAAGGAGAGCGGAGAAGGGGAAAAGGGCACGACGGTTTGTAC
CAGGGACTCAGCACTGCTACGAAGGATACTTATGACGCTCTCCACA
TGCAAGCCCTGCCACCTAGG (SEQ ID NO. 9)
CD3 zeta AA 
RVKFSRSADAPAYQQGQNQLYNELNLGRREEYDVLDKRRGRDPEMG
GKPRRKNPQEGLYNELQKDKMAEAYSEIGMKGERRRGKGHDGLYQG
LSTATKDTYDALHMQALPPR (SEQ ID NO. 10)
CD28 DNA 
ATTGAGGTGATGTATCCACCGCCTTACCTGGATAACGAAAAGAGTA
ACGGTACCATCATTCACGTGAAAGGTAAACACCTGTGTCCTTCTCC
CCTCTTCCCCGGGCCATCAAAGCCC (SEQ ID NO. 11)
CD28 AA
   IEVMYPPPYLDNEKSNGTIIHVKGKHLCPSPLFPGPSKP (SEQ ID NO. 12)
CD8 DNA extracellular & transmembrane domain 
GCTGCAGCATTGAGCAACTCAATAATGTATTTAGTCACTTTGTAC
CAGTGTTCTTGCCGGCTAAGCCTACTACCACACCCGCTCCACGGCC
 
ACCTACCCCAGCTCCTACCATCGCTTCACAGCCTCTGTCCCTGCGCC
CAGAGGCTTGCCGACCGGCCGCAGGGGGCGCTGTTCATACCAGAG
GACTGGATTTCGCCTGCGATATCTATATCTGGGCACCCCTGGCCGG
AACCTGCGGCGTACTCCTGCTGTCCCTGGTCATCACGCTCTATTGTA
ATCACAGGAAC (SEQ ID NO. 13)
CD8 AA extracellular & transmembrane Domain 
AAALSNSIMYFSHFVPVFLPAKPTTTPAPRPPTPAPTIASQPLSLRPEAC
RPAAGGAVHTRGLDFACDIYIWAPLAGTCGVLLLSLVITLYCNHRN
 (SEQ ID NO. 14)
Clone 24C1 HC DNA 
CAGGTGCAGCTGCAGGAATCCGGACCGGGGCTGGTGAAGCCCAGC
GAGACTCTGAGTCTCACGTGTACAGTTTCTGGAGGTAGCATTAGCT
CCTACTATTGGTCATGGATAAGGCAGCCCCCGGGAAGGGATTGG
AATGGATCGGCTATATTTACTACAGTGGGAGCACCAATTACAACCC
CTCACTGAAGTCTAGAGTTACAATCAGCGTTGACACCTCAAAGAAT
CAGTTCAGTTTGAAATTGTCTAGCGTCACAGCAGCTGATACAGCCG
TCTATTATTGTGTTTCTCTGGTCTATTGCGGTGGGGATTGTTACAGT
GGCTTTGACTATTGGGGGCAGGGTACTCTGGTTACAGTTTCTTCC
 (SEQ ID NO. 15)
Clone 24C1 HC AA (CDRs Underlined) 
QVQLQESGPGLVKPSETLSLTCTVSGGSISSYYWSWIRQPPGKGLEWIG
Y
IYYSGSTNYNPSLKSRVTISVDTSKNQFSLKLSSVTAADTAVYYCVSLV
YCGGDCYSGFDYWGQGTLVTVSS (SEQ ID NO. 16)
Clone 24C1 HC AA CDR1: GGSISSY (SEQ ID NO. 17)
Clone 24C1 HC AA CDR2: YYSGS (SEQ ID NO. 18)
Clone 24C1 HC AA CDR3: LVYCGGDCYS GFDY (SEQ ID NO. 19)
Clone 24C1 LC DNA 
GACATCCAGTTGACACAGAGCCCGAGTTCCTTGTCCGCCTCCGTCG
GGGATAGAGTGTCATTTACCTGTCAGGCCTCTCAGGATATTAATAA
CTTTCTGAATTGGTATCAGCAAAAGCCCGGAAAGGCACCCAAGCTG
 
TTGATTTACGACGCCAGTAACCTGGAGACAGGCGTGCCCTCCCGGT
TTAGTGGTAGCGGAAGCGGTACGGATTTACCTTTACTATCAGCTC
TCTCCAACCCGAAGACATTGCAACCTACTATTGTCAACAATATGGA
AACCTGCCTTTTACATTTGGCGGCGGCACCAAGGTGGAGATTAAGC
GG (SEQ ID NO. 20)
Clone 24C1 LC AA (CDRs Underlined) 
DIQLTQSPSSLSASVGDRVSFTCQASQDINNFLNWYQQKPGKAPKLLIY
DASNLETGVPSRFSGSGSGTDFTFTISSLQPEDIATYYCQQYGNLPFTFG
GGTKVEIKR (SEQ ID NO. 21)
Clone 24C1 LC CDR1 AA: QASQDINNFLN (SEQ ID NO. 22)
Clone 24C1 LC CDR2 AA: DASNLET (SEQ ID NO. 23)
Clone 24C1 LC CDR3 AA: QQYGNLPFT (SEQ ID NO. 24)
Clone 24C1 CD28T CD3 zeta CAR DNA Heavy & Light Chains 
ATGGCACTCCCCGTAACTGCTCTGCTGCTGCCGTTGGCATTGCTCCT
GCACGCCGCACGCCCGCAGGTCCAACTGCAAGAAAGCGGACCCGG
ACTGGTGAAGCCTTCTGAGACACTTAGTCTGACGTGCACGGTCAGT
GGCGGCTCCATCTCCTCCTATTATTGGTCATGGATACGACAACCCC
CAGGTAAGGGCCTGGAATGGATTGGCTATATCTACTATTCAGGAAG
CACGAACTACAATCCCAGCCTGAAGTCCCGAGTGACAATTTCAGTA
GATACCAGTAAAAACCAGTTCAGTCTTAAACTGTCAAGCGTGACAG
CTGCCGACACCGCTGTGTATTACTGCGTCTCACTGGTGTATTGTGG
AGGGGATTGTTATAGCGGGTTCGATTATTGGGGACAGGGAACCCTG
GTGACTGTATCTTCCGGCGGCGGCGGCTCAGGGGGTGGCGGTAGTG
GCGGTGGGGGTTCCGATATTCAACTGACACAATCCCCCAGCTCACT
CAGCGCCAGCGTGGGGGACAGGGTTAGCTTTACCTGTCAAGCCTCT
CAGGATATAAATAACTTTCTGAACTGGTATCAACAGAAGCCTGGGA
AGGCGCCCAAACTCCTGATCTATGATGCGTCCAACCTGGAAACTGG
CGTGCCTTCACGCTTTAGCGGCTCTGGCAGTGGTACAGACTTCACT
TTTACCATCTCTTCACTTCAGCCGGAGGACATCGCCACATATTACTG
TCAACAGTACGGAAACTTGCCCTTTACTTTTGGAGGCGGCACCAAA
GTTGAAATCAAAAGGGCCGCTGCCCTGGATAACGAAAAGAGCAAT
GGGACTATAATACATGTTAAAGGAAAACACCTGTGTCCATCTCCCC
 
TGTTCCCTGGACCGTCAAAGCCATTTTGGGTGCTCGTGGTTGTCGGT
GGCGTTCTCGCCTGTTATAGCTTGCTGGTGACAGTAGCCTTCATTAT
CTTTTGGGTGAGATCCAAAAGAAGCCGCCTGCTCCATAGCGATTAC
ATGAATATGACTCCACGCCGCCCTGGCCCCACAAGGAAACACTACC
AGCCTTACGCACCACCTAGAGATTTCGCTGCCTATCGGAGCAGGGT
GAAGTTTTCCAGATCTGCAGATGCACCAGCGTATCAGCAGGGCCAG
AACCAACTGTATAACGAGCTCAACCTGGGACGCAGGGAAGAGTAT
GACGTTTTGGACAAGCGCAGAGGACGGGACCCTGAGATGGGTGGC
AAACCAAGACGAAAAAACCCCCAGGAGGGTCTCTATAATGAGCTG
CAGAAGGATAAGATGGCTGAAGCCTATTCTGAAATAGGCATGAAA
GGAGAGCGGAGAAGGGGAAAAGGGCACGACGGTTTGTACCAGGG
ACTCAGCACTGCTACGAAGGATACTTATGACGCTCTCCACATGCAA
GCCCTGCCACCTAGGTAA (SEQ ID NO. 25)
Clone 24C1 CD28T CD3 zeta CAR AA Heavy & Light Chains
   (Signal Peptide in **bold**) 
MALPVTALLLPLALLLHAARPQVQLQESGPGLVKPSETLSLTCTVSG
GSISSYYWSWIRQPPGKGLEWIGYIYYSGSTNYNPSLKSRVTISVDTSK
NQFSLKLSSVTAADTAVYYCVSLVYCGGDCYSGFDYWGQGTLVTVS
SGGGGSGGGGSGGGGSDIQLTQSPSSLSASVGDRVSFTCQASQDINNF
LNWYQQKPGKAPKLLIYDASNLETGVPSRFSGSGSGTDFTFTISSLQPE
DIATYYCQQYGNLPFTFGGGTKVEIKRAAALDNEKSNGTIIHVKGKHL
CPSPLFPGPSKPFWVLVVVGGVLACYSLLVTVAFIIFWVRSKRSRLLHS
DYMNMTPRRPGPTRKHYQPYAPPRDFAAYRSRVKFSRSADAPAYQQ
GQNQLYNELNLGRREEYDVLDKRRGRDPEMGGKPRRKNPQEGLYNE
LQKDKMAEAYSEIGMKGERRRGKGHDGLYQGLSTATKDTYDA
LHMQALPPR (SEQ ID NO. 26)
Clone 24C1 CD28T CD3 zeta CAR DNA Heavy & Light Chains 
CAGGTCCAACTGCAAGAAAGCGGACCCGGACTGGTGAAGCCTTCT
GAGACACTTAGTCTGACGTGCACGGTCAGTGGCGGCTCCATCTCCT
CCTATTATTGGTCATGGATACGACAACCCCCAGGTAAGGGCCTGGA
ATGGATTGGCTATATCTACTATTCAGGAAGCACGAACTACAATCCC
AGCCTGAAGTCCCGAGTGACAATTTCAGTAGATACCAGTAAAAAC
CAGTTCAGTCTTAAACTGTCAAGCGTGACAGCTGCCGACACCGCTG
 
TGTATTACTGCGTCTCACTGGTGTATTGTGGAGGGGATTGTTATAGC
GGGTTCGATTATTGGGGACAGGGAACCCTGGTGACTGTATCTTCCG
GCGGCGGCGGCTCAGGGGGTGGCGGTAGTGGCGGTGGGGGTTCCG
ATATTCAACTGACACAATCCCCCAGCTCACTCAGCGCCAGCGTGGG
GGACAGGGTTAGCTTTACCTGTCAAGCCTCTCAGGATATAAATAAC
TTTCTGAACTGGTATCAACAGAAGCCTGGGAAGGCGCCCAAACTCC
TGATCTATGATGCGTCCAACCTGGAAACTGGCGTGCCTTCACGCTT
TAGCGGCTCTGGCAGTGGTACAGACTTCACTTTTACCATCTCTTCAC
TTCAGCCGGAGGACATCGCCACATATTACTGTCAACAGTACGGAAA
CTTGCCCTTTACTTTTGGAGGCGGCACCAAAGTTGAAATCAAAAGG
GCCGCTGCCCTGGATAACGAAAAGAGCAATGGGACTATAATACAT
GTTAAAGGAAAACACCTGTGTCCATCTCCCCTGTTCCCTGGACCGT
CAAAGCCATTTTGGGTGCTCGTGGTTGTCGGTGGCGTTCTCGCCTGT
TATAGCTTGCTGGTGACAGTAGCCTTCATTATCTTTTGGGTGAGATC
CAAAAGAAGCCGCCTGCTCCATAGCGATTACATGAATATGACTCCA
CGCCGCCCTGGCCCCACAAGGAAACACTACCAGCCTTACGCACCAC
CTAGAGATTTCGCTGCCTATCGGAGCAGGGTGAAGTTTTCCAGATC
TGCAGATGCACCAGCGTATCAGCAGGGCCAGAACCAACTGTATAA
CGAGCTCAACCTGGGACGCAGGGAAGAGTATGACGTTTTGGACAA
GCGCAGAGGACGGGACCCTGAGATGGGTGGCAAACCAAGACGAA
AAAACCCCCAGGAGGGTCTCTATAATGAGCTGCAGAAGGATAAGA
TGGCTGAAGCCTATTCTGAAATAGGCATGAAAGGAGAGCGGAGAA
GGGGAAAGGGCACGACGGTTTGTACCAGGGACTCAGCACTGCTA
CGAAGGATACTTATGACGCTCTCCACATGCAAGCCCTGCCACCTAG
G (SEQ ID NO. 27)
Clone 24C1 CD28T CD3 zeta CAR AA Heavy & Light Chains 
QVQLQESGPGLVKPSETLSLTCTVSGGSISSYYWSWIRQPPGKGLEWIG
YIYYSGSTNYNPSLKSRVTISVDTSKNQFSLKLSSVTAADTAVYYCVSL
VYCGGDCYSGFDYWGQGTLVTVSSGGGGSGGGGSGGGGSDIQLTQS
PSSLSASVGDRVSFTCQASQDINNFLNWYQQKPGKAPKLLIYDASNLE
TGVPSRFSGSGSGTDFTFTISSLQPEDIATYYCQQYGNLPFTFGGGTKV
EIKRAAALDNEKSNGTIIHVKGKHLCPSPLFPGPSKPFWVLVVVGGVL
ACYSLLVTVAFIIFWVRSKRSRLLHSDYMNMTPRRPGPTRKHYQPYAP
 
PRDFAAYRSRVKFSRSADAPAYQQGQNQLYNELNLGRREEYDVLDK
RRGRDPEMGGKPRRKNPQEGLYNELQKDKMAEAYSEIGMKGERRRG
KGHDGLYQGLSTATKDTYDALHMQALPPR (SEQ ID NO. 28)
Clone 24C1 CD28 CD3 zeta CAR DNA Heavy & Light Chains 
ATGGCACTCCCCGTAACTGCTCTGCTGCTGCCGTTGGCATTGCTCCT
GCACGCCGCACGCCCGCAGGTGCAGCTGCAGGAATCCGGACCGGG
GCTGGTGAAGCCCAGCGAGACTCTGAGTCTCACGTGTACAGTTTCT
GGAGGTAGCATTAGCTCCTACTATTGGTCATGGATAAGGCAGCCCC
CCGGGAAGGGATTGGAATGGATCGGCTATATTTACTACAGTGGGA
GCACCAATTACAACCCCTCACTGAAGTCTAGAGTTACAATCAGCGT
TGACACCTCAAAGAATCAGTTCAGTTTGAAATTGTCTAGCGTCACA
GCAGCTGATACAGCCGTCTATTATTGTGTTTCTCTGGTCTATTGCGG
TGGGGATTGTTACAGTGGCTTTGACTATTGGGGGCAGGGTACTCTG
GTTACAGTTTCTTCCGGGGGGGGAGGCTCTGGGGGCGGAGGCTCA
GGTGGTGGAGGCAGCGACATCCAGTTGACACAGAGCCCGAGTTCC
TTGTCCGCCTCCGTCGGGGATAGAGTGTCATTTACCTGTCAGGCCT
CTCAGGATATTAATAACTTTCTGAATTGGTATCAGCAAAAGCCCGG
AAAGGCACCCAAGCTGTTGATTTACGACGCCAGTAACCTGGAGAC
AGGCGTGCCCTCCCGGTTTAGTGGTAGCGGAAGCGGTACGGATTTT
ACCTTTACTATCAGCTCTCTCCAACCCGAAGACATTGCAACCTACT
ATTGTCAACAATATGGAAACCTGCCTTTTACATTTGGCGGCGGCAC
CAAGGTGGAGATTAAGCGGGCGGCAGCTATTGAGGTGATGTATCC
ACCGCCTTACCTGGATAACGAAAAGAGTAACGGTACCATCATTCAC
GTGAAAGGTAAACACCTGTGTCCTTCTCCCCTCTTCCCCGGGCCAT
CAAAGCCCTTCTGGGTTCTTGTGGTCGTGGGAGGCGTGCTTGCTTG
TTATTCTCTGCTCGTTACCGTGGCGTTTATCATTTTTTGGGTTAGATC
CAAAAGAAGCCGCCTGCTCCATAGCGATTACATGAATATGACTCCA
CGCCGCCCTGGCCCCACAAGGAAACACTACCAGCCTTACGCACCAC
CTAGAGATTTCGCTGCCTATCGGAGCAGGGTGAAGTTTTCCAGATC
TGCAGATGCACCAGCGTATCAGCAGGGCCAGAACCAACTGTATAA
CGAGCTCAACCTGGGACGCAGGGAAGAGTATGACGTTTTGGACAA
GCGCAGAGGACGGGACCCTGAGATGGGTGGCAAACCAAGACGAA
AAAACCCCCAGGAGGGTCTCTATAATGAGCTGCAGAAGGATAAGA
 
TGGCTGAAGCCTATTCTGAAATAGGCATGAAAGGAGAGCGGAGAA
GGGGAAAAGGGCACGACGGTTTGTACCAGGGACTCAGCACTGCTA
CGAAGGATACTTATGACGCTCTCCACATGCAAGCCCTGCCACCTAG
GTAA (SEQ ID NO. 29)
Clone 24C1 CD28 CD3 zeta CAR AA Heavy & Light Chains
   (Signal Peptide in **Bold**) 
MALPVTALLLPLALLLHAARPQVQLQESGPGLVKPSETLSLTCTVSG
GSISSYYWSWIRQPPGKGLEWIGYIYYSGSTNYNPSLKSRVTISVDTSK
NQFSLKLSSVTAADTAVYYCVSLVYCGGDCYSGFDYWGQGTLVTVS
SGGGGSGGGGSGGGGSDIQLTQSPSSLSASVGDRVSFTCQASQDINNF
LNWYQQKPGKAPKLLIYDASNLETGVPSRFSGSGSGTDFTFTISSLQPE
DIATYYCQQYGNLPFTFGGGTKVEIKRAAAIEVMYPPPYLDNEKSNGT
IIHVKGKHLCPSPLFPGPSKPFWVLVVVGGVLACYSLLVTVAFIIFWVR
SKRSRLLHSDYMNMTPRRPGPTRKHYQPYAPPRDFAAYRSRVKFSRS
ADAPAYQQGQNQLYNELNLGRREEYDVLDKRRGRDPEMGGKPRRK
NPQEGLYNELQKDKMAEAYSEIGMKGERRRGKGHDGLYQGLSTATK
DTYDALHMQALPPR (SEQ ID NO. 30)
Clone 24C1 CD28 CD3 zeta CAR DNA Heavy & Light Chains 
CAGGTGCAGCTGCAGGAATCCGGACCGGGGCTGGTGAAGCCCAGC
GAGACTCTGAGTCTCACGTGTACAGTTTCTGGAGGTAGCATTAGCT
CCTACTATTGGTCATGGATAAGGCAGCCCCCGGGAAGGGATTGG
AATGGATCGGCTATATTTACTACAGTGGGAGCACCAATTACAACCC
CTCACTGAAGTCTAGAGTTACAATCAGCGTTGACACCTCAAAGAAT
CAGTTCAGTTTGAAATTGTCTAGCGTCACAGCAGCTGATACAGCCG
TCTATTATTGTGTTTCTCTGGTCTATTGCGGTGGGGATTGTTACAGT
GGCTTTGACTATTGGGGGCAGGGTACTCTGGTTACAGTTTCTTCCG
GGGGGGGAGGCTCTGGGGGCGGAGGCTCAGGTGGTGGAGGCAGCG
ACATCCAGTTGACACAGAGCCCGAGTTCCTTGTCCGCCTCCGTCGG
GGATAGAGTGTCATTTACCTGTCAGGCCTCTCAGGATATTAATAAC
TTTCTGAATTGGTATCAGCAAAAGCCCGGAAAGGCACCCAAGCTGT
TGATTTACGACGCCAGTAACCTGGAGACAGGCGTGCCCTCCCGGTT
TAGTGGTAGCGGAAGCGGTACGGATTTTACCTTTACTATCAGCTCT
CTCCAACCCGAAGACATTGCAACCTACTATTGTCAACAATATGGAA
 
ACCTGCCTTTTACATTTGGCGGCGGCACCAAGGTGGAGATTAAGCG
GGCGGCAGCTATTGAGGTGATGTATCCACCGCCTTACCTGGATAAC
GAAAAGAGTAACGGTACCATCATTCACGTGAAAGGTAAACACCTG
TGTCCTTCTCCCCTCTTCCCCGGGCCATCAAAGCCCTTCTGGGTTCT
TGTGGTCGTGGGAGGCGTGCTTGCTTGTTATTCTCTGCTCGTTACCG
TGGCGTTTATCATTTTTTGGGTTAGATCCAAAAGAAGCCGCCTGCT
CCATAGCGATTACATGAATATGACTCCACGCCGCCCTGGCCCCACA
AGGAAACACTACCAGCCTTACGCACCACCTAGAGATTTCGCTGCCT
ATCGGAGCAGGGTGAAGTTTTCCAGATCTGCAGATGCACCAGCGTA
TCAGCAGGGCCAGAACCAACTGTATAACGAGCTCAACCTGGGACG
CAGGGAAGAGTATGACGTTTTGGACAAGCGCAGAGGACGGGACCC
TGAGATGGGTGGCAAACCAAGACGAAAAAACCCCCAGGAGGGTCT
CTATAATGAGCTGCAGAAGGATAAGATGGCTGAAGCCTATTCTGA
AATAGGCATGAAAGGAGAGCGGAGAAGGGGAAAGGGCACGACG
GTTTGTACCAGGGACTCAGCACTGCTACGAAGGATACTTATGACGC
TCTCCACATGCAAGCCCTGCCACCTAGG (SEQ ID NO. 31)
Clone 24C1 CD28 CD3 zeta CAR AA Heavy & Light Chains 
QVQLQESGPGLVKPSETLSLTCTVSGGSISSYYWSWIRQPPGKGLEWIG
YIYYSGSTNYNPSLKSRVTISVDTSKNQFSLKLSSVTAADTAVYYCVSL
VYCGGDCYSGFDYWGQGTLVTVSSGGGGSGGGGSGGGGSDIQLTQS
PSSLSASVGDRVSFTCQASQDINNFLNWYQQKPGKAPKLLIYDASNLE
TGVPSRFSGSGSGTDFTFTISSLQPEDIATYYCQQYGNLPFTFGGGTKV
EIKRAAAIEVMYPPPYLDNEKSNGTIIHVKGKHLCPSPLFPGPSKPFWV
LVVVGGVLACYSLLVTVAFIIFWVRSKRSRLLHSDYMNMTPRRPGPTR
KHYQPYAPPRDFAAYRSRVKFSRSADAPAYQQGQNQLYNELNLGRRE
EYDVLDKRRGRDPEMGGKPRRKNPQEGLYNELQKDKMAEAYSEIGM
KGERRRGKGHDGLYQGLST ATKDTYDALHMQALPPR (SEQ ID NO.
32)
Clone 24C1 CD8 CD3 zeta CAR DNA Heavy & Light Chains 
ATGGCACTCCCCGTAACTGCTCTGCTGCTGCCGTTGGCATTGCTCCT
GCACGCCGCACGCCCGCAGGTGCAATTGCAAGAGTCCGGCCCCGG
ACTCGTTAAACCCAGTGAGACGCTTAGCCTGACCTGTACCGTCTCA
GGGGGCAGCATCTCCTCTTATTACTGGAGCTGGATCAGGCAGCCTC
 
CAGGAAAAGGCCTTGAATGGATTGGGTACATCTACTACTCTGGCTC
AACAAATTATAATCCATCCCTGAAGTCCCGCGTGACTATCTCTGTG
GACACCAGCAAGAATCAGTTTTCACTGAAGTTGTCTAGTGTTACCG
CGGCCGACACCGCCGTATACTACTGTGTGTCTCTTGTGTACTGTGG
CGGCGACTGCTATTCCGGGTTCGACTACTGGGGCCAAGGGACTCTG
GTAACCGTGTCCTCAGGCGGCGGCGGGTCAGGAGGAGGCGGCAGT
GGAGGTGGCGGCTCCGACATCCAGCTGACACAATCACCATCTTCCC
TTTCAGCTTCAGTCGGGGACAGAGTGTCCTTCACATGCCAGGCCAG
CCAGGATATCAATAACTTCCTGAACTGGTACCAACAGAAACCCGG
AAAGGCTCCAAAGCTCCTGATCTATGATGCTTCCAACCTGGAGACC
GGCGTGCCCTCCAGGTTCAGTGGTTCAGGATCAGGCACTGACTTTA
CGTTCACCATATCCAGTCTTCAGCCCGAAGACATTGCAACCTATTA
CTGCCAACAATACGGGAACCTTCCCTTTACATTCGGAGGCGGCACC
AAGGTGGAAATCAAAAGGGCTGCAGCATTGAGCAACTCAATAATG
TATTTTAGTCACTTTGTACCAGTGTTCTTGCCGGCTAAGCCTACTAC
CACACCCGCTCCACGGCCACCTACCCCAGCTCCTACCATCGCTTCA
CAGCCTCTGTCCCTGCGCCCAGAGGCTTGCCGACCGGCCGCAGGGG
GCGCTGTTCATACCAGAGGACTGGATTTCGCCTGCGATATCTATAT
CTGGGCACCCCTGGCCGGAACCTGCGGCGTACTCCTGCTGTCCCTG
GTCATCACGCTCTATTGTAATCACAGGAACAGATCCAAAAGAAGCC
GCCTGCTCCATAGCGATTACATGAATATGACTCCACGCCGCCCTGG
CCCCACAAGGAAACACTACCAGCCTTACGCACCACCTAGAGATTTC
GCTGCCTATCGGAGCAGGGTGAAGTTTTCCAGATCTGCAGATGCAC
CAGCGTATCAGCAGGGCCAGAACCAACTGTATAACGAGCTCAACC
TGGGACGCAGGGAAGAGTATGACGTTTTGGACAAGCGCAGAGGAC
GGGACCCTGAGATGGGTGGCAAACCAAGACGAAAAAACCCCCAGG
AGGGTCTCTATAATGAGCTGCAGAAGGATAAGATGGCTGAAGCCT
ATTCTGAAATAGGCATGAAAGGAGAGCGGAGAAGGGGAAAAGGG
CACGACGGTTTGTACCAGGGACTCAGCACTGCTACGAAGGATACTT
ATGACGCTCTCCACATGCAAGCCCTGCCACCTAGGTAA (SEQ ID
NO. 33)
Clone 24C1 CD8 CD3 zeta CAR AA Heavy & Light Chains
   (Signal peptide in **bold**) 
MALPVTALLLPLALLLHAARPQVQLQESGPGLVKPSETLSLTCTVSG
GSISSYYWSWIRQPPGKGLEWIGYIYYSGSTNYNPSLKSRVTISVDTSK
NQFSLKLSSVTAADTAVYYCVSLVYCGGDCYSGFDYWGQGTLVTVS
SGGGGSGGGGSGGGGSDIQLTQSPSSLSASVGDRVSFTCQASQDINNF
LNWYQQKPGKAPKLLIYDASNLETGVPSRFSGSGSGTDFTFTISSLQPE
DIATYYCQQYGNLPFTFGGGTKVEIKRAAALSNSIMYFSHFVPVFLPA
KPTTTPAPRPPTPAPTIASQPLSLRPEACRPAAGGAVHTRGLDFACDIYI
WAPLAGTCGVLLLSLVITLYCNHRNRSKRSRLLHSDYMNMTPRRPGP
TRKHYQPYAPPRDFAAYRSRVKFSRSADAPAYQQGQNQLYNELNLGR
REEYDVLDKRRGRDPEMGGKPRRKNPQEGLYNELQKDKMAEAYSEI
GMKGERRRGKGHDGLYQGLSTATKDTYDALHMQALPPR (SEQ ID
NO. 34)
Clone 24C1 CD8 CD3 zeta CAR DNA Heavy & Light Chains 
CAGGTGCAATTGCAAGAGTCCGGCCCCGGACTCGTTAAACCCAGTG
AGACGCTTAGCCTGACCTGTACCGTCTCAGGGGGCAGCATCTCCTC
TTATTACTGGAGCTGGATCAGGCAGCCTCCAGGAAAGGCCTTGAA
TGGATTGGGTACATCTACTACTCTGGCTCAACAAATTATAATCCAT
CCCTGAAGTCCCGCGTGACTATCTCTGTGGACACCAGCAAGAATCA
GTTTTCACTGAAGTTGTCTAGTGTTACCGCGGCCGACACCGCCGTA
TACTACTGTGTGTCTCTTGTGTACTGTGGCGGCGACTGCTATTCCGG
GTTCGACTACTGGGGCCAAGGGACTCTGGTAACCGTGTCCTCAGGC
GGCGGCGGGTCAGGAGGAGGCGGCAGTGGAGGTGGCGGCTCCGAC
ATCCAGCTGACACAATCACCATCTTCCCTTTCAGCTTCAGTCGGGG
ACAGAGTGTCCTTCACATGCCAGGCCAGCCAGGATATCAATAACTT
CCTGAACTGGTACCAACAGAAACCCGGAAAGGCTCCAAAGCTCCT
GATCTATGATGCTTCCAACCTGGAGACCGGCGTGCCCTCCAGGTTC
AGTGGTTCAGGATCAGGCACTGACTTTACGTTCACCATATCCAGTC
TTCAGCCCGAAGACATTGCAACCTATTACTGCCAACAATACGGGAA
CCTTCCCTTTACATTCGGAGGCGGCACCAAGGTGGAAATCAAAAGG
GCTGCAGCATTGAGCAACTCAATAATGTATTTTAGTCACTTTGTAC
CAGTGTTCTTGCCGGCTAAGCCTACTACCACACCCGCTCCACGGCC
ACCTACCCCAGCTCCTACCATCGCTTCACAGCCTCTGTCCCTGCGCC
CAGAGGCTTGCCGACCGGCCGCAGGGGGCGCTGTTCATACCAGAG
 
GACTGGATTTCGCCTGCGATATCTATATCTGGGCACCCCTGGCCGG
AACCTGCGGCGTACTCCTGCTGTCCCTGGTCATCACGCTCTATTGTA
ATCACAGGAACAGATCCAAAAGAAGCCGCCTGCTCCATAGCGATT
ACATGAATATGACTCCACGCCGCCCTGGCCCCACAAGGAAACACT
ACCAGCCTTACGCACCACCTAGAGATTTCGCTGCCTATCGGAGCAG
GGTGAAGTTTTCCAGATCTGCAGATGCACCAGCGTATCAGCAGGGC
CAGAACCAACTGTATAACGAGCTCAACCTGGGACGCAGGGAAGAG
TATGACGTTTTGGACAAGCGCAGAGGACGGGACCCTGAGATGGGT
GGCAAACCAAGACGAAAAAACCCCCAGGAGGGTCTCTATAATGAG
CTGCAGAAGGATAAGATGGCTGAAGCCTATTCTGAAATAGGCATG
AAAGGAGAGCGGAGAAGGGGAAAAGGGCACGACGGTTTGTACCA
GGGACTCAGCACTGCTACGAAGGATACTTATGACGCTCTCCACATG
CAAGCCCTGCCACCTAGG (SEQ ID NO. 35)
Clone 24C1 CD8 CD3 zeta CAR AA Heavy & Light Chains 
QVQLQESGPGLVKPSETLSLTCTVSGGSISSYYWSWIRQPPGKGLEWIG
YIYYSGSTNYNPSLKSRVTISVDTSKNQFSLKLSSVTAADTAVYYCVSL
VYCGGDCYSGFDYWGQGTLVTVSSGGGGSGGGGSGGGGSDIQLTQS
PSSLSASVGDRVSFTCQASQDINNFLNWYQQKPGKAPKLLIYDASNLE
TGVPSRFSGSGSGTDFTFTISSLQPEDIATYYCQQYGNLPFTFGGGTKV
EIKRAAALSNSIMYFSHFVPVFLPAKPTTTPAPRPPTPAPTIASQPLSLRP
EACRPAAGGAVHTRGLDFACDIYIWAPLAGTCGVLLLSLVITLYCNHR
NRSKRSRLLHSDYMNMTPRRPGPTRKHYQPYAPPRDFAAYRSRVKFS
RSADAPAYQQGQNQLYNELNLGRREEYDVLDKRRGRDPEMGGKPRR
KNPQEGLYNELQKDKMAEAYSEIGMKGERRRGKGHDGLYQGLSTAT
K DTYDALHMQALPPR (SEQ ID NO. 36)
Clone 24C1 CD28T CD3 zeta CAR DNA Heavy & Light Chains 
ATGGCACTCCCCGTAACTGCTCTGCTGCTGCCGTTGGCATTGCTCCT
GCACGCCGCACGCCCGGATATCCAGCTCACGCAATCCCCCTCAAGC
TTGAGTGCCTCCGTGGGCGACCGGGTGTCCTTCACATGTCAGGCAA
GCCAAGACATAAATAATTTCCTGAATTGGTACCAACAAAAACCCG
GCAAGGCTCCCAAACTCCTGATTTATGATGCCTCCAATCTGGAGAC
CGGGGTCCCTTCTAGATTCAGCGGAAGTGGCAGCGGCACAGACTTT
ACATTTACTATCTCTTCTCTGCAACCAGAGGACATCGCCACATACT
 
ATTGCCAGCAATACGGCAATCTGCCCTTCACCTTCGGAGGCGGAAC
CAAGGTAGAAATTAAAGGGGCGGTGGAGGCTCCGGAGGGGGG
GCTCTGGCGGAGGGGGCTCCCAAGTACAATTGCAGGAGTCAGGGC
CTGGACTCGTGAAGCCTTCAGAAACTTTGTCACTGACATGTACAGT
GTCCGGCGGAAGCATTTCCAGTTACTATTGGTCCTGGATTAGACAG
CCACCCGGCAAAGGACTGGAATGGATTGGATATATCTACTACTCTG
GATCTACAAACTATAATCCCAGCCTCAAATCCAGGGTCACTATTAG
TGTGGATACATCAAAGAATCAGTTCTCCTTGAAGCTGAGCTCAGTC
ACTGCTGCCGACACCGCAGTGTACTATTGTGAGCCTGGTCTACT
GCGGCGGAGATTGCTACAGCGGTTTCGATTACTGGGGCCAGGGCA
CCCTGGTTACCGTTAGTTCCGCGGCTGCTCTTGATAACGAGAAGTC
CAACGGTACGATTATCCACGTTAAGGGTAAGCACCTTTGCCCTAGC
CCGCTGTTCCCAGGCCCCAGTAAGCCCTTTTGGGTCCTCGTTGTGGT
AGGTGGGGTACTCGCCTGCTACTCCCTGCTCGTCACTGTCGCATTC
ATCATCTTCTGGGTCAGATCCAAAAGAAGCCGCCTGCTCCATAGCG
ATTACATGAATATGACTCCACGCCGCCCTGGCCCCACAAGGAAACA
CTACCAGCCTTACGCACCACCTAGAGATTTCGCTGCCTATCGGAGC
AGGGTGAAGTTTTCCAGATCTGCAGATGCACCAGCGTATCAGCAGG
GCCAGAACCAACTGTATAACGAGCTCAACCTGGGACGCAGGGAAG
AGTATGACGTTTTGGACAAGCGCAGAGGACGGGACCCTGAGATGG
GTGGCAAACCAAGACGAAAAAACCCCCAGGAGGGTCTCTATAATG
AGCTGCAGAAGGATAAGATGGCTGAAGCCTATTCTGAAATAGGCA
TGAAAGGAGAGCGGAGAAGGGGAAAAGGGCACGACGGTTTGTAC
CAGGGACTCAGCACTGCTACGAAGGATACTTATGACGCTCTCCACA
TGCAAGCCCTGCCACCTAGGTAA (SEQ ID NO. 37)
Clone 24C1 CD28T CD3 zeta CAR AA Heavy & Light Chains
   (Signal Peptide in **Bold**) 
MALPVTALLLPLALLLHAARPDIQLTQSPSSLSASVGDRVSFTCQAS
QDINNFLNWYQQKPGKAPKLLIYDASNLETGVPSRFSGSGSGTDFTFTI
SSLQPEDIATYYCQQYGNLPFTFGGGTKVEIKRGGGGSGGGGSGGGGS
QVQLQESGPGLVKPSETLSLTCTVSGGSISSYYWSWIRQPPGKGLEWIG
YIYYSGSTNYNPSLKSRVTISVDTSKNQFSLKLSSVTAADTAVYYCVSL
VYCGGDCYSGFDYWGQGTLVTVSSAAALDNEKSNGTIIHVKGKHLCP
 
SPLFPGPSKPFWVLVVVGGVLACYSLLVTVAFIIFWVRSKRSRLLHSD
YMNMTPRRPGPTRKHYQPYAPPRDFAAYRSRVKFSRSADAPAYQQG
QNQLYNELNLGRREEYDVLDKRRGRDPEMGGKPRRKNPQEGLYNEL
QKDKMAEAYSEIGMKGERRRGKGHDGLYQGLSTATKDTYDA
LHMQALPPR (SEQ ID NO. 38)
Clone 24C1 CD28T CD3 zeta CAR DNA Heavy & Light Chains 
GATATCCAGCTCACGCAATCCCCCTCAAGCTTGAGTGCCTCCGTGG
GCGACCGGGTGTCCTTCACATGTCAGGCAAGCCAAGACATAAATA
ATTTCCTGAATTGGTACCAACAAAAACCCGGCAAGGCTCCCAAACT
CCTGATTTATGATGCCTCCAATCTGGAGACCGGGGTCCCTTCTAGA
TTCAGCGGAAGTGGCAGCGGCACAGACTTTACATTTACTATCTCTT
CTCTGCAACCAGAGGACATCGCCACATACTATTGCCAGCAATACGG
CAATCTGCCCTTCACCTTCGGAGGCGGAACCAAGGTAGAAATTAAA
AGGGGCGGTGGAGGCTCCGGAGGGGGGGCTCTGGCGGAGGGGG
CTCCCAAGTACAATTGCAGGAGTCAGGCCTGGACTCGTGAAGCCT
TCAGAAACTTTGTCACTGACATGTACAGTGTCCGGCGGAAGCATTT
CCAGTTACTATTGGTCCTGGATTAGACAGCCACCCGGCAAAGGACT
GGAATGGATTGGATATATCTACTACTCTGGATCTACAAACTATAAT
CCCAGCCTCAAATCCAGGGTCACTATTAGTGTGGATACATCAAAGA
ATCAGTTCTCCTTGAAGCTGAGCTCAGTCACTGCTGCCGACACCGC
AGTGTACTATTGTGTGAGCCTGGTCTACTGCGGCGGAGATTGCTAC
AGCGGTTTCGATTACTGGGGCCAGGGCACCCTGGTTACCGTTAGTT
CCGCGGCTGCTCTTGATAACGAGAAGTCCAACGGTACGATTATCCA
CGTTAAGGGTAAGCACCTTTGCCCTAGCCCGCTGTTCCCAGGCCCC
AGTAAGCCCTTTTGGGTCCTCGTTGTGGTAGGTGGGGTACTCGCCT
GCTACTCCCTGCTCGTCACTGTCGCATTCATCATCTTCTGGGTCAGA
TCCAAAAGAAGCCGCCTGCTCCATAGCGATTACATGAATATGACTC
CACGCCGCCCTGGCCCCACAAGGAAACACTACCAGCCTTACGCACC
ACCTAGAGATTTCGCTGCCTATCGGAGCAGGGTGAAGTTTTCCAGA
TCTGCAGATGCACCAGCGTATCAGCAGGGCCAGAACCAACTGTAT
AACGAGCTCAACCTGGGACGCAGGGAAGAGTATGACGTTTTGGAC
AAGCGCAGAGGACGGGACCCTGAGATGGGTGGCAAACCAAGACG
AAAAAACCCCCAGGAGGGTCTCTATAATGAGCTGCAGAAGGATAA
 
GATGGCTGAAGCCTATTCTGAAATAGGCATGAAAGGAGAGCGGAG
AAGGGGAAAAGGGCACGACGGTTTGTACCAGGGACTCAGCACTGC
TACGAAGGATACTTATGACGCTCTCCACATGCAAGCCCTGCCACCT
AGG (SEQ ID NO. 39)
Clone 24C1 CD28T CD3 zeta CAR AA Heavy & Light Chains 
DIQLTQSPSSLSASVGDRVSFTCQASQDINNFLNWYQQKPGKAPKLLIY
DASNLETGVPSRFSGSGSGTDFTFTISSLQPEDIATYYCQQYGNLPFTFG
GGTKVEIKRGGGGSGGGGSGGGGSQVQLQESGPGLVKPSETLSLTCT
VSGGSISSYYWSWIRQPPGKGLEWIGYIYYSGSTNYNPSLKSRVTISVD
TSKNQFSLKLSSVTAADTAVYYCVSLVYCGGDCYSGFDYWGQGTLV
TVSSAAALDNEKSNGTIIHVKGKHLCPSPLFPGPSKPFWVLVVVGGVL
ACYSLLVTVAFIIFWVRSKRSRLLHSDYMNMTPRRPGPTRKHYQPYAP
PRDFAAYRSRVKFSRSADAPAYQQGQNQLYNELNLGRREEYDVLDK
RRGRDPEMGGKPRRKNPQEGLYNELQKDKMAEAYSEIGMKGERRRG
KGHDGLYQGLSTATKDTYDALHMQALPPR (SEQ ID NO. 40)
Clone 24C1 CD28 CD3 zeta CAR DNA AA Heavy & Light Chains 
ATGGCACTCCCCGTAACTGCTCTGCTGCTGCCGTTGGCATTGCTCCT
GCACGCCGCACGCCCGGATATCCAGCTGACCCAGTCTCCATCCTCT
TTGAGTGCCTCCGTGGGTGACCGCGTCTCTTTCACTTGCCAAGCCA
GCCAAGACATCAACAACTTTCTGAATTGGTACCAGCAGAAACCAG
GCAAAGCACCAAAGCTCCTCATCTACGACGCCTCCAACCTGGAAAC
CGGGGTGCCCAGCAGGTTTAGCGGAGCGGTTCTGGCACGGATTTT
ACGTTCACCATCTCCTCTCTGCAGCCCGAGGATATAGCTACTTATTA
CTGTCAGCAGTACGGGAATCTGCCATTTACTTTTGGGGGTGGAACT
AAGGTGGAAATCAAAAGGGGCGGCGGGGGAAGCGGGGGCGGGGG
CTCAGGTGGCGGAGGGAGCCAGGTGCAACTCCAGGAAAGTGGCCC
AGGATTGGTGAAGCCCAGCGAGACCCTTTCCCTTACTTGTACTGTT
AGCGGAGGCAGCATAAGCAGCTACTATTGGTCCTGGATCAGACAG
CCACCAGGGAAAGGGCTTGAATGGATTGGCTACATTTACTATTCCG
GGTCCACCAACTACAACCCATCCCTCAAGTCCCGCGTGACAATTTC
CGTCGACACAAGCAAGAACCAGTTCTCCCTGAAACTTAGTAGCGTC
ACTGCTGCAGATACAGCAGTGTACTATTGTGTCAGCCTTGTCTACT
GTGGCGGCGACTGCTACAGTGGCTTTGATTACTGGGGACAGGGCAC
 
GCTCGTGACAGTGTCCAGCGCTGCGGCTATCGAGGTAATGTATCCG
CCACCGTATCTGGACAACGAGAAGTCTAATGGACAATCATTCACG
TGAAGGGGAAGCACCTGTGTCCATCCCCCCTGTTTCCGGGTCCCAG
TAAACCCTTCTGGGTGCTTGTTGTCGTTGGCGGGGTGCTGGCCTGCT
ATTCCCTGCTGGTGACCGTCGCGTTTATTATTTCTGGGTTAGATCC
AAAAGAAGCCGCCTGCTCCATAGCGATTACATGAATATGACTCCAC
GCCGCCCTGGCCCCACAAGGAAACACTACCAGCCTTACGCACCACC
TAGAGATTTCGCTGCCTATCGGAGCAGGGTGAAGTTTTCCAGATCT
GCAGATGCACCAGCGTATCAGCAGGGCCAGAACCAACTGTATAAC
GAGCTCAACCTGGGACGCAGGGAAGAGTATGACGTTTTGGACAAG
CGCAGAGGACGGGACCCTGAGATGGGTGGCAAACCAAGACGAAA
AAACCCCCAGGAGGGTCTCTATAATGAGCTGCAGAAGGATAAGAT
GGCTGAAGCCTATTCTGAAATAGGCATGAAAGGAGAGCGGAGAAG
GGGAAAAGGGCACGACGGTTTGTACCAGGGACTCAGCACTGCTAC
GAAGGATACTTATGACGCTCTCCACATGCAAGCCCTGCCACCTAGG
TAA (SEQ ID NO. 41)
Clone 24C1 CD28 CD3 zeta CAR AA Heavy & Light Chains
   (Signal Peptide in **Bold**) 
MALPVTALLLPLALLLHAARPDIQLTQSPSSLSASVGDRVSFTCQAS
QDINNFLNWYQQKPGKAPKLLIYDASNLETGVPSRFSGSGSGTDFTFTI
SSLQPEDIATYYCQQYGNLPFTFGGGTKVEIKRGGGGSGGGGSGGGGS
QVQLQESGPGLVKPSETLSLTCTVSGGSISSYYWSWIRQPPGKGLEWIG
YIYYSGSTNYNPSLKSRVTISVDTSKNQFSLKLSSVTAADTAVYYCVSL
VYCGGDCYSGFDYWGQGTLVTVSSAAAIEVMYPPPYLDNEKSNGTII
HVKGKHLCPSPLFPGPSKPFWVLVVVGGVLACYSLLVTVAFIIFWVRS
KRSRLLHSDYMNMTPRRPGPTRKHYQPYAPPRDFAAYRSRVKFSRSA
DAPAYQQGQNQLYNELNLGRREEYDVLDKRRGRDPEMGGKPRRKNP
QEGLYNELQKDKMAEAYSEIGMKGERRRGKGHDGLYQGLSTATKDT
YDALHMQALPPR (SEQ ID NO. 42)
Clone 24C1 CD28 CD3 zeta CAR DNA Heavy & Light Chains 
GATATCCAGCTGACCCAGTCTCCATCCTCTTTGAGTGCCTCCGTGG
GTGACCGCGTCTCTTTCACTTGCCAAGCCAGCCAAGACATCAACAA
CTTTCTGAATTGGTACCAGCAGAAACCAGGCAAAGCACCAAAGCT
 
CCTCATCTACGACGCCTCCAACCTGGAAACCGGGGTGCCCAGCAGG
TTTAGCGGGAGCGGTTCTGGCACGGATTTACGTTCACCATCTCCTC
TCTGCAGCCCGAGGATATAGCTACTTATTACTGTCAGCAGTACGGG
AATCTGCCATTTACTTTTGGGGGTGGAACTAAGGTGGAAATCAAAA
GGGGCGGCGGGGGAAGCGGGGGCGGGGGCTCAGGTGGCGGAGGG
AGCCAGGTGCAACTCCAGGAAAGTGGCCCAGGATTGGTGAAGCCC
AGCGAGACCCTTTCCCTTACTTGTACTGTTAGCGGAGGCAGCATAA
GCAGCTACTATTGGTCCTGGATCAGACAGCCACCAGGGAAAGGGC
TTGAATGGATTGGCTACATTTACTATTCCGGGTCCACCAACTACAA
CCCATCCCTCAAGTCCCGCGTGACAATTTCCGTCGACACAAGCAAG
AACCAGTTCTCCCTGAAACTTAGTAGCGTCACTGCTGCAGATACAG
CAGTGTACTATTGTGTCAGCCTTGTCTACTGTGGCGGCGACTGCTA
CAGTGGCTTTGATTACTGGGGACAGGGCACGCTCGTGACAGTGTCC
AGCGCTGCGGCTATCGAGGTAATGTATCCGCCACCGTATCTGGACA
ACGAGAAGTCTAATGGGACAATCATTCACGTGAAGGGGAAGCACC
TGTGTCCATCCCCCCTGTTTCCGGGTCCCAGTAAACCCTTCTGGGTG
CTTGTTGTCGTTGGCGGGGTGCTGGCCTGCTATTCCCTGCTGGTGAC
CGTCGCGTTTATTATTTTCTGGGTTAGATCCAAAAGAAGCCGCCTG
CTCCATAGCGATTACATGAATATGACTCCACGCCGCCCTGGCCCCA
CAAGGAAACACTACCAGCCTTACGCACCACCTAGAGATTTCGCTGC
CTATCGGAGCAGGGTGAAGTTTTCCAGATCTGCAGATGCACCAGCG
TATCAGCAGGGCCAGAACCAACTGTATAACGAGCTCAACCTGGGA
CGCAGGGAAGAGTATGACGTTTTGGACAAGCGCAGAGGACGGGAC
CCTGAGATGGGTGGCAAACCAAGACGAAAAAACCCCCAGGAGGGT
CTCTATAATGAGCTGCAGAAGGATAAGATGGCTGAAGCCTATTCTG
AAATAGGCATGAAAGGAGAGCGGAGAAGGGGAAAAGGGCACGAC
GGTTTGTACCAGGGACTCAGCACTGCTACGAAGGATACTTATGACG
CTCTCCACATGCAAGCCCTGCCACCTAGG (SEQ ID NO. 43)
Clone 24C1 CD28 CD3 zeta CAR AA Heavy & Light Chains 
DIQLTQSPSSLSASVGDRVSFTCQASQDINNFLNWYQQKPGKAPKLLIY
DASNLETGVPSRFSGSGSGTDFTFTISSLQPEDIATYYCQQYGNLPFTFG
GGTKVEIKRGGGGSGGGGSGGGGSQVQLQESGPGLVKPSETLSLTCT
VSGGSISSYYWSWIRQPPGKGLEWIGYIYYSGSTNYNPSLKSRVTISVD
 
TSKNQFSLKLSSVTAADTAVYYCVSLVYCGGDCYSGFDYWGQGTLV
TVSSAAAIEVMYPPPYLDNEKSNGTIIHVKGKHLCPSPLFPGPSKPFWV
LVVVGGVLACYSLLVTVAFIIFWVRSKRSRLLHSDYMNMTPRRPGPTR
KHYQPYAPPRDFAAYRSRVKFSRSADAPAYQQGQNQLYNELNLGRRE
EYDVLDKRRGRDPEMGGKPRRKNPQEGLYNELQKDKMAEAYSEIGM
KGERRRGKGHDGLYQGLSTATKDTYDALHMQALPPR (SEQ ID NO.
44)
Clone 24C1 CD8 CD3 zeta CAR DNA Heavy & Light Chains 
ATGGCACTCCCCGTAACTGCTCTGCTGCTGCCGTTGGCATTGCTCCT
GCACGCCGCACGCCCGGACATTCAATTGACCCAGTCCCCTAGCAGT
CTCTCAGCAAGTGTGGGAGATAGGGTGTCATTCACCTGTCAGGCTT
CACAGGACATCAACAACTTCCTCAATTGGTATCAGCAGAAGCCAG
GGAAGGCACCAAAGCTGCTCATATATGACGCTTCAAACCTTGAAAC
CGGAGTACCTAGCCGCTTCAGCGGAAGCGGATCAGGGACTGACTT
CACTTTTACCATCTCTTCACTGCAGCCCGAAGACATCGCCACATAC
TACTGCCAGCAGTACGGAAACTTGCCTTTTACATTTGGGGGCGGCA
CCAAAGTGGAGATTAAGCGAGGGGGAGGCGGCTCAGGAGGCGGTG
GCTCCGGAGGCGGGGGTTCCCAGGTCCAGCTCCAGGAATCCGGCC
CAGGTCTGGTTAAGCCCAGTGAAACTTTGTCCCTCACGTGTACTGT
GAGCGGTGGTTCAATCTCCTCATACTATTGGTCTTGGATACGGCAA
CCTCCTGGAAAGGGCCTCGAGTGGATCGGCTATATCTACTATAGTG
GCTCCACTAATTACAACCCTTCCCTCAAGTCCAGAGTCACCATTTCC
GTGGACACATCTAAGAACCAGTTCAGTCTGAAGTTGTCCAGCGTTA
CAGCCGCAGACACAGCCGTTTATTACTGTGTGTCTCTTGTTTACTGC
GGGGGAGACTGTTATAGCGGCTTCGATTACTGGGGCCAGGGCACCT
TGGTCACAGTCTCTTCCGCGGCCGCCCTCTCTAACAGTATTATGTAC
TTTTCTCATTTTGTACCCGTGTTCCTTCCCGCTAAGCCAACTACTAC
CCCGGCCCCACGGCCGCCTACCCCTGCACCCACAATAGCCAGTCAG
CCTTTGAGCCTGAGACCTGAGGCTTGTCGGCCGGCTGCTGGGGGTG
CAGTGCACACACGAGGTCTTGATTTTGCTTGCGACATATACATCTG
GGCCCCTCTGGCCGGGACCTGTGGGGTGCTGCTTCTGAGCTTGGTC
ATCACGCTCTATTGCAACCATCGCAACAGATCCAAAAGAAGCCGCC
TGCTCCATAGCGATTACATGAATATGACTCCACGCCGCCCTGGCCC
 
CACAAGGAAACACTACCAGCCTTACGCACCACCTAGAGATTTCGCT
GCCTATCGGAGCAGGGTGAAGTTTCCAGATCTGCAGATGCACCAG
CGTATCAGCAGGGCCAGAACCAACTGTATAACGAGCTCAACCTGG
GACGCAGGGAAGAGTATGACGTTTTGGACAAGCGCAGAGGACGGG
ACCCTGAGATGGGTGGCAAACCAAGACGAAAAACCCCCAGGAGG
GTCTCTATAATGAGCTGCAGAAGGATAAGATGGCTGAAGCCTATTC
TGAAATAGGCATGAAAGGAGAGCGGAGAAGGGGAAAAGGGCACG
ACGGTTTGTACCAGGGACTCAGCACTGCTACGAAGGATACTTATGA
CGCTCTCCACATGCAAGCCCTGCCACCTAGGTAA (SEQ ID NO. 45)
Clone 24C1 CD8 CD3 zeta CAR AA Heavy & Light Chains
   (Signal Peptide in **Bold**) 
MALPVTALLLPLALLLHAARPDIQLTQSPSSLSASVGDRVSFTCQAS
QDINNFLNWYQQKPGKAPKLLIYDASNLETGVPSRFSGSGSGTDFTFTI
SSLQPEDIATYYCQQYGNLPFTFGGGTKVEIKRGGGGSGGGGSGGGGS
QVQLQESGPGLVKPSETLSLTCTVSGGSISSYYWSWIRQPPGKGLEWIG
YIYYSGSTNYNPSLKSRVTISVDTSKNQFSLKLSSVTAADTAVYYCVSL
VYCGGDCYSGFDYWGQGTLVTVSSAAALSNSIMYFSHFVPVFLPAKP
TTTPAPRPPTPAPTIASQPLSLRPEACRPAAGGAVHTRGLDFACDIYIW
APLAGTCGVLLLSLVITLYCNHRNRSKRSRLLHSDYMNMTPRRPGPTR
KHYQPYAPPRDFAAYRSRVKFSRSADAPAYQQGQNQLYNELNLGRRE
EYDVLDKRRGRDPEMGGKPRRKNPQEGLYNELQKDKMAEAYSEIGM
KGERRRGKGHDGLYQGLSTATKDTYDALHMQALPPR (SEQ ID NO.
46)
Clone 24C1 CD8 CD3 zeta CAR DNA Heavy & Light Chains 
GACATTCAATTGACCCAGTCCCCTAGCAGTCTCTCAGCAAGTGTGG
GAGATAGGGTGTCATTCACCTGTCAGGCTTCACAGGACATCAACAA
CTTCCTCAATTGGTATCAGCAGAAGCCAGGGAAGGCACCAAAGCT
GCTCATATATGACGCTTCAAACCTTGAAACCGGAGTACCTAGCCGC
TTCAGCGGAAGCGGATCAGGGACTGACTTCACTTTTACCATCTCTT
CACTGCAGCCCGAAGACATCGCCACATACTACTGCCAGCAGTACG
GAAACTTGCCTTTTACATTTGGGGGCGGCACCAAAGTGGAGATTAA
GCGAGGGGGAGGCGGCTCAGGAGGCGGTGGCTCCGGAGGCGGGG
GTTCCCAGGTCCAGCTCCAGGAATCCGGCCCAGGTCTGGTTAAGCC
 
CAGTGAAACTTTGTCCCTCACGTGTACTGTGAGCGGTGGTTCAATC
TCCTCATACTATTGGTCTTGGATACGGCAACCTCCTGGAAAGGGCC
TCGAGTGGATCGGCTATATCTACTATAGTGGCTCCACTAATTACAA
CCCTTCCCTCAAGTCCAGAGTCACCATTTCCGTGGACACATCTAAG
AACCAGTTCAGTCTGAAGTTGTCCAGCGTTACAGCCGCAGACACAG
CCGTTTATTACTGTGTGTCTCTTGTTTACTGCGGGGGAGACTGTTAT
AGCGGCTTCGATTACTGGGGCCAGGGCACCTTGGTCACAGTCTCTT
CCGCGGCCGCCCTCTCTAACAGTATTATGTACTTTTCTCATTTTGTA
CCCGTGTTCCTTCCCGCTAAGCCAACTACTACCCCGGCCCCACGGC
CGCCTACCCCTGCACCCACAATAGCCAGTCAGCCTTTGAGCCTGAG
ACCTGAGGCTTGTCGGCCGGCTGCTGGGGGTGCAGTGCACACACG
AGGTCTTGATTTTGCTTGCGACATATACATCTGGGCCCCTCTGGCCG
GGACCTGTGGGGTGCTGCTTCTGAGCTTGGTCATCACGCTCTATTG
CAACCATCGCAACAGATCCAAAAGAAGCCGCCTGCTCCATAGCGA
TTACATGAATATGACTCCACGCCGCCCTGGCCCCACAAGGAAACAC
TACCAGCCTTACGCACCACCTAGAGATTTCGCTGCCTATCGGAGCA
GGGTGAAGTTTTCCAGATCTGCAGATGCACCAGCGTATCAGCAGGG
CCAGAACCAACTGTATAACGAGCTCAACCTGGGACGCAGGGAAGA
GTATGACGTTTTGGACAAGCGCAGAGGACGGGACCCTGAGATGGG
TGGCAAACCAAGACGAAAAAACCCCCAGGAGGGTCTCTATAATGA
GCTGCAGAAGGATAAGATGGCTGAAGCCTATTCTGAAATAGGCAT
GAAAGGAGAGCGGAGAAGGGGAAAAGGGCACGACGGTTTGTACC
AGGGACTCAGCACTGCTACGAAGGATACTTATGACGCTCTCCACAT
GCAAGCCCTGCCACCTAGG (SEQ ID NO. 47)
Clone 24C1 CD8 CD3 zeta CAR AA Heavy & Light Chains 
DIQLTQSPSSLSASVGDRVSFTCQASQDINNFLNWYQQKPGKAPKLLIY
DASNLETGVPSRFSGSGSGTDFTFTISSLQPEDIATYYCQQYGNLPFTFG
GGTKVEIKRGGGGSGGGGSGGGGSQVQLQESGPGLVKPSETLSLTCT
VSGGSISSYYWSWIRQPPGKGLEWIGYIYYSGSTNYNPSLKSRVTISVD
TSKNQFSLKLSSVTADTAVYYCVSLVYCGGDCYSGFDYWGQGTLVT
VSSAAALSNSIMYFSHFVPVFLPAKPTTTPAPRPPTPAPTIASQPLSLRPE
ACRPAAGGAVHTRGLDFACDIYIWAPLAGTCGVLLLSLVITLYCNHRN
RSKRSRLLHSDYMNMTPRRPGPTRKHYQPYAPPRDFAAYRSRVKFSR
 
SADAPAYQQGQNQLYNELNLGREEYDVLDKRRGRDPEMGGKPRRKN
PQEGLYNELQKDKMAEAYSEIGMKGERRRGKGHDGLYQGLSTATK
DTYDALHMQA LPPR (SEQ ID NO. 48)
Clone 24C8 Heavy Chain (HC) DNA 
CAGGTACAGCTGCAGGAATCTGGGCCCGGACTTGTCAAGCCAAGT
CAGACACTTTCTCTTACATGTACCGTGAGCGGCGGAAGTATAAGCA
GTGGAGGCTTTTACTGGTCTTGGATACGGCAGCACCCAGGCAAAGG
CTTGGAGTGGATTGGATACATTCATCATTCAGGATCTACACACTAT
AATCCATCCCTTAAGTCCCGGGTCACCATTAGCATTGATACGTCTA
AGAATCTGTTCAGTCTCAGGCTGTCCTCCGTCACTGCTGCCGACAC
AGCCGTGTACTACTGCGCCTCCTTGGTTTACTGCGGAGGCGACTGT
TATAGCGGCTTTGATTATTGGGGGCAGGGGACCCTCGTAACCGTGA
GCTCT (SEQ ID NO. 49)
Clone 24C8 AA HC (CDRs in Underline) 
QVQLQESGPGLVKPSQTLSLTCTVSGGSISSGGFYWSWIRQHPGKGLE
WIGYIHHSGSTHYNPSLKSRVTISIDTSKNLFSLRLSSVTAADTAVYYC
ASLVYCGGDCYSGFDYWGQGTLVTVSS (SEQ ID NO. 50)
Clone 24C8 HC CDR1 AA: GGSISSGGF (SEQ ID NO. 51)
Clone 24C8 HC CDR2 AA: HHSGS (SEQ ID NO. 52)
Clone 24C8 HC CDR3 AA: LVYCGGDCYS GFDY (SEQ ID NO. 53)
Clone 24C8 Light Chain (LC) DNA 
GATATCCAGCTCACTCAAAGCCCCTCTAGTCTCTCTGCCTCAGTGG
GGGATCGGGTCAGTTTACTTGTCAAGCTTCACAGGATATCAACAA
CTTCCTTAATTGGTATCAGCAGAAGCCAGGAAAAGCACCCAAGCTG
CTCATCTATGATGCCTCAAATTTGGAGACGGGTGTTCCCAGTCGAT
TCTCTGGGTCAGGGTCCGGGACCGACTTTACGTTTACGATCTCCTCT
CTGCAGCCCGAAGACATCGCCACATACTATTGTCAACAGTACGGCA
ACTTGCCTTTCACATTTGGGGGCGGGACTAAGGTTGAAATCAAGAG
G (SEQ ID NO. 54)
Clone 24C8 LC AA (CDRs in Underline) 
DIQLTQSPSSLSASVGDRVSFTCQASQDINNFLNWYQQKPGKAPKLLIY
DASNLETGVPSRFSGSGSGTDFTFTISSLQPEDIATYYCQQYGNLPFTFG
GGTKVEIKR (SEQ ID NO. 55)
Clone 24C8 LC CDR1 AA: QASQDINNFLN (SEQ ID NO. 56)
Clone 24C8 LC CDR2 AA: DASNLET (SEQ ID NO. 57)
Clone 24C8 LC CDR3 AA: QQYGNLPFT (SEQ ID NO. 58)
Clone 24C8 CD28T CD3 zeta CAR DNA Heavy & Light Chains 
ATGGCACTCCCCGTAACTGCTCTGCTGCTGCCGTTGGCATTGCTCCT
GCACGCCGCACGCCCGCAGGTACAGCTGCAGGAATCTGGCCCGG
ACTTGTCAAGCCAAGTCAGACACTTTCTCTTACATGTACCGTGAGC
GGCGGAAGTATAAGCAGTGGAGGCTTTACTGGTCTTGGATACGGC
AGCACCCAGGCAAAGGCTTGGAGTGGATTGGATACATTCATCATTC
AGGATCTACACACTATAATCCATCCCTTAAGTCCCGGGTCACCATT
AGCATTGATACGTCTAAGAATCTGTTCAGTCTCAGGCTGTCCTCCG
TCACTGCTGCCGACACAGCCGTGTACTACTGCGCCTCCTTGGTTTAC
TGCGGAGGCGACTGTTATAGCGGCTTTGATTATTGGGGGCAGGGA
CCCTCGTAACCGTGAGCTCTGGAGGGGGTGGAGCGGGGGAGGAG
GTTCAGGGGGGGGCGGCTCCGATATCCAGCTCACTCAAAGCCCCTC
TAGTCTCTCTGCCTCAGTGGGGGATCGGGTCAGTTTACTTGTCAA
GCTTCACAGGATATCAACAACTTCCTTAATTGGTATCAGCAGAAGC
CAGGAAAAGCACCCAAGCTGCTCATCTATGATGCCTCAAATTTGGA
GACGGGTGTTCCCAGTCGATTCTCTGGGTCAGGGTCCGGGACCGAC
TTTACGTTTACGATCTCCTCTCTGCAGCCCGAAGACATCGCCACAT
ACTATTGTCAACAGTACGGCAACTTGCCTTTCACATTTGGGGGCGG
GACTAAGGTTGAAATCAAGAGGGCCGCTGCACTGGACAATGAGAA
GTCCAACGGCACCATCATCCACGTGAAGGGCAAGCACCTGTGCCCT
AGTCCTCTGTTCCCAGGCCCATCCAAACCTTTTTGGGTTCTTGTTGT
GGTCGGGGGGGTGCTGGCCTGCTATTCTCTGCTGGTCACGGTGGCC
TTCATAATTTTCTGGGTTAGATCCAAAAGAAGCCGCCTGCTCCATA
GCGATTACATGAATATGACTCCACGCCGCCCTGGCCCCACAAGGAA
ACACTACCAGCCTTACGCACCACCTAGAGATTTCGCTGCCTATCGG
AGCAGGGTGAAGTTTTCCAGATCTGCAGATGCACCAGCGTATCAGC
 
AGGGCCAGAACCAACTGTATAACGAGCTCAACCTGGGACGCAGGG
AAGAGTATGACGTTTTGGACAAGCGCAGAGGACGGGACCCTGAGA
TGGGTGGCAAACCAAGACGAAAAAACCCCCAGGAGGGTCTCTATA
ATGAGCTGCAGAAGGATAAGATGGCTGAAGCCTATTCTGAAATAG
GCATGAAAGGAGAGCGGAGAAGGGGAAAAGGGCACGACGGTTTG
TACCAGGGACTCAGCACTGCTACGAAGGATACTTATGACGCTCTCC
ACATGCAAGCCCTGCCACCTAGGTAA (SEQ ID NO. 59)
Clone 24C8 CD28T CD3 zeta CAR AA Heavy & Light Chains
   (Signal Peptide in **Bold**) 
MALPVTALLLPLALLLHAARPQVQLQESGPGLVKPSQTLSLTCTVSG
GSISSGGFYWSWIRQHPGKGLEWIGYIHHSGSTHYNPSLKSRVTISIDTS
KNLFSLRLSSVTAADTAVYYCASLVYCGGDCYSGFDYWGQGTLVTV
SSGGGGSGGGGSGGGGSDIQLTQSPSSLSASVGDRVSFTCQASQDINNF
LNWYQQKPGKAPKLLIYDASNLETGVPSRFSGSGSGTDFTFTISSLQPE
DIATYYCQQYGNLPFTFGGGTKVEIKRAAALDNEKSNGTIIHVKGKHL
CPSPLFPGPSKPFWVLVVVGGVLACYSLLVTVAFIIFWVRSKRSRLLHS
DYMNMTPRRPGPTRKHYQPYAPPRDFAAYRSRVKFSRSADAPAYQQ
GQNQLYNELNLGRREEYDVLDKRRGRDPEMGGKPRRKNPQEGLYNE
LQKDKMAEAYSEIGMKGERRRGKGHDGLYQGLSTATKDTYDALHM
QALPPR (SEQ ID NO. 60)
Clone 24C8 CD28T CD3 zeta CAR DNA Heavy & Light Chains 
CAGGTACAGCTGCAGGAATCTGGGCCCGGACTTGTCAAGCCAAGT
CAGACACTTTCTCTTACATGTACCGTGAGCGGCGGAAGTATAAGCA
GTGGAGGCTTTTACTGGTCTTGGATACGGCAGCACCCAGGCAAAGG
CTTGGAGTGGATTGGATACATTCATCATTCAGGATCTACACACTAT
AATCCATCCCTTAAGTCCCGGGTCACCATTAGCATTGATACGTCTA
AGAATCTGTTCAGTCTCAGGCTGTCCTCCGTCACTGCTGCCGACAC
AGCCGTGTACTACTGCGCCTCCTTGGTTTACTGCGGAGGCGACTGT
TATAGCGGCTTTGATTATTGGGGGCAGGGGACCCTCGTAACCGTGA
GCTCTGGAGGGGGTGGGAGCGGGGGAGGAGGTTCAGGGGGGGGC
GGCTCCGATATCCAGCTCACTCAAAGCCCCTCTAGTCTCTCTGCCTC
AGTGGGGGATCGGGTCAGTTTTACTTGTCAAGCTTCACAGGATATC
AACAACTTCCTTAATTGGTATCAGCAGAAGCCAGGAAAAGCACCC
 
AAGCTGCTCATCTATGATGCCTCAAATTTGGAGACGGGTGTTCCCA
GTCGATTCTCTGGGTCAGGGTCCGGGACCGACTTTACGTTACGAT
CTCCTCTCTGCAGCCCGAAGACATCGCCACATACTATTGTCAACAG
TACGGCAACTTGCCTTTCACATTTGGGGGCGGGACTAAGGTTGAAA
TCAAGAGGGCCGCTGCACTGGACAATGAGAAGTCCAACGGCACCA
TCATCCACGTGAAGGGCAAGCACCTGTGCCCTAGTCCTCTGTTCCC
AGGCCCATCCAAACCTTTTTGGGTTCTTGTTGTGGTCGGGGGGGTG
CTGGCCTGCTATTCTCTGCTGGTCACGGTGGCCTTCATAATTTTCTG
GGTTAGATCCAAAAGAAGCCGCCTGCTCCATAGCGATTACATGAAT
ATGACTCCACGCCGCCCTGGCCCCACAAGGAAACACTACCAGCCTT
ACGCACCACCTAGAGATTTCGCTGCCTATCGGAGCAGGGTGAAGTT
TTCCAGATCTGCAGATGCACCAGCGTATCAGCAGGGCCAGAACCA
ACTGTATAACGAGCTCAACCTGGGACGCAGGGAAGAGTATGACGT
TTTGGACAAGCGCAGAGGACGGGACCCTGAGATGGGTGGCAAACC
AAGACGAAAAAACCCCCAGGAGGGTCTCTATAATGAGCTGCAGAA
GGATAAGATGGCTGAAGCCTATTCTGAAATAGGCATGAAAGGAGA
GCGGAGAAGGGGAAAAGGGCACGACGGTTTGTACCAGGGACTCAG
CACTGCTACGAAGGATACTTATGACGCTCTCCACATGCAAGCCCTG
CCACCTAGG (SEQ ID NO. 61)
Clone 24C8 CD28T CD3 zeta CAR AA Heavy & Light Chains 
QVQLQESGPGLVKPSQTLSLTCTVSGGSISSGGFYWSWIRQHPGKGLE
WIGYIHHSGSTHYNPSLKSRVTISIDTSKNLFSLRLSSVTAADTAVYYC
ASLVYCGGDCYSGFDYWGQGTLVTVSSGGGGSGGGGSGGGGSDIQL
TQSPSSLSASVGDRVSFTCQASQDINNFLNWYQQKPGKAPKLLIYDAS
NLETGVPSRFSGSGSGTDFTFTISSLQPEDIATYYCQQYGNLPFTFGGGT
KVEIKRAAALDNEKSNGTIIHVKGKHLCPSPLFPGPSKPFWVLVVVGG
VLACYSLLVTVAFIIFWVRSKRSRLLHSDYMNMTPRRPGPTRKHYQPY
APPRDFAAYRSRVKFSRSADAPAYQQGQNQLYNELNLGRREEYDVLD
KRRGRDPEMGGKPRRKNPQEGLYNELQKDKMAEAYSEIGMKGERRR
GKGHDGLYQGLSTATKDTYDALHMQALPPR (SEQ ID NO. 62)
Clone 24C8 CD28 CD3 zeta CAR DNA Heavy & Light Chains 
ATGGCACTCCCCGTAACTGCTCTGCTGCTGCCGTTGGCATTGCTCCT
GCACGCCGCACGCCCGCAGGTGCAGCTGCAGGAAAGCGGTCCGGG
 
ACTTGTCAAGCCGTCCCAAACGCTGAGTCTGACGTGTACTGTCTCT
GGTGGCTCTATTTCTTCCGGGGGCTTTATTGGTCTTGGATCAGACA
ACACCCTGGCAAAGGGCTGGAGTGGATAGGGTATATTCACCACTCT
GGGTCCACTCACTACAACCCATCATTGAAATCCAGAGTGACTATCT
CAATCGACACATCCAAGAACCTTTTCAGCCTGAGGTTGTCATCAGT
TACCGCCGCTGACACCGCGGTGTATTATTGCGCCTCTCTCGTGTACT
GCGGTGGCGATTGTTATAGTGGCTTTGACTACTGGGGGCAGGGGAC
ATTGGTTACCGTTTCAAGTGGAGGCGGTGGGTCTGGCGGGGGCGGT
AGCGGAGGTGGGGGGAGCGACATACAGCTTACGCAGAGCCCCTCC
AGCCTTTCAGCCTCCGTGGGGGATAGGGTGTCCTTTACCTGCCAGG
CTTCCCAGGACATAAACAACTTCCTCAATTGGTATCAGCAAAAGCC
CGGGAAAGCACCAAAGCTGCTCATCTACGATGCCAGCAACCTGGA
AACCGGAGTGCCGTCTCGCTTCTCTGGAAGTGGCAGTGGGACCGAT
TTCACTTTTACAATCTCAAGTTTGCAGCCAGAAGACATTGCAACAT
ACTACTGTCAACAGTACGGCAATCTCCCCTTTACATTTGGGGGGGG
AACTAAAGTGGAGATTAAGCGCGCTGCAGCCATTGAAGTTATGTAT
CCGCCCCCGTATCTGGATAACGAGAAATCTAATGGTACCATAATAC
ATGTGAAGGGGAAGCACCTCTGTCCATCACCGCTGTTCCCCGGCCC
TTCAAAACCTTTCTGGGTACTCGTTGTCGTGGGTGGAGTTCTGGCCT
GCTATAGTCTGCTGGTGACCGTGGCGTTTATCATCTTCTGGGTAAG
ATCCAAAAGAAGCCGCCTGCTCCATAGCGATTACATGAATATGACT
CCACGCCGCCCTGGCCCCACAAGGAAACACTACCAGCCTTACGCAC
CACCTAGAGATTTCGCTGCCTATCGGAGCAGGGTGAAGTTTTCCAG
ATCTGCAGATGCACCAGCGTATCAGCAGGGCCAGAACCAACTGTA
TAACGAGCTCAACCTGGGACGCAGGGAAGAGTATGACGTTTTGGA
CAAGCGCAGAGGACGGGACCCTGAGATGGGTGGCAAACCAAGACG
AAAAAACCCCCAGGAGGGTCTCTATAATGAGCTGCAGAAGGATAA
GATGGCTGAAGCCTATTCTGAAATAGGCATGAAAGGAGAGCGGAG
AAGGGGAAAAGGGCACGACGGTTTGTACCAGGGACTCAGCACTGC
TACGAAGGATACTTATGACGCTCTCCACATGCAAGCCCTGCCACCT
AGGTAA (SEQ ID NO. 63)
Clone 24C8 CD28 CD3 zeta CAR AA Heavy & Light Chains
   (Signal Peptide in **Bold**) 
MALPVTALLLPLALLLHAARPQVQLQESGPGLVKPSQTLSLTCTVSG
GSISSGGFYWSWIRQHPGKGLEWIGYIHHSGSTHYNPSLKSRVTISIDTS
KNLFSLRLSSVTAADTAVYYCASLVYCGGDCYSGFDYWGQGTLVTV
SSGGGGSGGGGSGGGGSDIQLTQSPSSLSASVGDRVSFTCQASQDINNF
LNWYQQKPGKAPKLLIYDASNLETGVPSRFSGSGSGTDFTFTISSLQPE
DIATYYCQQYGNLPFTFGGGTKVEIKRAAAIEVMYPPPYLDNEKSNGT
IIHVKGKHLCPSPLFPGPSKPFWVLVVVGGVLACYSLLVTVAFIIFWVR
SKRSRLLHSDYMNMTPRRPGPTRKHYQPYAPPRDFAAYRSRVKFSRS
ADAPAYQQGQNQLYNELNLGRREEYDVLDKRRGRDPEMGGKPRRK
NPQEGLYNELQKDKMAEAYSEIGMKGERRRGKGHDGLYQGLSTATK
DTYDALHMQALPPR (SEQ ID NO. 64)
Clone 24C8 CD28 CD3 zeta CAR DNA Heavy & Light Chains 
CAGGTGCAGCTGCAGGAAAGCGGTCCGGGACTTGTCAAGCCGTCC
CAAACGCTGAGTCTGACGTGTACTGTCTCTGGTGGCTCTATTTCTTC
CGGGGGCTTTTATTGGTCTTGGATCAGACAACACCCTGGCAAAGGG
CTGGAGTGGATAGGGTATATTCACCACTCTGGGTCCACTCACTACA
ACCCATCATTGAAATCCAGAGTGACTATCTCAATCGACACATCCAA
GAACCTTTTCAGCCTGAGGTTGTCATCAGTTACCGCCGCTGACACC
GCGGTGTATTATTGCGCCTCTCTCGTGTACTGCGGTGGCGATTGTTA
TAGTGGCTTTGACTACTGGGGGCAGGGACATTGGTTACCGTTTCA
AGTGGAGGCGGTGGGTCTGGCGGGGGCGGTAGCGGAGGTGGGGGG
AGCGACATACAGCTTACGCAGAGCCCCTCCAGCCTTTCAGCCTCCG
TGGGGGATAGGGTGTCCTTTACCTGCCAGGCTTCCCAGGACATAAA
CAACTTCCTCAATTGGTATCAGCAAAAGCCCGGGAAAGCACCAAA
GCTGCTCATCTACGATGCCAGCAACCTGGAAACCGGAGTGCCGTCT
CGCTTCTCTGGAAGTGGCAGTGGGACCGATTTCACTTTTACAATCT
CAAGTTTGCAGCCAGAAGACATTGCAACATACTACTGTCAACAGTA
CGGCAATCTCCCCTTTACATTTGGGGGGGAACTAAAGTGGAGATT
AAGCGCGCTGCAGCCATTGAAGTTATGTATCCGCCCCCGTATCTGG
ATAACGAGAAATCTAATGGTACCATAATACATGTGAAGGGAAGC
ACCTCTGTCCATCACCGCTGTTCCCCGGCCCTTCAAAACCTTTCTGG
GTACTCGTTGTCGTGGGTGGAGTTCTGGCCTGCTATAGTCTGCTGGT
GACCGTGGCGTTTATCATCTTCTGGGTAAGATCCAAAAGAAGCCGC
 
CTGCTCCATAGCGATTACATGAATATGACTCCACGCCGCCCTGGCC
CCACAAGGAAACACTACCAGCCTTACGCACCACCTAGAGATTTCGC
TGCCTATCGGAGCAGGGTGAAGTTTCCAGATCTGCAGATGCACCA
GCGTATCAGCAGGGCCAGAACCAACTGTATAACGAGCTCAACCTG
GGACGCAGGGAAGAGTATGACGTTTTGGACAAGCGCAGAGGACGG
GACCCTGAGATGGGTGGCAAACCAAGACGAAAAAACCCCCAGGAG
GGTCTCTATAATGAGCTGCAGAAGGATAAGATGGCTGAAGCCTATT
CTGAAATAGGCATGAAAGGAGAGCGGAGAAGGGGAAAAGGGCAC
GACGGTTTGTACCAGGGACTCAGCACTGCTACGAAGGATACTTATG
ACGCTCTCCACATGCAAGCCCTGCCACCTAGG (SEQ ID NO. 65)
Clone 24C8 CD28 CD3 zeta CAR AA Heavy & Light Chains 
QVQLQESGPGLVKPSQTLSLTCTVSGGSISSGGFYWSWIRQHPGKGLE
WIGYIHHSGSTHYNPSLKSRVTISIDTSKNLFSLRLSSVTAADTAVYYC
ASLVYCGGDCYSGFDYWGQGTLVTVSSGGGGSGGGGSGGGGSDIQL
TQSPSSLSASVGDRVSFTCQASQDINNFLNWYQQKPGKAPKLLIYDAS
NLETGVPSRFSGSGSGTDFTFTISSLQPEDIATYYCQQYGNLPFTFGGGT
KVEIKRAAAIEVMYPPPYLDNEKSNGTIIHVKGKHLCPSPLFPGPSKPF
WVLVVVGGVLACYSLLVTVAFIIFWVRSKRSRLLHSDYMNMTPRRPG
PTRKHYQPYAPPRDFAAYRSRVKFSRSADAPAYQQGQNQLYNELNLG
RREEYDVLDKRRGRDPEMGGKPRRKNPQEGLYNELQKDKMAEAYSE
IGMKGERRRGKGHDGLYQGLSTATKDTYDALHMQALPPR(SEQ ID
NO. 66)
Clone 24C8 CD8 CD3 zeta CAR DNA Heavy & Light Chains 
ATGGCACTCCCCGTAACTGCTCTGCTGCTGCCGTTGGCATTGCTCCT
GCACGCCGCACGCCCGCAGGTGCAGTTGCAGGAAAGCGGGCCTGG
CCTTGTGAAACCAAGCCAGACACTGAGCCTGACATGCACTGTGTCC
GGCGGGTCCATATCTTCCGGGGGTTTTATTGGTCCTGGATACGCC
AGCATCCCGGGAAAGGACTTGAATGGATTGGATATATCCACCATTC
CGGAAGCACCCACTACAATCCAAGCCTTAAATCCCGGGTGACAATC
TCCATCGACACCTCAAAGAATCTTTTTCCCTGCGGTTGTCTTCAGT
AACTGCCGCCGATACCGCTGTGTACTACTGTGCCAGCCTCGTCTAT
TGCGGCGGAGATTGTTATTCTGGGTTCGATTATTGGGGTCAAGGCA
CACTGGTAACTGTCAGCAGCGGAGGCGGCGGTTCCGGGGGCGGGG
 
GCAGTGGAGGGGGCGGATCTGACATTCAGCTTACGCAGTCCCCATC
TTCACTTAGCGCCAGCGTTGGCGATCGGGTCAGCTTCACGTGTCAA
GCAAGTCAGGATATCAACAACTTTCTTAACTGGTACCAGCAGAAGC
CAGGCAAGGCACCCAAGTTGCTGATTTACGATGCTTCTAACCTCGA
GACGGGAGTGCCTAGCCGCTTCTCCGGGAGCGGCAGCGGCACAGA
CTTTACCTTTACGATTTCCAGTCTGCAGCCAGAGGATATAGCAACT
TATTACTGTCAGCAGTATGGCAACCTCCCTTTTACCTTCGGTGGTGG
CACAAAGGTCGAGATTAAAAGAGCCGCAGCGTTGTCCAACTCCAT
AATGTATTTTTCTCATTTTGTGCCCGTCTTTCTGCCTGCCAAACCTA
CCACCACCCCCGCCCCACGACCACCTACTCCAGCCCCCACCATCGC
CTCCCAGCCCCTCAGCCTGAGGCCAGAGGCTTGTCGCCCTGCTGCG
GGGGGCGCTGTCCATACCAGAGGACTCGACTTCGCCTGCGATATTT
ATATATGGGCCCCCCTCGCCGGCACCTGCGGAGTCTTGCTCCTGAG
CCTTGTGATCACGCTTTATTGTAACCATCGGAATAGATCCAAAAGA
AGCCGCCTGCTCCATAGCGATTACATGAATATGACTCCACGCCGCC
CTGGCCCCACAAGGAAACACTACCAGCCTTACGCACCACCTAGAG
ATTTCGCTGCCTATCGGAGCAGGGTGAAGTTTCCAGATCTGCAGA
TGCACCAGCGTATCAGCAGGGCCAGAACCAACTGTATAACGAGCT
CAACCTGGGACGCAGGGAAGAGTATGACGTTTTGGACAAGCGCAG
AGGACGGGACCCTGAGATGGGTGGCAAACCAAGACGAAAAAACCC
CCAGGAGGGTCTCTATAATGAGCTGCAGAAGGATAAGATGGCTGA
AGCCTATTCTGAAATAGGCATGAAAGGAGAGCGGAGAAGGGGAAA
AGGGCACGACGGTTTGTACCAGGGACTCAGCACTGCTACGAAGGA
TACTTATGACGCTCTCCACATGCAAGCCCTGCCACCTAGGTAA
 (SEQ ID NO. 67)
Clone 24C8 CD8 CD3 zeta CAR AA Heavy & Light Chains
   (Signal Peptide in **Bold**) 
MALPVTALLLPLALLLHAARPQVQLQESGPGLVKPSQTLSLTCTVSG
GSISSGGFYWSWIRQHPGKGLEWIGYIHHSGSTHYNPSLKSRVTISIDTS
KNLFSLRLSSVTAADTAVYYCASLVYCGGDCYSGFDYWGQGTLVTV
SSGGGGSGGGGSGGGGSDIQLTQSPSSLSASVGDRVSFTCQASQDINNF
LNWYQQKPGKAPKLLIYDASNLETGVPRFSGSGSGTDFTFTISSLQPED
IATYYCQYGNLPFTFGGGTKVEIKRAAALSNSIMYFSHFVPVFLPAKPT
 
TTPAPRPPTPAPTIASQPLSLRPEACRPAAGGAVHTRGLDFACDIYIWA
PLAGTCGVLLLSLVITLYCNHRNRSKRSRLLHSDYMNMTPRRPGPTRK
HYQPYAPPRDFAAYRSRVKFSRSADAPAYQQGQNQLYNELNLGRREE
YDVLDKRRGRDPEMGGKPRRKNPQEGLYNELQKDKMAEAYSEIGMK
GERRRGKGHDGLYQGLSTATKDTYDALHMQALPPR (SEQ ID NO. 68)
Clone 24C8 CD8 CD3 zeta CAR DNA Heavy & Light Chains 
CAGGTGCAGTTGCAGGAAAGCGGGCCTGGCCTTGTGAAACCAAGC
CAGACACTGAGCCTGACATGCACTGTGTCCGGCGGGTCCATATCTT
CCGGGGGTTTTATTGGTCCTGGATACGCCAGCATCCCGGGAAAGG
ACTTGAATGGATTGGATATATCCACCATTCCGGAAGCACCCACTAC
AATCCAAGCCTTAAATCCCGGGTGACAATCTCCATCGACACCTCAA
AGAATCTTTTTCCCTGCGGTTGTCTTCAGTAACTGCCGCCGATACC
GCTGTGTACTACTGTGCCAGCCTCGTCTATTGCGGCGGAGATTGTT
ATTCTGGGTTCGATTATTGGGGTCAAGGCACACTGGTAACTGTCAG
CAGCGGAGGCGGCGGTTCCGGGGGCGGGGGCAGTGGAGGGGGCG
GATCTGACATTCAGCTTACGCAGTCCCCATCTTCACTTAGCGCCAG
CGTTGGCGATCGGGTCAGCTTCACGTGTCAAGCAAGTCAGGATATC
AACAACTTTCTTAACTGGTACCAGCAGAAGCCAGGCAAGGCACCC
AAGTTGCTGATTTACGATGCTTCTAACCTCGAGACGGGAGTGCCTA
GCCGCTTCTCCGGGAGCGGCAGCGGCACAGACTTTACCTTTACGAT
TTCCAGTCTGCAGCCAGAGGATATAGCAACTTATTACTGTCAGCAG
TATGGCAACCTCCCTTTTACCTTCGGTGGTGGCACAAAGGTCGAGA
TTAAAAGAGCCGCAGCGTTGTCCAACTCCATAATGTATTTTTCTCAT
TTTGTGCCCGTCTTTCTGCCTGCCAAACCTACCACCACCCCCGCCCC
ACGACCACCTACTCCAGCCCCCACCATCGCCTCCCAGCCCCTCAGC
CTGAGGCCAGAGGCTTGTCGCCCTGCTGCGGGGGGCGCTGTCCATA
CCAGAGGACTCGACTTCGCCTGCGATATTTATATATGGGCCCCCCT
CGCCGGCACCTGCGGAGTCTTGCTCCTGAGCCTTGTGATCACGCTT
TATTGTAACCATCGGAATAGATCCAAAAGAAGCCGCCTGCTCCATA
GCGATTACATGAATATGACTCCACGCCGCCCTGGCCCCACAAGGAA
ACACTACCAGCCTTACGCACCACCTAGAGATTTCGCTGCCTATCGG
AGCAGGGTGAAGTTTTCCAGATCTGCAGATGCACCAGCGTATCAGC
AGGGCCAGAACCAACTGTATAACGAGCTCAACCTGGGACGCAGGG
 
AAGAGTATGACGTTTTGGACAAGCGCAGAGGACGGACCCTGAGA
TGGGTGGCAAACCAAGACGAAAAACCCCCAGGAGGGTCTCTATA
ATGAGCTGCAGAAGGATAAGATGGCTGAAGCCTATTCTGAAATAG
GCATGAAAGGAGAGCGGAGAAGGGGAAAAGGGCACGACGGTTTG
TACCAGGGACTCAGCACTGCTACGAAGGATACTTATGACGCTCTCC
ACATGCAAGCCCTGCCACCTAGG (SEQ ID NO. 69)
Clone 24C8 CD8 CD3 zeta CAR AA Heavy & Light Chains 
QVQLQESGPGLVKPSQTLSLTCTVSGGSISSGGFYWSWIRQHPGKGLE
WIGYIHHSGSTHYNPSLKSRVTISIDTSKNLFSLRLSSVTAADTAVYYC
ASLVYCGGDCYSGFDYWGQGTLVTVSSGGGGSGGGGSGGGGSDIQL
TQSPSSLSASVGDRVSFTCQASQDINNFLNWYQQKPGKAPKLLIYDAS
NLETGVPSRFSGSGSGTDFTFTISSLQPEDIATYYCQQYGNLPFTFGGGT
KVEIKRAAALSNSIMYFSHFVPVFLPAKPTTTPAPRPPTPAPTIASQPLS
LRPEACRPAAGGAVHTRGLDFACDIYIWAPLAGTCGVLLLSLVITLYC
NHRNRSKRSRLLHSDYMNMTPRRPGPTRKHYQPYAPPRDFAAYRSRV
KFSRSADAPAYQQGQNQLYNELNLGRREEYDVLDKRRGRDPEMGGK
PRRKNPQEGLYNELQKDKMAEAYSEIGMKGERRRGKGHDGLYQGLS
TA TKDTYDALHM QALPPR (SEQ ID NO. 70)
Clone 20C5.1 HC DNA 
CAGGTCCAACTGGTGCAGTCCGGAGCCGAAGTCAAGAAACCAGGT
GCCTCCGTTAAAGTGAGTTGCAAAGTCTCTGGATACACTCTGACCG
AGCTCTCTATGCACTGGGTCCGGCAGGCCCCCGGCAAGGGATTGGA
ATGGATGGGCGGGTTCGATCCTGAGGACGGAGAGACTATCTACGC
TCAAAAATTCCAGGGACGAGTGACTGTGACCGAAGACACTAGTAC
CGACACTGCCTACATGGAACTTTCCTCTCTGCGATCAGAAGATACC
GCAGTGTACTACTGTGCTACTGAATCTAGGGGCATTGGATGGCCCT
ACTTCGATTACTGGGGTCAGGGAACTCTGGTGACTGTCTCCAGC
 (SEQ ID NO. 71)
Clone 20C5.1 AA HC (CDRs in Underline) 
QVQLVQSGAEVKKPGASVKVSCKVSGYTLTELSMHWVRQAPGKGLE
WMGGFDPEDGETIYAQKFQGRVTVTEDTSTDTAYMELSSLRSEDTAV
YYCATESRGIGWPYFDYWGQGTLVTVSS (SEQ ID NO. 72)
Clone 20C5.1 HC AA CDR1: GYTLTEL (SEQ ID NO. 73)
Clone 20C5.1 HC AA CDR2: DPEDGE (SEQ ID NO. 74)
Clone 20C5.1 HC AA CDR3: ESRGIGWPYFDY (SEQ ID NO. 75)
Clone 20C5.1 LC DNA 
GATATTCAGATGACTCAATCTCCTTCTTCTCTGTCCGCTTCCGTGGG
CGATAGAGTGACCATTACTTGTAGGGCGTCCCAGTCAATCTCCAGT
TATTTGAATTGGTATCAGCAGAAGCCCGGGAAAGCACCTAAGCTGT
TGATCAGCGGGGCTTCTAGCCTGAAGAGTGGGGTACCTTCACGGTT
CAGCGGAAGCGGAAGCGGAACCGATTTCACCCTGACTATCAGCAG
CCTGCCACCTGAGGACTTTGCAACTTACTACTGCCAACAGTCATAC
AGCACTCCGATCACTTTCGGCCAGGGCACCCGGCTCGAAATCAAGC
GC (SEQ ID NO. 76)
Clone 20C5.1 AA LC (CDRs in Underline) 
DIQMTQSPSSLSASVGDRVTITCRASQSISSYLNWYQQKPGKAPKLLIS
GASSLKSGVPSRFSGSGSGTDFTLTISSLPPEDFATYYCQQSYSTPITFG
QGTRLEIKR (SEQ ID NO. 77)
Clone 20C5.1 AA LC CDR1: RASQSISSYLN (SEQ ID NO. 78)
Clone 20C5.1 AA LC CDR2: GASSLKS (SEQ ID NO. 79)
Clone 20C5.1 AA LC CDR3: QQSYSTPIT (SEQ ID NO. 80)
Clone 20C5.1 CD28T CD3 zeta CAR DNA Heavy & Light Chains 
ATGGCACTCCCCGTAACTGCTCTGCTGCTGCCGTTGGCATTGCTCCT
GCACGCCGCACGCCCGCAGGTCCAACTGGTGCAGTCCGGAGCCGA
AGTCAAGAAACCAGGTGCCTCCGTTAAAGTGAGTTGCAAAGTCTCT
GGATACACTCTGACCGAGCTCTCTATGCACTGGGTCCGGCAGGCCC
CCGGCAAGGGATTGGAATGGATGGGCGGGTTCGATCCTGAGGACG
GAGAGACTATCTACGCTCAAAAATTCCAGGGACGAGTGACTGTGA
CCGAAGACACTAGTACCGACACTGCCTACATGGAACTTTCCTCTCT
GCGATCAGAAGATACCGCAGTGTACTACTGTGCTACTGAATCTAGG
GGCATTGGATGGCCCTACTTCGATTACTGGGGTCAGGGAACTCTGG
TGACTGTCTCCAGCGGTGGAGGTGGCAGCGGTGGTGGCGGAAGCG
GGGGGGGCGGCTCTGATATTCAGATGACTCAATCTCCTTCTTCTCT
GTCCGCTTCCGTGGGCGATAGAGTGACCATTACTTGTAGGGCGTCC
 
CAGTCAATCTCCAGTTATTTGAATTGGTATCAGCAGAAGCCCGGGA
AAGCACCTAAGCTGTTGATCAGCGGGGCTTCTAGCCTGAAGAGTGG
GGTACCTTCACGGTTCAGCGGAAGCGGAAGCGGAACCGATTTCAC
CCTGACTATCAGCAGCCTGCCACCTGAGGACTTTGCAACTTACTAC
TGCCAACAGTCATACAGCACTCCGATCACTTTCGGCCAGGGCACCC
GGCTCGAAATCAAGCGCGCTGCTGCTTTGGACAATGAGAAGTCAA
ACGGCACCATCATACATGTTAAAGGTAAACATCTGTGTCCCTCCCC
GCTGTTCCCCGGCCCTTCCAAACCGTTCTGGGTTCTGGTGGTGGTCG
GAGGCGTACTCGCTTGCTATAGTCTGCTGGTAACTGTCGCCTTCATC
ATCTTTTGGGTGAGATCCAAAAGAAGCCGCCTGCTCCATAGCGATT
ACATGAATATGACTCCACGCCGCCCTGGCCCCACAAGGAAACACT
ACCAGCCTTACGCACCACCTAGAGATTTCGCTGCCTATCGGAGCAG
GGTGAAGTTTTCCAGATCTGCAGATGCACCAGCGTATCAGCAGGGC
CAGAACCAACTGTATAACGAGCTCAACCTGGGACGCAGGGAAGAG
TATGACGTTTTGGACAAGCGCAGAGGACGGGACCCTGAGATGGGT
GGCAAACCAAGACGAAAAAACCCCCAGGAGGGTCTCTATAATGAG
CTGCAGAAGGATAAGATGGCTGAAGCCTATTCTGAAATAGGCATG
AAAGGAGAGCGGAGAAGGGGAAAAGGGCACGACGGTTTGTACCA
GGGACTCAGCACTGCTACGAAGGATACTTATGACGCTCTCCACATG
CAAGCCCTGCCACCTAGGTAA (SEQ ID NO. 81)
Clone 20C5.1 CD28T CD3 zeta CAR AA Heavy & Light Chains
   (Signal Peptide in **Bold**) 
MALPVTALLLPLALLLHAARPQVQLVQSGAEVKKPGASVKVSCKVS
GYTLTELSMHWVRQAPGKGLEWMGGFDPEDGETIYAQKFQGRVTVT
EDTSTDTAYMELSSLRSEDTAVYYCATESRGIGWPYFDYWGQGTLVT
VSSGGGGSGGGGSGGGGSDIQMTQSPSSLSASVGDRVTITCRASQSISS
YLNWYQQKPGKAPKLLISGASSLKSGVPSRFSGSGSGTDFTLTISSLPPE
DFATYYCQQSYSTPITFGQGTRLEIKRAAALDNEKSNGTIIHVKGKHLC
PSPLFPGPSKPFWVLVVVGGVLACYSLLVTVAFIIFWVRSKRSRLLHSD
YMNMTPRRPGPTRKHYQPYAPPRDFAAYRSRVKFSRSADAPAYQQG
QNQLYNELNLGRREEYDVLDKRRGRDPEMGGKPRRKNPQEGLYNEL
QKDKMAEAYSEIGMKGERRRGKGHDGLYQGLSTATKDTYDALHMQ
ALPPR (SEQ ID NO. 82)
Clone 20C5.1 CD28T CD3 zeta CAR DNA Heavy & Light Chains 
CAGGTCCAACTGGTGCAGTCCGGAGCCGAAGTCAAGAAACCAGGT
GCCTCCGTTAAAGTGAGTTGCAAAGTCTCTGGATACACTCTGACCG
AGCTCTCTATGCACTGGGTCCGGCAGGCCCCCGGCAAGGGATTGGA
ATGGATGGGCGGGTTCGATCCTGAGGACGGAGAGACTATCTACGC
TCAAAAATTCCAGGGACGAGTGACTGTGACCGAAGACACTAGTAC
CGACACTGCCTACATGGAACTTTCCTCTCTGCGATCAGAAGATACC
GCAGTGTACTACTGTGCTACTGAATCTAGGGGCATTGGATGGCCCT
ACTTCGATTACTGGGGTCAGGGAACTCTGGTGACTGTCTCCAGCGG
TGGAGGTGGCAGCGGTGGTGGCGGAAGCGGGGGGGCGGCTCTGA
TATTCAGATGACTCAATCTCCTTCTTCTCTGTCCGCTTCCGTGGGCG
ATAGAGTGACCATTACTTGTAGGGCGTCCCAGTCAATCTCCAGTTA
TTTGAATTGGTATCAGCAGAAGCCCGGGAAAGCACCTAAGCTGTTG
ATCAGCGGGGCTTCTAGCCTGAAGAGTGGGGTACCTTCACGGTTCA
GCGGAAGCGGAAGCGGAACCGATTTCACCCTGACTATCAGCAGCC
TGCCACCTGAGGACTTTGCAACTTACTACTGCCAACAGTCATACAG
CACTCCGATCACTTTCGGCCAGGGCACCCGGCTCGAAATCAAGCGC
GCTGCTGCTTTGGACAATGAGAAGTCAAACGGCACCATCATACATG
TTAAAGGTAAACATCTGTGTCCCTCCCCGCTGTTCCCCGGCCCTTCC
AAACCGTTCTGGGTTCTGGTGGTGGTCGGAGGCGTACTCGCTTGCT
ATAGTCTGCTGGTAACTGTCGCCTTCATCATCTTTTGGGTGAGATCC
AAAAGAAGCCGCCTGCTCCATAGCGATTACATGAATATGACTCCAC
GCCGCCCTGGCCCCACAAGGAAACACTACCAGCCTTACGCACCACC
TAGAGATTTCGCTGCCTATCGGAGCAGGGTGAAGTTTTCCAGATCT
GCAGATGCACCAGCGTATCAGCAGGGCCAGAACCAACTGTATAAC
GAGCTCAACCTGGGACGCAGGGAAGAGTATGACGTTTTGGACAAG
CGCAGAGGACGGGACCCTGAGATGGGTGGCAAACCAAGACGAAA
AAACCCCCAGGAGGGTCTCTATAATGAGCTGCAGAAGGATAAGAT
GGCTGAAGCCTATTCTGAAATAGGCATGAAAGGAGAGCGGAGAAG
GGGAAAAGGGCACGACGGTTTGTACCAGGGACTCAGCACTGCTAC
GAAGGATACTTATGACGCTCTCCACATGCAAGCCCTGCCACCTAGG
 (SEQ ID NO. 83)
Clone 20C5.1 CD28T CD3 zeta CAR AA Heavy & Light Chains 
QVQLVQSGAEVKKPGASVKVSCKVSGYTLTELSMHWVRQAPGKGLE
WMGGFDPEDGETIYAQKFQGRVTVTEDTSTDTAYMELSSLRSEDTAV
YYCATESRGIGWPYFDYWGQGTLVTVSSGGGGSGGGGSGGGGSDIQ
MTQSPSSLSASVGDRVTITCRASQSISSYLNWYQQKPGKAPKLLISGAS
SLKSGVPSRFSGSGSGTDFTLTISSLPPEDFATYYCQQSYSTPITFGQGT
RLEIKRAAALDNEKSNGTIIHVKGKHLCPSPLFPGPSKPFWVLVVVGG
VLACYSLLVTVAFIIFWVRSKRSRLLHSDYMNMTPRRPGPTRKHYQPY
APPRDFAAYRSRVKFSRSADAPAYQQGQNQLYNELNLGRREEYDVLD
KRRGRDPEMGGKPRRKNPQEGLYNELQKDKMAEAYSEIGMKGERRR
GKGHDGLYQGLSTATKDTYDALHMQALPPR (SEQ ID NO. 84)
Clone 20C5.1 CD28 CD3 zeta CAR DNA Heavy & Light Chains 
ATGGCACTCCCCGTAACTGCTCTGCTGCTGCCGTTGGCATTGCTCCT
GCACGCCGCACGCCCGCAGGTGCAGCTTGTGCAGAGCGGGGCCGA
GGTGAAGAAGCCCGGGGCCAGCGTCAAAGTGTCCTGTAAGGTCAG
CGGTTACACCCTCACCGAGCTGAGCATGCACTGGGTACGGCAGGCT
CCCGGCAAAGGTCTTGAGTGGATGGGTGGATTTGATCCAGAAGAT
GGAGAGACTATCTACGCCCAGAAGTTCCAGGGCCGGGTCACCGTA
ACAGAAGACACCTCAACTGACACCGCTTACATGGAGCTGAGTTCAC
TGCGGTCCGAGGACACGGCCGTGTATTATTGTGCCACCGAGAGCCG
CGGAATCGGATGGCCTTACTTCGACTACTGGGGACAGGGTACACTT
GTTACAGTATCATCCGGGGGTGGCGGCTCTGGTGGGGGCGGCTCCG
GAGGGGGTGGATCAGATATCCAAATGACTCAAAGTCCAAGTTCCCT
GTCTGCCTCAGTCGGAGATAGAGTCACCATAACCTGCAGGGCAAGT
CAGTCCATCTCCTCCTATCTGAACTGGTACCAACAGAAACCTGGAA
AGGCGCCTAAGCTCCTGATCTCCGGAGCCTCATCTTTGAAATCCGG
TGTCCCATCTCGCTTCAGTGGCTCTGGAAGCGGTACAGATTTTACTT
TGACCATTAGCAGCCTCCCACCGGAAGACTTTGCTACATATTACTG
CCAGCAGTCTTACTCAACCCCAATCACCTTCGGGCAAGGCACCAGA
CTCGAAATAAAAAGAGCAGCTGCTATCGAGGTTATGTACCCACCGC
CGTACTTGGATAACGAAAAAAGCAATGGGACCATCATTCATGTGA
AGGGTAAGCACCTTTGCCCTAGCCCACTGTTTCCTGGCCCGAGTAA
ACCCTTTTGGGTACTTGTGGTCGTCGGCGGCGTGCTGGCCTGCTACT
CACTCCTGGTTACCGTCGCATTCATCATCTTTTGGGTGAGATCCAAA
 
AGAAGCCGCCTGCTCCATAGCGATTACATGAATATGACTCCACGCC
GCCCTGGCCCCACAAGGAAACACTACCAGCCTTACGCACCACCTAG
AGATTTCGCTGCCTATCGGAGCAGGGTGAAGTTTTCCAGATCTGCA
GATGCACCAGCGTATCAGCAGGGCCAGAACCAACTGTATAACGAG
CTCAACCTGGGACGCAGGGAAGAGTATGACGTTTTGGACAAGCGC
AGAGGACGGGACCCTGAGATGGGTGGCAAACCAAGACGAAAAAA
CCCCCAGGAGGGTCTCTATAATGAGCTGCAGAAGGATAAGATGGC
TGAAGCCTATTCTGAAATAGGCATGAAAGGAGAGCGGAGAAGGGG
AAAAGGGCACGACGGTTTGTACCAGGGACTCAGCACTGCTACGAA
GGATACTTATGACGCTCTCCACATGCAAGCCCTGCCACCTAGGTAA
 (SEQ ID NO. 85)
Clone 20C5.1 CD28 CD3 zeta CAR AA Heavy & Light Chains
   (Signal Peptide in Bold) 
MALPVTALLLPLALLLHAARPQVQLVQSGAEVKKPGASVKVSCKVS
GYTLTELSMHWVRQAPGKGLEWMGGFDPEDGETIYAQKFQGRVTVT
EDTSTDTAYMELSSLRSEDTAVYYCATESRGIGWPYFDYWGQGTLVT
VSSGGGGSGGGGSGGGGSDIQMTQSPSSLSASVGDRVTITCRASQSISS
YLNWYQQKPGKAPKLLISGASSLKSGVPSRFSGSGSGTDFTLTISSLPPE
DFATYYCQQSYSTPITFGQGTRLEIKRAAAIEVMYPPPYLDNEKSNGTII
HVKGKHLCPSPLFPGPSKPFWVLVVVGGVLACYSLLVTVAFIIFWVRS
KRSRLLHSDYMNMTPRRPGPTRKHYQPYAPPRDFAAYRSRVKFSRSA
DAPAYQQGQNQLYNELNLGRREEYDVLDKRRGRDPEMGGKPRRKNP
QEGLYNELQKDKMAEAYSEIGMKGERRRGKGHDGLYQGLST
ATKDTYDALH MQALPPR (SEQ ID NO. 86)
Clone 20C5.1 CD28 CD3 zeta CAR DNA Heavy & Light Chains 
CAGGTGCAGCTTGTGCAGAGCGGGGCCGAGGTGAAGAAGCCCGGG
GCCAGCGTCAAAGTGTCCTGTAAGGTCAGCGGTTACACCCTCACCG
AGCTGAGCATGCACTGGGTACGGCAGGCTCCCGGCAAAGGTCTTG
AGTGGATGGGTGGATTTGATCCAGAAGATGGAGAGACTATCTACG
CCCAGAAGTTCCAGGGCCGGGTCACCGTAACAGAAGACACCTCAA
CTGACACCGCTTACATGGAGCTGAGTTCACTGCGGTCCGAGGACAC
GGCCGTGTATTATTGTGCCACCGAGAGCCGCGGAATCGGATGGCCT
TACTTCGACTACTGGGGACAGGGTACACTTGTTACAGTATCATCCG
 
GGGGTGGCGGCTCTGGTGGGGGCGGCTCCGGAGGGGGTGGATCAG
ATATCCAAATGACTCAAAGTCCAAGTTCCCTGTCTGCCTCAGTCGG
AGATAGAGTCACCATAACCTGCAGGGCAAGTCAGTCCATCTCCTCC
TATCTGAACTGGTACCAACAGAAACCTGGAAAGGCGCCTAAGCTC
CTGATCTCCGGAGCCTCATCTTTGAAATCCGGTGTCCCATCTCGCTT
CAGTGGCTCTGGAAGCGGTACAGATTTACTTTGACCATTAGCAGC
CTCCCACCGGAAGACTTTGCTACATATTACTGCCAGCAGTCTTACT
CAACCCCAATCACCTTCGGGCAAGGCACCAGACTCGAAATAAAAA
GAGCAGCTGCTATCGAGGTTATGTACCCACCGCCGTACTTGGATAA
CGAAAAAAGCAATGGGACCATCATTCATGTGAAGGGTAAGCACCT
TTGCCCTAGCCCACTGTTTCCTGGCCCGAGTAAACCCTTTTGGGTAC
TTGTGGTCGTCGGCGGCGTGCTGGCCTGCTACTCACTCCTGGTTACC
GTCGCATTCATCATCTTTTGGGTGAGATCCAAAAGAAGCCGCCTGC
TCCATAGCGATTACATGAATATGACTCCACGCCGCCCTGGCCCCAC
AAGGAAACACTACCAGCCTTACGCACCACCTAGAGATTTCGCTGCC
TATCGGAGCAGGGTGAAGTTTTCCAGATCTGCAGATGCACCAGCGT
ATCAGCAGGGCCAGAACCAACTGTATAACGAGCTCAACCTGGGAC
GCAGGGAAGAGTATGACGTTTTGGACAAGCGCAGAGGACGGGACC
CTGAGATGGGTGGCAAACCAAGACGAAAAAACCCCCAGGAGGGTC
TCTATAATGAGCTGCAGAAGGATAAGATGGCTGAAGCCTATTCTGA
AATAGGCATGAAAGGAGAGCGGAGAAGGGGAAAAGGGCACGACG
GTTTGTACCAGGGACTCAGCACTGCTACGAAGGATACTTATGACGC
TCTCCACATGCAAGCCCTGCCACCTAGG (SEQ ID NO. 87)
Clone 20C5.1 CD28 CD3 zeta CAR AA Heavy & Light Chains 
QVQLVQSGAEVKKPGASVKVSCKVSGYTLTELSMHWVRQAPGKGLE
WMGGFDPEDGETIYAQKFQGRVTVTEDTSTDTAYMELSSLRSEDTAV
YYCATESRGIGWPYFDYWGQGTLVTVSSGGGGSGGGGSGGGGSDIQ
MTQSPSSLSASVGDRVTITCRASQSISSYLNWYQQKPGKAPKLLISGAS
SLKSGVPSRFSGSGSGTDFTLTISSLPPEDFATYYCQQSYSTPITFGQGT
RLEIKRAAAIEVMYPPPYLDNEKSNGTIIHVKGKHLCPSPLFPGPSKPF
WVLVVVGGVLACYSLLVTVAFIIFWVRSKRSRLLHSDYMNMTPRRPG
PTRKHYQPYAPPRDFAAYRSRVKFSRSADAPAYQQGQNQLYNELNLG
RREEYDVLDKRRGRDPEMGGKPRRKNPQEGLYNELQKDKMAEAYSE
 
IGMKGERRRGKGHDGLYQGLSTATKDTYDALHM QALPPR (SEQ ID
NO. 88)
Clone 20C5.1 CD8 CD3 zeta CAR DNA Heavy & Light Chains 
ATGGCACTCCCCGTAACTGCTCTGCTGCTGCCGTTGGCATTGCTCCT
GCACGCCGCACGCCCGCAGGTGCAGTTGGTGCAAAGCGGCGCAGA
AGTTAAGAAACCTGGGGCGTCAGTTAAGGTGTCTTGCAAAGTATCT
GGCTATACCCTCACTGAGCTGTCCATGCATTGGGTAAGGCAGGCTC
CTGGAAAGGGGCTCGAATGGATGGGAGGATTTGACCCTGAAGACG
GAGAGACCATCTACGCCCAGAAATTCCAGGGTAGAGTAACAGTGA
CTGAGGACACTAGCACTGACACAGCGTACATGGAGCTGAGTTCTCT
GAGAAGTGAGGACACAGCCGTTTACTACTGCGCTACCGAGTCCAG
AGGTATTGGCTGGCCATACTTCGACTATTGGGGTCAGGGCACCCTG
GTTACAGTGAGTTCAGGAGGCGGGGGCTCTGGGGGGGGCGGTTCC
GGAGGGGGGGGCTCAGATATACAGATGACGCAGAGTCCATCAAGT
CTCTCAGCCAGCGTGGGAGATCGCGTGACTATTACTTGCCGCGCCA
GCCAGAGTATTAGCTCCTATCTGAATTGGTACCAGCAAAAGCCCGG
GAAGGCCCCTAAGCTTCTGATTTCTGGCGCCTCCTCTTTGAAGTCA
GGTGTGCCAAGCAGATTTAGCGGGTCTGGAAGTGGCACTGACTTTA
CACTTACTATCTCCAGCCTGCCCCCAGAGGATTTTGCCACATATTAC
TGTCAGCAAAGCTACTCTACTCCAATCACTTTCGGCCAGGGCACAA
GATTGGAGATTAAGAGGGCTGCCGCACTTTCAAATTCCATCATGTA
TTTCAGCCATTTTGTGCCTGTTTTCTTCCGGCCAAACCTACAACCA
CTCCCGCCCCACGCCCACCTACTCCCGCCCCTACCATTGCCTCCCAG
CCTCTGTCTCTTAGACCTGAGGCTTGTAGACCTGCTGCCGGCGGAG
CCGTGCACACTCGCGGTCTGGACTTCGCCTGCGACATCTATATCTG
GGCCCCTCTGGCCGGCACCTGCGGCGTTCTCCTTCTCTCACTCGTAA
TCACACTCTATTGCAATCACAGGAACAGATCCAAAAGAAGCCGCCT
GCTCCATAGCGATTACATGAATATGACTCCACGCCGCCCTGGCCCC
ACAAGGAAACACTACCAGCCTTACGCACCACCTAGAGATTTCGCTG
CCTATCGGAGCAGGGTGAAGTTTTCCAGATCTGCAGATGCACCAGC
GTATCAGCAGGGCCAGAACCAACTGTATAACGAGCTCAACCTGGG
ACGCAGGGAAGAGTATGACGTTTTGGACAAGCGCAGAGGACGGGA
CCCTGAGATGGGTGGCAAACCAAGACGAAAAAACCCCCAGGAGGG
 
TCTCTATAATGAGCTGCAGAAGGATAAGATGGCTGAAGCCTATTCT
GAAATAGGCATGAAAGGAGAGCGGAGAAGGGGAAAAGGGCACGA
CGGTTTGTACCAGGGACTCAGCACTGCTACGAAGGATACTTATGAC
GCTCTCCACATGCAAGCCCTGCCACCTAGGTAA (SEQ ID NO. 89)
Clone 20C5.1 CD8 CD3 zeta CAR AA Heavy & Light Chains
   (Signal Peptide in **Bold**) 
MALPVTALLLPLALLLHAARPQVQLVQSGAEVKKPGASVKVSCKVS
GYTLTELSMHWVRQAPGKGLEWMGGFDPEDGETIYAQKFQGRVTVT
EDTSTDTAYMELSSLRSEDTAVYYCATESRGIGWPYFDYWGQGTLVT
VSSGGGGSGGGGSGGGGSDIQMTQSPSSLSASVGDRVTITCRASQSISS
YLNWYQQKPGKAPKLLISGASSLKSGVPSRFSGSGSGTDFTLTISSLPPE
DFATYYCQQSYSTPITFGQGTRLEIKRAAALSNSIMYFSHFVPVFLPAK
PTTTPAPRPPTPAPTIASQPLSLRPEACRPAAGGAVHTRGLDFACDIYIW
APLAGTCGVLLLSLVITLYCNHRNRSKRSRLLHSDYMNMTPRRPGPTR
KHYQPYAPPRDFAAYRSRVKFSRSADAPAYQQGQNQLYNELNLGRRE
EYDVLDKRRGRDPEMGGKPRRKNPQEGLYNELQKDKMAEAYSEIGM
KGERRRGKGHDGLYQGLSTATKDTYDALHMQALPPR (SEQ ID NO.
90)
Clone 20C5.1 CD8 CD3 zeta CAR DNA Heavy & Light Chains 
CAGGTGCAGTTGGTGCAAAGCGGCGCAGAAGTTAAGAAACCTGGG
GCGTCAGTTAAGGTGTCTTGCAAAGTATCTGGCTATACCCTCACTG
AGCTGTCCATGCATTGGGTAAGGCAGGCTCCTGGAAAGGGGCTCG
AATGGATGGGAGGATTTGACCCTGAAGACGGAGAGACCATCTACG
CCCAGAAATTCCAGGGTAGAGTAACAGTGACTGAGGACACTAGCA
CTGACACAGCGTACATGGAGCTGAGTTCTCTGAGAAGTGAGGACA
CAGCCGTTTACTACTGCGCTACCGAGTCCAGAGGTATTGGCTGGCC
ATACTTCGACTATTGGGGTCAGGGCACCCTGGTTACAGTGAGTTCA
GGAGGCGGGGGCTCTGGGGGGGGCGGTTCCGGAGGGGGGGCTCA
GATATACAGATGACGCAGAGTCCATCAAGTCTCTCAGCCAGCGTGG
GAGATCGCGTGACTATTACTTGCCGCGCCAGCCAGAGTATTAGCTC
CTATCTGAATTGGTACCAGCAAAAGCCCGGGAAGGCCCCTAAGCTT
CTGATTTCTGGCGCCTCCTCTTTGAAGTCAGGTGTGCCAAGCAGAT
TTAGCGGGTCTGGAAGTGGCACTGACTTTACACTTACTATCTCCAG
 
CCTGCCCCCAGAGGATTTTGCCACATATTACTGTCAGCAAAGCTAC
TCTACTCCAATCACTTTCGGCCAGGGCACAAGATTGGAGATTAAGA
GGGCTGCCGCACTTTCAAATTCCATCATGTATTTCAGCCATTTTGTG
CCTGTTTTTCTTCCGGCCAAACCTACAACCACTCCCGCCCCACGCCC
ACCTACTCCCGCCCCTACCATTGCCTCCCAGCCTCTGTCTCTTAGAC
CTGAGGCTTGTAGACCTGCTGCCGGCGGAGCCGTGCACACTCGCGG
TCTGGACTTCGCCTGCGACATCTATATCTGGGCCCCTCTGGCCGGC
ACCTGCGGCGTTCTCCTTCTCTCACTCGTAATCACACTCTATTGCAA
TCACAGGAACAGATCCAAAAGAAGCCGCCTGCTCCATAGCGATTA
CATGAATATGACTCCACGCCGCCCTGGCCCCACAAGGAAACACTAC
CAGCCTTACGCACCACCTAGAGATTTCGCTGCCTATCGGAGCAGGG
TGAAGTTTTCCAGATCTGCAGATGCACCAGCGTATCAGCAGGGCCA
GAACCAACTGTATAACGAGCTCAACCTGGGACGCAGGGAAGAGTA
TGACGTTTTGGACAAGCGCAGAGGACGGGACCCTGAGATGGGTGG
CAAACCAAGACGAAAAAACCCCCAGGAGGGTCTCTATAATGAGCT
GCAGAAGGATAAGATGGCTGAAGCCTATTCTGAAATAGGCATGAA
AGGAGAGCGGAGAAGGGGAAAAGGGCACGACGGTTTGTACCAGG
GACTCAGCACTGCTACGAAGGATACTTATGACGCTCTCCACATGCA
AGCCCTGCCACCTAGG (SEQ ID NO. 91)
Clone 20C5.1 CD8 CD3 zeta CAR AA Heavy & Light Chains 
QVQLVQSGAEVKKPGASVKVSCKVSGYTLTELSMHWVRQAPGKGLE
WMGGFDPEDGETIYAQKFQGRVTVTEDTSTDTAYMELSSLRSEDTAV
YYCATESRGIGWPYFDYWGQGTLVTVSSGGGGSGGGGSGGGGSDIQ
MTQSPSSLSASVGDRVTITCRASQSISSYLNWYQQKPGKAPKLLISGAS
SLKSGVPSRFSGSGSGTDFTLTISSLPPEDFATYYCQQSYSTPITFGQGT
RLEIKRAAALSNSIMYFSHFVPVFLPAKPTTTPAPRPPTPAPTIASQPLSL
RPEACRPAAGGAVHTRGLDFACDIYIWAPLAGTCGVLLLSLVITLYCN
HRNRSKRSRLLHSDYMNMTPRRPGPTRKHYQPYAPPRDFAAYRSRVK
FSRSADAPAYQQGQNQLYNELNLGRREEYDVLDKRRGRDPEMGGKP
RRKNPQEGLYNELQKDKMAEAYSEIGMKGERRRGKGHDGLYQGLST
ATKDTYDALHMQALPPR (SEQ ID NO. 92)
Clone 20C5.2 HC DNA 
CAGGTCCAGTTGGTCGAAAGTGGCGGTGGTGTAGTGCAGCCGGGC
CGCAGTTTGAGGCTTTCCTGTGCGGCTTCAGGCTTTACTTTTCCAG
CTATGGAATGCACTGGGTGCGGCAGGCCCCCGGCAAAGGACTTGA
GTGGGTGGCCGTCATTTCTTATGACGGATCAGATAAGTACTACGTG
GACAGCGTCAAGGGCAGATTCACCATCTCTAGGGACAACAGTAAA
AATAGACTCTACCTCCAGATGAATAGCCTCAGAGCTGAAGACACG
GCCGTCTACTATTGTGCTCGGGAGCGGTATAGTGGCAGAGACTACT
GGGGGCAGGGCACACTCGTTACAGTGAGTAGC (SEQ ID NO. 93)
Clone 20C5.2 AA HC (CDRs in Underline) 
QVQLVESGGGVVQPGRSLRLSCAASGFTFSSYGMHWVRQAPGKGLE
WVAVISYDGSDKYYVDSVKGRFTISRDNSKNRLYLQMNSLRAEDTAV
YYCARERYSGRDYWGQGTLVTVSS (SEQ ID NO. 94)
Clone 20C5.2 HC AA CDR1: GFTFSSY (SEQ ID NO. 95)
Clone 20C5.2 HC AA CDR2: SYDGSD (SEQ ID NO. 96)
Clone 20C5.2 HC AA CDR3: ERYSGRDY (SEQ ID NO. 97)
Clone 20C5.2 LC DNA 
GAGATTGTTATGACCCAGAGTCCTGCGACCCTCTCAGTCAGCCCCG
GGGAGCGCGCAACTTTGTCTTGCAGAGCTAGTCAGTCCGTGTCCTC
TCTTCTGACATGGTACCAGCAAAAGCCCGGGCAGGCTCCGCGCCTT
TTGATCTTTGGGGCTTCAACAAGAGCCACTGGGATTCCCGCACGAT
TCTCTGGCTCCGGGAGCGGTACTGGTTTCACCCTGACGATTAGCAG
TCTCCAGAGCGAGGACTTCGCCGTATACTACTGCCAGCAGTACGAT
ACGTGGCCATTCACTTTTGGACCAGGGACTAAAGTGGATTTTAAGC
GC (SEQ ID NO. 98)
Clone 20C5.2 AA LC (CDRs in Underline) 
EIVMTQSPATLSVSPGERATLSCRASQSVSSLLTWYQQKPGQAPRLLIF
GASTRATGIPARFSGSGSGTGFTLTISSLQSEDFAVYYCQQYDTWPFTF
GPGTKVDFKR (SEQ ID NO. 99)
Clone 20C5.2 AA LC CDR1: RASQSVSSLLT (SEQ ID NO. 100)
Clone 20C5.2 AA LC CDR2: GASTRAT (SEQ ID NO. 101)
Clone 20C5.2 AA LC CDR3: QQYDTWPFT (SEQ ID NO. 102)
Clone 20C5.2 CD28T CD3 zeta CAR DNA Heavy & Light Chains 
ATGGCACTCCCCGTAACTGCTCTGCTGCTGCCGTTGGCATTGCTCCT
GCACGCCGCACGCCCGCAGGTCCAGTTGGTCGAAAGTGGCGGTGG
TGTAGTGCAGCCGGGCCGCAGTTTGAGGCTTTCCTGTGCGGCTTCA
GGCTTTACTTTTTCCAGCTATGGAATGCACTGGGTGCGGCAGGCCC
CCGGCAAAGGACTTGAGTGGGTGGCCGTCATTTCTTATGACGGATC
AGATAAGTACTACGTGGACAGCGTCAAGGGCAGATTCACCATCTCT
AGGGACAACAGTAAAAATAGACTCTACCTCCAGATGAATAGCCTC
AGAGCTGAAGACACGGCCGTCTACTATTGTGCTCGGGAGCGGTATA
GTGGCAGAGACTACTGGGGGCAGGGCACACTCGTTACAGTGAGTA
GCGGCGGAGGAGGGAGTGGGGGCGGTGGCTCCGGTGGAGGAGGTT
CTGAGATTGTTATGACCCAGAGTCCTGCGACCCTCTCAGTCAGCCC
CGGGGAGCGCGCAACTTTGTCTTGCAGAGCTAGTCAGTCCGTGTCC
TCTCTTCTGACATGGTACCAGCAAAAGCCCGGGCAGGCTCCGCGCC
TTTTGATCTTTGGGGCTTCAACAAGAGCCACTGGATTCCCGCACG
ATTCTCTGGCTCCGGGAGCGGTACTGGTTTCACCCTGACGATTAGC
AGTCTCCAGAGCGAGGACTTCGCCGTATACTACTGCCAGCAGTACG
ATACGTGGCCATTCACTTTTGGACCAGGGACTAAAGTGGATTTTAA
GCGCGCCGCCGCTCTCGATAACGAAAAGTCAAATGGCACCATAAT
CCACGTCAAAGGCAAGCACCTGTGCCCTTCCCCGCTCTTCCCCGGA
CCCAGTAAACCATTTTGGGTGCTGGTTGTTGTGGGGGGCGTGCTGG
CCTGCTATAGCCTTTTGGTCACTGTAGCCTTCATTATTTTTTGGGTC
AGATCCAAAAGAAGCCGCCTGCTCCATAGCGATTACATGAATATG
ACTCCACGCCGCCCTGGCCCCACAAGGAAACACTACCAGCCTTACG
CACCACCTAGAGATTTCGCTGCCTATCGGAGCAGGGTGAAGTTTTC
CAGATCTGCAGATGCACCAGCGTATCAGCAGGGCCAGAACCAACT
GTATAACGAGCTCAACCTGGGACGCAGGGAAGAGTATGACGTTTT
GGACAAGCGCAGAGGACGGGACCCTGAGATGGGTGGCAAACCAA
GACGAAAAAACCCCCAGGAGGGTCTCTATAATGAGCTGCAGAAGG
ATAAGATGGCTGAAGCCTATTCTGAAATAGGCATGAAAGGAGAGC
GGAGAAGGGGAAAAGGGCACGACGGTTTGTACCAGGGACTCAGCA
CTGCTACGAAGGATACTTATGACGCTCTCCACATGCAAGCCCTGCC
ACCTAGGTAA (SEQ ID NO. 103)
Clone 20C5.2 CD28T CD3 zeta CAR AA Heavy & Light Chains
   (Signal Peptide in Bold) 
MALPVTALLLPLALLLHAARPQVQLVESGGGVVQPGRSLRLSCAAS
GFTFSSYGMHWVRQAPGKGLEWVAVISYDGSDKYYVDSVKGRFTISR
DNSKNRLYLQMNSLRAEDTAVYYCARERYSGRDYWGQGTLVTVSSG
GGGSGGGGSGGGGSEIVMTQSPATLSVSPGERATLSCRASQSVSSLLT
WYQQKPGQAPRLLIFGASTRATGIPARFSGSGSGTGFTLTISSLQSEDFA
VYYCQQYDTWPFTFGPGTKVDFKRAAALDNEKSNGTIIHVKGKHLCP
SPLFPGPSKPFWVLVVVGGVLACYSLLVTVAFIIFWVRSKRSRLLHSD
YMNMTPRRPGPTRKHYQPYAPPRDFAAYRSRVKFSRSADAPAYQQG
QNQLYNELNLGRREEYDVLDKRRGRDPEMGGKPRRKNPQEGLYNEL
QKDKMAEAYSEIGMKGERRRGKGHDGLYQGLSTATK
DTYDALHMQALPPR (SEQ ID NO. 104)
Clone 20C5.2 CD28T CD3 zeta CAR DNA Heavy & Light Chains 
CAGGTCCAGTTGGTCGAAAGTGGCGGTGGTGTAGTGCAGCCGGGC
CGCAGTTTGAGGCTTTCCTGTGCGGCTTCAGGCTTTACTTTTCCAG
CTATGGAATGCACTGGGTGCGGCAGGCCCCCGGCAAAGGACTTGA
GTGGGTGGCCGTCATTTCTTATGACGGATCAGATAAGTACTACGTG
GACAGCGTCAAGGGCAGATTCACCATCTCTAGGGACAACAGTAAA
AATAGACTCTACCTCCAGATGAATAGCCTCAGAGCTGAAGACACG
GCCGTCTACTATTGTGCTCGGGAGCGGTATAGTGGCAGAGACTACT
GGGGGCAGGGCACACTCGTTACAGTGAGTAGCGGCGGAGGAGGGA
GTGGGGGCGGTGGCTCCGGTGGAGGAGGTTCTGAGATTGTTATGAC
CCAGAGTCCTGCGACCCTCTCAGTCAGCCCCGGGGAGCGCGCAACT
TTGTCTTGCAGAGCTAGTCAGTCCGTGTCCTCTCTTCTGACATGGTA
CCAGCAAAAGCCCGGGCAGGCTCCGCGCCTTTTGATCTTTGGGGCT
TCAACAAGAGCCACTGGGATTCCCGCACGATTCTCTGGCTCCGGGA
GCGGTACTGGTTTCACCCTGACGATTAGCAGTCTCCAGAGCGAGGA
CTTCGCCGTATACTACTGCCAGCAGTACGATACGTGGCCATTCACT
TTTGGACCAGGGACTAAAGTGGATTTAAGCGCGCCGCCGCTCTCG
ATAACGAAAAGTCAAATGGCACCATAATCCACGTCAAAGGCAAGC
ACCTGTGCCCTTCCCCGCTCTTCCCCGGACCCAGTAAACCATTTGG
GTGCTGGTTGTTGTGGGGGGCGTGCTGGCCTGCTATAGCCTTTTGGT
 
CACTGTAGCCTTCATTATTTTTGGGTCAGATCCAAAAGAAGCCGC
CTGCTCCATAGCGATTACATGAATATGACTCCACGCCGCCCTGGCC
CCACAAGGAAACACTACCAGCCTTACGCACCACCTAGAGATTTCGC
TGCCTATCGGAGCAGGGTGAAGTTTCCAGATCTGCAGATGCACCA
GCGTATCAGCAGGGCCAGAACCAACTGTATAACGAGCTCAACCTG
GGACGCAGGGAAGAGTATGACGTTTTGGACAAGCGCAGAGGACGG
GACCCTGAGATGGGTGGCAAACCAAGACGAAAAAACCCCCAGGAG
GGTCTCTATAATGAGCTGCAGAAGGATAAGATGGCTGAAGCCTATT
CTGAAATAGGCATGAAAGGAGAGCGGAGAAGGGGAAAAGGGCAC
GACGGTTTGTACCAGGGACTCAGCACTGCTACGAAGGATACTTATG
ACGCTCTCCACATGCAAGCCCTGCCACCTAGG (SEQ ID NO. 105)
Clone 20C5.2 CD28T CD3 zeta CAR AA Heavy & Light Chains 
QVQLVESGGGVVQPGRSLRLSCAASGFTFSSYGMHWVRQAPGKGLE
WVAVISYDGSDKYYVDSVKGRFTISRDNSKNRLYLQMNSLRAEDTAV
YYCARERYSGRDYWGQGTLVTVSSGGGGSGGGGSGGGGSEIVMTQS
PATLSVSPGERATLSCRASQSVSSLLTWYQQKPGQAPRLLIFGASTRAT
GIPARFSGSGSGTGFTLTISSLQSEDFAVYYCQQYDTWPFTFGPGTKVD
FKRAAALDNEKSNGTIIHVKGKHLCPSPLFPGPSKPFWVLVVVGGVLA
CYSLLVTVAFIIFWVRSKRSRLLHSDYMNMTPRRPGPTRKHYQPYAPP
RDFAAYRSRVKFSRSADAPAYQQGQNQLYNELNLGRREEYDVLDKR
RGRDPEMGGKPRRKNPQEGLYNELQKDKMAEAYSEIGMKGERRRGK
GHDGLYQGLSTATKDTYDALHMQALPPR (SEQ ID NO. 106)
Clone 20C5.2 CD28 CD3 zeta CAR DNA Heavy & Light Chains 
ATGGCACTCCCCGTAACTGCTCTGCTGCTGCCGTTGGCATTGCTCCT
GCACGCCGCACGCCCGCAGGTGCAGCTCGTGGAGTCTGGCGGCGG
CGTGGTCCAGCCCGGCCGGTCCCTGCGCCTGTCCTGCGCCGCCAGC
GGGTTTACTTTTTCCTCCTACGGCATGCACTGGGTGCGCCAGGCTCC
CGGCAAGGGCCTCGAGTGGGTCGCCGTGATCTCATACGATGGGTCA
GACAAATACTATGTCGATTCTGTTAAAGGCGGTTTACCATTTCAA
GAGATAACTCTAAGAATAGGCTGTATTTGCAGATGAACAGCCTGA
GGGCTGAAGATACCGCAGTGTACTATTGCGCTAGGGAGCGGTATA
GTGGCCGCGATTACTGGGGACAGGGTACACTGGTGACCGTGAGCT
CTGGGGGTGGCGGAAGCGGGGGTGGCGGAAGCGGCGGAGGGGGT
 
AGTGAAATTGTGATGACCCAGTCTCCGGCTACACTTTCAGTCTCCC
CTGGGGAGAGAGCTACACTGTCATGCAGAGCGTCCCAGTCCGTCTC
TTCTCTCCTTACCTGGTATCAGCAGAAGCCCGGCCAGGCTCCTCGA
CTGCTGATCTTCGGTGCCTCCACAAGGGCGACCGGGATTCCAGCCC
GCTTCTCAGGTTCTGGGAGCGGAACTGGTTTCACTTTGACAATCAG
TTCACTGCAGTCAGAGGATTTCGCCGTGTACTACTGCCAGCAATAC
GACACATGGCCATTCACTTTCGGACCCGGTACCAAAGTCGATTTCA
AGAGAGCCGCGGCCATCGAGGTTATGTACCCACCACCATATCTGGA
CAATGAAAAAGCAATGGAACCATTATCCATGTGAAGGGTAAACA
CCTCTGCCCTAGCCACTTTTCCCTGGCCCATCAAAGCCCTTCTGGG
TCTTGGTGGTCGTGGGGGGTGTGCTGGCCTGTTACAGCCTTCTGGT
GACGGTTGCTTTCATTATCTTCTGGGTTAGATCCAAAAGAAGCCGC
CTGCTCCATAGCGATTACATGAATATGACTCCACGCCGCCCTGGCC
CCACAAGGAAACACTACCAGCCTTACGCACCACCTAGAGATTTCGC
TGCCTATCGGAGCAGGGTGAAGTTTTCCAGATCTGCAGATGCACCA
GCGTATCAGCAGGGCCAGAACCAACTGTATAACGAGCTCAACCTG
GGACGCAGGGAAGAGTATGACGTTTTGGACAAGCGCAGAGGACGG
GACCCTGAGATGGGTGGCAAACCAAGACGAAAAAACCCCCAGGAG
GGTCTCTATAATGAGCTGCAGAAGGATAAGATGGCTGAAGCCTATT
CTGAAATAGGCATGAAAGGAGAGCGGAGAAGGGGAAAAGGCAC
GACGGTTTGTACCAGGGACTCAGCACTGCTACGAAGGATACTTATG
ACGCTCTCCACATGCAAGCCCTGCCACCTAGGTAA (SEQ ID NO.
107)
Clone 20C5.2 CD28 CD3 zeta CAR AA Heavy & Light Chains
   (Signal Peptide in Bold) 
MALPVTALLLPLALLLHAARPQVQLVESGGGVVQPGRSLRLSCAAS
GFTFSSYGMHWVRQAPGKGLEWVAVISYDGSDKYYVDSVKGRFTISR
DNSKNRLYLQMNSLRAEDTAVYYCARERYSGRDYWGQGTLVTVSSG
GGGSGGGGSGGGGSEIVMTQSPATLSVSPGERATLSCRASQSVSSLLT
WYQQKPGQAPRLLIFGASTRATGIPARFSGSGSGTGFTLTISSLQSEDFA
VYYCQQYDTWPFTFGPGTKVDFKRAAAIEVMYPPPYLDNEKSNGTIIH
VKGKHLCPSPLFPGPSKPFWVLVVVGGVLACYSLLVTVAFIIFWVRSK
RSRLLHSDYMNMTPRRPGPTRKHYQPYAPPRDFAAYRSRVKFSRSAD
 
APAYQQGQNQLYNELNLGRREEYDVLDKRRGRDPEMGGKPRRKNPQ
EGLYNELQKDKMAEAYSEIGMKGERRRGKGHDGLYQGLSTATKDTY
DALHMQALPPR  (SEQ ID NO. 108)
Clone 20C5.2 CD28 CD3 zeta CAR DNA Heavy & Light Chains 
CAGGTGCAGCTCGTGGAGTCTGGCGGCGGCGTGGTCCAGCCCGGC
CGGTCCCTGCGCCTGTCCTGCGCCGCCAGCGGGTTTACTTTTCCTC
CTACGGCATGCACTGGGTGCGCCAGGCTCCCGGCAAGGGCCTCGA
GTGGGTCGCCGTGATCTCATACGATGGGTCAGACAAATACTATGTC
GATTCTGTTAAAGGGCGGTTTACCATTTCAAGAGATAACTCTAAGA
ATAGGCTGTATTTGCAGATGAACAGCCTGAGGGCTGAAGATACCG
CAGTGTACTATTGCGCTAGGGAGCGGTATAGTGGCCGCGATTACTG
GGGACAGGGTACACTGGTGACCGTGAGCTCTGGGGGTGGCGGAAG
CGGGGGTGGCGGAAGCGGCGGAGGGGGTAGTGAAATTGTGATGAC
CCAGTCTCCGGCTACACTTTCAGTCTCCCCTGGGGAGAGAGCTACA
CTGTCATGCAGAGCGTCCCAGTCCGTCTCTTCTCTCCTTACCTGGTA
TCAGCAGAAGCCCGGCCAGGCTCCTCGACTGCTGATCTTCGGTGCC
TCCACAAGGGCGACCGGGATTCCAGCCCGCTTCTCAGGTTCTGGGA
GCGGAACTGGTTTCACTTTGACAATCAGTTCACTGCAGTCAGAGGA
TTTCGCCGTGTACTACTGCCAGCAATACGACACATGGCCATTCACT
TTCGGACCCGGTACCAAAGTCGATTTCAAGAGAGCCGCGGCCATCG
AGGTTATGTACCCACCACCATATCTGGACAATGAAAAAGCAATG
GAACCATTATCCATGTGAAGGGTAAACACCTCTGCCCTAGCCCACT
TTTCCCTGGCCCATCAAAGCCCTTCTGGGTCTTGGTGGTCGTGGGG
GGTGTGCTGGCCTGTTACAGCCTTCTGGTGACGGTTGCTTTCATTAT
CTTCTGGGTTAGATCCAAAAGAAGCCGCCTGCTCCATAGCGATTAC
ATGAATATGACTCCACGCCGCCCTGGCCCCACAAGGAAACACTACC
AGCCTTACGCACCACCTAGAGATTTCGCTGCCTATCGGAGCAGGGT
GAAGTTTTCCAGATCTGCAGATGCACCAGCGTATCAGCAGGGCCAG
AACCAACTGTATAACGAGCTCAACCTGGGACGCAGGGAAGAGTAT
GACGTTTTGGACAAGCGCAGAGGACGGGACCCTGAGATGGGTGGC
AAACCAAGACGAAAAAACCCCCAGGAGGGTCTCTATAATGAGCTG
CAGAAGGATAAGATGGCTGAAGCCTATTCTGAAATAGGCATGAAA
GGAGAGCGGAGAAGGGGAAAAGGGCACGACGGTTTGTACCAGGG
 
ACTCAGCACTGCTACGAAGGATACTTATGACGCTCTCCACATGCAA
GCCCTGCCACCTAGG (SEQ ID NO. 109)
Clone 20C5.2 CD28 CD3 zeta CAR AA Heavy & Light Chains 
QVQLVESGGGVVQPGRSLRLSCAASGFTFSSYGMHWVRQAPGKGLE
WVAVISYDGSDKYYVDSVKGRFTISRDNSKNRLYLQMNSLRAEDTAV
YYCARERYSGRDYWGQGTLVTVSSGGGGSGGGGSGGGGSEIVMTQS
PATLSVSPGERATLSCRASQSVSSLLTWYQQKPGQAPRLLIFGASTRAT
GIPARFSGSGSGTGFTLTISSLQSEDFAVYYCQQYDTWPFTFGPGTKVD
FKRAAAIEVMYPPPYLDNEKSNGTIIHVKGKHLCPSPLFPGPSKPFWVL
VVVGGVLACYSLLVTVAFIIFWVRSKRSRLLHSDYMNMTPRRPGPTR
KHYQPYAPPRDFAAYRSRVKFSRSADAPAYQQGQNQLYNELNLGRRE
EYDVLDKRRGRDPEMGGKPRRKNPQEGLYNELQKDKMAEAYSEIGM
KGERRRGKGHDGLYQGLSTATKDTYDALHMQALPPR (SEQ ID NO.
110)
Clone 20C5.2 CD8 CD3 zeta CAR DNA Heavy & Light Chains 
ATGGCACTCCCCGTAACTGCTCTGCTGCTGCCGTTGGCATTGCTCCT
GCACGCCGCACGCCCGCAGGTGCAGTTGGTTGAATCAGGAGGGG
TGTGGTGCAACCCGGTCGGTCACTGCGCCTCAGTTGTGCTGCTTCC
GGGTTTACTTTCAGCTCATATGGGATGCACTGGGTACGGCAGGCTC
CAGGTAAAGGCTTGGAATGGGTGGCGGTGATCAGCTATGACGGCT
CTGACAAATATTATGTGGACTCCGTGAAAGGCAGATTCACCATCAG
TCGAGACAACTCAAAGAATAGACTCTACTTGCAGATGAATAGCCTC
CGGGCCGAAGATACTGCAGTCTATTATTGCGCCCGGGAGCGCTACA
GTGGAAGAGACTATTGGGGGCAAGGAACTCTTGTCACAGTCTCATC
TGGCGGCGGCGGCAGCGGTGGGGGCGGATCTGGCGGGGGCGGCAG
CGAAATCGTTATGACTCAGAGTCCTGCCACACTGAGCGTTAGCCCT
GGTGAGAGAGCAACACTTAGCTGCAGAGCTAGTCAGAGTGTTTCC
AGTCTTTTGACATGGTACCAACAGAAGCCCGGTCAAGCTCCACGAC
TGCTCATCTTCGGTGCATCCACCCGCGCAACCGGGATACCCGCCCG
GTTTTCCGGTTCTGGAAGTGGCACAGGATTCACGCTCACCATTTCTT
CTCTGCAGTCTGAAGACTTTGCCGTGTATTACTGCCAGCAGTACGA
TACCTGGCCCTTTACCTTTGGCCCAGGTACTAAAGTGGATTTTAAA
CGAGCTGCTGCACTTTCCAATAGTATTATGTACTTTTCACATTTTGT
 
GCCCGTGTTCCTGCCTGCGAAGCCTACGACAACCCCAGCCCCTAGG
CCGCCCACACCGGCCCCAACTATTGCCTCCCAGCCATTGTCTCTGA
GACCCGAAGCTTGCAGACCTGCTGCTGGAGGCGCCGTTCACACCCG
AGGATTGGATTTCGCATGTGACATTTACATCTGGGCCCCTTTGGCC
GGAACCTGCGGTGTGCTGCTGCTGTCACTCGTGATTACACTTTACT
GCAACCACCGAAACAGATCCAAAAGAAGCCGCCTGCTCCATAGCG
ATTACATGAATATGACTCCACGCCGCCCTGGCCCCACAAGGAAACA
CTACCAGCCTTACGCACCACCTAGAGATTTCGCTGCCTATCGGAGC
AGGGTGAAGTTTTCCAGATCTGCAGATGCACCAGCGTATCAGCAGG
GCCAGAACCAACTGTATAACGAGCTCAACCTGGGACGCAGGGAAG
AGTATGACGTTTTGGACAAGCGCAGAGGACGGGACCCTGAGATGG
GTGGCAAACCAAGACGAAAAAACCCCCAGGAGGGTCTCTATAATG
AGCTGCAGAAGGATAAGATGGCTGAAGCCTATTCTGAAATAGGCA
TGAAAGGAGAGCGGAGAAGGGGAAAAGGGCACGACGGTTTGTAC
CAGGGACTCAGCACTGCTACGAAGGATACTTATGACGCTCTCCACA
TGCAAGCCCTGCCACCTAGGTAA (SEQ ID NO. 111)
Clone 20C5.2 CD8 CD3 zeta CAR AA Heavy & Light Chains
   (Signal peptide in Bold) 
MALPVTALLLPLALLLHAARPQVQLVESGGGVVQPGRSLRLSCAAS
GFTFSSYGMHWVRQAPGKGLEWVAVISYDGSDKYYVDSVKGRFTISR
DNSKNRLYLQMNSLRAEDTAVYYCARERYSGRDYWGGTLVTVSSGG
GGSGGGGSGGGGSEIVMTQSPATLSVSPGERATLSCRASQSVSSLLTW
YQQKPGQAPRLLIFGASTRATGIPARFSGSGSGTGFTLTISSLQSEDFAV
YYCQQYDTWPFTFGPGTKVDFKRAAALSNSIMYFSHFVPVFLPAKPTT
TPAPRPPTPAPTIASQPLSLRPEACRPAAGGAVHTRGLDFACDIYIWAP
LAGTCGVLLLSLVITLYCNHRNRSKRSRLLHSDYMNMTPRRPGPTRK
HYQPYAPPRDFAAYRSRVKFSRSADAPAYQQGQNQLYNELNLGRREE
YDVLDKRRGRDPEMGGKPRRKNPQEGLYNELQKDKMAEAYSEIGMK
GERRRGKGHDGLYQGLSTATKDTYDALHMQALPPR (SEQ ID NO.
112)
Clone 20C5.2 CD8 CD3 zeta CAR DNA Heavy & Light Chains 
CAGGTGCAGTTGGTTGAATCAGGAGGGGGTGTGGTGCAACCCGGT
CGGTCACTGCGCCTCAGTTGTGCTGCTTCCGGGTTTACTTTCAGCTC
 
ATATGGGATGCACTGGGTACGGCAGGCTCCAGGTAAAGGCTTGGA
ATGGGTGGCGGTGATCAGCTATGACGGCTCTGACAAATATTATGTG
GACTCCGTGAAAGGCAGATTCACCATCAGTCGAGACAACTCAAAG
AATAGACTCTACTTGCAGATGAATAGCCTCCGGGCCGAAGATACTG
CAGTCTATTATTGCGCCCGGGAGCGCTACAGTGGAAGAGACTATTG
GGGGCAAGGAACTCTTGTCACAGTCTCATCTGGCGGCGGCGGCAG
CGGTGGGGGCGGATCTGGCGGGGGCGGCAGCGAAATCGTTATGAC
TCAGAGTCCTGCCACACTGAGCGTTAGCCCTGGTGAGAGAGCAAC
ACTTAGCTGCAGAGCTAGTCAGAGTGTTTCCAGTCTTTTGACATGG
TACCAACAGAAGCCCGGTCAAGCTCCACGACTGCTCATCTTCGGTG
CATCCACCCGCGCAACCGGGATACCCGCCCGGTTTTCCGGTTCTGG
AAGTGGCACAGGATTCACGCTCACCATTTCTTCTCTGCAGTCTGAA
GACTTTGCCGTGTATTACTGCCAGCAGTACGATACCTGGCCCTTTA
CCTTTGGCCCAGGTACTAAAGTGGATTTTAAACGAGCTGCTGCACT
TTCCAATAGTATTATGTACTTTTCACATTTTGTGCCCGTGTTCCTGC
CTGCGAAGCCTACGACAACCCCAGCCCCTAGGCCGCCCACACCGG
CCCCAACTATTGCCTCCCAGCCATTGTCTCTGAGACCCGAAGCTTG
CAGACCTGCTGCTGGAGGCGCCGTTCACACCCGAGGATTGGATTTC
GCATGTGACATTTACATCTGGGCCCCTTTGGCCGGAACCTGCGGTG
TGCTGCTGCTGTCACTCGTGATTACACTTTACTGCAACCACCGAAA
CAGATCCAAAAGAAGCCGCCTGCTCCATAGCGATTACATGAATATG
ACTCCACGCCGCCCTGGCCCCACAAGGAAACACTACCAGCCTTACG
CACCACCTAGAGATTTCGCTGCCTATCGGAGCAGGGTGAAGTTTTC
CAGATCTGCAGATGCACCAGCGTATCAGCAGGGCCAGAACCAACT
GTATAACGAGCTCAACCTGGGACGCAGGGAAGAGTATGACGTTTT
GGACAAGCGCAGAGGACGGGACCCTGAGATGGGTGGCAAACCAA
GACGAAAAAACCCCCAGGAGGGTCTCTATAATGAGCTGCAGAAGG
ATAAGATGGCTGAAGCCTATTCTGAAATAGGCATGAAAGGAGAGC
GGAGAAGGGGAAAAGGGCACGACGGTTTGTACCAGGGACTCAGCA
CTGCTACGAAGGATACTTATGACGCTCTCCACATGCAAGCCCTGCC
ACCTAGG (SEQ ID NO. 113)
Clone 20C5.2 CD8 CD3 zeta CAR AA Heavy & Light Chains 
QVQLVESGGGVVQPGRSLRLSCAASGFTFSSYGMHWVRQAPGKGLE
WVAVISYDGSDKYYVDSVKGRFTISRDNSKNRLYLQMNSLRAEDTAV
YYCARERYSGRDYWGQGTLVTVSSGGGGSGGGGSGGGGSEIVMTQS
PATLSVSPGERATLSCRASQSVSSLLTWYQQKPGQAPRLLIFGASTRAT
GIPARFSGSGSGTGFTLTISSLQSEDFAVYYCQQYDTWPFTFGPGTKVD
FKRAAALSNSIMYFSHFVPVFLPAKPTTTPAPRPPTPAPTIASQPLSLRP
EACRPAAGGAVHTRGLDFACDIYIWAPLAGTCGVLLLSLVITLYCNHR
NRSKRSRLLHSDYMNMTPRRPGPTRKHYQPYAPPRDFAAYRSRVKFS
RSADAPAYQQGQNQLYNELNLGRREEYDVLDKRRGRDPEMGGKPRR
KNPQEGLYNELQKDKMAEAYSEIGMKGERRRGKGHDGLYQGLSTAT
KDTYDALHMQALPPR (SEQ ID NO. 114)
Clone 20C5.2 CD28T CD3 zeta CAR DNA Heavy & Light Chains 
ATGGCACTCCCCGTAACTGCTCTGCTGCTGCCGTTGGCATTGCTCCT
GCACGCCGCACGCCCGGAGATTGTGATGACCCAGTCCCCTGCTACC
CTGTCCGTCAGTCCGGGCGAGAGAGCCACCTTGTCATGCCGGGCCA
GCCAGTCCGTCAGCAGTCTCCTGACTTGGTATCAGCAAAAACCAGG
GCAGGCACCGCGGCTTTTGATTTTTGGTGCAAGCACACGCGCCACT
GGCATTCCAGCTAGGTTTTCTGGAAGTGGATCTGGGACAGGCTTCA
CTCTGACAATCAGTAGCCTGCAGAGTGAGGACTTTGCTGTTTACTA
CTGTCAACAGTACGACACCTGGCCATTCACATTCGGCCCGGCACC
AAGGTCGACTTCAAGAGGGGCGGTGGAGGTTCAGGTGGTGGCGGG
TCAGGCGGCGGTGGGTCTCAGGTTCAACTGGTGGAATCAGGTGGC
GGCGTTGTCCAACCGGGGCGATCACTTCGACTTTCCTGTGCTGCCT
CAGGCTTTACTTTTTCATCCTATGGGATGCACTGGGTTCGGCAGGCT
CCCGGAAAAGGACTCGAGTGGGTTGCAGTGATCTCTTACGATGGCT
CAGACAAGTATTATGTGGACTCAGTCAAGGGGAGATTCACAATAA
GCCGAGACAACTCCAAAAACCGGCTTTATCTCCAGATGAACAGCCT
TAGAGCGGAAGATACCGCGGTATACTACTGTGCCCGCGAGAGGTA
TTCCGGCAGAGACTACTGGGGACAGGGCACACTGGTCACCGTGAG
TTCTGCCGCAGCGCTCGATAACGAAAAGAGCAACGGAACCATTAT
CCACGTTAAGGGCAAGCACCTGTGCCCCAGTCCCCTCTTCCCAGGA
CCATCTAAACCCTTCTGGGTTCTGGTAGTAGTTGGAGGGGTCCTTG
CATGTTACTCCCTTTTGGTCACCGTCGCCTTCATTATTTTCTGGGTG
 
AGATCCAAAAGAAGCCGCCTGCTCCATAGCGATTACATGAATATG
ACTCCACGCCGCCCTGGCCCCACAAGGAAACACTACCAGCCTTACG
CACCACCTAGAGATTTCGCTGCCTATCGGAGCAGGGTGAAGTTTC
CAGATCTGCAGATGCACCAGCGTATCAGCAGGGCCAGAACCAACT
GTATAACGAGCTCAACCTGGGACGCAGGGAAGAGTATGACGTTTT
GGACAAGCGCAGAGGACGGACCCTGAGATGGGTGGCAAACCAA
GACGAAAAACCCCCAGGAGGGTCTCTATAATGAGCTGCAGAAGG
ATAAGATGGCTGAAGCCTATTCTGAAATAGGCATGAAAGGAGAGC
GGAGAAGGGGAAAAGGGCACGACGGTTTGTACCAGGGACTCAGCA
CTGCTACGAAGGATACTTATGACGCTCTCCACATGCAAGCCCTGCC
ACCTAGGTAA (SEQ ID NO. 115)
Clone 20C5.2 CD28T CD3 zeta CAR AA Heavy & Light Chains
   (Signal Peptide in Bold) 
MALPVTALLLPLALLLHAARPEIVMTQSPATLSVSPGERATLSCRAS
QSVSSLLTWYQQKPGQAPRLLIFGASTRATGIPARFSGSGSGTGFTLTIS
SLQSEDFAVYYCQQYDTWPFTFGPGTKVDFKRGGGGSGGGGSGGGG
SQVQLVESGGGVVQPGRSLRLSCAASGFTFSSYGMHWVRQAPGKGLE
WVAVISYDGSDKYYVDSVKGRFTISRDNSKNRLYLQMNSLRAEDTAV
YYCARERYSGRDYWGQGTLVTVSSAAALDNEKSNGTIIHVKGKHLCP
SPLFPGPSKPFWVLVVVGGVLACYSLLVTVAFIIFWVRSKRSRLLHSD
YMNMTPRRPGPTRKHYQPYAPPRDFAAYRSRVKFSRSADAPAYQQG
QNQLYNELNLGRREEYDVLDKRRGRDPEMGGKPRRKNPQEGLYNEL
QKDKMAEAYSEIGMKGERRRGKGHDGLYQGLSTATKDTYDALHMQ
ALPPR (SEQ ID NO. 116)
Clone 20C5.2 CD28T CD3 zeta CAR DNA Heavy & Light Chains 
GAGATTGTGATGACCCAGTCCCCTGCTACCCTGTCCGTCAGTCCGG
GCGAGAGAGCCACCTTGTCATGCCGGGCCAGCCAGTCCGTCAGCA
GTCTCCTGACTTGGTATCAGCAAAAACCAGGGCAGGCACCGCGGCT
TTTGATTTTTGGTGCAAGCACACGCGCCACTGGCATTCCAGCTAGG
TTTTCTGGAAGTGGATCTGGGACAGGCTTCACTCTGACAATCAGTA
GCCTGCAGAGTGAGGACTTTGCTGTTTACTACTGTCAACAGTACGA
CACCTGGCCATTCACATTCGGGCCCGGCACCAAGGTCGACTTCAAG
AGGGGCGGTGGAGGTTCAGGTGGTGGCGGGTCAGGCGGCGGTGGG
 
TCTCAGGTTCAACTGGTGGAATCAGGTGGCGGCGTTGTCCAACCGG
GGCGATCACTTCGACTTTCCTGTGCTGCCTCAGGCTTTACTTTTTCA
TCCTATGGGATGCACTGGGTTCGGCAGGCTCCGGAAAAGGACTCG
AGTGGGTTGCAGTGATCTCTTACGATGGCTCAGACAAGTATTATGT
GGACTCAGTCAAGGGGAGATTCACAATAAGCCGAGACAACTCCAA
AAACCGGCTTTATCTCCAGATGAACAGCCTTAGAGCGGAAGATACC
GCGGTATACTACTGTGCCCGCGAGAGGTATTCCGGCAGAGACTACT
GGGGACAGGGCACACTGGTCACCGTGAGTTCTGCCGCAGCGCTCG
ATAACGAAAAGAGCAACGGAACCATTATCCACGTTAAGGGCAAGC
ACCTGTGCCCCAGTCCCCTCTTCCCAGGACCATCTAAACCCTTCTGG
GTTCTGGTAGTAGTTGGAGGGGTCCTTGCATGTTACTCCCTTTTGGT
CACCGTCGCCTTCATTATTTTCTGGGTGAGATCCAAAAGAAGCCGC
CTGCTCCATAGCGATTACATGAATATGACTCCACGCCGCCCTGGCC
CCACAAGGAAACACTACCAGCCTTACGCACCACCTAGAGATTTCGC
TGCCTATCGGAGCAGGGTGAAGTTTTCCAGATCTGCAGATGCACCA
GCGTATCAGCAGGGCCAGAACCAACTGTATAACGAGCTCAACCTG
GGACGCAGGGAAGAGTATGACGTTTTGGACAAGCGCAGAGGACGG
GACCCTGAGATGGGTGGCAAACCAAGACGAAAAAACCCCCAGGAG
GGTCTCTATAATGAGCTGCAGAAGGATAAGATGGCTGAAGCCTATT
CTGAAATAGGCATGAAAGGAGAGCGGAGAAGGGGAAAAGGCAC
GACGGTTTGTACCAGGGACTCAGCACTGCTACGAAGGATACTTATG
ACGCTCTCCACATGCAAGCCCTGCCACCTAGG (SEQ ID NO. 117)
Clone 20C5.2 CD28T CD3 zeta CAR AA Heavy & Light Chains 
EIVMTQSPATLSVSPGERATLSCRASQSVSSLLTWYQQKPGQAPRLLIF
GASTRATGIPARFSGSGSGTGFTLTISSLQSEDFAVYYCQQYDTWPFTF
GPGTKVDFKRGGGGSGGGGSGGGGSQVQLVESGGGVVQPGRSLRLS
CAASGFTFSSYGMHWVRQAPGKGLEWVAVISYDGSDKYYVDSVKGR
FTISRDNSKNRLYLQMNSLRAEDTAVYYCARERYSGRDYWGQGTLV
TVSSAAALDNEKSNGTIIHVKGKHLCPSPLFPGPSKPFWVLVVVGGVL
ACYSLLVTVAFIIFWVRSKRSRLLHSDYMNMTPRRPGPTRKHYQPYAP
PRDFAAYRSRVKFSRSADAPAYQQGQNQLYNELNLGRREEYDVLDK
RRGRDPEMGGKPRRKNPQEGLYNELQKDKMAEAYSEIGMKGERRRG
KGHDGLYQGLSTATKDTYDALHMQALPPR (SEQ ID NO. 118)
Clone 20C5.2 CD28 CD3 zeta CAR DNA Heavy & Light Chains 
ATGGCACTCCCCGTAACTGCTCTGCTGCTGCCGTTGGCATTGCTCCT
GCACGCCGCACGCCCGGAGATCGTCATGACACAGAGTCCAGCTAC
CCTGAGCGTGTCCCCTGGAGAGAGAGCCACCCTGTCCTGTAGGGCT
AGTCAGAGTGTGTCCAGCCTCCTCACCTGGTATCAACAGAAGCCTG
GTCAAGCTCCCCGGCTGCTTATCTTCGGGGCCAGCACGCGAGCCAC
AGGCATCCCGGCCAGATTCTCTGGCTCTGGCAGTGGCACCGGGTTC
ACTCTCACGATCTCATCCCTGCAGTCAGAGGATTTCGCTGTGTATTA
CTGTCAGCAGTACGATACATGGCCCTTCACCTTCGGCCCGGGCACA
AAAGTAGATTTCAAGCGCGGCGGCGGGGGTAGTGGGGGCGGGGGA
TCAGGAGGAGGGGGCTCCCAAGTACAGCTGGTTGAGAGCGGCGGC
GGGGTGGTTCAGCCCGGGCGCAGCCTCAGGCTGAGTTGCGCAGCA
TCAGGATTCACATTCAGTTCTTATGGAATGCATTGGGTCAGACAGG
CTCCCGGGAAGGGCCTTGAATGGGTGGCAGTCATTAGCTACGACG
GAAGCGATAAGTACTATGTGGACTCAGTTAAAGGAGATTTACTAT
CAGCCGCGACAATTCCAAAAACAGATTGTATTTGCAGATGAACTCC
CTCAGGGCGGAGGACACTGCTGTATATTACTGCGCACGAGAGAGA
TACTCCGGCCGAGACTATTGGGGCCAAGGAACATTGGTAACTGTGA
GCTCCGCCGCAGCTATTGAGGTCATGTACCCCCCACCTTATCTCGA
TAATGAGAAGAGTAATGGGACTATAATTCACGTAAAGGGCAAACA
CCTGTGCCCTTCCCCGCTGTTTCCAGGTCCAAGTAAGCCGTTCTGGG
TCCTGGTTGTGGTGGGAGGGGTGCTGGCCTGCTATTCTCTGTTGGTT
ACCGTGGCCTTTATCATTTTCTGGGTGAGATCCAAAAGAAGCCGCC
TGCTCCATAGCGATTACATGAATATGACTCCACGCCGCCCTGGCCC
CACAAGGAAACACTACCAGCCTTACGCACCACCTAGAGATTTCGCT
GCCTATCGGAGCAGGGTGAAGTTTTCCAGATCTGCAGATGCACCAG
CGTATCAGCAGGGCCAGAACCAACTGTATAACGAGCTCAACCTGG
GACGCAGGGAAGAGTATGACGTTTTGGACAAGCGCAGAGGACGGG
ACCCTGAGATGGGTGGCAAACCAAGACGAAAAAACCCCCAGGAGG
GTCTCTATAATGAGCTGCAGAAGGATAAGATGGCTGAAGCCTATTC
TGAAATAGGCATGAAAGGAGAGCGGAGAAGGGGAAAAGGGCACG
ACGGTTTGTACCAGGGACTCAGCACTGCTACGAAGGATACTTATGA
CGCTCTCCACATGCAAGCCCTGCCACCTAGGTAA (SEQ ID NO. 119)
Clone 20C5.2 CD28 CD3 zeta CAR AA Heavy & Light Chains
   (Signal Peptide in Bold) 
MALPVTALLLPLALLLHAARPEIVMTQSPATLSVSPGERATLSCRAS
QSVSSLLTWYQQKPGQAPRLLIFGASTRATGIPARFSGSGSGTGFTLTIS
SLQSEDFAVYYCQQYDTWPFTFGPGTKVDFKRGGGGSGGGGSGGGG
SQVQLVESGGGVVQPGRSLRLSCAASGFTFSSYGMHWVRQAPGKGLE
WVAVISYDGSDKYYVDSVKGRFTISRDNSKNRLYLQMNSLRAEDTAV
YYCARERYSGRDYWGQGTLVTVSSAAAIEVMYPPPYLDNEKSNGTII
HVKGKHLCPSPLFPGPSKPFWVLVVVGGVLACYSLLVTVAFIIFWVRS
KRSRLLHSDYMNMTPRRPGPTRKHYQPYAPPRDFAAYRSRVKFSRSA
DAPAYQQGQNQLYNELNLGRREEYDVLDKRRGRDPEMGGKPRRKNP
QEGLYNELQKDKMAEAYSEIGMKGERRRGKGHDGLYQGLSTATKD
TYDALHMQALPPR (SEQ ID NO. 120)
Clone 20C5.2 CD28 CD3 zeta CAR DNA Heavy & Light Chains 
GAGATCGTCATGACACAGAGTCCAGCTACCCTGAGCGTGTCCCCTG
GAGAGAGAGCCACCCTGTCCTGTAGGGCTAGTCAGAGTGTGTCCA
GCCTCCTCACCTGGTATCAACAGAAGCCTGGTCAAGCTCCCCGGCT
GCTTATCTTCGGGGCCAGCACGCGAGCCACAGGCATCCCGGCCAG
ATTCTCTGGCTCTGGCAGTGGCACCGGGTTCACTCTCACGATCTCAT
CCCTGCAGTCAGAGGATTTCGCTGTGTATTACTGTCAGCAGTACGA
TACATGGCCCTTCACCTTCGGCCCGGGCACAAAAGTAGATTTCAAG
CGCGGCGGCGGGGGTAGTGGGGGCGGGGGATCAGGAGGAGGGGG
CTCCCAAGTACAGCTGGTTGAGAGCGGCGGCGGGGTGGTTCAGCC
CGGGCGCAGCCTCAGGCTGAGTTGCGCAGCATCAGGATTCACATTC
AGTTCTTATGGAATGCATTGGGTCAGACAGGCTCCCGGAAGGGCC
TTGAATGGGTGGCAGTCATTAGCTACGACGGAAGCGATAAGTACT
ATGTGGACTCAGTTAAAGGGAGATTTACTATCAGCCGCGACAATTC
CAAAAACAGATTGTATTTGCAGATGAACTCCCTCAGGGCGGAGGA
CACTGCTGTATATTACTGCGCACGAGAGATACTCCGGCCGAGAC
TATTGGGGCCAAGGAACATTGGTAACTGTGAGCTCCGCCGCAGCTA
TTGAGGTCATGTACCCCCCACCTTATCTCGATAATGAGAAGAGTAA
TGGGACTATAATTCACGTAAAGGGCAAACACCTGTGCCCTTCCCCG
CTGTTTCCAGGTCCAAGTAAGCCGTTCTGGGTCCTGGTTGTGGTGG
 
GAGGGGTGCTGGCCTGCTATTCTCTGTTGGTTACCGTGGCCTTTATC
ATTTTCTGGGTGAGATCCAAAAGAAGCCGCCTGCTCCATAGCGATT
ACATGAATATGACTCCACGCCGCCCTGGCCCCACAAGGAAACACT
ACCAGCCTTACGCACCACCTAGAGATTTCGCTGCCTATCGGAGCAG
GGTGAAGTTTTCCAGATCTGCAGATGCACCAGCGTATCAGCAGGGC
CAGAACCAACTGTATAACGAGCTCAACCTGGGACGCAGGGAAGAG
TATGACGTTTTGGACAAGCGCAGAGGACGGGACCCTGAGATGGGT
GGCAAACCAAGACGAAAAAACCCCCAGGAGGGTCTCTATAATGAG
CTGCAGAAGGATAAGATGGCTGAAGCCTATTCTGAAATAGGCATG
AAAGGAGAGCGGAGAAGGGGAAAAGGGCACGACGGTTTGTACCA
GGGACTCAGCACTGCTACGAAGGATACTTATGACGCTCTCCACATG
CAAGCCCTGCCACCTAGG (SEQ ID NO. 121)
Clone 20C5.2 CD28 CD3 zeta CAR AA Heavy & Light Chains 
EIVMTQSPATLSVSPGERATLSCRASQSVSSLLTWYQQKPGQAPRLLIF
GASTRATGIPARFSGSGSGTGFTLTISSLQSEDFAVYYCQQYDTWPFTF
GPGTKVDFKRGGGGSGGGGSGGGGSQVQLVESGGGVVQPGRSLRLS
CAASGFTFSSYGMHWVRQAPGKGLEWVAVISYDGSDKYYVDSVKGR
FTISRDNSKNRLYLQMNSLRAEDTAVYYCARERYSGRDYWGQGTLV
TVSSAAAIEVMYPPPYLDNEKSNGTIIHVKGKHLCPSPLFPGPSKPFWV
LVVVGGVLACYSLLVTVAFIIFWVRSKRSRLLHSDYMNMTPRRPGPTR
KHYQPYAPPRDFAAYRSRVKFSRSADAPAYQQGQNQLYNELNLGRRE
EYDVLDKRRGRDPEMGGKPRRKNPQEGLYNELQKDKMAEAYSEIGM
KGERRRGKGHDGLYQGLSTATKDTYDALHMQALPPR (SEQ ID NO.
122)
Clone 20C5.2 CD8 CD3 zeta CAR DNA Heavy & Light Chains 
ATGGCACTCCCCGTAACTGCTCTGCTGCTGCCGTTGGCATTGCTCCT
GCACGCCGCACGCCCGGAAATAGTGATGACTCAGTCCCCGGCCAC
CCTCAGCGTGTCCCCGGGGAGCGAGCGACCCTGTCATGCAGGGCT
TCCCAGAGTGTCAGCTCCCTGCTCACTTGGTATCAGCAAAAGCCGG
GGCAGGCTCCCCGCCTCCTCATCTTCGGGGCATCAACTAGGGCCAC
CGGCATTCCTGCAAGATTTTCCGGGTCTGGCAGCGGCACCGGCTTC
ACCCTTACCATTAGCTCTCTGCAGTCTGAGGACTTCGCCGTTTACTA
TTGTCAGCAGTATGATACTTGGCCCTTTACCTTCGGTCCCGGAACTA
 
AGGTGGACTTCAAGCGCGGGGGGGTGGATCTGGAGGTGGTGGCT
CCGGGGGCGGTGGAAGCCAGGTCCAGTTGGTTGAGAGCGGCGGCG
GAGTGGTGCAGCCCGGGAGGTCCTTGCGGCTGAGCTGTGCAGCCTC
CGGTTTTACTTTTCTAGCTATGGAATGCATTGGGTAAGACAGGCTC
CCGGAAAAGGCCTCGAGTGGGTGGCGGTCATTAGCTATGATGGAT
CTGATAAATACTATGTGGACTCAGTTAAGGGGCGCTTCACAATCTC
AAGAGACAATAGCAAAAATAGACTGTACCTGCAGATGAATAGTCT
GCGCGCCGAGGACACTGCCGTGTACTACTGCGCCCGCGAGAGATA
CAGCGGACGGGATTACTGGGGCCAGGGTACCCTCGTAACGGTGTC
CTCCGCTGCCGCCCTTAGCAACAGCATTATGTACTTTTCTCATTTCG
TGCCAGTCTTTCTCCCAGCAAAGCCCACCACTACCCCGGCCCCCAG
GCCGCCTACTCCTGCCCCCACTATCGCGTCTCAGCCTCTCTCCTTGC
GGCCCGAGGCCTGCCGGCCAGCCGCAGGGGGCGCCGTACATACTC
GGGGTTTGGATTTCGCTTGCGACATATATATTTGGGCCCCCCTCGCC
GGCACATGTGGAGTGCTGCTCCTGAGTCTCGTTATAACCCTCTATT
GCAACCATAGAAACAGATCCAAAAGAAGCCGCCTGCTCCATAGCG
ATTACATGAATATGACTCCACGCCGCCCTGGCCCCACAAGGAAACA
CTACCAGCCTTACGCACCACCTAGAGATTTCGCTGCCTATCGGAGC
AGGGTGAAGTTTTCCAGATCTGCAGATGCACCAGCGTATCAGCAGG
GCCAGAACCAACTGTATAACGAGCTCAACCTGGGACGCAGGGAAG
AGTATGACGTTTTGGACAAGCGCAGAGGACGGGACCCTGAGATGG
GTGGCAAACCAAGACGAAAAAACCCCCAGGAGGGTCTCTATAATG
AGCTGCAGAAGGATAAGATGGCTGAAGCCTATTCTGAAATAGGCA
TGAAAGGAGAGCGGAGAAGGGGAAAAGGGCACGACGGTTTGTAC
CAGGGACTCAGCACTGCTACGAAGGATACTTATGACGCTCTCCACA
TGCAAGCCCTGCCACCTAGGTAA (SEQ ID NO. 123)
Clone 20C5.2 CD8 CD3 zeta CAR AA Heavy & Light Chains
   (Signal Peptide in Bold) 
MALPVTALLLPLALLLHAARPEIVMTQSPATLSVSPGERATLSCRAS
QSVSSLLTWYQQKPGQAPRLLIFGASTRATGIPARFSGSGSGTGFTLTIS
SLQSEDFAVYYCQQYDTWPFTFGPGTKVDFKRGGGGSGGGGSGGGG
SQVQLVESGGGVVQPGRSLRLSCAASGFTFSSYGMHWVRQAPGKGLE
WVAVISYDGSDKYYVDSVKGRFTISRDNSKNRLYLQMNSLRAEDTAV
 
YYCARERYSGRDYWGQGTLVTVSSAAALSNSIMYFSHFVPVFLPAKP
TTTPAPRPPTPAPTIASQPLSLRPEACRPAAGGAVHTRGLDFACDIYIW
APLAGTCGVLLLSLVITLYCNHRNRSKRSRLLHSDYMNMTPRRPGPTR
KHYQPYAPPRDFAAYRSRVKFSRSADAPAYQQGQNQLYNELNLGRRE
EYDVLDKRRGRDPEMGGKPRRKNPQEGLYNELQKDKMAEAYSEIGM
KGERRRGKGHDGLYQGLSTATKDTYDALHMQALPPR (SEQ ID NO.
124)
Clone 20C5.2 CD8 CD3 zeta CAR DNA Heavy & Light Chains 
GAAATAGTGATGACTCAGTCCCCGGCCACCCTCAGCGTGTCCCCG
GGGAGCGAGCGACCCTGTCATGCAGGGCTTCCAGAGTGTCAGCTC
CCTGCTCACTTGGTATCAGCAAAAGCCGGGGCAGGCTCCCCGCCTC
CTCATCTTCGGGGCATCAACTAGGGCCACCGGCATTCCTGCAAGAT
TTTCCGGGTCTGGCAGCGGCACCGGCTTCACCCTTACCATTAGCTCT
CTGCAGTCTGAGGACTTCGCCGTTTACTATTGTCAGCAGTATGATA
CTTGGCCCTTTACCTTCGGTCCCGGAACTAAGGTGGACTTCAAGCG
CGGGGGGGTGGATCTGGAGGTGGTGGCTCCGGGGGCGGTGGAAG
CCAGGTCCAGTTGGTTGAGAGCGGCGGAGTGGTGCAGCCCGG
GAGGTCCTTGCGGCTGAGCTGTGCAGCCTCCGGTTTACTTTTCTA
GCTATGGAATGCATTGGGTAAGACAGGCTCCCGGAAAAGGCCTCG
AGTGGGTGGCGGTCATTAGCTATGATGGATCTGATAAATACTATGT
GGACTCAGTTAAGGGGCGCTTCACAATCTCAAGAGACAATAGCAA
AAATAGACTGTACCTGCAGATGAATAGTCTGCGCGCCGAGGACAC
TGCCGTGTACTACTGCGCCCGCGAGAGATACAGCGGACGGATTA
CTGGGGCCAGGGTACCCTCGTAACGGTGTCCTCCGCTGCCGCCCTT
AGCAACAGCATTATGTACTTTTCTCATTTCGTGCCAGTCTTTCTCCC
AGCAAAGCCCACCACTACCCCGGCCCCCAGGCCGCCTACTCCTGCC
CCCACTATCGCGTCTCAGCCTCTCTCCTTGCGGCCCGAGGCCTGCC
GGCCAGCCGCAGGGGGCGCCGTACATACTCGGGGTTGGATTTCGC
TTGCGACATATATATTTGGGCCCCCCTCGCCGGCACATGTGGAGTG
CTGCTCCTGAGTCTCGTTATAACCCTCTATTGCAACCATAGAAACA
GATCCAAAAGAAGCCGCCTGCTCCATAGCGATTACATGAATATGAC
TCCACGCCGCCCTGGCCCCACAAGGAAACACTACCAGCCTTACGCA
CCACCTAGAGATTTCGCTGCCTATCGGAGCAGGGTGAAGTTTTCCA
 
GATCTGCAGATGCACCAGCGTATCAGCAGGGCCAGAACCAACTGT
ATAACGAGCTCAACCTGGGACGCAGGGAAGAGTATGACGTTTTGG
ACAAGCGCAGAGGACGGGACCCTGAGATGGGTGGCAAACCAAGAC
GAAAAAACCCCCAGGAGGGTCTCTATAATGAGCTGCAGAAGGATA
AGATGGCTGAAGCCTATTCTGAAATAGGCATGAAAGGAGAGCGGA
GAAGGGGAAAAGGGCACGACGGTTTGTACCAGGGACTCAGCACTG
CTACGAAGGATACTTATGACGCTCTCCACATGCAAGCCCTGCCACC
TAGG (SEQ ID NO. 125)
Clone 20C5.2 CD8 CD3 zeta CAR AA Heavy & Light Chains 
EIVMTQSPATLSVSPGERATLSCRASQSVSSLLTWYQQKPGQAPRLLIF
GASTRATGIPARFSGSGSGTGFTLTISSLQSEDFAVYYCQQYDTWPFTF
GPGTKVDFKRGGGGSGGGGSGGGGSQVQLVESGGGVVQPGRSLRLS
CAASGFTFSSYGMHWVRQAPGKGLEWVAVISYDGSDKYYVDSVKGR
FTISRDNSKNRLYLQMNSLRAEDTAVYYCARERYSGRDYWGQGTLV
TVSSAAALSNSIMYFSHFVPVFLPAKPTTTPAPRPPTPAPTIASQPLSLRP
EACRPAAGGAVHTRGLDFACDIYIWAPLAGTCGVLLLSLVITLYCNHR
NRSKRSRLLHSDYMNMTPRRPGPTRKHYQPYAPPRDFAAYRSRVKFS
RSADAPAYQQGQNQLYNELNLGRREEYDVLDKRRGRDPEMGGKPRR
KNPQEGLYNELQKDKMAEAYSEIGMKGERRRGKGHDGLYQGLSTAT
KDTYDALHMQALPPR (SEQ ID NO. 126)
CAR Signal Peptide DNA 
ATGGCACTCCCCGTAACTGCTCTGCTGCTGCCGTTGGCATTGCTCCT
GCACGCCGCACGCCCG (SEQ ID NO. 127)
CAR Signal Peptide: MALPVTALLLPLALLLHAARP (SEQ ID NO. 128)
scFv G4S linker DNA
   GGCGGTGGAGGCTCCGGAGGGGGGGGCTCTGGCGGAGGGGGCTCC (SEQ ID NO. 129)
scFv G4s linker: GGGGSGGGGSGGGGS (SEQ ID NO. 130)
scFv Whitlow linker DNA 
GGGTCTACATCCGGCTCCGGGAAGCCCGGAAGTGGCGAAGGTAGT
ACAAAGGGG (SEQ ID NO. 131)
scFv Whitlow linker: GSTSGSGKPGSGEGSTKG (SEQ ID NO. 132)
CD28 AA Extracellular Domain 
MLRLLLALNLFPSIQVTGNKILVKQSPMLVAYDNAVNLSCKYSYNLFS
REFRASLHKGLDSAVEVCVVYGNYSQQLQVYSKTGFNCDGKLGNES
VTFYLQNLYVNQTDIYFCKIEVMYPPPYLDNEKSNGTIIHVKGKHLCPS
PLFPGPSKP (SEQ ID NO. 133)
GX₂X₃X₄X₅X₆X₇X₈X₉ (SEQ ID NO: 134)
X₁X₂X₃X₄X₅X₆ (SEQ ID NO: 135)
X₁X₂X₃X₄X₅X₆X₇X₈X₉X₁₀X₁₁X₁₂DY (SEQ ID NO: 136)
X₁ASQX₅X₆X₇X₈X₉LX₁₁ (SEQ ID NO: 137)
X₁ASX₄X₅X₆X₇ (SEQ ID NO: 138)
QQX₃X₄X₅X₆PX₈T (SEQ ID NO: 139)
4-1BB Nucleic Acid Sequence (intracellular domain) 
AAGCGCGGCAGGAAGAAGCTCCTCTACATTTTAAGCAGCCTTTTA
TGAGGCCCGTACAGACAACACAGGAGGAAGATGGCTGTAGCTGCA
GATTTCCCGAGGAGGAGGAAGGTGGGTGCGAGCTG (SEQ ID NO.
140)
4-1BB AA (intracellular domain)
   KRGRKKLLYIFKQPFMRPVQTTQEEDGCSCRFPEEEEGGCEL (SEQ ID NO. 141)
OX40 AA
   RRDQRLPPDAHKPPGGGSFRTPIQEEQADAHSTLAKI (SEQ ID NO. 142)
Leader Sequence AA
   MALPVTALLLPLALLLHAARP (SEQ ID NO: 143)
Additional G4S linker: GGGGSGGGGSGGGGSGGGGS (SEQ ID NO. 144)
CD3 zeta variant AA 
RVKFSRSADAPAYKQGQNQLYNELNLGRREEYDVLDKRRGRDPEMGGKPRRKNPQ
EGLYNELQKDKMAEAYSEIGMKGERRRGKGHDGLYQGLSTATKDTYDALHMQAL
PPR (SEQ ID NO. 145)
Axi Cel (KTE-C19) DNA (SEQ ID NO. 146) 
Atgcttctcctggtgacaagccttctgctctgtgagttaccacacccagcattcctcctgatcccagacatccagatgacacagactaca
tcctccctgtctgcctctctgggagacagagtcaccatcagttgcagggcaagtcaggacattagtaaatatttaaattggtatcagcaga
aaccagatggaactgttaaactcctgatctaccatacatcaagattacactcaggagtcccatcaaggttcagtggcagtgggtctggaa
cagattattctctcaccattagcaacctggagcaagaagatattgccacttacttttgccaacagggtaatacgcttccgtacacgttcgg
aggggggactaagttggaaataacaggctccacctctggatccggcaagcccggatctggcgagggatccaccaagggcgaggtg
aaactgcaggagtcaggacctggcctggtggcgccctcacagagcctgtccgtcacatgcactgtctcaggggtctcattacccgact
atggtgtaagctggattcgccagcctccacgaaagggtctggagtggctgggagtaatatggggtagtgaaaccacatactataattca
gctctcaaatccagactgaccatcatcaaggacaactccaagagccaagttttcttaaaaatgaacagtctgcaaactgatgacacagc
catttactactgtgccaaacattattactacggtggtagctatgctatggactactggggtcaaggaacctcagtcaccgtctcctcagcg
gccgcaattgaagttatgtatcctcctccttacctagacaatgagaagagcaatggaaccattatccatgtgaaagggaaacacctttgtc
caagtcccctatttcccggaccttctaagcccttttgggtgctggtggtggttgggggagtcctggcttgctatagcttgctagtaacagtg
gcctttattattttctgggtgaggagtaagaggagcaggctcctgcacagtgactacatgaacatgactccccgccgccccgggccca
cccgcaagcattaccagccctatgccccaccacgcgacttcgcagcctatcgctccagagtgaagttcagcaggagcgcagacgcc
cccgcgtaccagcagggccagaaccagctctataacgagctcaatctaggacgaagagaggagtacgatgttttggacaagagacg
tggccgggaccctgagatggggggaaagccgagaaggaagaaccctcaggaaggcctgtacaatgaactgcagaaagataagat
ggcggaggcctacagtgagattgggatgaaaggcgagcgccggaggggcaaggggcacgatggcctttaccagggtctcagtac
agccaccaaggacacctacgacgcccttcacatgcaggccctgccccctcgc
Axi Cel (KTE-C19) AA (SEQ ID NO. 147) 
MLLLVTSLLLCELPHPAFLLIPDIQMTQTTSSLSASLGDRVTISCRASQDISKYLNWYQ
QKPDGTVKLLIYHTSRLHSGVPSRFSGSGSGTDYSLTISNLEQEDIATYFCQQGNTLPY
TFGGGTKLEITGSTSGSGKPGSGEGSTKGEVKLQESGPGLVAPSQSLSVTCTVSGVSL
PDYGVSWIRQPPRKGLEWLGVIWGSETTYYNSALKSRLTIIKDNSKSQVFLKMNSLQ
TDDTAIYYCAKHYYYGGSYAMDYWGQGTSVTVSSAAAIEVMYPPPYLDNEKSNGTI
IHVKGKHLCPSPLFPGPSKPFWVLVVVGGVLACYSLLVTVAFIIFWVRSKRSRLLHSD
 
YMNMTPRRPGPTRKHYQPYAPPRDFAAYRSRVKFSRSADAPAYQQGQNQLYNELN
LGRREEYDVLDKRRGRDPEMGGKPRRKNPQEGLYNELQKDKMAEAYSEIGMKGER
RRGKGHDGLYQGLSTATKDTYDALHMQALPPR
Humanized anti-CD19 CAR DNA (SEQ ID NO. 148) 
ATGGCACTCCCCGTAACTGCTCTGCTGCTGCCGTTGGCATTGCTCCTGCACGCCG
CACGCCCGGATATTCAAATGACCCAGTCCCCGTCCTCCCTGAGTGCCTCCGTCGG
TGACCGTGTTACGATTACCTGCCGTGCGAGCCAAGACATCTCTAAATACCTGAAC
TGGTATCAGCAAAAACCGGATCAGGCACCGAAACTGCTGATCAAACATACCTCA
CGTCTGCACTCGGGTGTGCCGAGCCGCTTTAGTGGTTCCGGCTCAGGTACCGATT
ACACCCTGACGATCAGCTCTCTGCAGCCGGAAGACTTTGCCACGTATTACTGCCA
GCAAGGTAATACCCTGCCGTATACGTTCGGCCAAGGTACCAAACTGGAAATCAA
AGGCTCGACGAGCGGCTCTGGTAAACCGGGCTCTGGTGAAGGCAGTACCAAAGG
TGAAGTGCAGCTGGTTGAAAGCGGTGGTGGTCTGGTTCAACCGGGTCGTTCCCTG
CGTCTGTCATGTACGGCGAGTGGTGTCTCCCTGCCGGACTATGGCGTGTCCTGGA
TTCGTCAGCCGCCGGGTAAAGGCCTGGAATGGATTGGTGTCATCTGGGGCAGTG
AAACCACGTATTACAACTCGGCCCTGAAAAGCCGTTTCACCATCTCTCGCGATAA
CAGTAAAAATACGCTGTACCTGCAGATGAATAGCCTGCGCGCGGAAGACACCGC
CGTTTACTACTGCGCAAAACATTACTACTACGGTGGCAGCTATGCTATGGATTAC
TGGGGTCAAGGCACGCTGGTCACCGTTTCGTCAGCCGCTGCCCTTGATAATGAAA
AGTCAAACGGAACAATCATTCACGTGAAGGGCAAGCACCTCTGTCCGTCACCCTT
GTTCCCTGGTCCATCCAAGCCATTCTGGGTGTTGGTCGTAGTGGGTGGAGTCCTC
GCTTGTTACTCTCTGCTCGTCACCGTGGCTTTTATAATCTTCTGGGTTAGATCCAA
AAGAAGCCGCCTGCTCCATAGCGATTACATGAATATGACTCCACGCCGCCCTGGC
CCCACAAGGAAACACTACCAGCCTTACGCACCACCTAGAGATTTCGCTGCCTATC
GGAGCAGGGTGAAGTTTTCCAGATCTGCAGATGCACCAGCGTATCAGCAGGGCC
 
AGAACCAACTGTATAACGAGCTCAACCTGGGACGCAGGGAAGAGTATGACGTTT
TGGACAAGCGCAGAGGACGGGACCCTGAGATGGGTGGCAAACCAAGACGAAAA
AACCCCCAGGAGGGTCTCTATAATGAGCTGCAGAAGGATAAGATGGCTGAAGCC
TATTCTGAAATAGGCATGAAAGGAGAGCGGAGAAGGGGAAAAGGGCACGACGG
TTTGTACCAGGGACTCAGCACTGCTACGAAGGATACTTATGACGCTCTCCACATG
CAAGCCCTGCCAcctagg
Humanized anti-CD19 CAR AA (SEQ ID NO. 149) 
MALPVTALLLPLALLLHAARPDIQMTQSPSSLSASVGDRVTITCRASQDISKYLNWY
QQKPDQAPKLLIKHTSRLHSGVPSRFSGSGSGTDYTLTISSLQPEDFATYYCQQGNTL
PYTFGQGTKLEIKGSTSGSGKPGSGEGSTKGEVQLVESGGGLVQPGRSLRLSCTASG
VSLPDYGVSWIRQPPGKGLEWIGVIWGSETTYYNSALKSRFTISRDNSKNTLYLQMN
SLRAEDTAVYYCAKHYYYGGSYAMDYWGQGTLVTVSSAAALDNEKSNGTIIHVKG
KHLCPSPLFPGPSKPFWVLVVVGGVLACYSLLVTVAFIIFWVRSKRSRLLHSDYMNM
TPRRPGPTRKHYQPYAPPRDFAAYRSRVKFSRSADAPAYQQGQNQLYNELNLGRRE
EYDVLDKRRGRDPEMGGKPRRKNPQEGLYNELQKDKMAEAYSEIGMKGERRRGK
GHDGLYQGLSTATKDTYDALHMQALPPR
Fully human anti-CD19 CAR DNA (SEQ ID NO. 150) 
Atggccctgcctgtgacagctctgctgctgcccctggccctgctgctgcatgccgccagacctgagatcgtgctgacccagtctcccg

gcaccctgtctctcagcccaggagagagagccaccctgagctgcagagccagccagagcgtgtccagcagctacctggcctggtat

cagcagaagcccggacaggcccccagactgctgatctacggcgccagctctagagccaccggcatccccgacagattcagcggca

gcggcagtggcaccgacttcaccctgaccatcagcagactggaacccgaggacttcgccgtgtactactgccagcagtacggcagc

agccggttcaccttcggccctggcaccaaggtggacatcaagggcagcacctccggcagcggcaagcctggctctggcgagggct

ctaccaagggccaggtgcagctggtgcagtctggcgccgaagtgaagaaacccggctctagcgtgaaggtgtcctgcaaggacag

cggcggcaccttcagcagctacgccatcagctgggtgcgccaggccccaggacaggggctggaatggatgggcggcatcatcccc

atcttcggcaccaccaactacgcccagcagttccagggcagagtgaccatcaccgccgacgagagcaccagcaccgcctacatgg
 
aactgagcagcctgcggagcgaggacacagccgtgtattactgtgcccgcgaggccgtggccgccgactggctggatccttgggg

acagggcaccctggtgacagtgtccagcttcgtgcccgtgttcctgcccgccaagcctaccaccacccctgcccctagacctcccacc

ccagccccaacaatcgccagccagcctctgtccctgcggcccgaagcctgtagacctgctgccggcggagccgtgcacaccagag

gcctggacttcgcctgcgatatctacatctgggcccctctggccggcacctgtggcgtgctgctgctgagcctggtgatcaccctgtact

gcaaccaccggaacagaagcaagcggagccggctgctgcacagcgactacatgaacatgaccccaagacggcctggccccaccc

ggaagcactaccagccttacgcccctcccagagacttcgccgcctaccggtccagagtgaagttcagcagatccgccgacgcccct

gcctaccagcagggacagaaccagctgtacaacgagctgaacctgggcagacgggaagagtacgacgtgctggacaagcggaga

ggccgggaccccgagatgggcggaaagcccagacggaagaacccccaggaaggcctgtataacgaactgcagaaagacaagat

ggccgaggcctacagcgagatcggcatgaagggcgagcggaggcgcggcaagggccacgatggcctgtaccagggcctgagc

accgccaccaaggacacctacgacgccctgcacatgcaggccctgcccccaga
Fully human anti-CD19 CAR AA (SEQ ID NO. 151) 
MALPVTALLLPLALLLHAARPEIVLTQSPGTLSLSPGERATLSCRASQSVSSSYLAWY
QQKPGQAPRLLIYGASSRATGIPDRFSGSGSGTDFTLTISRLEPEDFAVYYCQQYGSSR
FTFGPGTKVDIKGSTSGSGKPGSGEGSTKGQVQLVQSGAEVKKPGSSVKVSCKDSGG
TFSSYAISWVRQAPGQGLEWMGGIIPIFGTTNYAQQFQGRVTITADESTSTAYMELSS
LRSEDTAVYYCAREAVAADWLDPWGQGTLVTVSSFVPVFLPAKPTTTPAPRPPTPAP
TIASQPLSLRPEACRPAAGGAVHTRGLDFACDIYIWAPLAGTCGVLLLSLVITLYCNH
RNRSKRSRLLHSDYMNMTPRRPGPTRKHYQPYAPPRDFAAYRSRVKFSRSADAPAY
QQGQNQLYNELNLGRREEYDVLDKRRGRDPEMGGKPRRKNPQEGLYNELQKDKM
AEAYSEIGMKGERRRGKGHDGLYQGLSTATKDTYDALHMQALPPR
MAGE A3/A6 TCR DNA (SEQ ID NO. 152) 
atggcatgccctggcttcctgtgggcacttgtgatctccacctgtcttgaatttagcatggctcagacagtcactcagtctcaaccagaga

tgtctgtgcaggaggcagagaccgtgaccctgagctgcacatatgacaccagtgagagtgattattatttattctggtacaagcagcctc

ccagcaggcagatgattctcgttattcgccaagaagcttataagcaacagaatgcaacagagaatcgtttctctgtgaacttccagaaag

cagccaaatccttcagtctcaagatctcagactcacagctgggggatgccgcgatgtatttctgtgctctccggagctcaggaacctac
 
aaatacatctttggaacaggcaccaggctgaaggttttagcaaatatccagaaccctgaacctgctgtgtaccagttaaaagatcctcgg
tctcaggacagcaccctctgcctgttcaccgactttgactcccaaatcaatgtgccgaaaaccatggaatctggaacgttcatcactgac
aaaactgtgctggacatgaaagctatggattccaagagcaatggggccattgcctggagcaaccagacaagcttcacctgccaagat
atcttcaaagagaccaacgccacctaccccagttcagacgttccctgtgatgccacgttgactgagaaaagctttgaaacagatatgaa
cctaaactttcaaaacctgtcagttatgggactccgaatcctcctgctgaaagtagccggatttaacctgctcatgacgctgaggctgtgg
tccagtcgggccaagcggtccggatccggagccaccaacttcagcctgctgaagcaggccggcgacgtggaggagaaccccggc
cccatgggcaccaggctcctcttctgggtggccttctgtctcctgggggcagatcacacaggagctggagtctcccagtcccccagta
acaaggtcacagagaagggaaaggatgtagagctcaggtgtgatccaatttcaggtcatactgccctttactggtaccgacagagcct
ggggcagggcctggagtttttaatttacttccaaggcaacagtgcaccagacaaatcagggctgcccagtgatcgcttctctgcagaga
ggactgggggatccgtctccactctgacgatccagcgcacacagcaggaggactcggccgtgtatctctgtgccagcatccggaca
gggcctttttctctggaaacaccatatattttggagagggaagttggctcactgttgtagaggacctgagaaacgtgaccccacccaag
gtctccttgtttgagccatcaaaagcagagattgcaaacaaacaaaaggctaccctcgtgtgcttggccaggggcttcttccctgaccac
gtggagctgagctggtgggtgaatggcaaggaggtccacagtggggtcagcacggaccctcaggcctacaaggagagcaattata
gctactgcctgagcagccgcctgagggtctctgctaccttctggcacaatcctcgaaaccacttccgctgccaagtgcagttccatggg
ctttcagaggaggacaagtggccagagggctcacccaaacctgtcacacagaacatcagtgcagaggcctggggccgagcagact
gtggaatcacttcagcatcctatcatcaggggggttctgtctgcaaccatcctctatgagatcctactggggaaggccaccctatatgctgt
gctggtcagtggcctggtgctgatggctatggtcaaaagaaagaactcatga
MAGE A3/A6 TCR AA (SEQ ID NO. 153) 
M A C P G F L W A L V I S T C L E F S M A Q T V T Q S Q P E M S V Q E A E T V T L

S C T Y D T S E S D Y Y L F W Y K Q P P S R Q M I L V I R Q E A Y K Q Q N A T E N

R F S V N F Q K A A K S F S L K I S D S Q L G D A A M Y F C A L R S S G T Y K Y I F

G T G T R L K V L A N I Q N P E P A V Y Q L K D P R S Q D S T L C L F T D F D S Q I

N V P K T M E S G T F I T D K T V L D M K A M D S K S N G A I A W S N Q T S F T C

Q D I F K E T N A T Y P S S D V P C D A T L T E K S F E T D M N L N F Q N L S V M

G L R I L L L K V A G F N L L M T L R L W S S R A K R S G S G A T N F S L L K Q A
 
G D V E E N P G P M G T R L L F W V A F C L L G A D H T G A G V S Q S P S N K V T

E K G K D V E L R C D P I S G H T A L Y W Y R Q S L G Q G L E F L I Y F Q G N S A

P D K S G L P S D R F S A E R T G G S V S T L T I Q R T Q Q E D S A V Y L C A S I R T

G P F F S G N T I Y F G E G S W L T V V E D L R N V T P P K V S L F E P S K A E I A

N K Q K A T L V C L A R G F F P D H V E L S W W V N G K E V H S G V S T D P Q A

Y K E S N Y S Y C L S S R L R V S A T F W H N P R N H F R C Q V Q F H G L S E E D

K W P E G S P K P V T Q N I S A E A W G R A D C G I T S A S Y H Q G V L S A T I L

Y E I L L G K A T L Y A V L V S G L V L M A M V K R K N S Stop
Anti-CLL-1 CAR DNA (SEQ ID NO. 154) 
ATGGCACTCCCCGTAACTGCTCTGCTGCTGCCGTTGGCATTGCTCCTGCACGCCG
CACGCCCGCAGGTCCAACTGCAAGAAAGCGGACCCGGACTGGTGAAGCCTTCTG
AGACACTTAGTCTGACGTGCACGGTCAGTGGCGGCTCCATCTCCTCCTATTATTG
GTCATGGATACGACAACCCCCAGGTAAGGGCCTGGAATGGATTGGCTATATCTA
CTATTCAGGAAGCACGAACTACAATCCCAGCCTGAAGTCCCGAGTGACAATTTC
AGTAGATACCAGTAAAAACCAGTTCAGTCTTAAACTGTCAAGCGTGACAGCTGC
CGACACCGCTGTGTATTACTGCGTCTCACTGGTGTATTGTGGAGGGGATTGTTAT
AGCGGGTTCGATTATTGGGGACAGGGAACCCTGGTGACTGTATCTTCCGGCGGC
GGCGGCTCAGGGGGTGGCGGTAGTGGCGGTGGGGGTTCCGATATTCAACTGACA
CAATCCCCCAGCTCACTCAGCGCCAGCGTGGGGGACAGGGTTAGCTTTACCTGTC
AAGCCTCTCAGGATATAAATAACTTTCTGAACTGGTATCAACAGAAGCCTGGGA
AGGCGCCCAAACTCCTGATCTATGATGCGTCCAACCTGGAAACTGGCGTGCCTTC
ACGCTTTAGCGGCTCTGGCAGTGGTACAGACTTCACTTTTACCATCTCTTCACTTC
AGCCGGAGGACATCGCCACATATTACTGTCAACAGTACGGAAACTTGCCCTTTAC
TTTTGGAGGCGGCACCAAAGTTGAAATCAAAGGGCCGCTGCCCTGGATAACGA
AAAGAGCAATGGGACTATAATACATGTTAAAGGAAAACACCTGTGTCCATCTCC
 
CCTGTTCCCTGGACCGTCAAAGCCATTTTGGGTGCTCGTGGTTGTCGGTGGCGTT
CTCGCCTGTTATAGCTTGCTGGTGACAGTAGCCTTCATTATCTTTTGGGTGAGATC
CAAAAGAAGCCGCCTGCTCCATAGCGATTACATGAATATGACTCCACGCCGCCCT
GGCCCCACAAGGAAACACTACCAGCCTTACGCACCACCTAGAGATTTCGCTGCCT
ATCGGAGCCGAGTGAAATTTCTAGATCAGCTGATGCTCCCGCCTATCAGCAGGG
ACAGAATCAACTTTACAATGAGCTGAACCTGGGTCGCAGAGAAGAGTACGACGT
TTTGGACAAACGCCGGGGCCGAGATCCTGAGATGGGGGGAAGCCGAGAAGGA
AGAATCCTCAAGAAGGCCTGTACAACGAGCTTCAAAAAGACAAAATGGCTGAGG
CGTACTCTGAGATCGGCATGAAGGGCGAGCGGAGACGAGGCAAGGGTCACGAT
GGCTTGTATCAGGGCCTGAGTACAGCCACAAAGGACACCTATGACGCCCTCCAC
ATGCAGGCACTGCCCCCACGC
Anti-CLL-1 CAR AA (SEQ ID NO. 155) 
MALPVTALLLPLALLLHAARPQVQLQESGPGLVKPSETLSLTCTVSGGSISSYYWSWI
RQPPGKGLEWIGYIYYSGSTNYNPSLKSRVTISVDTSKNQFSLKLSSVTAADTAVYYC
VSLVYCGGDCYSGFDYWGQGTLVTVSSGGGGSGGGGSGGGGSDIQLTQSPSSLSAS
VGDRVSFTCQASQDINNFLNWYQQKPGKAPKLLIYDASNLETGVPSRFSGSGSGTDF
TFTISSLQPEDIATYYCQQYGNLPFTFGGGTKVEIKRAAALDNEKSNGTIIHVKGKHL
CPSPLFPGPSKPFWVLVVVGGVLACYSLLVTVAFIIFWVRSKRSRLLHSDYMNMTPR
RPGPTRKHYQPYAPPRDFAAYRSRVKFSRSADAPAYQQGQNQLYNELNLGRREEYD
VLDKRRGRDPEMGGKPRRKNPQEGLYNELQKDKMAEAYSEIGMKGERRRGKGHD
GLYQGLSTATKDTYDALHMQALPPR
Anti-BCMA CAR DNA (SEQ ID NO. 156) 
ATGGCACTCCCCGTAACTGCTCTGCTGCTGCCGTTGGCATTGCTCCTGCACGCCG
CACGCCCGCAGGTGCAGCTGGTGGAGTCTGGGGGAGGCGTGGTCCAGCCTGGGA
GGTCCCTGAGACTCTCCTGTGCAGCGTCTGGATTCACCTTCAGTAGCTATGGCAT
 
GCACTGGGTCCGCCAGGCTCCAGGCAAGGGGCTGGAGTGGGTGGCAGTTATATC
GTATGATGGAAGTAATAAATACTATGCAGACTCCGTGAAGGGCCGATTCACCAT
CTCCAGAGACAATTCCAAGAACACGCTGTATCTGCAAATGAACAGCCTGAGAGC
CGAGGACACGGCGGTGTACTACTGCGTCAAGGGGCCGTTGCAGGAGCCGCCATA
CGATTATGGAATGGACGTATGGGGCCAGGGAACAACTGTCACCGTCTCCTCAGG
GTCTACATCCGGCTCCGGGAAGCCCGGAAGTGGCGAAGGTAGTACAAAGGGGGA
AATAGTGATGACGCAGTCTCCAGCCACCCTGTCTGTGTCTCCAGGGGAAAGAGC
CACCCTCTCCTGCAGGGCCAGTCAGAGTGTTAGCAGCAACTTAGCCTGGTACCAG
CAGAAACCTGGCCAGGCTCCCAGGCTCCTCATCTATAGCGCATCCACCAGGGCC
ACTGGTATCCCAGCCAGGTTCAGTGGCAGTGGGTCTGGGACAGAGTTCACTCTCA
CCATCAGCAGCCTGCAGTCTGAAGATTTTGCAGTTTATTACTGTCAGCAGCACCA
CGTCTGGCCTCTCACTTTTGGCGGAGGGACCAAGGTTGAGATCAAACGGGCCGCT
GCCCTTGATAATGAAAAGTCAAACGGAACAATCATTCACGTGAAGGGCAAGCAC
CTCTGTCCGTCACCCTTGTTCCCTGGTCCATCCAAGCCATTCTGGGTGTTGGTCGT
AGTGGGTGGAGTCCTCGCTTGTTACTCTCTGCTCGTCACCGTGGCTTTTATAATCT
TCTGGGTTAGATCCAAAAGAAGCCGCCTGCTCCATAGCGATTACATGAATATGAC
TCCACGCCGCCCTGGCCCCACAAGGAAACACTACCAGCCTTACGCACCACCTAG
AGATTTCGCTGCCTATCGGAGCAGGGTGAAGTTTTCCAGATCTGCAGATGCACCA
GCGTATCAGCAGGGCCAGAACCAACTGTATAACGAGCTCAACCTGGGACGCAGG
GAAGAGTATGACGTTTTGGACAAGCGCAGAGGACGGGACCCTGAGATGGGTGGC
AAACCAAGACGAAAAAACCCCCAGGAGGGTCTCTATAATGAGCTGCAGAAGGAT
AAGATGGCTGAAGCCTATTCTGAAATAGGCATGAAAGGAGAGCGGAGAAGGGG
AAAAGGGCACGACGGTTTGTACCAGGGACTCAGCACTGCTACGAAGGATACTTA
TGACGCTCTCCACATGCAAGCCCTGCCACCTAGG
Anti-BCMA CAR AA (SEQ ID NO. 157) 
MALPVTALLLPLALLLHAARPQVQLVESGGGVVQPGRSLRLSCAASGFTFSSYGMH
WVRQAPGKGLEWVAVISYDGSNKYYADSVKGRFTISRDNSKNTLYLQMNSLRAED
TAVYYCVKGPLQEPPYDYGMDVWGQGTTVTVSSGSTSGSGKPGSGEGSTKGEIVMT
QSPATLSVSPGERATLSCRASQSVSSNLAWYQQKPGQAPRLLIYSASTRATGIPARFS
GSGSGTEFTLTISSLQSEDFAVYYCQQHHVWPLTFGGGTKVEIKRAAALDNEKSNGT
IIHVKGKHLCPSPLFPGPSKPFWVLVVVGGVLACYSLLVTVAFIIFWVRSKRSRLLHS
DYMNMTPRRPGPTRKHYQPYAPPRDFAAYRSRVKFSRSADAPAYQQGQNQLYNEL
NLGRREEYDVLDKRRGRDPEMGGKPRRKNPQEGLYNELQKDKMAEAYSEIGMKGE
RRRGKGHDGLYQGLSTATKDTYDALHMQALPPR
C185 E7₁₁₋₁₉ / HLA-A^{∗}02:01 Specific TCR DNA (SEQ ID NO. 158) 
atgggcaccagactgttcttttatgtggccctgtgtctgctgtggaccggccacatggatgccggaattacacagagccccagacacaa

agtgaccgagacaggcacccctgtgacactgagatgccaccagaccgagaaccaccgctacatgtactggtacagacaggatccag

gccacggcctgagactgatccactacagctacggcgtgaaggacaccgacaagggcgaagtgtctgacggctacagcgtgtccag

aagcaagaccgaggatttcctgctgaccctggaaagcgccacaagcagccagaccagcgtgtacttttgtgccatcagcggctacaa

gaacaccgaggcctttttcggccaaggcaccaggctgacagtggtggaagatctgaagaacgtgttcccacctgaggtggccgtgttt

gagccttctgaggccgagatcagccacacacagaaagccacactcgtgtgcctggccaccggcttttatcccgatcacgtggaactgt

cttggtgggtcaacggcaaagaggtgcacagcggcgttagcacagaccctcagcctctgaaagagcagcccgctctgaacgacag

cagatactgtctgagcagcagactgagagtgtccgccaccttctggcagaaccccagaaaccacttcagatgccaggtgcagttctac

ggcctgtccgagaatgacgagtggacccaggatagagccaagccagtgacacagattgtgtctgccgaagcctggggcagagccg

attgtggctttacaagcgagagctaccagcagggcgtgctgtctgccacaatcctgtatgagatcctgctgggcaaagccactctgtac

gctgtgctggtgtctgccctggtgctgatggccatggtcaagagaaaggacagcagaggcagagccaagagatctggcagcggcg

ccacaaactttagcctgctgaaacaggccggcgacgtggaagagaaccctggacctatgaagtccctgcgggtgctgctggttattct

gtggctgcagctgagctgggtttggagccagggacagcaagtgatgcagatccctcagtaccagcacgtgcaagaaggcgaggact

tcaccacctactgcaacagcagcacaaccctgagcaacatccagtggtacaagcagaggcctggcgggcaccctgtgtttctgatcc

agctggttaagagcggcgaagtgaagaagcagaagcggctgacctttcagttcggcgaggccaagaagaacagcagcctgcacatt
 
accgccacacagaccaccgacgtgggcacatatttttgcgctggcagagaaggcggcagcgagaagctggtttttggcaagggcac
caaactgaccgtgaatcccgacattcagaaccccgatccagccgtgtaccagctgagagacagcaagagcagcgacaagagcgtgt
gtctgttcaccgacttcgactcccagaccaatgtgtcccagagcaaggactccgacgtgtacatcaccgataagaccgtgctggacat
gcggagcatggacttcaagagcaatagcgccgtggcttggagcaacaagagcgactttgcctgcgccaacgccttcaacaacagca
tcatccccgaggacacattcttcccaagtcctgagagcagctgcgacgtgaagctggtggaaaagagcttcgagacagacaccaacc
tgaacttccagaacctgagcgtgatcggcttcagaatcctgctgctgaaggtggccggcttcaacctgctgatgactctgagactgtggt
ccagctgaattcggatccaagcttaggcctgctcgctttcttgctgtcccatttctattaaaggttcctttgttc
C185 E7₁₁₋₁₉ / HLA-A^{∗}02:01 Specific TCR AA (SEQ ID NO. 159) 
MGTRLFFYVALCLLWTGHMDAGITQSPRHKVTETGTPVTLRCHQTENHRYMYWYR
QDPGHGLRLIHYSYGVKDTDKGEVSDGYSVSRSKTEDFLLTLESATSSQTSVYFCAIS
GYKNTEAFFGQGTRLTVVEDLKNVFPPEVAVFEPSEAEISHTQKATLVCLATGFYPD
HVELSWWVNGKEVHSGVSTDPQPLKEQPALNDSRYCLSSRLRVSATFWQNPRNHFR
CQVQFYGLSENDEWTQDRAKPVTQIVSAEAWGRADCGFTSESYQQGVLSATILYEIL
LGKATLYAVLVSALVLMAMVKRKDSRGRAKRSGSGATNFSLLKQAGDVEENPGPM
KSLRVLLVILWLQLSWVWSQGQQVMQIPQYQHVQEGEDFTTYCNSSTTLSNIQWYK
QRPGGHPVFLIQLVKSGEVKKQKRLTFQFGEAKKNSSLHITATQTTDVGTYFCAGRE
GGSEKLVFGKGTKLTVNPDIQNPDPAVYQLRDSKSSDKSVCLFTDFDSQTNVSQSKD
SDVYITDKTVLDMRSMDFKSNSAVAWSNKSDFACANAFNNSIIPEDTFFPSPESSCDV
KLVEKSFETDTNLNFQNLSVIGFRILLLKVAGFNLLMTLRLWSS
E7₁₁₋₁₉ / HLA-A^{∗}02:01 Specific TCR DNA (SEQ ID NO. 160) 
atgggacctggattgctttgttgggccctgctgtgtctgcttggagctggacttgtggatgccggcgtgacacagtctcccacacacctg

atcaagaccagaggccagcaagtgaccctgagatgtagccctaagagcggccacgacaccgtgtcttggtatcagcaggctcttggc

cagggacctcagttcatcttccagtactacgaggaagaggaacggcagcggggcaacttccctgatagattctctggccatcagttcc

ccaactacagcagcgagctgaacgtgaacgctctgctgctgggcgatagcgccctgtatctgtgtgccagttctcttggttggagaggc

ggcagatacaacgagcagttctttggccctggcaccagactgaccgtgctggaagatctgaagaacgtgttcccacctgaggtggcc
 
gtgtttgagccttctgaggccgagatcagccacacacagaaagccacactcgtgtgcctggccaccggcttttatcccgatcacgtgg

aactgtcttggtgggtcaacggcaaagaggtgcacagcggcgttagcacagaccctcagcctctgaaagagcagcccgctctgaac

gacagcagatactgtctgagcagcagactgagagtgtccgccaccttctggcagaaccccagaaaccacttcagatgccaggtgcag

ttctacggcctgtccgagaatgacgagtggacccaggatagagccaagccagtgacacagattgtgtctgccgaagcctggggcag

agccgattgtggctttacaagcgagagctaccagcagggcgtgctgtctgccacaatcctgtatgagatcctgctgggcaaagccact

ctgtacgctgtgctggtgtctgccctggtgctgatggccatggtcaagagaaaggacagcagaggcagagccaagagatctggcag

cggcgccacaaactttagcctgctgaaacaggccggcgacgtggaagagaaccctggacctatgaagtccctgcgggtgctgctgg

ttattctgtggctgcagctgagctgggtttggagccagggacagaacatcgaccagcctaccgagatgacagccaccgaaggcgcc

atcgtgcagatcaattgcacctaccagaccagcggcttcaacggcctgttctggtatcaacagcatgccggcgaggcccctaccttcct

gagctataatgtgctggacggcctggaagaaaagggcagattcagcagcttcctgtccagaagcaagggctacagctacctgctgct

gaaagaactccagatgaaggacagcgcctcctacctgtgtgcctccgtggatggaaacaacagactggccttcggcaagggcaacc

aggtggtggtcatccccgacattcagaaccccgatccagccgtgtaccagctgagagacagcaagagcagcgacaagagcgtgtgt

ctgttcaccgacttcgactcccagaccaatgtgtcccagagcaaggactccgacgtgtacatcaccgataagaccgtgctggacatgc

ggagcatggacttcaagagcaatagcgccgtggcttggagcaacaagagcgactttgcctgcgccaacgccttcaacaacagcatca

tccccgaggacacattcttcccaagtcctgagagcagctgcgacgtgaagctggtggaaaagagcttcgagacagacaccaacctga

acttccagaacctgagcgtgatcggcttcagaatcctgctgctgaaggtggccggcttcaacctgctgatgactctgagactgtggtcc

agctgaattcggatccaagcttaggcctgctcgctttcttgctgtcccatttctattaaaggttcctttgttc
E7₁₁₋₁₉ / HLA-A^{∗}02:01 Specific TCR AA (SEQ ID NO. 161) 
MGPGLLCWALLCLLGAGLVDAGVTQSPTHLIKTRGQQVTLRCSPKSGHDTVSWYQ
QALGQGPQFIFQYYEEEERQRGNFPDRFSGHQFPNYSSELNVNALLLGDSALYLCAS
SLGWRGGRYNEQFFGPGTRLTVLEDLKNVFPPEVAVFEPSEAEISHTQKATLVCLAT
GFYPDHVELSWWVNGKEVHSGVSTDPQPLKEQPALNDSRYCLSSRLRVSATFWQNP
RNHFRCQVQFYGLSENDEWTQDRAKPVTQIVSAEAWGRADCGFTSESYQQGVLSAT
ILYEILLGKATLYAVLVSALVLMAMVKRKDSRGRAKRSGSGATNFSLLKQAGDVEE
NPGPMKSLRVLLVILWLQLSWVWSQGQNIDQPTEMTATEGAIVQINCTYQTSGFNG
 
LFWYQQHAGEAPTFLSYNVLDGLEEKGRFSSFLSRSKGYSYLLLKELQMKDSASYLC
ASVDGNNRLAFGKGNQVVVIPDIQNPDPAVYQLRDSKSSDKSVCLFTDFDSQTNVSQ
SKDSDVYITDKTVLDMRSMDFKSNSAVAWSNKSDFACANAFNNSIIPEDTFFPSPESS
CDVKLVEKSFETDTNLNFQNLSVIGFRILLLKVAGFNLLMTLRLWSS
Axi Cel (KTE-C19) Leader (CSF2RA) DNA (SEQ ID NO. 162)
   Atgcttctcctggtgacaagccttctgctctgtgagttaccacacccagcattcctcctgatccca
Axi Cel (KTE-C19) Leader (CSF2RA) AA (SEQ ID NO. 163)
   MLLLVTSLLLCELPHPAFLLIP
Axi Cel (KTE-C19) scFv heavy chain DNA (SEQ ID NO. 164) 
gacatccagatgacacagactacatcctccctgtctgcctctctgggagacagagtcaccatcagttgcagggcaagtcaggacatta

gtaaatatttaaattggtatcagcagaaaccagatggaactgttaaactcctgatctaccatacatcaagattacactcaggagtcccatc

aaggttcagtggcagtgggtctggaacagattattctctcaccattagcaacctggagcaagaagatattgccacttacttttgccaacag

ggtaatacgcttccgtacacgttcggaggggggactaagttggaaataaca
Axi Cel (KTE-C19) scFv heavy chain AA (SEQ ID NO. 165) 
DIQMTQTTSSLSASLGDRVTISCRASQDISKYLNWYQQKPDGTVKLLIYHTSRLHSGV
PSRFSGSGSGTDYSLTISNLEQEDIATYFCQQGNTLPYTFGGGTKLEIT
Axi Cel (KTE-C19) Linker (Whitlow) DNA (SEQ ID NO. 166) ggctccacctctggatccggcaagcccggatctggcgagggatccaccaagggc
Axi Cel (KTE-C 19) Linker (Whitlow) AA (SEQ ID NO. 167)
   GSTSGSGKPGSGEGSTKG
Axi Cel (KTE-C 19) scFv light chain DNA (SEQ ID NO. 168) 
gaggtgaaactgcaggagtcaggacctggcctggtggcgccctcacagagcctgtccgtcacatgcactgtctcaggggtctcattac

ccgactatggtgtaagctggattcgccagcctccacgaaagggtctggagtggctgggagtaatatggggtagtgaaaccacatacta

taattcagctctcaaatccagactgaccatcatcaaggacaactccaagagccaagttttcttaaaaatgaacagtctgcaaactgatga

cacagccatttactactgtgccaaacattattactacggtggtagctatgctatggactactggggtcaaggaacctcagtcaccgtctcc
 tca
Axi Cel (KTE-C19) scFv light chain AA (SEQ ID NO. 169) 
EVKLQESGPGLVAPSQSLSVTCTVSGVSLPDYGVSWIRQPPRKGLEWLGVIWGSETT
YYNSALKSRLTIIKDNSKSQVFLKMNSLQTDDTAIYYCAKHYYYGGSYAMDYWGQ
GTSVTVSS
Axi Cel (KTE-C19) Minispacer DNA (SEQ ID NO. 170)
   gcggccgca
Axi Cel (KTE-C19) Minispacer AA (SEQ ID NO. 171)
   AAA
Axi Cel (KTE-C 19) CD28 spacer (extracellular / TM region of CD28) DNA (SEQ ID NO. 172) 
attgaagttatgtatcctcctccttacctagacaatgagaagagcaatggaaccattatccatgtgaaagggaaacacctttgtccaagtc

ccctatttcccggaccttctaagcccttttgggtgctggtggtggttgggggagtcctggcttgctatagcttgctagtaacagtggccttt

attattttctgggtg
Axi Cel (KTE-C 19) CD28 spacer (extracellular / TM region of CD28) AA (SEQ ID NO. 173) 
IEVMYPPPYLDNEKSNGTIIHVKGKHLCPSPLFPGPSKPFWVLVVVGGVLACYSLLVT

VAFIIFWV
Axi Cel (KTE-C19) CD28 costimulatory (intracellular region of CD28) DNA (SEQ ID NO. 174) 
aggagtaagaggagcaggctcctgcacagtgactacatgaacatgactccccgccgccccgggcccacccgcaagcattaccagc

cctatgccccaccacgcgacttcgcagcctatcgctcc
Axi Cel (KTE-C 19) CD28 costimulatory (intracellular region of CD28) AA (SEQ ID NO. 175)
   RSKRSRLLHSDYMNMTPRRPGPTRKHYQPYAPPRDFAAYRS
Axi Cel (KTE-C19) CD3 zeta DNA (SEQ ID NO. 176) 
agagtgaagttcagcaggagcgcagacgcccccgcgtaccagcagggccagaaccagctctataacgagctcaatctaggacgaa

gagaggagtacgatgttttggacaagagacgtggccgggaccctgagatggggggaaagccgagaaggaagaaccctcaggaag

gcctgtacaatgaactgcagaaagataagatggcggaggcctacagtgagattgggatgaaaggcgagcgccggaggggcaagg

ggcacgatggcctttaccagggtctcagtacagccaccaaggacacctacgacgcccttcacatgcaggccctgccccctcgc
Axi Cel (KTE-C19) CD3 zeta AA (SEQ ID NO. 177) 
RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQAL
PPR
Axi Cel (KTE-C19) VL CDR1 (Chothia) (SEQ ID NO. 178)
   RASQDISKYLN
Axi Cel (KTE-C 19) VL CDR2 (Chothia) (SEQ ID NO. 179)
   HTSRLHS
Axi Cel (KTE-C 19) VL CDR3 (Chothia) (SEQ ID NO. 180)
   QQGNTLPYT
Axi Cel (KTE-C19) VH CDR1 (Chothia) (SEQ ID NO. 181)
   GVSLPDY
Axi Cel (KTE-C 19) VH CDR2 (Chothia) (SEQ ID NO. 182)
   WGSET
Axi Cel (KTE-C19) VH CDR3 (Chothia) (SEQ ID NO. 183)
   HYYYGGSYAMDY
Axi Cel (KTE-C19) VL CDR1 (Kabat) (SEQ ID NO. 184)
   RASQDISKYLN
Axi Cel (KTE-C19) VL CDR2 (Kabat) (SEQ ID NO. 185)
   HTSRLHS
Axi Cel (KTE-C19) VL CDR3 (Kabat) (SEQ ID NO. 186)
   QQGNTLPYT
Axi Cel (KTE-C19) VH CDR1 (Kabat) (SEQ ID NO. 187)
   DYGVS
Axi Cel (KTE-C19) VH CDR2 (Kabat) (SEQ ID NO. 188)
   VIWGSETTYYNSALKS
Axi Cel (KTE-C19) VH CDR3 (Kabat) (SEQ ID NO. 189)
   HYYYGGSYAMDY
Humanized anti-CD 19 CAR CD8 Leader DNA (SEQ ID NO. 190) 
ATGGCACTCCCCGTAACTGCTCTGCTGCTGCCGTTGGCATTGCTCCTGCACGCCG

CACGCCCG
Humanized anti-CD19 CAR CD8 Leader AA (SEQ ID NO. 191)
   MALPVTALLLPLALLLHAARP
Humanized anti-CD19 CAR scFv heavy chain DNA (SEQ ID NO. 192) 
GATATTCAAATGACCCAGTCCCCGTCCTCCCTGAGTGCCTCCGTCGGTGACCGTG
TTACGATTACCTGCCGTGCGAGCCAAGACATCTCTAAATACCTGAACTGGTATCA
GCAAAAACCGGATCAGGCACCGAAACTGCTGATCAAACATACCTCACGTCTGCA
CTCGGGTGTGCCGAGCCGCTTTAGTGGTTCCGGCTCAGGTACCGATTACACCCTG
ACGATCAGCTCTCTGCAGCCGGAAGACTTTGCCACGTATTACTGCCAGCAAGGTA
ATACCCTGCCGTATACGTTCGGCCAAGGTACCAAACTGGAAATCAAA
Humanized anti-CD19 CAR scFv heavy chain AA (SEQ ID NO. 193) 
DIQMTQSPSSLSASVGDRVTITCRASQDISKYLNWYQQKPDQAPKLLIKHTSRLHSGV
PSRFSGSGSGTDYTLTISSLQPEDFATYYCQQGNTLPYTFGQGTKLEIK
Humanized anti-CD 19 CAR Linker (Whitlow) DNA (SEQ ID NO. 194)
   GGCTCGACGAGCGGCTCTGGTAAACCGGGCTCTGGTGAAGGCAGTACCAAAGGT
Humanized anti-CD 19 CAR Linker (Whitlow) AA (SEQ ID NO. 195)
   GSTSGSGKPGSGEGSTKG
Humanized anti-CD 19 CAR scFv light chain DNA (SEQ ID NO. 196) 
GAAGTGCAGCTGGTTGAAAGCGGTGGTGGTCTGGTTCAACCGGGTCGTTCCCTGC
GTCTGTCATGTACGGCGAGTGGTGTCTCCCTGCCGGACTATGGCGTGTCCTGGAT
TCGTCAGCCGCCGGGTAAAGGCCTGGAATGGATTGGTGTCATCTGGGGCAGTGA
AACCACGTATTACAACTCGGCCCTGAAAAGCCGTTTCACCATCTCTCGCGATAAC
AGTAAAAATACGCTGTACCTGCAGATGAATAGCCTGCGCGGAAGACACCGCC
GTTTACTACTGCGCAAAACATTACTACTACGGTGGCAGCTATGCTATGGATTACT
GGGGTCAAGGCACGCTGGTCACCGTTTCGTCA
Humanized anti-CD 19 CAR scFv light chain AA (SEQ ID NO. 197) 
EVQLVESGGGLVQPGRSLRLSCTASGVSLPDYGVSWIRQPPGKGLEWIGVIWGSETT
YYNSALKSRFTISRDNSKNTLYLQMNSLRAEDTAVYYCAKHYYYGGSYAMDYWG
QGTLVTVSS
Humanized anti-CD19 CAR Minispacer DNA (SEQ ID NO. 198)
   GCCGCTGCC
Humanized anti-CD19 CAR Minispacer AA (SEQ ID NO. 199)
   AAA
Humanized anti-CD 19 CAR CD28T spacer (extracellular / TM region of CD28) DNA (SEQ ID NO. 200) 
CTTGATAATGAAAAGTCAAACGGAACAATCATTCACGTGAAGGGCAAGCACCTC
TGTCCGTCACCCTTGTTCCCTGGTCCATCCAAGCCATTCTGGGTGTTGGTCGTAGT
GGGTGGAGTCCTCGCTTGTTACTCTCTGCTCGTCACCGTGGCTTTTATAATCTTCT
GGGTT
Humanized anti-CD19 CAR CD28T spacer (extra / TM region of CD28) AA (SEQ ID NO. 201)
   LDNEKSNGTIIHVKGKHLCPSPLFPGPSKPFWVLVVVGGVLACYSLLVTVAFIIFWV
Humanized anti-CD 19 CAR CD28 costiminulatory (intracellular region of CD28) DNA (SEQ ID NO. 202) 
AGATCCAAAAGAAGCCGCCTGCTCCATAGCGATTACATGAATATGACTCCACGC
CGCCCTGGCCCCACAAGGAAACACTACCAGCCTTACGCACCACCTAGAGATTTC
GCTGCCTATCGGAGC
Humanized anti-CD 19 CAR costimulatory (intracellular region of CD28) AA (SEQ ID NO. 203)
   RSKRSRLLHSDYMNMTPRRPGPTRKHYQPYAPPRDFAAYRS
Humanized anti-CD 19 CAR CD3 zeta DNA (SEQ ID NO. 204) 
AGGGTGAAGTTTTCCAGATCTGCAGATGCACCAGCGTATCAGCAGGGCCAGAAC
CAACTGTATAACGAGCTCAACCTGGGACGCAGGGAAGAGTATGACGTTTTGGAC
AAGCGCAGAGGACGGGACCCTGAGATGGGTGGCAAACCAAGACGAAAAAACCC
CCAGGAGGGTCTCTATAATGAGCTGCAGAAGGATAAGATGGCTGAAGCCTATTC
TGAAATAGGCATGAAAGGAGAGCGGAGAAGGGGAAAAGGGCACGACGGTTTGT
ACCAGGGACTCAGCACTGCTACGAAGGATACTTATGACGCTCTCCACATGCAAG
CCCTGCCAcctagg
Humanized anti-CD 19 CAR CD3 zeta AA (SEQ ID NO. 205) 
RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQAL
PPR
Humanized anti-CD 19 CAR VL fully human anti-CD 19 CDR1 (Chothia) (SEQ ID NO. 206)
   RASQSVSSSYLA
Humanized anti-CD 19 CAR VL fully human anti-CD 19 CDR2 (Chothia) (SEQ ID NO. 207)
   GASSRAT
Humanized anti-CD 19 CAR VL fully human anti-CD 19 CDR3 (Chothia) (SEQ ID NO. 208)
   QQYGSSRFT
Humanized anti-CD 19 CAR VH fully human anti-CD 19 CDR1 (Chothia) (SEQ ID NO. 209)
   GGTFSSY
Humanized anti-CD 19 CAR VH fully human anti-CD 19 CDR2 (Chothia) (SEQ ID NO. 210)
   IPIFGT
Humanized anti-CD 19 CAR VH fully human anti-CD 19 CDR3 (Chothia) (SEQ ID NO. 211)
   EAVAADWLDP
Humanized anti-CD19 CAR VL fully human anti-CD19 CDR1 (Kabat) (SEQ ID NO. 212)
   RASQSVSSSYLA
Humanized anti-CD 19 CAR VL fully human anti-CD 19 CDR2 (Kabat) (SEQ ID NO. 213)
   GASSRAT
Humanized anti-CD 19 CAR VL fully human anti-CD 19 CDR3 (Kabat) (SEQ ID NO. 214)
   QQYGSSRFT
Humanized anti-CD19 CAR VH fully human anti-CD19 CDR1 (Kabat) (SEQ ID NO. 215)
   SYAIS
Humanized anti-CD 19 CAR VH fully human anti-CD 19 CDR2 (Kabat) (SEQ ID NO. 216)
   GIIPIFGTTNYAQQFQG
Humanized anti-CD 19 CAR VH fully human anti-CD 19 CDR3 (Kabat) (SEQ ID NO. 217)
   EAVAADWLDP
Fully human anti-CD19 CAR Leader (CD8a) DNA (SEQ ID NO. 218) Atggccctgcctgtgacagctctgctgctgcccctggccctgctgctgcatgccgccagacct
Fully human anti-CD19 CAR Leader (CD8a) AA (SEQ ID NO. 219)
   MALPVTALLLPLALLLHAARP
Fully human anti-CD19 CAR scFv light chain DNA (SEQ ID NO. 220) 
gagatcgtgctgacccagtctcccggcaccctgtctctcagcccaggagagagagccaccctgagctgcagagccagccagagcgt

gtccagcagctacctggcctggtatcagcagaagcccggacaggcccccagactgctgatctacggcgccagctctagagccaccg

gcatccccgacagattcagcggcagcggcagtggcaccgacttcaccctgaccatcagcagactggaacccgaggacttcgccgtg

tactactgccagcagtacggcagcagccggttcaccttcggccctggcaccaaggtggacatcaag
Fully human anti-CD19 CAR scFv light chain AA (SEQ ID NO. 221) 
EIVLTQSPGTLSLSPGERATLSCRASQSVSSSYLAWYQQKPGQAPRLLIYGASSRATGI

PDRFSGSGSGTDFTLTISRLEPEDFAVYYCQQYGSSRFTFGPGTKVDIK
Fully human anti-CD19 CAR Linker (Whitlow) DNA (SEQ ID NO. 222) ggcagcacctccggcagcggcaagcctggctctggcgagggctctaccaagggc
Fully human anti-CD19 CAR Linker (Whitlow) AA (SEQ ID NO. 223)
   GSTSGSGKPGSGEGSTKG
Fully human anti-CD19 CAR scFv heavy chain DNA (SEQ ID NO. 224) 
caggtgcagctggtgcagtctggcgccgaagtgaagaaacccggctctagcgtgaaggtgtcctgcaaggacagcggcggcacctt

cagcagctacgccatcagctgggtgcgccaggccccaggacaggggctggaatggatgggcggcatcatccccatcttcggcacc

accaactacgcccagcagttccagggcagagtgaccatcaccgccgacgagagcaccagcaccgcctacatggaactgagcagcc

tgcggagcgaggacacagccgtgtattactgtgcccgcgaggccgtggccgccgactggctggatccttggggacagggcaccct

ggtgacagtgtccagc
Fully human anti-CD19 CAR scFv heavy chain AA (SEQ ID NO. 225) 
QVQLVQSGAEVKKPGSSVKVSCKDSGGTFSSYAISWVRQAPGQGLEWMGGIIPIFGT
TNYAQQFQGRVTITADESTSTAYMELSSLRSEDTAVYYCAREAVAADWLDPWGQG
TLVTVSS
Fully human anti-CD19 CAR CD8a spacer and TM region DNA (SEQ ID NO. 226) 
ttcgtgcccgtgttcctgcccgccaagcctaccaccacccctgcccctagacctcccaccccagccccaacaatcgccagccagcct

ctgtccctgcggcccgaagcctgtagacctgctgccggcggagccgtgcacaccagaggcctggacttcgcctgcgatatctacatc

tgggcccctctggccggcacctgtggcgtgctgctgctgagcctggtgatcaccctgtactgcaaccaccggaac
Fully human anti-CD19 CAR CD8a spacer and TM region AA (SEQ ID NO. 227) 
FVPVFLPAKPTTTPAPRPPTPAPTIASQPLSLRPEACRPAAGGAVHTRGLDFACDIYIW
APLAGTCGVLLLSLVITLYCNHRN
Fully human anti-CD19 CAR CD28 costim. DNA (SEQ ID NO. 228) 
agaagcaagcggagccggctgctgcacagcgactacatgaacatgaccccaagacggcctggccccacccggaagcactaccag

ccttacgcccctcccagagacttcgccgcctaccggtcc
Fully human anti-CD19 CAR CD28 costim. AA (SEQ ID NO. 229)
   RSKRSRLLHSDYMNMTPRRPGPTRKHYQPYAPPRDFAAYRS
Fully human anti-CD19 CAR CD3 zeta DNA (SEQ ID NO. 230) agagtgaagttcagcagatccgccgacgcccctgcctaccagcagggacagaaccagctgtacaacgagctgaacctgggcagac 
gggaagagtacgacgtgctggacaagcggagaggccgggaccccgagatgggcggaaagcccagacggaagaacccccagga

aggcctgtataacgaactgcagaaagacaagatggccgaggcctacagcgagatcggcatgaagggcgagcggaggcgcggca

agggccacgatggcctgtaccagggcctgagcaccgccaccaaggacacctacgacgccctgcacatgcaggccctgccccccca

ga
Fully human anti-CD19 CAR CD3 zeta AA (SEQ ID NO. 231) 
RVKFSRSADAPAYQQGQNQLYNELNLGRREEYDVLDKRRGRDPEMGGKPRRKNPQ

EGLYNELQKDKMAEAYSEIGMKGERRRGKGHDGLYQGLSTATKDTYDALHMQAL

PPR
MAGE A3 / A6 TCR Construct - Variable alpha chain - AV38-2 DNA (SEQ ID NO. 232) 
atggcatgccctggcttcctgtgggcacttgtgatctccacctgtcttgaatttagcatggctcagacagtcactcagtctcaaccagaga

tgtctgtgcaggaggcagagaccgtgaccctgagctgcacatatgacaccagtgagagtgattattatttattctggtacaagcagcctc

ccagcaggcagatgattctcgttattcgccaagaagcttataagcaacagaatgcaacagagaatcgtttctctgtgaacttccagaaag

cagccaaatccttcagtctcaagatctcagactcacagctgggggatgccgcgatgtatttctgtgct
MAGE A3 / A6 TCR Construct - Variable alpha chain - AV38-2 AA (SEQ ID NO. 233) 
M A C P G F L W A L V I S T C L E F S M A Q T V T Q S Q P E M S V Q E A E T V T L

S C T Y D T S E S D Y Y L F W Y K Q P P S R Q M I L V I R Q E A Y K Q Q N A T E N

R F S V N F Q K A A K S F S L K I S D S Q L G D A A M Y F C A L R S S G T Y K Y I F

G T G T R L K V L A N
MAGE A3 / A6 TCR Construct - TRAJ40 DNA (SEQ ID NO. 234) ctccggagctcaggaacctacaaatacatctttggaacaggcaccaggctgaaggttttagcaaat
MAGE A3 / A6 TCR Construct - Murine constant alpha - Murine CA DNA (SEQ ID NO. 235) atccagaaccctgaacctgctgtgtaccagttaaaagatcctcggtctcaggacagcaccctctgcctgttcaccgactttgactcccaa 
atcaatgtgccgaaaaccatggaatctggaacgttcatcactgacaaaactgtgctggacatgaaagctatggattccaagagcaatgg

ggccattgcctggagcaaccagacaagcttcacctgccaagatatcttcaaagagaccaacgccacctaccccagttcagacgttccc

tgtgatgccacgttgactgagaaaagctttgaaacagatatgaacctaaactttcaaaacctgtcagttatgggactccgaatcctcctgc

tgaaagtagccggatttaacctgctcatgacgctgaggctgtggtccagt
MAGE A3 / A6 TCR Construct - Murine constant alpha - Murine CA AA (SEQ ID NO. 236) 
I Q N P E P A V Y Q L K D P R S Q D S T L C L F T D F D S Q I N V P K T M E S G T F
I T D K T V L D M K A M D S K S N G A I A W S N Q T S F T C Q D I F K E T N A T Y
P S S D V P C D A T L T E K S F E T D M N L N F Q N L S V M G L R I L L L K V A G F
N L L M T L R L W S S
MAGE A3 / A6 TCR Construct - Furin-SG SG-P2A DNA (SEQ ID NO. 237) cgggccaagcggtccggatccggagccaccaacttcagcctgctgaagcaggccggcgacgtggaggagaaccccggcccc
MAGE A3 / A6 TCR Construct - Furin-SG SG-P2A AA (SEQ ID NO. 238)
   RAKRSGSGATNFSLLKQAGDVEENPGP
MAGE A3 / A6 TCR Construct - Variable beta chain - BV7-2 DNA (SEQ ID NO. 239) 
atgggcaccaggctcctcttctgggtggccttctgtctcctgggggcagatcacacaggagctggagtctcccagtcccccagtaaca

aggtcacagagaagggaaaggatgtagagctcaggtgtgatccaatttcaggtcatactgccctttactggtaccgacagagcctggg

gcagggcctggagtttttaatttacttccaaggcaacagtgcaccagacaaatcagggctgcccagtgatcgcttctctgcagagagga

ctgggggatccgtctccactctgacgatccagcgcacacagcaggaggactcggccgtgtatctctgtgccagca
MAGE A3 / A6 TCR Construct - Variable beta chain - BV7-2 AA (SEQ ID NO. 240) 
M G T R L L F W V A F C L L G A D H T G A G V S Q S P S N K V T E K G K D V E L

R C D P I S G H T A L Y W Y R Q S L G Q G L E F L I Y F Q G N S A P D K S G L P S D

R F S A E R T G G S V S T L T I Q R T Q Q E D S A V Y L C A S I R T G P F F S G N T I
 Y F G E G S W L T V V E
MAGE A3 / A6 TCR Construct - TRBJ1-3 DNA (SEQ ID NO. 241) tccggacagggccttttttctctggaaacaccatatattttggagagggaagttggctcactgttgtagag
MAGE A3 / A6 TCR Construct - Murine constant beta - Murine CB1 DNA (SEQ ID NO. 242) 
gacctgagaaacgtgaccccacccaaggtctccttgtttgagccatcaaaagcagagattgcaaacaaacaaaaggctaccctcgtgt

gcttggccaggggcttcttccctgaccacgtggagctgagctggtgggtgaatggcaaggaggtccacagtggggtcagcacggac

cctcaggcctacaaggagagcaattatagctactgcctgagcagccgcctgagggtctctgctaccttctggcacaatcctcgaaacc

acttccgctgccaagtgcagttccatgggctttcagaggaggacaagtggccagagggctcacccaaacctgtcacacagaacatca

gtgcagaggcctggggccgagcagactgtggaatcacttcagcatcctatcatcaggggggttctgtctgcaaccatcctctatgagatc

ctactggggaaggccaccctatatgctgtgctggtcagtggcctggtgctgatggctatggtcaaaagaaagaactcatga
MAGE A3 / A6 TCR Construct - Murine constant beta - Murine CB1 AA (SEQ ID NO. 243) 
D L R N V T P P K V S L F E P S K A E I A N K Q K A T L V C L A R G F F P D H V E L

S W W V N G K E V H S G V S T D P Q A Y K E S N Y S Y C L S S R L R V S A T F W

H N P R N H F R C Q V Q F H G L S E E D K W P E G S P K P V T Q N I S A E A W G R

A D C G I T S A S Y H Q G V L S A T I L Y E I L L G K A T L Y A V L V S G L V L M A

M V K R K N S Stop
MAGE A3 / A6 TCR Va CDR1 (SEQ ID NO. 244)
   TYDTSESDYYLF
MAGE A3 / A6 TCR Va CDR2 (SEQ ID NO. 245)
   QEAYKQQ
MAGE A3 / A6 TCR Va CDR3 (SEQ ID NO. 246)
   ALRSSGTYKYI
MAGE A3 / A6 TCR Vb CDR1 (SEQ ID NO. 247)
   DPISGHTALY
MAGE A3 / A6 TCR Vb CDR2 (SEQ ID NO. 248)
   FQGNSAPDKSG
MAGE A3 / A6 TCR Vb CDR3 (SEQ ID NO. 249)
   ASIRTGPFFSGNTIY
Anti-CLL-1 CAR CD8 Leader DNA (SEQ ID NO. 250) 
ATGGCACTCCCCGTAACTGCTCTGCTGCTGCCGTTGGCATTGCTCCTGCACGCCG

CACGCCCG
Anti-CLL-1 CAR CD8 Leader AA (SEQ ID NO. 251)
   MALPVTALLLPLALLLHAARP
Anti-CLL-1 CAR scFv heavy chain DNA (SEQ ID NO. 252) 
CAGGTCCAACTGCAAGAAAGCGGACCCGGACTGGTGAAGCCTTCTGAGACACTT
AGTCTGACGTGCACGGTCAGTGGCGGCTCCATCTCCTCCTATTATTGGTCATGGA
TACGACAACCCCCAGGTAAGGGCCTGGAATGGATTGGCTATATCTACTATTCAGG
AAGCACGAACTACAATCCCAGCCTGAAGTCCCGAGTGACAATTTCAGTAGATAC
CAGTAAAAACCAGTTCAGTCTTAAACTGTCAAGCGTGACAGCTGCCGACACCGC
TGTGTATTACTGCGTCTCACTGGTGTATTGTGGAGGGGATTGTTATAGCGGGTTC
GATTATTGGGGACAGGGAACCCTGGTGACTGTATCTTCC
Anti-CLL-1 CAR scFv heavy chain AA (SEQ ID NO. 253) 
QVQLQESGPGLVKPSETLSLTCTVSGGSISSYYWSWIRQPPGKGLEWIGYIYYSGSTN
YNPSLKSRVTISVDTSKNQFSLKLSSVTAADTAVYYCVSLVYCGGDCYSGFDYWGQ
GTLVTVSS
Anti-CLL-1 CAR G4S linker DNA (SEQ ID NO. 254)
   GGCGGCGGCGGCTCAGGGGGTGGCGGTAGTGGCGGTGGGGGTTCC
Anti-CLL-1 CAR G4S linker AA (SEQ ID NO. 255)
   GGGGSGGGGSGGGGS
Anti-CLL-1 CAR scFv light chain DNA (SEQ ID NO. 256) 
GATATTCAACTGACACAATCCCCCAGCTCACTCAGCGCCAGCGTGGGGGACAGG
GTTAGCTTTACCTGTCAAGCCTCTCAGGATATAAATAACTTTCTGAACTGGTATC
AACAGAAGCCTGGGAAGGCGCCCAAACTCCTGATCTATGATGCGTCCAACCTGG
AAACTGGCGTGCCTTCACGCTTTAGCGGCTCTGGCAGTGGTACAGACTTCACTTT
TACCATCTCTTCACTTCAGCCGGAGGACATCGCCACATATTACTGTCAACAGTAC
GGAAACTTGCCCTTTACTTTTGGAGGCGGCACCAAAGTTGAAATCAAAAGG
Anti-CLL-1 CAR scFv light chain AA (SEQ ID NO. 257) 
DIQLTQSPSSLSASVGDRVSFTCQASQDINNFLNWYQQKPGKAPKLLIYDASNLETGV

PSRFSGSGSGTDFTFTISSLQPEDIATYYCQQYGNLPFTFGGGTKVEIKR
Anti-CLL-1 CAR Minispacer DNA (SEQ ID NO. 258)
   GCCGCTGCC
Anti-CLL-1 CAR Minispacer AA (SEQ ID NO. 259)
   AAA
Anti-CLL-1 CAR CD28T (extracellular / TM region of CD28) DNA (SEQ ID NO. 260) 
CTGGATAACGAAAAGAGCAATGGGACTATAATACATGTTAAAGGAAAACACCTG
TGTCCATCTCCCCTGTTCCCTGGACCGTCAAAGCCATTTGGGTGCTCGTGGTTGT
CGGTGGCGTTCTCGCCTGTTATAGCTTGCTGGTGACAGTAGCCTTCATTATCTTTT
GGGTG
Anti-CLL-1 CAR CD28T (extracellular / TM region of CD28) AA (SEQ ID NO. 261)
   LDNEKSNGTIIHVKGKHLCPSPLFPGPSKPFWVLVVVGGVLACYSLLVTVAFIIFWV
Anti-CLL-1 CAR CD28 (intracellular costimulatory region of CD28) DNA (SEQ ID NO. 262) 
AGATCCAAAAGAAGCCGCCTGCTCCATAGCGATTACATGAATATGACTCCACGC
CGCCCTGGCCCCACAAGGAAACACTACCAGCCTTACGCACCACCTAGAGATTTC
GCTGCCTATCGGAGC
Anti-CLL-1 CAR CD28 (intracellular costimulatory region of CD28) AA (SEQ ID NO. 263)
   RSKRSRLLHSDYMNMTPRRPGPTRKHYQPYAPPRDFAAYRS
Anti-CLL-1 CAR CD3 zeta DNA (SEQ ID NO. 264) 
CGAGTGAAATTTTCTAGATCAGCTGATGCTCCCGCCTATCAGCAGGGACAGAATC
AACTTTACAATGAGCTGAACCTGGGTCGCAGAGAAGAGTACGACGTTTTGGACA
AACGCCGGGGCCGAGATCCTGAGATGGGGGGAAGCCGAGAAGGAAGAATCCT
CAAGAAGGCCTGTACAACGAGCTTCAAAAAGACAAAATGGCTGAGGCGTACTCT
GAGATCGGCATGAAGGGCGAGCGGAGACGAGGCAAGGGTCACGATGGCTTGTA
TCAGGGCCTGAGTACAGCCACAAAGGACACCTATGACGCCCTCCACATGCAGGC
ACTGCCCCCACGC
Anti-CLL-1 CAR CD3 zeta AA (SEQ ID NO. 265) 
RVKFSRSADAPAYQQGQNQLYNELNLGRREEYDVLDKRRGRDPEMGGKPRRKNPQ

EGLYNELQKDKMAEAYSEIGMKGERRRGKGHDGLYQGLSTATKDTYDALHMQAL

PPR
CLL scFv (24C1) VL CDR1 (SEQ ID NO. 266)
   QASQDINNFLN
CLL scFv (24C1) VL CDR2 (SEQ ID NO. 267)
   DASNLET
CLL scFv (24C1) VL CDR3 (SEQ ID NO. 268)
   QQYGNLPFT
CLL scFv (24C1) VH CDR1 (SEQ ID NO. 269)
   GGSISSY
CLL scFv (24C1) VH CDR2 (SEQ ID NO. 270)
   YYSGS
CLL scFv (24C1) VH CDR3 (SEQ ID NO. 271)
   LVYCGGDCYSGFDY
Anti-BCMA CAR Leader (CD8a) DNA (SEQ ID NO. 272) 
ATGGCACTCCCCGTAACTGCTCTGCTGCTGCCGTTGGCATTGCTCCTGCACGCCG

CACGCCCG
Anti-BCMA CAR Leader (CD8a) AA (SEQ ID NO. 273)
   MALPVTALLLPLALLLHAARP
Anti-BCMA CAR scFv heavy chain DNA (SEQ ID NO. 274) 
CAGGTGCAGCTGGTGGAGTCTGGGGGAGGCGTGGTCCAGCCTGGGAGGTCCCTG
AGACTCTCCTGTGCAGCGTCTGGATTCACCTTCAGTAGCTATGGCATGCACTGGG
TCCGCCAGGCTCCAGGCAAGGGGCTGGAGTGGGTGGCAGTTATATCGTATGATG
GAAGTAATAAATACTATGCAGACTCCGTGAAGGGCCGATTCACCATCTCCAGAG
ACAATTCCAAGAACACGCTGTATCTGCAAATGAACAGCCTGAGAGCCGAGGACA
CGGCGGTGTACTACTGCGTCAAGGGGCCGTTGCAGGAGCCGCCATACGATTATG
GAATGGACGTATGGGGCCAGGGAACAACTGTCACCGTCTCCTCA
Anti-BCMA CAR scFv heavy chain AA (SEQ ID NO. 275) 
QVQLVESGGGVVQPGRSLRLSCAASGFTFSSYGMHWVRQAPGKGLEWVAVISYDG
SNKYYADSVKGRFTISRDNSKNTLYLQMNSLRAEDTAVYYCVKGPLQEPPYDYGM
DVWGQGTTVTVSS
Anti-BCMA CAR Linker (Whitlow) DNA (SEQ ID NO. 276)
   GGGTCTACATCCGGCTCCGGGAAGCCCGGAAGTGGCGAAGGTAGTACAAAGGGG
Anti-BCMA CAR Linker (Whitlow) AA (SEQ ID NO. 277)
   GSTSGSGKPGSGEGSTKG
Anti-BCMA CAR scFv light chain DNA (SEQ ID NO. 278) 
GAAATAGTGATGACGCAGTCTCCAGCCACCCTGTCTGTGTCTCCAGGGGAAAGA
GCCACCCTCTCCTGCAGGGCCAGTCAGAGTGTTAGCAGCAACTTAGCCTGGTACC
AGCAGAAACCTGGCCAGGCTCCCAGGCTCCTCATCTATAGCGCATCCACCAGGG
CCACTGGTATCCCAGCCAGGTTCAGTGGCAGTGGGTCTGGGACAGAGTTCACTCT
CACCATCAGCAGCCTGCAGTCTGAAGATTTTGCAGTTTATTACTGTCAGCAGCAC
CACGTCTGGCCTCTCACTTTTGGCGGAGGGACCAAGGTTGAGATCAAACGG
Anti-BCMA CAR scFv light chain AA (SEQ ID NO. 279) 
EIVMTQSPATLSVSPGERATLSCRASQSVSSNLAWYQQKPGQAPRLLIYSASTRATGI

PARFSGSGSGTEFTLTISSLQSEDFAVYYCQQHHVWPLTFGGGTKVEIKR
Anti-BCMA CAR Minispacer DNA (SEQ ID NO. 280)
   GCCGCTGCC
Anti-BCMA CAR Minispacer AA (SEQ ID NO. 281)
   AAA
Anti-BCMA CAR CD28T spacer DNA (SEQ ID NO. 282) 
CTTGATAATGAAAAGTCAAACGGAACAATCATTCACGTGAAGGGCAAGCACCTC
TGTCCGTCACCCTTGTTCCCTGGTCCATCCAAGCCATTCTGGGTGTTGGTCGTAGT
GGGTGGAGTCCTCGCTTGTTACTCTCTGCTCGTCACCGTGGCTTTTATAATCTTCT
GGGTT
Anti-BCMA CAR CD28T spacer AA (SEQ ID NO. 283)
   LDNEKSNGTIIHVKGKHLCPSPLFPGPSKPFWVLVVVGGVLACYSLLVTVAFIIFWV
Anti-BCMA CAR CD28 costimulatory region DNA (SEQ ID NO. 284) AGATCCAAAAGAAGCCGCCTGCTCCATAGCGATTACATGAATATGACTCCACGC 
CGCCCTGGCCCCACAAGGAAACACTACCAGCCTTACGCACCACCTAGAGATTTC

GCTGCCTATCGGAGC
Anti-BCMA CAR CD28 costimulatory region AA (SEQ ID NO. 285)
   RSKRSRLLHSDYMNMTPRRPGPTRKHYQPYAPPRDFAAYRS
Anti-BCMA CAR CD3 zeta DNA (SEQ ID NO. 286) 
AGGGTGAAGTTTTCCAGATCTGCAGATGCACCAGCGTATCAGCAGGGCCAGAAC
CAACTGTATAACGAGCTCAACCTGGGACGCAGGGAAGAGTATGACGTTTTGGAC
AAGCGCAGAGGACGGGACCCTGAGATGGGTGGCAAACCAAGACGAAAAAACCC
CCAGGAGGGTCTCTATAATGAGCTGCAGAAGGATAAGATGGCTGAAGCCTATTC
TGAAATAGGCATGAAAGGAGAGCGGAGAAGGGGAAAAGGGCACGACGGTTTGT
ACCAGGGACTCAGCACTGCTACGAAGGATACTTATGACGCTCTCCACATGCAAG
CCCTGCCACCTAGG
Anti-BCMA CAR CD3 zeta AA (SEQ ID NO. 287) 
RVKFSRSADAPAYQQGQNQLYNELNLGRREEYDVLDKRRGRDPEMGGKPRRKNPQ

EGLYNELQKDKMAEAYSEIGMKGERRRGKGHDGLYQGLSTATKDTYDALHMQAL

PPR
Anti-BCMA CAR VL CDR1 (SEQ ID NO. 288)
   RASQSVSSNLA
Anti-BCMA CAR VL CDR2 (SEQ ID NO. 289)
   SASTRAT
Anti-BCMA CAR VL CDR3 (SEQ ID NO. 290)
   QQHHVWPLTF
Anti-BCMA CAR VH CDR1 (SEQ ID NO. 291)
   GFTFSSY
Anti-BCMA CAR VH CDR2 (SEQ ID NO. 292)
   VISYDGSNKYYADSVKG
Anti-BCMA CAR VH CDR3 (SEQ ID NO. 293)
   VKGPLQEPPYDYGMDV
C185 E7₁₁₋₁₉/HLA-A^{∗}02:01 specific TCR - TCR beta chain variable region DNA (SEQ ID NO. 294) 
atgggcaccagactgttcttttatgtggccctgtgtctgctgtggaccggccacatggatgccggaattacacagagccccagacacaa

agtgaccgagacaggcacccctgtgacactgagatgccaccagaccgagaaccaccgctacatgtactggtacagacaggatccag

gccacggcctgagactgatccactacagctacggcgtgaaggacaccgacaagggcgaagtgtctgacggctacagcgtgtccag

aagcaagaccgaggatttcctgctgaccctggaaagcgccacaagcagccagaccagcgtgtacttttgtgccatcagcggctacaa

gaacaccgaggcctttttcggccaaggcaccaggctgacagtggtg
C185 E7₁₁₋₁₉/HLA-A^{∗}02:01 specific TCR - TCR beta chain variable region AA (SEQ ID NO. 295) 
MGTRLFFYVALCLLWTGHMDAGITQSPRHKVTETGTPVTLRCHQTENHRYMYWYR
QDPGHGLRLIHYSYGVKDTDKGEVSDGYSVSRSKTEDFLLTLESATSSQTSVYFCAIS
GYKNTEAFFGQGTRLTVV
C185 E7₁₁₋₁₉/HLA-A^{∗}02:01 specific TCR - TCR beta chain constant region DNA (SEQ ID NO. 296) 
gaagatctgaagaacgtgttcccacctgaggtggccgtgtttgagccttctgaggccgagatcagccacacacagaaagccacactc

gtgtgcctggccaccggcttttatcccgatcacgtggaactgtcttggtgggtcaacggcaaagaggtgcacagcggcgttagcacag

accctcagcctctgaaagagcagcccgctctgaacgacagcagatactgtctgagcagcagactgagagtgtccgccaccttctggc

agaaccccagaaaccacttcagatgccaggtgcagttctacggcctgtccgagaatgacgagtggacccaggatagagccaagcca

gtgacacagattgtgtctgccgaagcctggggcagagccgattgtggctttacaagcgagagctaccagcagggcgtgctgtctgcc

acaatcctgtatgagatcctgctgggcaaagccactctgtacgctgtgctggtgtctgccctggtgctgatggccatggtcaagagaaa

ggacagcagaggc
C185 E7₁₁₋₁₉/HLA-A^{∗}02:01 specific TCR - TCR beta chain constant region AA (SEQ ID NO. 297) 
EDLKNVFPPEVAVFEPSEAEISHTQKATLVCLATGFYPDHVELSWWVNGKEVHSGV
STDPQPLKEQPALNDSRYCLSSRLRVSATFWQNPRNHFRCQVQFYGLSENDEWTQD
RAKPVTQIVSAEAWGRADCGFTSESYQQGVLSATILYEILLGKATLYAVLVSALVLM
AMVKRKDSRG
C185 E7₁₁₋₁₉/HLA-A^{∗}02:01 specific TCR - P2A peptide (with Furin cleavage site and linker) DNA (SEQ ID NO. 298)
   agagccaagagatctggcagcggcgccacaaactttagcctgctgaaacaggccggcgacgtggaagagaaccctggacct
C185 E7₁₁₋₁₉/HLA-A^{∗}02:01 specific TCR - P2A peptide (with Furin cleavage site and linker) AA (SEQ ID NO. 299)
   RAKRSGSGATNFSLLKQAGDVEENPGP
C185 E7₁₁₋₁₉/HLA-A^{∗}02:01 specific TCR- TCR alpha chain variable region DNA (SEQ ID NO. 300) 
atgaagtccctgcgggtgctgctggttattctgtggctgcagctgagctgggtttggagccagggacagcaagtgatgcagatccctca

gtaccagcacgtgcaagaaggcgaggacttcaccacctactgcaacagcagcacaaccctgagcaacatccagtggtacaagcag

aggcctggcgggcaccctgtgtttctgatccagctggttaagagcggcgaagtgaagaagcagaagcggctgacctttcagttcggc

gaggccaagaagaacagcagcctgcacattaccgccacacagaccaccgacgtgggcacatatttttgcgctggcagagaaggcg

gcagcgagaagctggtttttggcaagggcaccaaactgaccgtgaatccc
C185 E7₁₁₋₁₉/HLA-A^{∗}02:01 specific TCR - TCR alpha chain variable region AA (SEQ ID NO. 301) 
MKSLRVLLVILWLQLSWVWSQGQQVMQIPQYQHVQEGEDFTTYCNSSTTLSNIQW
YKQRPGGHPVFLIQLVKSGEVKKQKRLTFQFGEAKKNSSLHITATQTTDVGTYFCAG
REGGSEKLVFGKGTKLTVNP
C185 E7₁₁₋₁₉/HLA-A^{∗}02:01 specific TCR - TCR alpha chain constant region DNA (SEQ ID NO. 302) 
gacattcagaaccccgatccagccgtgtaccagctgagagacagcaagagcagcgacaagagcgtgtgtctgttcaccgacttcgac

tcccagaccaatgtgtcccagagcaaggactccgacgtgtacatcaccgataagaccgtgctggacatgcggagcatggacttcaag

agcaatagcgccgtggcttggagcaacaagagcgactttgcctgcgccaacgccttcaacaacagcatcatccccgaggacacattc

ttcccaagtcctgagagcagctgcgacgtgaagctggtggaaaagagcttcgagacagacaccaacctgaacttccagaacctgagc

gtgatcggcttcagaatcctgctgctgaaggtggccggcttcaacctgctgatgactctgagactgtggtccagctgaattcggatccaa

gcttaggcctgctcgctttcttgctgtcccatttctattaaaggttcctttgttc
C185 E7₁₁₋₁₉/HLA-A^{∗}02:01 specific TCR - TCR alpha chain constant region AA (SEQ ID NO. 303) 
DIQNPDPAVYQLRDSKSSDKSVCLFTDFDSQTNVSQSKDSDVYITDKTVLDMRSMDF
KSNSAVAWSNKSDFACANAFNNSIIPEDTFFPSPESSCDVKLVEKSFETDTNLNFQNL
SVIGFRILLLKVAGFNLLMTLRLWSS
C185 E7₁₁₋₁₉/HLA-A^{∗}02:01 specific TCR - alpha CDR1 (SEQ ID NO. 304)
   TTLSN
C185 E7₁₁₋₁₉/HLA-A^{∗}02:01 specific TCR - alpha CDR2 (SEQ ID NO. 305)
   LVKSGEV
C185 E7₁₁₋₁₉/HLA-A^{∗}02:01 specific TCR - alpha CDR3 (SEQ ID NO. 306)
   AGREGGSEKLV
C185 E7₁₁₋₁₉/HLA-A^{∗}02:01 specific TCR - beta CDR1 (SEQ ID NO. 307)
   ENHRY
C185 E7₁₁₋₁₉/HLA-A^{∗}02:01 specific TCP-beta CDR2 (SEQ ID NO. 308)
   SYGVKD
C185 E7₁₁₋₁₉/HLA-A^{∗}02:01 specific TCR - beta CDR3 (SEQ ID NO. 309)
   AISGYKNTEAF
E7₁₁₋₁₉/HLA-A^{∗}02:01 specific TCR - TCR beta chain variable region DNA
   (SEQ ID NO. 310) 
atgggacctggattgctttgttgggccctgctgtgtctgcttggagctggacttgtggatgccggcgtgacacagtctcccacacacctg

atcaagaccagaggccagcaagtgaccctgagatgtagccctaagagcggccacgacaccgtgtcttggtatcagcaggctcttggc

cagggacctcagttcatcttccagtactacgaggaagaggaacggcagcggggcaacttccctgatagattctctggccatcagttcc

ccaactacagcagcgagctgaacgtgaacgctctgctgctgggcgatagcgccctgtatctgtgtgccagttctcttggttggagaggc

ggcagatacaacgagcagttctttggccctggcaccagactgaccgtgctg
E7₁₁₋₁₉/HLA-A^{∗}02:01 specific TCR - TCR beta chain variable region AA (SEQ ID NO. 311) 
MGPGLLCWALLCLLGAGLVDAGVTQSPTHLIKTRGQQVTLRCSPKSGHDTVSWYQ
QALGQGPQFIFQYYEEEERQRGNFPDRFSGHQFPNYSSELNVNALLLGDSALYLCAS
SLGWRGGRYNEQFFGPGTRLTVL
E7₁₁₋₁₉/HLA-A^{∗}02:01 specific TCR - TCR beta chain constant region DNA (SEQ ID NO. 312) 
gaagatctgaagaacgtgttcccacctgaggtggccgtgtttgagccttctgaggccgagatcagccacacacagaaagccacactc

gtgtgcctggccaccggcttttatcccgatcacgtggaactgtcttggtgggtcaacggcaaagaggtgcacagcggcgttagcacag

accctcagcctctgaaagagcagcccgctctgaacgacagcagatactgtctgagcagcagactgagagtgtccgccaccttctggc

agaaccccagaaaccacttcagatgccaggtgcagttctacggcctgtccgagaatgacgagtggacccaggatagagccaagcca

gtgacacagattgtgtctgccgaagcctggggcagagccgattgtggctttacaagcgagagctaccagcagggcgtgctgtctgcc

acaatcctgtatgagatcctgctgggcaaagccactctgtacgctgtgctggtgtctgccctggtgctgatggccatggtcaagagaaa

ggacagcagaggc
E7₁₁₋₁₉/HLA-A^{∗}02:01 specific TCR - TCR beta chain constant region AA (SEQ ID NO. 313) 
EDLKNVFPPEVAVFEPSEAEISHTQKATLVCLATGFYPDHVELSWWVNGKEVHSGV
STDPQPLKEQPALNDSRYCLSSRLRVSATFWQNPRNHFRCQVQFYGLSENDEWTQD
RAKPVTQIVSAEAWGRADCGFTSESYQQGVLSATILYEILLGKATLYAVLVSALVLM
AMVKRKDSRG
E7₁₁₋₁₉/HLA-A^{∗}02:01 specific TCR - P2A (with Furin cleavage site and linker) DNA (SEQ ID NO. 314)
   agagccaagagatctggcagcggcgccacaaactttagcctgctgaaacaggccggcgacgtggaagagaaccctggacct
E7₁₁₋₁₉/HLA-A^{∗}02:01 specific TCR - P2A (with Furin cleavage site and linker) AA (SEQ ID NO. 315)
   RAKRSGSGATNFSLLKQAGDVEENPGP
E7₁₁₋₁₉/HLA-A^{∗}02:01 specific TCR - TCR alpha chain variable region DNA (SEQ ID NO. 316) 
atgaagtccctgcgggtgctgctggttattctgtggctgcagctgagctgggtttggagccagggacagaacatcgaccagcctaccg

agatgacagccaccgaaggcgccatcgtgcagatcaattgcacctaccagaccagcggcttcaacggcctgttctggtatcaacagc

atgccggcgaggcccctaccttcctgagctataatgtgctggacggcctggaagaaaagggcagattcagcagcttcctgtccagaa

gcaagggctacagctacctgctgctgaaagaactccagatgaaggacagcgcctcctacctgtgtgcctccgtggatggaaacaaca

gactggccttcggcaagggcaaccaggtggtggtcatcccc
E7₁₁₋₁₉/HLA-A^{∗}02:01 specific TCR - TCR alpha chain variable region AA (SEQ ID NO. 317) 
MKSLRVLLVILWLQLSWVWSQGQNIDQPTEMTATEGAIVQINCTYQTSGFNGLFWY
QQHAGEAPTFLSYNVLDGLEEKGRFSSFLSRSKGYSYLLLKELQMKDSASYLCASVD
GNNRLAFGKGNQVVVIP
E7₁₁₋₁₉/HLA-A^{∗}02:01 specific TCR - TCR alpha chain constant region DNA (SEQ ID NO. 318) 
gacattcagaaccccgatccagccgtgtaccagctgagagacagcaagagcagcgacaagagcgtgtgtctgttcaccgacttcgac

tcccagaccaatgtgtcccagagcaaggactccgacgtgtacatcaccgataagaccgtgctggacatgcggagcatggacttcaag

agcaatagcgccgtggcttggagcaacaagagcgactttgcctgcgccaacgccttcaacaacagcatcatccccgaggacacattc

ttcccaagtcctgagagcagctgcgacgtgaagctggtggaaaagagcttcgagacagacaccaacctgaacttccagaacctgagc

gtgatcggcttcagaatcctgctgctgaaggtggccggcttcaacctgctgatgactctgagactgtggtccagctgaattcggatccaa
 gcttaggcctgctcgctttcttgctgtcccatttctattaaaggttcctttgttc
E7₁₁₋₁₉/HLA-A^{∗}02:01 specific TCR - TCR alpha chain constant region AA (SEQ ID NO. 319) 
DIQNPDPAVYQLRDSKSSDKSVCLFTDFDSQTNVSQSKDSDVYITDKTVLDMRSMDF
KSNSAVAWSNKSDFACANAFNNSIIPEDTFFPSPESSCDVKLVEKSFETDTNLNFQNL
SVIGFRILLLKVAGFNLLMTLRLWSS
E7₁₁₋₁₉/HLA-A^{∗}02:01 specific TCR - alpha CDR1 (SEQ ID NO. 320)
   TSGFNG
E7₁₁₋₁₉/HLA-A^{∗}02:01 specific TCR - alpha CDR2 (SEQ ID NO. 321)
   NVLDGL
E7₁₁₋₁₉/HLA-A^{∗}02:01 specific TCR - alpha CDR3 (SEQ ID NO. 322)
   ASVDGNNRLA
E7₁₁₋₁₉/HLA-A^{∗}02:01 specific TCR - beta CDR1 (SEQ ID NO. 323)
   SGHDT
E7₁₁₋₁₉/HLA-A^{∗}02:01 specific TCR - beta CDR2 (SEQ ID NO. 324)
   YYEEEE
E7₁₁₋₁₉/HLA-A^{∗}02:01 specific TCR - beta CDR3 (SEQ ID NO. 325)
   ASSLGWRGGRYNEQF

In particular, the present invention pertains to the following:
1. A method of performing a patient-specific immunotherapy procedure, the method comprising:
   receiving, by a computing device, a cell order request to create transfected T cells for a patient;
   generating, by the computing device, a patient-specific identifier associated with the cell order request, the patient-specific identifier comprising a patient identity element, a sales order identifier, and a cell order lot number;
   initiating, by the computing device, a process to create transfected T cells for infusion into the patient's bloodstream, the process comprising:
      performing a leukapheresis procedure on a sample of the patient's blood to collect T cells from the sample,
      transferring the collected T cells to a container;
      labeling the container with the patient-specific identifier,
      transmitting the collected T cells to a manufacturing facility,
      creating transfected T cells from the collected T cells using a cell modification technique,
      receiving the transfected T cells from the manufacturing facility, and
      infusing the transfected T cells into the patient's bloodstream, wherein the computing device records a tracking event for each step in the process, each
   tracking event including the patient-specific identifier, and wherein the tracking events comprise a chain of custody of the patient's T cells during the
   process.
2. The method of item 1, wherein the transfected T cells are created by transfecting the collected T cells with a polynucleotide encoding a chimeric antigen receptor (CAR), the CAR comprising an antigen binding molecule that specifically binds to a target molecule.
3. The method of item 2, wherein the antigen binding molecule is a single chain variable fragment.
4. The method of item 2, wherein the target molecule is a blood borne cancer-associated antigen.
5. The method of item 2, wherein the target molecule is a viral infection-associated antigen.
6. The method of item 2, wherein the chimeric antigen receptor (CAR) further comprises at least one costimulatory domain.
7. The method of item 2, wherein the chimeric antigen receptor (CAR) further comprises at least one activating domain.
8. The method of item 2, wherein the polynucleotide is a component of a vector.
9. The method of item 1, wherein the transfected T cells are created by transfecting the collected T cells with a polynucleotide encoding a T cell receptor (TCR).
10. The method of item 9, wherein the TCR binds to a tumor-associated antigen.
11. The method of item 9, wherein the TCR binds to a viral infection-associated antigen.
12. The method of item 9, wherein the polynucleotide is a component of a vector.
13. The method of item 1, wherein initiating a process to create transfected T cells further comprises:
   receiving, by the computing device, indicia that the transfected T cells have been shipped from a first site after being created; and
   receiving indicia that the transfected T cells have been received at a second site before being infused,
   wherein the computing device records a tracking event upon receiving the indicia that the transfected T cells have been shipped, and
   wherein the computing device records a tracking event upon receiving the indicia that the transfected T cells have been received at a second site.
14. The method of item 1, wherein the patient identity element comprises a first patient ID associated with the immunotherapy procedure and a second patient ID associated with a facility that administers one or more of: the leukapheresis procedure or the infusion of the transfected T cells.
15. The method of item 1, wherein the computing device stores the tracking events in an ordered sequence.
16. A method for tracking a cell order during an immunotherapy procedure, the method comprising:
   receiving, by a computing device, a cell order request for creating transfected T cells for a patient;
   generating, by the computing device, a patient-specific identifier associated with the cell order request, the patient-specific identifier comprising a patient identity element, a sales order identifier, and a cell order lot number;
   monitoring, by the computing device, a process to create transfected T cells for infusion into the patient's bloodstream, the process comprising:
      receiving indicia that a leukapheresis procedure has been performed on a sample of the patient's blood to collect T cells from the sample,
      receiving indicia that the collected T cells have been transferred to a container,
      receiving indicia that the container has been labeled with the patient-specific identifier,
      receiving indicia that the collected T cells have been transmitted to a manufacturing facility,
      receiving indicia that transfected T cells have been created from the collected T cells using a cell modification technique,
      receiving indicia that the transfected T cells have been received from the manufacturing facility, and
      receiving indicia that the transfected T cells have been infused into the patient's bloodstream,
   recording, by the computing device, a tracking event when indicia is received, each tracking event including the patient-specific identifier, and
   maintaining, by the computing device, a chain of custody of the patient's T cells by storing the tracking events during the process.
17. The method of item 16, wherein the transfected T cells are created by transfecting the collected T cells with a polynucleotide encoding a chimeric antigen receptor (CAR), the CAR comprising an antigen binding molecule that specifically binds to a target molecule.
18. The method of item 17, wherein the antigen binding molecule is a single chain variable fragment.
19. The method of item 17, wherein the target molecule is a blood borne cancer-associated antigen.
20. The method of item 17, wherein the target molecule is a viral infection-associated antigen.
21. The method of item 17, wherein the chimeric antigen receptor (CAR) further comprises at least one costimulatory domain.
22. The method of item 17, wherein the chimeric antigen receptor (CAR) further comprises at least one activating domain.
23. The method of item 17, wherein the polynucleotide is a component of a vector.
24. The method of item 16, wherein the transfected T cells are created by transfecting the collected T cells with a polynucleotide encoding a T cell receptor (TCR).
25. The method of item 24, wherein the TCR binds to a tumor-associated antigen.
26. The method of item 24, wherein the TCR binds to a viral infection-associated antigen.
27. The method of item 24, wherein the polynucleotide is a component of a vector.
28. The method of item 16, wherein initiating a process to create transfected T cells further comprises:
   receiving, by the computing device, indicia that the transfected T cells have been shipped from a first site after being created; and
   receiving indicia that the transfected T cells have been received at a second site before being infused,
   wherein the computing device records a tracking event upon receiving the indicia that the transfected T cells have been shipped, and
   wherein the computing device records a tracking event upon receiving the indicia that the transfected T cells have been received at a second site.
29. The method of item 16, wherein the patient identity element comprises a first patient ID associated with the immunotherapy procedure and a second patient ID associated with a facility that administers one or more of: the leukapheresis procedure or the infusion of the transfected T cells.
30. The method of item 16, wherein the computing device stores the tracking events in an ordered sequence.
31. A method of performing a patient-specific immunotherapy procedure, the method comprising:
   receiving a cell order request to create transfected T cells for a patient;
   generating, by an event tracking module executed on a processor, a patient-specific identifier associated with the cell order request;
   initiating a process to create transfected T cells for infusion into the patient's bloodstream, the process comprising:
      performing a leukapheresis procedure on a sample of the patient's blood to collect T cells from the sample,
      transferring the collected T cells to a container;
      labeling the container with the patient-specific identifier,
      transmitting the collected T cells to a manufacturing facility,
      creating transfected T cells from the collected T cells using a cell modification technique,
      receiving the transfected T cells from the manufacturing facility, and
      infusing the transfected T cells into the patient's bloodstream,
   receiving at the event tracking module, from a first client device located at the point of the leukapheresis procedure, a first tracking event that confirms the leukapheresis procedure and contains the patient-specific identifier;
   integrating, by the event tracking module, the first tracking event in a data structure pertaining to the patient-specific identifier, wherein the data structure is stored in a database and the integrating step records a first timestamp with the first tracking event;
   receiving, at the event tracking module, from a second client device located at the manufacturing facility, a second tracking event that confirms the receipt of the collected T cells at the manufacturing facility and contains the patient-specific identifier; and
   integrating, by the event tracking module, the second tracking event in the data structure pertaining to the patient-specific identifier, wherein the integrating step records a second timestamp with the second tracking event.
32. A method of performing a patient-specific immunotherapy procedure, the method comprising:
   receiving, by a tracking module executed on a processor, a cell order request to create transfected T cells for a patient;
   generating, by the tracking module, a patient-specific identifier associated with the cell order request, the patient-specific identifier identifying a patient, and a cell order lot;
   generating, in a database, a data record for tracking the cell order, the data record identified in the database according to the patient-specific identifier;
   receiving, by the tracking module, a first tracking event indicating that the collected T cells are ready for shipment to a manufacturing facility;
   updating the data record corresponding to the patient-specific identifier in accordance with the first tracking event;
   receiving, by the tracking module based on the container having been received by the manufacturing facility, a second tracking event indicating that the collected T cells have been received by a manufacturing facility;
   updating the data record corresponding to the patient-specific identifier in accordance with the second tracking event;
   receiving, by the tracking module based on the manufacturing facility having created transfected T cells from the collected T cells using a cell modification technique, a third tracking event indicating that the transfected T cells have been created;
   updating the data record corresponding to the patient-specific identifier in accordance with the third tracking event;
   receiving, by the tracking module based on the transfected T cells having been received from the manufacturing facility, a fourth tracking event indicating that the transfected T cells have been received;
   updating the data record corresponding to the patient-specific identifier in accordance with the fourth tracking event;
   receiving, by the tracking module based on the transfected T cells having been infused into the patient's bloodstream, a fifth tracking event indicating that the transfected T cells have been infused into the patient's bloodstream; and updating the data record corresponding to the patient-specific identifier in accordance with the fifth tracking event,
   wherein each of the first, second, third, fourth, and fifth tracking events contains the patient-specific identifier, a timestamp, and an event identifier, and
   wherein the data record corresponding to the patient-specific identifier stores, in an ordered sequence, the first, second, third, fourth, and fifth tracking events when the data record is updated in accordance with the respective events.

## Claims

1. Transfected T cells for use in a patient-specific method of immunotherapy, the method comprising:
(i) obtaining T cells collected by leukapheresis from the patient's blood; and
(ii) transferring the collected T cells to a container which is labeled with a patient-specific identifier;
(iii) transmitting the container comprising the collected T cells to a manufacturing facility;
(iv) creating transfected T cells from the collected T cells using a cell modification technique, wherein the transfected T cells are created by transfecting the collected T cells with a polynucleotide encoding (a) a chimeric antigen receptor (CAR) or (b) a T cell receptor (TCR);
(v) receiving the transfected T cells from the manufacturing facility; and
(vi) infusing the transfected T cells into the patient's bloodstream,
wherein a tracking event is recorded for each step of the method, each tracking event including the patient-specific identifier, and wherein the tracking events comprise a chain of custody of the patient's T cells during the method.

2. The transfected T cells for use of claim 1, wherein the transfected T cells are created by transfecting the collected T cells with a polynucleotide encoding a chimeric antigen receptor (CAR), the CAR comprising an antigen binding molecule that specifically binds to a target molecule.

3. The transfected T cells for use of claim 2, wherein the target molecule is a blood borne cancer-associated antigen.

4. The transfected T cells for use of claim 2, wherein the target molecule is a viral infection-associated antigen.

5. The transfected T cells for use of claim 1, wherein the chimeric antigen receptor (CAR) further comprises at least one costimulatory domain or at least one activating domain.

6. The transfected T cells for use of claim 1, wherein the polynucleotide is a component of a vector.

7. The transfected T cells for use of claim 1, wherein the TCR binds to a tumor-associated antigen or to a viral infection-associated antigen.

8. The transfected T cells for use of claim 1, wherein the patient identity element comprises a first patient ID associated with the immunotherapy procedure and a second patient ID associated with a facility that administers one or more of: the leukapheresis procedure or the infusion of the transfected T cells.

9. The transfected T cells for use of claim 3, wherein
(a) the costimulatory domain comprises CD28 or CD8, wherein the costimulatory domain preferably comprises a sequence selected from SEQ ID NO: 2, SEQ ID NO: 4, SEQ ID NO: 6, SEQ ID NO: 8, and SEQ ID NO: 14 or
(b) the activating domain comprises CD3.

10. The transfected T cells for use of claim 9, wherein the activating domain comprises a sequence according to SEQ ID NO: 10.

11. The transfected T cells for use of claim 1, wherein the CAR comprises one of the following:
(a) an anti-CD19 scFv, CD8, and 4-1BB;
(b) an anti-BCMA scFv and the CD8;
(c) an anti-CD19 scFv, CD28, and the 4-1BB;
(d) an anti-CD22 scFv and the CD8;
(e) the anti-CD19 scFv, the CD28, and EGFRt/19-28z/4-1BBL;
(f) an anti-MUC16 scFv and the CD28;
(g) an anti-CD 171;
(h) an anti-CD123 and the CD28;
(i) an anti-BCMA, the CD8, and the 4-IBB;
(j) an anti-CD 19 and CD28; and
(k) an anti-CD19 and the CD8; or the CD28.

12. The transfected T cells for use of claim 1, wherein the CAR comprises one of the following:
(a) a leader sequence (CSF2RA), an anti-CD 19 scFv, a Whitlow linker, a CD28 spacer, a CD28 costimulatory domain, and CD3 zeta;
(b) a leader sequence (CD8), an anti-CD19 scFv, a Whitlow linker, a CD28T spacer, a CD28 costimulatory domain, and CD3 zeta;
(c) a leader sequence (CD8a), an anti-CD19 scFv, a Whitlow linker, a CD8a spacer and transmembrane domain, a CD28 costimulatory domain, and CD3 zeta;
(d) a leader sequence (CD8), an anti-CLL-1 scFv, a G4S linker, a Minispacer, a CD28T, an intracellular costimulatory region of CD28, and CD3 zeta; and
(e) a leader sequence (CD8a), an anti-BCMA scFv, a Whitlow linker, a CD28T spacer, a CD28 costimulatory domain, and CD3 zeta.

13. The transfected T cells for use of claim 12, wherein the CAR of (a) comprises a sequence of SEQ ID NO: 147 and wherein the CAR of (b) comprises a sequence of SEQ ID NO: 149 and wherein the CAR of (c) comprises a sequence of SEQ ID NO: 151 and wherein the CAR of (d) comprises a sequence of SEQ ID NO: 155 and wherein the CAR of (e) comprises a sequence of SEQ ID NO: 157.

14. The transfected T cells for use of claim 1, wherein the TCR comprises one of the following:
(a) an AV38-2 variable alpha chain, a BV7-2 variable beta chain, a murine constant alpha domain, a murine constant beta domain, TRAJ40, Furin-SG SG-P2A, and TRBJ1-3; and
(b) a TCR beta chain variable region, a TCR beta chain constant region, a P2A peptide with a Furin cleavage site and a linker, a TCR alpha chain variable region, and a TCR alpha chain constant region.

15. The transfected T cells for use of claim 14, wherein the TCR of (a) comprises a sequence of SEQ ID NO: 153 and wherein the TCR of (b) comprises a sequence of SEQ ID NO: 159 or SEQ ID NO: 161.
